(19) Europäisches Patentamt / European Patent Office / Office européen des brevets

(11) **EP 3 828 544 A1**

(12) **EUROPEAN PATENT APPLICATION**

(43) Date of publication:
02.06.2021 Bulletin 2021/22

(51) Int Cl.:
*G01N 33/53* (2006.01)   *G01N 33/68* (2006.01)
*C12Q 1/6883* (2018.01)

(21) Application number: 20201404.9

(22) Date of filing: 08.01.2012

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR

(30) Priority: 08.01.2011 US 201161430989 P
08.01.2011 US 201161430986 P
08.01.2011 US 201161430977 P
08.01.2011 US 201161430979 P
08.01.2011 US 201161430980 P
08.01.2011 US 201161430982 P
08.01.2011 US 201161430987 P
08.01.2011 US 201161430981 P
08.01.2011 US 201161430984 P
08.01.2011 US 201161430983 P
08.01.2011 US 201161430988 P
08.01.2011 US 201161430978 P
08.01.2011 US 201161430985 P

(62) Document number(s) of the earlier application(s) in
accordance with Art. 76 EPC:
16001228.2 / 3 133 398
12732048.9 / 2 661 626

(71) Applicant: Astute Medical, Inc.
San Diego, CA 92121 (US)

(72) Inventors:
• ANDERBERG, Joseph
Encinitas, CA 92024 (US)
• GRAY, Jeff
Solana Beach, CA 92075 (US)
• MCPHERSON, Paul
Encinitas, CA 92024 (US)
• NAKAMURA, Kevin
Encinitas, CA 92024 (US)
• KAMPF, James Patrick
San Deigo, CA 92130 (US)

(74) Representative: **Wilding, James Roger et al**
**Mathys & Squire**
**The Shard**
**32 London Bridge Street**
**London SE1 9SG (GB)**

Remarks:
•This application was filed on 12-10-2020 as a
divisional application to the application mentioned
under INID code 62.
•Claims filed after the date of filing of the application
/ after the date of receipt of the divisional application
(Rule 68(4) EPC).

(54) **METHODS AND COMPOSITIONS FOR DIAGNOSIS AND PROGNOSIS OF RENAL INJURY AND RENAL FAILURE**

(57) The present invention relates to methods and compositions for monitoring, diagnosis, prognosis, and determination of treatment regimens in subjects suffering from or suspected of having a renal injury. In particular, the invention relates to using a one or more assays configured to detect a kidney injury marker selected from the group consisting of Proheparin-binding EGF-like growth factor, Tenascin C, Angiopoietin-related protein 4, Fibroblast growth factor 19, Fibroblast growth factor 21, Heparin-binding growth factor 1, Angiopoietin-related protein 6, Proepiregulin, Probetacellulin, Amphiregulin, Angiogenin, Thrombospondin-2, and Collagen alpha-1(XVIII) chain as diagnostic and prognostic biomarkers in renal injuries.

EP 3 828 544 A1

SEQUENCE LISTING

<110> ASTUTE MEDICAL, INC.

<120> METHODS AND COMPOSITIONS FOR DIAGNOSIS AND PROGNOSIS OF RENAL
INJURY AND RENAL FAILURE

<130> P64051EP-D2-PCT

<140> EP Divisional application based on EP16001228.2
<141> 2012-01-08

<150> 61/430,977
<151> 2011-01-08

<150> 61/430,978
<151> 2011-01-08

<150> 61/430,979
<151> 2011-01-08

<150> 61/430,980
<151> 2011-01-08

<150> 61/430,981
<151> 2011-01-08

<150> 61/430,982
<151> 2011-01-08

<150> 61/430,983
<151> 2011-01-08

<150> 61/430,984
<151> 2011-01-08

<150> 61/430,985
<151> 2011-01-08

<150> 61/430,986
<151> 2011-01-08

<150> 61/430,987
<151> 2011-01-08

<150> 61/430,988
<151> 2011-01-08

<150> 61/430,989
<151> 2011-01-08

<160> 17

<170> PatentIn version 3.5

<210> 1
<211> 208
<212> PRT
<213> Homo sapiens

<400> 1
Met Lys Leu Leu Pro Ser Val Val Leu Lys Leu Phe Leu Ala Ala Val
1               5                   10                  15

```
Leu Ser Ala Leu Val Thr Gly Glu Ser Leu Glu Arg Leu Arg Arg Gly
            20                  25                  30

Leu Ala Ala Gly Thr Ser Asn Pro Asp Pro Pro Thr Val Ser Thr Asp
            35                  40                  45

Gln Leu Leu Pro Leu Gly Gly Gly Arg Asp Arg Lys Val Arg Asp Leu
        50                  55                  60

Gln Glu Ala Asp Leu Asp Leu Leu Arg Val Thr Leu Ser Ser Lys Pro
65                  70                  75                  80

Gln Ala Leu Ala Thr Pro Asn Lys Glu Glu His Gly Lys Arg Lys Lys
                85                  90                  95

Lys Gly Lys Gly Leu Gly Lys Lys Arg Asp Pro Cys Leu Arg Lys Tyr
            100                 105                 110

Lys Asp Phe Cys Ile His Gly Glu Cys Lys Tyr Val Lys Glu Leu Arg
            115                 120                 125

Ala Pro Ser Cys Ile Cys His Pro Gly Tyr His Gly Glu Arg Cys His
    130                 135                 140

Gly Leu Ser Leu Pro Val Glu Asn Arg Leu Tyr Thr Tyr Asp His Thr
145                 150                 155                 160

Thr Ile Leu Ala Val Val Ala Val Val Leu Ser Ser Val Cys Leu Leu
                165                 170                 175

Val Ile Val Gly Leu Leu Met Phe Arg Tyr His Arg Arg Gly Gly Tyr
            180                 185                 190

Asp Val Glu Asn Glu Glu Lys Val Lys Leu Gly Met Thr Asn Ser His
            195                 200                 205

<210> 2
<211> 2201
<212> PRT
<213> Homo sapiens

<400> 2
Met Gly Ala Met Thr Gln Leu Leu Ala Gly Val Phe Leu Ala Phe Leu
1               5                   10                  15

Ala Leu Ala Thr Glu Gly Gly Val Leu Lys Lys Val Ile Arg His Lys
            20                  25                  30
```

271

Arg Gln Ser Gly Val Asn Ala Thr Leu Pro Glu Glu Asn Gln Pro Val
35                    40                 45

Val Phe Asn His Val Tyr Asn Ile Lys Leu Pro Val Gly Ser Gln Cys
50                    55                 60

Ser Val Asp Leu Glu Ser Ala Ser Gly Glu Lys Asp Leu Ala Pro Pro
65            70              75                    80

Ser Glu Pro Ser Glu Ser Phe Gln Glu His Thr Val Asp Gly Glu Asn
            85              90              95

Gln Ile Val Phe Thr His Arg Ile Asn Ile Pro Arg Arg Ala Cys Gly
        100             105             110

Cys Ala Ala Ala Pro Asp Val Lys Glu Leu Leu Ser Arg Leu Glu Glu
        115             120             125

Leu Glu Asn Leu Val Ser Ser Leu Arg Glu Gln Cys Thr Ala Gly Ala
    130             135             140

Gly Cys Cys Leu Gln Pro Ala Thr Gly Arg Leu Asp Thr Arg Pro Phe
145             150             155             160

Cys Ser Gly Arg Gly Asn Phe Ser Thr Glu Gly Cys Gly Cys Val Cys
            165             170             175

Glu Pro Gly Trp Lys Gly Pro Asn Cys Ser Glu Pro Glu Cys Pro Gly
            180             185             190

Asn Cys His Leu Arg Gly Arg Cys Ile Asp Gly Gln Cys Ile Cys Asp
        195             200             205

Asp Gly Phe Thr Gly Glu Asp Cys Ser Gln Leu Ala Cys Pro Ser Asp
    210             215             220

Cys Asn Asp Gln Gly Lys Cys Val Asn Gly Val Cys Ile Cys Phe Glu
225             230             235             240

Gly Tyr Ala Gly Ala Asp Cys Ser Arg Glu Ile Cys Pro Val Pro Cys
            245             250             255

Ser Glu Glu His Gly Thr Cys Val Asp Gly Leu Cys Val Cys His Asp
        260             265             270

Gly Phe Ala Gly Asp Asp Cys Asn Lys Pro Leu Cys Leu Asn Asn Cys
    275             280             285

272

```
Tyr Asn Arg Gly Arg Cys Val Glu Asn Glu Cys Val Cys Asp Glu Gly
    290             295             300

Phe Thr Gly Glu Asp Cys Ser Glu Leu Ile Cys Pro Asn Asp Cys Phe
305             310             315             320

Asp Arg Gly Arg Cys Ile Asn Gly Thr Cys Tyr Cys Glu Glu Gly Phe
            325             330             335

Thr Gly Glu Asp Cys Gly Lys Pro Thr Cys Pro His Ala Cys His Thr
        340             345             350

Gln Gly Arg Cys Glu Glu Gly Gln Cys Val Cys Asp Glu Gly Phe Ala
        355             360             365

Gly Val Asp Cys Ser Glu Lys Arg Cys Pro Ala Asp Cys His Asn Arg
    370             375             380

Gly Arg Cys Val Asp Gly Arg Cys Glu Cys Asp Asp Gly Phe Thr Gly
385             390             395             400

Ala Asp Cys Gly Glu Leu Lys Cys Pro Asn Gly Cys Ser Gly His Gly
            405             410             415

Arg Cys Val Asn Gly Gln Cys Val Cys Asp Glu Gly Tyr Thr Gly Glu
        420             425             430

Asp Cys Ser Gln Leu Arg Cys Pro Asn Asp Cys His Ser Arg Gly Arg
        435             440             445

Cys Val Glu Gly Lys Cys Val Cys Glu Gln Gly Phe Lys Gly Tyr Asp
    450             455             460

Cys Ser Asp Met Ser Cys Pro Asn Asp Cys His Gln His Gly Arg Cys
465             470             475             480

Val Asn Gly Met Cys Val Cys Asp Asp Gly Tyr Thr Gly Glu Asp Cys
            485             490             495

Arg Asp Arg Gln Cys Pro Arg Asp Cys Ser Asn Arg Gly Leu Cys Val
        500             505             510

Asp Gly Gln Cys Val Cys Glu Asp Gly Phe Thr Gly Pro Asp Cys Ala
        515             520             525

Glu Leu Ser Cys Pro Asn Asp Cys His Gly Gln Gly Arg Cys Val Asn
```

530                          535                          540

Gly Gln Cys Val Cys His Glu Gly Phe Met Gly Lys Asp Cys Lys Glu
545                     550                     555                     560

Gln Arg Cys Pro Ser Asp Cys His Gly Gln Gly Arg Cys Val Asp Gly
                    565                     570                     575

Gln Cys Ile Cys His Glu Gly Phe Thr Gly Leu Asp Cys Gly Gln His
               580                     585                     590

Ser Cys Pro Ser Asp Cys Asn Asn Leu Gly Gln Cys Val Ser Gly Arg
          595                     600                     605

Cys Ile Cys Asn Glu Gly Tyr Ser Gly Glu Asp Cys Ser Glu Val Ser
     610                     615                     620

Pro Pro Lys Asp Leu Val Val Thr Glu Val Thr Glu Glu Thr Val Asn
625                     630                     635                     640

Leu Ala Trp Asp Asn Glu Met Arg Val Thr Glu Tyr Leu Val Val Tyr
               645                     650                     655

Thr Pro Thr His Glu Gly Gly Leu Glu Met Gln Phe Arg Val Pro Gly
          660                     665                     670

Asp Gln Thr Ser Thr Ile Ile Gln Glu Leu Glu Pro Gly Val Glu Tyr
          675                     680                     685

Phe Ile Arg Val Phe Ala Ile Leu Glu Asn Lys Lys Ser Ile Pro Val
     690                     695                     700

Ser Ala Arg Val Ala Thr Tyr Leu Pro Ala Pro Glu Gly Leu Lys Phe
705                     710                     715                     720

Lys Ser Ile Lys Glu Thr Ser Val Glu Val Glu Trp Asp Pro Leu Asp
               725                     730                     735

Ile Ala Phe Glu Thr Trp Glu Ile Ile Phe Arg Asn Met Asn Lys Glu
          740                     745                     750

Asp Glu Gly Glu Ile Thr Lys Ser Leu Arg Arg Pro Glu Thr Ser Tyr
          755                     760                     765

Arg Gln Thr Gly Leu Ala Pro Gly Gln Glu Tyr Glu Ile Ser Leu His
     770                     775                     780

```
Ile Val Lys Asn Asn Thr Arg Gly Pro Gly Leu Lys Arg Val Thr Thr
785             790             795                 800

Thr Arg Leu Asp Ala Pro Ser Gln Ile Glu Val Lys Asp Val Thr Asp
            805             810                 815

Thr Thr Ala Leu Ile Thr Trp Phe Lys Pro Leu Ala Glu Ile Asp Gly
            820             825             830

Ile Glu Leu Thr Tyr Gly Ile Lys Asp Val Pro Gly Asp Arg Thr Thr
            835             840             845

Ile Asp Leu Thr Glu Asp Glu Asn Gln Tyr Ser Ile Gly Asn Leu Lys
    850             855             860

Pro Asp Thr Glu Tyr Glu Val Ser Leu Ile Ser Arg Arg Gly Asp Met
865             870             875                 880

Ser Ser Asn Pro Ala Lys Glu Thr Phe Thr Thr Gly Leu Asp Ala Pro
            885             890             895

Arg Asn Leu Arg Arg Val Ser Gln Thr Asp Asn Ser Ile Thr Leu Glu
            900             905             910

Trp Arg Asn Gly Lys Ala Ala Ile Asp Ser Tyr Arg Ile Lys Tyr Ala
        915             920             925

Pro Ile Ser Gly Gly Asp His Ala Glu Val Asp Val Pro Lys Ser Gln
    930             935             940

Gln Ala Thr Thr Lys Thr Thr Leu Thr Gly Leu Arg Pro Gly Thr Glu
945             950             955                 960

Tyr Gly Ile Gly Val Ser Ala Val Lys Glu Asp Lys Glu Ser Asn Pro
            965             970             975

Ala Thr Ile Asn Ala Ala Thr Glu Leu Asp Thr Pro Lys Asp Leu Gln
            980             985             990

Val Ser Glu Thr Ala Glu Thr Ser Leu Thr Leu Leu Trp Lys Thr Pro
        995             1000            1005

Leu Ala Lys Phe Asp Arg Tyr Arg Leu Asn Tyr Ser Leu Pro Thr
    1010            1015            1020

Gly Gln Trp Val Gly Val Gln Leu Pro Arg Asn Thr Thr Ser Tyr
    1025            1030            1035
```

275

```
Val Leu  Arg Gly Leu Glu Pro  Gly Gln Glu Tyr Asn  Val Leu Leu
    1040                 1045                 1050

Thr Ala  Glu Lys Gly Arg His  Lys Ser Lys Pro Ala  Arg Val Lys
    1055                 1060                 1065

Ala Ser  Thr Glu Gln Ala Pro  Glu Leu Glu Asn Leu  Thr Val Thr
    1070                 1075                 1080

Glu Val  Gly Trp Asp Gly Leu  Arg Leu Asn Trp Thr  Ala Ala Asp
    1085                 1090                 1095

Gln Ala  Tyr Glu His Phe Ile  Ile Gln Val Gln Glu  Ala Asn Lys
    1100                 1105                 1110

Val Glu  Ala Ala Arg Asn Leu  Thr Val Pro Gly Ser  Leu Arg Ala
    1115                 1120                 1125

Val Asp  Ile Pro Gly Leu Lys  Ala Ala Thr Pro Tyr  Thr Val Ser
    1130                 1135                 1140

Ile Tyr  Gly Val Ile Gln Gly  Tyr Arg Thr Pro Val  Leu Ser Ala
    1145                 1150                 1155

Glu Ala  Ser Thr Gly Glu Thr  Pro Asn Leu Gly Glu  Val Val Val
    1160                 1165                 1170

Ala Glu  Val Gly Trp Asp Ala  Leu Lys Leu Asn Trp  Thr Ala Pro
    1175                 1180                 1185

Glu Gly  Ala Tyr Glu Tyr Phe  Phe Ile Gln Val Gln  Glu Ala Asp
    1190                 1195                 1200

Thr Val  Glu Ala Ala Gln Asn  Leu Thr Val Pro Gly  Gly Leu Arg
    1205                 1210                 1215

Ser Thr  Asp Leu Pro Gly Leu  Lys Ala Ala Thr His  Tyr Thr Ile
    1220                 1225                 1230

Thr Ile  Arg Gly Val Thr Gln  Asp Phe Ser Thr Thr  Pro Leu Ser
    1235                 1240                 1245

Val Glu  Val Leu Thr Glu Glu  Val Pro Asp Met Gly  Asn Leu Thr
    1250                 1255                 1260

Val Thr  Glu Val Ser Trp Asp  Ala Leu Arg Leu Asn  Trp Thr Thr
    1265                 1270                 1275
```

```
Pro Asp Gly Thr Tyr Asp Gln  Phe Thr Ile Gln Val  Gln Glu Ala
    1280                1285                1290

Asp Gln Val Glu Glu Ala His  Asn Leu Thr Val Pro  Gly Ser Leu
    1295                1300                1305

Arg Ser Met Glu Ile Pro Gly  Leu Arg Ala Gly Thr  Pro Tyr Thr
    1310                1315                1320

Val Thr Leu His Gly Glu Val  Arg Gly His Ser Thr  Arg Pro Leu
    1325                1330                1335

Ala Val Glu Val Val Thr Glu  Asp Leu Pro Gln Leu  Gly Asp Leu
    1340                1345                1350

Ala Val Ser Glu Val Gly Trp  Asp Gly Leu Arg Leu  Asn Trp Thr
    1355                1360                1365

Ala Ala Asp Asn Ala Tyr Glu  His Phe Val Ile Gln  Val Gln Glu
    1370                1375                1380

Val Asn Lys Val Glu Ala Ala  Gln Asn Leu Thr Leu  Pro Gly Ser
    1385                1390                1395

Leu Arg Ala Val Asp Ile Pro  Gly Leu Glu Ala Ala  Thr Pro Tyr
    1400                1405                1410

Arg Val Ser Ile Tyr Gly Val  Ile Arg Gly Tyr Arg  Thr Pro Val
    1415                1420                1425

Leu Ser Ala Glu Ala Ser Thr  Ala Lys Glu Pro Glu  Ile Gly Asn
    1430                1435                1440

Leu Asn Val Ser Asp Ile Thr  Pro Glu Ser Phe Asn  Leu Ser Trp
    1445                1450                1455

Met Ala Thr Asp Gly Ile Phe  Glu Thr Phe Thr Ile  Glu Ile Ile
    1460                1465                1470

Asp Ser Asn Arg Leu Leu Glu  Thr Val Glu Tyr Asn  Ile Ser Gly
    1475                1480                1485

Ala Glu Arg Thr Ala His Ile  Ser Gly Leu Pro Pro  Ser Thr Asp
    1490                1495                1500

Phe Ile Val Tyr Leu Ser Gly  Leu Ala Pro Ser Ile  Arg Thr Lys
```

277

```
         1505                      1510                      1515

Thr Ile Ser Ala Thr Ala Thr  Thr Glu Ala Leu Pro  Leu Leu Glu
    1520                 1525                 1530

Asn Leu Thr Ile Ser Asp Ile  Asn Pro Tyr Gly Phe  Thr Val Ser
    1535                 1540                 1545

Trp Met Ala Ser Glu Asn Ala  Phe Asp Ser Phe Leu  Val Thr Val
    1550                 1555                 1560

Val Asp Ser Gly Lys Leu Leu  Asp Pro Gln Glu Phe  Thr Leu Ser
    1565                 1570                 1575

Gly Thr Gln Arg Lys Leu Glu  Leu Arg Gly Leu Ile  Thr Gly Ile
    1580                 1585                 1590

Gly Tyr Glu Val Met Val Ser  Gly Phe Thr Gln Gly  His Gln Thr
    1595                 1600                 1605

Lys Pro Leu Arg Ala Glu Ile  Val Thr Glu Ala Glu  Pro Glu Val
    1610                 1615                 1620

Asp Asn Leu Leu Val Ser Asp  Ala Thr Pro Asp Gly  Phe Arg Leu
    1625                 1630                 1635

Ser Trp Thr Ala Asp Glu Gly  Val Phe Asp Asn Phe  Val Leu Lys
    1640                 1645                 1650

Ile Arg Asp Thr Lys Lys Gln  Ser Glu Pro Leu Glu  Ile Thr Leu
    1655                 1660                 1665

Leu Ala Pro Glu Arg Thr Arg  Asp Ile Thr Gly Leu  Arg Glu Ala
    1670                 1675                 1680

Thr Glu Tyr Glu Ile Glu Leu  Tyr Gly Ile Ser Lys  Gly Arg Arg
    1685                 1690                 1695

Ser Gln Thr Val Ser Ala Ile  Ala Thr Thr Ala Met  Gly Ser Pro
    1700                 1705                 1710

Lys Glu Val Ile Phe Ser Asp  Ile Thr Glu Asn Ser  Ala Thr Val
    1715                 1720                 1725

Ser Trp Arg Ala Pro Thr Ala  Gln Val Glu Ser Phe  Arg Ile Thr
    1730                 1735                 1740
```

278

```
Tyr Val Pro Ile Thr Gly Gly   Thr Pro Ser Met Val   Thr Val Asp
    1745                1750                1755

Gly Thr Lys Thr Gln Thr Arg   Leu Val Lys Leu Ile   Pro Gly Val
    1760                1765                1770

Glu Tyr Leu Val Ser Ile Ile   Ala Met Lys Gly Phe   Glu Glu Ser
    1775                1780                1785

Glu Pro Val Ser Gly Ser Phe   Thr Thr Ala Leu Asp   Gly Pro Ser
    1790                1795                1800

Gly Leu Val Thr Ala Asn Ile   Thr Asp Ser Glu Ala   Leu Ala Arg
    1805                1810                1815

Trp Gln Pro Ala Ile Ala Thr   Val Asp Ser Tyr Val   Ile Ser Tyr
    1820                1825                1830

Thr Gly Glu Lys Val Pro Glu   Ile Thr Arg Thr Val   Ser Gly Asn
    1835                1840                1845

Thr Val Glu Tyr Ala Leu Thr   Asp Leu Glu Pro Ala   Thr Glu Tyr
    1850                1855                1860

Thr Leu Arg Ile Phe Ala Glu   Lys Gly Pro Gln Lys   Ser Ser Thr
    1865                1870                1875

Ile Thr Ala Lys Phe Thr Thr   Asp Leu Asp Ser Pro   Arg Asp Leu
    1880                1885                1890

Thr Ala Thr Glu Val Gln Ser   Glu Thr Ala Leu Leu   Thr Trp Arg
    1895                1900                1905

Pro Pro Arg Ala Ser Val Thr   Gly Tyr Leu Leu Val   Tyr Glu Ser
    1910                1915                1920

Val Asp Gly Thr Val Lys Glu   Val Ile Val Gly Pro   Asp Thr Thr
    1925                1930                1935

Ser Tyr Ser Leu Ala Asp Leu   Ser Pro Ser Thr His   Tyr Thr Ala
    1940                1945                1950

Lys Ile Gln Ala Leu Asn Gly   Pro Leu Arg Ser Asn   Met Ile Gln
    1955                1960                1965

Thr Ile Phe Thr Thr Ile Gly   Leu Leu Tyr Pro Phe   Pro Lys Asp
    1970                1975                1980
```

```
Cys Ser Gln Ala Met Leu Asn Gly Asp Thr Thr Ser Gly Leu Tyr
    1985                1990            1995

Thr Ile Tyr Leu Asn Gly Asp Lys Ala Glu Ala Leu Glu Val Phe
    2000                2005            2010

Cys Asp Met Thr Ser Asp Gly Gly Gly Trp Ile Val Phe Leu Arg
    2015                2020            2025

Arg Lys Asn Gly Arg Glu Asn Phe Tyr Gln Asn Trp Lys Ala Tyr
    2030                2035            2040

Ala Ala Gly Phe Gly Asp Arg Arg Glu Glu Phe Trp Leu Gly Leu
    2045                2050            2055

Asp Asn Leu Asn Lys Ile Thr Ala Gln Gly Gln Tyr Glu Leu Arg
    2060                2065            2070

Val Asp Leu Arg Asp His Gly Glu Thr Ala Phe Ala Val Tyr Asp
    2075                2080            2085

Lys Phe Ser Val Gly Asp Ala Lys Thr Arg Tyr Lys Leu Lys Val
    2090                2095            2100

Glu Gly Tyr Ser Gly Thr Ala Gly Asp Ser Met Ala Tyr His Asn
    2105                2110            2115

Gly Arg Ser Phe Ser Thr Phe Asp Lys Asp Thr Asp Ser Ala Ile
    2120                2125            2130

Thr Asn Cys Ala Leu Ser Tyr Lys Gly Ala Phe Trp Tyr Arg Asn
    2135                2140            2145

Cys His Arg Val Asn Leu Met Gly Arg Tyr Gly Asp Asn Asn His
    2150                2155            2160

Ser Gln Gly Val Asn Trp Phe His Trp Lys Gly His Glu His Ser
    2165                2170            2175

Ile Gln Phe Ala Glu Met Lys Leu Arg Pro Ser Asn Phe Arg Asn
    2180                2185            2190

Leu Glu Gly Arg Arg Lys Arg Ala
    2195                2200
```

<210> 3
<211> 406

<212> PRT
<213> Homo sapiens

<400> 3

```
Met Ser Gly Ala Pro Thr Ala Gly Ala Ala Leu Met Leu Cys Ala Ala
1               5                   10                  15

Thr Ala Val Leu Leu Ser Ala Gln Gly Gly Pro Val Gln Ser Lys Ser
            20                  25                  30

Pro Arg Phe Ala Ser Trp Asp Glu Met Asn Val Leu Ala His Gly Leu
            35                  40                  45

Leu Gln Leu Gly Gln Gly Leu Arg Glu His Ala Glu Arg Thr Arg Ser
            50                  55                  60

Gln Leu Ser Ala Leu Glu Arg Arg Leu Ser Ala Cys Gly Ser Ala Cys
65                  70                  75                  80

Gln Gly Thr Glu Gly Ser Thr Asp Leu Pro Leu Ala Pro Glu Ser Arg
                85                  90                  95

Val Asp Pro Glu Val Leu His Ser Leu Gln Thr Gln Leu Lys Ala Gln
            100                 105                 110

Asn Ser Arg Ile Gln Gln Leu Phe His Lys Val Ala Gln Gln Gln Arg
            115                 120                 125

His Leu Glu Lys Gln His Leu Arg Ile Gln His Leu Gln Ser Gln Phe
        130                 135                 140

Gly Leu Leu Asp His Lys His Leu Asp His Glu Val Ala Lys Pro Ala
145                 150                 155                 160

Arg Arg Lys Arg Leu Pro Glu Met Ala Gln Pro Val Asp Pro Ala His
                165                 170                 175

Asn Val Ser Arg Leu His Arg Leu Pro Arg Asp Cys Gln Glu Leu Phe
            180                 185                 190

Gln Val Gly Glu Arg Gln Ser Gly Leu Phe Glu Ile Gln Pro Gln Gly
            195                 200                 205

Ser Pro Pro Phe Leu Val Asn Cys Lys Met Thr Ser Asp Gly Gly Trp
        210                 215                 220

Thr Val Ile Gln Arg Arg His Asp Gly Ser Val Asp Phe Asn Arg Pro
225                 230                 235                 240
```

```
Trp Glu Ala Tyr Lys Ala Gly Phe Gly Asp Pro His Gly Glu Phe Trp
            245             250             255

Leu Gly Leu Glu Lys Val His Ser Ile Thr Gly Asp Arg Asn Ser Arg
            260             265             270

Leu Ala Val Gln Leu Arg Asp Trp Asp Gly Asn Ala Glu Leu Leu Gln
            275             280             285

Phe Ser Val His Leu Gly Gly Glu Asp Thr Ala Tyr Ser Leu Gln Leu
    290             295             300

Thr Ala Pro Val Ala Gly Gln Leu Gly Ala Thr Thr Val Pro Pro Ser
305             310             315             320

Gly Leu Ser Val Pro Phe Ser Thr Trp Asp Gln Asp His Asp Leu Arg
            325             330             335

Arg Asp Lys Asn Cys Ala Lys Ser Leu Ser Gly Gly Trp Trp Phe Gly
            340             345             350

Thr Cys Ser His Ser Asn Leu Asn Gly Gln Tyr Phe Arg Ser Ile Pro
            355             360             365

Gln Gln Arg Gln Lys Leu Lys Lys Gly Ile Phe Trp Lys Thr Trp Arg
            370             375             380

Gly Arg Tyr Tyr Pro Leu Gln Ala Thr Thr Met Leu Ile Gln Pro Met
385             390             395             400

Ala Ala Glu Ala Ala Ser
                405


<210> 4
<211> 216
<212> PRT
<213> Homo sapiens

<400> 4
Met Arg Ser Gly Cys Val Val Val His Val Trp Ile Leu Ala Gly Leu
1               5               10              15

Trp Leu Ala Val Ala Gly Arg Pro Leu Ala Phe Ser Asp Ala Gly Pro
            20              25              30

His Val His Tyr Gly Trp Gly Asp Pro Ile Arg Leu Arg His Leu Tyr
            35              40              45
```

```
Thr Ser Gly Pro His Gly Leu Ser Ser Cys Phe Leu Arg Ile Arg Ala
    50              55              60

Asp Gly Val Val Asp Cys Ala Arg Gly Gln Ser Ala His Ser Leu Leu
65              70              75              80

Glu Ile Lys Ala Val Ala Leu Arg Thr Val Ala Ile Lys Gly Val His
            85              90              95

Ser Val Arg Tyr Leu Cys Met Gly Ala Asp Gly Lys Met Gln Gly Leu
        100             105             110

Leu Gln Tyr Ser Glu Glu Asp Cys Ala Phe Glu Glu Glu Ile Arg Pro
        115             120             125

Asp Gly Tyr Asn Val Tyr Arg Ser Glu Lys His Arg Leu Pro Val Ser
        130             135             140

Leu Ser Ser Ala Lys Gln Arg Gln Leu Tyr Lys Asn Arg Gly Phe Leu
145             150             155             160

Pro Leu Ser His Phe Leu Pro Met Leu Pro Met Val Pro Glu Glu Pro
            165             170             175

Glu Asp Leu Arg Gly His Leu Glu Ser Asp Met Phe Ser Ser Pro Leu
        180             185             190

Glu Thr Asp Ser Met Asp Pro Phe Gly Leu Val Thr Gly Leu Glu Ala
        195             200             205

Val Arg Ser Pro Ser Phe Glu Lys
    210             215
```

```
<210> 5
<211> 209
<212> PRT
<213> Homo sapiens

<400> 5
Met Asp Ser Asp Glu Thr Gly Phe Glu His Ser Gly Leu Trp Val Ser
1               5               10              15

Val Leu Ala Gly Leu Leu Leu Gly Ala Cys Gln Ala His Pro Ile Pro
            20              25              30

Asp Ser Ser Pro Leu Leu Gln Phe Gly Gly Gln Val Arg Gln Arg Tyr
        35              40              45

Leu Tyr Thr Asp Asp Ala Gln Gln Thr Glu Ala His Leu Glu Ile Arg
```

                    50                        55                        60

Glu Asp Gly Thr Val Gly Gly Ala Ala Asp Gln Ser Pro Glu Ser Leu
65                  70                  75                  80

Leu Gln Leu Lys Ala Leu Lys Pro Gly Val Ile Gln Ile Leu Gly Val
                85                  90                  95

Lys Thr Ser Arg Phe Leu Cys Gln Arg Pro Asp Gly Ala Leu Tyr Gly
            100                 105                 110

Ser Leu His Phe Asp Pro Glu Ala Cys Ser Phe Arg Glu Leu Leu Leu
            115                 120                 125

Glu Asp Gly Tyr Asn Val Tyr Gln Ser Glu Ala His Gly Leu Pro Leu
            130                 135                 140

His Leu Pro Gly Asn Lys Ser Pro His Arg Asp Pro Ala Pro Arg Gly
145                 150                 155                 160

Pro Ala Arg Phe Leu Pro Leu Pro Gly Leu Pro Pro Ala Leu Pro Glu
            165                 170                 175

Pro Pro Gly Ile Leu Ala Pro Gln Pro Pro Asp Val Gly Ser Ser Asp
            180                 185                 190

Pro Leu Ser Met Val Gly Pro Ser Gln Gly Arg Ser Pro Ser Tyr Ala
            195                 200                 205

Ser


<210> 6
<211> 155
<212> PRT
<213> Homo sapiens

<400> 6
Met Ala Glu Gly Glu Ile Thr Thr Phe Thr Ala Leu Thr Glu Lys Phe
1               5                   10                  15

Asn Leu Pro Pro Gly Asn Tyr Lys Lys Pro Lys Leu Leu Tyr Cys Ser
            20                  25                  30

Asn Gly Gly His Phe Leu Arg Ile Leu Pro Asp Gly Thr Val Asp Gly
            35                  40                  45

Thr Arg Asp Arg Ser Asp Gln His Ile Gln Leu Gln Leu Ser Ala Glu
            50                  55                  60

284

```
Ser Val Gly Glu Val Tyr Ile Lys Ser Thr Glu Thr Gly Gln Tyr Leu
65              70              75                      80


Ala Met Asp Thr Asp Gly Leu Leu Tyr Gly Ser Gln Thr Pro Asn Glu
                85              90              95


Glu Cys Leu Phe Leu Glu Arg Leu Glu Glu Asn His Tyr Asn Thr Tyr
            100             105             110


Ile Ser Lys Lys His Ala Glu Lys Asn Trp Phe Val Gly Leu Lys Lys
            115             120             125


Asn Gly Ser Cys Lys Arg Gly Pro Arg Thr His Tyr Gly Gln Lys Ala
    130             135             140


Ile Leu Phe Leu Pro Leu Pro Val Ser Ser Asp
145             150             155
```

<210> 7
<211> 470
<212> PRT
<213> Homo sapiens

<400> 7

```
Met Gly Lys Pro Trp Leu Arg Ala Leu Gln Leu Leu Leu Leu Leu Gly
1               5               10              15


Ala Ser Trp Ala Arg Ala Gly Ala Pro Arg Cys Thr Tyr Thr Phe Val
            20              25              30


Leu Pro Pro Gln Lys Phe Thr Gly Ala Val Cys Trp Ser Gly Pro Ala
        35              40              45


Ser Thr Arg Ala Thr Pro Glu Ala Ala Asn Ala Ser Glu Leu Ala Ala
    50              55              60


Leu Arg Met Arg Val Gly Arg His Glu Glu Leu Leu Arg Glu Leu Gln
65              70              75              80


Arg Leu Ala Ala Ala Asp Gly Ala Val Ala Gly Glu Val Arg Ala Leu
            85              90              95


Arg Lys Glu Ser Arg Gly Leu Ser Ala Arg Leu Gly Gln Leu Arg Ala
        100             105             110


Gln Leu Gln His Glu Ala Gly Pro Gly Ala Gly Pro Gly Ala Asp Leu
        115             120             125
```

Gly Ala Glu Pro Ala Ala Ala Leu Ala Leu Leu Gly Glu Arg Val Leu
        130             135             140

Asn Ala Ser Ala Glu Ala Gln Arg Ala Ala Ala Arg Phe His Gln Leu
145             150             155             160

Asp Val Lys Phe Arg Glu Leu Ala Gln Leu Val Thr Gln Gln Ser Ser
            165             170             175

Leu Ile Ala Arg Leu Glu Arg Leu Cys Pro Gly Gly Ala Gly Gly Gln
            180             185             190

Gln Gln Val Leu Pro Pro Pro Pro Leu Val Pro Val Val Pro Val Arg
            195             200             205

Leu Val Gly Ser Thr Ser Asp Thr Ser Arg Met Leu Asp Pro Ala Pro
        210             215             220

Glu Pro Gln Arg Asp Gln Thr Gln Arg Gln Gln Glu Pro Met Ala Ser
225             230             235             240

Pro Met Pro Ala Gly His Pro Ala Val Pro Thr Lys Pro Val Gly Pro
            245             250             255

Trp Gln Asp Cys Ala Glu Ala Arg Gln Ala Gly His Glu Gln Ser Gly
            260             265             270

Val Tyr Glu Leu Arg Val Gly Arg His Val Val Ser Val Trp Cys Glu
        275             280             285

Gln Gln Leu Glu Gly Gly Gly Trp Thr Val Ile Gln Arg Arg Gln Asp
        290             295             300

Gly Ser Val Asn Phe Phe Thr Thr Trp Gln His Tyr Lys Ala Gly Phe
305             310             315             320

Gly Arg Pro Asp Gly Glu Tyr Trp Leu Gly Leu Glu Pro Val Tyr Gln
            325             330             335

Leu Thr Ser Arg Gly Asp His Glu Leu Leu Val Leu Leu Glu Asp Trp
        340             345             350

Gly Gly Arg Gly Ala Arg Ala His Tyr Asp Gly Phe Ser Leu Glu Pro
        355             360             365

Glu Ser Asp His Tyr Arg Leu Arg Leu Gly Gln Tyr His Gly Asp Ala
370             375             380

286

```
Gly Asp Ser Leu Ser Trp His Asn Asp Lys Pro Phe Ser Thr Val Asp
385             390             395             400

Arg Asp Arg Asp Ser Tyr Ser Gly Asn Cys Ala Leu Tyr Gln Arg Gly
            405             410             415

Gly Trp Trp Tyr His Ala Cys Ala His Ser Asn Leu Asn Gly Val Trp
        420             425             430

His His Gly Gly His Tyr Arg Ser Arg Tyr Gln Asp Gly Val Tyr Trp
        435             440             445

Ala Glu Phe Arg Gly Gly Ala Tyr Ser Leu Arg Lys Ala Ala Met Leu
    450             455             460

Ile Arg Pro Leu Lys Leu
465             470


<210> 8
<211> 169
<212> PRT
<213> Homo sapiens

<400> 8
Met Thr Ala Gly Arg Arg Met Glu Met Leu Cys Ala Gly Arg Val Pro
1               5               10              15

Ala Leu Leu Leu Cys Leu Gly Phe His Leu Leu Gln Ala Val Leu Ser
        20              25              30

Thr Thr Val Ile Pro Ser Cys Ile Pro Gly Glu Ser Ser Asp Asn Cys
        35              40              45

Thr Ala Leu Val Gln Thr Glu Asp Asn Pro Arg Val Ala Gln Val Ser
    50              55              60

Ile Thr Lys Cys Ser Ser Asp Met Asn Gly Tyr Cys Leu His Gly Gln
65              70              75              80

Cys Ile Tyr Leu Val Asp Met Ser Gln Asn Tyr Cys Arg Cys Glu Val
            85              90              95

Gly Tyr Thr Gly Val Arg Cys Glu His Phe Phe Leu Thr Val His Gln
        100             105             110

Pro Leu Ser Lys Glu Tyr Val Ala Leu Thr Val Ile Leu Ile Ile Leu
        115             120             125
```

```
Phe Leu Ile Thr Val Val Gly Ser Thr Tyr Tyr Phe Cys Arg Trp Tyr
    130             135             140

Arg Asn Arg Lys Ser Lys Glu Pro Lys Lys Glu Tyr Glu Arg Val Thr
145             150             155             160

Ser Gly Asp Pro Glu Leu Pro Gln Val
                165
```

<210> 9
<211> 178
<212> PRT
<213> Homo sapiens

<400> 9

```
Met Asp Arg Ala Ala Arg Cys Ser Gly Ala Ser Ser Leu Pro Leu Leu
1               5               10              15

Leu Ala Leu Ala Leu Gly Leu Val Ile Leu His Cys Val Val Ala Asp
        20              25              30

Gly Asn Ser Thr Arg Ser Pro Glu Thr Asn Gly Leu Leu Cys Gly Asp
        35              40              45

Pro Glu Glu Asn Cys Ala Ala Thr Thr Thr Gln Ser Lys Arg Lys Gly
    50              55              60

His Phe Ser Arg Cys Pro Lys Gln Tyr Lys His Tyr Cys Ile Lys Gly
65              70              75              80

Arg Cys Arg Phe Val Val Ala Glu Gln Thr Pro Ser Cys Val Cys Asp
                85              90              95

Glu Gly Tyr Ile Gly Ala Arg Cys Glu Arg Val Asp Leu Phe Tyr Leu
            100             105             110

Arg Gly Asp Arg Gly Gln Ile Leu Val Ile Cys Leu Ile Ala Val Met
        115             120             125

Val Val Phe Ile Ile Leu Val Ile Gly Val Cys Thr Cys Cys His Pro
    130             135             140

Leu Arg Lys Arg Arg Lys Arg Lys Lys Glu Glu Glu Met Glu Thr
145             150             155             160

Leu Gly Lys Asp Ile Thr Pro Ile Asn Glu Asp Ile Glu Glu Thr Asn
            165             170             175
```

Ile Ala

<210> 10
<211> 252
<212> PRT
<213> Homo sapiens

<400> 10
Met Arg Ala Pro Leu Leu Pro Pro Ala Pro Val Val Leu Ser Leu Leu
1               5                   10                  15

Ile Leu Gly Ser Gly His Tyr Ala Ala Gly Leu Asp Leu Asn Asp Thr
            20                  25                  30

Tyr Ser Gly Lys Arg Glu Pro Phe Ser Gly Asp His Ser Ala Asp Gly
        35                  40                  45

Phe Glu Val Thr Ser Arg Ser Glu Met Ser Ser Gly Ser Glu Ile Ser
        50                  55                  60

Pro Val Ser Glu Met Pro Ser Ser Ser Glu Pro Ser Ser Gly Ala Asp
65                  70                  75                  80

Tyr Asp Tyr Ser Glu Glu Tyr Asp Asn Glu Pro Gln Ile Pro Gly Tyr
                85                  90                  95

Ile Val Asp Asp Ser Val Arg Val Glu Gln Val Val Lys Pro Pro Gln
            100                 105                 110

Asn Lys Thr Glu Ser Glu Asn Thr Ser Asp Lys Pro Lys Arg Lys Lys
            115                 120                 125

Lys Gly Gly Lys Asn Gly Lys Asn Arg Arg Asn Arg Lys Lys Lys Asn
            130                 135                 140

Pro Cys Asn Ala Glu Phe Gln Asn Phe Cys Ile His Gly Glu Cys Lys
145                 150                 155                 160

Tyr Ile Glu His Leu Glu Ala Val Thr Cys Lys Cys Gln Gln Glu Tyr
                165                 170                 175

Phe Gly Glu Arg Cys Gly Glu Lys Ser Met Lys Thr His Ser Met Ile
                180                 185                 190

Asp Ser Ser Leu Ser Lys Ile Ala Leu Ala Ala Ile Ala Ala Phe Met
            195                 200                 205

Ser Ala Val Ile Leu Thr Ala Val Ala Val Ile Thr Val Gln Leu Arg

210                    215                    220

Arg Gln Tyr Val Arg Lys Tyr Glu Gly Glu Ala Glu Glu Arg Lys Lys
225             230             235             240

Leu Arg Gln Glu Asn Gly Asn Val His Ala Ile Ala
                245             250

<210> 11
<211> 147
<212> PRT
<213> Homo sapiens

<400> 11
Met Val Met Gly Leu Gly Val Leu Leu Leu Val Phe Val Leu Gly Leu
1               5               10              15

Gly Leu Thr Pro Pro Thr Leu Ala Gln Asp Asn Ser Arg Tyr Thr His
            20              25              30

Phe Leu Thr Gln His Tyr Asp Ala Lys Pro Gln Gly Arg Asp Asp Arg
        35              40              45

Tyr Cys Glu Ser Ile Met Arg Arg Arg Gly Leu Thr Ser Pro Cys Lys
        50              55              60

Asp Ile Asn Thr Phe Ile His Gly Asn Lys Arg Ser Ile Lys Ala Ile
65              70              75              80

Cys Glu Asn Lys Asn Gly Asn Pro His Arg Glu Asn Leu Arg Ile Ser
                85              90              95

Lys Ser Ser Phe Gln Val Thr Thr Cys Lys Leu His Gly Gly Ser Pro
            100             105             110

Trp Pro Pro Cys Gln Tyr Arg Ala Thr Ala Gly Phe Arg Asn Val Val
        115             120             125

Val Ala Cys Glu Asn Gly Leu Pro Val His Leu Asp Gln Ser Ile Phe
        130             135             140

Arg Arg Pro
145

<210> 12
<211> 1172
<212> PRT
<213> Homo sapiens

<400> 12

```
Met Val Trp Arg Leu Val Leu Leu Ala Leu Trp Val Trp Pro Ser Thr
1               5                   10                  15

Gln Ala Gly His Gln Asp Lys Asp Thr Thr Phe Asp Leu Phe Ser Ile
                20                  25                  30

Ser Asn Ile Asn Arg Lys Thr Ile Gly Ala Lys Gln Phe Arg Gly Pro
                35                  40                  45

Asp Pro Gly Val Pro Ala Tyr Arg Phe Val Arg Phe Asp Tyr Ile Pro
        50                  55                  60

Pro Val Asn Ala Asp Asp Leu Ser Lys Ile Thr Lys Ile Met Arg Gln
65                  70                  75                  80

Lys Glu Gly Phe Phe Leu Thr Ala Gln Leu Lys Gln Asp Gly Lys Ser
                85                  90                  95

Arg Gly Thr Leu Leu Ala Leu Glu Gly Pro Gly Leu Ser Gln Arg Gln
                100                 105                 110

Phe Glu Ile Val Ser Asn Gly Pro Ala Asp Thr Leu Asp Leu Thr Tyr
            115                 120                 125

Trp Ile Asp Gly Thr Arg His Val Val Ser Leu Glu Asp Val Gly Leu
    130                 135                 140

Ala Asp Ser Gln Trp Lys Asn Val Thr Val Gln Val Ala Gly Glu Thr
145                 150                 155                 160

Tyr Ser Leu His Val Gly Cys Asp Leu Ile Asp Ser Phe Ala Leu Asp
            165                 170                 175

Glu Pro Phe Tyr Glu His Leu Gln Ala Glu Lys Ser Arg Met Tyr Val
            180                 185                 190

Ala Lys Gly Ser Ala Arg Glu Ser His Phe Arg Gly Leu Leu Gln Asn
        195                 200                 205

Val His Leu Val Phe Glu Asn Ser Val Glu Asp Ile Leu Ser Lys Lys
        210                 215                 220

Gly Cys Gln Gln Gly Gln Gly Ala Glu Ile Asn Ala Ile Ser Glu Asn
225                 230                 235                 240

Thr Glu Thr Leu Arg Leu Gly Pro His Val Thr Thr Glu Tyr Val Gly
            245                 250                 255
```

```
Pro Ser Ser Glu Arg Arg Pro Glu Val Cys Glu Arg Ser Cys Glu Glu
        260             265             270

Leu Gly Asn Met Val Gln Glu Leu Ser Gly Leu His Val Leu Val Asn
        275             280             285

Gln Leu Ser Glu Asn Leu Lys Arg Val Ser Asn Asp Asn Gln Phe Leu
        290             295             300

Trp Glu Leu Ile Gly Gly Pro Pro Lys Thr Arg Asn Met Ser Ala Cys
305             310             315             320

Trp Gln Asp Gly Arg Phe Phe Ala Glu Asn Glu Thr Trp Val Val Asp
            325             330             335

Ser Cys Thr Thr Cys Thr Cys Lys Lys Phe Lys Thr Ile Cys His Gln
        340             345             350

Ile Thr Cys Pro Pro Ala Thr Cys Ala Ser Pro Ser Phe Val Glu Gly
        355             360             365

Glu Cys Cys Pro Ser Cys Leu His Ser Val Asp Gly Glu Glu Gly Trp
        370             375             380

Ser Pro Trp Ala Glu Trp Thr Gln Cys Ser Val Thr Cys Gly Ser Gly
385             390             395             400

Thr Gln Gln Arg Gly Arg Ser Cys Asp Val Thr Ser Asn Thr Cys Leu
            405             410             415

Gly Pro Ser Ile Gln Thr Arg Ala Cys Ser Leu Ser Lys Cys Asp Thr
        420             425             430

Arg Ile Arg Gln Asp Gly Gly Trp Ser His Trp Ser Pro Trp Ser Ser
        435             440             445

Cys Ser Val Thr Cys Gly Val Gly Asn Ile Thr Arg Ile Arg Leu Cys
        450             455             460

Asn Ser Pro Val Pro Gln Met Gly Gly Lys Asn Cys Lys Gly Ser Gly
465             470             475             480

Arg Glu Thr Lys Ala Cys Gln Gly Ala Pro Cys Pro Ile Asp Gly Arg
            485             490             495

Trp Ser Pro Trp Ser Pro Trp Ser Ala Cys Thr Val Thr Cys Ala Gly
        500             505             510
```

```
Gly Ile Arg Glu Arg Thr Arg Val Cys Asn Ser Pro Glu Pro Gln Tyr
    515                 520             525

Gly Gly Lys Ala Cys Val Gly Asp Val Gln Glu Arg Gln Met Cys Asn
    530                 535             540

Lys Arg Ser Cys Pro Val Asp Gly Cys Leu Ser Asn Pro Cys Phe Pro
545             550             555             560

Gly Ala Gln Cys Ser Ser Phe Pro Asp Gly Ser Trp Ser Cys Gly Ser
            565             570             575

Cys Pro Val Gly Phe Leu Gly Asn Gly Thr His Cys Glu Asp Leu Asp
            580             585             590

Glu Cys Ala Leu Val Pro Asp Ile Cys Phe Ser Thr Ser Lys Val Pro
    595             600             605

Arg Cys Val Asn Thr Gln Pro Gly Phe His Cys Leu Pro Cys Pro Pro
    610             615             620

Arg Tyr Arg Gly Asn Gln Pro Val Gly Val Gly Leu Glu Ala Ala Lys
625             630             635             640

Thr Glu Lys Gln Val Cys Glu Pro Glu Asn Pro Cys Lys Asp Lys Thr
            645             650             655

His Asn Cys His Lys His Ala Glu Cys Ile Tyr Leu Gly His Phe Ser
        660             665             670

Asp Pro Met Tyr Lys Cys Glu Cys Gln Thr Gly Tyr Ala Gly Asp Gly
    675             680             685

Leu Ile Cys Gly Glu Asp Ser Asp Leu Asp Gly Trp Pro Asn Leu Asn
    690             695             700

Leu Val Cys Ala Thr Asn Ala Thr Tyr His Cys Ile Lys Asp Asn Cys
705             710             715             720

Pro His Leu Pro Asn Ser Gly Gln Glu Asp Phe Asp Lys Asp Gly Ile
            725             730             735

Gly Asp Ala Cys Asp Asp Asp Asp Asn Asp Gly Val Thr Asp Glu
    740             745             750

Lys Asp Asn Cys Gln Leu Leu Phe Asn Pro Arg Gln Ala Asp Tyr Asp
```

755     760     765

Lys Asp Glu Val Gly Asp Arg Cys Asp Asn Cys Pro Tyr Val His Asn
 770    775    780

Pro Ala Gln Ile Asp Thr Asp Asn Asn Gly Glu Gly Asp Ala Cys Ser
785    790    795    800

Val Asp Ile Asp Gly Asp Asp Val Phe Asn Glu Arg Asp Asn Cys Pro
   805    810    815

Tyr Val Tyr Asn Thr Asp Gln Arg Asp Thr Asp Gly Asp Gly Val Gly
   820    825    830

Asp His Cys Asp Asn Cys Pro Leu Val His Asn Pro Asp Gln Thr Asp
  835    840    845

Val Asp Asn Asp Leu Val Gly Asp Gln Cys Asp Asn Asn Glu Asp Ile
 850    855    860

Asp Asp Asp Gly His Gln Asn Asn Gln Asp Asn Cys Pro Tyr Ile Ser
865    870    875    880

Asn Ala Asn Gln Ala Asp His Asp Arg Asp Gly Gln Gly Asp Ala Cys
   885    890    895

Asp Pro Asp Asp Asp Asn Asp Gly Val Pro Asp Asp Arg Asp Asn Cys
   900    905    910

Arg Leu Val Phe Asn Pro Asp Gln Glu Asp Leu Asp Gly Asp Gly Arg
  915    920    925

Gly Asp Ile Cys Lys Asp Asp Phe Asp Asn Asp Asn Ile Pro Asp Ile
 930    935    940

Asp Asp Val Cys Pro Glu Asn Asn Ala Ile Ser Glu Thr Asp Phe Arg
945    950    955    960

Asn Phe Gln Met Val Pro Leu Asp Pro Lys Gly Thr Thr Gln Ile Asp
   965    970    975

Pro Asn Trp Val Ile Arg His Gln Gly Lys Glu Leu Val Gln Thr Ala
   980    985    990

Asn Ser Asp Pro Gly Ile Ala Val Gly Phe Asp Glu Phe Gly Ser Val
  995    1000    1005

294

```
Asp Phe  Ser Gly Thr Phe Tyr  Val Asn Thr Asp Arg  Asp Asp Asp
    1010             1015             1020


Tyr Ala  Gly Phe Val Phe Gly  Tyr Gln Ser Ser Ser  Arg Phe Tyr
    1025             1030             1035


Val Val  Met Trp Lys Gln Val  Thr Gln Thr Tyr Trp  Glu Asp Gln
    1040             1045             1050


Pro Thr  Arg Ala Tyr Gly Tyr  Ser Gly Val Ser Leu  Lys Val Val
    1055             1060             1065


Asn Ser  Thr Thr Gly Thr Gly  Glu His Leu Arg Asn  Ala Leu Trp
    1070             1075             1080


His Thr  Gly Asn Thr Pro Gly  Gln Val Arg Thr Leu  Trp His Asp
    1085             1090             1095


Pro Arg  Asn Ile Gly Trp Lys  Asp Tyr Thr Ala Tyr  Arg Trp His
    1100             1105             1110


Leu Thr  His Arg Pro Lys Thr  Gly Tyr Ile Arg Val  Leu Val His
    1115             1120             1125


Glu Gly  Lys Gln Val Met Ala  Asp Ser Gly Pro Ile  Tyr Asp Gln
    1130             1135             1140


Thr Tyr  Ala Gly Gly Arg Leu  Gly Leu Phe Val Phe  Ser Gln Glu
    1145             1150             1155


Met Val  Tyr Phe Ser Asp Leu  Lys Tyr Glu Cys Arg  Asp Ile
    1160             1165             1170


<210> 13
<211> 1754
<212> PRT
<213> Homo sapiens

<400> 13
Met Ala Pro Tyr Pro Cys Gly Cys His Ile Leu Leu Leu Phe Cys
1               5               10                  15


Cys Leu Ala Ala Ala Arg Ala Asn Leu Leu Asn Leu Asn Trp Leu Trp
            20              25                  30


Phe Asn Asn Glu Asp Thr Ser His Ala Ala Thr Thr Ile Pro Glu Pro
        35              40                  45


Gln Gly Pro Leu Pro Val Gln Pro Thr Ala Asp Thr Thr Thr His Val
```

295

```
                50                              55                              60

        Thr Pro Arg Asn Gly Ser Thr Glu Pro Ala Thr Ala Pro Gly Ser Pro
        65                  70                  75                  80

        Glu Pro Pro Ser Glu Leu Leu Glu Asp Gly Gln Asp Thr Pro Thr Ser
                        85                  90                  95

        Ala Glu Ser Pro Asp Ala Pro Glu Glu Asn Ile Ala Gly Val Gly Ala
                        100                 105                 110

        Glu Ile Leu Asn Val Ala Lys Gly Ile Arg Ser Phe Val Gln Leu Trp
                        115                 120                 125

        Asn Asp Thr Val Pro Thr Glu Ser Leu Ala Arg Ala Glu Thr Leu Val
                        130                 135                 140

        Leu Glu Thr Pro Val Gly Pro Leu Ala Leu Ala Gly Pro Ser Ser Thr
        145                 150                 155                 160

        Pro Gln Glu Asn Gly Thr Thr Leu Trp Pro Ser Arg Gly Ile Pro Ser
                        165                 170                 175

        Ser Pro Gly Ala His Thr Thr Glu Ala Gly Thr Leu Pro Ala Pro Thr
                        180                 185                 190

        Pro Ser Pro Pro Ser Leu Gly Arg Pro Trp Ala Pro Leu Thr Gly Pro
                        195                 200                 205

        Ser Val Pro Pro Pro Ser Ser Gly Arg Ala Ser Leu Ser Ser Leu Leu
                210                 215                 220

        Gly Gly Ala Pro Pro Trp Gly Ser Leu Gln Asp Pro Asp Ser Gln Gly
        225                 230                 235                 240

        Leu Ser Pro Ala Ala Ala Ala Pro Ser Gln Gln Leu Gln Arg Pro Asp
                        245                 250                 255

        Val Arg Leu Arg Thr Pro Leu Leu His Pro Leu Val Met Gly Ser Leu
                        260                 265                 270

        Gly Lys His Ala Ala Pro Ser Ala Phe Ser Ser Gly Leu Pro Gly Ala
                        275                 280                 285

        Leu Ser Gln Val Ala Val Thr Thr Leu Thr Arg Asp Ser Gly Ala Trp
                290                 295                 300
```

Val Ser His Val Ala Asn Ser Val Gly Pro Gly Leu Ala Asn Asn Ser
305                 310             315                 320

Ala Leu Leu Gly Ala Asp Pro Glu Ala Pro Ala Gly Arg Cys Leu Pro
                325             330                 335

Leu Pro Pro Ser Leu Pro Val Cys Gly His Leu Gly Ile Ser Arg Phe
            340             345                 350

Trp Leu Pro Asn His Leu His His Glu Ser Gly Glu Gln Val Arg Ala
            355             360                 365

Gly Ala Arg Ala Trp Gly Gly Leu Leu Gln Thr His Cys His Pro Phe
    370             375             380

Leu Ala Trp Phe Phe Cys Leu Leu Leu Val Pro Pro Cys Gly Ser Val
385             390             395                 400

Pro Pro Pro Ala Pro Pro Pro Cys Cys Gln Phe Cys Glu Ala Leu Gln
            405             410                 415

Asp Ala Cys Trp Ser Arg Leu Gly Gly Gly Arg Leu Pro Val Ala Cys
            420             425             430

Ala Ser Leu Pro Thr Gln Glu Asp Gly Tyr Cys Val Leu Ile Gly Pro
        435             440             445

Ala Ala Glu Arg Ile Ser Glu Glu Val Gly Leu Leu Gln Leu Leu Gly
    450             455             460

Asp Pro Pro Pro Gln Gln Val Thr Gln Thr Asp Asp Pro Asp Val Gly
465             470             475                 480

Leu Ala Tyr Val Phe Gly Pro Asp Ala Asn Ser Gly Gln Val Ala Arg
            485             490                 495

Tyr His Phe Pro Ser Leu Phe Phe Arg Asp Phe Ser Leu Leu Phe His
            500             505             510

Ile Arg Pro Ala Thr Glu Gly Pro Gly Val Leu Phe Ala Ile Thr Asp
        515             520             525

Ser Ala Gln Ala Met Val Leu Leu Gly Val Lys Leu Ser Gly Val Gln
    530             535             540

Asp Gly His Gln Asp Ile Ser Leu Leu Tyr Thr Glu Pro Gly Ala Gly
545             550             555                 560

```
Gln Thr His Thr Ala Ala Ser Phe Arg Leu Pro Ala Phe Val Gly Gln
            565                 570                 575

Trp Thr His Leu Ala Leu Ser Val Ala Gly Gly Phe Val Ala Leu Tyr
            580                 585                 590

Val Asp Cys Glu Glu Phe Gln Arg Met Pro Leu Ala Arg Ser Ser Arg
            595                 600                 605

Gly Leu Glu Leu Glu Pro Gly Ala Gly Leu Phe Val Ala Gln Ala Gly
    610                 615                 620

Gly Ala Asp Pro Asp Lys Phe Gln Gly Val Ile Ala Glu Leu Lys Val
625                 630                 635                 640

Arg Arg Asp Pro Gln Val Ser Pro Met His Cys Leu Asp Glu Glu Gly
            645                 650                 655

Asp Asp Ser Asp Gly Ala Ser Gly Asp Ser Gly Ser Gly Leu Gly Asp
            660                 665                 670

Ala Arg Glu Leu Leu Arg Glu Glu Thr Gly Ala Ala Leu Lys Pro Arg
    675                 680                 685

Leu Pro Ala Pro Pro Pro Val Thr Thr Pro Pro Leu Ala Gly Gly Ser
    690                 695                 700

Ser Thr Glu Asp Ser Arg Ser Glu Glu Val Glu Glu Gln Thr Thr Val
705                 710                 715                 720

Ala Ser Leu Gly Ala Gln Thr Leu Pro Gly Ser Asp Ser Val Ser Thr
            725                 730                 735

Trp Asp Gly Ser Val Arg Thr Pro Gly Gly Arg Val Lys Glu Gly Gly
            740                 745                 750

Leu Lys Gly Gln Lys Gly Glu Pro Gly Val Pro Gly Pro Pro Gly Arg
            755                 760                 765

Ala Gly Pro Pro Gly Ser Pro Cys Leu Pro Gly Pro Pro Gly Leu Pro
    770                 775                 780

Cys Pro Val Ser Pro Leu Gly Pro Ala Gly Pro Ala Leu Gln Thr Val
785                 790                 795                 800

Pro Gly Pro Gln Gly Pro Pro Gly Pro Pro Gly Arg Asp Gly Thr Pro
            805                 810                 815
```

298

```
Gly Arg Asp Gly Glu Pro Gly Asp Pro Gly Glu Asp Gly Lys Pro Gly
        820             825             830

Asp Thr Gly Pro Gln Gly Phe Pro Gly Thr Pro Gly Asp Val Gly Pro
        835             840             845

Lys Gly Asp Lys Gly Asp Pro Gly Val Gly Glu Arg Gly Pro Pro Gly
        850             855             860

Pro Gln Gly Pro Pro Gly Pro Pro Gly Pro Ser Phe Arg His Asp Lys
865             870             875             880

Leu Thr Phe Ile Asp Met Glu Gly Ser Gly Phe Gly Gly Asp Leu Glu
            885             890             895

Ala Leu Arg Gly Pro Arg Gly Phe Pro Gly Pro Pro Gly Pro Pro Gly
        900             905             910

Val Pro Gly Leu Pro Gly Glu Pro Gly Arg Phe Gly Val Asn Ser Ser
        915             920             925

Asp Val Pro Gly Pro Ala Gly Leu Pro Gly Val Pro Gly Arg Glu Gly
        930             935             940

Pro Pro Gly Phe Pro Gly Leu Pro Gly Pro Pro Gly Pro Pro Gly Arg
945             950             955             960

Glu Gly Pro Pro Gly Arg Thr Gly Gln Lys Gly Ser Leu Gly Glu Ala
            965             970             975

Gly Ala Pro Gly His Lys Gly Ser Lys Gly Ala Pro Gly Pro Ala Gly
            980             985             990

Ala Arg Gly Glu Ser Gly Leu Ala  Gly Ala Pro Gly Pro  Ala Gly Pro
        995             1000            1005

Pro Gly  Pro Pro Gly Pro Pro  Gly Pro Pro Gly Pro  Gly Leu Pro
    1010            1015            1020

Ala Gly  Phe Asp Asp Met Glu  Gly Ser Gly Gly Pro  Phe Trp Ser
    1025            1030            1035

Thr Ala  Arg Ser Ala Asp Gly  Pro Gln Gly Pro Pro  Gly Leu Pro
    1040            1045            1050

Gly Leu  Lys Gly Asp Pro Gly  Val Pro Gly Leu Pro  Gly Ala Lys
```

                    1055                        1060                        1065


        Gly Glu  Val Gly Ala Asp Gly  Val Pro Gly Phe Pro  Gly Leu Pro
            1070                    1075                    1080


        Gly Arg  Glu Gly Ile Ala Gly  Pro Gln Gly Pro Lys  Gly Asp Arg
            1085                    1090                    1095


        Gly Ser  Arg Gly Glu Lys Gly  Asp Pro Gly Lys Asp  Gly Val Gly
            1100                    1105                    1110


        Gln Pro  Gly Leu Pro Gly Pro  Pro Gly Pro Pro Gly  Pro Val Val
            1115                    1120                    1125


        Tyr Val  Ser Glu Gln Asp Gly  Ser Val Leu Ser Val  Pro Gly Pro
            1130                    1135                    1140


        Glu Gly  Arg Pro Gly Phe Ala  Gly Phe Pro Gly Pro  Ala Gly Pro
            1145                    1150                    1155


        Lys Gly  Asn Leu Gly Ser Lys  Gly Glu Arg Gly Ser  Pro Gly Pro
            1160                    1165                    1170


        Lys Gly  Glu Lys Gly Glu Pro  Gly Ser Ile Phe Ser  Pro Asp Gly
            1175                    1180                    1185


        Gly Ala  Leu Gly Pro Ala Gln  Lys Gly Ala Lys Gly  Glu Pro Gly
            1190                    1195                    1200


        Phe Arg  Gly Pro Pro Gly Pro  Tyr Gly Arg Pro Gly  Tyr Lys Gly
            1205                    1210                    1215


        Glu Ile  Gly Phe Pro Gly Arg  Pro Gly Arg Pro Gly  Met Asn Gly
            1220                    1225                    1230


        Leu Lys  Gly Glu Lys Gly Glu  Pro Gly Asp Ala Ser  Leu Gly Phe
            1235                    1240                    1245


        Gly Met  Arg Gly Met Pro Gly  Pro Pro Gly Pro Pro  Gly Pro Pro
            1250                    1255                    1260


        Gly Pro  Pro Gly Thr Pro Val  Tyr Asp Ser Asn Val  Phe Ala Glu
            1265                    1270                    1275


        Ser Ser  Arg Pro Gly Pro Pro  Gly Leu Pro Gly Asn  Gln Gly Pro
            1280                    1285                    1290


300

```
Pro Gly Pro Lys Gly Ala Lys  Gly Glu Val Gly Pro  Pro Gly Pro
    1295            1300                1305

Pro Gly Gln Phe Pro Phe Asp  Phe Leu Gln Leu Glu  Ala Glu Met
    1310            1315                1320

Lys Gly Glu Lys Gly Asp Arg  Gly Asp Ala Gly Gln  Lys Gly Glu
    1325            1330                1335

Arg Gly Glu Pro Gly Gly Gly  Gly Phe Phe Gly Ser  Ser Leu Pro
    1340            1345                1350

Gly Pro Pro Gly Pro Pro Gly  Pro Pro Gly Pro Arg  Gly Tyr Pro
    1355            1360                1365

Gly Ile Pro Gly Pro Lys Gly  Glu Ser Ile Arg Gly  Gln Pro Gly
    1370            1375                1380

Pro Pro Gly Pro Gln Gly Pro  Pro Gly Ile Gly Tyr  Glu Gly Arg
    1385            1390                1395

Gln Gly Pro Pro Gly Pro Pro  Gly Pro Pro Gly Pro  Pro Ser Phe
    1400            1405                1410

Pro Gly Pro His Arg Gln Thr  Ile Ser Val Pro Gly  Pro Pro Gly
    1415            1420                1425

Pro Pro Gly Pro Pro Gly Pro  Pro Gly Thr Met Gly  Ala Ser Ser
    1430            1435                1440

Gly Val Arg Leu Trp Ala Thr  Arg Gln Ala Met Leu  Gly Gln Val
    1445            1450                1455

His Glu Val Pro Glu Gly Trp  Leu Ile Phe Val Ala  Glu Gln Glu
    1460            1465                1470

Glu Leu Tyr Val Arg Val Gln  Asn Gly Phe Arg Lys  Val Gln Leu
    1475            1480                1485

Glu Ala Arg Thr Pro Leu Pro  Arg Gly Thr Asp Asn  Glu Val Ala
    1490            1495                1500

Ala Leu Gln Pro Pro Val Val  Gln Leu His Asp Ser  Asn Pro Tyr
    1505            1510                1515

Pro Arg Arg Glu His Pro His  Pro Thr Ala Arg Pro  Trp Arg Ala
    1520            1525                1530
```

```
Asp Asp  Ile Leu Ala Ser Pro  Pro Arg Leu Pro Glu  Pro Gln Pro
    1535                 1540             1545

Tyr Pro  Gly Ala Pro His His  Ser Ser Tyr Val His  Leu Arg Pro
    1550                 1555             1560

Ala Arg  Pro Thr Ser Pro Pro  Ala His Ser His Arg  Asp Phe Gln
    1565                 1570             1575

Pro Val  Leu His Leu Val Ala  Leu Asn Ser Pro Leu  Ser Gly Gly
    1580                 1585             1590

Met Arg  Gly Ile Arg Gly Ala  Asp Phe Gln Cys Phe  Gln Gln Ala
    1595                 1600             1605

Arg Ala  Val Gly Leu Ala Gly  Thr Phe Arg Ala Phe  Leu Ser Ser
    1610                 1615             1620

Arg Leu  Gln Asp Leu Tyr Ser  Ile Val Arg Arg Ala  Asp Arg Ala
    1625                 1630             1635

Ala Val  Pro Ile Val Asn Leu  Lys Asp Glu Leu Leu  Phe Pro Ser
    1640                 1645             1650

Trp Glu  Ala Leu Phe Ser Gly  Ser Glu Gly Pro Leu  Lys Pro Gly
    1655                 1660             1665

Ala Arg  Ile Phe Ser Phe Asp  Gly Lys Asp Val Leu  Arg His Pro
    1670                 1675             1680

Thr Trp  Pro Gln Lys Ser Val  Trp His Gly Ser Asp  Pro Asn Gly
    1685                 1690             1695

Arg Arg  Leu Thr Glu Ser Tyr  Cys Glu Thr Trp Arg  Thr Glu Ala
    1700                 1705             1710

Pro Ser  Ala Thr Gly Gln Ala  Ser Ser Leu Leu Gly  Gly Arg Leu
    1715                 1720             1725

Leu Gly  Gln Ser Ala Ala Ser  Cys His His Ala Tyr  Ile Val Leu
    1730                 1735             1740

Cys Ile  Glu Asn Ser Phe Met  Thr Ala Ser Lys
    1745                 1750
```

<210> 14
<211> 35

```
<212> PRT
<213> Homo sapiens

<400> 14
Gln Asp Ala Cys Trp Ser Arg Leu Gly Gly Gly Arg Leu Pro Val Ala
1               5                   10                  15


Cys Ala Ser Leu Pro Thr Gln Glu Asp Gly Tyr Cys Val Leu Ile Gly
            20                  25                  30


Pro Ala Ala
        35



<210> 15
<211> 35
<212> PRT
<213> Homo sapiens

<400> 15
Met Ala Pro Arg Cys Pro Trp Pro Trp Pro Arg Arg Arg Arg Leu Leu
1               5                   10                  15


Asp Val Leu Ala Pro Leu Val Leu Leu Leu Gly Val Arg Ala Ala Ser
            20                  25                  30


Ala Glu Pro
        35



<210> 16
<211> 4
<212> PRT
<213> Homo sapiens

<400> 16
Ile Gln Leu Gln
1



<210> 17
<211> 4
<212> PRT
<213> Homo sapiens

<400> 17
Thr Asp Thr Lys
1
```

## Description

The present application claims priority to U.S. Provisional Patent Application Nos. 61/430,977 filed January 8, 2011; 61/430,978 filed January 8, 2011; 61/430,979 filed January 8, 2011; 61/430,980 filed January 8, 2011; 61/430,981 filed January 8, 2011; 61/430,982 filed January 8, 2011; 61/430,983 filed January 8, 2011; 61/430,984 filed January 8, 2011; 61/430,985 filed January 8, 2011; 61/430,986 filed January 8, 2011; 61/430,987 filed January 8, 2011; 61/430,988 filed January 8, 2011; and 61/430,989 filed January 8, 2011, each of which is hereby incorporated in its entirety including all tables, figures, and claims.

BACKGROUND OF THE INVENTION

The following discussion of the background of the invention is merely provided to aid the reader in understanding the invention and is not admitted to describe or constitute prior art to the present invention.

The kidney is responsible for water and solute excretion from the body. Its functions include maintenance of acid-base balance, regulation of electrolyte concentrations, control of blood volume, and regulation of blood pressure. As such, loss of kidney function through injury and/or disease results in substantial morbidity and mortality. A detailed discussion of renal injuries is provided in Harrison's Principles of Internal Medicine, 17th Ed., McGraw Hill, New York, pages 1741-1830, which are hereby incorporated by reference in their entirety. Renal disease and/or injury may be acute or chronic. Acute and chronic kidney disease are described as follows (from Current Medical Diagnosis & Treatment 2008, 47th Ed, McGraw Hill, New York, pages 785-815, which are hereby incorporated by reference in their entirety): "Acute renal failure is worsening of renal function over hours to days, resulting in the retention of nitrogenous wastes (such as urea nitrogen) and creatinine in the blood. Retention of these substances is called azotemia. Chronic renal failure (chronic kidney disease) results from an abnormal loss of renal function over months to years".

Acute renal failure (ARF, also known as acute kidney injury, or AKI) is an abrupt (typically detected within about 48 hours to 1 week) reduction in glomerular filtration. This loss of filtration capacity results in retention of nitrogenous (urea and creatinine) and non-nitrogenous waste products that are normally excreted by the kidney, a reduction in urine output, or both. It is reported that ARF complicates about 5% of hospital admissions, 4-15% of cardiopulmonary bypass surgeries, and up to 30% of intensive care admissions. ARF may be categorized as prerenal, intrinsic renal, or postrenal in causation. Intrinsic renal disease can be further divided into glomerular, tubular, interstitial, and vascular abnormalities. Major causes of ARF are described in the following table, which is adapted from the Merck Manual, 17th ed., Chapter 222, and which is hereby incorporated by reference in their entirety:

| Type | Risk Factors |
| --- | --- |
| **Prerenal** | |
| ECF volume depletion | Excessive diuresis, hemorrhage, GI losses, loss of intravascular fluid into the extravascular space (due to ascites, peritonitis, pancreatitis, or burns), loss of skin and mucus membranes, renal salt- and water-wasting states |
| Low cardiac output | Cardiomyopathy, MI, cardiac tamponade, pulmonary embolism, pulmonary hypertension, positive-pressure mechanical ventilation |
| Low systemic vascular resistance | Septic shock, liver failure, antihypertensive drugs |
| Increased renal vascular resistance | NSAIDs, cyclosporines, tacrolimus, hypercalcemia, anaphylaxis, anesthetics, renal artery obstruction, renal vein thrombosis, sepsis, hepatorenal syndrome |
| Decreased efferent arteriolar tone (leading to decreased GFR from reduced glomerular transcapillary pressure, especially in patients with bilateral renal artery stenosis) | ACE inhibitors or angiotensin II receptor blockers |
| **Intrinsic Renal** | |
| Acute tubular injury | Ischemia (prolonged or severe prerenal state): surgery, hemorrhage, arterial or venous obstruction; Toxins: NSAIDs, cyclosporines, tacrolimus, aminoglycosides, foscarnet, ethylene glycol, hemoglobin, myoglobin, ifosfamide, heavy metals, methotrexate, radiopaque contrast agents, streptozotocin |

(continued)

| Intrinsic Renal | |
|---|---|
| Acute glomerulonephritis | ANCA-associated: Crescentic glomerulonephritis, polyarteritis nodosa, Wegener's granulomatosis; Anti-GBM glomerulonephritis: Goodpasture's syndrome; Immune-complex: Lupus glomerulonephritis, postinfectious glomerulonephritis, cryoglobulinemic glomerulonephritis |
| Acute tubulointerstitial nephritis | Drug reaction (eg, β-lactams, NSAIDs, sulfonamides, ciprofloxacin, thiazide diuretics, furosemide, phenytoin, allopurinol, pyelonephritis, papillary necrosis |
| Acute vascular nephropathy | Vasculitis, malignant hypertension, thrombotic microangiopathies, scleroderma, atheroembolism |
| Infiltrative diseases | Lymphoma, sarcoidosis, leukemia |
| Postrenal | |
| Tubular precipitation | Uric acid (tumor lysis), sulfonamides, triamterene, acyclovir, indinavir, methotrexate, ethylene glycol ingestion, myeloma protein, myoglobin |
| Ureteral obstruction | Intrinsic: Calculi, clots, sloughed renal tissue, fungus ball, edema, malignancy, congenital defects; Extrinsic: Malignancy, retroperitoneal fibrosis, ureteral trauma during surgery or high impact injury |
| Bladder obstruction | Mechanical: Benign prostatic hyperplasia, prostate cancer, bladder cancer, urethral strictures, phimosis, paraphimosis, urethral valves, obstructed indwelling urinary catheter; Neurogenic: Anticholinergic drugs, upper or lower motor neuron lesion |

In the case of ischemic ARF, the course of the disease may be divided into four phases. During an initiation phase, which lasts hours to days, reduced perfusion of the kidney is evolving into injury. Glomerular ultrafiltration reduces, the flow of filtrate is reduced due to debris within the tubules, and back leakage of filtrate through injured epithelium occurs. Renal injury can be mediated during this phase by reperfusion of the kidney. Initiation is followed by an extension phase which is characterized by continued ischemic injury and inflammation and may involve endothelial damage and vascular congestion. During the maintenance phase, lasting from 1 to 2 weeks, renal cell injury occurs, and glomerular filtration and urine output reaches a minimum. A recovery phase can follow in which the renal epithelium is repaired and GFR gradually recovers. Despite this, the survival rate of subjects with ARF may be as low as about 60%.

Acute kidney injury caused by radiocontrast agents (also called contrast media) and other nephrotoxins such as cyclosporine, antibiotics including aminoglycosides and anticancer drugs such as cisplatin manifests over a period of days to about a week. Contrast induced nephropathy (CIN, which is AKI caused by radiocontrast agents) is thought to be caused by intrarenal vasoconstriction (leading to ischemic injury) and from the generation of reactive oxygen species that are directly toxic to renal tubular epithelial cells. CIN classically presents as an acute (onset within 24-48h) but reversible (peak 3-5 days, resolution within 1 week) rise in blood urea nitrogen and serum creatinine.

A commonly reported criteria for defining and detecting AKI is an abrupt (typically within about 2-7 days or within a period of hospitalization) elevation of serum creatinine. Although the use of serum creatinine elevation to define and detect AKI is well established, the magnitude of the serum creatinine elevation and the time over which it is measured to define AKI varies considerably among publications. Traditionally, relatively large increases in serum creatinine such as 100%, 200%, an increase of at least 100% to a value over 2 mg/dL and other definitions were used to define AKI. However, the recent trend has been towards using smaller serum creatinine rises to define AKI. The relationship between serum creatinine rise, AKI and the associated health risks are reviewed in Praught and Shlipak, Curr Opin Nephrol Hypertens 14:265-270, 2005 and Chertow et al, J Am Soc Nephrol 16: 3365-3370, 2005, which, with the references listed therein, are hereby incorporated by reference in their entirety. As described in these publications, acute worsening renal function (AKI) and increased risk of death and other detrimental outcomes are now known to be associated with very small increases in serum creatinine. These increases may be determined as a relative (percent) value or a nominal value. Relative increases in serum creatinine as small as 20% from the pre-injury value have been reported to indicate acutely worsening renal function (AKI) and increased health risk, but the more commonly reported value to define AKI and increased health risk is a relative increase of at least 25%. Nominal increases as small as 0.3 mg/dL, 0.2 mg/dL or even 0.1 mg/dL have been reported to indicate worsening renal function and increased risk of death. Various time periods for the serum creatinine to rise to these threshold values have been used to define AKI, for example, ranging from 2 days, 3 days, 7 days, or a variable period defined as the time the patient is in the hospital or intensive care unit. These studies indicate there is not a particular threshold serum creatinine rise (or time period for the rise) for worsening renal function or AKI, but rather a continuous increase in risk with increasing magnitude of serum creatinine rise.

One study (Lassnigg et all, J Am Soc Nephrol 15:1597-1605, 2004, hereby incorporated by reference in its entirety) investigated both increases and decreases in serum creatinine. Patients with a mild fall in serum creatinine of -0.1 to -0.3 mg/dL following heart surgery had the lowest mortality rate. Patients with a larger fall in serum creatinine (more than or equal to -0.4 mg/dL) or any increase in serum creatinine had a larger mortality rate. These findings caused the authors to conclude that even very subtle changes in renal function (as detected by small creatinine changes within 48 hours of surgery) seriously effect patient's outcomes. In an effort to reach consensus on a unified classification system for using serum creatinine to define AKI in clinical trials and in clinical practice, Bellomo et al., Crit Care. 8(4):R204-12, 2004, which is hereby incorporated by reference in its entirety, proposes the following classifications for stratifying AKI patients:

"Risk": serum creatinine increased 1.5 fold from baseline OR urine production of <0.5 ml/kg body weight/hr for 6 hours;

"Injury": serum creatinine increased 2.0 fold from baseline OR urine production <0.5 ml/kg/hr for 12 h;

"Failure": serum creatinine increased 3.0 fold from baseline OR creatinine >355 $\mu$mol/l (with a rise of >44) or urine output below 0.3 ml/kg/hr for 24 h or anuria for at least 12 hours;

And included two clinical outcomes:

"Loss": persistent need for renal replacement therapy for more than four weeks.

"ESRD": end stage renal disease-the need for dialysis for more than 3 months.

These criteria are called the RIFLE criteria, which provide a useful clinical tool to classify renal status. As discussed in Kellum, Crit. Care Med. 36: S141-45, 2008 and Ricci et al., Kidney Int. 73, 538-546, 2008, each hereby incorporated by reference in its entirety, the RIFLE criteria provide a uniform definition of AKI which has been validated in numerous studies.

More recently, Mehta et al., Crit. Care 11:R31 (doi:10.1186.cc5713), 2007, hereby incorporated by reference in its entirety, proposes the following similar classifications for stratifying AKI patients, which have been modified from RIFLE:

"Stage I": increase in serum creatinine of more than or equal to 0.3 mg/dL ($\geq$26.4 $\mu$mol/L) or increase to more than or equal to 150% (1.5-fold) from baseline OR urine output less than 0.5 mL/kg per hour for more than 6 hours;

"Stage II": increase in serum creatinine to more than 200% (> 2-fold) from baseline OR urine output less than 0.5 mL/kg per hour for more than 12 hours;

"Stage III": increase in serum creatinine to more than 300% (> 3-fold) from baseline OR serum creatinine $\geq$354 $\mu$mol/L accompanied by an acute increase of at least 44 $\mu$mol/L OR urine output less than 0.3 mL/kg per hour for 24 hours or anuria for 12 hours.

The CIN Consensus Working Panel (McCollough et al, Rev Cardiovasc Med. 2006; 7(4):177-197, hereby incorporated by reference in its entirety) uses a serum creatinine rise of 25% to define Contrast induced nephropathy (which is a type of AKI).Although various groups propose slightly different criteria for using serum creatinine to detect AKI, the consensus is that small changes in serum creatinine, such as 0.3 mg/dL or 25%, are sufficient to detect AKI (worsening renal function) and that the magnitude of the serum creatinine change is an indicator of the severity of the AKI and mortality risk.

Although serial measurement of serum creatinine over a period of days is an accepted method of detecting and diagnosing AKI and is considered one of the most important tools to evaluate AKI patients, serum creatinine is generally regarded to have several limitations in the diagnosis, assessment and monitoring of AKI patients. The time period for serum creatinine to rise to values (e.g., a 0.3 mg/dL or 25% rise) considered diagnostic for AKI can be 48 hours or longer depending on the definition used. Since cellular injury in AKI can occur over a period of hours, serum creatinine elevations detected at 48 hours or longer can be a late indicator of injury, and relying on serum creatinine can thus delay diagnosis of AKI. Furthermore, serum creatinine is not a good indicator of the exact kidney status and treatment needs during the most acute phases of AKI when kidney function is changing rapidly. Some patients with AKI will recover fully, some will need dialysis (either short term or long term) and some will have other detrimental outcomes including death, major adverse cardiac events and chronic kidney disease. Because serum creatinine is a marker of filtration rate, it does not differentiate between the causes of AKI (pre-renal, intrinsic renal, post-renal obstruction, atheroembolic, etc) or the category or location of injury in intrinsic renal disease (for example, tubular, glomerular or interstitial in origin). Urine output is similarly limited, Knowing these things can be of vital importance in managing and treating patients with AKI.

These limitations underscore the need for better methods to detect and assess AKI, particularly in the early and subclinical stages, but also in later stages when recovery and repair of the kidney can occur. Furthermore, there is a need to better identify patients who are at risk of having an AKI.

BRIEF SUMMARY OF THE INVENTION

It is an object of the invention to provide methods and compositions for evaluating renal function in a subject. As described herein, measurement of one or more biomarkers selected from the group consisting of Proheparin-binding EGF-like growth factor, Tenascin C, Angiopoietin-related protein 4, Fibroblast growth factor 19, Fibroblast growth factor 21, Heparin-binding growth factor 1, Angiopoietin-related protein 6, Proepiregulin, Probetacellulin, Amphiregulin, Angiogenin, Thrombospondin-2, and Collagen alpha-1(XVIII) chain (each referred to herein as a "kidney injury marker") can be used for diagnosis, prognosis, risk stratification, staging, monitoring, categorizing and determination of further diagnosis and treatment regimens in subjects suffering or at risk of suffering from an injury to renal function, reduced renal function, and/or acute renal failure (also called acute kidney injury).

The kidney injury markers of the present invention may be used, individually or in panels comprising a plurality of kidney injury markers, for risk stratification (that is, to identify subjects at risk for a future injury to renal function, for future progression to reduced renal function, for future progression to ARF, for future improvement in renal function, *etc.*); for diagnosis of existing disease (that is, to identify subjects who have suffered an injury to renal function, who have progressed to reduced renal function, who have progressed to ARF, *etc.*); for monitoring for deterioration or improvement of renal function; and for predicting a future medical outcome, such as improved or worsening renal function, a decreased or increased mortality risk, a decreased or increased risk that a subject will require renal replacement therapy (*i.e.,* hemodialysis, peritoneal dialysis, hemofiltration, and/or renal transplantation, a decreased or increased risk that a subject will recover from an injury to renal function, a decreased or increased risk that a subject will recover from ARF, a decreased or increased risk that a subject will progress to end stage renal disease, a decreased or increased risk that a subject will progress to chronic renal failure, a decreased or increased risk that a subject will suffer rejection of a transplanted kidney, *etc.*

In a first aspect, the present invention relates to methods for evaluating renal status in a subject. These methods comprise performing an assay method that is configured to detect one or more biomarkers selected from the group consisting of Proheparin-binding EGF-like growth factor, Tenascin C, Angiopoietin-related protein 4, Fibroblast growth factor 19, Fibroblast growth factor 21, Heparin-binding growth factor 1, Angiopoietin-related protein 6, Proepiregulin, Probetacellulin, Amphiregulin, Angiogenin, Thrombospondin-2, and Collagen alpha-1(XVIII) chain is/are then correlated to the renal status of the subject. This correlation to renal status may include correlating the assay result(s) to one or more of risk stratification, diagnosis, prognosis, staging, classifying and monitoring of the subject as described herein. Thus, the present invention utilizes one or more kidney injury markers of the present invention for the evaluation of renal injury.

In certain embodiments, the methods for evaluating renal status described herein are methods for risk stratification of the subject; that is, assigning a likelihood of one or more future changes in renal status to the subject. In these embodiments, the assay result(s) is/are correlated to one or more such future changes. The following are preferred risk stratification embodiments.

In preferred risk stratification embodiments, these methods comprise determining a subject's risk for a future injury to renal function, and the assay result(s) is/are correlated to a likelihood of such a future injury to renal function. For example, the measured concentration(s) may each be compared to a threshold value. For a "positive going" kidney injury marker, an increased likelihood of suffering a future injury to renal function is assigned to the subject when the measured concentration is above the threshold, relative to a likelihood assigned when the measured concentration is below the threshold. For a "negative going" kidney injury marker, an increased likelihood of suffering a future injury to renal function is assigned to the subject when the measured concentration is below the threshold, relative to a likelihood assigned when the measured concentration is above the threshold.

In other preferred risk stratification embodiments, these methods comprise determining a subject's risk for future reduced renal function, and the assay result(s) is/are correlated to a likelihood of such reduced renal function. For example, the measured concentrations may each be compared to a threshold value. For a "positive going" kidney injury marker, an increased likelihood of suffering a future reduced renal function is assigned to the subject when the measured concentration is above the threshold, relative to a likelihood assigned when the measured concentration is below the threshold. For a "negative going" kidney injury marker, an increased likelihood of future reduced renal function is assigned to the subject when the measured concentration is below the threshold, relative to a likelihood assigned when the measured concentration is above the threshold.

In still other preferred risk stratification embodiments, these methods comprise determining a subject's likelihood for a future improvement in renal function, and the assay result(s) is/are correlated to a likelihood of such a future improvement in renal function. For example, the measured concentration(s) may each be compared to a threshold value. For a "positive going" kidney injury marker, an increased likelihood of a future improvement in renal function is assigned to the subject when the measured concentration is below the threshold, relative to a likelihood assigned when the measured concentration is above the threshold. For a "negative going" kidney injury marker, an increased likelihood of a future improvement in renal function is assigned to the subject when the measured concentration is above the threshold, relative to a likelihood assigned when the measured concentration is below the threshold.

In yet other preferred risk stratification embodiments, these methods comprise determining a subject's risk for progression to ARF, and the result(s) is/are correlated to a likelihood of such progression to ARF. For example, the measured concentration(s) may each be compared to a threshold value. For a "positive going" kidney injury marker, an increased likelihood of progression to ARF is assigned to the subject when the measured concentration is above the threshold, relative to a likelihood assigned when the measured concentration is below the threshold. For a "negative going" kidney injury marker, an increased likelihood of progression to ARF is assigned to the subject when the measured concentration is below the threshold, relative to a likelihood assigned when the measured concentration is above the threshold.

And in other preferred risk stratification embodiments, these methods comprise determining a subject's outcome risk, and the assay result(s) is/are correlated to a likelihood of the occurrence of a clinical outcome related to a renal injury suffered by the subject. For example, the measured concentration(s) may each be compared to a threshold value. For a "positive going" kidney injury marker, an increased likelihood of one or more of: acute kidney injury, progression to a worsening stage of AKI, mortality, a requirement for renal replacement therapy, a requirement for withdrawal of renal toxins, end stage renal disease, heart failure, stroke, myocardial infarction, progression to chronic kidney disease, etc., is assigned to the subject when the measured concentration is above the threshold, relative to a likelihood assigned when the measured concentration is below the threshold. For a "negative going" kidney injury marker, an increased likelihood of one or more of: acute kidney injury, progression to a worsening stage of AKI, mortality, a requirement for renal replacement therapy, a requirement for withdrawal of renal toxins, end stage renal disease, heart failure, stroke, myocardial infarction, progression to chronic kidney disease, etc., is assigned to the subject when the measured concentration is below the threshold, relative to a likelihood assigned when the measured concentration is above the threshold.

In such risk stratification embodiments, preferably the likelihood or risk assigned is that an event of interest is more or less likely to occur within 180 days of the time at which the body fluid sample is obtained from the subject. In particularly preferred embodiments, the likelihood or risk assigned relates to an event of interest occurring within a shorter time period such as 18 months, 120 days, 90 days, 60 days, 45 days, 30 days, 21 days, 14 days, 7 days, 5 days, 96 hours, 72 hours, 48 hours, 36 hours, 24 hours, 12 hours, or less. A risk at 0 hours of the time at which the body fluid sample is obtained from the subject is equivalent to diagnosis of a current condition.

In preferred risk stratification embodiments, the subject is selected for risk stratification based on the pre-existence in the subject of one or more known risk factors for prerenal, intrinsic renal, or postrenal ARF. For example, a subject undergoing or having undergone major vascular surgery, coronary artery bypass, or other cardiac surgery; a subject having pre-existing congestive heart failure, preeclampsia, eclampsia, diabetes mellitus, hypertension, coronary artery disease, proteinuria, renal insufficiency, glomerular filtration below the normal range, cirrhosis, serum creatinine above the normal range, or sepsis; or a subject exposed to NSAIDs, cyclosporines, tacrolimus, aminoglycosides, foscarnet, ethylene glycol, hemoglobin, myoglobin, ifosfamide, heavy metals, methotrexate, radiopaque contrast agents, or streptozotocin are all preferred subjects for monitoring risks according to the methods described herein. This list is not meant to be limiting. By "pre-existence" in this context is meant that the risk factor exists at the time the body fluid sample is obtained from the subject. In particularly preferred embodiments, a subject is chosen for risk stratification based on an existing diagnosis of injury to renal function, reduced renal function, or ARF.

In other embodiments, the methods for evaluating renal status described herein are methods for diagnosing a renal injury in the subject; that is, assessing whether or not a subject has suffered from an injury to renal function, reduced renal function, or ARF. In these embodiments, the assay result(s), for example measured concentration(s) of one or more biomarkers selected from the group consisting of Proheparin-binding EGF-like growth factor, Tenascin C, Angiopoietin-related protein 4, Fibroblast growth factor 19, Fibroblast growth factor 21, Heparin-binding growth factor 1, Angiopoietin-related protein 6, Proepiregulin, Probetacellulin, Amphiregulin, Angiogenin, Thrombospondin-2, and Collagen alpha-1(XVIII) chain is/are correlated to the occurrence or nonoccurrence of a change in renal status. The following are preferred diagnostic embodiments.

In preferred diagnostic embodiments, these methods comprise diagnosing the occurrence or nonoccurrence of an injury to renal function, and the assay result(s) is/are correlated to the occurrence or nonoccurrence of such an injury. For example, each of the measured concentration(s) may be compared to a threshold value. For a positive going marker, an increased likelihood of the occurrence of an injury to renal function is assigned to the subject when the measured concentration is above the threshold (relative to the likelihood assigned when the measured concentration is below the threshold); alternatively, when the measured concentration is below the threshold, an increased likelihood of the nonoccurrence of an injury to renal function may be assigned to the subject (relative to the likelihood assigned when the measured concentration is above the threshold). For a negative going marker, an increased likelihood of the occurrence of an injury to renal function is assigned to the subject when the measured concentration is below the threshold (relative to the likelihood assigned when the measured concentration is above the threshold); alternatively, when the measured concentration is above the threshold, an increased likelihood of the nonoccurrence of an injury to renal function may be assigned to the subject (relative to the likelihood assigned when the measured concentration is below the threshold).

In other preferred diagnostic embodiments, these methods comprise diagnosing the occurrence or nonoccurrence of reduced renal function, and the assay result(s) is/are correlated to the occurrence or nonoccurrence of an injury causing reduced renal function. For example, each of the measured concentration(s) may be compared to a threshold value. For a positive going marker, an increased likelihood of the occurrence of an injury causing reduced renal function is assigned to the subject when the measured concentration is above the threshold (relative to the likelihood assigned when the measured concentration is below the threshold); alternatively, when the measured concentration is below the threshold, an increased likelihood of the nonoccurrence of an injury causing reduced renal function may be assigned to the subject (relative to the likelihood assigned when the measured concentration is above the threshold). For a negative going marker, an increased likelihood of the occurrence of an injury causing reduced renal function is assigned to the subject when the measured concentration is below the threshold (relative to the likelihood assigned when the measured concentration is above the threshold); alternatively, when the measured concentration is above the threshold, an increased likelihood of the nonoccurrence of an injury causing reduced renal function may be assigned to the subject (relative to the likelihood assigned when the measured concentration is below the threshold).

In yet other preferred diagnostic embodiments, these methods comprise diagnosing the occurrence or nonoccurrence of ARF, and the assay result(s) is/are correlated to the occurrence or nonoccurrence of an injury causing ARF. For example, each of the measured concentration(s) may be compared to a threshold value. For a positive going marker, an increased likelihood of the occurrence of ARF is assigned to the subject when the measured concentration is above the threshold (relative to the likelihood assigned when the measured concentration is below the threshold); alternatively, when the measured concentration is below the threshold, an increased likelihood of the nonoccurrence of ARF may be assigned to the subject (relative to the likelihood assigned when the measured concentration is above the threshold). For a negative going marker, an increased likelihood of the occurrence of ARF is assigned to the subject when the measured concentration is below the threshold (relative to the likelihood assigned when the measured concentration is above the threshold); alternatively, when the measured concentration is above the threshold, an increased likelihood of the nonoccurrence of ARF may be assigned to the subject (relative to the likelihood assigned when the measured concentration is below the threshold).

In still other preferred diagnostic embodiments, these methods comprise diagnosing a subject as being in need of renal replacement therapy, and the assay result(s) is/are correlated to a need for renal replacement therapy. For example, each of the measured concentration(s) may be compared to a threshold value. For a positive going marker, an increased likelihood of the occurrence of an injury creating a need for renal replacement therapy is assigned to the subject when the measured concentration is above the threshold (relative to the likelihood assigned when the measured concentration is below the threshold); alternatively, when the measured concentration is below the threshold, an increased likelihood of the nonoccurrence of an injury creating a need for renal replacement therapy may be assigned to the subject (relative to the likelihood assigned when the measured concentration is above the threshold). For a negative going marker, an increased likelihood of the occurrence of an injury creating a need for renal replacement therapy is assigned to the subject when the measured concentration is below the threshold (relative to the likelihood assigned when the measured concentration is above the threshold); alternatively, when the measured concentration is above the threshold, an increased likelihood of the nonoccurrence of an injury creating a need for renal replacement therapy may be assigned to the subject (relative to the likelihood assigned when the measured concentration is below the threshold).

In still other preferred diagnostic embodiments, these methods comprise diagnosing a subject as being in need of renal transplantation, and the assay result(s0 is/are correlated to a need for renal transplantation. For example, each of the measured concentration(s) may be compared to a threshold value. For a positive going marker, an increased likelihood of the occurrence of an injury creating a need for renal transplantation is assigned to the subject when the measured concentration is above the threshold (relative to the likelihood assigned when the measured concentration is below the threshold); alternatively, when the measured concentration is below the threshold, an increased likelihood of the nonoccurrence of an injury creating a need for renal transplantation may be assigned to the subject (relative to the likelihood assigned when the measured concentration is above the threshold). For a negative going marker, an increased likelihood of the occurrence of an injury creating a need for renal transplantation is assigned to the subject when the measured concentration is below the threshold (relative to the likelihood assigned when the measured concentration is above the threshold); alternatively, when the measured concentration is above the threshold, an increased likelihood of the nonoccurrence of an injury creating a need for renal transplantation may be assigned to the subject (relative to the likelihood assigned when the measured concentration is below the threshold).

In still other embodiments, the methods for evaluating renal status described herein are methods for monitoring a renal injury in the subject; that is, assessing whether or not renal function is improving or worsening in a subject who has suffered from an injury to renal function, reduced renal function, or ARF. In these embodiments, the assay result(s), for example measured concentration(s) of one or more biomarkers selected from the group consisting of Proheparin-binding EGF-like growth factor, Tenascin C, Angiopoietin-related protein 4, Fibroblast growth factor 19, Fibroblast growth factor 21, Heparin-binding growth factor 1, Angiopoietin-related protein 6, Proepiregulin, Probetacellulin, Amphiregulin, Angiogenin, Thrombospondin-2, and Collagen alpha-1(XVIII) chain is/are correlated to the occurrence or nonoccurrence of a change in renal status. The following are preferred monitoring embodiments.

In preferred monitoring embodiments, these methods comprise monitoring renal status in a subject suffering from an injury to renal function, and the assay result(s) is/are correlated to the occurrence or nonoccurrence of a change in renal status in the subject. For example, the measured concentration(s) may be compared to a threshold value. For a positive going marker, when the measured concentration is above the threshold, a worsening of renal function may be assigned to the subject; alternatively, when the measured concentration is below the threshold, an improvement of renal function may be assigned to the subject. For a negative going marker, when the measured concentration is below the threshold, a worsening of renal function may be assigned to the subject; alternatively, when the measured concentration is above the threshold, an improvement of renal function may be assigned to the subject.

In other preferred monitoring embodiments, these methods comprise monitoring renal status in a subject suffering from reduced renal function, and the assay result(s) is/are correlated to the occurrence or nonoccurrence of a change in renal status in the subject. For example, the measured concentration(s) may be compared to a threshold value. For a positive going marker, when the measured concentration is above the threshold, a worsening of renal function may be assigned to the subject; alternatively, when the measured concentration is below the threshold, an improvement of renal function may be assigned to the subject. For a negative going marker, when the measured concentration is below the threshold, a worsening of renal function may be assigned to the subject; alternatively, when the measured concentration is above the threshold, an improvement of renal function may be assigned to the subject.

In yet other preferred monitoring embodiments, these methods comprise monitoring renal status in a subject suffering from acute renal failure, and the assay result(s) is/are correlated to the occurrence or nonoccurrence of a change in renal status in the subject. For example, the measured concentration(s) may be compared to a threshold value. For a positive going marker, when the measured concentration is above the threshold, a worsening of renal function may be assigned to the subject; alternatively, when the measured concentration is below the threshold, an improvement of renal function may be assigned to the subject. For a negative going marker, when the measured concentration is below the threshold, a worsening of renal function may be assigned to the subject; alternatively, when the measured concentration is above the threshold, an improvement of renal function may be assigned to the subject.

In other additional preferred monitoring embodiments, these methods comprise monitoring renal status in a subject at risk of an injury to renal function due to the pre-existence of one or more known risk factors for prerenal, intrinsic renal, or postrenal ARF, and the assay result(s) is/are correlated to the occurrence or nonoccurrence of a change in renal status in the subject. For example, the measured concentration(s) may be compared to a threshold value. For a positive going marker, when the measured concentration is above the threshold, a worsening of renal function may be assigned to the subject; alternatively, when the measured concentration is below the threshold, an improvement of renal function may be assigned to the subject. For a negative going marker, when the measured concentration is below the threshold, a worsening of renal function may be assigned to the subject; alternatively, when the measured concentration is above the threshold, an improvement of renal function may be assigned to the subject.

In still other embodiments, the methods for evaluating renal status described herein are methods for classifying a renal injury in the subject; that is, determining whether a renal injury in a subject is prerenal, intrinsic renal, or postrenal; and/or further subdividing these classes into subclasses such as acute tubular injury, acute glomerulonephritis acute tubulointerstitial nephritis, acute vascular nephropathy, or infiltrative disease; and/or assigning a likelihood that a subject will progress to a particular RIFLE stage. In these embodiments, the assay result(s), for example measured concentration(s) of one or more biomarkers selected from the group consisting of Proheparin-binding EGF-like growth factor, Tenascin C, Angiopoietin-related protein 4, Fibroblast growth factor 19, Fibroblast growth factor 21, Heparin-binding growth factor 1, Angiopoietin-related protein 6, Proepiregulin, Probetacellulin, Amphiregulin, Angiogenin, Thrombospondin-2, and Collagen alpha-1(XVIII) chain is/are correlated to a particular class and/or subclass. The following are preferred classification embodiments.

In preferred classification embodiments, these methods comprise determining whether a renal injury in a subject is prerenal, intrinsic renal, or postrenal; and/or further subdividing these classes into subclasses such as acute tubular injury, acute glomerulonephritis acute tubulointerstitial nephritis, acute vascular nephropathy, or infiltrative disease; and/or assigning a likelihood that a subject will progress to a particular RIFLE stage, and the assay result(s) is/are correlated to the injury classification for the subject. For example, the measured concentration may be compared to a threshold value, and when the measured concentration is above the threshold, a particular classification is assigned; alternatively, when the measured concentration is below the threshold, a different classification may be assigned to the subject.

A variety of methods may be used by the skilled artisan to arrive at a desired threshold value for use in these methods. For example, the threshold value may be determined from a population of normal subjects by selecting a concentration representing the 75th, 85th, 90th, 95th, or 99th percentile of a kidney injury marker measured in such normal subjects. Alternatively, the threshold value may be determined from a "diseased" population of subjects, e.g., those suffering from an injury or having a predisposition for an injury (e.g., progression to ARF or some other clinical outcome such as death, dialysis, renal transplantation, etc.), by selecting a concentration representing the 75th, 85th, 90th, 95th, or 99th percentile of a kidney injury marker measured in such subjects. In another alternative, the threshold value may be determined from a prior measurement of a kidney injury marker in the same subject; that is, a temporal change in the level of a kidney injury marker in the subject may be used to assign risk to the subject.

The foregoing discussion is not meant to imply, however, that the kidney injury markers of the present invention must be compared to corresponding individual thresholds. Methods for combining assay results can comprise the use of multivariate logistical regression, loglinear modeling, neural network analysis, n-of-m analysis, decision tree analysis, calculating ratios of markers, etc. This list is not meant to be limiting. In these methods, a composite result which is determined by combining individual markers may be treated as if it is itself a marker; that is, a threshold may be determined for the composite result as described herein for individual markers, and the composite result for an individual patient compared to this threshold.

The ability of a particular test to distinguish two populations can be established using ROC analysis. For example, ROC curves established from a "first" subpopulation which is predisposed to one or more future changes in renal status, and a "second" subpopulation which is not so predisposed can be used to calculate a ROC curve, and the area under the curve provides a measure of the quality of the test. Preferably, the tests described herein provide a ROC curve area greater than 0.5, preferably at least 0.6, more preferably 0.7, still more preferably at least 0.8, even more preferably at least 0.9, and most preferably at least 0.95.

In certain aspects, the measured concentration of one or more kidney injury markers, or a composite of such markers, may be treated as continuous variables. For example, any particular concentration can be converted into a corresponding probability of a future reduction in renal function for the subject, the occurrence of an injury, a classification, etc. In yet another alternative, a threshold that can provide an acceptable level of specificity and sensitivity in separating a population of subjects into "bins" such as a "first" subpopulation (e.g., which is predisposed to one or more future changes in renal status, the occurrence of an injury, a classification, etc.) and a "second" subpopulation which is not so predisposed. A threshold value is selected to separate this first and second population by one or more of the following measures of test accuracy:

an odds ratio greater than 1, preferably at least about 2 or more or about 0.5 or less, more preferably at least about 3 or more or about 0.33 or less, still more preferably at least about 4 or more or about 0.25 or less, even more preferably at least about 5 or more or about 0.2 or less, and most preferably at least about 10 or more or about 0.1 or less;

a specificity of greater than 0.5, preferably at least about 0.6, more preferably at least about 0.7, still more preferably at least about 0.8, even more preferably at least about 0.9 and most preferably at least about 0.95, with a corresponding sensitivity greater than 0.2, preferably greater than about 0.3, more preferably greater than about 0.4, still more preferably at least about 0.5, even more preferably about 0.6, yet more preferably greater than about 0.7, still more preferably greater than about 0.8, more preferably greater than about 0.9, and most preferably greater than about 0.95;

a sensitivity of greater than 0.5, preferably at least about 0.6, more preferably at least about 0.7, still more preferably at least about 0.8, even more preferably at least about 0.9 and most preferably at least about 0.95, with a corresponding specificity greater than 0.2, preferably greater than about 0.3, more preferably greater than about 0.4, still more preferably at least about 0.5, even more preferably about 0.6, yet more preferably greater than about 0.7, still more preferably greater than about 0.8, more preferably greater than about 0.9, and most preferably greater than about 0.95;

at least about 75% sensitivity, combined with at least about 75% specificity;

a positive likelihood ratio (calculated as sensitivity/(1-specificity)) of greater than 1, at least about 2, more preferably at least about 3, still more preferably at least about 5, and most preferably at least about 10; or

a negative likelihood ratio (calculated as (1-sensitivity)/specificity) of less than 1, less than or equal to about 0.5, more preferably less than or equal to about 0.3, and most preferably less than or equal to about 0.1.

The term "about" in the context of any of the above measurements refers to +/- 5% of a given measurement.

Multiple thresholds may also be used to assess renal status in a subject. For example, a "first" subpopulation which is predisposed to one or more future changes in renal status, the occurrence of an injury, a classification, etc., and a "second" subpopulation which is not so predisposed can be combined into a single group. This group is then subdivided into three or more equal parts (known as tertiles, quartiles, quintiles, etc., depending on the number of subdivisions). An odds ratio is assigned to subjects based on which subdivision they fall into. If one considers a tertile, the lowest or highest tertile can be used as a reference for comparison of the other subdivisions. This reference subdivision is assigned an odds ratio of 1. The second tertile is assigned an odds ratio that is relative to that first tertile. That is, someone in the second tertile might be 3 times more likely to suffer one or more future changes in renal status in comparison to someone in the first tertile. The third tertile is also assigned an odds ratio that is relative to that first tertile.

In certain embodiments, the assay method is an immunoassay. Antibodies for use in such assays will specifically bind a full length kidney injury marker of interest, and may also bind one or more polypeptides that are "related" thereto, as that term is defined hereinafter. Numerous immunoassay formats are known to those of skill in the art. Preferred body fluid samples are selected from the group consisting of urine, blood, serum, saliva, tears, and plasma. In the case of those kidney injury markers which are membrane proteins as described hereinafter, preferred assays detect soluble forms thereof.

The foregoing method steps should not be interpreted to mean that the kidney injury marker assay result(s) is/are used in isolation in the methods described herein. Rather, additional variables or other clinical indicia may be included in the methods described herein. For example, a risk stratification, diagnostic, classification, monitoring, etc. method may combine the assay result(s) with one or more variables measured for the subject selected from the group consisting of demographic information (e.g., weight, sex, age, race), medical history (e.g., family history, type of surgery, pre-existing disease such as aneurism, congestive heart failure, preeclampsia, eclampsia, diabetes mellitus, hypertension, coronary artery disease, proteinuria, renal insufficiency, or sepsis, type of toxin exposure such as NSAIDs, cyclosporines, tacrolimus, aminoglycosides, foscarnet, ethylene glycol, hemoglobin, myoglobin, ifosfamide, heavy metals, methotrexate, radiopaque contrast agents, or streptozotocin), clinical variables (e.g., blood pressure, temperature, respiration rate), risk scores (APACHE score, PREDICT score, TIMI Risk Score for UA/NSTEMI, Framingham Risk Score, risk scores of Thakar et al. (J. Am. Soc. Nephrol. 16: 162-68, 2005), Mehran et al. (J. Am. Coll. Cardiol. 44: 1393-99, 2004), Wijeysundera et al. (JAMA 297: 1801-9, 2007), Goldstein and Chawla (Clin. J. Am. Soc. Nephrol. 5: 943-49, 2010), or Chawla et al. (Kidney Intl. 68: 2274-80, 2005)), a glomerular filtration rate, an estimated glomerular filtration rate, a urine production rate, a serum or plasma creatinine concentration, a urine creatinine concentration, a fractional excretion of sodium, a urine sodium concentration, a urine creatinine to serum or plasma creatinine ratio, a urine specific gravity, a urine osmolality, a urine urea nitrogen to plasma urea nitrogen ratio, a plasma BUN to creatnine ratio, a renal failure index calculated as urine sodium / (urine creatinine / plasma creatinine), a serum or plasma neutrophil gelatinase (NGAL) concentration, a urine NGAL concentration, a serum or plasma cystatin C concentration, a serum or plasma cardiac troponin concentration, a serum or plasma BNP concentration, a serum or plasma NTproBNP concentration, and a serum or plasma proBNP concentration. Other measures of renal function which may be combined with one or more kidney injury marker assay result(s) are described hereinafter and in Harrison's Principles of Internal Medicine, 17th Ed., McGraw Hill, New York, pages 1741-1830, and Current Medical Diagnosis & Treatment 2008, 47th Ed, McGraw Hill, New York, pages 785-815, each of which are hereby incorporated by reference in their entirety.

When more than one marker is measured, the individual markers may be measured in samples obtained at the same time, or may be determined from samples obtained at different (e.g., an earlier or later) times. The individual markers may also be measured on the same or different body fluid samples. For example, one kidney injury marker may be measured in a serum or plasma sample and another kidney injury marker may be measured in a urine sample. In addition, assignment of a likelihood may combine an individual kidney injury marker assay result with temporal changes in one or more additional variables.

In various related aspects, the present invention also relates to devices and kits for performing the methods described herein. Suitable kits comprise reagents sufficient for performing an assay for at least one of the described kidney injury markers, together with instructions for performing the described threshold comparisons.

In certain embodiments, reagents for performing such assays are provided in an assay device, and such assay devices may be included in such a kit. Preferred reagents can comprise one or more solid phase antibodies, the solid phase antibody comprising antibody that detects the intended biomarker target(s) bound to a solid support. In the case of sandwich immunoassays, such reagents can also include one or more detectably labeled antibodies, the detectably labeled antibody comprising antibody that detects the intended biomarker target(s) bound to a detectable label. Additional optional elements that may be provided as part of an assay device are described hereinafter.

Detectable labels may include molecules that are themselves detectable (e.g., fluorescent moieties, electrochemical labels, ecl (electrochemical luminescence) labels, metal chelates, colloidal metal particles, etc.) as well as molecules that may be indirectly detected by production of a detectable reaction product (e.g., enzymes such as horseradish peroxidase, alkaline phosphatase, etc.) or through the use of a specific binding molecule which itself may be detectable (e.g., a labeled antibody that binds to the second antibody, biotin, digoxigenin, maltose, oligohistidine, 2,4-dintrobenzene, phenylarsenate, ssDNA, dsDNA, etc.).

Generation of a signal from the signal development element can be performed using various optical, acoustical, and electrochemical methods well known in the art. Examples of detection modes include fluorescence, radiochemical detection, reflectance, absorbance, amperometry, conductance, impedance, interferometry, ellipsometry, etc. In certain of these methods, the solid phase antibody is coupled to a transducer (e.g., a diffraction grating, electrochemical sensor, etc) for generation of a signal, while in others, a signal is generated by a transducer that is spatially separate from the solid phase antibody (e.g., a fluorometer that employs an excitation light source and an optical detector). This list is not meant to be limiting. Antibody-based biosensors may also be employed to determine the presence or amount of analytes that optionally eliminate the need for a labeled molecule.

DETAILED DESCRIPTION OF THE INVENTION

The present invention relates to methods and compositions for diagnosis, differential diagnosis, risk stratification, monitoring, classifying and determination of treatment regimens in subjects suffering or at risk of suffering from injury to renal function, reduced renal function and/or acute renal failure through measurement of one or more kidney injury markers. In various embodiments, a measured concentration of one or more biomarkers selected from the group consisting of Proheparin-binding EGF-like growth factor, Tenascin C, Angiopoietin-related protein 4, Fibroblast growth factor 19, Fibroblast growth factor 21, Heparin-binding growth factor 1, Angiopoietin-related protein 6, Proepiregulin, Probetacellulin, Amphiregulin, Angiogenin, Thrombospondin-2, and Collagen alpha-1(XVIII) chain or one or more markers related thereto, are correlated to the renal status of the subject.

For purposes of this document, the following definitions apply:

As used herein, an "injury to renal function" is an abrupt (within 14 days, preferably within 7 days, more preferably within 72 hours, and still more preferably within 48 hours) measurable reduction in a measure of renal function. Such an injury may be identified, for example, by a decrease in glomerular filtration rate or estimated GFR, a reduction in urine output, an increase in serum creatinine, an increase in serum cystatin C, a requirement for renal replacement therapy, *etc.* "Improvement in Renal Function" is an abrupt (within 14 days, preferably within 7 days, more preferably within 72 hours, and still more preferably within 48 hours) measurable increase in a measure of renal function. Preferred methods for measuring and/or estimating GFR are described hereinafter.

As used herein, "reduced renal function" is an abrupt (within 14 days, preferably within 7 days, more preferably within 72 hours, and still more preferably within 48 hours) reduction in kidney function identified by an absolute increase in serum creatinine of greater than or equal to 0.1 mg/dL ($\geq$8.8 $\mu$mol/L), a percentage increase in serum creatinine of greater than or equal to 20% (1.2-fold from baseline), or a reduction in urine output (documented oliguria of less than 0. 5 ml/kg per hour).

As used herein, "acute renal failure" or "ARF" is an abrupt (within 14 days, preferably within 7 days, more preferably within 72 hours, and still more preferably within 48 hours) reduction in kidney function identified by an absolute increase in serum creatinine of greater than or equal to 0.3 mg/dl ($\geq$26.4 $\mu$mol/l), a percentage increase in serum creatinine of greater than or equal to 50% (1. 5-fold from baseline), or a reduction in urine output (documented oliguria of less than 0.5 ml/kg per hour for at least 6 hours). This term is synonymous with "acute kidney injury" or "AKI."

As used herein, the term "Proheparin-binding EGF-like growth factor" refers to one or more polypeptides present in a biological sample that are derived from the Proheparin-binding EGF-like growth factor precursor (Swiss-Prot Q99075 (SEQ ID NO: 1)):

```
          10          20          30          40          50          60
     MKLLPSVVLK  LFLAAVLSAL  VTGESLERLR  RGLAAGTSNP  DPPTVSTDQL  LPLGGGRDRK

          70          80          90         100         110         120
     VRDLQEADLD  LLRVTLSSKP  QALATPNKEE  HGKRKKKGKG  LGKKRDPCLR  KYKDFCIHGE

         130         140         150         160         170         180
     CKYVKELRAP  SCICHPGYHG  ERCHGLSLPV  ENRLYTYDHT  TILAVVAVVL  SSVCLLVIVG

         190         200
     LLMFRYHRRG  GYDVENEEKV  KLGMTNSH
```

Most preferably, the Proheparin-binding EGF-like growth factor assay detects one or more soluble forms of Proheparin-binding EGF-like growth factor. Proheparin-binding EGF-like growth factor is a type I membrane protein having a large extracellular domain, most or all of which is present in soluble forms of Proheparin-binding EGF-like growth factor generated either through alternative splicing event which deletes all or a portion of the transmembrane domain, or by proteolysis of the membrane-bound form. In the case of an immunoassay, one or more antibodies that bind to epitopes within this extracellular domain may be used to detect these soluble form(s). The following domains have been identified in Proheparin-binding EGF-like growth factor:

| Residues | Length | Domain ID |
|---|---|---|
| 1-19 | 19 | Signal peptide |
| 20-208 | 189 | Proheparin-binding EGF-like growth factor |
| 63-148 | 86 | Heparin-binding EGF-like growth factor |
| 20-62 | 43 | Propeptide (alternative endings at 72, 73, 76, and 81) |
| 149-208 | 60 | Propeptide |
| 20-160 | 141 | Extracellular |
| 161-184 | 24 | Transmembrane |
| 185-208 | 24 | Cytoplasmic |

As used herein, the term "Tenascin" refers to one or more polypeptides present in a biological sample that are derived from the Tenascin precursor (Swiss-Prot P24821 (SEQ ID NO: 2))

```
              10          20          30          40          50          60
       MGAMTQLLAG  VFLAFLALAT  EGGVLKKVIR  HKRQSGVNAT  LPEENQPVVF  NHVYNIKLPV

              70          80          90         100         110         120
       GSQCSVDLES  ASGEKDLAPP  SEPSESFQEH  TVDGENQIVF  THRINIPRRA  CGCAAAPDVK

             130         140         150         160         170         180
       ELLSRLEELE  NLVSSLREQC  TAGAGCCLQP  ATGRLDTRPF  CSGRGNFSTE  GCGCVCEPGW

             190         200         210         220         230         240
       KGPNCSEPEC  PGNCHLRGRC  IDGQCICDDG  FTGEDCSQLA  CPSDCNDQGK  CVNGVCICFE

             250         260         270         280         290         300
       GYAGADCSRE  ICPVPCSEEH  GTCVDGLCVC  HDGFAGDDCN  KPLCLNNCYN  RGRCVENECV

             310         320         330         340         350         360
       CDEGFTGEDC  SELICPNDCF  DRGRCINGTC  YCEEGFTGED  CGKPTCPHAC  HTQGRCEEGQ

             370         380         390         400         410         420
       CVCDEGFAGV  DCSEKRCPAD  CHNRGRCVDG  RCECDDGFTG  ADCGELKCPN  GCSGHGRCVN

             430         440         450         460         470         480
       GQCVCDEGYT  GEDCSQLRCP  NDCHSRGRCV  EGKCVCEQGF  KGYDCSDMSC  PNDCHQHGRC

             490         500         510         520         530         540
```

VNGMCVCDDG YTGEDCRDRQ CPRDCSNRGL CVDGQCVCED GFTGPDCAEL SCPNDCHGQG

```
        550        560        570        580        590        600
RCVNGQCVCH EGFMGKDCKE QRCPSDCHGQ GRCVDGQCIC HEGFTGLDCG QHSCPSDCNN

        610        620        630        640        650        660
LGQCVSGRCI CNEGYSGEDC SEVSPPKDLV VTEVTEETVN LAWDNEMRVT EYLVVYTPTH

        670        680        690        700        710        720
EGGLEMQFRV PGDQTSTIIQ ELEPGVEYFI RVFAILENKK SIPVSARVAT YLPAPEGLKF

        730        740        750        760        770        780
KSIKETSVEV EWDPLDIAFE TWEIIFRNMN KEDEGEITKS LRRPETSYRQ TGLAPGQEYE

        790        800        810        820        830        840
ISLHIVKNNT RGPGLKRVTT TRLDAPSQIE VKDVTDTTAL ITWFKPLAEI DGIELTYGIK

        850        860        870        880        890        900
DVPGDRTTID LTEDENQYSI GNLKPDTEYE VSLISRRGDM SSNPAKETFT TGLDAPRNLR

        910        920        930        940        950        960
RVSQTDNSIT LEWRNGKAAI DSYRIKYAPI SGGDHAEVDV PKSQQATTKT TLTGLRPGTE

        970        980        990       1000       1010       1020
YGIGVSAVKE DKESNPATIN AATELDTPKD LQVSETAETS LTLLWKTPLA KFDRYRLNYS

       1030       1040       1050       1060       1070       1080
LPTGQWVGVQ LPRNTTSYVL RGLEPGQEYN VLLTAEKGRH KSKPARVKAS TEQAPELENL

       1090       1100       1110       1120       1130       1140
TVTEVGWDGL RLNWTAADQA YEHFIIQVQE ANKVEAARNL TVPGSLRAVD IPGLKAATPY

       1150       1160       1170       1180       1190       1200
TVSIYGVIQG YRTPVLSAEA STGETPNLGE VVVAEVGWDA LKLNWTAPEG AYEYFFIQVQ

       1210       1220       1230       1240       1250       1260
EADTVEAAQN LTVPGGLRST DLPGLKAATH YTITIRGVTQ DFSTTPLSVE VLTEEVPDMG

       1270       1280       1290       1300       1310       1320
NLTVTEVSWD ALRLNWTTPD GTYDQFTIQV QEADQVEEAH NLTVPGSLRS MEIPGLRAGT

       1330       1340       1350       1360       1370       1380
PYTVTLHGEV RGHSTRPLAV EVVTEDLPQL GDLAVSEVGW DGLRLNWTAA DNAYEHFVIQ

       1390       1400       1410       1420       1430       1440
VQEVNKVEAA QNLTLPGSLR AVDIPGLEAA TPYRVSIYGV IRGYRTPVLS AEASTAKEPE

       1450       1460       1470       1480       1490       1500
IGNLNVSDIT PESFNLSWMA TDGIFETFTI EIIDSNRLLE TVEYNISGAE RTAHISGLPP

       1510       1520       1530       1540       1550       1560
STDFIVYLSG LAPSIRTKTI SATATTEALP LLENLTISDI NPYGFTVSWM ASENAFDSFL

       1570       1580       1590       1600       1610       1620
VTVVDSGKLL DPQEFTLSGT QRKLELRGLI TGIGYEVMVS GFTQGHQTKP LRAEIVTEAE
```

```
        1630       1640       1650       1660       1670       1680
  PEVDNLLVSD ATPDGFRLSW TADEGVFDNF VLKIRDTKKQ SEPLEITLLA PERTRDITGL

        1690       1700       1710       1720       1730       1740
  REATEYEIEL YGISKGRRSQ TVSAIATTAM GSPKEVIFSD ITENSATVSW RAPTAQVESF

        1750       1760       1770       1780       1790       1800
  RITYVPITGG TPSMVTVDGT KTQTRLVKLI PGVEYLVSII AMKGFEESEP VSGSFTTALD

        1810       1820       1830       1840       1850       1860
  GPSGLVTANI TDSEALARWQ PAIATVDSYV ISYTGEKVPE ITRTVSGNTV EYALTDLEPA

        1870       1880       1890       1900       1910       1920
  TEYTLRIFAE KGPQKSSTIT AKFTTDLDSP RDLTATEVQS ETALLTWRPP RASVTGYLLV

        1930       1940       1950       1960       1970       1980
  YESVDGTVKE VIVGPDTTSY SLADLSPSTH YTAKIQALNG PLRSNMIQTI FTTIGLLYPF

        1990       2000       2010       2020       2030       2040
  PKDCSQAMLN GDTTSGLYTI YLNGDKAEAL EVFCDMTSDG GGWIVFLRRK NGRENFYQNW

        2050       2060       2070       2080       2090       2100
  KAYAAGFGDR REEFWLGLDN LNKITAQGQY ELRVDLRDHG ETAFAVYDKF SVGDAKTRYK

        2110       2120       2130       2140       2150       2160
  LKVEGYSGTA GDSMAYHNGR SFSTFDKDTD SAITNCALSY KGAFWYRNCH RVNLMGRYGD

        2170       2180       2190       2200
  NNHSQGVNWF HWKGHEHSIQ FAEMKLRPSN FRNLEGRRKR A
```

The following domains have been identified in Tenascin:

| Residues | Length | Domain ID |
|---|---|---|
| 1-22 | 22 | Signal peptide |
| 23-2201 | 2179 | Tenascin |
| 1072-1435 | | Missing in isoform 2 |
| 1527-1617 | | Missing in isoform 2 |
| 1072-1435 | | Missing in isoform 3 |
| 1527-1617 | | Missing in isoform 4 |
| 1072-1617 | | Missing in isoform 5 |
| 1072-1708 | | Missing in isoform 6 |

As used herein, the term "Angiopoietin-related protein 4" refers to one or more polypeptides present in a biological sample that are derived from the Angiopoietin-related protein 4 precursor (Swiss-Prot Q9BY76 (SEQ ID NO: 3))

```
         10         20         30         40         50         60
MSGAPTAGAA LMLCAATAVL LSAQGGPVQS KSPRFASWDE MNVLAHGLLQ LGQGLREHAE

         70         80         90        100        110        120
RTRSQLSALE RRLSACGSAC QGTEGSTDLP LAPESRVDPE VLHSLQTQLK AQNSRIQQLF

        130        140        150        160        170        180
HKVAQQQRHL EKQHLRIQHL QSQFGLLDHK HLDHEVAKPA RRKRLPEMAQ PVDPAHNVSR

        190        200        210        220        230        240
LHRLPRDCQE LFQVGERQSG LFEIQPQGSP PFLVNCKMTS DGGWTVIQRR HDGSVDFNRP

        250        260        270        280        290        300
WEAYKAGFGD PHGEFWLGLE KVHSITGDRN SRLAVQLRDW DGNAELLQFS VHLGGEDTAY

        310        320        330        340        350        360
SLQLTAPVAG QLGATTVPPS GLSVPFSTWD QDHDLRRDKN CAKSLSGGWW FGTCSHSNLN

        370        380        390        400
GQYFRSIPQQ RQKLKKGIFW KTWRGRYYPL QATTMLIQPM AAEAAS
```

The following domains have been identified in Angiopoietin-related protein 4:

| Residues | Length | Domain ID |
|---|---|---|
| 1-25 | 25 | Signal peptide |
| 26-406 | 381 | Angiopoietin-related protein 4 |

As used herein, the term "Fibroblast growth factor 19" refers to one or more polypeptides present in a biological sample that are derived from the Fibroblast growth factor 19 precursor (Swiss-Prot O95750 (SEQ ID NO: 4))

```
         10         20         30         40         50         60
MRSGCVVVHV WILAGLWLAV AGRPLAFSDA GPHVHYGWGD PIRLRHLYTS GPHGLSSCFL

         70         80         90        100        110        120
RIRADGVVDC ARGQSAHSLL EIKAVALRTV AIKGVHSVRY LCMGADGKMQ GLLQYSEEDC

        130        140        150        160        170        180
AFEEEIRPDG YNVYRSEKHR LPVSLSSAKQ RQLYKNRGFL PLSHFLPMLP MVPEEPEDLR

        190        200        210
GHLESDMFSS PLETDSMDPF GLVTGLEAVR SPSFEK
```

The following domains have been identified in Fibroblast growth factor 19:

| Residues | Length | Domain ID |
|---|---|---|
| 1-24 | 24 | Signal peptide |
| 25-216 | 192 | Fibroblast growth factor 19 |

As used herein, the term "Fibroblast growth factor 21" refers to one or more polypeptides present in a biological sample that are derived from the Fibroblast growth factor 21 precursor (Swiss-Prot Q9NSA1 (SEQ ID NO: 5))

```
          10          20          30          40          50          60
     MDSDETGFEH  SGLWVSVLAG  LLLGACQAHP  IPDSSPLLQF  GGQVRQRYLY  TDDAQQTEAH

          70          80          90         100         110         120
     LEIREDGTVG  GAADQSPESL  LQLKALKPGV  IQILGVKTSR  FLCQRPDGAL  YGSLHFDPEA

         130         140         150         160         170         180
     CSFRELLLED  GYNVYQSEAH  GLPLHLPGNK  SPHRDPAPRG  PARFLPLPGL  PPALPEPPGI

         190         200
     LAPQPPDVGS  SDPLSMVGPS  QGRSPSYAS
```

The following domains have been identified in Fibroblast growth factor 21:

| Residues | Length | Domain ID |
|---|---|---|
| 1-28 | 28 | Signal peptide |
| 29-209 | 181 | Fibroblast growth factor 21 |

As used herein, the term "Heparin-binding growth factor 1" refers to one or more polypeptides present in a biological sample that are derived from the Heparin-binding growth factor 1 precursor (Swiss-Prot P05230 (SEQ ID NO: 6))

```
          10          20          30          40          50          60
     MAEGEITTFT  ALTEKFNLPP  GNYKKPKLLY  CSNGGHFLRI  LPDGTVDGTR  DRSDQHIQLQ

          70          80          90         100         110         120
     LSAESVGEVY  IKSTETGQYL  AMDTDGLLYG  SQTPNEECLF  LERLEENHYN  TYISKKHAEK

         130         140         150
     NWFVGLKKNG  SCKRGPRTHY  GQKAILFLPL  PVSSD
```

The following domains have been identified in Heparin-binding growth factor 1:

| Residues | Length | Domain ID |
|---|---|---|
| 1-15 | 15 | Signal peptide |
| 16-155 | 140 | Heparin-binding growth factor 1 |
| 57-60 | | IQLQ → TDTK in isoform 2 |
| 61-155 | | Missing in isoform 2 |

As used herein, the term "Angiopoietin-related protein 6" refers to one or more polypeptides present in a biological sample that are derived from the Angiopoietin-related protein 6 precursor (Swiss-Prot Q8NI99 (SEQ ID NO: 7))

```
            10         20         30         40         50         60
      MGKPWLRALQ LLLLLGASWA RAGAPRCTYT FVLPPQKFTG AVCWSGPAST RATPEAANAS

            70         80         90        100        110        120
      ELAALRMRVG RHEELLRELQ RLAAADGAVA GEVRALRKES RGLSARLGQL RAQLQHEAGP

           130        140        150        160        170        180
      GAGPGADLGA EPAAALALLG ERVLNASAEA QRAAARFHQL DVKFRELAQL VTQQSSLIAR

           190        200        210        220        230        240
      LERLCPGGAG GQQQVLPPPP LVPVVPVRLV GSTSDTSRML DPAPEPQRDQ TQRQQEPMAS

           250        260        270        280        290        300
      PMPAGHPAVP TKPVGPWQDC AEARQAGHEQ SGVYELRVGR HVVSVWCEQQ LEGGGWTVIQ

           310        320        330        340        350        360
      RRQDGSVNFF TTWQHYKAGF GRPDGEYWLG LEPVYQLTSR GDHELLVLLE DWGGRGARAH

           370        380        390        400        410        420
      YDGFSLEPES DHYRLRLGQY HGDAGDSLSW HNDKPFSTVD RDRDSYSGNC ALYQRGGWWY

           430        440        450        460        470
      HACAHSNLNG VWHHGGHYRS RYQDGVYWAE FRGGAYSLRK AAMLIRPLKL
```

The following domains have been identified in Angiopoietin-related protein 6:

| Residues | Length | Domain ID |
|---|---|---|
| 1-20 | 20 | Signal peptide |
| 21-470 | 450 | Angiopoietin-related protein 6 |

As used herein, the term "Proepiregulin" refers to one or more polypeptides present in a biological sample that are derived from the Proepiregulin precursor (Swiss-Prot 014944 (SEQ ID NO: 8)):

```
            10         20         30         40         50         60
      MTAGRRMEML CAGRVPALLL CLGFHLLQAV LSTTVIPSCI PGESSDNCTA LVQTEDNPRV

            70         80         90        100        110        120
      AQVSITKCSS DMNGYCLHGQ CIYLVDMSQN YCRCEVGYTG VRCEHFFLTV HQPLSKEYVA

           130        140        150        160
      LTVILIILFL ITVVGSTYYF CRWYRNRKSK EPKKEYERVT SGDPELPQV
```

Most preferably, the Proepiregulin assay detects one or more soluble forms of Proepiregulin. Proepiregulin is a type I membrane protein having a large extracellular domain, most or all of which is present in soluble forms of Proepiregulin generated either through alternative splicing event which deletes all or a portion of the transmembrane domain, or by proteolysis of the membrane-bound form. In the case of an immunoassay, one or more antibodies that bind to epitopes within this extracellular domain may be used to detect these soluble form(s). Preferred assays detect Proepiregulin. The following domains have been identified in Proepiregulin:

| Residues | Length | Domain ID |
|---|---|---|
| 1-29 | 29 | Signal peptide |
| 30-169 | 140 | Proepiregulin |
| 60-108 | 49 | Proepiregulin |

(continued)

| Residues | Length | Domain ID |
|----------|--------|-----------|
| 109-169 | 61 | Propeptide |
| 60-119 | 60 | Extracellular |
| 120-140 | 21 | Transmembrane |
| 141-169 | 61 | Cytoplasmic |

As used herein, the term "Probetacellulin" refers to one or more polypeptides present in a biological sample that are derived from the Probetacellulin precursor (Swiss-Prot P35070 (SEQ ID NO: 9)):

```
            10         20         30         40         50         60
    MDRAARCSGA SSLPLLLALA LGLVILHCVV ADGNSTRSPE TNGLLCGDPE ENCAATTTQS

            70         80         90        100        110        120
    KRKGHFSRCP KQYKHYCIKG RCRFVVAEQT PSCVCDEGYI GARCERVDLF YLRGDRGQIL

           130        140        150        160        170
    VICLIAVMVV FIILVIGVCT CCHPLRKRRK RKKKEEEMET LGKDITPINE DIEETNIA
```

Most preferably, the Probetacellulin assay detects one or more soluble forms of Probetacellulin. Probetacellulin is a type I membrane protein having a large extracellular domain, most or all of which is present in soluble forms of Probetacellulin generated either through alternative splicing event which deletes all or a portion of the transmembrane domain, or by proteolysis of the membrane-bound form. In the case of an immunoassay, one or more antibodies that bind to epitopes within this extracellular domain may be used to detect these soluble form(s). The following domains have been identified in Probetacellulin:

| Residues | Length | Domain ID |
|----------|--------|-----------|
| 1-31 | 31 | Signal peptide |
| 32-178 | 147 | Probetacellulin |
| 32-111 | 80 | Betacellulin |
| 112-178 | 67 | Propeptide |
| 32-118 | 87 | Extracellular |
| 119-139 | 21 | Transmembrane |
| 140-178 | 39 | Cytoplasmic |

As used herein, the term "Amphiregulin" refers to one or more polypeptides present in a biological sample that are derived from the Amphiregulin precursor (Swiss-Prot P15514 (SEQ ID NO: 10)):

```
            10         20         30         40         50         60
    MRAPLLPPAP VVLSLLILGS GHYAAGLDLN DTYSGKREPF SGDHSADGFE VTSRSEMSSG

            70         80         90        100        110        120
    SEISPVSEMP SSSEPSSGAD YDYSEEYDNE PQIPGYIVDD SVRVEQVVKP PQNKTESENT

           130        140        150        160        170        180
    SDKPKRKKKG GKNGKNRRNR KKKNPCNAEF QNFCIHGECK YIEHLEAVTC KCQQEYFGER

           190        200        210        220        230        240
    CGEKSMKTHS MIDSSLSKIA LAAIAAFMSA VILTAVAVIT VQLRRQYVRK YEGEAEERKK

           250
    LRQENGNVHA IA
```

The following domains have been identified in Amphiregulin:

| Residues | Length | Domain ID |
|---|---|---|
| 1-19 | 19 | Signal peptide |
| 20-100 | 81 | Propeptide |
| 101-184 | 84 | Amphiregulin |
| 185-252 | 68 | Propeptide |
| 199-221 | 23 | Transmembrane |

As used herein, the term "Angiogenin" refers to one or more polypeptides present in a biological sample that are derived from the Angiogenin precursor (Swiss-Prot P03950 (SEQ ID NO: 11))

```
          10         20         30         40         50         60
  MVMGLGVLLL VFVLGLGLTP PTLAQDNSRY THFLTQHYDA KPQGRDDRYC ESIMRRRGLT

          70         80         90        100        110        120
  SPCKDINTFI HGNKRSIKAI CENKNGNPHR ENLRISKSSF QVTTCKLHGG SPWPPCQYRA

         130        140
  TAGFRNVVVA CENGLPVHLD QSIFRRP
```

The following domains have been identified in Angiogenin:

| Residues | Length | Domain ID |
|---|---|---|
| 1-24 | 24 | Signal peptide |
| 25-147 | 123 | Angiogenin |

As used herein, the term "Thrombospondin-2" refers to one or more polypeptides present in a biological sample that are derived from the Thrombospondin-2 precursor (Swiss-Prot P35442 (SEQ ID NO: 12))

```
          10         20         30         40         50         60
  MVWRLVLLAL WVWPSTQAGH QDKDTTFDLF SISNINRKTI GAKQFRGPDP GVPAYRFVRF

          70         80         90        100        110        120
  DYIPPVNADD LSKITKIMRQ KEGFFLTAQL KQDGKSRGTL LALEGPGLSQ RQFEIVSNGP

         130        140        150        160        170        180
  ADTLDLTYWI DGTRHVVSLE DVGLADSQWK NVTVQVAGET YSLHVGCDLI DSFALDEPFY

         190        200        210        220        230        240
  EHLQAEKSRM YVAKGSARES HFRGLLQNVH LVFENSVEDI LSKKGCQQGQ GAEINAISEN

         250        260        270        280        290        300
  TETLRLGPHV TTEYVGPSSE RRPEVCERSC EELGNMVQEL SGLHVLVNQL SENLKRVSND

         310        320        330        340        350        360
  NQFLWELIGG PPKTRNMSAC WQDGRFFAEN ETWVVDSCTT CTCKKFKTIC HQITCPPATC

         370        380        390        400        410        420
  ASPSFVEGEC CPSCLHSVDG EEGWSPWAEW TQCSVTCGSG TQQRGRSCDV TSNTCLGPSI
```

```
         430        440        450        460        470        480
    QTRACSLSKC DTRIRQDGGW SHWSPWSSCS VTCGVGNITR IRLCNSPVPQ MGGKNCKGSG

         490        500        510        520        530        540
    RETKACQGAP CPIDGRWSPW SPWSACTVTC AGGIRERTRV CNSPEPQYGG KACVGDVQER

         550        560        570        580        590        600
    QMCNKRSCPV DGCLSNPCFP GAQCSSFPDG SWSCGSCPVG FLGNGTHCED LDECALVPDI

         610        620        630        640        650        660
    CFSTSKVPRC VNTQPGFHCL PCPPRYRGNQ PVGVGLEAAK TEKQVCEPEN PCKDKTHNCH

         670        680        690        700        710        720
    KHAECIYLGH FSDPMYKCEC QTGYAGDGLI CGEDSDLDGW PNLNLVCATN ATYHCIKDNC

         730        740        750        760        770        780
    PHLPNSGQED FDKDGIGDAC DDDDDNDGVT DEKDNCQLLF NPRQADYDKD EVGDRCDNCP

         790        800        810        820        830        840
    YVHNPAQIDT DNNGEGDACS VDIDGDDVFN ERDNCPYVYN TDQRDTDGDG VGDHCDNCPL

         850        860        870        880        890        900
    VHNPDQTDVD NDLVGDQCDN NEDIDDDGHQ NNQDNCPYIS NANQADHDRD GQGDACDPDD

         910        920        930        940        950        960
    DNDGVPDDRD NCRLVFNPDQ EDLDGDGRGD ICKDDFDNDN IPDIDDVCPE NNAISETDFR

         970        980        990       1000       1010       1020
    NFQMVPLDPK GTTQIDPNWV IRHQGKELVQ TANSDPGIAV GFDEFGSVDF SGTFYVNTDR

        1030       1040       1050       1060       1070       1080
    DDDYAGFVFG YQSSSRFYVV MWKQVTQTYW EDQPTRAYGY SGVSLKVVNS TTGTGEHLRN

        1090       1100       1110       1120       1130       1140
    ALWHTGNTPG QVRTLWHDPR NIGWKDYTAY RWHLTHRPKT GYIRVLVHEG KQVMADSGPI

        1150       1160       1170
    YDQTYAGGRL GLFVFSQEMV YFSDLKYECR DI
```

The following domains have been identified in Thrombospondin-2:

| Residues | Length | Domain ID |
|----------|--------|-----------|
| 1-18 | 18 | Signal peptide |
| 19-1172 | 1154 | Thrombospondin-2 |

As used herein, the term "Collagen alpha-1 (XVIII) chain" refers to one or more polypeptides present in a biological sample that are derived from the Collagen alpha-1(XVIII) chain precursor (Swiss-Prot P39060 (SEQ ID NO: 13))

```
        10         20         30         40         50         60
MAPYPCGCHI LLLLFCCLAA ARANLLNLNW LWFNNEDTSH AATTIPEPQG PLPVQPTADT

        70         80         90        100        110        120
TTHVTPRNGS TEPATAPGSP EPPSELLEDG QDTPTSAESP DAPEENIAGV GAEILNVAKG

       130        140        150        160        170        180
IRSFVQLWND TVPTESLARA ETLVLETPVG PLALAGPSST PQENGTTLWP SRGIPSSPGA

       190        200        210        220        230        240
HTTEAGTLPA PTPSPPSLGR PWAPLTGPSV PPPSSGRASL SSLLGGAPPW GSLQDPDSQG

       250        260        270        280        290        300
LSPAAAAPSQ QLQRPDVRLR TPLLHPLVMG SLGKHAAPSA FSSGLPGALS QVAVTTLTRD

       310        320        330        340        350        360
SGAWVSHVAN SVGPGLANNS ALLGADPEAP AGRCLPLPPS LPVCGHLGIS RFWLPNHLHH

       370        380        390        400        410        420
ESGEQVRAGA RAWGGLLQTH CHPFLAWFFC LLLVPPCGSV PPPAPPPCCQ FCEALQDACW

       430        440        450        460        470        480
SRLGGGRLPV ACASLPTQED GYCVLIGPAA ERISEEVGLL QLLGDPPPQQ VTQTDDPDVG

       490        500        510        520        530        540
LAYVFGPDAN SGQVARYHFP SLFFRDFSLL FHIRPATEGP GVLFAITDSA QAMVLLGVKL

       550        560        570        580        590        600
SGVQDGHQDI SLLYTEPGAG QTHTAASFRL PAFVGQWTHL ALSVAGGFVA LYVDCEEFQR

       610        620        630        640        650        660
MPLARSSRGL ELEPGAGLFV AQAGGADPDK FQGVIAELKV RRDPQVSPMH CLDEEGDDSD

       670        680        690        700        710        720
GASGDSGSGL GDARELLREE TGAALKPRLP APPPVTTPPL AGGSSTEDSR SEEVEEQTTV

       730        740        750        760        770        780
ASLGAQTLPG SDSVSTWDGS VRTPGGRVKE GGLKGQKGEP GVPGPPGRAG PPGSPCLPGP

       790        800        810        820        830        840
PGLPCPVSPL GPAGPALQTV PGPQGPPGPP GRDGTPGRDG EPGDPGEDGK PGDTGPQGFP

       850        860        870        880        890        900
GTPGDVGPKG DKGDPGVGER GPPGPQGPPG PPGPSFRHDK LTFIDMEGSG FGGDLEALRG

       910        920        930        940        950        960
PRGFPGPPGP PGVPGLPGEP GRFGVNSSDV PGPAGLPGVP GREGPPGFPG LPGPPGPPGR

       970        980        990       1000       1010       1020
EGPPGRTGQK GSLGEAGAPG HKGSKGAPGP AGARGESGLA GAPGPAGPPG PPGPPGPPGP

      1030       1040       1050       1060       1070       1080
GLPAGFDDME GSGGPFWSTA RSADGPQGPP GLPGLKGDPG VPGLPGAKGE VGADGVPGFP

      1090       1100       1110       1120       1130       1140
GLPGREGIAG PQGPKGDRGS RGEKGDPGKD GVGQPGLPGP PGPPGPVVYV SEQDGSVLSV
```

22

```
      1150       1160       1170       1180       1190       1200
 PGPEGRPGFA GFPGPAGPKG NLGSKGERGS PGPKGEKGEP GSIFSPDGGA LGPAQKGAKG

      1210       1220       1230       1240       1250       1260
 EPGFRGPPGP YGRPGYKGEI GFPGRPGRPG MNGLKGEKGE PGDASLGFGM RGMPGPPGPP

      1270       1280       1290       1300       1310       1320
 GPPGPPGTPV YDSNVFAESS RPGPPGLPGN QGPPGPKGAK GEVGPPGPPG QFPFDFLQLE

      1330       1340       1350       1360       1370       1380
 AEMKGEKGDR GDAGQKGERG EPGGGGFFGS SLPGPPGPPG PPGPRGYPGI PGPKGESIRG

      1390       1400       1410       1420       1430       1440
 QPGPPGPQGP PGIGYEGRQG PPGPPGPPGP PSFPGPHRQT ISVPGPPGPP GPPGPPGTMG

      1450       1460       1470       1480       1490       1500
 ASSGVRLWAT RQAMLGQVHE VPEGWLIFVA EQEELYVRVQ NGFRKVQLEA RTPLPRGTDN

      1510       1520       1530       1540       1550       1560
 EVAALQPPVV QLHDSNPYPR REHPHPTARP WRADDILASP PRLPEPQPYP GAPHHSSYVH

      1570       1580       1590       1600       1610       1620
 LRPARPTSPP AHSHRDFQPV LHLVALNSPL SGGMRGIRGA DFQCFQQARA VGLAGTFRAF

      1630       1640       1650       1660       1670       1680
 LSSRLQDLYS IVRRADRAAV PIVNLKDELL FPSWEALFSG SEGPLKPGAR IFSFDGKDVL

      1690       1700       1710       1720       1730       1740
 RHPTWPQKSV WHGSDPNGRR LTESYCETWR TEAPSATGQA SSLLGGRLLG QSAASCHHAY

      1750
 IVLCIENSFM TASK
```

The following domains have been identified in Collagen alpha-1 (XVIII) chain. Preferred assays detect Endostatin:

| Residues | Length | Domain ID |
|---|---|---|
| 1-23 | 23 | Signal peptide |
| 24-1754 | 1731 | Collagen alpha-1(XVIII) chain |
| 1572-1754183 | | Endostatin |
| 216-450 | | Missing in isoform 2 |
| 1-415 | | Missing in isoform 3 |
| 416-450 | | QDACWSRLGGGRLPVACASLPTQEDGYCVLIGPAA (SEQ ID NO: 14) → MAPRCPWPWPRRRRLLDVLAPLVLLLGVRAASAEP (SEQ ID NO: 15) in isoform 3 |

As used herein, the term "relating a signal to the presence or amount" of an analyte reflects the following understanding. Assay signals are typically related to the presence or amount of an analyte through the use of a standard curve calculated using known concentrations of the analyte of interest. As the term is used herein, an assay is "configured to detect" an analyte if an assay can generate a detectable signal indicative of the presence or amount of a physiologically relevant concentration of the analyte. Because an antibody epitope is on the order of 8 amino acids, an immunoassay configured to detect a marker of interest will also detect polypeptides related to the marker sequence, so long as those polypeptides contain the epitope(s) necessary to bind to the antibody or antibodies used in the assay. The term "related marker" as used herein with regard to a biomarker such as one of the kidney injury markers described herein refers to one or more fragments, variants, etc., of a particular marker or its biosynthetic parent that may be detected as a surrogate for the marker itself or as independent biomarkers. The term also refers to one or more polypeptides present in a biological sample that are derived from the biomarker precursor complexed to additional species, such as binding proteins, receptors, heparin, lipids, sugars, etc.

In this regard, the skilled artisan will understand that the signals obtained from an immunoassay are a direct result of complexes formed between one or more antibodies and the target biomolecule (*i.e.,* the analyte) and polypeptides containing the necessary epitope(s) to which the antibodies bind. While such assays may detect the full length biomarker and the assay result be expressed as a concentration of a biomarker of interest, the signal from the assay is actually a result of all such "immunoreactive" polypeptides present in the sample. Expression of biomarkers may also be determined by means other than immunoassays, including protein measurements (such as dot blots, western blots, chromatographic methods, mass spectrometry, *etc.*) and nucleic acid measurements (mRNA quatitation). This list is not meant to be limiting.

The term "positive going" marker as that term is used herein refer to a marker that is determined to be elevated in subjects suffering from a disease or condition, relative to subjects not suffering from that disease or condition. The term "negative going" marker as that term is used herein refer to a marker that is determined to be reduced in subjects suffering from a disease or condition, relative to subjects not suffering from that disease or condition.

The term "subject" as used herein refers to a human or non-human organism. Thus, the methods and compositions described herein are applicable to both human and veterinary disease. Further, while a subject is preferably a living organism, the invention described herein may be used in post-mortem analysis as well. Preferred subjects are humans, and most preferably "patients," which as used herein refers to living humans that are receiving medical care for a disease or condition. This includes persons with no defined illness who are being investigated for signs of pathology.

Preferably, an analyte is measured in a sample. Such a sample may be obtained from a subject, or may be obtained from biological materials intended to be provided to the subject. For example, a sample may be obtained from a kidney being evaluated for possible transplantation into a subject, and an analyte measurement used to evaluate the kidney for preexisting damage. Preferred samples are body fluid samples.

The term "body fluid sample" as used herein refers to a sample of bodily fluid obtained for the purpose of diagnosis, prognosis, classification or evaluation of a subject of interest, such as a patient or transplant donor. In certain embodiments, such a sample may be obtained for the purpose of determining the outcome of an ongoing condition or the effect of a treatment regimen on a condition. Preferred body fluid samples include blood, serum, plasma, cerebrospinal fluid, urine, saliva, sputum, and pleural effusions. In addition, one of skill in the art would realize that certain body fluid samples would be more readily analyzed following a fractionation or purification procedure, for example, separation of whole blood into serum or plasma components.

The term "diagnosis" as used herein refers to methods by which the skilled artisan can estimate and/or determine the probability ("a likelihood") of whether or not a patient is suffering from a given disease or condition. In the case of the present invention, "diagnosis" includes using the results of an assay, most preferably an immunoassay, for a kidney injury marker of the present invention, optionally together with other clinical characteristics, to arrive at a diagnosis (that is, the occurrence or nonoccurrence) of an acute renal injury or ARF for the subject from which a sample was obtained and assayed. That such a diagnosis is "determined" is not meant to imply that the diagnosis is 100% accurate. Many biomarkers are indicative of multiple conditions. The skilled clinician does not use biomarker results in an informational vacuum, but rather test results are used together with other clinical indicia to arrive at a diagnosis. Thus, a measured biomarker level on one side of a predetermined diagnostic threshold indicates a greater likelihood of the occurrence of disease in the subject relative to a measured level on the other side of the predetermined diagnostic threshold.

Similarly, a prognostic risk signals a probability ("a likelihood") that a given course or outcome will occur. A level or a change in level of a prognostic indicator, which in turn is associated with an increased probability of morbidity (e.g., worsening renal function, future ARF, or death) is referred to as being "indicative of an increased likelihood" of an adverse outcome in a patient.

Marker Assays

In general, immunoassays involve contacting a sample containing or suspected of containing a biomarker of interest with at least one antibody that specifically binds to the biomarker. A signal is then generated indicative of the presence or amount of complexes formed by the binding of polypeptides in the sample to the antibody. The signal is then related to the presence or amount of the biomarker in the sample. Numerous methods and devices are well known to the skilled artisan for the detection and analysis of biomarkers. *See, e.g.,* U.S. Patents 6,143,576; 6,113,855; 6,019,944; 5,985,579; 5,947,124; 5,939,272; 5,922,615; 5,885,527; 5,851,776; 5,824,799; 5,679,526; 5,525,524; and 5,480,792, and The Immunoassay Handbook, David Wild, ed. Stockton Press, New York, 1994, each of which is hereby incorporated by reference in its entirety, including all tables, figures and claims.

The assay devices and methods known in the art can utilize labeled molecules in various sandwich, competitive, or non-competitive assay formats, to generate a signal that is related to the presence or amount of the biomarker of interest. Suitable assay formats also include chromatographic, mass spectrographic, and protein "blotting" methods. Additionally, certain methods and devices, such as biosensors and optical immunoassays, may be employed to determine the presence or amount of analytes without the need for a labeled molecule. *See, e.g.,* U.S. Patents 5,631,171; and 5,955,377, each of which is hereby incorporated by reference in its entirety, including all tables, figures and claims. One skilled in the art also recognizes that robotic instrumentation including but not limited to Beckman ACCESS®, Abbott AXSYM®, Roche ELECSYS®, Dade Behring STRATUS® systems are among the immunoassay analyzers that are capable of performing immunoassays. But any suitable immunoassay may be utilized, for example, enzyme-linked immunoassays (ELISA), radioimmunoassays (RIAs), competitive binding assays, and the like.

Antibodies or other polypeptides may be immobilized onto a variety of solid supports for use in assays. Solid phases that may be used to immobilize specific binding members include include those developed and/or used as solid phases in solid phase binding assays. Examples of suitable solid phases include membrane filters, cellulose-based papers, beads (including polymeric, latex and paramagnetic particles), glass, silicon wafers, microparticles, nanoparticles, TentaGels, AgroGels, PEGA gels, SPOCC gels, and multiple-well plates. An assay strip could be prepared by coating the antibody or a plurality of antibodies in an array on solid support. This strip could then be dipped into the test sample and then processed quickly through washes and detection steps to generate a measurable signal, such as a colored spot. Antibodies or other polypeptides may be bound to specific zones of assay devices either by conjugating directly to an assay device surface, or by indirect binding. In an example of the later case, antibodies or other polypeptides may be immobilized on particles or other solid supports, and that solid support immobilized to the device surface.

Biological assays require methods for detection, and one of the most common methods for quantitation of results is to conjugate a detectable label to a protein or nucleic acid that has affinity for one of the components in the biological system being studied. Detectable labels may include molecules that are themselves detectable (*e.g.*, fluorescent moieties, electrochemical labels, metal chelates, *etc.*) as well as molecules that may be indirectly detected by production of a detectable reaction product (*e.g.*, enzymes such as horseradish peroxidase, alkaline phosphatase, *etc.*) or by a specific binding molecule which itself may be detectable (e.g., biotin, digoxigenin, maltose, oligohistidine, 2,4-dintrobenzene, phenylarsenate, ssDNA, dsDNA, etc.).

Preparation of solid phases and detectable label conjugates often comprise the use of chemical cross-linkers. Cross-linking reagents contain at least two reactive groups, and are divided generally into homofunctional cross-linkers (containing identical reactive groups) and heterofunctional cross-linkers (containing non-identical reactive groups). Homobifunctional cross-linkers that couple through amines, sulfhydryls or react non-specifically are available from many commercial sources. Maleimides, alkyl and aryl halides, alpha-haloacyls and pyridyl disulfides are thiol reactive groups. Maleimides, alkyl and aryl halides, and alpha-haloacyls react with sulfhydryls to form thiol ether bonds, while pyridyl disulfides react with sulfhydryls to produce mixed disulfides. The pyridyl disulfide product is cleavable. Imidoesters are also very useful for protein-protein cross-links. A variety of heterobifunctional cross-linkers, each combining different attributes for successful conjugation, are commercially available.

In certain aspects, the present invention provides kits for the analysis of the described kidney injury markers. The kit comprises reagents for the analysis of at least one test sample which comprise at least one antibody that a kidney injury marker. The kit can also include devices and instructions for performing one or more of the diagnostic and/or prognostic correlations described herein. Preferred kits will comprise an antibody pair for performing a sandwich assay, or a labeled species for performing a competitive assay, for the analyte. Preferably, an antibody pair comprises a first antibody conjugated to a solid phase and a second antibody conjugated to a detectable label, wherein each of the first and second antibodies that bind a kidney injury marker. Most preferably each of the antibodies are monoclonal antibodies. The instructions for use of the kit and performing the correlations can be in the form of labeling, which refers to any written or recorded material that is attached to, or otherwise accompanies a kit at any time during its manufacture, transport, sale or use. For example, the term labeling encompasses advertising leaflets and brochures, packaging materials, instructions, audio or video cassettes, computer discs, as well as writing imprinted directly on kits.

Antibodies

The term "antibody" as used herein refers to a peptide or polypeptide derived from, modeled after or substantially encoded by an immunoglobulin gene or immunoglobulin genes, or fragments thereof, capable of specifically binding an antigen or epitope. *See, e.g.* Fundamental Immunology, 3rd Edition, W.E. Paul, ed., Raven Press, N.Y. (1993); Wilson (1994; J. Immunol. Methods 175:267-273; Yarmush (1992) J. Biochem. Biophys. Methods 25:85-97. The term antibody includes antigen-binding portions, i.e., "antigen binding sites," (e.g., fragments, subsequences, complementarity determining regions (CDRs)) that retain capacity to bind antigen, including (i) a Fab fragment, a monovalent fragment consisting of the VL, VH, CL and CH1 domains; (ii) a F(ab')2 fragment, a bivalent fragment comprising two Fab fragments linked by a disulfide bridge at the hinge region; (iii) a Fd fragment consisting of the VH and CH1 domains; (iv) a Fv fragment consisting of the VL and VH domains of a single arm of an antibody, (v) a dAb fragment (Ward et al., (1989) Nature 341:544-546), which consists of a VH domain; and (vi) an isolated complementarity determining region (CDR). Single chain antibodies are also included by reference in the term "antibody."

Antibodies used in the immunoassays described herein preferably specifically bind to a kidney injury marker of the present invention. The term "specifically binds" is not intended to indicate that an antibody binds exclusively to its intended target since, as noted above, an antibody binds to any polypeptide displaying the epitope(s) to which the antibody binds. Rather, an antibody "specifically binds" if its affinity for its intended target is about 5-fold greater when compared to its affinity for a non-target molecule which does not display the appropriate epitope(s). Preferably the affinity of the antibody will be at least about 5 fold, preferably 10 fold, more preferably 25-fold, even more preferably 50-fold, and most preferably 100-fold or more, greater for a target molecule, than its affinity for a non-target molecule. In preferred embodiments, Preferred antibodies bind with affinities of at least about $10^7$ M$^{-1}$, and preferably between about $10^8$ M$^{-1}$ to about $10^9$ M$^{-1}$, about $10^9$ M$^{-1}$ to about $10^{10}$ M$^{-1}$, or about $10^{10}$ M$^{-1}$ to about $10^{12}$ M$^{-1}$ .

Affinity is calculated as $K_d = k_{off}/k_{on}$ ($k_{off}$ is the dissociation rate constant, $K_{on}$ is the association rate constant and $K_d$ is the equilibrium constant). Affinity can be determined at equilibrium by measuring the fraction bound (r) of labeled ligand at various concentrations (c). The data are graphed using the Scatchard equation: r/c = K(n-r): where r = moles of bound ligand/mole of receptor at equilibrium; c = free ligand concentration at equilibrium; K = equilibrium association constant; and n = number of ligand binding sites per receptor molecule. By graphical analysis, r/c is plotted on the Y-axis versus r on the X-axis, thus producing a Scatchard plot. Antibody affinity measurement by Scatchard analysis is well known in the art. *See, e.g.,* van Erp et al., J. Immunoassay 12: 425-43, 1991; Nelson and Griswold, Comput. Methods Programs Biomed. 27: 65-8, 1988.

The term "epitope" refers to an antigenic determinant capable of specific binding to an antibody. Epitopes usually consist of chemically active surface groupings of molecules such as amino acids or sugar side chains and usually have specific three dimensional structural characteristics, as well as specific charge characteristics. Conformational and nonconformational epitopes are distinguished in that the binding to the former but not the latter is lost in the presence of denaturing solvents.

Numerous publications discuss the use of phage display technology to produce and screen libraries of polypeptides for binding to a selected analyte. *See, e.g,* Cwirla et al., Proc. Natl. Acad. Sci. USA 87, 6378-82, 1990; Devlin et al., Science 249, 404-6, 1990, Scott and Smith, Science 249, 386-88, 1990; and Ladner et al., U.S. Pat. No. 5,571,698. A basic concept of phage display methods is the establishment of a physical association between DNA encoding a polypeptide to be screened and the polypeptide. This physical association is provided by the phage particle, which displays a polypeptide as part of a capsid enclosing the phage genome which encodes the polypeptide. The establishment of a physical association between polypeptides and their genetic material allows simultaneous mass screening of very large numbers of phage bearing different polypeptides. Phage displaying a polypeptide with affinity to a target bind to the target and these phage are enriched by affinity screening to the target. The identity of polypeptides displayed from these phage can be determined from their respective genomes. Using these methods a polypeptide identified as having a binding affinity for a desired target can then be synthesized in bulk by conventional means. *See, e.g.,* U.S. Patent No. 6,057,098, which is hereby incorporated in its entirety, including all tables, figures, and claims.

The antibodies that are generated by these methods may then be selected by first screening for affinity and specificity with the purified polypeptide of interest and, if required, comparing the results to the affinity and specificity of the antibodies with polypeptides that are desired to be excluded from binding. The screening procedure can involve immobilization of the purified polypeptides in separate wells of microtiter plates. The solution containing a potential antibody or groups of antibodies is then placed into the respective microtiter wells and incubated for about 30 min to 2 h. The microtiter wells are then washed and a labeled secondary antibody (for example, an anti-mouse antibody conjugated to alkaline phosphatase if the raised antibodies are mouse antibodies) is added to the wells and incubated for about 30 min and then washed. Substrate is added to the wells and a color reaction will appear where antibody to the immobilized polypeptide(s) are present.

The antibodies so identified may then be further analyzed for affinity and specificity in the assay design selected. In the development of immunoassays for a target protein, the purified target protein acts as a standard with which to judge the sensitivity and specificity of the immunoassay using the antibodies that have been selected. Because the binding affinity of various antibodies may differ; certain antibody pairs (e.g., in sandwich assays) may interfere with one another sterically, etc., assay performance of an antibody may be a more important measure than absolute affinity and specificity of an antibody.

While the present application describes antibody-based binding assays in detail, alternatives to antibodies as binding species in assays are well known in the art. These include receptors for a particular target, aptamers, etc. Aptamers are oligonucleic acid or peptide molecules that bind to a specific target molecule. Aptamers are usually created by selecting them from a large random sequence pool, but natural aptamers also exist. High-affinity aptamers containing modified nucleotides conferring improved characteristics on the ligand, such as improved in vivo stability or improved delivery characteristics. Examples of such modifications include chemical substitutions at the ribose and/or phosphate and/or base positions, and may include amino acid side chain functionalities.

Assay Correlations

The term "correlating" as used herein in reference to the use of biomarkers refers to comparing the presence or amount of the biomarker(s) in a patient to its presence or amount in persons known to suffer from, or known to be at risk of, a given condition; or in persons known to be free of a given condition. Often, this takes the form of comparing an assay result in the form of a biomarker concentration to a predetermined threshold selected to be indicative of the occurrence or nonoccurrence of a disease or the likelihood of some future outcome.

Selecting a diagnostic threshold involves, among other things, consideration of the probability of disease, distribution of true and false diagnoses at different test thresholds, and estimates of the consequences of treatment (or a failure to treat) based on the diagnosis. For example, when considering administering a specific therapy which is highly efficacious and has a low level of risk, few tests are needed because clinicians can accept substantial diagnostic uncertainty. On the other hand, in situations where treatment options are less effective and more risky, clinicians often need a higher degree of diagnostic certainty. Thus, cost/benefit analysis is involved in selecting a diagnostic threshold.

Suitable thresholds may be determined in a variety of ways. For example, one recommended diagnostic threshold for the diagnosis of acute myocardial infarction using cardiac troponin is the 97.5th percentile of the concentration seen in a normal population. Another method may be to look at serial samples from the same patient, where a prior "baseline" result is used to monitor for temporal changes in a biomarker level.

Population studies may also be used to select a decision threshold. Reciever Operating Characteristic ("ROC") arose from the field of signal dectection therory developed during World War II for the analysis of radar images, and ROC analysis is often used to select a threshold able to best distinguish a "diseased" subpopulation from a "nondiseased" subpopulation. A false positive in this case occurs when the person tests positive, but actually does not have the disease. A false negative, on the other hand, occurs when the person tests negative, suggesting they are healthy, when they actually do have the disease. To draw a ROC curve, the true positive rate (TPR) and false positive rate (FPR) are determined as the decision threshold is varied continuously. Since TPR is equivalent with sensitivity and FPR is equal to 1 - specificity, the ROC graph is sometimes called the sensitivity vs (1 - specificity) plot. A perfect test will have an area under the ROC curve of 1.0; a random test will have an area of 0.5. A threshold is selected to provide an acceptable level of specificity and sensitivity.

In this context, "diseased" is meant to refer to a population having one characteristic (the presence of a disease or condition or the occurrence of some outcome) and "nondiseased" is meant to refer to a population lacking the characteristic. While a single decision threshold is the simplest application of such a method, multiple decision thresholds may be used. For example, below a first threshold, the absence of disease may be assigned with relatively high confidence, and above a second threshold the presence of disease may also be assigned with relatively high confidence. Between the two thresholds may be considered indeterminate. This is meant to be exemplary in nature only.

In addition to threshold comparisons, other methods for correlating assay results to a patient classification (occurrence or nonoccurrence of disease, likelihood of an outcome, etc.) include decision trees, rule sets, Bayesian methods, and neural network methods. These methods can produce probability values representing the degree to which a subject belongs to one classification out of a plurality of classifications.

Measures of test accuracy may be obtained as described in Fischer et al., Intensive Care Med. 29: 1043-51, 2003, and used to determine the effectiveness of a given biomarker. These measures include sensitivity and specificity, predictive values, likelihood ratios, diagnostic odds ratios, and ROC curve areas. The area under the curve ("AUC") of a ROC plot is equal to the probability that a classifier will rank a randomly chosen positive instance higher than a randomly chosen negative one. The area under the ROC curve may be thought of as equivalent to the Mann-Whitney U test, which tests for the median difference between scores obtained in the two groups considered if the groups are of continuous data, or to the Wilcoxon test of ranks.

As discussed above, suitable tests may exhibit one or more of the following results on these various measures: a specificity of greater than 0.5, preferably at least 0.6, more preferably at least 0.7, still more preferably at least 0.8, even more preferably at least 0.9 and most preferably at least 0.95, with a corresponding sensitivity greater than 0.2, preferably greater than 0.3, more preferably greater than 0.4, still more preferably at least 0.5, even more preferably 0.6, yet more preferably greater than 0.7, still more preferably greater than 0.8, more preferably greater than 0.9, and most preferably greater than 0.95; a sensitivity of greater than 0.5, preferably at least 0.6, more preferably at least 0.7, still more preferably at least 0.8, even more preferably at least 0.9 and most preferably at least 0.95, with a corresponding specificity greater than 0.2, preferably greater than 0.3, more preferably greater than 0.4, still more preferably at least 0.5, even more preferably 0.6, yet more preferably greater than 0.7, still more preferably greater than 0.8, more preferably greater than 0.9, and most preferably greater than 0.95; at least 75% sensitivity, combined with at least 75% specificity; a ROC curve area of greater than 0.5, preferably at least 0.6, more preferably 0.7, still more preferably at least 0.8, even more preferably at least 0.9, and most preferably at least 0.95; an odds ratio different from 1, preferably at least about 2 or more or about 0.5 or less, more preferably at least about 3 or more or about 0.33 or less, still more preferably at least about 4 or more or about 0.25 or less, even more preferably at least about 5 or more or about 0.2 or less, and most preferably at least about 10 or more or about 0.1 or less; a positive likelihood ratio (calculated as sensitivity/(1-specificity)) of greater than 1, at least 2, more preferably at least 3, still more preferably at least 5, and most preferably at least 10; and or a negative likelihood ratio (calculated as (1-sensitivity)/specificity) of less than 1, less than or equal to 0.5, more preferably less than or equal to 0.3, and most preferably less than or equal to 0.1

Additional clinical indicia may be combined with the kidney injury marker assay result(s) of the present invention. These include other biomarkers related to renal status. Examples include the following, which recite the common biomarker name, followed by the Swiss-Prot entry number for that biomarker or its parent: Actin (P68133); Adenosine deaminase binding protein (DPP4, P27487); Alpha-1-acid glycoprotein 1 (P02763); Alpha-1-microglobulin (P02760); Albumin (P02768); Angiotensinogenase (Renin, P00797); Annexin A2 (P07355); Beta-glucuronidase (P08236); B-2-microglobulin (P61679); Beta-galactosidase (P16278); BMP-7 (P18075); Brain natriuretic peptide (proBNP, BNP-32, NTproBNP; P16860); Calcium-binding protein Beta (S100-beta, P04271); Carbonic anhydrase (Q16790); Casein Kinase 2 (P68400); Ceruloplasmin (P00450); Clusterin (P10909); Complement C3 (P01024); Cysteine-rich protein (CYR61, O00622); Cytochrome C (P99999); Epidermal growth factor (EGF, P01133); Endothelin-1 (P05305); Exosomal Fetuin-A (P02765); Fatty acid-binding protein, heart (FABP3, P05413); Fatty acid-binding protein, liver (P07148); Ferritin (light chain, P02793; heavy chain P02794); Fructose-1,6-biphosphatase (P09467); GRO-alpha (CXCL1, (P09341); Growth Hormone (P01241); Hepatocyte growth factor (P14210); Insulin-like growth factor I (P01343); Immunoglobulin G; Immunoglobulin Light Chains (Kappa and Lambda); Interferon gamma (P01308); Lysozyme (P61626); Interleukin-1alpha (P01583); Interleukin-2 (P60568); Interleukin-4 (P60568); Interleukin-9 (P15248); Interleukin-12p40 (P29460); Interleukin-13 (P35225); Interleukin-16 (Q14005); L1 cell adhesion molecule (P32004); Lactate dehydrogenase (P00338); Leucine Aminopeptidase (P28838); Meprin A-alpha subunit (Q16819); Meprin A-beta subunit (Q16820); Midkine (P21741); MIP2-alpha (CXCL2, P19875); MMP-2 (P08253); MMP-9 (P14780); Netrin-1 (095631); Neutral endopeptidase (P08473); Osteopontin (P10451); Renal papillary antigen 1 (RPA1); Renal papillary antigen 2 (RPA2); Retinol binding protein (P09455); Ribonuclease; S100 calcium-binding protein A6 (P06703); Serum Amyloid P Component (P02743); Sodium/Hydrogen exchanger isoform (NHE3, P48764); Spermidine/spermine N1-acetyltransferase (P21673); TGF-Beta1 (P01137); Transferrin (P02787); Trefoil factor 3 (TFF3, Q07654); Toll-Like protein 4 (O00206); Total protein; Tubulointerstitial nephritis antigen (Q9UJW2); Uromodulin (Tamm-Horsfall protein, P07911).

For purposes of risk stratification, Adiponectin (Q15848); Alkaline phosphatase (P05186); Aminopeptidase N (P15144); CalbindinD28k (P05937); Cystatin C (P01034); 8 subunit of FIFO ATPase (P03928); Gamma-glutamyltransferase (P19440); GSTa (alpha-glutathione-S-transferase, P08263); GSTpi (Glutathione-S-transferase P; GST class-pi; P09211); IGFBP-1 (P08833); IGFBP-2 (P18065); IGFBP-6 (P24592); Integral membrane protein 1 (Itm1, P46977); Interleukin-6 (P05231); Interleukin-8 (P10145); Interleukin-18 (Q14116); IP-10 (10 kDa interferon-gamma-induced protein, P02778); IRPR (IFRD1, O00458); Isovaleryl-CoA dehydrogenase (IVD, P26440); I-TAC/CXCL11 (014625); Keratin 19 (P08727); Kim-1 (Hepatitis A virus cellular receptor 1, O43656); L-arginine:glycine amidinotransferase (P50440); Leptin (P41159); Lipocalin2 (NGAL, P80188); MCP-1 (P13500); MIG (Gamma-interferon-induced monokine Q07325); MIP-1a (P10147); MIP-3a (P78556); MIP-1beta (P13236); MIP-1d (Q16663); NAG (N-acetyl-beta-D-glucosaminidase, P54802); Organic ion transporter (OCT2, 015244); Osteoprotegerin (014788); P8 protein (O60356); Plasminogen activator inhibitor 1 (PAI-1, P05121); ProANP(1-98) (P01160); Protein phosphatase 1-beta (PPI-beta, P62140); Rab GDI-beta (P50395); Renal kallikrein (Q86U61 ); RT1.B-1 (alpha) chain of the integral membrane protein (Q5Y7A8); Soluble tumor necrosis factor receptor superfamily member 1A (sTNFR-I, P19438); Soluble tumor necrosis factor receptor superfamily member 1B (sTNFR-II, P20333); Tissue inhibitor of metalloproteinases 3 (TIMP-3, P35625); uPAR (Q03405) may be combined with the kidney injury marker assay result(s) of the present invention.

Other clinical indicia which may be combined with the kidney injury marker assay result(s) of the present invention includes demographic information (e.g., weight, sex, age, race), medical history (e.g., family history, type of surgery, pre-existing disease such as aneurism, congestive heart failure, preeclampsia, eclampsia, diabetes mellitus, hypertension, coronary artery disease, proteinuria, renal insufficiency, or sepsis, type of toxin exposure such as NSAIDs, cyclosporines, tacrolimus, aminoglycosides, foscarnet, ethylene glycol, hemoglobin, myoglobin, ifosfamide, heavy metals, methotrexate, radiopaque contrast agents, or streptozotocin), clinical variables (e.g., blood pressure, temperature, respiration rate), risk scores (APACHE score, PREDICT score, TIMI Risk Score for UA/NSTEMI, Framingham Risk Score), a urine total protein measurement, a glomerular filtration rate, an estimated glomerular filtration rate, a urine production rate, a serum or plasma creatinine concentration, a renal papillary antigen 1 (RPA1) measurement; a renal papillary antigen 2 (RPA2) measurement; a urine creatinine concentration, a fractional excretion of sodium, a urine sodium concentration, a urine creatinine to serum or plasma creatinine ratio, a urine specific gravity, a urine osmolality, a urine urea nitrogen to plasma urea nitrogen ratio, a plasma BUN to creatnine ratio, and/or a renal failure index calculated as urine sodium / (urine creatinine / plasma creatinine). Other measures of renal function which may be combined with the kidney injury marker assay result(s) are described hereinafter and in Harrison's Principles of Internal Medicine, 17th Ed., McGraw Hill, New York, pages 1741-1830, and Current Medical Diagnosis & Treatment 2008, 47th Ed, McGraw Hill, New York, pages 785-815, each of which are hereby incorporated by reference in their entirety.

Combining assay results/clinical indicia in this manner can comprise the use of multivariate logistical regression, loglinear modeling, neural network analysis, n-of-m analysis, decision tree analysis, etc. This list is not meant to be limiting.

Diagnosis of Acute Renal Failure

As noted above, the terms "acute renal (or kidney) injury" and "acute renal (or kidney) failure" as used herein are defined in part in terms of changes in serum creatinine from a baseline value. Most definitions of ARF have common elements, including the use of serum creatinine and, often, urine output. Patients may present with renal dysfunction without an available baseline measure of renal function for use in this comparison. In such an event, one may estimate a baseline serum creatinine value by assuming the patient initially had a normal GFR. Glomerular filtration rate (GFR) is the volume of fluid filtered from the renal (kidney) glomerular capillaries into the Bowman's capsule per unit time. Glomerular filtration rate (GFR) can be calculated by measuring any chemical that has a steady level in the blood, and is freely filtered but neither reabsorbed nor secreted by the kidneys. GFR is typically expressed in units of ml/min:

$$GFR = \frac{\text{Urine Concentration} \times \text{Urine Flow}}{\text{Plasma Concentration}}$$

By normalizing the GFR to the body surface area, a GFR of approximately 75-100 ml/min per 1.73 $m^2$ can be assumed. The rate therefore measured is the quantity of the substance in the urine that originated from a calculable volume of blood.

There are several different techniques used to calculate or estimate the glomerular filtration rate (GFR or eGFR). In clinical practice, however, creatinine clearance is used to measure GFR. Creatinine is produced naturally by the body (creatinine is a metabolite of creatine, which is found in muscle). It is freely filtered by the glomerulus, but also actively secreted by the renal tubules in very small amounts such that creatinine clearance overestimates actual GFR by 10-20%. This margin of error is acceptable considering the ease with which creatinine clearance is measured.

Creatinine clearance (CCr) can be calculated if values for creatinine's urine concentration ($U_{Cr}$), urine flow rate (V), and creatinine's plasma concentration ($P_{Cr}$) are known. Since the product of urine concentration and urine flow rate yields creatinine's excretion rate, creatinine clearance is also said to be its excretion rate ($U_{Cr} \times V$) divided by its plasma concentration. This is commonly represented mathematically as:

$$C_{Cr} = \frac{U_{Cr} \times V}{P_{Cr}}$$

Commonly a 24 hour urine collection is undertaken, from empty-bladder one morning to the contents of the bladder the following morning, with a comparative blood test then taken:

$$C_{Cr} = \frac{U_{Cr} \times 24\text{-hour volume}}{P_{Cr} \times 24 \times 60 mins}$$

To allow comparison of results between people of different sizes, the CCr is often corrected for the body surface area (BSA) and expressed compared to the average sized man as ml/min/1.73 m2. While most adults have a BSA that approaches 1.7 (1.6-1.9), extremely obese or slim patients should have their CCr corrected for their actual BSA:

$$C_{Cr-corrected} = \frac{C_{Cr} \times 1.73}{BSA}$$

The accuracy of a creatinine clearance measurement (even when collection is complete) is limited because as glomerular filtration rate (GFR) falls creatinine secretion is increased, and thus the rise in serum creatinine is less. Thus, creatinine excretion is much greater than the filtered load, resulting in a potentially large overestimation of the GFR (as much as a twofold difference). However, for clinical purposes it is important to determine whether renal function is stable or getting worse or better. This is often determined by monitoring serum creatinine alone. Like creatinine clearance, the serum creatinine will not be an accurate reflection of GFR in the non-steady-state condition of ARF. Nonetheless, the degree to which serum creatinine changes from baseline will reflect the change in GFR. Serum creatinine is readily and easily measured and it is specific for renal function.

For purposes of determining urine output on a Urine output on a mL/kg/hr basis, hourly urine collection and measurement is adequate. In the case where, for example, only a cumulative 24-h output was available and no patient weights are provided, minor modifications of the RIFLE urine output criteria have been described. For example, Bagshaw et al., Nephrol. Dial. Transplant. 23: 1203-1210, 2008, assumes an average patient weight of 70 kg, and patients are assigned a RIFLE classification based on the following: <35 mL/h (Risk), <21 mL/h (Injury) or <4 mL/h (Failure).

Selecting a Treatment Regimen

Once a diagnosis is obtained, the clinician can readily select a treatment regimen that is compatible with the diagnosis, such as initiating renal replacement therapy, withdrawing delivery of compounds that are known to be damaging to the kidney, kidney transplantation, delaying or avoiding procedures that are known to be damaging to the kidney, modifying diuretic administration, initiating goal directed therapy, etc. The skilled artisan is aware of appropriate treatments for numerous diseases discussed in relation to the methods of diagnosis described herein. See, e.g., Merck Manual of Diagnosis and Therapy, 17th Ed. Merck Research Laboratories, Whitehouse Station, NJ, 1999. In addition, since the methods and compositions described herein provide prognostic information, the markers of the present invention may be used to monitor a course of treatment. For example, improved or worsened prognostic state may indicate that a particular treatment is or is not efficacious.

One skilled in the art readily appreciates that the present invention is well adapted to carry out the objects and obtain the ends and advantages mentioned, as well as those inherent therein. The examples provided herein are representative of preferred embodiments, are exemplary, and are not intended as limitations on the scope of the invention.

Example 1: Contrast-induced nephropathy sample collection

The objective of this sample collection study is to collect samples of plasma and urine and clinical data from patients before and after receiving intravascular contrast media. Approximately 250 adults undergoing radiographic/angiographic procedures involving intravascular administration of iodinated contrast media are enrolled. To be enrolled in the study, each patient must meet all of the following inclusion criteria and none of the following exclusion criteria:

Inclusion Criteria

males and females 18 years of age or older;

undergoing a radiographic / angiographic procedure (such as a CT scan or coronary intervention) involving the intravascular administration of contrast media;

expected to be hospitalized for at least 48 hours after contrast administration.

able and willing to provide written informed consent for study participation and to comply with all study procedures.

Exclusion Criteria

renal transplant recipients;

acutely worsening renal function prior to the contrast procedure;

already receiving dialysis (either acute or chronic) or in imminent need of dialysis at enrollment;

expected to undergo a major surgical procedure (such as involving cardiopulmonary bypass) or an additional imaging procedure with contrast media with significant risk for further renal insult within the 48 hrs following contrast administration;

participation in an interventional clinical study with an experimental therapy within the previous 30 days;

known infection with human immunodeficiency virus (HIV) or a hepatitis virus.

Immediately prior to the first contrast administration (and after any pre-procedure hydration), an EDTA anti-coagulated blood sample (10 mL) and a urine sample (10 mL) are collected from each patient. Blood and urine samples are then collected at 4 ($\pm$0.5), 8 ($\pm$1), 24 ($\pm$2) 48 ($\pm$2), and 72 ($\pm$2) hrs following the last administration of contrast media during the index contrast procedure. Blood is collected via direct venipuncture or via other available venous access, such as an existing femoral sheath, central venous line, peripheral intravenous line or hep-lock. These study blood samples are processed to plasma at the clinical site, frozen and shipped to Astute Medical, Inc., San Diego, CA. The study urine samples are frozen and shipped to Astute Medical, Inc.

Serum creatinine is assessed at the site immediately prior to the first contrast administration (after any pre-procedure hydration) and at 4 ($\pm$0.5), 8 ($\pm$1), 24 ($\pm$2) and 48 ($\pm$2)), and 72 ($\pm$2) hours following the last administration of contrast (ideally at the same time as the study samples are obtained). In addition, each patient's status is evaluated through day 30 with regard to additional serum and urine creatinine measurements, a need for dialysis, hospitalization status, and adverse clinical outcomes (including mortality).

Prior to contrast administration, each patient is assigned a risk based on the following assessment: systolic blood pressure <80 mm Hg = 5 points; intra-arterial balloon pump = 5 points; congestive heart failure (Class III-IV or history of pulmonary edema) = 5 points; age >75 yrs = 4 points; hematocrit level <39% for men, <35% for women = 3 points; diabetes = 3 points; contrast media volume = 1 point for each 100 mL; serum creatinine level >1.5 g/dL = 4 points OR estimated GFR 40-60 mL/min/1.73 m$^2$ = 2 points, 20-40 mL/min/1.73 m$^2$ = 4 points, < 20 mL/min/1.73 m$^2$ = 6 points. The risks assigned are as follows: risk for CIN and dialysis: 5 or less total points = risk of CIN - 7.5%, risk of dialysis - 0.04%; 6-10 total points = risk of CIN - 14%, risk of dialysis - 0.12%; 11-16 total points = risk of CIN - 26.1%, risk of dialysis - 1.09%; > 16 total points = risk of CIN - 57.3%, risk of dialysis - 12.8%.

Example 2: Cardiac surgery sample collection

The objective of this sample collection study is to collect samples of plasma and urine and clinical data from patients before and after undergoing cardiovascular surgery, a procedure known to be potentially damaging to kidney function. Approximately 900 adults undergoing such surgery are enrolled. To be enrolled in the study, each patient must meet all of the following inclusion criteria and none of the following exclusion criteria:

Inclusion Criteria

males and females 18 years of age or older;

undergoing cardiovascular surgery;

Toronto/Ottawa Predictive Risk Index for Renal Replacement risk score of at least 2 (Wijeysundera et al., JAMA 297: 1801-9, 2007); and

able and willing to provide written informed consent for study participation and to comply with all study procedures.

Exclusion Criteria

known pregnancy;

previous renal transplantation;

acutely worsening renal function prior to enrollment (e.g., any category of RIFLE criteria);

already receiving dialysis (either acute or chronic) or in imminent need of dialysis at enrollment;

currently enrolled in another clinical study or expected to be enrolled in another clinical study within 7 days of cardiac surgery that involves drug infusion or a therapeutic intervention for AKI;

known infection with human immunodeficiency virus (HIV) or a hepatitis virus.

Within 3 hours prior to the first incision (and after any pre-procedure hydration), an EDTA anti-coagulated blood sample (10 mL), whole blood (3 mL), and a urine sample (35 mL) are collected from each patient. Blood and urine samples are then collected at 3 ($\pm$0.5), 6 ($\pm$0.5), 12 ($\pm$1), 24 ($\pm$2) and 48 ($\pm$2) hrs following the procedure and then daily on days 3 through 7 if the subject remains in the hospital. Blood is collected via direct venipuncture or via other available venous access, such as an existing femoral sheath, central venous line, peripheral intravenous line or hep-lock. These study blood samples are frozen and shipped to Astute Medical, Inc., San Diego, CA. The study urine samples are frozen and shipped to Astute Medical, Inc.

Example 3: Acutely ill subject sample collection

The objective of this study is to collect samples from acutely ill patients. Approximately 1900 adults expected to be in the ICU for at least 48 hours will be enrolled. To be enrolled in the study, each patient must meet all of the following inclusion criteria and none of the following exclusion criteria:

Inclusion Criteria
males and females 18 years of age or older;
Study population 1: approximately 300 patients that have at least one of:

shock (SBP < 90 mmHg and/or need for vasopressor support to maintain MAP > 60 mmHg and/or documented drop in SBP of at least 40 mmHg); and
sepsis;

Study population 2: approximately 300 patients that have at least one of:

IV antibiotics ordered in computerized physician order entry (CPOE) within 24 hours of enrollment;
contrast media exposure within 24 hours of enrollment;
increased Intra-Abdominal Pressure with acute decompensated heart failure; and
severe trauma as the primary reason for ICU admission and likely to be hospitalized in the ICU for 48 hours after enrollment;

Study population 3: approximately 300 patients expected to be hospitalized through acute care setting (ICU or ED) with a known risk factor for acute renal injury (e.g. sepsis, hypotension/shock (Shock = systolic BP < 90 mmHg and/or the need for vasopressor support to maintain a MAP > 60 mmHg and/or a documented drop in SBP > 40 mmHg), major trauma, hemorrhage, or major surgery); and/or expected to be hospitalized to the ICU for at least 24 hours after enrollment;
Study population 4: approximately 1000 patients that are 21 years of age or older, within 24 hours of being admitted into the ICU, expected to have an indwelling urinary catheter for at least 48 hours after enrollment, and have at least one of the following acute conditions within 24 hours prior to enrollment:

(i) respiratory SOFA score of $\geq$2 (PaO2/FiO2 <300), (ii) cardiovascular SOFA score of $\geq$ 1 (MAP < 70 mm Hg and/or any vasopressor required).
Exclusion Criteria
known pregnancy;
institutionalized individuals;
previous renal transplantation;
known acutely worsening renal function prior to enrollment (e.g., any category of RIFLE criteria);
received dialysis (either acute or chronic) within 5 days prior to enrollment or in imminent need of dialysis at the time of enrollment;
known infection with human immunodeficiency virus (HIV) or a hepatitis virus;
meets any of the following:

(i) active bleeding with an anticipated need for > 4 units PRBC in a day;

(ii) hemoglobin < 7 g/dL;

(iii) any other condition that in the physician's opinion would contraindicate drawing serial blood samples for clinical study purposes;

meets only the SBP < 90 mmHg inclusion criterion set forth above, and does not have shock in the attending physician's or principal investigator's opinion;

After obtaining informed consent, an EDTA anti-coagulated blood sample (10 mL) and a urine sample (25-50 mL) are collected from each patient. Blood and urine samples are then collected at 4 ($\pm$ 0.5) and 8 ($\pm$ 1) hours after contrast administration (if applicable); at 12 ($\pm$ 1), 24 ($\pm$ 2), 36 ($\pm$ 2), 48 ($\pm$ 2), 60 ($\pm$ 2), 72 ($\pm$ 2), and 84 ($\pm$ 2) hours after enrollment, and thereafter daily up to day 7 to day 14 while the subject is hospitalized. Blood is collected via direct venipuncture or via other available venous access, such as an existing femoral sheath, central venous line, peripheral intravenous line or hep-lock. These study blood samples are processed to plasma at the clinical site, frozen and shipped to Astute Medical, Inc., San Diego, CA. The study urine samples are frozen and shipped to Astute Medical, Inc.

Example 4. Immunoassay format

Analytes are measured using standard sandwich enzyme immunoassay techniques. A first antibody which binds the analyte is immobilized in wells of a 96 well polystyrene microplate. Analyte standards and test samples are pipetted into the appropriate wells and any analyte present is bound by the immobilized antibody. After washing away any unbound substances, a horseradish peroxidase-conjugated second antibody which binds the analyte is added to the wells, thereby forming sandwich complexes with the analyte (if present) and the first antibody. Following a wash to remove any unbound antibody-enzyme reagent, a substrate solution comprising tetramethylbenzidine and hydrogen peroxide is added to the wells. Color develops in proportion to the amount of analyte present in the sample. The color development is stopped and the intensity of the color is measured at 540 nm or 570 nm. An analyte concentration is assigned to the test sample by comparison to a standard curve determined from the analyte standards.

Units for the concentrations reported in the following data tables are as follows: Proheparin-binding EGF-like growth factor - pg/mL, Tenascin C - pg/mL, Angiopoietin-related protein 4 - ng/mL, Fibroblast growth factor 19 - ng/mL, Fibroblast growth factor 21 - ng/mL, Heparin-binding growth factor 1 - pg/mL, Angiopoietin-related protein 6 - ng/mL, Proepiregulin - pg/mL, Probetacellulin - pg/mL, Amphiregulin - pg/mL, Angiogenin - pg/mL, Thrombospondin-2 - pg/mL, and Collagen alpha-1(XVIII) chain (endostatin domain) - pg/mL,. In the case of those kidney injury markers which are membrane proteins as described herein, the assays used in these examples detect soluble forms thereof.

Example 5. Apparently Healthy Donor and Chronic Disease Patient Samples

Human urine samples from donors with no known chronic or acute disease ("Apparently Healthy Donors") were purchased from two vendors (Golden West Biologicals, Inc., 27625 Commerce Center Dr., Temecula, CA 92590 and Virginia Medical Research, Inc., 915 First Colonial Rd., Virginia Beach, VA 23454). The urine samples were shipped and stored frozen at less than -20° C. The vendors supplied demographic information for the individual donors including gender, race (Black /White), smoking status and age.

Human urine samples from donors with various chronic diseases ("Chronic Disease Patients") including congestive heart failure, coronary artery disease, chronic kidney disease, chronic obstructive pulmonary disease, diabetes mellitus and hypertension were purchased from Virginia Medical Research, Inc., 915 First Colonial Rd., Virginia Beach, VA 23454. The urine samples were shipped and stored frozen at less than -20 degrees centigrade. The vendor provided a case report form for each individual donor with age, gender, race (Black/White), smoking status and alcohol use, height, weight, chronic disease(s) diagnosis, current medications and previous surgeries.

Example 6. Use of Kidney Injury Markers for evaluating renal status in patients

Patients from the intensive care unit (ICU) were enrolled in the following study. Each patient was classified by kidney status as non-injury (0), risk of injury (R), injury (I), and failure (F) according to the maximum stage reached within 7 days of enrollment as determined by the RIFLE criteria. EDTA anti-coagulated blood samples (10 mL) and a urine samples (25-30 mL) were collected from each patient at enrollment, 4 ($\pm$ 0.5) and 8 ($\pm$ 1) hours after contrast administration (if applicable); at 12 ($\pm$ 1), 24 ($\pm$ 2), and 48 ($\pm$ 2) hours after enrollment, and thereafter daily up to day 7 to day 14 while the subject is hospitalized. Markers were each measured by standard immunoassay methods using commercially available assay reagents in the urine samples and the plasma component of the blood samples collected.

Two cohorts were defined to represent a "diseased" and a "normal" population. While these terms are used for convenience, "diseased" and "normal" simply represent two cohorts for comparison (say RIFLE 0 vs RIFLE R, I and F; RIFLE 0 vs RIFLE R; RIFLE 0 and R vs RIFLE I and F; etc.). The time "prior max stage" represents the time at which a sample is collected, relative to the time a particular patient reaches the lowest disease stage as defined for that cohort, binned into three groups which are +/- 12 hours. For example, "24 hr prior" which uses 0 vs R, I, F as the two cohorts would mean 24 hr (+/- 12 hours) prior to reaching stage R (or I if no sample at R, or F if no sample at R or I).

A receiver operating characteristic (ROC) curve was generated for each biomarker measured and the area under each ROC curve (AUC) is determined. Patients in Cohort 2 were also separated according to the reason for adjudication to cohort 2 as being based on serum creatinine measurements (sCr), being based on urine output (UO), or being based on either serum creatinine measurements or urine output. Using the same example discussed above (0 vs R, I, F), for those patients adjudicated to stage R, I, or F on the basis of serum creatinine measurements alone, the stage 0 cohort may include patients adjudicated to stage R, I, or F on the basis of urine output; for those patients adjudicated to stage R, I, or F on the basis of urine output alone, the stage 0 cohort may include patients adjudicated to stage R, I, or F on the basis of serum creatinine measurements; and for those patients adjudicated to stage R, I, or F on the basis of serum creatinine measurements or urine output, the stage 0 cohort contains only patients in stage 0 for both serum creatinine measurements and urine output. Also, in the data for patients adjudicated on the basis of serum creatinine measurements or urine output, the adjudication method which yielded the most severe RIFLE stage is used.

The ability to distinguish cohort 1 from Cohort 2 was determined using ROC analysis. SE is the standard error of the AUC, n is the number of sample or individual patients ("pts," as indicated). Standard errors are calculated as described in Hanley, J. A., and McNeil, B.J., The meaning and use of the area under a receiver operating characteristic (ROC) curve. Radiology (1982) 143: 29-36; p values are calculated with a two-tailed Z-test. An AUC < 0.5 is indicative of a negative going marker for the comparison, and an AUC > 0.5 is indicative of a positive going marker for the comparison.

Various threshold (or "cutoff') concentrations were selected, and the associated sensitivity and specificity for distinguishing cohort 1 from cohort 2 are determined. OR is the odds ratio calculated for the particular cutoff concentration, and 95% CI is the confidence interval for the odds ratio.

Fig. 1: Comparison of marker levels in urine samples collected from Cohort 1 (patients that did not progress beyond RIFLE stage 0) and in urine samples collected from subjects at 0, 24 hours, and 48 hours prior to reaching stage R, I or F in Cohort 2.

**Angiogenin**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 4070 | 8980 | 4070 | 7030 | 4070 | 5170 |
| Average | 7410 | 12000 | 7410 | 9920 | 7410 | 7950 |
| Stdev | 7830 | 9230 | 7830 | 8050 | 7830 | 7320 |
| p(t-test) | | 2.1E-10 | | 0.0014 | | 0.65 |
| Min | 55.7 | 132 | 55.7 | 97.6 | 55.7 | 650 |
| Max | 30600 | 30600 | 30600 | 30600 | 30600 | 24000 |
| n (Samp) | 430 | 195 | 430 | 132 | 430 | 46 |
| n (Patient) | 203 | 195 | 203 | 132 | 203 | 46 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 5850 | 7630 | 5850 | 9740 | 5850 | 7540 |
| Average | 9700 | 11300 | 9700 | 11000 | 9700 | 10100 |
| Stdev | 8780 | 9140 | 8780 | 8280 | 8780 | 8050 |
| p(t-test) | | 0.19 | | 0.30 | | 0.76 |
| Min | 0.00873 | 677 | 0.00873 | 97.6 | 0.00873 | 426 |
| Max | 30600 | 30600 | 30600 | 30600 | 30600 | 30600 |
| n (Samp) | 1121 | 58 | 1121 | 51 | 1121 | 36 |
| n (Patient) | 395 | 58 | 395 | 51 | 395 | 36 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 4280 | 11300 | 4280 | 6950 | 4280 | 4040 |
| Average | 8070 | 12700 | 8070 | 9800 | 8070 | 8380 |
| Stdev | 8270 | 9210 | 8270 | 7750 | 8270 | 7700 |
| p(t-test) | | 6.8E-10 | | 0.033 | | 0.81 |
| Min | 55.7 | 132 | 55.7 | 311 | 55.7 | 650 |
| Max | 30600 | 30600 | 30600 | 30600 | 30600 | 22100 |
| n (Samp) | 506 | 182 | 506 | 129 | 506 | 43 |
| n (Patient) | 215 | 182 | 215 | 129 | 215 | 43 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.66 | 0.57 | 0.66 | 0.61 | 0.55 | 0.59 | 0.55 | 0.54 | 0.52 |
| SE | 0.024 | 0.040 | 0.025 | 0.029 | 0.042 | 0.029 | 0.046 | 0.050 | 0.046 |
| p | 1.7E-10 | 0.10 | 3.1E-10 | 2.5E-4 | 0.25 | 0.0013 | 0.32 | 0.39 | 0.60 |
| nCohort 1 | 430 | 1121 | 506 | 430 | 1121 | 506 | 430 | 1121 | 506 |
| nCohort 2 | 195 | 58 | 182 | 132 | 51 | 129 | 46 | 36 | 43 |
| Cutoff 1 | 4060 | 4060 | 4350 | 3490 | 4300 | 3700 | 2680 | 4330 | 2620 |
| Sens 1 | 70% | 71% | 70% | 70% | 71% | 71% | 72% | 72% | 72% |
| Spec 1 | 50% | 39% | 51% | 44% | 41% | 43% | 36% | 41% | 34% |
| Cutoff 2 | 2910 | 3420 | 3350 | 2480 | 2230 | 2770 | 2090 | 2850 | 1880 |
| Sens 2 | 80% | 81% | 80% | 80% | 80% | 81% | 80% | 81% | 81% |
| Spec 2 | 38% | 33% | 40% | 35% | 23% | 35% | 30% | 28% | 26% |
| Cutoff 3 | 1680 | 1810 | 1820 | 1390 | 1440 | 1840 | 1510 | 1680 | 1510 |
| Sens 3 | 90% | 91% | 90% | 90% | 90% | 91% | 91% | 92% | 91% |
| Spec 3 | 23% | 18% | 24% | 18% | 14% | 25% | 19% | 16% | 19% |
| Cutoff 4 | 8010 | 14500 | 9520 | 8010 | 14500 | 9520 | 8010 | 14500 | 9520 |
| Sens 4 | 53% | 36% | 53% | 45% | 39% | 41% | 37% | 25% | 33% |
| Spec 4 | 70% | 70% | 70% | 70% | 70% | 70% | 70% | 70% | 70% |
| Cutoff 5 | 14000 | 19700 | 16100 | 14000 | 19700 | 16100 | 14000 | 19700 | 16100 |
| Sens 5 | 42% | 29% | 42% | 35% | 16% | 28% | 24% | 22% | 26% |
| Spec 5 | 80% | 80% | 80% | 80% | 80% | 80% | 80% | 80% | 80% |
| Cutoff 6 | 20400 | 22700 | 20400 | 20400 | 22700 | 20400 | 20400 | 22700 | 20400 |
| Sens 6 | 16% | 12% | 18% | 8% | 4% | 7% | 4% | 8% | 2% |
| Spec 6 | 92% | 90% | 91% | 92% | 90% | 91% | 92% | 90% | 91% |
| OR Quart 2 | 1.8 | 1.8 | 1.8 | 1.7 | 0.72 | 2.3 | 2.3 | 1.2 | 2.3 |
| | 0.031 | 0.16 | 0.048 | 0.096 | 0.49 | 0.011 | 0.080 | 0.78 | 0.082 |

(continued)

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| p Value | 1.1 | 0.79 | 1.0 | 0.91 | 0.29 | 1.2 | 0.91 | 0.39 | 0.90 |
| 95% CI of OR Quart2 | 3.1 | 4.2 | 3.1 | 3.1 | 1.8 | 4.2 | 5.9 | 3.5 | 5.8 |
| OR Quart | 2.2 | 1.8 | 2.5 | 1.9 | 1.5 | 2.4 | 1.8 | 2.4 | 1.0 |
| 3 | 0.0041 | 0.16 | 0.0011 | 0.036 | 0.33 | 0.0054 | 0.24 | 0.077 | 1.0 |
| p Value | 1.3 | 0.79 | 1.4 | 1.0 | 0.68 | 1.3 | 0.68 | 0.91 | 0.34 |
| 95% CI of OR Quart3 | 3.7 | 4.2 | 4.4 | 3.5 | 3.2 | 4.5 | 4.7 | 6.3 | 2.9 |
| OR Quart | 4.5 | 1.9 | 4.9 | 3.0 | 1.5 | 3.0 | 1.8 | 1.5 | 2.1 |
| 4 | 1.5E-8 | 0.12 | 5.2E-9 | 2.6E-4 | 0.33 | 5.0E-4 | 0.24 | 0.44 | 0.12 |
| p Value | 2.7 | 0.85 | 2.9 | 1.7 | 0.68 | 1.6 | 0.68 | 0.53 | 0.82 |
| 95% CI of OR Quart4 | 7.5 | 4.4 | 8.3 | 5.4 | 3.2 | 5.5 | 4.7 | 4.3 | 5.4 |

**Angiopoietin-related protein 4**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 11.7 | 11.6 | 11.7 | 11.8 | 11.7 | 11.5 |
| Average | 38.9 | 38.7 | 38.9 | 65.8 | 38.9 | 55.3 |
| Stdev | 79.2 | 92.2 | 79.2 | 172 | 79.2 | 122 |
| p(t-test) |  | 0.98 |  | 0.025 |  | 0.37 |
| Min | 0.000949 | 0.000466 | 0.000949 | 0.000981 | 0.000949 | 0.633 |
| Max | 538 | 789 | 538 | 1370 | 538 | 453 |
| n (Samp) | 363 | 168 | 363 | 103 | 363 | 22 |
| n (Patient) | 151 | 168 | 151 | 103 | 151 | 22 |

| sCr only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 12.4 | 18.6 | 12.4 | 17.2 | 12.4 | 9.37 |
| Average | 44.1 | 50.5 | 44.1 | 79.9 | 44.1 | 63.1 |
| Stdev | 108 | 70.1 | 108 | 145 | 108 | 135 |
| p(t-test) |  | 0.68 |  | 0.046 |  | 0.39 |
| Min | 0.000734 | 0.577 | 0.000734 | 1.82 | 0.000734 | 2.38 |
| Max | 1370 | 276 | 1370 | 645 | 1370 | 453 |
| n (Samp) | 960 | 49 | 960 | 39 | 960 | 25 |

(continued)

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| n (Patient) | 317 | 49 | 317 | 39 | 317 | 25 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 12.3 | 11.2 | 12.3 | 12.0 | 12.3 | 11.5 |
| Average | 45.8 | 40.8 | 45.8 | 56.2 | 45.8 | 35.1 |
| Stdev | 91.7 | 100 | 91.7 | 164 | 91.7 | 78.1 |
| p(t-test) | | 0.57 | | 0.38 | | 0.58 |
| Min | 0.000466 | 0.000466 | 0.000466 | 0.000981 | 0.000466 | 0.633 |
| Max | 624 | 789 | 624 | 1370 | 624 | 388 |
| n (Samp) | 444 | 155 | 444 | 104 | 444 | 24 |
| n (Patient) | 172 | 155 | 172 | 104 | 172 | 24 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.51 | 0.58 | 0.48 | 0.50 | 0.58 | 0.47 | 0.47 | 0.46 | 0.46 |
| SE | 0.027 | 0.044 | 0.027 | 0.032 | 0.049 | 0.032 | 0.065 | 0.060 | 0.062 |
| p | 0.66 | 0.076 | 0.53 | 0.97 | 0.11 | 0.32 | 0.59 | 0.53 | 0.49 |
| nCohort 1 | 363 | 960 | 444 | 363 | 960 | 444 | 363 | 960 | 444 |
| nCohort 2 | 168 | 49 | 155 | 103 | 39 | 104 | 22 | 25 | 24 |
| Cutoff 1 Sens 1 Spec 1 | 7.08 | 10.1 | 6.96 | 5.73 | 10.2 | 5.39 | 5.10 | 7.26 | 5.20 |
| | 70% | 71% | 70% | 71% | 72% | 70% | 73% | 72% | 71% |
| | 32% | 42% | 29% | 26% | 42% | 23% | 25% | 30% | 23% |
| Cutoff 2 Sens 2 Spec 2 | 4.53 | 5.80 | 4.61 | 4.21 | 7.47 | 3.76 | 2.96 | 5.34 | 2.60 |
| | 80% | 82% | 80% | 81% | 82% | 81% | 82% | 80% | 83% |
| | 23% | 23% | 20% | 20% | 31% | 15% | 13% | 21% | 9% |
| Cutoff 3 Sens 3 Spec 3 | 2.96 | 3.15 | 3.08 | 2.53 | 3.85 | 2.52 | 1.52 | 4.48 | 1.52 |
| | 90% | 92% | 90% | 90% | 92% | 90% | 91% | 92% | 92% |
| | 13% | 10% | 11% | 10% | 14% | 9% | 5% | 18% | 4% |
| Cutoff 4 Sens 4 Spec 4 | 22.2 | 24.2 | 24.5 | 22.2 | 24.2 | 24.5 | 22.2 | 24.2 | 24.5 |
| | 32% | 45% | 26% | 33% | 31% | 27% | 27% | 16% | 33% |
| | 70% | 70% | 70% | 70% | 70% | 70% | 70% | 70% | 70% |
| Cutoff 5 Sens 5 Spec 5 | 36.8 | 42.2 | 44.4 | 36.8 | 42.2 | 44.4 | 36.8 | 42.2 | 44.4 |
| | 21% | 31% | 20% | 22% | 28% | 17% | 18% | 16% | 17% |
| | 80% | 80% | 80% | 80% | 80% | 80% | 80% | 80% | 80% |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Cutoff 6 Sens 6 Spec 6 | 96.1 | 96.1 | 107 | 96.1 | 96.1 | 107 | 96.1 | 96.1 | 107 |
| | 8% | 14% | 7% | 14% | 21% | 11% | 14% | 16% | 4% |
| | 90% | 90% | 90% | 90% | 90% | 90% | 90% | 90% | 90% |
| OR Quart2 p Value 95% CI of OR Quart2 | 1.3 | 0.90 | 1.2 | 0.85 | 1.5 | 1.2 | 1.0 | 1.5 | 0.24 |
| | 0.31 | 0.82 | 0.51 | 0.61 | 0.44 | 0.64 | 0.99 | 0.52 | 0.073 |
| | 0.78 | 0.36 | 0.71 | 0.46 | 0.53 | 0.63 | 0.28 | 0.42 | 0.049 |
| | 2.2 | 2.2 | 2.0 | 1.6 | 4.3 | 2.1 | 3.6 | 5.4 | 1.1 |
| OR Quart3 p Value 95% CI of OR Quart3 | 1.0 | 1.1 | 1.3 | 0.86 | 2.0 | 0.85 | 1.2 | 2.0 | 0.61 |
| | 0.92 | 0.82 | 0.29 | 0.63 | 0.16 | 0.62 | 0.74 | 0.25 | 0.40 |
| | 0.60 | 0.46 | 0.79 | 0.46 | 0.75 | 0.45 | 0.36 | 0.61 | 0.19 |
| | 1.7 | 2.6 | 2.2 | 1.6 | 5.5 | 1.6 | 4.2 | 6.9 | 1.9 |
| OR Quart4 p Value 95% CI of OR Quart4 | 1.3 | 2.0 | 1.1 | 1.0 | 2.0 | 1.4 | 1.2 | 1.8 | 1.1 |
| | 0.38 | 0.092 | 0.76 | 0.91 | 0.16 | 0.23 | 0.74 | 0.36 | 0.80 |
| | 0.75 | 0.89 | 0.64 | 0.57 | 0.75 | 0.79 | 0.36 | 0.51 | 0.42 |
| | 2.1 | 4.3 | 1.8 | 1.9 | 5.5 | 2.6 | 4.2 | 6.2 | 3.1 |

**Angiopoietin-related protein 6**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.105 | 0.0450 | 0.105 | 0.109 | 0.105 | 0.000350 |
| Average | 1.04 | 1.27 | 1.04 | 1.52 | 1.04 | 0.471 |
| Stdev | 2.57 | 7.27 | 2.57 | 3.99 | 2.57 | 1.02 |
| p(t-test) | | 0.60 | | 0.15 | | 0.33 |
| Min | 1.00E-4 | 1.00E-4 | 1.00E-4 | 0.000110 | 1.00E-4 | 0.000110 |
| Max | 25.9 | 89.0 | 25.9 | 22.4 | 25.9 | 4.24 |
| n (Samp) | 333 | 156 | 333 | 97 | 333 | 20 |
| n (Patient) | 141 | 156 | 141 | 97 | 141 | 20 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.0677 | 0.214 | 0.0677 | 0.0456 | 0.0677 | 0.000364 |
| Average | 1.10 | 0.704 | 1.10 | 3.20 | 1.10 | 0.501 |
| Stdev | 3.58 | 1.44 | 3.58 | 14.6 | 3.58 | 1.05 |
| p(t-test) | | 0.45 | | 0.0063 | | 0.42 |

(continued)

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Min | 1.00E-4 | 1.00E-4 | 1.00E-4 | 0.000120 | 1.00E-4 | 1.00E-4 |
| Max | 70.5 | 8.76 | 70.5 | 89.0 | 70.5 | 4.24 |
| n (Samp) | 864 | 47 | 864 | 37 | 864 | 23 |
| n (Patient) | 292 | 47 | 292 | 37 | 292 | 23 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.151 | 0.0456 | 0.151 | 0.0635 | 0.151 | 0.000566 |
| Average | 1.06 | 1.38 | 1.06 | 1.64 | 1.06 | 0.329 |
| Stdev | 2.44 | 7.63 | 2.44 | 4.46 | 2.44 | 0.615 |
| p(t-test) | | 0.45 | | 0.081 | | 0.17 |
| Min | 1.00E-4 | 1.00E-4 | 1.00E-4 | 0.000110 | 1.00E-4 | 0.000110 |
| Max | 25.9 | 89.0 | 25.9 | 22.5 | 25.9 | 2.35 |
| n (Samp) | 414 | 143 | 414 | 97 | 414 | 21 |
| n (Patient) | 162 | 143 | 162 | 97 | 162 | 21 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.46 | 0.55 | 0.45 | 0.49 | 0.51 | 0.48 | 0.37 | 0.41 | 0.37 |
| SE | 0.028 | 0.044 | 0.028 | 0.033 | 0.049 | 0.033 | 0.069 | 0.063 | 0.067 |
| p | 0.18 | 0.25 | 0.088 | 0.69 | 0.81 | 0.52 | 0.061 | 0.16 | 0.047 |
| nCohort 1 | 333 | 864 | 414 | 333 | 864 | 414 | 333 | 864 | 414 |
| nCohort 2 | 156 | 47 | 143 | 97 | 37 | 97 | 20 | 23 | 21 |
| Cutoff 1 | 0.000336 | 0.00640 | 0.000278 | 0.000336 | 0.000336 | 0.000336 | 0.000206 | 0.000244 | 0.000206 |
| Sens 1 | 72% | 70% | 74% | 73% | 70% | 73% | 75% | 78% | 76% |
| Spec 1 | 23% | 45% | 23% | 23% | 30% | 24% | 11% | 18% | 12% |
| Cutoff 2 | 0.000249 | 0.000469 | 0.000249 | 0.000249 | 0.000249 | 0.000249 | 0.000203 | 0.000213 | 0.000203 |
| Sens 2 | 83% | 81% | 80% | 81% | 81% | 84% | 80% | 83% | 81% |
| Spec 2 | 16% | 33% | 17% | 16% | 25% | 17% | 9% | 17% | 10% |
| Cutoff 3 | 0.000203 | 0.000244 | 0.000123 | 0.000123 | 0.000157 | 0.000203 | 0.000122 | 0.000122 | 0.000122 |
| Sens 3 | 90% | 91% | 94% | 93% | 92% | 91% | 90% | 96% | 90% |
| Spec 3 | 9% | 18% | 7% | 6% | 11% | 10% | 6% | 6% | 7% |
| Cutoff 4 | 0.610 | 0.548 | 0.708 | 0.610 | 0.548 | 0.708 | 0.610 | 0.548 | 0.708 |
| Sens 4 | 27% | 38% | 24% | 32% | 32% | 28% | 30% | 22% | 19% |
| Spec 4 | 70% | 70% | 70% | 70% | 70% | 70% | 70% | 70% | 70% |
| Cutoff 5 | 1.28 | 1.17 | 1.54 | 1.28 | 1.17 | 1.54 | 1.28 | 1.17 | 1.54 |
| Sens 5 | 15% | 17% | 13% | 20% | 24% | 15% | 10% | 17% | 5% |
| Spec 5 | 80% | 80% | 80% | 80% | 80% | 80% | 80% | 80% | 80% |
| Cutoff 6 | 2.70 | 2.51 | 2.99 | 2.70 | 2.51 | 2.99 | 2.70 | 2.51 | 2.99 |
| Sens 6 | 6% | 4% | 6% | 9% | 14% | 10% | 5% | 4% | 0% |
| Spec 6 | 90% | 90% | 90% | 90% | 90% | 90% | 90% | 90% | 90% |
| OR Quart | 1.1 | 1.4 | 1.3 | 1.3 | 1.9 | 1.3 | 1.4 | 0.59 | 2.0 |
| 2 | 0.86 | 0.49 | 0.31 | 0.40 | 0.18 | 0.41 | 0.69 | 0.48 | 0.42 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| p Value | 0.61 | 0.55 | 0.77 | 0.70 | 0.75 | 0.69 | 0.30 | 0.14 | 0.37 |
| 95% CI of OR Quart2 | 1.8 | 3.5 | 2.3 | 2.5 | 4.9 | 2.5 | 6.3 | 2.5 | 11 |
| OR Quart 3 | 1.3 | 2.1 | 1.3 | 0.94 | 0.85 | 1.4 | 1.0 | 1.6 | 3.7 |
| | 0.31 | 0.10 | 0.31 | 0.86 | 0.78 | 0.33 | 0.99 | 0.40 | 0.11 |
| p Value | 0.77 | 0.87 | 0.77 | 0.48 | 0.28 | 0.72 | 0.20 | 0.52 | 0.75 |
| 95% CI of OR Quart3 | 2.3 | 4.9 | 2.3 | 1.8 | 2.6 | 2.6 | 5.2 | 5.0 | 18 |
| OR Quart 4 | 1.4 | 1.5 | 1.7 | 1.3 | 1.6 | 1.4 | 3.7 | 1.4 | 4.3 |
| | 0.25 | 0.37 | 0.070 | 0.40 | 0.34 | 0.32 | 0.054 | 0.55 | 0.070 |
| p Value | 0.80 | 0.61 | 0.96 | 0.70 | 0.61 | 0.73 | 0.98 | 0.44 | 0.89 |
| 95% CI of OR Quart4 | 2.3 | 3.8 | 2.9 | 2.5 | 4.2 | 2.6 | 14 | 4.5 | 21 |

EP 3 828 544 A1

**Amphiregulin**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 20.6 | 27.6 | 20.6 | 32.6 | 20.6 | 36.8 |
| Average | 46.4 | 39.6 | 46.4 | 101 | 46.4 | 103 |
| Stdev | 99.6 | 48.0 | 99.6 | 289 | 99.6 | 271 |
| p(t-test) | | 0.45 | | 0.0066 | | 0.017 |
| Min | 0.00401 | 0.00389 | 0.00401 | 0.00401 | 0.00401 | 3.84 |
| Max | 1310 | 300 | 1310 | 2190 | 1310 | 1270 |
| n (Samp) | 268 | 138 | 268 | 104 | 268 | 35 |
| n (Patient) | 148 | 138 | 148 | 104 | 148 | 35 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 23.5 | 31.6 | 23.5 | 46.4 | 23.5 | 40.2 |
| Average | 60.4 | 54.7 | 60.4 | 109 | 60.4 | 40.4 |
| Stdev | 177 | 65.1 | 177 | 172 | 177 | 28.4 |
| p(t-test) | | 0.84 | | 0.10 | | 0.59 |
| Min | 0.00389 | 6.31 | 0.00389 | 6.70 | 0.00389 | 4.90 |
| Max | 2190 | 289 | 2190 | 987 | 2190 | 124 |
| n (Samp) | 663 | 38 | 663 | 37 | 663 | 23 |
| n (Patient) | 288 | 38 | 288 | 37 | 288 | 23 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 23.9 | 29.8 | 23.9 | 37.2 | 23.9 | 39.6 |
| Average | 43.7 | 42.7 | 43.7 | 108 | 43.7 | 134 |
| Stdev | 64.1 | 49.9 | 64.1 | 320 | 64.1 | 289 |
| p(t-test) | | 0.87 | | 8.3E-4 | | 6.8E-6 |
| Min | 0.00131 | 0.00389 | 0.00131 | 0.00401 | 0.00131 | 3.84 |
| Max | 480 | 300 | 480 | 2190 | 480 | 1270 |
| n (Samp) | 313 | 127 | 313 | 102 | 313 | 32 |
| n (Patient) | 152 | 127 | 152 | 102 | 152 | 32 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.54 | 0.58 | 0.54 | 0.60 | 0.68 | 0.60 | 0.57 | 0.59 | 0.60 |
| SE | 0.030 | 0.050 | 0.031 | 0.034 | 0.050 | 0.033 | 0.053 | 0.063 | 0.055 |
| p | 0.24 | 0.12 | 0.19 | 0.0031 | 3.0E-4 | 0.0032 | 0.18 | 0.17 | 0.069 |
| nCohort 1 | 268 | 663 | 313 | 268 | 663 | 313 | 268 | 663 | 313 |
| nCohort 2 | 138 | 38 | 127 | 104 | 37 | 102 | 35 | 23 | 32 |
| Cutoff 1 | 16.0 | 16.8 | 16.3 | 18.3 | 24.0 | 20.9 | 16.3 | 22.1 | 18.5 |
| Sens 1 | 70% | 71% | 70% | 70% | 70% | 71% | 71% | 74% | 72% |
| Spec 1 | 40% | 38% | 37% | 45% | 51% | 46% | 40% | 48% | 41% |
| Cutoff 2 | 11.2 | 12.1 | 13.2 | 14.2 | 16.8 | 15.1 | 9.15 | 9.15 | 12.7 |
| Sens 2 | 80% | 82% | 80% | 81% | 81% | 80% | 83% | 83% | 81% |
| Spec 2 | 26% | 26% | 29% | 35% | 38% | 35% | 19% | 18% | 27% |
| Cutoff 3 | 7.44 | 9.02 | 6.50 | 8.80 | 14.5 | 9.55 | 5.56 | 6.50 | 5.56 |
| Sens 3 | 91% | 92% | 91% | 90% | 92% | 90% | 91% | 91% | 91% |
| Spec 3 | 15% | 18% | 13% | 18% | 32% | 20% | 11% | 13% | 11% |
| Cutoff 4 | 41.2 | 43.2 | 43.8 | 41.2 | 43.2 | 43.8 | 41.2 | 43.2 | 43.8 |
| Sens 4 | 28% | 37% | 31% | 43% | 57% | 43% | 43% | 48% | 44% |
| Spec 4 | 70% | 70% | 70% | 70% | 70% | 70% | 70% | 70% | 70% |
| Cutoff 5 | 54.4 | 54.7 | 56.0 | 54.4 | 54.7 | 56.0 | 54.4 | 54.7 | 56.0 |
| Sens 5 | 18% | 29% | 20% | 29% | 46% | 27% | 20% | 22% | 25% |
| Spec 5 | 80% | 80% | 81% | 80% | 80% | 81% | 80% | 80% | 81% |
| Cutoff 6 | 97.1 | 92.1 | 92.1 | 97.1 | 92.1 | 92.1 | 97.1 | 92.1 | 92.1 |
| Sens 6 | 7% | 13% | 8% | 14% | 35% | 14% | 11% | 4% | 19% |
| Spec 6 | 90% | 90% | 90% | 90% | 90% | 90% | 90% | 90% | 90% |
| OR Quart 2 | 1.2 | 1.3 | 1.5 | 1.7 | 5.8 | 1.4 | 0.47 | 0.39 | 0.65 |
| | 0.57 | 0.61 | 0.22 | 0.12 | 0.024 | 0.31 | 0.23 | 0.27 | 0.52 |
| p Value | 0.65 | 0.47 | 0.80 | 0.87 | 1.3 | 0.72 | 0.13 | 0.075 | 0.18 |
| 95% CI of OR Quart2 | 2.2 | 3.6 | 2.7 | 3.5 | 27 | 2.9 | 1.6 | 2.0 | 2.4 |
| OR Quart 3 | 2.1 | 1.5 | 2.0 | 1.9 | 3.1 | 1.8 | 1.7 | 1.6 | 2.2 |
| | 0.013 | 0.46 | 0.026 | 0.062 | 0.17 | 0.099 | 0.26 | 0.40 | 0.14 |
| p Value | 1.2 | 0.54 | 1.1 | 0.97 | 0.61 | 0.90 | 0.67 | 0.52 | 0.77 |
| 95% CI of OR Quart3 | 3.8 | 3.9 | 3.6 | 3.8 | 15 | 3.5 | 4.4 | 5.1 | 6.1 |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| OR Quart | 1.2 | 1.8 | 1.5 | 2.5 | 9.9 | 2.6 | 1.3 | 1.6 | 1.7 |
| 4 | 0.47 | 0.25 | 0.22 | 0.0091 | 0.0023 | 0.0052 | 0.64 | 0.41 | 0.31 |
| p Value | 0.68 | 0.67 | 0.80 | 1.3 | 2.3 | 1.3 | 0.47 | 0.52 | 0.60 |
| 95% CI of OR Quart4 | 2.3 | 4.6 | 2.7 | 4.8 | 43 | 5.0 | 3.4 | 5.1 | 5.0 |

**Betacellulin**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.752 | 0.909 | 0.752 | 1.07 | 0.752 | 0.909 |
| Average | 1.30 | 1.47 | 1.30 | 1.57 | 1.30 | 1.08 |
| Stdev | 2.01 | 1.98 | 2.01 | 2.12 | 2.01 | 1.06 |
| p(t-test) | | 0.42 | | 0.24 | | 0.53 |
| Min | 0.00179 | 0.00179 | 0.00179 | 0.00179 | 0.00179 | 0.00230 |
| Max | 23.1 | 14.9 | 23.1 | 11.6 | 23.1 | 4.42 |
| n (Samp) | 268 | 138 | 268 | 104 | 268 | 35 |
| n (Patient) | 148 | 138 | 148 | 104 | 148 | 35 |
| sCr only | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 0.761 | 1.65 | 0.761 | 1.62 | 0.761 | 1.70 |
| Average | 1.41 | 1.72 | 1.41 | 1.75 | 1.41 | 1.43 |
| Stdev | 2.42 | 1.77 | 2.42 | 1.92 | 2.42 | 1.33 |
| p(t-test) | | 0.44 | | 0.39 | | 0.97 |
| Min | 0.00179 | 0.00179 | 0.00179 | 0.00246 | 0.00179 | 0.00230 |
| Max | 28.3 | 6.72 | 28.3 | 8.64 | 28.3 | 4.42 |
| n (Samp) | 663 | 38 | 663 | 37 | 663 | 23 |
| n (Patient) | 288 | 38 | 288 | 37 | 288 | 23 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.354 | 1.11 | 0.354 | 1.40 | 0.354 | 1.11 |
| Average | 1.15 | 1.55 | 1.15 | 1.66 | 1.15 | 1.19 |
| Stdev | 1.55 | 1.99 | 1.55 | 2.03 | 1.55 | 0.989 |

(continued)

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| p(t-test) | | 0.022 | | 0.0078 | | 0.87 |
| Min | 0.00179 | 0.00179 | 0.00179 | 0.00179 | 0.00179 | 0.00240 |
| Max | 8.77 | 14.9 | 8.77 | 11.6 | 8.77 | 3.36 |
| n (Samp) | 313 | 127 | 313 | 102 | 313 | 32 |
| n (Patient) | 152 | 127 | 152 | 102 | 152 | 32 |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| AUC | 0.53 | 0.59 | 0.58 | 0.52 | 0.58 | 0.57 | 0.50 | 0.54 | 0.58 |
| SE | 0.030 | 0.050 | 0.031 | 0.034 | 0.050 | 0.033 | 0.052 | 0.063 | 0.055 |
| p | 0.31 | 0.064 | 0.012 | 0.61 | 0.11 | 0.051 | 0.93 | 0.47 | 0.12 |
| nCohort 1 | 268 | 663 | 313 | 268 | 663 | 313 | 268 | 663 | 313 |
| nCohort 2 | 138 | 38 | 127 | 104 | 37 | 102 | 35 | 23 | 32 |
| Cutoff 1 | 0.0522 | 0.108 | 0.0742 | 0.0305 | 0.354 | 0.0407 | 0.0435 | 0.0209 | 0.208 |
| Sens 1 | 71% | 71% | 72% | 71% | 70% | 71% | 77% | 74% | 72% |
| Spec 1 | 32% | 38% | 40% | 24% | 45% | 32% | 31% | 25% | 48% |
| Cutoff 2 | 0.00352 | 0.0378 | 0.00352 | 0.00342 | 0.0209 | 0.00342 | 0.0209 | 0.00342 | 0.0742 |
| Sens 2 | 81% | 84% | 81% | 83% | 81% | 81% | 80% | 83% | 81% |
| Spec 2 | 19% | 29% | 24% | 16% | 25% | 20% | 24% | 17% | 40% |
| Cutoff 3 | 0.00246 | 0.00342 | 0.00246 | 0.00230 | 0.00342 | 0.00230 | 0.00246 | 0.00230 | 0.00332 |
| Sens 3 | 92% | 92% | 93% | 93% | 92% | 92% | 91% | 96% | 91% |
| Spec 3 | 10% | 17% | 13% | 6% | 17% | 6% | 10% | 6% | 15% |
| Cutoff 4 | 1.74 | 1.76 | 1.58 | 1.74 | 1.76 | 1.58 | 1.74 | 1.76 | 1.58 |
| Sens 4 | 39% | 47% | 43% | 33% | 41% | 40% | 29% | 35% | 38% |
| Spec 4 | 72% | 70% | 70% | 72% | 70% | 70% | 72% | 70% | 70% |
| Cutoff 5 | 2.17 | 2.35 | 2.11 | 2.17 | 2.35 | 2.11 | 2.17 | 2.35 | 2.11 |
| Sens 5 | 27% | 29% | 31% | 20% | 22% | 29% | 11% | 30% | 22% |
| Spec 5 | 81% | 80% | 80% | 81% | 80% | 80% | 81% | 80% | 80% |
|  | 0hr prior to AKI stage | | | 24hr prior to AKI stage | | | 48hr prior to AKI stage | | |
|  | sCr or UO | sCr only | UO only | sCr or UO | sCr only | UO only | sCr or UO | sCr only | UO only |
| Cutoff 6 | 3.12 | 3.36 | 3.12 | 3.12 | 3.36 | 3.12 | 3.12 | 3.36 | 3.12 |
| Sens 6 | 14% | 16% | 14% | 16% | 14% | 19% | 3% | 4% | 3% |
| Spec 6 | 90% | 91% | 90% | 90% | 91% | 90% | 90% | 91% | 90% |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| OR Quart | 1.1 | 2.3 | 1.0 | 0.47 | 0.61 | 0.57 | 0.99 | 0.79 | 1.5 |
| 2 | 0.69 | 0.13 | 0.88 | 0.030 | 0.40 | 0.11 | 0.98 | 0.73 | 0.52 |
| p Value | 0.63 | 0.78 | 0.57 | 0.24 | 0.20 | 0.28 | 0.35 | 0.21 | 0.42 |
| 95% CI of OR Quart2 | 2.0 | 6.7 | 1.9 | 0.93 | 1.9 | 1.1 | 2.8 | 3.0 | 5.7 |
| OR Quart | 0.87 | 1.6 | 1.1 | 0.90 | 1.7 | 1.0 | 1.4 | 1.4 | 3.7 |
| 3 | 0.65 | 0.40 | 0.76 | 0.75 | 0.26 | 0.90 | 0.48 | 0.56 | 0.029 |
| p Value | 0.48 | 0.52 | 0.60 | 0.49 | 0.68 | 0.55 | 0.54 | 0.44 | 1.1 |
| 95% Clot OR Quart3 | 1.6 | 5.1 | 2.0 | 1.7 | 4.1 | 2.0 | 3.7 | 4.6 | 12 |
| OR Quart | 1.5 | 2.9 | 2.0 | 0.95 | 1.4 | 1.6 | 0.99 | 1.4 | 2.4 |
| 4 | 0.16 | 0.043 | 0.021 | 0.87 | 0.48 | 0.14 | 0.98 | 0.57 | 0.17 |
| p Value | 0.85 | 1.0 | 1.1 | 0.51 | 0.55 | 0.86 | 0.35 | 0.44 | 0.70 |
| 95% CI of OR Quart4 | 2.7 | 8.3 | 3.5 | 1.8 | 3.6 | 2.9 | 2.8 | 4.5 | 8.0 |

**Endostatin**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 5040 | 6160 | 5040 | 5290 | 5040 | 4110 |
| Average | 18600 | 14200 | 18600 | 14600 | 18600 | 12700 |
| Stdev | 34200 | 23500 | 34200 | 22100 | 34200 | 23300 |
| p(t-test) | | 0.11 | | 0.21 | | 0.26 |
| Min | 323 | 300 | 323 | 281 | 323 | 485 |
| Max | 238000 | 173000 | 238000 | 133000 | 238000 | 132000 |
| n (Samp) | 430 | 195 | 430 | 132 | 430 | 46 |
| n (Patient) | 203 | 195 | 203 | 132 | 203 | 46 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 5580 | 8680 | 5580 | 6800 | 5580 | 4300 |
| Average | 16400 | 28200 | 16400 | 19500 | 16400 | 18400 |
| Stdev | 28100 | 47400 | 28100 | 30200 | 28100 | 35800 |
| p(t-test) | | 0.0028 | | 0.43 | | 0.68 |
| Min | 0.0130 | 300 | 0.0130 | 281 | 0.0130 | 876 |
| Max | 238000 | 190000 | 238000 | 133000 | 238000 | 171000 |
| n (Samp) | 1121 | 58 | 1121 | 51 | 1121 | 36 |
| n (Patient) | 395 | 58 | 395 | 51 | 395 | 36 |
| UO only | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 5490 | 6080 | 5490 | 5320 | 5490 | 4170 |
| Average | 19900 | 14100 | 19900 | 13200 | 19900 | 15700 |
| Stdev | 34400 | 24200 | 34400 | 17500 | 34400 | 29600 |
| p(t-test) | | 0.036 | | 0.033 | | 0.44 |
| Min | 323 | 389 | 323 | 531 | 323 | 485 |
| Max | 238000 | 180000 | 238000 | 84400 | 238000 | 173000 |
| n (Samp) | 506 | 182 | 506 | 129 | 506 | 43 |
| n (Patient) | 215 | 182 | 215 | 129 | 215 | 43 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.53 | 0.57 | 0.52 | 0.51 | 0.53 | 0.51 | 0.46 | 0.47 | 0.48 |
| SE | 0.025 | 0.040 | 0.025 | 0.029 | 0.042 | 0.029 | 0.046 | 0.050 | 0.046 |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| P | 0.19 | 0.070 | 0.32 | 0.66 | 0.55 | 0.82 | 0.33 | 0.53 | 0.65 |
| nCohort 1 | 430 | 1121 | 506 | 430 | 1121 | 506 | 430 | 1121 | 506 |
| nCohort 2 | 195 | 58 | 182 | 132 | 51 | 129 | 46 | 36 | 43 |
| Cutoff 1 | 3560 | 4110 | 3670 | 3370 | 3090 | 3480 | 2670 | 3160 | 2970 |
| Sens 1 | 70% | 71% | 70% | 70% | 71% | 71% | 72% | 72% | 72% |
| Spec 1 | 37% | 39% | 37% | 36% | 28% | 36% | 28% | 29% | 31% |
| Cutoff 2 | 2780 | 2380 | 3060 | 2420 | 2420 | 2740 | 1870 | 2400 | 2440 |
| Sens 2 | 80% | 81% | 80% | 80% | 80% | 81% | 80% | 81% | 81% |
| Spec 2 | 30% | 19% | 32% | 25% | 20% | 30% | 17% | 20% | 25% |
| Cutoff 3 | 1810 | 1670 | 2040 | 1760 | 1150 | 1880 | 904 | 1340 | 1290 |
| Sens 3 | 90% | 91% | 90% | 90% | 90% | 91% | 91% | 92% | 91% |
| Spec 3 | 16% | 12% | 19% | 16% | 6% | 17% | 6% | 8% | 9% |
| Cutoff 4 | 11600 | 11400 | 13400 | 11600 | 11400 | 13400 | 11600 | 11400 | 13400 |
| Sens 4 | 29% | 41% | 25% | 29% | 33% | 28% | 22% | 22% | 26% |
| Spec 4 | 70% | 70% | 70% | 70% | 70% | 70% | 70% | 70% | 70% |
| Cutoff 5 | 24300 | 21900 | 28700 | 24300 | 21900 | 28700 | 24300 | 21900 | 28700 |
| Sens 5 | 14% | 29% | 12% | 20% | 24% | 14% | 15% | 22% | 16% |
| Spec 5 | 80% | 80% | 80% | 80% | 80% | 80% | 80% | 80% | 80% |
| Cutoff 6 | 51700 | 44600 | 58300 | 51700 | 44600 | 58300 | 51700 | 44600 | 58300 |
| Sens 6 | 6% | 17% | 4% | 10% | 14% | 5% | 7% | 11% | 7% |
| Spec 6 | 90% | 90% | 90% | 90% | 90% | 90% | 90% | 90% | 90% |
| OR Quart | 1.8 | 0.91 | 2.8 | 1.3 | 0.68 | 1.9 | 1.2 | 1.0 | 1.1 |
| 2 | 0.025 | 0.83 | 9.4E-5 | 0.41 | 0.39 | 0.021 | 0.64 | 0.99 | 0.80 |
| p Value | 1.1 | 0.40 | 1.7 | 0.72 | 0.29 | 1.1 | 0.50 | 0.37 | 0.44 |
| 95% CI of OR Quart2 | 2.9 | 2.1 | 4.7 | 2.2 | 1.6 | 3.4 | 3.1 | 2.7 | 2.9 |
| OR Quart | 2.0 | 1.3 | 2.7 | 1.2 | 1.0 | 1.7 | 1.8 | 1.4 | 1.8 |
| 3 | 0.0050 | 0.45 | 1.4E-4 | 0.47 | 1.0 | 0.070 | 0.20 | 0.48 | 0.20 |
| p Value | 1.2 | 0.63 | 1.6 | 0.70 | 0.46 | 0.96 | 0.74 | 0.55 | 0.74 |
| 95% CI of OR Quart3 | 3.3 | 2.9 | 4.6 | 2.2 | 2.2 | 3.0 | 4.2 | 3.5 | 4.2 |
| OR Quart | 1.4 | 1.6 | 1.5 | 1.2 | 1.2 | 1.1 | 1.2 | 1.1 | 1.0 |
| 4 | 0.21 | 0.20 | 0.13 | 0.49 | 0.57 | 0.66 | 0.64 | 0.80 | 0.99 |
| p Value | 0.83 | 0.77 | 0.88 | 0.69 | 0.59 | 0.63 | 0.50 | 0.43 | 0.39 |
| 95% CI ot OR Quart4 | 2.3 | 3.4 | 2.6 | 2.1 | 2.6 | 2.1 | 3.1 | 3.0 | 2.6 |

**Proepiregulin**

| sCr or UO | Ohr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 3.35 | 3.36 | 3.35 | 3.79 | 3.35 | 3.13 |
| Average | 4.65 | 5.50 | 4.65 | 6.97 | 4.65 | 6.32 |
| Stdev | 5.33 | 5.69 | 5.33 | 9.72 | 5.33 | 9.52 |
| p(t-test) | | 0.14 | | 0.0041 | | 0.13 |
| Min | 0.0801 | 0.0298 | 0.0801 | 0.0298 | 0.0801 | 0.376 |
| Max | 46.9 | 33.3 | 46.9 | 81.9 | 46.9 | 43.0 |
| n (Samp) | 264 | 133 | 264 | 99 | 264 | 33 |
| n (Patient) | 147 | 133 | 147 | 99 | 147 | 33 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 3.54 | 3.07 | 3.54 | 3.69 | 3.54 | 2.94 |
| Average | 6.54 | 4.01 | 6.54 | 5.23 | 6.54 | 5.23 |
| Stdev | 13.5 | 3.62 | 13.5 | 4.09 | 13.5 | 8.08 |
| p(t-test) | | 0.25 | | 0.56 | | 0.65 |
| Min | 0.000104 | 0.0298 | 0.000104 | 0.0298 | 0.000104 | 0.434 |
| Max | 279 | 16.5 | 279 | 15.8 | 279 | 37.5 |
| n (Samp) | 644 | 38 | 644 | 36 | 644 | 22 |
| n (Patient) | 285 | 38 | 285 | 36 | 285 | 22 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 3.36 | 3.82 | 3.36 | 3.88 | 3.36 | 3.13 |
| Average | 4.92 | 5.83 | 4.92 | 7.66 | 4.92 | 7.18 |
| Stdev | 6.15 | 5.78 | 6.15 | 11.2 | 6.15 | 9.67 |
| p(t-test) | | 0.16 | | 0.0024 | | 0.067 |
| Min | 0.000201 | 0.256 | 0.000201 | 0.235 | 0.000201 | 0.376 |
| Max | 49.4 | 33.3 | 49.4 | 81.9 | 49.4 | 43.0 |
| n (Samp) | 308 | 121 | 308 | 94 | 308 | 31 |
| n (Patient) | 151 | 121 | 151 | 94 | 151 | 31 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.55 | 0.44 | 0.57 | 0.57 | 0.53 | 0.57 | 0.51 | 0.44 | 0.52 |
| SE | 0.031 | 0.049 | 0.031 | 0.034 | 0.050 | 0.034 | 0.054 | 0.064 | 0.055 |
| p | 0.12 | 0.22 | 0.021 | 0.037 | 0.58 | 0.036 | 0.87 | 0.35 | 0.66 |
| nCohort 1 | 264 | 644 | 308 | 264 | 644 | 308 | 264 | 644 | 308 |
| nCohort 2 | 133 | 38 | 121 | 99 | 36 | 94 | 33 | 22 | 31 |
| Cutoff 1 | 1.95 | 1.66 | 2.23 | 2.10 | 2.43 | 2.12 | 1.02 | 1.56 | 1.21 |
| Sens 1 | 71% | 71% | 70% | 71% | 72% | 70% | 73% | 73% | 71% |
| Spec 1 | 33% | 25% | 34% | 35% | 37% | 33% | 18% | 24% | 20% |
| Cutoff 2 | 1.66 | 1.16 | 1.87 | 1.48 | 1.68 | 1.42 | 0.935 | 1.06 | 0.990 |
| Sens 2 | 80% | 82% | 80% | 81% | 81% | 81% | 82% | 82% | 81% |
| Spec 2 | 28% | 17% | 31% | 25% | 26% | 24% | 16% | 16% | 16% |
| Cutoff 3 | 1.06 | 0.477 | 1.36 | 0.733 | 0.899 | 0.733 | 0.717 | 0.795 | 0.795 |
| Sens 3 | 90% | 92% | 90% | 91% | 92% | 90% | 91% | 91% | 90% |
| Spec 3 | 19% | 6% | 22% | 12% | 12% | 12% | 11% | 11% | 13% |
| Cutoff 4 | 5.15 | 6.29 | 5.26 | 5.15 | 6.29 | 5.26 | 5.15 | 6.29 | 5.26 |
| Sens 4 | 38% | 21% | 40% | 44% | 36% | 45% | 36% | 14% | 42% |
| Spec 4 | 70% | 70% | 70% | 70% | 70% | 70% | 70% | 70% | 70% |
| Cutoff 5 | 6.60 | 9.07 | 6.57 | 6.60 | 9.07 | 6.57 | 6.60 | 9.07 | 6.57 |
| Sens 5 | 30% | 11% | 31% | 38% | 19% | 40% | 27% | 14% | 35% |
| Spec 5 | 80% | 80% | 80% | 80% | 80% | 80% | 80% | 80% | 80% |
| Cutoff 6 | 10.5 | 13.6 | 10.6 | 10.5 | 13.6 | 10.6 | 10.5 | 13.6 | 10.6 |
| Sens 6 | 14% | 3% | 16% | 21% | 6% | 22% | 18% | 9% | 23% |
| Spec 6 | 90% | 90% | 90% | 90% | 90% | 90% | 90% | 90% | 90% |
| OR Quart 2 | 1.9 | 1.2 | 2.4 | 1.1 | 1.5 | 0.99 | 0.51 | 2.4 | 0.56 |
| | 0.036 | 0.78 | 0.0063 | 0.77 | 0.46 | 0.97 | 0.20 | 0.21 | 0.29 |
| p Value | 1.0 | 0.41 | 1.3 | 0.57 | 0.54 | 0.50 | 0.18 | 0.61 | 0.19 |
| 95% CI of OR Quart2 | 3.5 | 3.3 | 4.5 | 2.2 | 3.9 | 1.9 | 1.4 | 9.5 | 1.6 |
| OR Quart 3 | 0.95 | 1.8 | 1.3 | 0.61 | 1.6 | 0.61 | 0.51 | 1.7 | 0.27 |
| | 0.87 | 0.24 | 0.50 | 0.19 | 0.34 | 0.20 | 0.20 | 0.48 | 0.054 |
| p Value | 0.50 | 0.68 | 0.65 | 0.29 | 0.61 | 0.29 | 0.18 | 0.40 | 0.072 |
| 95% CI of OR Quart3 | 1.8 | 4.6 | 2.4 | 1.3 | 4.3 | 1.3 | 1.4 | 7.2 | 1.0 |

(continued)

| 0hr prior to AKI stage | | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| OR Quart | 2.0 | 1.6 | 2.5 | 2.2 | 1.1 | 2.3 | 0.88 | 2.4 | 1.2 |
| 4 | 0.020 | 0.33 | 0.0048 | 0.014 | 0.79 | 0.010 | 0.79 | 0.21 | 0.67 |
| p Value | 1.1 | 0.61 | 1.3 | 1.2 | 0.41 | 1.2 | 0.35 | 0.61 | 0.49 |
| 95% CI of OR Quart4 | 3.7 | 4.3 | 4.6 | 4.2 | 3.2 | 4.2 | 2.2 | 9.5 | 3.0 |

**Heparin-binding growth factor 1**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 876 | 759 | 876 | 657 | 876 | 572 |
| Average | 1250 | 1120 | 1250 | 1250 | 1250 | 915 |
| Stdev | 1560 | 1350 | 1560 | 1570 | 1560 | 972 |
| p(t-test) | | 0.30 | | 0.98 | | 0.15 |
| Min | 0.609 | 12.9 | 0.609 | 8.16 | 0.609 | 0.779 |
| Max | 16700 | 13500 | 16700 | 7750 | 16700 | 3960 |
| n (Samp) | 430 | 195 | 430 | 132 | 430 | 46 |
| n (Patient) | 203 | 195 | 203 | 132 | 203 | 46 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 843 | 688 | 843 | 882 | 843 | 548 |
| Average | 1240 | 908 | 1240 | 917 | 1240 | 1000 |
| Stdev | 1480 | 889 | 1480 | 749 | | 1120 |
| p(t-test) | | 0.090 | | 0.12 | | 0.34 |
| Min | 0.00328 | 1.77 | 0.00328 | 29.5 | 0.00328 | 13.3 |
| Max | 16700 | 4350 | 16700 | 2920 | 16700 | 3960 |
| n (Samp) | 1121 | 58 | 1121 | 51 | 1121 | |
| n (Patient) | 395 | 58 | 395 | 51 | 395 | 36 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 858 | 772 | 858 | 658 | 858 | 595 |

(continued)

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Average | 1200 | 1170 | 1200 | 1270 | 1200 | 927 |
| Stdev | 1480 | 1490 | 1480 | 1580 | 1480 | 973 |
| p(t-test) | | 0.84 | | 0.65 | | 0.24 |
| Min | 0.609 | 12.9 | 0.609 | 8.16 | 0.609 | 0.779 |
| Max | 16700 | 13500 | 16700 | 7750 | 16700 | 3960 |
| n (Samp) | 506 | 182 | 506 | 129 | 506 | 43 |
| n (Patient) | 215 | 182 | 215 | 129 | 215 | 43 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | only | UO only | sCr or UO | only | UO only |
| AUC | 0.48 | 0.43 | 0.49 | 0.47 | 0.45 | 0.49 | 0.42 | 0.42 | 0.43 |
| SE | 0.025 | 0.040 | 0.025 | 0.029 | 0.042 | 0.029 | 0.046 | 0.050 | 0.047 |
| | sCr or UO | sCr only | UO only | sCr or UO | sCr only | UO only | sCr or UO | sCr only | UO only |
| P | 0.52 | 0.086 | 0.84 | 0.33 | 0.26 | 0.60 | 0.082 | 0.13 | 0.16 |
| nCohort 1 | 430 | 1121 | 506 | 430 | 1121 | 506 | 430 | 1121 | 506 |
| nCohort 2 | 195 | 58 | 182 | 132 | 51 | 129 | 46 | 36 | 43 |
| Cutoff 1 | 477 | 358 | 466 | 318 | 262 | 360 | 359 | 262' | 359 |
| Sens 1 | 70% | 71% | 70% | 70% | 71% | 71% | 72% | 72% | 72% |
| Spec 1 | 31% | 24% | 31% | 23% | 18% | 26% | 25% | 18% | 26% |
| Cutoff 2 | 358 | 231 | 289 | 231 | 113 | 260 | 202 | 167 | 211 |
| Sens 2 | 80% | 81% | 80% | 80% | 80% | 81% | 80% | 81% | 81% |
| Spec 2 | 25% | 15% | 21% | 17% | 8% | 20% | 16% | 12% | 16% |
| Cutoff 3 | 170 | 122 | 159 | 103 | 87.4 | 121 | 41.6 | 54.1 | 51.2 |
| Sens 3 | 90% | 91% | 90% | 90% | 90% | 91% | 91% | 92% | 91% |
| Spec 3 | 13% | 9% | 13% | 10% | 7% | 10% | 5% | 4% | 6% |
| Cutoff 4 | 1440 | 1420 | 1400 | 1440 | 1420 | 1400 | 1440 | 1420 | 1400 |
| Sens 4 | 25% | 21% | 26% | 30% | 27% | 29% | 22% | 31% | 21% |
| Spec 4 | 70% | 70% | 70% | 70% | 70% | 70% | 70% | 70% | 70% |
| Cutoff 5 | 1750 | 1800 | 1720 | 1750 | 1800 | 1720 | 1750 | 1800 | 1720 |
| Sens 5 | 15% | 12% | 16% | 23% | 10% | 24% | 17% | 19% | 14% |
| Spec 5 | 80% | 80% | 80% | 80% | 80% | 80% | 80% | 80% | 80% |
| Cutoff 6 | 2430 | 2550 | 2410 | 2430 | 2550 | 2410 | 2430 | 2550 | 2410 |
| Sens 6 | 9% | 9% | 10% | 11% | 4% | 12% | 9% | 11% | 9% |
| Spec 6 | 90% | 90% | 90% | 90% | 90% | 90% | 90% | 90% | 90% |
| OR Quart | 0.98 | 1.4 | 1.0 | 0.66 | 1.5 | 0.74 | 0.88 | 0.44 | 1.6 |

(continued)

| | sCr or UO | sCr only | UO only | sCr or UO | sCr only | UO only | sCr or UO | sCr only | UO only |
|---|---|---|---|---|---|---|---|---|---|
| 2 | 0.93 | 0.41 | 1.0 | 0.16 | 0.33 | 0.31 | 0.80 | 0.17 | 0.33 |
| p Value | 0.60 | 0.62 | 0.61 | 0.37 | 0.68 | 0.42 | 0.33 | 0.13 | 0.61 |
| 95% CI OR Quart2 | 1.6 | 3.3 | 1.6 | 1.2 | 3.2 | 1.3 | 2.4 | 1.4 | 4.3 |
| OR Quart 3 | 1.9 | 1.6 | 1.6 | 0.89 | 0.63 | 1.2 | 1.9 | 1.2 | 2.0 |
| | 0.0085 | 0.23 | 0.058 | 0.67 | 0.34 | 0.42 | 0.14 | 0.64 | 0.16 |
| p Value | 1.2 | 0.73 | 0.98 | 0.51 | 0.24 | 0.73 | 0.80 | 0.50 | 0.76 |
| 95% CI of OR Quart3 | 3.0 | 3.7 | 2.5 | 1.5 | 1.6 | 2.1 | 4.5 | 3.0 | 5.1 |
| OR Quart 4 | 0.95 | 1.9 | 0.94 | 1.2 | 1.6 | 1.1 | 1.5 | 1.4 | 1.8 |
| | 0.83 | 0.12 | 0.80 | 0.48 | 0.25 | 0.76 | 0.37 | 0.50 | 0.23 |
| p Value | 0.58 | 0.84 | 0.57 | 0.71 | 0.73 | 0.63 | 0.62 | 0.56 | 0.69 |
| 95% Clot OR Quart4 | 1.6 | 4.1 | 1.5 | 2.1 | 3.4 | 1.9 | 3.7 | 3.3 | 4.7 |

**Fibroblast growth factor 19**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 2.96E-5 | 2.68E-5 | 2.96E-5 | 3.44E-5 | 2.96E-5 | 2.66E-5 |
| Average | 0.00439 | 0.00352 | 0.00439 | 0.00561 | 0.00439 | 0.00939 |
| Stdev | 0.0252 | 0.0302 | 0.0252 | 0.0463 | 0.0252 | 0.0411 |
| p(t-test) | | 0.73 | | 0.73 | | 0.39 |
| Min | 5.62E-6 | 5.62E-6 | 5.62E-6 | 5.62E-6 | 5.62E-6 | 1.34E-5 |
| Max | 0.260 | 0.388 | 0.260 | 0.470 | 0.260 | 0.193 |
| n (Samp) | 363 | 168 | 363 | 103 | 363 | 22 |
| n (Patient) | 151 | 168 | 151 | 103 | 151 | 22 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 2.83E-5 | 2.83E-5 | 2.83E-5 | 2.68E-5 | 2.83E-5 | 2.63E-5 |
| Average | 0.00440 | 0.00168 | 0.00440 | 0.0109 | 0.00440 | 0.000380 |
| Stdev | 0.0283 | 0.00444 | 0.0283 | 0.0620 | 0.0283 | 0.00166 |
| p(t-test) | | 0.50 | | 0.19 | | 0.48 |
| Min | 5.62E-6 | 5.62E-6 | 5.62E-6 | 5.62E-6 | 5.62E-6 | 5.62E-6 |
| Max | 0.470 | 0.0164 | 0.470 | 0.388 | 0.470 | 0.00836 |
| n (Samp) | 960 | 49 | 960 | 39 | 960 | 25 |

(continued)

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| n (Patient) | 317 | 49 | 317 | 39 | 317 | 25 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 2.83E-5 | 2.68E-5 | 2.83E-5 | 4.13E-5 | 2.83E-5 | 3.20E-5 |
| Average | 0.00568 | 0.00415 | 0.00568 | 0.00569 | 0.00568 | 0.0300 |
| Stdev | 0.0323 | 0.0320 | 0.0323 | 0.0461 | 0.0323 | 0.106 |
| p(t-test) | | 0.61 | | 1.00 | | 0.0033 |
| Min | 5.62E-6 | 5.62E-6 | 5.62E-6 | 5.62E-6 | 5.62E-6 | 1.34E-5 |
| Max | 0.359 | 0.388 | 0.359 | 0.470 | 0.359 | 0.492 |
| n (Samp) | 444 | 155 | 444 | 104 | 444 | 24 |
| n (Patient) | 172 | 155 | 172 | 104 | 172 | 24 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.47 | 0.51 | 0.48 | 0.53 | 0.50 | 0.56 | 0.45 | 0.45 | 0.55 |
| SE | 0.027 | 0.042 | 0.027 | 0.033 | 0.047 | 0.032 | 0.065 | 0.060 | 0.062 |
| P | 0.20 | 0.87 | 0.49 | 0.39 | 0.92 | 0.048 | 0.47 | 0.40 | 0.39 |
| nCohort 1 | 363 | 960 | 444 | 363 | 960 | 444 | 363 | 960 | 444 |
| nCohort 2 | 168 | 49 | 155 | 103 | 39 | 104 | 22 | 25 | 24 |
| Cutoff 1 | 2.17E-5 | 1.92E-5 | 2.17E-5 | 2.46E-5 | 1.92E-5 | 2.46E-5 | 1.88E-5 | 2.17E-5 | 2.17E-5 |
| Sens 1 | 71% | 76% | 72% | 72% | 74% | 71% | 77% | 76% | 71% |
| Spec 1 | 25% | 25% | 27% | 33% | 25% | 35% | 19% | 28% | 27% |
| Cutoff 2 | 1.82E-5 | 1.82E-5 | 1.82E-5 | 1.92E-5 | 1.82E-5 | 1.92E-5 | 1.82E-5 | 1.88E-5 | 1.88E-5 |
| Sens 2 | 86% | 86% | 85% | 81% | 87% | 81% | 86% | 80% | 83% |
| Spec 2 | 16% | 18% | 16% | 23% | 18% | 23% | 16% | 22% | 18% |
| Cutoff 3 | 1.34E-5 | 7.53E-6 | 1.34E-5 | 1.82E-5 | 0 | 1.82E-5 | 1.34E-5 | 5.62E-6 | 1.34E-5 |
| Sens 3 | 93% | 94% | 93% | 91% | 100% | 92% | 91% | 92% | 92% |
| Spec 3 | 10% | 5% | 10% | 16% | 0% | 16% | 10% | 3% | 10% |
| Cutoff 4 | 8.63E-5 | 4.39E-5 | 4.39E-5 | 8.63E-5 | 4.39E-5 | 4.39E-5 | 8.63E-5 | 4.39E-5 | 4.39E-5 |
| Sens 4 | 24% | 31% | 26% | 35% | 36% | 43% | 27% | 20% | 42% |
| Spec 4 | 71% | 72% | 70% | 71% | 72% | 70% | 71% | 72% | 70% |
| Cutoff 5 | 0.000168 | 0.000114 | 0.000114 | 0.000168 | 0.000114 | 0.000114 | 0.000168 | 0.000114 | 0.000114 |
| Sens 5 | 11% | 18% | 17% | 19% | 31% | 27% | 18% | 16% | 33% |
| Spec 5 | 81% | 81% | 80% | 81% | 81% | 80% | 81% | 81% | 80% |
| Cutoff 6 | 0.00442 | 0.00364 | 0.00364 | 0.00442 | 0.00364 | 0.00364 | 0.00442 | 0.00364 | 0.00364 |
| Sens 6 | 8% | 14% | 8% | 8% | 10% | 10% | 9% | 4% | 25% |
| Spec 6 | 90% | 91% | 90% | 90% | 91% | 90% | 90% | 91% | 90% |
| OR Quart | 0.80 | 1.2 | 0.90 | 0.89 | 1.00 | 0.76 | 0.49 | 0.80 | 0.56 |
| 2 | 0.42 | 0.69 | 0.68 | 0.72 | 0.99 | 0.41 | 0.32 | 0.74 | 0.36 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| p Value | 0.47 | 0.53 | 0.53 | 0.47 | 0.41 | 0.39 | 0.12 | 0.21 | 0.16 |
| 95% CI of OR Quart2 | 1.4 | 2.6 | 1.5 | 1.7 | 2.4 | 1.5 | 2.0. | 3.0 | 2.0 |
| OR Quart 3 | 1.3 | 1.0 | 1.2 | 1.2 | 0.49 | 1.5 | 0.83 | 2.1 | 0.56 |
| | 0.36 | 1.0 | 0.43 | 0.64 | 0.20 | 0.18 | 0.77 | 0.20 | 0.36 |
| p Value | 0.76 | 0.44 | 0.73 | 0.63 | 0.16 | 0.83 | 0.25 | 0.69 | 0.16 |
| 95% CI of OR Quart3 | 2.1 | 2.3 | 2.0 | 2.1 | 1.4 | 2.7 | 2.8 | 6.1 | 2.0 |
| OR Quart 4 | 1.1 | 0.91 | 1.2 | 1.1 | 1.4 | 1.2 | 1.4 | 1.2 | 1.3 |
| | 0.66 | 0.82 | 0.57 | 0.78 | 0.41 | 0.54 | 0.57 | 0.76 | 0.61 |
| p Value | 0.67 | 0.39 | 0.69 | 0.59 | 0.62 | 0.66 | 0.46 | 0.36 | 0.47 |
| 95% CI of OR Quart4 | 1.9 | 2.1 | 1.9 | 2.0 | 3.3 | 2.2 | 4.1 | 4.0 | 3.6 |

EP 3 828 544 A1

**Fibroblast growth factor 21**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.0153 | 0.0125 | 0.0153 | 0.0150 | 0.0153 | 0.00875 |
| Average | 0.113 | 0.157 | 0.113 | 0.196 | 0.113 | 0.234 |
| Stdev | 0.326 | 0.507 | 0.326 | 0.597 | 0.326 | 0.658 |
| p(t-test) | | 0.23 | | 0.064 | | 0.12 |
| Min | 1.14E-9 | 1.40E-9 | 1.14E-9 | 1.14E-9 | 1.14E-9 | 1.40E-9 |
| Max | 3.07 | 3.58 | 3.07 | 4.47 | 3.07 | 2.56 |
| n (Samp) | 363 | 168 | 363 | 103 | 363 | 22 |
| n (Patient) | 151 | 168 | 151 | 103 | 151 | 22 |
| sCr only | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 0.0141 | 0.0212 | 0.0141 | 0.0165 | 0.0141 | 0.00900 |
| Average | 0.160 | 0.225 | 0.160 | 0.237 | 0.160 | 0.218 |
| Stdev | 0.532 | 0.660 | 0.532 | 0.626 | 0.532 | 0.618 |
| p(t-test) | | 0.41 | | 0.38 | | 0.59 |
| Min | 1.14E-9 | 4.16E-6 | 1.14E-9 | 0.000163 | 1.14E-9 | 6.78E-7 |
| Max | 8.92 | 3.42 | 8.92 | 3.58 | 8.92 | 2.56 |
| n (Samp) | 960 | 49 | 960 | 39 | 960 | 25 |
| n (Patient) | 317 | 49 | 317 | 39 | 317 | 25 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.0157 | 0.0112 | 0.0157 | 0.0152 | 0.0157 | 0.00954 |
| Average | 0.132 | 0.159 | 0.132 | 0.183 | 0.132 | 0.271 |
| Stdev | 0.359 | 0.491 | 0.359 | 0.594 | 0.359 | 0.778 |
| p(t-test) | | 0.46 | | 0.26 | | 0.090 |
| Min | 1.14E-9 | 1.40E-9 | 1.14E-9 | 1.14E-9 | 1.14E-9 | 1.40E-9 |
| Max | 3.07 | 3.58 | 3.07 | 4.47 | 3.07 | 2.96 |
| n (Samp) | 444 | 155 | 444 | 104 | 444 | 24 |
| n (Patient) | 172 | 155 | 172 | 104 | 172 | 24 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.47 | 0.55 | 0.45 | 0.49 | 0.56 | 0.47 | 0.40 | 0.44 | 0.43 |
| SE | 0.027 | 0.043 | 0.027 | 0.032 | 0.049 | 0.032 | 0.066 | 0.060 | 0.062 |
| p | 0.33 | 0.26 | 0.083 | 0.76 | 0.18 | 0.32 | 0.11 | 0.30 | 0.23 |
| nCohort 1 | 363 | 960 | 444 | 363 | 960 | 444 | 363 | 960 | 444 |
| nCohort 2 | 168 | 49 | 155 | 103 | 39 | 104 | 22 | 25 | 24 |
| Cutoff 1 | 0.00513 | 0.00681 | 0.00479 | 0.00401 | 0.00811 | 0.00397 | 0.00605 | 0.00494 | 0.00605 |
| Sens 1 | 70% | 71% | 70% | 71% | 72% | 70% | 73% | 72% | 71% |
| Spec 1 | 26% | 32% | 23% | 22% | 35% | 21% | 29% | 25% | 28% |
| Cutoff 2 | 0.00273 | 0.00498 | 0.00273 | 0.00198 | 0.00544 | 0.00198 | 0.00115 | 0.00372 | 0.00115 |
| Sens 2 | 80% | 82% | 80% | 81% | 82% | 81% | 82% | 80% | 83% |
| Spec 2 | 17% | 25% | 15% | 12% | 27% | 11% | 8% | 20% | 8% |
| Cutoff 3 | 0.00105 | 0.00219 | 0.00105 | 0.000652 | 0.00108 | 0.000685 | 8.37E-7 | 0.000661 | 8.37E-7 |
| Sens 3 | 90% | 92% | 90% | 90% | 92% | 90% | 91% | 92% | 92% |
| Spec 3 | 8% | 13% | 7% | 7% | 7% | 6% | 2% | 6% | 2% |
| Cutoff 4 | 0.0369 | 0.0340 | 0.0438 | 0.0369 | 0.0340 | 0.0438 | 0.0369 | 0.0340 | 0.0438 |
| Sens 4 | 27% | 37% | 25% | 34% | 44% | 28% | 18% | 24% | 25% |
| Spec 4 | 70% | 70% | 70% | 70% | 70% | 70% | 70% | 70% | 70% |
| Cutoff 5 | 0.0727 | 0.0744 | 0.110 | 0.0727 | 0.0744 | 0.110 | 0.0727 | 0.0744 | 0.110 |
| Sens 5 | 18% | 24% | 17% | 25% | 31% | 18% | 18% | 20% | 17% |
| Spec 5 | 80% | 80% | 80% | 80% | 80% | 80% | 80% | 80% | 80% |
| | sCr or UO | sCr only | UO only | sCr or | UO sCr only | UO only | sCr or UO | sCr only | UO only |
| Cutoff 6 | 0.291 | 0.361 | 0.347 | 0.291 | 0.361 | 0.347 | 0.291 | 0.361 | 0.347 |
| Sens 6 | 12% | 12% | 10% | 16% | 15% | 12% | 14% | 12% | 12% |
| Spec 6 | 90% | 90% | 90% | 90% | 90% | 90% | 90% | 90% | 90% |
| OR Quart | 0.83 | 1.1 | 0.93 | 0.71 | 1.4 | 0.95 | 0.24 | 0.60 | 0.32 |

(continued)

| | sCr or UO | sCr only | UO only | sCr or | UO sCr only | UO only | sCr or UO | sCr only | UO only |
|---|---|---|---|---|---|---|---|---|---|
| 2 | 0.50 | 0.82 | 0.78 | 0.28 | 0.47 | 0.88 | 0.21 | 0.48 | 0.17 |
| p Value | 0.49 | 0.46 | 0.54 | 0.38 | 0.54 | 0.52 | 0.027 | 0.14 | 0.064 |
| 95% CI of OR Quart2 | 1.4 | 2.6 | 1.6 | 1.3 | 3.8 | 1.8 | 2.2 | 2.5 | 1.6 |
| OR Quart | 1.2 | 1.3 | 1.2 | 0.59 | 1.00 | 0.65 | 3.0 | 2.1 | 1.5 |
| 3 | 0.51 | 0.52 | 0.59 | 0.11 | 0.99 | 0.19 | 0.068 | 0.20 | 0.43 |
| p Value | 0.71 | 0.57 | 0.68 | 0.31 | 0.34 | 0.34 | 0.92 | 0.69 | 0.53 |
| 95% CI of OR Quart3 | 2.0 | 3.1 | 2.0 | 1.1 | 2.9 | 1.2 | 9.8 | 6.1 | 4.5 |
| OR Quart | 1.2 | 1.5 | 1.6 | 1.2 | 2.2 | 1.5 | 1.6 | 1.4 | 1.2 |
| 4 | 0.57 | 0.31 | 0.087 | 0.53 | 0.090 | 0.19 | 0.51 | 0.56 | 0.78 |
| p Value | 0.69 | 0.67 | 0.94 | 0.67 | 0.88 | 0.82 | 0.42 | 0.44 | 0.38 |
| 95% CI of OR Quart4 | 1.9 | 3.5 | 2.6 | 2.2 | 5.5 | 2.6 | 5.7 | 4.5 | 3.6 |

**Heparin-binding EGF-like growth factor**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 134 | 130 | 134 | 159 | 134 | 141 |
| Average | 140 | 141 | 140 | 160 | 140 | 159 |
| Stdev | 52.5 | 60.1 | 52.5 | 71.7 | 52.5 | 64.0 |
| p(t-test) | | 0.81 | | 0.0056 | | 0.061 |
| Min | 31.0 | 40.9 | 31.0 | 52.1 | 31.0 | 67.2 |
| Max | 311 | 360 | 311 | 444 | 311 | 312 |
| n (Samp) | 250 | 131 | 250 | 95 | 250 | 32 |
| n (Patient) | 145 | 131 | 145 | 95 | 145 | 32 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 132 | 126 | 132 | 164 | 132 | 137 |
| Average | 142 | 137 | 142 | 155 | 142 | 142 |
| Stdev | 60.2 | 51.4 | 60.2 | 57.1 | 60.2 | 53.3 |
| p(t-test) | | 0.67 | | 0.19 | | 1.00 |
| Min | 31.0 | 47.9 | 31.0 | 55.2 | 31.0 | 64.6 |
| Max | 444 | 249 | 444 | 287 | 444 | 241 |
| n (Samp) | 619 | 37 | 619 | 35 | 619 | 21 |
| n (Patient) | 284 | 37 | 284 | 35 | 284 | 21 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 130 | 140 | 130 | 162 | 130 | 162 |
| Average | 136 | 146 | 136 | 162 | 136 | 168 |
| Stdev | 51.8 | 62.2 | 51.8 | 72.8 | 51.8 | 65.3 |
| p(t-test) | | 0.11 | | 2.7E-4 | | 0.0028 |
| Min | 31.0 | 40.9 | 31.0 | 52.1 | 31.0 | 67.2 |
| Max | 311 | 360 | 311 | 444 | 311 | 312 |
| n (Samp) | 291 | 119 | 291 | 91 | 291 | 29 |
| n (Patient) | 149 | 119 | 149 | 91 | 149 | 29 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.49 | 0.49 | 0.53 | 0.57 | 0.58 | 0.60 | 0.58 | 0.51 | 0.64 |
| SE | 0.031 | 0.049 | 0.032 | 0.035 | 0.052 | 0.035 | 0.056 | 0.065 | 0.058 |
| p | 0.75 | 0.81 | 0.29 | 0.048 | 0.13 | 0.0065 | 0.17 | 0.84 | 0.015 |
| nCohort 1 | 250 | 619 | 291 | 250 | 619 | 291 | 250 | 619 | 291 |
| nCohort 2 | 131 | 37 | 119 | 95 | 35 | 91 | 32 | 21 | 29 |
| Cutoff 1 | 105 | 109 | 105 | 107 | 116 | 111 | 122 | 99.6 | 131 |
| Sens 1 | 70% | 70% | 71% | 71% | 71% | 70% | 72% | 71% | 72% |
| Spec 1 | 28% | 35% | 33% | 30% | 39% | 36% | 40% | 29% | 52% |
| Cutoff 2 | 86.4 | 95.7 | 85.9 | 88.0 | 99.6 | 87.8 | 97.1 | 97.5 | 90.2 |
| Sens 2 | 80% | 81% | 81% | 80% | 80% | 80% | 81% | 81% | 83% |
| Spec 2 | 16% | 26% | 17% | 18% | 29% | 19% | 23% | 28% | 21% |
| Cutoff 3 | 76.2 | 76.7 | 76.5 | 74.7 | 80.9 | 74.0 | 88.0 | 76.2 | 77.4 |
| Sens 3 | 90% | 92% | 91% | 91% | 91% | 90% | 91% | 90% | 93% |
| Spec 3 | 9% | 12% | 10% | 8% | 15% | 8% | 18% | 11% | 11% |
| Cutoff 4 | 162 | 166 | 161 | 162 | 166 | 161 | 162 | 166 | 161 |
| Sens 4 | 32% | 24% | 36% | 48% | 49% | 51% | 41% | 38% | 52% |
| Spec 4 | 70% | 70% | 70% | 70% | 70% | 70% | 70% | 70% | 70% |
| Cutoff 5 | 183 | 190 | 180 | 183 | 190 | 180 | 183 | 190 | 180 |
| Sens 5 | 21% | 16% | 25% | 33% | 29% | 35% | 31% | 24% | 45% |
| Spec 5 | 80% | 80% | 80% | 80% | 80% | 80% | 80% | 80% | 80% |
| Cutoff 6 | 203 | 225 | 203 | 203 | 225 | 203 | 203 | 225 | 203 |
| Sens 6 | 14% | 11% | 16% | 24% | 9% | 26% | 25% | 5% | 31% |
| Spec 6 | 90% | 90% | 90% | 90% | 90% | 90% | 90% | 90% | 90% |
| OR Quart 2 | 0.66 | 1.3 | 0.77 | 0.45 | 0.99 | 0.60 | 0.59 | 1.5 | 0.48 |
| | 0.18 | 0.61 | 0.41 | 0.032 | 0.99 | 0.18 | 0.37 | 0.52 | 0.31 |
| p Value | 0.36 | 0.47 | 0.42 | 0.22 | 0.34 | 0.29 | 0.18 | 0.42 | 0.12 |
| 95% CI of OR Quart2 | 1.2 | 3.6 | 1.4 | 0.94 | 2.9 | 1.3 | 1.9 | 5.5 | 2.0 |
| OR Quart 3 | 0.85 | 2.1 | 0.70 | 0.84 | 1.2 | 1.0 | 0.73 | 1.3 | 1.0 |
| | 0.58 | 0.12 | 0.27 | 0.61 | 0.79 | 1.0 | 0.57 | 0.74 | 1.0 |
| p Value | 0.47 | 0.82 | 0.38 | 0.43 | 0.41 | 0.50 | 0.24 | 0.33 | 0.31 |
| 95% CI of OR Quart3 | 1.5 | 5.3 | 1.3 | 1.6 | 3.2 | 2.0 | 2.2 | 4.8 | 3.2 |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| OR Quart | 1.0 | 1.0 | 1.3 | 1.3 | 1.9 | 2.0 | 1.7 | 1.5 | 2.6 |
| 4 | 0.96 | 1.0 | 0.33 | 0.36 | 0.18 | 0.031 | 0.25 | 0.52 | 0.063 |
| p Value | 0.57 | 0.34 | 0.75 | 0.71 | 0.75 | 1.1 | 0.67 | 0.42 | 0.95 |
| 95% CI of OR Quart4 | 1.8 | 2.9 | 2.4 | 2.5 | 4.9 | 3.8 | 4.5 | 5.5 | 7.2 |

**Thrombospondin-2**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1150 | 1450 | 1150 | 1130 | 1150 | 927 |
| Average | 1960 | 2200 | 1960 | 2870 | 1960 | 1940 |
| Stdev | 2780 | 2360 | 2780 | 7500 | 2780 | 3240 |
| p(t-test) | | 0.29 | | 0.035 | | 0.97 |
| Min | 33.0 | 47.5 | 33.0 | 23.5 | 33.0 | 105 |
| Max | 33000 | 21300 | 33000 | 68100 | 33000 | 19700 |
| n (Samp) | 430 | 195 | 430 | 132 | 430 | 46 |
| n (Patient) | 203 | 195 | 203 | 132 | 203 | 46 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1380 | 1460 | 1380 | 1250 | 1380 | 906 |
| Average | 2390 | 2200 | 2390 | 1670 | 2390 | 1470 |
| Stdev | 3860 | 2030 | 3860 | 1630 | 3860 | 1630 |
| p(t-test) | | 0.70 | | 0.18 | | 0.16 |
| Min | 0.0376 | 47.5 | 0.0376 | 23.5 | 0.0376 | 160 |
| Max | 68100 | 8500 | 68100 | 8210 | 68100 | 7210 |
| n (Samp) | 1121 | 58 | 1121 | 51 | 1121 | 36 |
| n (Patient) | 395 | 58 | 395 | 51 | 395 | 36 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1250 | 1470 | 1250 | 1170 | 1250 | 1080 |

(continued)

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Average | 2040 | 2280 | 2040 | 3010 | 2040 | 2270 |
| Stdev | 2810 | 2400 | 2810 | 7590 | 2810 | 3520 |
| p(t-test) | | 0.29 | | 0.021 | | 0.61 |
| Min | 13.4 | 122 | 13.4 | 99.8 | 13.4 | 105 |
| Max | 33000 | 21300 | 33000 | 68100 | 33000 | 19700 |
| n (Samp) | 506 | 182 | 506 | 129 | 506 | 43 |
| n (Patient) | 215 | 182 | 215 | 129 | 215 | 43 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.56 | 0.53 | 0.56 | 0.51 | 0.46 | 0.51 | 0.46 | 0.40 | 0.48 |
| SE | 0.025 | 0.039 | 0.025 | 0.029 | 0.042 | 0.029 | 0.046 | 0.051 | 0.046 |
| p | 0.015 | 0.47 | 0.017 | 0.81 | 0.35 | 0.83 | 0.40 | 0.041 | 0.67 |
| nCohort 1 | 430 | 1121 | 506 | 430 | 1121 | 506 | 430 | 1121 | 506 |
| nCohort 2 | 195 | 58 | 182 | 132 | 51 | 129 | 46 | 36 | 43 |
| Cutoff 1 | 843 | 999 | 891 | 655 | 914 | 671 | 525 | 589 | 575 |
| Sens 1 | 70% | 71% | 70% | 70% | 71% | 71% | 72% | 72% | 72% |
| Spec 1 | 37% | 38% | 37% | 29% | 35% | 27% | 23% | 23% | 22% |
| Cutoff 2 | 557 | 546 | 619 | 489 | 435 | 502 | 450 | 409 | 450 |
| Sens 2 | 80% | 81% | 80% | 80% | 80% | 81% | 80% | 81% | 81% |
| Spec 2 | 23% | 21% | 25% | 21% | 16% | 20% | 20% | 16% | 19% |
| Cutoff 3 | 314 | 217 | 348 | 362 | 171 | 401 | 275 | 272 | 277 |
| Sens 3 | 90% | 91% | 90% | 90% | 92% | 91% | 91% | 92% | 91% |
| Spec 3 | 13% | 6% | 14% | 16% | 5% | 18% | 12% | 8% | 11% |
| Cutoff 4 | 1870 | 2320 | 2010 | 1870 | 2320 | 2010 | 1870 | 2320 | 2010 |
| Sens 4 | 42% | 33% | 40% | 34% | 16% | 33% | 28% | 17% | 33% |
| Spec 4 | 70% | 70% | 70% | 70% | 70% | 70% | 70% | 70% | 70% |
| Cutoff 5 | 2630 | 3200 | 2780 | 2630 | 3200 | 2780 | 2630 | 3200 | 2780 |
| Sens 5 | 28% | 24% | 29% | 23% | 14% | 24% | 17% | 14% | 19% |
| Spec 5 | 80% | 80% | 80% | 80% | 80% | 80% | 80% | 80% | 80% |
| Cutoff 6 | 4290 | 5100 | 4310 | 4290 | 5100 | 4310 | 4290 | 5100 | 4310 |
| Sens 6 | 15% | 9% | 15% | 12% | 6% | 15% | 9% | 6% | 12% |
| Spec 6 | 90% | 90% | 90% | 90% | 90% | 90% | 90% | 90% | 90% |

(continued)

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| OR Quart | 0.94 | 1.1 | 1.2 | 0.85 | 1.8 | 1.1 | 0.89 | 0.83 | 0.62 |
| 2 | 0.80 | 0.84 | 0.38 | 0.55 | 0.20 | 0.70 | 0.81 | 0.77 | 0.34 |
| p Value | 0.57 | 0.49 | 0.76 | 0.49 | 0.74 | 0.65 | 0.35 | 0.25 | 0.23 |
| 95% CI of OR Quart2 | 1.5 | 2.4 | 2.1 | 1.5 | 4.3 | 1.9 | 2.3 | 2.8 | 1.7 |
| OR Quart | 1.2 | 1.3 | 1.1 | 0.85 | 2.3 | 0.84 | 1.2 | 2.1 | 1.1 |
| 3 | 0.39 | 0.45 | 0.70 | 0.57 | 0.051 | 0.55 | 0.65 | 0.16 | 0.81 |
| p Value | 0.76 | 0.63 | 0.67 | 0.49 | 1.00 | 0.48 | 0.51 | 0.76 | 0.47 |
| 95% CI of OR Quart3 | 2.0 | 2.9 | 1.8 | 1.5 | 5.5 | 1.5 | 2.9 | 5.5 | 2.6 |
| OR Quart | 1.7 | 1.4 | 1.8 | 0.99 | 1.4 | 1.1 | 1.6 | 2.2 | 1.2 |
| 4 | 0.035 | 0.35 | 0.017 | 0.97 | 0.49 | 0.80 | 0.29 | 0.11 | 0.66 |
| p Value | 1.0 | 0.67 | 1.1 | 0.58 | 0.55 | 0.62 | 0.68 | 0.84 | 0.52 |
| 95% CI of OR Quart4 | 2.7 | 3.1 | 2.9 | 1.7 | 3.5 | 1.8 | 3.7 | 5.9 | 2.8 |

**Tenascin**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 9.36 | 10.6 | 9.36 | 10.9 | 9.36 | 16.5 |
| Average | 14.9 | 17.8 | 14.9 | 99.7 | 14.9 | 24.7 |
| Stdev | 34.0 | 22.7 | 34.0 | 743 | 34.0 | 53.5 |
| p(t-test) |  | 0.37 |  | 0.063 |  | 0.14 |
| Min | 0.00398 | 0.00398 | 0.00398 | 0.00398 | 0.00398 | 0.0184 |
| Max | 470 | 134 | 470 | 7540 | 470 | 317 |
| n (Samp) | 268 | 138 | 268 | 104 | 268 | 35 |
| n (Patient) | 148 | 138 | 148 | 104 | 148 | 35 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 10.7 | 6.88 | 10.7 | 11.2 | 10.7 | 4.09 |
| Average | 29.4 | 11.0 | 29.4 | 40.6 | 29.4 | 11.8 |
| Stdev | 295 | 12.7 | 295 | 154 | 295 | 13.6 |
| p(t-test) | | 0.70 | | 0.82 | | 0.77 |
| Min | 0.00398 | 0.0132 | 0.00398 | 0.00398 | 0.00398 | 0.0184 |
| Max | 7540 | 50.9 | 7540 | 945 | 7540 | 44.4 |
| n (Samp) | 663 | 38 | 663 | 37 | 663 | 23 |
| n (Patient) | 288 | 38 | 288 | 37 | 288 | 23 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 9.36 | 10.8 | 9.36 | 9.71 | 9.36 | 16.8 |
| Average | 17.9 | 19.0 | 17.9 | 93.1 | 17.9 | 31.5 |
| Stdev | 61.5 | 24.0 | 61.5 | 749 | 61.5 | 60.0 |
| p(t-test) | | 0.85 | | 0.079 | | 0.24 |
| Min | 0.00398 | 0.00398 | 0.00398 | 0.00398 | 0.00398 | 0.0184 |
| Max | 945 | 134 | 945 | 7540 | 945 | 317 |
| n (Samp) | 313 | 127 | 313 | 101 | 313 | 32 |
| n (Patient) | 152 | 127 | 152 | 101 | 152 | 32 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO sCr | only | UO only | sCr or UO | sCr only | UO only |
| AUC | 0.55 | 0.47 | 0.55 | 0.56 | 0.55 | 0.54 | 0.62 | 0.47 | 0.62 |
| SE | 0.030 | 0.049 | 0.031 | 0.034 | 0.050 | 0.033 | 0.053 | 0.062 | 0.055 |
| | sCr or UO | sCr only | UO only | sCr or UO | sCr only | UO only | sCr or UO | sCr only | UO only |
| p | 0.13 | 0.49 | 0.14 | 0.068 | 0.36 | 0.19 | 0.027 | 0.67 | 0.032 |
| nCohort 1 | 268 | 663 | 313 | 268 | 663 | 313 | 268 | 663 | 313 |
| nCohort 2 | 138 | 38 | 127 | 104 | 37 | 101 | 35 | 23 | 32 |
| Cutoff 1 | 0.533 | 0.338 | 0.338 | 1.39 | 0.338 | 0.338 | 0.452 | 0.178 | 3.21 |
| Sens 1 | 70% | 71% | 73% | 70% | 76% | 71% | 71 % | 78% | 72% |
| Spec 1 | 32% | 28% | 29% | 35% | 28% | 29% | 31% | 26% | 37% |
| Cutoff 2 | 0.0840 | 0.117 | 0.0748 | 0.103 | 0.315 | 0.0840 | 0.338 | 0.167 | 0.315 |
| Sens 2 | 80% | 82% | 80% | 83% | 84% | 80% | 80% | 83% | 84% |
| Spec 2 | 22% | 23% | 19% | 24% | 26% | 22% | 29% | 24% | 28% |
| Cutoff 3 | 0.0184 | 0.0187 | 0.0179 | 0.0187 | 0.177 | 0.0187 | 0.103 | 0.0179 | 0.0840 |

(continued)

| | sCr or UO | sCr only | UO only | sCr or UO | sCr only | UO only | sCr or UO | sCr only | UO only |
|---|---|---|---|---|---|---|---|---|---|
| Sens 3 | 91% | 97% | 91% | 91% | 92% | 91% | 91% | 100% | 91% |
| Spec 3 | 7% | 11% | 7% | 9% | 24% | 10% | 24% | 9% | 22% |
| Cutoff 4 | 15.6 | 18.2 | 16.5 | 15.6 | 18.2 | 16.5 | 15.6 | 18.2 | 16.5 |
| Sens 4 | 40% | 21% | 39% | 42% | 46% | 42% | 51% | 35% | 50% |
| Spec 4 | 70% | 70% | 71% | 70% | 70% | 71% | 70% | 70% | 71% |
| Cutoff 5 | 20.7 | 25.9 | 21.9 | 20.7 | 25.9 | 21.9 | 20.7 | 25.9 | 21.9 |
| Sens 5 | 31% | 11% | 32% | 31% | 27% | 30% | 34% | 17% | 28% |
| Spec 5 | 80% | 80% | 80% | 80% | 80% | 80% | 80% | 80% | 80% |
| Cutoff 6 | 30.7 | 39.8 | 31.2 | 30.7 | 39.8 | 31.2 | 30.7 | 39.8 | 31.2 |
| Sens 6 | 22% | 5% | 24% | 18% | 11% | 21% | 14% | 4% | 22% |
| Spec 6 | 90% | 90% | 90% | 90% | 90% | 90% | 90% | 90% | 90% |
| OR Quart 2 | 0.86 | 1.2 | 1.0 | 1.7 | 2.7 | 1.2 | 3.2 | 0.83 | 1.2 |
| | 0.62 | 0.78 | 1.0 | 0.100 | 0.062 | 0.65 | 0.089 | 0.77 | 0.76 |
| p Value | 0.47 | 0.41 | 0.55 | 0.90 | 0.95 | 0.61 | 0.84 | 0.25 | 0.36 |
| 95% CI of OR Quart2 | 1.6 | 3.3 | 1.8 | 3.4 | 7.8 | 2.2 | 12 | 2.8 | 4.1 |
| OR Quart 3 | 0.79 | 1.9 | 0.95 | 0.76 | 1.4 | 0.74 | 2.8 | 1.2 | 1.9 |
| | 0.44 | 0.17 | 0.88 | 0.46 | 0.56 | 0.38 | 0.14 | 0.78 | 0.27 |
| p Value | 0.43 | 0.75 | 0.52 | 0.37 | 0.44 | 0.37 | 0.72 | 0.39 | 0.61 |
| 95% Clot OR Quart3 | 1.4 | 5.0 | 1.7 | 1.6 | 4.6 | 1.5 | 11 | 3.6 | 5.9 |
| OR Quart 4 | 1.8 | 1.5 | 1.7 | 2.5 | 2.5 | 1.7 | 5.9 | 0.83 | 2.6 |
| | 0.052 | 0.45 | 0.084 | 0.0049 | 0.091 | 0.097 | 0.0068 | 0.77 | 0.087 |
| p Value | 1.00 | 0.54 | 0.94 | 1.3 | 0.86 | 0.91 | 1.6 | 0.25 | 0.87 |
| 95% CI of OR Quart4 | 3.1 | 3.9 | 2.9 | 4.8 | 7.3 | 3.1 | 21 | 2.8 | 7.7 |

Fig. 2: Comparison of marker levels in urine samples collected from Cohort 1 (patients that did not progress beyond RIFLE stage 0 or R) and in urine samples collected from subjects at 0,24 hours, and 48 hours prior to reaching stage I or F in Cohort 2.

Angiogenin

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 5340 | 10900 | 5340 | 9120 | 5340 | 5200 |
| Average | 8920 | 12500 | 8920 | 11000 | 8920 | 9060 |
| Stdev | 8290 | 8910 | 8290 | 8520 | 8290 | 8710 |
| p(t-test) | | 6.5E-5 | | 0.018 | | 0.90 |

(continued)

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Min | 3.00873 | 75.9 | 0.00873 | 303 | 0.00873 | 54.6 |
| Max | 30600 | 30600 | 30600 | 30600 | 30600 | 30600 |
| n (Samp) | 895 | 99 | 895 | 102 | 895 | 57 |
| n (Patient) | 374 | 99 | 374 | 102 | 374 | 57 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 6050 | 12100 | 6050 | 14400 | 6050 | 11200 |
| Average | 9720 | 12000 | 9720 | 13300 | 9720 | 11700 |
| Stdev | 8640 | 8770 | 8640 | 9680 | 8640 | 8390 |
| p(t-test) | | 0.24 | | 0.035 | | 0.25 |
| Min | 0.00873 | 110 | 0.00873 | 772 | 0.00873 | 174 |
| Max | 30600 | 27800 | 30600 | 30600 | 30600 | 30600 |
| n (Samp) | 1357 | 20 | 1357 | 26 | 1357 | 25 |
| n (Patient) | 470 | 20 | 470 | 26 | 470 | 25 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 5710 | 10900 | 5710 | 8180 | 5710 | 4400 |
| Average | 9470 | 12500 | 9470 | 10200 | 9470 | 8210 |
| Stdev | 8660 | 9100 | 8660 | 7920 | 8660 | 8360 |
| p(t-test) | | 0.0017 | | 0.46 | | 0.32 |
| Min | 0.00873 | 75.9 | 0.00873 | 303 | 0.00873 | 54.6 |
| Max | 30600 | 30600 | 30600 | 30600 | 30600 | 27300 |
| n (Samp) | 962 | 91 | 962 | 94 | 962 | 49 |
| n (Patient) | 367 | 91 | 367 | 94 | 367 | 49 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.62 | 0.57 | 0.60 | 0.59 | 0.60 | 0.55 | 0.50 | 0.58 | 0.45 |
| SE | 0.031 | 0.067 | 0.033 | 0.031 | 0.059 | 0.032 | 0.040 | 0.060 | 0.043 |
| p | 9.1E-5 | 0.28 | 0.0015 | 0.0051 | 0.078 | 0.12 | 0.95 | 0.19 | 0.28 |
| nCohort 1 | 895 | 1357 | 962 | 895 | 1357 | 962 | 895 | 1357 | 962 |
| nCohort 2 | 99 | 20 | 91 | 102 | 26 | 94 | 57 | 25 | 49 |
| Cutoff 1 | 5290 | 4780 | 4920 | 4180 | 4300 | 4180 | 2200 | 6020 | 2120 |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Sens 1 | 71% | 70% | 70% | 71% | 73% | 70% | 70% | 72% | 71% |
| Spec 1 | 49% | 43% | 46% | 43% | 40% | 42% | 24% | 50% | 22% |
| Cutoff 2 | 3820 | 3870 | 4020 | 2940 | 3510 | 2820 | 1690 | 3240 | 1610 |
| Sens 2 | 81% | 80% | 80% | 80% | 81% | 81% | 81% | 80% | 82% |
| Spec 2 | 39% | 36% | 39% | 31% | 33% | 28% | 18% | 31% | 16% |
| Cutoff 3 | 1110 | 1110 | 1440 | 2230 | 1440 | 2000 | 989 | 1680 | 989 |
| Sens 3 | 91% | 90% | 90% | 90% | 92% | 90% | 91% | 92% | 92% |
| Spec 3 | 12% | 10% | 15% | 24% | 13% | 21% | 10% | 16% | 9% |
| Cutoff 4 | 12000 | 14500 | 14000 | 12000 | 14500 | 14000 | 12000 | 14500 | 14000 |
| Sens 4 | 47% | 45% | 42% | 38% | 50% | 28% | 32% | 40% | 24% |
| Spec 4 | 70% | 70% | 70% | 70% | 70% | 70% | 70% | 70% | 70% |
| Cutoff 5 | 18100 | 19700 | 19100 | 18100 | 19700 | 19100 | 18100 | 19700 | 19100 |
| Sens 5 | 36% | 25% | 36% | 23% | 35% | 16% | 21% | 28% | 20% |
| Spec 5 | 80% | 80% | 80% | 80% | 80% | 80% | 80% | 80% | 80% |
| Cutoff 6 | 20400 | 22100 | 22200 | 20400 | 22100 | 22200 | 20400 | 22100 | 22200 |
| Sens 6 | 17% | 10% | 13% | 11% | 15% | 7% | 11% | 8% | 8% |
| Spec 6 | 91% | 90% | 90% | 91% | 90% | 90% | 91% | 90% | 90% |
| OR Quart | 1.1 | 0.75 | 1.2 | 2.3 | 1.00 | 1.5 | 0.86 | 1.00 | 0.72 |
| 2 | 0.87 | 0.70 | 0.59 | 0.017 | 1.00 | 0.25 | 0.70 | 1.00 | 0.48 |
| p Value | 0.52 | 0.17 | 0.60 | 1.2 | 0.29 | 0.76 | 0.40 | 0.25 | 0.28 |
| 95% CI of OR Quart2 | 2.2 | 3.4 | 2.5 | 4.6 | 3.5 | 2.9 | 1.8 | 4.0 | 1.8 |
| OR Quart | 1.8 | 1.3 | 1.7 | 2.4 | 1.2 | 2.1 | 0.72 | 2.5 | 1.2 |
| 3 | 0.082 | 0.74 | 0.14 | 0.012 | 0.77 | 0.017 | 0.42 | 0.12 | 0.68 |
| p Value | 0.93 | 0.33 | 0.85 | 1.2 | 0.36 | 1.1 | 0.32 | 0.79 | 0.52 |
| 95% CI of OR Quart3 | 3.4 | 4.7 | 3.2 | 4.7 | 4.0 | 4.0 | 1.6 | 8.2 | 2.7 |
| OR Quart | 2.7 | 2.0 | 2.4 | 2.7 | 2.0 | 1.5 | 1.2 | 1.8 | 1.6 |
| 4 | 0.0015 | 0.26 | 0.0057 | 0.0042 | 0.20 | 0.25 | 0.59 | 0.37 | 0.24 |
| p Value | 1.5 | 0.60 | 1.3 | 1.4 | 0.68 | 0.76 | 0.60 | 0.51 | 0.73 |
| 95% CI ot OR Quart4 | 5.0 | 6.8 | 4.6 | 5.2 | 6.0 | 2.9 | 2.5 | 6.1 | 3.5 |

Angiopoietin-related protein 4

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 11.6 | 14.5 | 11.6 | 14.1 | 11.6 | 9.57 |
| Average | 41.8 | 50.7 | 41.8 | 76.3 | 41.8 | 55.9 |
| Stdev | 94.7 | 102 | 94.7 | 179 | 94.7 | 149 |
| p(t-test) | | 0.43 | | 0.0045 | | 0.36 |
| Min | 0.000466 | 1.68 | 0.000466 | 1.61 | 0.000466 | 0.794 |
| Max | 789 | 647 | 789 | 1370 | 789 | 878 |
| n (Samp) | 754 | 78 | 754 | 86 | 754 | 42 |
| | Cohort 1 | Cohort 2 | Cohort 1 | Cohort 2 | Cohort 1 | cohort 2 |
| n (Patient) | 298 | 78 | 298 | 86 | 298 | 42 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 12.6 | 27.8 | 12.6 | 18.2 | 12.6 | 13.5 |
| Average | 45.4 | 116 | 45.4 | 86.3 | 45.4 | 103 |
| Stdev | 107 | 182 | 107 | 117 | 107 | 218 |
| p(t-test) | | 0.0067 | | 0.072 | | 0.028 |
| Min | 0.000466 | 3.69 | 0.000466 | 2.98 | 0.000466 | 2.01 |
| Max | 1370 | 647 | 1370 | 413 | 1370 | 878 |
| n (Samp) | 1164 | 18 | 1164 | 23 | 1164 | 18 |
| n (Patient) | 379 | 18 | 379 | 23 | 379 | 18 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 12.0 | 13.8 | 12.0 | 13.4 | 12.0 | 9.31 |
| Average | 48.1 | 35.6 | 48.1 | 70.6 | 48.1 | 38.0 |
| Stdev | 104 | 51.2 | 104 | 182 | 104 | 76.2 |
| p(t-test) | | 0.32 | | 0.089 | | 0.57 |
| Min | 0.000466 | 1.68 | 0.000466 | 0.000466 | 0.000466 | 0.794 |
| Max | 878 | 301 | 878 | 1370 | 878 | 400 |
| n (Samp) | 838 | 70 | 838 | 80 | 838 | 36 |
| n (Patient) | 306 | 70 | 306 | 80 | 306 | 36 |

| | | 0hr prior to AKI stage | | | 24hr prior to AKI stage | | | 48hr prior to AKI stage | | |
|---|---|---|---|---|---|---|---|---|---|---|
| | | sCr or UO | sCr only | UO only | sCr or UO | sCr only | UO only | sCr or UO | sCr only | UO only |
| AUC | | 0.57 | 0.67 | 0.53 | 0.56 | 0.65 | 0.52 | 0.50 | 0.54 | 0.47 |
| SE | | 0.035 | 0.071 | 0.036 | 0.034 | 0.063 | 0.034 | 0.046 | 0.070 | 0.050 |
| p | | 0.048 | 0.018 | 0.39 | 0.078 | 0.019 | 0.51 | 0.92 | 0.60 | 0.50 |
| nCohort 1 | | 754 | 1164 | 838 | 754 | 1164 | 838 | 754 | 1164 | 838 |
| nCohort 2 | | 78 | 18 | 70 | 86 | 23 | 80 | 42 | 18 | 36 |
| Cutoff 1 | | 8.30 | 14.8 | 8.19 | 7.57 | 14.0 | 7.57 | 7.08 | 7.57 | 6.86 |
| Sens 1 | | 71% | 72% | 70% | 71% | 74% | 70% | 71% | 72% | 72% |
| Spec 1 | | 38% | 55% | 36% | 35% | 53% | 34% | 32% | 31% | 31% |
| Cutoff 2 | | 6.34 | 7.15 | 5.86 | 5.80 | 10.8 | 5.34 | 5.18 | 5.18 | 4.93 |
| Sens 2 | | 81% | 83% | 80% | 80% | 83% | 80% | 81% | 83% | 81% |
| Spec 2 | | 29% | 29% | 25% | 26% | 43% | 23% | 24% | 21% | 21% |
| Cutoff 3 | | 3.72 | 4.54 | 3.66 | 3.39 | 6.96 | 3.28 | 3.66 | 2.62 | 3.49 |
| Sens 3 | | 91% | 94% | 90% | 91% | 91% | 90% | 90% | 94% | 92% |
| Spec 3 | | 15% | 18% | 14% | 14% | 28% | 12% | 15% | 8% | 13% |
| Cutoff 4 | | 22.1 | 24.4 | 23.7 | 22.1 | 24.4 | 23.7 | 22.1 | 24.4 | 23.7 |
| Sens 4 | | 38% | 56% | 33% | 35% | 39% | 32% | 26% | 33% | 28% |
| Spec 4 | | 70% | 70% | 70% | 70% | 70% | 70% | 70% | 70% | 70% |
| Cutoff 5 | | 37.2 | 44.0 | 43.1 | 37.2 | 44.0 | 43.1 | 37.2 | 44.0 | 43.1 |
| Sens 5 | | 31% | 39% | 27% | 30% | 39% | 26% | 21% | 28% | 22% |
| Spec 5 | | 80% | 80% | 80% | 80% | 80% | 80% | 80% | 80% | 80% |
| Cutoff 6 | | 93.7 | 99.5 | 133 | 93.7 | 99.5 | 133 | 93.7 | 99.5 | 133 |
| Sens 6 | | 14% | 28% | 7% | 19% | 30% | 15% | 12% | 28% | 8% |
| Spec 6 | | 90% | 90% | 90% | 90% | 90% | 90% | 90% | 90% | 90% |
| OR Quart 2 | | 1.5 | 1.00 | 1.4 | 1.3 | 2.0 | 0.94 | 0.79 | 1.2 | 0.55 |
| | | 0.29 | 1.00 | 0.37 | 0.50 | 0.42 | 0.85 | 0.63 | 0.74 | 0.29 |
| p Value | | 0.72 | 0.14 | 0.68 | 0.65 | 0.36 | 0.48 | 0.31 | 0.33 | 0.18 |
| 95% CI of OR Quart2 | | 3.0 | 7.1 | 2.8 | 2.5 | 11 | 1.8 | 2.0 | 4.7 | 1.7 |
| OR Quart 3 | | 1.4 | 3.6 | 1.1 | 1.3 | 4.1 | 1.1 | 1.3 | 0.75 | 1.4 |
| | | 0.37 | 0.12 | 0.85 | 0.50 | 0.077 | 0.87 | 0.52 | 0.70 | 0.50 |
| p Value | | 0.68 | 0.73 | 0.51 | 0.65 | 0.86 | 0.55 | 0.57 | 0.17 | 0.56 |
| 95% CI of OR Quart3 | | 2.9 | 17 | 2.3 | 2.5 | 19 | 2.0 | 3.1 | 3.4 | 3.3 |

(continued)

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| OR Quart | 1.9 | 3.5 | 1.6 | 1.7 | 4.6 | 1.2 | 1.1 | 1.5 | 1.1 |
| 4 | 0.068 | 0.12 | 0.17 | 0.11 | 0.052 | 0.53 | 0.82 | 0.53 | 0.81 |
| p Value | 0.95 | 0.73 | 0.81 | 0.88 | 0.98 | 0.65 | 0.46 | 0.42 | 0.45 |
| 95% CI of. OR Quart4 | 3.8 | 17 | 3.3 | 3.2 | 21 | 2.3 | 2.7 | 5.4 | 2.8 |

**Amphiregulin**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 23.2 | 27.7 | 23.2 | 35.1 | 23.2 | 31.2 |
| Average | 54.1 | 48.4 | 54.1 | 167 | 54.1 | 73.3 |
| Stdev | 141 | 61.9 | 141 | 527 | 141 | 176 |
| p(t-test) |  | 0.74 |  | 2.8E-5 |  | 0.42 |
| Min | 0.00401 | 1.75 | 0.00401 | 1.75 | 0.00401 | 0.00413 |
| Max | 1640 | 300 | 1640 | 3480 | 1640 | 1070 |
| n (Samp) | 597 | 69 | 597 | 76 | 597 | 38 |
| n (Patient) | 279 | 69 | 279 | 76 | 279 | 38 |

| sCr only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 24.7 | 64.2 | 24.7 | 42.6 | 24.7 | 66.6 |
| Average | 59.4 | 91.9 | 59.4 | 165 | 59.4 | 94.2 |
| Stdev | 165 | 88.9 | 165 | 403 | 165 | 80.8 |
| p(t-test) |  | 0.55 |  | 0.012 |  | 0.43 |
| Min | 0.00131 | 22.5 | 0.00131 | 6.70 | 0.00131 | 9.39 |
| Max | 2190 | 289 | 2190 | 1710 | 2190 | 246 |
| n (Samp) | 827 | 9 | 827 | 17 | 827 | 14 |
| n (Patient) | 352 | 9 | 352 | 17 | 352 | 14 |

| UO only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 24.6 | 27.7 | 24.6 | 33.2 | 24.6 | 26.6 |

(continued)

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Average | 51.6 | 44.1 | 51.6 | 150 | 51.6 | 121 |
| Stdev | 125 | 55.6 | 125 | 510 | 125 | 331 |
| p(t-test) | | 0.63 | | 1.2E-4 | | 0.0059 |
| Min | 0.00131 | 1.75 | 0.00131 | 1.75 | 0.00131 | 0.00413 |
| Max | 1640 | 300 | 1640 | 3480 | 1640 | 1710 |
| n (Samp) | 604 | 66 | 604 | 72 | 604 | 35 |
| n (Patient) | 263 | 66 | 263 | 72 | 263 | 35 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.54 | 0.76 | 0.51 | 0.60 | 0.69 | 0.58 | 0.54 | 0.74 | 0.53 |
| SE | 0.037 | 0.093 | 0.038 | 0.036 | 0.072 | 0.037 | 0.049 | 0.077 | 0.051 |
| p | 0.24 | 0.0047 | 0.69 | 0.0080 | 0.0072 | 0.037 | 0.42 | 0.0014 | 0.60 |
| nCohort 1 | 597 | 827 | 604 | 597 | 827 | 604 | 597 | 827 | 604 |
| nCohort 2 | 69 | 9 | 66 | 76 | 17 | 72 | 38 | 14 | 35 |
| Cutoff 1 | 17.1 | 45.7 | 15.5 | 20.9 | 35.3 | 20.6 | 15.1 | 40.4 | 15.1 |
| Sens 1 | 71% | 78% | 71% | 71% | 71% | 71% | 71% | 71% | 71% |
| Spec 1 | 38% | 71% | 33% | 46% | 63% | 44% | 34% | 67% | 32% |
| Cutoff 2 | 12.3 | 24.3 | 11.6 | 15.9 | 32.4 | 15.9 | 10.6 | 25.8 | 11.5 |
| Sens 2 | 81% | 89% | 80% | 80% | 82% | 81% | 82% | 86% | 80% |
| Spec 2 | 26% | 49% | 23% | 35% | 60% | 34% | 21% | 52% | 23% |
| Cutoff 3 | 6.31 | 22.3 | 6.31 | 4.81 | 14.6 | 4.81 | 5.07 | 25.0 | 5.07 |
| Sens 3 | 91% | 100% | 91% | 91% | 94% | 90% | 92% | 93% | 91% |
| Spec 3 | 12% | 46% | 12% | 9% | 31% | 9% | 9% | 51% | 9% |
| Cutoff 4 | 42.8 | 43.9 | 43.9 | 42.8 | 43.9 | 43.9 | 42.8 | 43.9 | 43.9 |
| Sens 4 | 32% | 78% | 29% | 39% | 47% | 36% | 42% | 57% | 37% |
| Spec 4 | 70% | 70% | 70% | 70% | 70% | 70% | 70% | 70% | 70% |
| Cutoff 5 | 53.8 | 57.2 | 55.2 | 53.8 | 57.2 | 55.2 | 53.8 | 57.2 | 55.2 |
| Sens 5 | 25% | 56% | 21% | 32% | 29% | 29% | 32% | 57% | 23% |
| Spec 5 | 80% | 80% | 80% | 80% | 80% | 80% | 80% | 80% | 80% |
| Cutoff 6 | 88.4 | 96.9 | 87.2 | 88.4 | 96.9 | 87.2 | 88.4 | 96.9 | 87.2 |
| Sens 6 | 13% | 22% | 12% | 18% | 24% | 17% | 13% | 36% | 14% |
| Spec 6 | 90% | 90% | 90% | 90% | 90% | 90% | 90% | 90% | 90% |

(continued)

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| OR Quart 2 | 1.1 | 2.0 | 1.1 | 1.1 | 1.0 | 1.1 | 0.99 | 0 | 0.88 |
|  | 0.85 | <0.57 | 0.86 | 0.84 | 1.0 | 0.84 | 0.99 | na | 0.79 |
| p Value | 0.49 | >0.18 | 0.50 | 0.49 | 0.062 | 0.49 | 0.38 | na | 0.33 |
| 95% CI of OR Quart2 | 2.4 | na | 2.3 | 2.4 | 16 | 2.4 | 2.6 | na | 2.3 |
| OR Quart 3 | 1.8 | >2.0 | 1.5 | 2.0 | 9.4 | 1.7 | 0.88 | 5.1 | 0.76 |
|  | 0.11 | <0.57 | 0.28 | 0.059 | 0.035 | 0.15 | 0.79 | 0.14 | 0.60 |
| p Value | 0.87 | >0.18 | 0.72 | 0.97 | 1.2 | 0.82 | 0.33 | 0.59 | 0.28 |
| 95% CI of OR Quart3 | 3.7 | na | 3.1 | 4.1 | 75 | 3.5 | 2.3 | 44 | 2.1 |
| OR Quart 4 | 1.6 | >5.1 | 1.2 | 2.1 | 6.1 | 2.0 | 1.4 | 8.2 | 1.2 |
|  | 0.21 | <0.14 | 0.58 | 0.044 | 0.094 | 0.059 | 0.51 | 0.048 | 0.66 |
| p Value | 0.77 | >0.59 | 0.59 | 1.0 | 0.73 | 0.97 | 0.55 | 1.0 | 0.50 |
| 95% CI of OR Quart4 | 3.3 | na | 2.6 | 4.2 | 52 | 4.0 | 3.3 | 66 | 3.1 |

**Betacellulin**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 0.793 | 1.51 | 0.793 | 1.46 | 0.793 | 1.46 |
| Average | 1.42 | 1.43 | 1.42 | 1.47 | 1.42 | 1.31 |
| Stdev | 2.26 | 1.58 | 2.26 | 1.91 | 2.26 | 1.22 |
| p(t-test) |  | 0.95 |  | 0.85 |  | 0.77 |
| Min | 0.00179 | 0.00179 | 0.00179 | 0.00179 | 0.00179 | 0.00179 |
| Max | 27.4 | 9.73 | 27.4 | 11.6 | 27.4 | 4.74 |
| n (Samp) | 597 | 69 | 597 | 76 | 597 | 38 |
| n (Patient) | 279 | 69 | 279 | 76 | 279 | 38 |

| sCr only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 0.761 | 2.17 | 0.761 | 1.56 | 0.761 | 1.72 |
| Average | 1.40 | 2.29 | 1.40 | 1.31 | 1.40 | 1.71 |
| Stdev | 2.30 | 1.19 | 2.30 | 1.13 | 2.30 | 1.74 |
| p(t-test) |  | 0.25 |  | 0.87 |  | 0.63 |
| Min | 0.00179 | 0.00342 | 0.00179 | 0.00352 | 0.00179 | 0.00246 |
| Max | 28.3 | 3.74 | 28.3 | 3.65 | 28.3 | 5.61 |
| n (Samp) | 827 | 9 | 827 | 17 | 827 | 14 |
| n (Patient) | 352 | 9 | 352 | 17 | 352 | 14 |

| UO only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 0.793 | 1.11 | 0.793 | 1.46 | 0.793 | 1.43 |
| Average | 1.39 | 1.31 | 1.39 | 1.55 | 1.39 | 1.26 |
| Stdev | 2.08 | 1.57 | 2.08 | 1.98 | 2.08 | 1.24 |
| p(t-test) |  | 0.76 |  | 0.54 |  | 0.71 |
| Min | 0.00179 | 0.00179 | 0.00179 | 0.00179 | 0.00179 | 0.00179 |
| Max | 27.4 | 9.73 | 27.4 | 11.6 | 27.4 | 4.74 |
| n (Samp) | 604 | 66 | 604 | 72 | 604 | 35 |
| n (Patient) | 263 | 66 | 263 | 72 | 263 | 35 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.52 | 0.73 | 0.50 | 0.51 | 0.55 | 0.52 | 0.53 | 0.55 | 0.52 |
| SE | 0.037 | 0.097 | 0.038 | 0.035 | 0.073 | 0.036 | 0.049 | 0.080 | 0.051 |
| p | 0.62 | 0.018 | 0.93 | 0.80 | 0.48 | 0.53 | 0.61 | 0.52 | 0.76 |
| nCohort 1 | 597 | 827 | 604 | 597 | 827 | 604 | 597 | 827 | 604 |
| nCohort 2 | 69 | 9 | 66 | 76 | 17 | 72 | 38 | 14 | 35 |
| Cutoff 1 | 0.0407 | 1.97 | 0.0305 | 0.0407 | 0.0522 | 0.0435 | 0.0742 | 0.0209 | 0.0522 |
| Sens 1 | 71% | 78% | 71% | 71% | 71% | 71% | 71% | 71% | 71% |
| Spec 1 | 27% | 73% | 25% | 27% | 33% | 31% | 33% | 25% | 32% |
| Cutoff 2 | 0.00332 | 1.10 | 0.00332 | 0.00342 | 0.0178 | 0.00342 | 0.00342 | 0.00332 | 0.00342 |
| Sens 2 | 83% | 89% | 82% | 86% | 82% | 85% | 87% | 86% | 86% |
| Spec 2 | 12% | 54% | 14% | 16% | 23% | 18% | 16% | 14% | 18% |
| Cutoff 3 | 0.00184 | 0.00332 | 0.00184 | 0.00246 | 0.00342 | 0.00246 | 0.00240 | 0.00240 | 0.00240 |
| Sens 3 | 94% | 100% | 94% | 93% | 100% | 93% | 95% | 100% | 94% |
| Spec 3 | 3% | 14% | 3% | 9% | 18% | 11% | 7% | 8% | 9% |
| Cutoff 4 | 1.87 | 1.87 | 1.87 | 1.87 | 1.87 | 1.87 | 1.87 | 1.87 | 1.87 |
| Sens 4 | 36% | 78% | 30% | 29% | 35% | 32% | 29% | 29% | 31% |
| Spec 4 | 71% | 71% | 71% | 71% | 71% | 71% | 71% | 71% | 71% |
| Cutoff 5 | 2.41 | 2.41 | 2.41 | 2.41 | 2.41 | 2.41 | 2.41 | 2.41 | 2.41 |
| Sens 5 | 26% | 44% | 23% | 21% | 18% | 24% | 21% | 29% | 20% |
| Spec 5 | 80% | 80% | 80% | 80% | 80% | 80% | 80% | 80% | 80% |
| Cutoff 6 | 3.36 | 3.36 | 3.36 | 3.36 | 3.36 | 3.36 | 3.36 | 3.36 | 3.36 |
| Sens 6 | 7% | 11% | 6% | 12% | 6% | 14% | 5% | 14% | 6% |
| Spec 6 | 91% | 91% | 90% | 91% | 91% | 90% | 91% | 91% | 90% |
| OR Quart | 0.55 | 0 | 0.83 | 0.69 | 0.50 | 1.0 | 0.99 | 0 | 0.99 |
| 2 | 0.13 | na | 0.61 | 0.29 | 0.42 | 1.0 | 0.99 | na | 0.99 |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| p Value | 0.25 | na | 0.41 | 0.34 | 0.090 | 0.49 | 0.36 | na | 0.36 |
| 95% CI of OR Quart2 | 1.2 | na | 1.7 | 1.4 | 2.7 | 2.0 | 2.7 | na | 2.7 |
| OR Quart 3 | 0.88 | 3.0 | 0.60 | 1.1 | 1.8 | 1.1 | 1.8 | 1.0 | 1.4 |
| | 0.72 | 0.34 | 0.19 | 0.87 | 0.37 | 0.72 | 0.20 | 1.0 | 0.49 |
| p Value | 0.44 | 0.31 | 0.28 | 0.56 | 0.51 | 0.57 | 0.74 | 0.29 | 0.55 |
| 95% CI of OR Quart3 | 1.8 | 29 | 1.3 | 2.0 | 6.2 | 2.3 | 4.4 | 3.5 | 3.6 |
| OR Quart 4 | 1.2 | 5.1 | 1.0 | 0.83 | 1.0 | 1.1 | 0.99 | 0.79 | 0.99 |
| | 0.63 | 0.14 | 0.98 | 0.60 | 1.0 | 0.72 | 0.99 | 0.73 | 0.99 |
| p Value | 0.61 | 0.59 | 0.51 | 0.43 | 0.25 | 0.57 | 0.36 | 0.21 | 0.36 |
| 95% CI of OR Quart4 | 2.3 | 44 | 2.0 | 1.6 | 4.1 | 2.3 | 2.7 | 3.0 | 2.7 |

**Endostatin**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 5250 | 6510 | 5250 | 6350 | 5250 | 4000 |
| Average | 17300 | 18400 | 17300 | 18000 | 17300 | 12000 |
| Stdev | 31000 | 35100 | 31000 | 27900 | 31000 | 22800 |
| p(t-test) | | 0.74 | | 0.83 | | 0.20 |
| Min | 0.0130 | 444 | 0.0130 | 957 | 0.0130 | 261 |
| Max | 238000 | 227000 | 238000 | 148000 | 238000 | 149000 |
| n (Samp) | 895 | 99 | 895 | 102 | 895 | 57 |
| n (Patient) | 374 | 99 | 374 | 102 | 374 | 57 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 5530 | 11500 | 5530 | 7760 | 5530 | 6440 |
| Average | 16600 | 39800 | 16600 | 22500 | 16600 | 22000 |
| Stdev | 28500 | 63800 | 28500 | 27900 | 28500 | 35100 |
| p(t-test) | | 4.4E-4 | | 0.30 | | 0.35 |
| Min | 3.0130 | 104 | 0.0130 | 930 | 0.0130 | 876 |
| Max | 238000 | 227000 | 238000 | 110000 | 238000 | 149000 |
| n (Samp) | 1357 | 20 | 1357 | 26 | 1357 | 25 |
| n (Patient) | 470 | 20 | 470 | 26 | 470 | 25 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 5760 | 6270 | 5760 | 6310 | 5760 | 4060 |
| Average | 18700 | 15800 | 18700 | 16800 | 18700 | 10300 |
| Stdev | 32100 | 28700 | 32100 | 28100 | 32100 | 14800 |
| p(t-test) | | 0.41 | | 0.57 | | 0.066 |
| Min | 0.0130 | 444 | 0.0130 | 957 | 0.0130 | 261 |
| Max | 238000 | 190000 | 238000 | 148000 | 238000 | 62900 |
| n (Samp) | 962 | 91 | 962 | 94 | 962 | 49 |
| n (Patient) | 367 | 91 | 367 | 94 | 367 | 49 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.57 | 0.64 | 0.53 | 0.57 | 0.60 | 0.53 | 0.45 | 0.53 | 0.43 |
| SE | 0.031 | 0.067 | 0.032 | 0.031 | 0.059 | 0.032 | 3.040 | 0.059 | 0.044 |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| p | 0.025 | 0.031 | 0.31 | 0.024 | 0.11 | 0.32 | 0.24 | 0.56 | 0.12 |
| nCohort 1 | 895 | 1357 | 962 | 895 | 1357 | 962 | 895 | 1357 | 962 |
| nCohort 2 | 99 | 20 | 91 | 102 | 26 | 94 | 57 | 25 | 49 |
| Cutoff 1 | 4740 | 6440 | 4710 | 4190 | 4220 | 4410 | 2670 | 3180 | 2670 |
| Sens 1 | 71% | 70% | 70% | 71% | 73% | 70% | 70% | 72% | 71% |
| Spec 1 | 47% | 56% | 44% | 43% | 40% | 42% | 27% | 29% | 25% |
| Cutoff 2 | 3990 | 4110 | 3970 | 3180 | 3340 | 3100 | 2090 | 2550 | 1890 |
| Sens 2 | 81% | 80% | 80% | 80% | 81% | 81% | 81% | 80% | 82% |
| Spec 2 | 41% | 39% | 38% | 32% | 31% | 29% | 19% | 22% | 15% |
| Cutoff 3 | 2150 | 3120 | 2150 | 2480 | 2420 | 2120 | 1290 | 1290 | 1290 |
| Sens 3 | 91% | 90% | 90% | 90% | 92% | 90% | 91% | 92% | 92% |
| Spec 3 | 19% | 29% | 18% | 24% | 21% | 17% | 9% | 8% | 7% |
| Cutoff 4 | 10700 | 11500 | 12800 | 10700 | 11500 | 12800 | 10700 | 11500 | 12800 |
| Sens 4 | 31% | 50% | 27% | 36% | 38% | 27% | 23% | 36% | 20% |
| Spec 4 | 70% | 70% | 70% | 70% | 70% | 70% | 70% | 70% | 70% |
| Cutoff 5 | 22400 | 22100 | 26200 | 22400 | 22100 | 26200 | 22400 | 22100 | 26200 |
| Sens 5 | 19% | 35% | 14% | 20% | 38% | 14% | 12% | 24% | 12% |
| Spec 5 | 80% | 80% | 80% | 80% | 80% | 80% | 80% | 80% | 80% |
| Cutoff 6 | 48800 | 45900 | 51900 | 48800 | 45900 | 51900 | 48800 | 45900 | 51900 |
| Sens 6 | 10% | 25% | 8% | 12% | 15% | 10% | 4% | 16% | 4% |
| Spec 6 | 90% | 90% | 90% | 90% | 90% | 90% | 90% | 90% | 90% |
| OR Quart 2 | 2.6 | 2.0 | 2.0 | 3.4 | 2.4 | 2.8 | 1.4 | 0.83 | 1.4 |
| | 0.0074 | 0.42 | 0.038 | 6.2E-4 | 0.22 | 0.0035 | 0.40 | 0.76 | 0.48 |
| p Value | 1.3 | 0.37 | 1.0 | 1.7 | 0.60 | 1.4 | 0.62 | 0.25 | 0.55 |
| 95% CI of OR Quart2 | 5.2 | 11 | 4.0 | 6.9 | 9.2 | 5.6 | 3.3 | 2.7 | 3.5 |
| OR Quart 3 | 2.8 | 2.5 | 2.3 | 2.7 | 2.0 | 2.7 | 1.9 | 0.83 | 2.1 |
| | 0.0034 | 0.27 | 0.014 | 0.0061 | 0.33 | 0.0051 | 0.12 | 0.76 | 0.10 |
| p Value | 1.4 | 0.49 | 1.2 | 1.3 | 0.50 | 1.3 | 0.84 | 0.25 | 0.87 |
| 95% CI of OR Quart3 | 5.6 | 13 | 4.4 | 5.6 | 8.1 | 5.4 | 4.1 | 2.7 | 4.9 |
| OR Quart 4 | 2.4 | 4.6 | 1.5 | 2.8 | 3.4 | 1.8 | 1.5 | 1.5 | 1.8 |
| | 0.015 | 0.053 | 0.29 | 0.0044 | 0.065 | 0.11 | 0.31 | 0.44 | 0.19 |
| p Value | 1.2 | 0.98 | 0.72 | 1.4 | 0.93 | 0.87 | 0.67 | 0.53 | 0.74 |
| 95% CI of. OR Quart4 | 4.8 | 21 | 3.0 | 5.8 | 12 | 3.8 | 3.5 | 4.3 | 4.4 |

**Proepiregulin**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 3.20 | 4.21 | 3.20 | 5.03 | 3.20 | 2.79 |
| Average | 5.72 | 5.80 | 5.72 | 8.53 | 5.72 | 5.54 |
| Stdev | 13.2 | 5.24 | 13.2 | 12.4 | 13.2 | 8.89 |
| p(t-test) | | 0.96 | | 0.088 | | 0.94 |
| Min | 0.0298 | 0.235 | 0.0298 | 0.00189 | 0.0298 | 0.000104 |
| Max | 279 | 31.4 | 279 | 81.9 | 279 | 43.0 |
| n (Samp) | 581 | 67 | 581 | 72 | 581 | 35 |
| n (Patient) | 276 | 67 | 276 | 72 | 276 | 35 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 3.45 | 4.45 | 3.45 | 5.98 | 3.45 | 4.31 |
| Average | 6.39 | 6.33 | 6.39 | 8.93 | 6.39 | 5.53 |
| Stdev | 13.2 | 5.02 | 13.2 | 12.7 | 13.2 | 3.77 |
| p(t-test) | | 0.99 | | 0.43 | | 0.81 |
| Min | 0.000104 | 0.847 | 0.000104 | 1.11 | 0.000104 | 0.667 |
| Max | 279 | 16.5 | 279 | 57.1 | 279 | 15.2 |
| n (Samp) | 801 | 9 | 801 | 17 | 801 | 13 |
| n (Patient) | 349 | 9 | 349 | 17 | 349 | 13 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 3.35 | 3.67 | 3.35 | 4.63 | 3.35 | 2.79 |
| Average | 5.95 | 5.55 | 5.95 | 7.97 | 5.95 | 7.04 |
| Stdev | 13.4 | 5.21 | 13.4 | 11.4 | 13.4 | 12.7 |
| p(t-test) | | 0.81 | | 0.23 | | 0.65 |
| Min | 0.000201 | 0.235 | 0.000201 | 0.00189 | 0.000201 | 0.000104 |
| Max | 279 | 31.4 | 279 | 81.9 | 279 | 57.1 |
| n (Samp) | 585 | 64 | 585 | 68 | 585 | 33 |
| n (Patient) | 260 | 64 | 260 | 68 | 260 | 33 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.59 | 0.60 | 0.56 | 0.61 | 0.65 | 0.58 | 0.45 | 0.59 | 0.44 |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| SE | 0.038 | 0.10 | 0.039 | 0.037 | 0.073 | 0.038 | 0.052 | 0.084 | 0.053 |
| p | 0.021 | 0.31 | 0.16 | 0.0029 | 0.041 | 0.026 | 0.29 | 0.27 | 0.28 |
| nCohort 1 | 581 | 801 | 585 | 581 | 801 | 585 | 581 | 801 | 585 |
| nCohort 2 | 67 | 9 | 64 | 72 | 17 | 68 | 35 | 13 | 33 |
| Cutoff 1 | 2.56 | 3.18 | 2.37 | 2.85 | 5.23 | 2.64 | 1.37 | 3.39 | 1.37 |
| Sens 1 | 70% | 78% | 70% | 71% | 71% | 71% | 71% | 77% | 73% |
| Spec 1 | 42% | 47% | 37% | 46% | 66% | 41% | 22% | 49% | 21% |
| Cutoff 2 | 2.17 | 3.15 | 1.88 | 1.86 | 2.56 | 1.80 | 0.806 | 2.95 | 0.806 |
| Sens 2 | 81% | 89% | 81% | 81% | 82% | 81% | 80% | 85% | 82% |
| Spec 2 | 37% | 47% | 31% | 32% | 39% | 29% | 12% | 44% | 11% |
| Cutoff 3 | 1.76 | 0.840 | 1.57 | 0.750 | 1.68 | 0.709 | 0.367 | 2.22 | 0.367 |
| Sens 3 | 91% | 100% | 91% | 90% | 94% | 91% | 91% | 92% | 91% |
| Spec 3 | 30% | 12% | 25% | 11% | 26% | 9% | 4% | 35% | 3% |
| Cutoff 4 | 5.47 | 6.00 | 5.71 | 5.47 | 6.00 | 5.71 | 5.47 | 6.00 | 5.71 |
| Sens 4 | 37% | 33% | 36% | 46% | 47% | 41% | 26% | 38% | 27% |
| Spec 4 | 70% | 70% | 70% | 70% | 70% | 70% | 70% | 70% | 70% |
| Cutoff 5 | 7.36 | 8.53 | 7.77 | 7.36 | 8.53 | 7.77 | 7.36 | 8.53 | 7.77 |
| Sens 5 | 24% | 33% | 22% | 33% | 29% | 32% | 23% | 15% | 21% |
| Spec 5 | 80% | 80% | 80% | 80% | 80% | 80% | 80% | 80% | 80% |
| Cutoff 6 | 12.1 | 13.6 | 12.5 | 12.1 | 13.6 | 12.5 | 12.1 | 13.6 | 12.5 |
| Sens 6 | 10% | 11% | 6% | 19% | 6% | 21% | 9% | 8% | 9% |
| Spec 6 | 90% | 90% | 90% | 90% | 90% | 90% | 90% | 90% | 90% |
| OR Quart 2 | 3.9 | 2.0 | 3.3 | 1.0 | 3.0 | 0.84 | 0.65 | 3.0 | 0.43 |
| | 0.0046 | 0.57 | 0.0082 | 1.0 | 0.34 | 0.67 | 0.43 | 0.34 | 0.17 |
| p Value | 1.5 | 0.18 | 1.4 | 0.42 | 0.31 | 0.36 | 0.23 | 0.31 | 0.13 |
| 95% CI of OR Quart2 | 9.9 | 22 | 8.0 | 2.4 | 29 | 1.9 | 1.9 | 29 | 1.4 |
| OR Quart 3 | 3.5 | 3.0 | 2.9 | 2.0 | 8.3 | 1.4 | 0.88 | 5.1 | 1.0 |
| | 0.010 | 0.34 | 0.018 | 0.067 | 0.047 | 0.35 | 0.80 | 0.14 | 1.0 |
| p Value | 1.3 | 0.31 | 1.2 | 0.95 | 1.0 | 0.68 | 0.33 | 0.59 | 0.39 |
| 95% CI of OR Quart3 | 8.9 | 29 | 7.2 | 4.4 | 67 | 3.0 | 2.4 | 44 | 2.6 |

(continued)

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| OR Quart 4 | 3.9 | 3.0 | 2.6 | 3.0 | 5.1 | 2.2 | 1.4 | 4.0 | 1.2 |
|  | 0.0046 | 0.34 | 0.041 | 0.0036 | 0.14 | 0.031 | 0.50 | 0.21 | 0.63 |
| p Value | 1.5 | 0.31 | 1.0 | 1.4 | 0.59 | 1.1 | 0.56 | 0.45 | 0.50 |
| 95% CI ot OR Quart4 | 9.9 | 29 | 6.4 | 6.2 | 44 | 4.4 | 3.3 | 36 | 3.1 |

**Heparin-binding growth factor 1**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 854 | 754 | 854 | 729 | 854 | 595 |
| Average | 1230 | 1000 | 1230 | 1140 | 1230 | 1060 |
| Stdev | 1510 | 922 | 1510 | 1170 | 1510 | 1420 |
| p(t-test) |  | 0.14 |  | 0.59 |  | 0.41 |
| Min | 0.00328 | 28.3 | 0.00328 | 8.50 | 0.00328 | 13.2 |
| Max | 16700 | 5510 | 16700 | 6000 | 16700 | 7750 |
| n (Samp) | 895 | 99 | 895 | 101 | 895 | 57 |
| n (Patient) | 374 | 99 | 374 | 101 | 374 | 57 |
| sCr only | 0hr prior to AKI stage | | 24hr prior to AKI stage | | 48hr prior to AKI stage | |
|  | Cohort 1 | Cohort 2 | Cohort 1 | Cohort 2 | Cohort 1 | Cohort 2 |
| Median | 847 | 536 | 847 | 545 | 847 | 289 |
| Average | 1220 | 631 | 1220 | 851 | 1220 | 664 |
| Stdev | 1460 | 445 | 1460 | 753 | 1460 | 802 |
| p(t-test) |  | 0.069 |  | 0.20 |  | 0.055 |
| Min | 0.00328 | 70.1 | 0.00328 | 90.5 | 0.00328 | 1.77 |
| Max | 16700 | 1870 | 16700 | 2920 | 16700 | 3380 |
| n (Samp) | 1357 | 20 | 1357 | 25 | 1357 | 25 |
| n (Patient) | 470 | 20 | 470 | 25 | 470 | 25 |

| UO only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 834 | 846 | 834 | 797 | 834 | 670 |
| Average | 1200 | 1040 | 1200 | 1170 | 1200 | 1140 |
| Stdev | 1490 | 941 | 1490 | 1190 | 1490 | 1470 |

(continued)

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| p(t-test) | | 0.32 | | 0.81 | | 0.78 |
| Min | 0.00328 | 28.3 | 0.00328 | 8.50 | 0.00328 | 13.2 |
| Max | 16700 | 5510 | 16700 | 6000 | 16700 | 7750 |
| n (Samp) | 961 | 91 | 961 | 94 | 961 | 49 |
| n (Patient) | 367 | 91 | 367 | 94 | 367 | 49 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.47 | 0.37 | 0.49 | 0.49 | 0.43 | 0.50 | 0.43 | 0.33 | 0.47 |
| SE | 0.031 | 0.067 | 0.032 | 0.030 | 0.060 | 0.031 | 0.041 | 0.060 | 0.043 |
| p | 0.35 | 0.055 | 0.79 | 0.75 | 0.24 | 0.98 | 0.10 | 0.0043 | 0.49 |
| nCohort 1 | 895 | 1357 | 961 | 895 | 1357 | 961 | 895 | 1357 | 961 |
| nCohort 2 | 99 | 20 | 91 | 101 | 25 | 94 | 57 | 25 | 49 |
| Cutoff 1 | 444 | 403 | 454 | 467 | 294 | 467 | 318 | 169 | 401 |
| Sens 1 | 71% | 70% | 70% | 70% | 72% | 70% | 70% | 72% | 71% |
| Spec 1 | 29% | 27% | 30% | 31% | 20% | 31% | 22% | 12% | 27% |
| Cutoff 2 | 249 | 242 | 280 | 359 | 247 | 359 | 192 | 77.7 | 287 |
| Sens 2 | 81% | 80% | 80% | 80% | 80% | 81% | 81% | 80% | 82% |
| Spec 2 | 17% | 17% | 19% | 24% | 17% | 24% | 13% | 6% | 19% |
| Cutoff 3 | 161 | 187 | 173 | 169 | 110 | 152 | 61.5 | 34.6 | 77.7 |
| Sens 3 | 91% | 90% | 90% | 90% | 92% | 90% | 91% | 92% | 92% |
| Spec 3 | 11% | 13% | 12% | 12% | 8% | 11% | 6% | 3% | 6% |
| Cutoff 4 | 1390 | 1400 | 1340 | 1390 | 1400 | 1340 | 1390 | 1400 | 1340 |
| Sens 4 | 26% | 5% | 29% | 27% | 20% | 27% | 25% | 20% | 27% |
| Spec 4 | 70% | 70% | 70% | 70% | 70% | 70% | 70% | 70% | 70% |
| Cutoff 5 | 1750 | 1780 | 1720 | 1750 | 1780 | 1720 | 1750 | 1780 | 1720 |
| Sens 5 | 16% | 5% | 16% | 21% | 12% | 21% | 12% | 8% | 14% |
| Spec 5 | 80% | 80% | 80% | 80% | 80% | 80% | 80% | 80% | 80% |
| Cutoff 6 | 2490 | 2520 | 2450 | 2490 | 2520 | 2450 | 2490 | 2520 | 2450 |
| Sens 6 | 6% | 0% | 8% | 11% | 4% | 12% | 9% | 4% | 8% |
| Spec 6 | 90% | 90% | 90% | 90% | 90% | 90% | 90% | 90% | 90% |
| OR Quart 2 | 1.4 | 5.1 | 1.5 | 1.0 | 1.0 | 1.4 | 1.3 | 2.5 | 1.1 |
| | 0.27 | 0.14 | 0.17 | 1.0 | 1.00 | 0.29 | 0.52 | 0.27 | 0.82 |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| p Value | 0.76 | 0.59 | 0.83 | 0.55 | 0.29 | 0.76 | 0.57 | 0.49 | 0.48 |
| 95% CI of OR Quart2 | 2.6 | 44 | 2.8 | 1.8 | 3.5 | 2.5 | 3.1 | 13 | 2.5 |
| OR Quart | 1.5 | 8.2 | 1.1 | 1.3 | 1.4 | 1.3 | 1.4 | 2.5 | 1.1 |
| 3 | 0.17 | 0.048 | 0.87 | 0.32 | 0.56 | 0.44 | 0.40 | 0.27 | 0.83 |
| p Value | 0.83 | 1.0 | 0.55 | 0.76 | 0.44 | 0.69 | 0.62 | 0.49 | 0.47 |
| 95% CI of OR Quart3 | 2.8 | 66 | 2.0 | 2.3 | 4.5 | 2.3 | 3.3 | 13 | 2.5 |
| OR Quart | 1.4 | 6.1 | 1.3 | 0.91 | 1.6 | 1.1 | 2.1 | 6.7 | 1.3 |
| 4 | 0.27 | 0.095 | 0.43 | 0.76 | 0.40 | 0.76 | 0.064 | 0.012 | 0.53 |
| p Value | 0.76 | 0.73 | 0.69 | 0.50 | 0.52 | 0.59 | 0.96 | 1.5 | 0.58 |
| 95% CI of OR Quart4 | 2.6 | 51 | 2.4 | 1.7 | 5.0 | 2.1 | 4.6 | 30 | 2.9 |

**Fibroblast growth factor 19**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 2.96E-5 | 2.75E-5 | 2.96E-5 | 3.44E-5 | 2.96E-5 | 2.58E-5 |
| Average | 0.00393 | 0.0150 | 0.00393 | 0.0190 | 0.00393 | 0.00519 |
| Stdev | 0.0267 | 0.116 | 0.0267 | 0.0885 | 0.0267 | 0.0298 |
| p(t-test) | | 0.033 | | 4.9E-4 | | 0.77 |
| Min | 5.62E-6 | 5.62E-6 | 5.62E-6 | 5.62E-6 | 5.62E-6 | 5.62E-6 |
| Max | 0.476 | 1.02 | 0.476 | 0.560 | 0.476 | 0.193 |
| n (Samp) | 754 | 78 | 754 | 86 | 754 | 42 |
| n (Patient) | 298 | 78 | 298 | 86 | 298 | 42 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 2.96E-5 | 3.28E-5 | 2.96E-5 | 2.63E-5 | 2.96E-5 | 2.66E-5 |
| Average | 0.00514 | 0.0622 | 0.00514 | 0.0258 | 0.00514 | 0.000233 |
| Stdev | 0.0328 | 0.240 | 0.0328 | 0.116 | 0.0328 | 0.000850 |

(continued)

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| p(t-test) | | 3.9E-8 | | 0.0068 | | 0.53 |
| Min | 5.62E-6 | 7.53E-6 | 5.62E-6 | 5.62E-6 | 5.62E-6 | 5.62E-6 |
| Max | 0.476 | 1.02 | 0.476 | 0.560 | 0.476 | 0.00364 |
| n (Samp) | 1164 | 18 | 1164 | 23 | 1164 | 18 |
| n (Patient) | 379 | 18 | 379 | 23 | 379 | 18 |
| UO only | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 2.83E-5 | 2.68E-5 | 2.83E-5 | 3.44E-5 | 2.83E-5 | 2.58E-5 |
| Average | 0.00941 | 0.0153 | 0.00941 | 0.0138 | 0.00941 | 0.00595 |
| Stdev | 0.0925 | 0.122 | 0.0925 | 0.0684 | 0.0925 | 0.0321 |
| p(t-test) | | 0.62 | | 0.68 | | 0.82 |
| Min | 5.62E-6 | 5.62E-6 | 5.62E-6 | 5.62E-6 | 5.62E-6 | 5.62E-6 |
| Max | 2.28 | 1.02 | 2.28 | 0.470 | 2.28 | 0.193 |
| n (Samp) | 838 | 70 | 838 | 80 | 838 | 36 |
| n (Patient) | 306 | 70 | 306 | 80 | 306 | 36 |

| | 0hr prior to AKI stage | | | 24hr prior to AKI stage | | | 48hr prior to AKI stage | | |
| --- | --- | --- | --- | --- | --- | --- | --- | --- | --- |
| | sCr or UO | sCr only | UO only | sCr or UO | sCr only | UO only | sCr or UO | sCr only | UO only |
| AUC | 0.48 | 0.54 | 0.47 | 0.55 | 0.50 | 0.57 | 0.43 | 0.41 | 0.46 |
| SE | 0.035 | 0.070 | 0.036 | 0.034 | 0.061 | 0.035 | 0.047 | 0.071 | 0.050 |
| p | 0.53 | 0.54 | 0.41 | 0.12 | 0.98 | 0.055 | 0.12 | 0.22 | 0.39 |
| nCohort 1 | 754 | 1164 | 838 | 754 | 1164 | 838 | 754 | 1164 | 838 |
| nCohort 2 | 78 | 18 | 70 | 86 | 23 | 80 | 42 | 18 | 36 |
| Cutoff 1 | 1.92E-5 | 1.88E-5 | 2.17E-5 | 2.46E-5 | 1.88E-5 | 2.46E-5 | 1.92E-5 | 2.17E-5 | 1.92E-5 |
| Sens 1 | 74% | 78% | 70% | 73% | 78% | 72% | 76% | 72% | 75% |
| Spec 1 | 23% | 20% | 28% | 34% | 20% | 35% | 23% | 28% | 24% |
| Cutoff 2 | 1.82E-5 | 1.82E-5 | 1.88E-5 | 1.92E-5 | 1.82E-5 | 1.92E-5 | 1.82E-5 | 1.92E-5 | 1.82E-5 |
| Sens 2 | 86% | 89% | 80% | 81% | 87% | 81% | 86% | 83% | 83% |
| Spec 2 | 15% | 17% | 20% | 23% | 17% | 24% | 15% | 24% | 16% |
| Cutoff 3 | 1.34E-5 | 7.53E-6 | 1.34E-5 | 1.82E-5 | 1.66E-5 | 1.82E-5 | 1.34E-5 | 1.82E-5 | 1.34E-5 |
| Sens 3 | 94% | 94% | 93% | 91% | 91% | 90% | 95% | 94% | 94% |
| Spec 3 | 9% | 5% | 9% | 15% | 13% | 16% | 9% | 17% | 9% |
| Cutoff 4 | 8.63E-5 | 4.39E-5 | 4.39E-5 | 8.63E-5 | 4.39E-5 | 4.39E-5 | 8.63E-5 | 4.39E-5 | 4.39E-5 |
| Sens 4 | 22% | 39% | 21% | 35% | 35% | 41% | 14% | 11% | 22% |
| Spec 4 | 73% | 72% | 70% | 73% | 72% | 70% | 73% | 72% | 70% |
| Cutoff 5 | 0.000168 | 0.000114 | 0.000114 | 0.000168 | 0.000114 | 0.000114 | 0.000168 | 0.000114 | 0.000114 |
| Sens 5 | 15% | 33% | 16% | 27% | 26% | 31% | 12% | 11% | 14% |
| Spec 5 | 84% | 80% | 80% | 84% | 80% | 80% | 84% | 80% | 80% |
| Cutoff 6 | 0.00325 | 0.00364 | 0.00364 | 0.00325 | 0.00364 | 0.00364 | 0.00325 | 0.00364 | 0.00364 |
| Sens 6 | 13% | 28% | 9% | 14% | 17% | 11% | 10% | 0% | 8% |
| Spec 6 | 90% | 90% | 90% | 90% | 90% | 90% | 90% | 90% | 90% |
| OR Quart | 1.1 | 0.49 | 1.6 | 1.1 | 0.28 | 1.1 | 1.3 | 1.5 | 1.7 |
| 2 | 0.86 | 0.32 | 0.20 | 0.74 | 0.12 | 0.73 | 0.59 | 0.65 | 0.31 |

(continued)

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| p Value |  | 0.12 | 0.78 | 0.58 | 0.058 | 0.56 | 0.46 | 0.25 | 0.61 |
| 95% CI of OR Quart2 | 2.1 | 2.0 | 3.5 | 2.1 | 1.4 | 2.3 | 4.0 | 9.1 | 4.8 |
| OR Quart 3 | 1.4 | 0.33 | 1.8 | 1.1 | 1.0 | 1.1 | 2.6 | 5.1 | 1.9 |
|  | 0.32 | 0.18 | 0.11 | 0.87 | 1.0 | 0.86 | 0.051 | 0.036 | 0.22 |
| p Value | 0.72 | 0.066 | 0.88 | 0.55 | 0.35 | 0.53 | 1.00 | 1.1 | 0.68 |
| 95% CI of OR Quart3 | 2.7 | 1.6 | 3.8 | 2.0 | 2.9 | 2.2 | 6.9 | 24 | 5.2 |
| OR Quart 4 | 1.2 | 1.2 | 1,5 | 1.4 | 1.0 | 1.9 | 2.2 | 1.5 | 1.5 |
|  | 0.61 | 0.78 | 0.26 | 0.27 | 0.99 | 0.046 | 0.11 | 0.65 | 0.43 |
| p Value | 0.61 | 0.39 | 0.73 | 0.76 | 0.35 | 1.0 | 0.84 | 0.25 | 0.53 |
| 95% CI of OR Quart4 | 2.4 | 3.5 | 3.3 | 2.7 | 2.9 | 3.6 | 6.0 | 9.1 | 4.4 |

**Fibroblast growth factor 21**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.0141 | 0.0142 | 0.0141 | 0.0159 | 0.0141 | 0.0141 |
| Average | 0.134 | 0.189 | 0.134 | 0.264 | 0.134 | 0.113 |
| Stdev | 0.490 | 0.614 | 0.490 | 0.778 | 0.490 | 0.249 |
| p(t-test) | | 0.36 | | 0.031 | | 0.78 |
| Min | 1.14E-9 | 1.14E-9 | 1.14E-9 | 1.40E-9 | 1.14E-9 | 0.000186 |
| Max | 8.92 | 4.66 | 8.92 | 4.47 | 8.92 | 1.05 |
| n (Samp) | 754 | 78 | 754 | 86 | 754 | 42 |
| n (Patient) | 298 | 78 | 298 | 86 | 298 | 42 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.0143 | 0.0158 | 0.0143 | 0.0371 | 0.0143 | 0.0137 |
| Average | 0.157 | 0.352 | 0.157 | 0.217 | 0.157 | 0.133 |
| Stdev | 0.510 | 1.09 | 0.510 | 0.706 | 0.510 | 0.209 |
| p(t-test) | | 0.12 | | 0.58 | | 0.85 |
| Min | 1.14E-9 | 0.00163 | 1.14E-9 | 7.54E-8 | 1.14E-9 | 0.000952 |
| Max | 8.92 | 4.66 | 8.92 | 3.42 | 8.92 | 0.635 |
| n (Samp) | 1164 | 18 | 1164 | 23 | 1164 | 18 |
| n (Patient) | 379 | 18 | 379 | 23 | 379 | 18 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.0145 | 0.0134 | 0.0145 | 0.0146 | 0.0145 | 0.0141 |
| Average | 0.183 | 0.131 | 0.183 | 0.235 | 0.183 | 0.0928 |
| Stdev | 0.639 | 0.352 | 0.639 | 0.722 | 0.639 | 0.237 |
| p(t-test) | | 0.50 | | 0.50 | | 0.40 |
| Min | 1.14E-9 | 1.14E-9 | 1.14E-9 | 1.40E-9 | 1.14E-9 | 0.000186 |
| Max | 8.92 | 2.40 | 8.92 | 4.47 | 8.92 | 1.05 |
| n (Samp) | 838 | 70 | 838 | 80 | 838 | 36 |
| n (Patient) | 306 | 70 | 306 | 80 | 306 | 36 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.51 | 0.55 | 0.49 | 0.53 | 0.61 | 0.51 | 0.49 | 0.54 | 0.46 |
| SE | 0.035 | 0.070 | 0.036 | 0.033 | 0.063 | 0.034 | 0.046 | 0.070 | 0.050 |
| p | 0.71 | 0.48 | 0.79 | 0.31 | 0.086 | 0.77 | 0.81 | 0.60 | 0.43 |
| nCohort 1 | 754 | 1164 | 838 | 754 | 1164 | 838 | 754 | 1164 | 838 |
| nCohort 2 | 78 | 18 | 70 | 86 | 23 | 80 | 42 | 18 | 36 |
| Cutoff 1 | 0.00681 | 0.00693 | 0.00611 | 0.00657 | 0.0157 | 0.00657 | 0.00752 | 0.00639 | 0.00324 |
| Sens 1 | 71% | 72% | 70% | 71% | 74% | 70% | 71% | 72% | 72% |
| Spec 1 | 34% | 33% | 30% | 33% | 53% | 32% | 35% | 31% | 18% |
| Cutoff 2 | 0.00435 | 0.00618 | 0.00378 | 0.00479 | 0.00811 | 0.00481 | 0.00254 | 0.00583 | 0.00204 |
| Sens 2 | 81% | 83% | 80% | 80% | 83% | 80% | 81% | 83% | 81% |
| Spec 2 | 24% | 30% | 21% | 26% | 36% | 25% | 16% | 29% | 13% |
| Cutoff 3 | 0.00151 | 0.00338 | 0.00151 | 0.00123 | 0.00124 | 0.00198 | 0.00105 | 0.00254 | 0.000965 |
| Sens 3 | 91% | 94% | 90% | 91% | 91% | 90% | 90% | 94% | 92% |
| Spec 3 | 10% | 19% | 9% | 9% | 8% | 12% | 8% | 15% | 7% |
| Cutoff 4 | 0.0343 | 0.0360 | 0.0369 | 0.0343 | 0.0360 | 0.0369 | 0.0343 | 0.0360 | 0.0369 |
| Sens 4 | 29% | 33% | 29% | 38% | 57% | 34% | 29% | 39% | 31% |
| Spec 4 | 70% | 70% | 70% | 70% | 70% | 70% | 70% | 70% | 70% |
| Cutoff 5 | 0.0695 | 0.0887 | 0.0945 | 0.0695 | 0.0887 | 0.0945 | 0.0695 | 0.0887 | 0.0945 |
| Sens 5 | 26% | 33% | 21% | 24% | 22% | 24% | 19% | 28% | 14% |
| Spec 5 | 80% | 80% | 80% | 80% | 80% | 80% | 80% | 80% | 80% |
| Cutoff 6 | 0.295 | 0.363 | 0.416 | 0.295 | 0.363 | 0.416 | 0.295 | 0.363 | 0.416 |
| Sens 6 | 17% | 17% | 10% | 15% | 9% | 12% | 14% | 17% | 8% |
| Spec 6 | 90% | 90% | 90% | 90% | 90% | 90% | 90% | 90% | 90% |
| OR Quart | 1.5 | 1.7 | 0.88 | 1.4 | 0.49 | 1.6 | 1.1 | 2.0 | 1.6 |
| 2 | 0.24 | 0.48 | 0.72 | 0.32 | 0.42 | 0.19 | 0.82 | 0.33 | 0.33 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| p Value | 0.76 | 0.40 | 0.44 | 0.72 | 0.090 | 0.80 | 0.46 | 0.50 | 0.61 |
| 95% CI of OR Quart2 | 2.9 | 7.1 | 1.8 | 2.7 | 2.7 | 3.0 | 2.7 | 8.1 | 4.2 |
| OR Quart | 1.2 | 1.3 | 1.1 | 1.1 | 2.0 | 0.93 | 1.0 | 1.3 | 1.0 |
| 3 | 0.60 | 0.70 | 0.86 | 0.73 | 0.25 | 0.85 | 1.0 | 0.70 | 1.0 |
| p Value | 0.60 | 0.30 | 0.54 | 0.57 | 0.60 | 0.45 | 0.41 | 0.30 | 0.34 |
| 95% CI of OR Quart3 | 2.4 | 6.0 | 2.1 | 2.2 | 6.8 | 1.9 | 2.5 | 6.0 | 2.9 |
| OR Quart | 1.3 | 2.0 | 0.94 | 1.7 | 2.3 | 1.6 | 1.1 | 1.7 | 1.6 |
| 4 | 0.49 | 0.33 | 0.86 | 0.11 | 0.17 | 0.15 | 0.82 | 0.48 | 0.33 |
| p Value | 0.64 | 0.50 | 0.47 | 0.88 | 0.69 | 0.84 | 0.46 | 0.40 | 0.61 |
| 95% CI of OR Quart4 | 2.5 | 8.1 | 1.9 | 3.2 | 7.5 | 3.1 | 2.7 | 7.1 | 4.2 |

**Thrombospondin-2**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1240 | 1920 | 1240 | 1530 | 1240 | 1120 |
| Average | 1930 | 2680 | 1930 | 4030 | 1930 | 1970 |
| Stdev | 2780 | 2580 | 2780 | 10600 | 2780 | 3040 |
| p(t-test) | | 0.011 | 2.9E-6 | 2.9E-6 | | 0.92 |
| Min | 0.0376 | 66.6 | 0.0376 | 108 | 0.0376 | 14.6 |
| Max | 45800 | 11900 | 45800 | 80100 | 45800 | 19700 |
| n (Samp) | 895 | 99 | 895 | 102 | 895 | 57 |
| n (Patient) | 374 | 99 | 374 | 102 | 374 | 57 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1370 | 1810 | 1370 | 1520 | 1370 | 1280 |
| Average | 2310 | 3240 | 2310 | 5550 | 2310 | 1900 |
| Stdev | 3600 | 2910 | 3600 | 15400 | 3600 | 1810 |
| p(t-test) | | 0.25 | | 7.9E-5 | | 0.57 |
| Min | 0.0376 | 227 | 0.0376 | 174 | 0.0376 | 201 |
| Max | 68100 | 8700 | 68100 | 80100 | 68100 | 7000 |
| n (Samp) | 1357 | 20 | 1357 | 26 | 1357 | 25 |
| n (Patient) | 470 | 20 | 470 | 26 | 470 | 25 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1280 | 1860 | 1280 | 1570 | 1280 | 1160 |
| Average | 2140 | 2550 | 2140 | 3340 | 2140 | 2160 |
| Stdev | 3830 | 2460 | 3830 | 7570 | 3830 | 3300 |
| p(t-test) | | 0.32 | | 0.010 | | 0.98 |
| Min | 0.0376 | 66.6 | 0.0376 | 108 | 0.0376 | 14.6 |
| Max | 80100 | 11900 | 80100 | 68100 | 80100 | 19700 |
| n (Samp) | 962 | 91 | 962 | 94 | 962 | 49 |
| n (Patient) | 367 | 91 | 367 | 94 | 367 | 49 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.59 | 0.62 | 0.57 | 0.57 | 0.57 | 0.56 | 0.47 | 0.49 | 0.46 |
| SE | 0.031 | 0.068 | 0.032 | 0.031 | 0.059 | 0.032 | 0.040 | 0.059 | 0.043 |
| p | 0.0029 | 0.064 | 0.032 | 0.030 | 0.21 | 0.083 | 0.40 | 0.87 | 0.41 |
| nCohort 1 | 895 | 1357 | 962 | 895 | 1357 | 962 | 895 | 1357 | 962 |
| nCohort 2 | 99 | 20 | 91 | 102 | 26 | 94 | 57 | 25 | 49 |
| Cutoff 1 | 899 | 1380 | 899 | 799 | 1020 | 799 | 575 | 679 | 541 |
| Sens 1 | 71 % | 70% | 70% | 71 % | 73% | 70% | 70% | 72% | 71% |
| Spec 1 | 37% | 51% | 35% | 34% | 39% | 32% | 23% | 26% | 21% |
| Cutoff 2 | 612 | 878 | 612 | 501 | 686 | 498 | 450 | 589 | 450 |
| Sens 2 | 81% | 80% | 80% | 80% | 81% | 81% | 81% | 80% | 82% |
| Spec 2 | 25% | 34% | 24% | 20% | 27% | 19% | 19% | 23% | 17% |
| Cutoff 3 | 293 | 831 | 332 | 348 | 275 | 353 | 214 | 354 | 191 |
| Sens 3 | 91% | 90% | 90% | 90% | 92% | 90% | 91% | 92% | 92% |
| Spec 3 | 10% | 32% | 11% | 14% | 9% | 13% | 7% | 13% | 5% |
| Cutoff 4 | 2000 | 2300 | 2070 | 2000 | 2300 | 2070 | 2000 | 2300 | 2070 |
| Sens 4 | 48% | 40% | 48% | 41% | 35% | 44% | 25% | 24% | 27% |
| Spec 4 | 70% | 70% | 70% | 70% | 70% | 70% | 70% | 70% | 70% |
| Cutoff 5 | 2680 | 3160 | 2840 | 2680 | 3160 | 2840 | 2680 | 3160 | 2840 |
| Sens 5 | 36% | 35% | 33% | 33% | 35% | 32% | 21% | 24% | 18% |
| Spec 5 | 80% | 80% | 80% | 80% | 80% | 80% | 80% | 80% | 80% |
| Cutoff 6 | 4110 | 4870 | 4360 | 4110 | 4870 | 4360 | 4110 | 4870 | 4360 |
| Sens 6 | 21% | 25% | 16% | 20% | 19% | 17% | 11% | 8% | 12% |
| Spec 6 | 90% | 90% | 90% | 90% | 90% | 90% | 90% | 90% | 90% |
| OR Quart 2 | 1.00 | 2.0 | 1.0 | 0.55 | 1.8 | 0.62 | 0.92 | 0.83 | 1.0 |
| | 0.99 | 0.42 | 1.0 | 0.076 | 0.37 | 0.15 | 0.84 | 0.77 | 1.0 |
| p Value | 0.52 | 0.37 | 0.52 | 0.28 | 0.51 | 0.32 | 0.41 | 0.25 | 0.43 |
| 95% CI of OR Quart2 | 1.9 | 11 | 1.9 | 1.1 | 6.1 | 1.2 | 2.1 | 2.8 | 2.4 |
| OR Quart 3 | 0.95 | 3.6 | 0.84 | 0.96 | 1.5 | 0.74 | 1.0 | 1.2 | 1.0 |
| | 0.87 | 0.12 | 0.61 | 0.88 | 0.53 | 0.35 | 1.0 | 0.78 | 1.0 |
| p Value | 0.49 | 0.73 | 0.43 | 0.54 | 0.42 | 0.40 | 0.45 | 0.39 | 0.43 |
| 95% CI of OR Quart3 | 1.8 | 17 | 1.6 | 1.7 | 5.4 | 1.4 | 2.2 | 3.5 | 2.4 |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| OR Quart | 2.2 | 3.5 | 1.8 | 1.4 | 2.3 | 1.4 | 1.5 | 1.2 | 1.5 |
| 4 | 0.0072 | 0.12 | 0.048 | 0.18 | 0.17 | 0.22 | 0.27 | 0.78 | 0.32 |
| p Value | 1.2 | 0.73 | 1.0 | 0.84 | 0.69 | 0.82 | 0.72 | 0.39 | 0.68 |
| 95% CI OR Quart4 | 3.9 | 17 | 3.2 | 2.5 | 7.5 | 2.4 | 3.1 | 3.5 | 3.3 |

**Tenascin**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 10.3 | 12.5 | 10.3 | 10.6 | 10.3 | 0.452 |
| Average | 17.8 | 18.7 | 17.8 | 126 | 17.8 | 22.1 |
| Stdev | 47.1 | 20.2 | 47.1 | 866 | 47.1 | 56.8 |
| p(t-test) | | 0.87 | | 0.0025 | | 0.59 |
| Min | 0.00398 | 0.00398 | 0.00398 | 0.00398 | 0.00398 | 0.00398 |
| Max | 945 | 91.5 | 945 | 7540 | 945 | 317 |
| n (Samp) | 597 | 69 | 597 | 76 | 597 | 37 |
| n (Patient) | 279 | 69 | 279 | 76 | 279 | 37 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 10.6 | 0.452 | 10.6 | 20.6 | 10.6 | 0.397 |
| Average | 28.3 | 5.80 | 28.3 | 21.0 | 28.3 | 12.3 |
| Stdev | 266 | 10.5 | 266 | 18.3 | 266 | 17.3 |
| p(t-test) | | 0.80 | | 0.91 | | 0.82 |
| Min | 0.00398 | 0.0132 | 0.00398 | 0.00398 | 0.00398 | 0.0184 |
| Max | 7540 | 31.7 | 7540 | 72.0 | 7540 | 50.9 |
| n (Samp) | 826 | 9 | 826 | 17 | 826 | 14 |
| n (Patient) | 352 | 9 | 352 | 17 | 352 | 14 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 10.6 | 12.5 | 10.6 | 9.71 | 10.6 | 2.31 |

(continued)

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Average | 17.7 | 19.1 | 17.7 | 131 | 17.7 | 25.1 |
| Stdev | 46.7 | 20.3 | 46.7 | 890 | 46.7 | 58.7 |
| p(t-test) | | 0.81 | | 0.0019 | | 0.38 |
| Min | 0.00398 | 0.00398 | 0.00398 | 0.00398 | 0.00398 | 0.00398 |
| Max | 945 | 91.5 | 945 | 7540 | 945 | 317 |
| n (Samp) | 604 | 66 | 604 | 72 | 604 | 34 |
| n (Patient) | 263 | 66 | 263 | 72 | 263 | 34 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.55 | 0.31 | 0.56 | 0.54 | 0.62 | 0.52 | 0.43 | 0.43 | 0.48 |
| SE | 0.037 | 0.099 | 0.038 | 0.036 | 0.073 | 0.036 | 0.050 | 0.080 | 0.051 |
| p | 0.20 | 0.054 | 0.12 | 0.33 | 0.10 | 0.63 | 0.19 | 0.41 | 0.71 |
| nCohort 1 | 597 | 826 | 604 | 597 | 826 | 604 | 597 | 826 | 604 |
| nCohort 2 | 69 | 9 | 66 | 76 | 17 | 72 | 37 | 14 | 34 |
| Cutoff 1 | 0.533 | 0.0187 | 4.89 | 1.39 | 10.0 | 0.338 | 0.117 | 0.315 | 0.315 |
| Sens 1 | 71% | 78% | 71% | 71% | 71% | 74% | 70% | 71% | 71% |
| Spec 1 | 31% | 10% | 38% | 32% | 49% | 28% | 23% | 27% | 27% |
| Cutoff 2 | 0.117 | 0.00398 | 0.315 | 0.117 | 5.43 | 0.0748 | 0.0208 | 0.0187 | 0.103 |
| Sens 2 | 81% | 100% | 82% | 80% | 82% | 83% | 81% | 86% | 82% |
| Spec 2 | 23% | 2% | 27% | 23% | 40% | 18% | 14% | 10% | 23% |
| Cutoff 3 | 0.0132 | 0.00398 | 0.0132 | 0.0190 | 0.338 | 0.0187 | 0.0179 | 0.0184 | 0.0179 |
| Sens 3 | 94% | 100% | 95% | 91% | 94% | 92% | 92% | 93% | 91% |
| Spec 3 | 3% | 2% | 4% | 12% | 29% | 11% | 7% | 9% | 8% |
| Cutoff 4 | 17.2 | 19.3 | 18.0 | 17.2 | 19.3 | 18.0 | 17.2 | 19.3 | 18.0 |
| Sens 4 | 42% | 11% | 41% | 37% | 53% | 35% | 32% | 29% | 38% |
| Spec 4 | 70% | 70% | 70% | 70% | 70% | 70% | 70% | 70% | 70% |
| Cutoff 5 | 25.6 | 28.1 | 25.8 | 25.6 | 28.1 | 25.8 | 25.6 | 28.1 | 25.8 |
| Sens 5 | 29% | 11% | 29% | 26% | 24% | 25% | 19% | 21% | 24% |
| Spec 5 | 80% | 81% | 80% | 80% | 81% | 80% | 80% | 81% | 80% |
| Cutoff 6 | 39.2 | 43.5 | 38.4 | 39.2 | 43.5 | 38.4 | 39.2 | 43.5 | 38.4 |
| Sens 6 | 13% | 0% | 14% | 16% | 6% | 17% | 14% | 7% | 15% |
| Spec 6 | 90% | 90% | 90% | 90% | 90% | 90% | 90% | 90% | 90% |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| OR Quart | 1.1 | 0 | 1.2 | 1.5 | 5.1 | 1.1 | 0.75 | 0.25 | 0.66 |
| 2 | 0.86 | na | 0.69 | 0.23 | 0.14 | 0.86 | 0.59 | 0.21 | 0.44 |
| p Value | 0.50 | na | 0.53 | 0.77 | 0.59 | 0.54 | 0.25 | 0.027 | 0.23 |
| 95% CI of OR Quart2 | 2.3 | na | 2.6 | 3.1 | 44 | 2.1 | 2.2 | 2.2 | 1.9 |
| OR Quart | 1.1 | 4.1 | 1.4 | 1.2 | 5.1 | 0.88 | 1.3 | 1.3 | 1.1 |
| 3 | 0.85 | 0.21 | 0.43 | 0.58 | 0.14 | 0.72 | 0.63 | 0.74 | 0.81 |
| p Value | 0.50 | 0.45 | 0.63 | 0.60 | 0.59 | 0.43 | 0.49 | 0.33 | 0.44 |
| 95% CI of OR Quart3 | 23 | 37 | 3.0 | 2.5 | 44 | 1.8 | 3.3 | 4.7 | 2.8 |
| OR Quart | 1.9 | 4.1 | 2.2 | 1.4 | 6.1 | 1.1 | 1.7 | 1.0 | 1.0 |
| 4 | 0.067 | 0.21 | 0.039 | 0.30 | 0.095 | 0.86 | 0.26 | 1.0 | 0.99 |
| p Value | 0.96 | 0.45 | 1.0 | 0.72 | 0.73 | 0.54 | 0.68 | 0.25 | 0.39 |
| 95% CI of OR Quart4 | 3.8 | 37 | 4.5 | 2.9 | 51 | 2.1 | 4.2 | 4.1 | 2.6 |

Fig. 3: Comparison of marker levels in urine samples collected within 12 hours of reaching stage R from Cohort 1 (patients that reached, but did not progress beyond, RIFLE stage R) and from Cohort 2 (patients that reached RIFLE stage I or F).

**Angiopoietin-related protein 4**

| | sCr or UO | | sCr only | | UO only | |
|---|---|---|---|---|---|---|
| | Cohort 1 | Cohort 2 | Cohort 1 | Cohort 2 | Cohort 1 | Cohort 2 |
| Median | 11.6 | 10.1 | 15.0 | 40.4 | 10.9 | 9.76 |
| Average | 39.6 | 39.3 | 49.2 | 70.6 | 36.1 | 43.8 |
| Stdev | 103 | 85.6 | 104 | 83.2 | 93.4 | 101 |
| p(t-test) | | 0.99 | | 0.44 | | 0.59 |
| Min | 0.000466 | 1.12 | 0.577 | 5.74 | 0.000466 | 0.00194 |

| | sCr or UO | | sCr only | | UO only | |
|---|---|---|---|---|---|---|
| | Cohort 1 | Cohort 2 | Cohort 1 | Cohort 2 | Cohort 1 | Cohort 2 |
| Max | 789 | 645 | 624 | 276 | 789 | 645 |

(continued)

|  | sCr or UO | | sCr only | | UO only | |
|---|---|---|---|---|---|---|
|  | Cohort 1 | Cohort 2 | Cohort 1 | Cohort 2 | Cohort 1 | Cohort 2 |
| n (Samp) | 128 | 85 | 41 | 18 | 115 | 73 |
| n (Patient) | 128 | 85 | 41 | 18 | 115 | 73 |

|  | At Enrollment | | |
|---|---|---|---|
|  | sCr or UO | sCr only | UO only |
| AUC | 0.51 | 0.66 | 0.52 |
| SE | 0.041 | 0.080 | 0.043 |
| p | 0.72 | 0.045 | 0.65 |
| nCohort 1 | 128 | 41 | 115 |
| nCohort 2 | 85 | 18 | 73 |
| Cutoff 1 | 6.86 | 12.5 | 6.65 |
| Sens 1 | 71% | 72% | 71% |
| Spec 1 | 31% | 46% | 32% |
| Cutoff 2 | 4.68 | 10.1 | 4.43 |
| Sens 2 | 80% | 83% | 81% |
| Spec 2 | 23% | 39% | 17% |
| Cutoff 3 | 3.17 | 5.81 | 2.85 |
| Sens 3 | 91% | 94% | 90% |
| Spec 3 | 12% | 24% | 9% |
| Cutoff 4 | 21.9 | 35.2 | 17.4 |
| Sens 4 | 34% | 61% | 42% |
| Spec 4 | 70% | 71% | 70% |
| Cutoff 5 | 35.2 | 55.4 | 28.9 |
| Sens 5 | 25% | 39% | 25% |
| Spec 5 | 80% | 80% | 80% |
| Cutoff 6 | 77.4 | 83.9 | 65.2 |
| Sens 6 | 11% | 22% | 16% |
| Spec 6 | 91% | 90% | 90% |
| OR Quart 2 | 0.93 | 1.3 | 0.84 |
| p Value | 0.84 | 0.74 | 0.67 |
| 95% CI of | 0.43 | 0.24 | 0.37 |
| OR Quart2 | 2.0 | 7.4 | 1.9 |
| OR Quart 3 | 0.62 | 1.3 | 0.57 |
| p Value | 0.23 | 0.74 | 0.20 |
| 95% CI of | 0.28 | 0.24 | 0.24 |
| OR Quart3 | 1.4 | 7.4 | 1.3 |

(continued)

|  | At Enrollment | | |
|---|---|---|---|
|  | sCr or UO | sCr only | UO only |
| OR Quart 4 | 0.97 | 3.2 | 1.1 |
| p Value | 0.93 | 0.16 | 0.84 |
| 95% CI of | 0.45 | 0.63 | 0.48 |
| OR Quart4 | 2.1 | 16 | 2.5 |

**Amphiregulin**

|  | sCr or UO | | sCr only | | UO only | |
|---|---|---|---|---|---|---|
|  | Cohort 1 | Cohort 2 | Cohort 1 | Cohort 2 | Cohort 1 | Cohort 2 |
| Median | 25.7 | 30.6 | 32.9 | 80.6 | 24.8 | 30.2 |
| Average | 60.4 | 121 | 69.1 | 481 | 54.7 | 39.6 |
| Stdev | 164 | 467 | 93.8 | 1010 | 170 | 40.2 |
| p(t-test) |  | 0.21 |  | 0.011 |  | 0.52 |
| Min | 1.21 | 0.00389 | 6.31 | 9.15 | 1.21 | 0.00389 |
| Max | 1640 | 3480 | 426 | 3480 | 1640 | 211 |
| n (Samp) | 113 | 67 | 41 | 13 | 96 | 54 |
| n (Patient) | 113 | 67 | 41 | 13 | 96 | 54 |

|  | At Enrollment | | |
|---|---|---|---|
|  | sCr or UO | sCr only | UO only |
| AUC | 0.52 | 0.69 | 0.53 |
| SE | 0.045 | 0.090 | 0.049 |
| p | 0.59 | 0.031 | 0.60 |
| nCohort 1 | 113 | 41 | 96 |
| nCohort 2 | 67 | 13 | 54 |
| Cutoff 1 | 16.3 | 37.8 | 16.8 |
| Sens 1 | 70% | 77% | 70% |
| Spec 1 | 33% | 61% | 34% |
| Cutoff 2 | 12.2 | 28.1 | 14.0 |
| Sens 2 | 81% | 85% | 81% |
| Spec 2 | 21% | 46% | 25% |
| Cutoff 3 | 9.94 | 9.15 | 10.5 |
| Sens 3 | 91% | 92% | 91% |
| Spec 3 | 16% | 12% | 17% |
| Cutoff 4 | 42.2 | 59.5 | 42.2 |

(continued)

|  | At Enrollment | | |
|---|---|---|---|
|  | sCr or UO | sCr only | UO only |
| Sens 4 | 31% | 62% | 30% |
| Spec 4 | 71% | 71% | 71% |
| Cutoff 5 | 56.0 | 82.1 | 50.9 |
| Sens 5 | 22% | 46% | 19% |
| Spec 5 | 81% | 80% | 80% |
| Cutoff 6 | 96.9 | 189 | 72.6 |
| Sens 6 | 12% | 31% | 13% |
| Spec 6 | 90% | 90% | 91% |
| OR Quart 2 | 0.91 | 0.42 | 0.96 |
| p Value | 0.82 | 0.51 | 0.94 |
| 95% CI of | 0.38 | 0.034 | 0.36 |
| OR Quart2 | 2.2 | 5.3 | 2.5 |
| OR Quart 3 | 1.4 | 2.4 | 2.0 |
| p Value | 0.39 | 0.36 | 0.16 |
| 95% CI of | 0.62 | 0.36 | 0.77 |
| OR Quart3 | 3.4 | 17 | 5.1 |
| OR Quart 4 | 1.0 | 4.1 | 0.96 |
| p Value | 1.0 | 0.13 | 0.94 |
| 95% CI of | 0.42 | 0.65 | 0.36 |
| OR Quart4 | 2.4 | 26 | 2.5 |

**Endostatin**

|  | sCr or UO | | sCr only | | UO only | |
|---|---|---|---|---|---|---|
|  | Cohort 1 | Cohort 2 | Cohort 1 | Cohort 2 | Cohort 1 | Cohort 2 |
| Median | 5200 | 5720 | 5200 | 31100 | 5620 | 5490 |
| Average | 14300 | 15400 | 16300 | 58800 | 14100 | 14600 |
| Stdev | 24200 | 28400 | 25000 | 67700 | 25600 | 27700 |
| p(t-test) |  | 0.75 |  | 2.6E-4 |  | 0.89 |
| Min | 0.0130 | 389 | 300 | 860 | 0.0130 | 389 |
| Max | 145000 | 173000 | 116000 | 190000 | 180000 | 148000 |
| n (Samp) | 155 | 96 | 51 | 19 | 138 | 83 |
| n (Patient) | 155 | 96 | 51 | 19 | 138 | 83 |

|  | At Enrollment | | |
|---|---|---|---|
|  | sCr or UO | sCr only | UO only |
| AUC | 0.53 | 0.73 | 0.51 |
| SE | 0.038 | 0.072 | 0.040 |
| p | 0.41 | 0.0012 | 0.74 |
| nCohort 1 | 155 | 51 | 138 |
| nCohort 2 | 96 | 19 | 83 |
| Cutoff 1 | 3630 | 9260 | 4360 |
| Sens 1 | 71% | 74% | 71% |
| Spec 1 | 39% | 65% | 42% |
| Cutoff 2 | 2820 | 4640 | 3080 |
| Sens 2 | 80% | 84% | 81% |
| Spec 2 | 28% | 45% | 27% |
| Cutoff 3 | 1600 | 2300 | 1850 |
| Sens 3 | 91% | 95% | 90% |
| Spec 3 | 11% | 29% | 12% |
| Cutoff 4 | 9260 | 12800 | 10500 |
| Sens 4 | 31% | 63% | 24% |
| Spec 4 | 70% | 71% | 70% |
| Cutoff 5 | 19200 | 21900 | 18200 |
| Sens 5 | 19% | 58% | 17% |
| Spec 5 | 80% | 80% | 80% |
| Cutoff 6 | 41300 | 57800 | 35800 |
| Sens 6 | 8% | 32% | 10% |
| Spec 6 | 90% | 90% | 91% |
| OR Quart 2 | 1.7 | 1.5 | 1.6 |
| p Value | 0.16 | 0.68 | 0.24 |
| 95% CI of | 0.81 | 0.22 | 0.73 |
| OR Quart2 | 3.5 | 10 | 3.5 |
| OR Quart 3 | 1.6 | 2.3 | 1.5 |
| p Value | 0.22 | 0.38 | 0.33 |
| 95% CI of | 0.76 | 0.36 | 0.68 |
| OR Quart3 | 3.3 | 15 | 3.2 |
| OR Quart 4 | 1.4 | 9.4 | 0.97 |
| p Value | 0.38 | 0.012 | 0.95 |
| 95% CI of | 0.66 | 1.6 | 0.44 |
| OR Quart4 | 2.9 | 54 | 2.2 |

**Fibroblast growth factor 19**

|  | sCr or UO | | sCr only | | UO only | |
| --- | --- | --- | --- | --- | --- | --- |
|  | Cohort 1 | Cohort 2 | Cohort 1 | Cohort 2 | Cohort 1 | Cohort 2 |
| Median | 3.21E-5 | 2.68E-5 | 4.39E-5 | 2.68E-5 | 2.83E-5 | 2.83E-5 |
| Average | 0.00453 | 0.00701 | 0.000785 | 0.00265 | 0.00506 | 0.00880 |
| Stdev | 0.0421 | 0.0426 | 0.00286 | 0.00580 | 0.0445 | 0.0466 |
| p(t-test) |  | 0.68 |  | 0.10 |  | 0.58 |
| Min | 5.62E-6 | 5.62E-6 | 5.62E-6 | 1.66E-5 | 5.62E-6 | 5.62E-6 |
| Max | 0.476 | 0.388 | 0.0164 | 0.0157 | 0.476 | 0.388 |
| n (Samp) | 128 | 85 | 41 | 18 | 115 | 73 |
| n (Patient) | 128 | 85 | 41 | 18 | 115 | 73 |

|  | At Enrollment | | |
| --- | --- | --- | --- |
|  | sCr or UO | sCr only | UO only |
| AUC | 0.48 | 0.55 | 0.49 |
| SE | 0.041 | 0.083 | 0.043 |
| p | 0.54 | 0.51 | 0.84 |
| nCohort 1 | 128 | 41 | 115 |
| nCohort 2 | 85 | 18 | 73 |
| Cutoff 1 | 1.92E-5 | 2.53E-5 | 2.17E-5 |
| Sens 1 | 75% | 72% | 71% |
| Spec 1 | 23% | 37% | 29% |
| Cutoff 2 | 1.82E-5 | 2.17E-5 | 1.82E-5 |
| Sens 2 | 86% | 83% | 85% |
| Spec 2 | 13% | 32% | 15% |
| Cutoff 3 | 1.66E-5 | 1.82E-5 | 1.66E-5 |
| Sens 3 | 91% | 94% | 90% |
| Spec 3 | 10% | 10% | 11% |
| Cutoff 4 | 0.000114 | 8.63E-5 | 8.63E-5 |
| Sens 4 | 21% | 39% | 23% |
| Spec 4 | 82% | 71% | 72% |
| Cutoff 5 | 0.000114 | 0.000114 | 0.000114 |
| Sens 5 | 21% | 33% | 19% |
| Spec 5 | 82% | 83% | 83% |
| Cutoff 6 | 0.00127 | 0.000197 | 0.000197 |
| Sens 6 | 15% | 22% | 18% |
| Spec 6 | 91% | 90% | 90% |
| OR Quart 2 | 0.69 | 6.9 | 1.6 |
| p Value | 0.35 | 0.037 | 0.29 |

(continued)

|  | At Enrollment | | |
|---|---|---|---|
|  | sCr or UO | sCr only | UO only |
| 95% CI of | 0.31 | 1.1 | 0.68 |
| OR Quart2 | 1.5 | 42 | 3.6 |
| OR Quart 3 | 1.2 | 0.92 | 1.2 |
| p Value | 0.64 | 0.94 | 0.67 |
| 95% CI of | 0.56 | 0.11 | 0.52 |
| OR Quart3 | 2.6 | 7.6 | 2.8 |
| OR Quart 4 | 1.0 | 4.0 | 1.2 |
| p Value | 0.94 | 0.14 | 0.67 |
| 95% CI of | 0.48 | 0.65 | 0.52 |
| OR Quart4 | 2.2 | 25 | 2.8 |

**Fibroblast growth factor 21**

|  | sCr or UO | | sCr only | | UO only | |
|---|---|---|---|---|---|---|
|  | Cohort 1 | Cohort 2 | Cohort 1 | Cohort 2 | Cohort 1 | Cohort 2 |
| Median | 0.0125 | 0.0121 | 0.0255 | 0.0979 | 0.0100 | 0.0112 |
| Average | 0.122 | 0.193 | 0.147 | 0.432 | 0.132 | 0.180 |
| Stdev | 0.413 | 0.614 | 0.477 | 0.837 | 0.443 | 0.580 |
| p(t-test) |  | 0.31 |  | 0.10 |  | 0.52 |
| Min | 1.40E-9 | 1.40E-9 | 4.16E-6 | 0.00126 | 1.40E-9 | 1.40E-9 |
| Max | 2.88 | 3.58 | 2.88 | 3.42 | 3.07 | 3.58 |
| n (Samp) | 128 | 85 | 41 | 18 | 115 | 73 |
| n (Patient) | 128 | 85 | 41 | 18 | 115 | 73 |

|  | At Enrollment | | |
|---|---|---|---|
|  | sCr or UO | sCr only | UO only |
| AUC | 0.52 | 0.70 | 0.52 |
| SE | 0.041 | 0.078 | 0.043 |
| p | 0.58 | 0.012 | 0.70 |
| nCohort 1 | 128 | 41 | 115 |
| nCohort 2 | 85 | 18 | 73 |
| Cutoff 1 | 0.00522 | 0.0330 | 0.00432 |
| Sens 1 | 71% | 72% | 71% |
| Spec 1 | 30% | 63% | 28% |
| Cutoff 2 | 0.00355 | 0.0192 | 0.00311 |

(continued)

|  | At Enrollment | | |
|---|---|---|---|
|  | sCr or UO | sCr only | UO only |
| Sens 2 | 80% | 83% | 81% |
| Spec 2 | 24% | 46% | 23% |
| Cutoff 3 | 0.00187 | 0.00324 | 0.00124 |
| Sens 3 | 91% | 94% | 90% |
| Spec 3 | 16% | 12% | 10% |
| Cutoff 4 | 0.0297 | 0.0419 | 0.0263 |
| Sens 4 | 34% | 61% | 34% |
| Spec 4 | 70% | 71% | 70% |
| Cutoff 5 | 0.0509 | 0.0696 | 0.0386 |
| Sens 5 | 24% | 50% | 26% |
| Spec 5 | 80% | 80% | 80% |
| Cutoff 6 | 0.145 | 0.135 | 0.256 |
| Sens 6 | 16% | 44% | 14% |
| Spec 6 | 91% | 90% | 90% |
| OR Quart 2 | 1.0 | 1.5 | 0.84 |
| p Value | 1.0 | 0.69 | 0.67 |
| 95% CI of | 0.46 | 0.21 | 0.36 |
| OR Quart2 | 2.2 | 11 | 1.9 |
| OR Quart 3 | 0.92 | 2.2 | 0.84 |
| p Value | 0.84 | 0.42 | 0.67 |
| 95% CI of | 0.42 | 0.33 | 0.36 |
| OR Quart3 | 2.0 | 14 | 1.9 |
| OR Quart 4 | 1.1 | 9.0 | 1.1 |
| p Value | 0.75 | 0.018 | 0.83 |
| 95% CI of | 0.52 | 1.5 | 0.48 |
| OR Quart4 | 2.4 | 55 | 2.5 |

Fig. 4: Comparison of the maximum marker levels in urine samples collected from Cohort 1 (patients that did not progress beyond RIFLE stage 0) and the maximum values in urine samples collected from subjects between enrollment and 0, 24 hours, and 48 hours prior to reaching stage F in Cohort 2.

**Angiogenin**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 6570 | 20400 | 6570 | 20400 | 6570 | 20400 |
| Average | 9580 | 18900 | 9580 | 17700 | 9580 | 17200 |

(continued)

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Stdev | 8320 | 9570 | 8320 | 10100 | 8320 | 9450 |
| p(t-test) | | 5.9E-9 | | 6.4E-7 | | 8.5E-5 |
| Min | 77.7 | 647 | 77.7 | 647 | 77.7 | 1090 |
| Max | 30600 | 30600 | 30600 | 30600 | 30600 | 30600 |
| n (Samp) | 203 | 36 | 203 | 34 | 203 | 22 |
| n (Patient) | 203 | 36 | 203 | 34 | 203 | 22 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 11200 | 22300 | 11200 | 21100 | 11200 | 20400 |
| Average | 13000 | 21900 | 13000 | 20600 | 13000 | 19400 |
| Stdev | 9540 | 8480 | 9540 | 9460 | 9540 | 8650 |
| p(t-test) | | 5.9E-5 | | 6.3E-4 | | 0.011 |
| Min | 0.00873 | 647 | 0.00873 | 647 | 0.00873 | 1820 |
| Max | 30600 | 30600 | 30600 | 30600 | 30600 | 30600 |
| n (Samp) | 395 | 20 | 395 | 20 | 395 | 15 |
| n (Patient) | 395 | 20 | 395 | 20 | 395 | 15 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 8170 | 20400 | 8170 | 20400 | 8170 | 20400 |
| Average | 10900 | 18000 | 10900 | 17200 | 10900 | 17600 |
| Stdev | 8900 | 9830 | 8900 | 10200 | 8900 | 10400 |
| p(t-test) | | 5.0E-4 | | 0.0029 | | 0.0090 |
| Min | 77.7 | 2260 | 77.7 | 1060 | 77.7 | 1090 |
| Max | 30600 | 30600 | 30600 | 30600 | 30600 | 30600 |
| n (Samp) | 215 | 22 | 215 | 20 | 215 | 13 |
| n (Patient) | 215 | 22 | 215 | 20 1215 | | 13 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.76 | 0.76 | 0.71 | 0.72 | 0.72 | 0.68 | 0.73 | 0.70 | 0.69 |
| SE | 0.048 | 0.064 | 0.064 | 0.052 | 0.066 | 0.068 | 0.064 | 0.077 | 0.084 |
| p | 4.7E-8 | 6.2E-5 | 0.0013 | 1.9E-5 | 0.0011 | 0.010 | 3.8E-4 | 0.011 | 0.023 |
| nCohort 1 | 203 | 395 | 215 | 203 | 395 | 215 | 203 | 395 | 215 |
| nCohort 2 | 36 | 20 | 22 | 34 | 20 | 20 | 22 | 15 | 13 |
| Cutoff 1 | 14600 | 20300 | 10900 | 11000 | 20300 | 10900 | 10000 | 15600 | 10000 |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Sens 1 | 72% | 80% | 73% | 71% | 75% | 70% | 73% | 73% | 77% |
| Spec 1 | 74% | 68% | 60% | 65% | 68% | 60% | 63% | 59% | 58% |
| Cutoff 2 | 9130 | 20300 | 5040 | 5010 | 15900 | 5040 | 7710 | 11700 | 5040 |
| Sens 2 | 81% | 80% | 82% | 82% | 80% | 80% | 82% | 80% | 85% |
| Spec 2 | 61% | 68% | 39% | 44% | 60% | 39% | 56% | 52% | 39% |
| Cutoff 3 | 3680 | 9330 | 3680 | 2230 | 6050 | 3010 | 3010 | 7710 | 3010 |
| Sens 3 | 92% | 90% | 91% | 91% | 90% | 90% | 91% | 93% | 92% |
| Spec 3 | 33% | 46% | 29% | 19% | 35% | 21% | 25% | 41% | 21% |
| Cutoff 4 | 12900 | 20400 | 16200 | 12900 | 20400 | 16200 | 12900 | 20400 | 16200 |
| Sens 4 | 72% | 55% | 59% | 68% | 50% | 60% | 64% | 40% | 62% |
| Spec 4 | 70% | 79% | 70% | 70% | 79% | 70% | 70% | 79% | 70% |
| Cutoff 5 | 20000 | 20900 | 20400 | 20000 | 20900 | 20400 | 20000 | 20900 | 20400 |
| Sens 5 | 64% | 55% | 36% | 59% | 50% | 35% | 59% | 40% | 31% |
| Spec 5 | 80% | 80% | 87% | 80% | 80% | 87% | 80% | 80% | 87% |
| Cutoff 6 | 20400 | 27700 | 24000 | 20400 | 27700 | 24000 | 20400 | 27700 | 24000 |
| Sens 6 | 42% | 35% | 27% | 38% | 30% | 25% | 32% | 20% | 31% |
| Spec 6 | 90% | 90% | 90% | 90% | 90% | 90% | 90% | 90% | 90% |
| OR Quart | 1.3 | 2.0 | 1.5 | 0.58 | 0.99 | 0.23 | 0.32 | 2.0 | 0.49 |
| 2 | 0.71 | 0.57 | 0.65 | 0.47 | 0.99 | 0.20 | 0.33 | 0.57 | 0.57 |
| p Value | 0.29 | 0,18 | 0.25 | 0.13 | 0.14 | 0.025 | 0.032 | 0.18 | 0.043 |
| 95% CI of OR Quart2 | 6.2 | 22 | 9.5 | 2.5 | 7.2 | 2.1 | 3.2 | 22 | 5.6 |
| OR Quart. | 2.9 | 6.2 | 2.6 | 1.2 | 3.1 | 0.98 | 1.7 | 6.3 | 1.0 |
| 3 | 0.13 | 0.093 | 0.26 | 0.75 | 0.17 | 0.98 | 0.47 | 0.091 | 1.0 |
| p Value | 0.72 | 0.74 | 0.49 | 0.35 | 0.61 | 0.23 | 0,39 | 0.75 | 0.14 |
| 95% Clof OR Quart3 | 11 | 53 | 14 | 4.2 | 16 | 4.1 | 7.6 | 53 | 7.4 |
| OR Quart | 10 | 12 | 7.1 | 5.4 | 5.4 | 3.1 | 5.2 | 6.2 | 4.5 |
| 4 | 4.0E-4 | 0.018 | 0.013 | 0.0019 | 0.033 | 0.067 | 0.014 | 0.093 | 0.065 |
| p Value | 2.8 | 1.5 | 1.5 | 1.9 | 1.1 | 0.92 | 1.4 | 0.74 | 0.91 |
| 95% CI of OR Quart4 | 36 | 95 | 33 | 16 | 25 | 10 | 19 | 53 | 22 |

**Angiopoietin-related protein 4**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 16.9 | 49.1 | 16.9 | 29.8 | 16.9 | 18.9 |

(continued)

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Average | 56.0 | 143 | 56.0 | 139 | 56.0 | 108 |
| Stdev | 99.2 | 195 | 99.2 | 202 | 99.2 | 221 |
| p(t-test) | | 3.0E-4 | | 7.6E-4 | | 0.079 |
| Min | 0.00423 | 4.54 | 0.00423 | 4.54 | 0.00423 | 6.66 |
| Max | 538 | 878 | 538 | 878 | 538 | 878 |
| n (Samp) | 151 | 32 | 151 | 30 | 151 | 18 |
| n (Patient) | 151 | 32 | 151 | 30 | 151 | 18 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 21.3 | 37.6 | 21.3 | 30.7 | 21.3 | 21.5 |
| Average | 74.3 | 160 | 74.3 | 155 | 74.3 | 137 |
| Stdev | 157 | 230 | 157 | 229 | 157 | 255 |
| p(t-test) | | 0.030 | | 0.041 | | 0.17 |
| Min | 0.00423 | 14.1 | 0.00423 | 14.1 | 0.00423 | 9.39 |
| Max | 1370 | 878 | 1370 | 878 | 1370 | 878 |
| n (Samp) | 317 | 18 | 317 | 18 | 317 | 13 |
| n (Patient) | 317 | 18 | 317 | 18 | 317 | 13 |
| UO only | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 19.2 | 36.5 | 19.2 | 19.8 | 19.2 | 12.0 |
| Average | 64.1 | 96.0 | 64.1 | 89.8 | 64.1 | 25.4 |
| Stdev | 109 | 130 | 109 | 139 | 109 | 33.3 |
| p(t-test) | | 0.24 | | 0.37 | | 0.27 |
| Min | 0.00423 | 4.54 | 0.00423 | 4.54 | 0.00423 | 6.66 |
| Max | 624 | 400 | 624 | 400 | 624 | 116 |
| n (Samp) | 172 | 19 | 172 | 17 | 172 | 10 |
| n (Patient) | 172 | 19 | 172 | 17 | 172 | 10 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.71 | 0.68 | 0.63 | 0.67 | 0.67 | 0.57 | 0.57 | 0.58 | 0.42 |
| SE | 0.055 | 0.071 | 0.072 | 0.058 | 0.072 | 0.075 | 0.074 | 0.084 | 0.097 |
| p | 1.4E-4 | 0.011 | 0.076 | 0.0027 | 0.021 | 0.35 | 0.35 | 0.34 | 0.41 |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| nCohort 1 | 151 | 317 | 172 | 151 | 317 | 172 | 151 | 317 | 172 |
| nCohort 2 | 32 | 18 | 19 | 30 | 18 | 17 | 18 | 13 | 10 |
| Cutoff 1 | 21.0 | 21.5 | 16.6 | 17.8 | 20.7 | 16.2 | 13.7 | 16.6 | 10.6 |
| Sens 1 | 72% | 72% | 74% | 70% | 72% | 71% | 72% | 77% | 70% |
| Spec 1 | 56% | 50% | 46% | 52% | 48% | 44% | 44% | 42% | 28% |
| Cutoff 2 | 16.9 | 17.8 | 12.5 | 16.6 | 16.9 | 11.4 | 11.2 | 16.2 | 9.29 |
| Sens 2 | 81% | 83% | 84% | 80% | 83% | 82% | 83% | 85% | 80% |
| Spec 2 | 50% | 44% | 38% | 49% | 43% | 33% | 34% | 41% | 26% |
| Cutoff 3 | 13.7 | 16.2 | 10.6 | 12.5 | 16.2 | 10.6 | 9.06 | 14.0 | 9.06 |
| Sens 3 | 91% | 94% | 95% | 90% | 94% | 94% | 94% | 92% | 90% |
| Spec 3 | 44% | 41% | 28% | 42% | 41% | 28% | 29% | 37% | 26% |
| Cutoff 4 | 35.6 | 39.5 | 48.2 | 35.6 | 39.5 | 48.2 | 35.6 | 39.5 | 48.2 |
| Sens 4 | 56% | 50% | 47% | 50% | 44% | 29% | 28% | 31% | 10% |
| Spec 4 | 70% | 70% | 70% | 70% | 70% | 70% | 70% | 70% | 70% |
| Cutoff 5 | 76.7 | 76.7 | 84.1 | 76.7 | 76.7 | 84.1 | 76.7 | 76.7 | 84.1 |
| Sens 5 | 41% | 33% | 32% | 33% | 33% | 24% | 22% | 23% | 10% |
| Spec 5 | 80% | 80% | 80% | 80% | 80% | 80% | 80% | 80% | 80% |
| Cutoff 6 | 151 | 207 | 170 | 151 | 207 | 170 | 151 | 207 | 170 |
| Sens 6 | 28% | 33% | 16% | 30% | 28% | 18% | 17% | 23% | 0% |
| Spec 6 | 90% | 90% | 90% | 90% | 90% | 90% | 90% | 90% | 90% |
| OR Quart 2 | 7.9 | >6.4 | 6.6 | 11 | >7.5 | 8.0 | 8.2 | 5.2 | 2.1 |
| | 0.058 | <0.089 | 0.087 | 0.026 | <0.061 | 0.056 | 0.054 | 0.14 | 0.55 |
| p Value | 0.93 | >0.75 | 0.76 | 1.3 | >0.91 | 0.95 | 0.96 | 0.59 | 0.18 |
| 95% CI of OR Quart2 | 67 | na | 57 | 91 | na | 68 | 70 | 45 | 24 |
| OR Quart 3 | 14 | >4.2 | 5.3 | 13 | >4.2 | 5.5 | 6.8 | 4.2 | 6.8 |
| | 0.014 | <0.21 | 0.13 | 0.018 | <0.21 | 0.13 | 0.082 | 0.21 | 0.083 |
| p Value | 1.7 | >0.45 | 0.60 | 1.5 | >0.45 | 0.61 | 0.79 | 0.45 | 0.78 |
| 95% CI of OR Quart3 | 110 | na | 48 | 100 | na | 49 | 59 | 38 | 58 |
| OR Quart 4 | 17 | >8.7 | 7.9 | 12 | >7.5 | 4.2 | 4.2 | 3.0 | 1.0 |
| | 0.0073 | <0.043 | 0.059 | 0.020 | <0.061 | 0.21 | 0.21 | 0.34 | 0.99 |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| p Value | 2.2 | >1.1 | 0.93 | 1.5 | >0.91 | 0.45 | 0.45 | 0.31 | 0.062 |
| 95% CI of OR Quart4 | 140 | na | 67 | 100 | na | 39 | 39 | 30 | 17 |

**Angiopoietin-related protein 6**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.292 | 0.543 | 0.292 | 0.257 | 0.292 | 0.183 |
| Average | 1.72 | 2.40 | 1.72 | 2.14 | 1.72 | 1.06 |
| Stdev | 3.60 | 4.44 | 3.60 | 4.57 | 3.60 | 1.92 |
| p(t-test) | | 0.38 | | 0.60 | | 0.47 |
| Min | 0.000122 | 0.000364 | 0.000122 | 0.000157 | 0.000122 | 0.000206 |
| Max | 25.9 | 22.4 | 25.9 | 22.4 | 25.9 | 6.31 |
| n (Samp) | 141 | 29 | 141 | 27 | 141 | 16 |
| n (Patient) | 141 | 29 | 141 | 27 | 141 | 16 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.421 | 0.399 | 0.421 | 0.399 | 0.421 | 0.218 |
| Average | 2.19 | 1.75 | 2.19 | 1.55 | 2.19 | 1.39 |
| Stdev | 5.66 | 2.25 | 5.66 | 2.20 | 5.66 | 2.13 |
| p(t-test) | 0.76 | 0.76 | | 0.66 | | 0.63 |
| Min | 0.000122 | 0.000364 | 0.000122 | 0.000364 | 0.000122 | 0.000364 |
| Max | 70.5 | 6.31 | 70.5 | 6.31 | 70.5 | 6.31 |
| n (Samp) | 292 | 16 | 292 | 16 | 292 | 12 |
| n (Patient) | 292 | 16 | 292 | 16 | 292 | 12 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.360 | 0.282 | 0.360 | 0.210 | 0.360 | 0.00357 |
| Average | 1.80 | 2.56 | 1.80 | 2.33 | 1.80 | 0.178 |
| Stdev | 3.47 | 5.39 | 3.47 | 5.68 | 3.47 | 0.269 |
| p(t-test) | | 0.41 | | 0.59 | | 0.16 |

(continued)

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Min | 0.000122 | 0.000364 | 0.000122 | 0.000157 | 0.000122 | 0.000206 |
| Max | 25.9 | 22.4 | 25.9 | 22.4 | 25.9 | 0.802 |
| n (Samp) | 162 | 18 | 162 | 16 | 162 | 9 |
| n (Patient) | 162 | 18 | 162 | 16 | 162 | 9 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.59 | 0.55 | 0.52 | 0.53 | 0.52 | 0.45 | 0.45 | 0.48 | 0.30 |
| SE | 0.060 | 0.076 | 0.073 | 0.061 | 0.075 | 0.077 | 0.078 | 0.086 | 0.100 |
| p | 0.15 | 0.48 | 0.74 | 0.68 | 0.75 | 0.50 | 0.50 | 0.84 | 0.047 |
| nCohort 1 | 141 | 292 | 162 | 141 | 292 | 162 | 141 | 292 | 162 |
| nCohort 2 | 29 | 16 | 18 | 27 | 16 | 16 | 16 | 12 | 9 |
| Cutoff 1 | 0.153 | 0.153 | 0.114 | 0.111 | 0.153 | 0.00224 | 0.00224 | 0.0480 | 0.00224 |
| Sens 1 | 72% | 75% | 72% | 70% | 75% | 88% | 81% | 75% | 78% |
| Spec 1 | 41% | 36% | 32% | 38% | 36% | 19% | 19% | 31% | 19% |
| Cutoff 2 | 0.0628 | 0.119 | 0.00224 | 0.00224 | 0.0480 | 0.00224 | 0.00224 | 0.00224 | 0.000336 |
| Sens 2 | 83% | 81% | 94% | 89% | 81% | 88% | 81% | 83% | 89% |
| Spec 2 | 35% | 34% | 19% | 19% | 31% | 19% | 19% | 19% | 10% |
| Cutoff 3 | 0.00224 | 0.00224 | 0.00224 | 0.000336 | 0.000336 | 0.000336 | 0.000336 | 0.000336 | 0.000205 |
| Sens 3 | 97% | 94% | 94% | 96% | 100% | 94% | 94% | 100% | 100% |
| Spec 3 | 19% | 19% | 19% | 11% | 10% | 10% | 11% | 10% | 1% |
| Cutoff 4 | 1.28 | 1.25 | 1.45 | 1.28 | 1.25 | 1.45 | 1.28 | 1.25 | 1.45 |
| Sens 4 | 34% | 38% | 33% | 26% | 31% | 25% | 19% | 33% | 0% |
| Spec 4 | 70% | 70% | 70% | 70% | 70% | 70% | 70% | 70% | 70% |
| Cutoff 5 | 2.60 | 2.44 | 2.67 | 2.60 | 2.44 | 2.67 | 2.60 | 2.44 | 2.67 |
| Sens 5 | 31% | 31% | 28% | 26% | 25% | 25% | 19% | 25% | 0% |
| Spec 5 | 80% | 80% | 80% | 80% | 80% | 80% | 80% | 80% | 80% |
| Cutoff 6 | 4.06 | 5.79 | 4.30 | 4.06 | 5.79 | 4.30 | 4.06 | 5.79 | 4.30 |
| Sens 6 | 21% | 6% | 17% | 19% | 6% | 12% | 12% | 8% | 0% |
| Spec 6 | 90% | 90% | 90% | 90% | 90% | 90% | 90% | 90% | 90% |
| OR Quart | 2.0 | 3.2 | 1.6 | 1.0 | 1.7 | 0.24 | 1.0 | 0.66 | >2.1 |
| 2 | 0.27 | 0.17 | 0.50 | 1.0 | 0.47 | 0.21 | 0.97 | 0.65 | <0.55 |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| p Value | 0.60 | 0.62 | 0.41 | 0.32 | 0.39 | 0.026 | 0.19 | 0.11 | >0.18 |
| 95% CI of OR Quart2 | 6.4 | 16 | 6.0 | 3.2 | 7.4 | 2.2 | 5.4 | 4.1 | na |
| OR Quart 3 | 1.2 | 1.5 | 0.73 | 0.83 | 1.4 | 1.6 | 1.4 | 1.4 | >3.2 |
| | 0.75 | 0.65 | 0.69 | 0.76 | 0.70 | 0.50 | 0.67 | 0.70 | <0.32 |
| p Value | 0.35 | 0.25 | 0.15 | 0.25 | 0.29 | 0.41 | 0.29 | 0.29 | >0.32 |
| 95% CI OR Quart3 | 4.4 | 9.4 | 3.5 | 2.7 | 6.3 | 6.0 | 6.8 | 6.3 | na |
| OR Quart 4 | 2.0 | 2.6 | 1.3 | 1.0 | 1.4 | 1.3 | 2.2 | 1.0 | >4.5 |
| | 0.27 | 0.26 | 0.73 | 1.0 | 0.70 | 0.70 | 0.28 | 1.0 | <0.19 |
| p Value | 0.60 | 0.49 | 0.32 | 0.32 | 0.29 | 0.33 | 0.52 | 0.20 | >0.48 |
| 95% CI of OR Quart4 | 6.4 | 14 | 5.1 | 3.2 | 6.3 | 5.3 | 9.7 | 5.1 | na |

**Amphiregulin**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 26.7 | 87.0 | 26.7 | 75.5 | 26.7 | 56.9 |
| Average | 60.5 | 383 | 60.5 | 374 | 60.5 | 94.2 |
| Stdev | 127 | 783 | 127 | 799 | 127 | 92.9 |
| p(t-test) | 4.4E-6 | 4.4E-6 | | 1.1E-5 | | 0.29 |
| Min | 0.140 | 12.7 | 0.140 | 12.7 | 0.140 | 23.2 |
| Max | 1310 | 3480 | 1310 | 3480 | 1310 | 364 |
| n (Samp) | 148 | 28 | 148 | 27 | 148 | 17 |
| n (Patient) | 148 | 28 | 148 | 27 | 148 | 17 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 33.6 | 129 | 33.6 | 73.8 | 33.6 | 73.2 |
| Average | 85.0 | 254 | 85.0 | 239 | 85.0 | 114 |
| Stdev | 230 | 424 | 230 | 426 | 230 | 99.8 |
| p(t-test) | | 0.0090 | | 0.017 | | 0.66 |
| Min | 0.140 | 12.7 | 0.140 | 12.7 | 0.140 | 23.2 |
| Max | 2190 | 1710 | 2190 | 1710 | 2190 | 364 |
| n (Samp) | 288 | 15 | 288 | 15 | 288 | 12 |
| n (Patient) | 288 | 15 | 288 | 15 | 288 | 12 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 33.6 | 77.2 | 33.6 | 75.5 | 33.6 | 39.8 |
| Average | 59.7 | 422 | 59.7 | 424 | 59.7 | 67.7 |
| Stdev | 80.1 | 914 | 80.1 | 941 | 80.1 | 79.0 |
| p(t-test) | | 3.1 E-6 | 4.8E-6 | 4.8E-6 | | 0.76 |
| Min | 0.140 | 13.4 | 0.140 | 13.4 | 0.140 | 13.4 |
| Max | 480 | 3480 | 480 | 3480 | 480 | 273 |
| n (Samp) | 152 | 18 | 152 | 17 | 152 | 10 |
| n (Patient) | 152 | 18 | 152 | 17 | 152 | 10 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.80 | 0.77 | 0.73 | 0.79 | 0.76 | 0.72 | 0.74 | 0.76 | 0.57 |
| SE | 0.052 | 0.072 | 0.070 | 0.054 | 0.073 | 0.073 | 0.071 | 0.082 | 0.097 |
| p | 7.1E-9 | 1.5E-4 | 8.2E-4 | 8.8E-8 | 3.3E-4 | 0.0026 | 7.5E-4 | 0.0014 | 0.45 |
| nCohort 1 | 148 | 288 | 152 | 148 | 288 | 152 | 148 | 288 | 152 |
| nCohort 2 | 28 | 15 | 18 | 27 | 15 | 17 | 17 | 12 | 10 |
| Cutoff 1 | 54.4 | 56.0 | 42.9 | 54.4 | 56.0 | 42.9 | 45.3 | 56.0 | 25.6 |
| Sens 1 | 71% | 73% | 72% | 70% | 73% | 71% | 71% | 75% | 70% |
| Spec 1 | 75% | 72% | 57% | 75% | 72% | 57% | 66% | 72% | 41% |
| Cutoff 2 | 40.8 | 40.8 | 33.6 | 40.8 | 40.8 | 33.6 | 33.6 | 40.8 | 24.6 |
| Sens 2 | 82% | 80% | 83% | 81% | 80% | 82% | 82% | 83% | 80% |
| Spec 2 | 62% | 56% | 51% | 62% | 56% | 51% | 57% | 56% | 39% |
| Cutoff 3 | 25.6 | 33.6 | 24.6 | 25.6 | 33.6 | 24.6 | 24.6 | 33.6 | 22.0 |
| Sens 3 | 93% | 93% | 94% | 93% | 93% | 94% | 94% | 92% | 90% |
| Spec 3 | 49% | 50% | 39% | 49% | 50% | 39% | 46% | 50% | 36% |
| Cutoff 4 | 48.2 | 54.5 | 53.4 | 48.2 | 54.5 | 53.4 | 48.2 | 54.5 | 53.4 |
| Sens 4 | 75% | 73% | 61% | 74% | 73% | 59% | 65% | 75% | 30% |
| Spec 4 | 70% | 70% | 70% | 70% | 70% | 70% | 70% | 70% | 70% |
| Cutoff 5 | 64.2 | 72.7 | 74.9 | 64.2 | 72.7 | 74.9 | 64.2 | 72.7 | 74.9 |
| Sens 5 | 64% | 67% | 56% | 63% | 60% | 53% | 47% | 58% | 20% |
| Spec 5 | 80% | 80% | 80% | 80% | 80% | 80% | 80% | 80% | 80% |
| Cutoff 6 | 125 | 133 | 128 | 125 | 133 | 128 | 125 | 133 | 128 |
| Sens 6 | 46% | 47% | 39% | 41% | 40% | 35% | 24% | 25% | 20% |
| Spec 6 | 91% | 90% | 90% | 91% | 90% | 90% | 91% | 90% | 90% |
| OR Quart 2 | 3.1 | 2.0 | 4.2 | 2.0 | 2.0 | 4.3 | >3.2 | >2.1 | 3.1 |
| | 0.33 | 0.57 | 0.21 | 0.58 | 0.57 | 0.20 | <0.32 | <0.56 | 0.34 |
| p Value | 0.31 | 0.18 | 0.45 | 0.17 | 0.18 | 0.46 | >0.32 | >0.18 | 0.31 |
| 95% CI of OR Quart2 | 31 | 23 | 39 | 23 | 23 | 40 | na | na | 31 |
| OR Quart 3 | 8.1 | 2.0 | 3.2 | 9.3 | 2.0 | 3.2 | >7.0 | >2.1 | 4.3 |
| | 0.055 | 0.57 | 0.33 | 0.039 | 0.57 | 0.33 | <0.077 | <0.56 | 0.20 |
| p Value | 0.96 | 0.18 | 0.31 | 1.1 | 0.18 | 0.31 | >0.81 | >0.18 | 0.46 |
| 95% CI of OR Quart3 | 69 | 23 | 32 | 78 | 23 | 32 | na | na | 41 |

(continued)

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| OR Quart 4 | 27 | 11 | 12 | 24 | 11 | 11 | >9.6 | >9.0 | 2.0 |
| p Value | 0.0018 | 0.023 | 0.019 | 0.0027 | 0.023 | 0.027 | <0.037 | <0.041 | 0.58 |
| 95% CI of OR Quart4 | 3.4 | 1.4 | 1.5 | 3.0 | 1.4 | 1.3 | >1.1 | >1.1 | 0.17 |
|  | 220 | 90 | 100 | 190 | 90 | 90 | na | na | 23 |

**Betacellulin**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 1.56 | 2.45 | 1.56 | 2.48 | 1.56 | 2.48 |
| Average | 1.86 | 3.11 | 1.86 | 3.03 | 1.86 | 2.65 |
| Stdev | 2.43 | 2.60 | 2.43 | 2.72 | 2.43 | 1.27 |
| p(t-test) |  | 0.015 |  | 0.025 |  | 0.19 |
| Min | 0.00230 | 0.00240 | 0.00230 | 0.00240 | 0.00230 | 0.00240 |
| Max | 23.1 | 11.6 | 23.1 | 11.6 | 23.1 | 4.42 |
| n (Samp) | 148 | 28 | 148 | 27 | 148 | 17 |
| n (Patient) | 148 | 28 | 148 | 27 | 148 | 17 |

| sCr only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 1.72 | 2.48 | 1.72 | 2.48 | 1.72 | 2.48 |
| Average | 2.22 | 2.80 | 2.22 | 2.77 | 2.22 | 2.59 |
| Stdev | 3.19 | 1.30 | 3.19 | 1.33 | 3.19 | 1.55 |
| p(t-test) |  | 0.49 |  | 0.51 |  | 0.70 |
| Min | 0.00230 | 0.00240 | 0.00230 | 0.00240 | 0.00230 | 0.00240 |
| Max | 28.3 | 4.42 | 28.3 | 4.42 | 28.3 | 4.42 |
| n (Samp) | 288 | 15 | 288 | 15 | 288 | 12 |
| n (Patient) | 288 | 15 | 288 | 15 | 288 | 12 |

| UO only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 1.56 | 2.09 | 1.56 | 2.17 | 1.56 | 2.29 |

(continued)

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Average | 1.76 | 3.26 | 1.76 | 3.18 | 1.76 | 2.60 |
| Stdev | 1.81 | 3.09 | 1.81 | 3.28 | 1.81 | 1.09 |
| p(t-test) | | 0.0027 | | 0.0064 | | 0.15 |
| Min | 0.00230 | 0.00240 | 0.00230 | 0.00240 | 0.00230 | 1.11 |
| Max | 8.77 | 11.6 | 8.77 | 11.6 | 8.77 | 4.42 |
| n (Samp) | 152 | 18 | 152 | 17 | 152 | 10 |
| n (Patient) | 152 | 18 | 152 | 17 | 152 | 10 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.70 | 0.69 | 0.70 | 0.67 | 0.68 | 0.65 | 0.71 | 0.64 | 0.72 |
| SE | 0.058 | 0.078 | 0.072 | 0.061 | 0.078 | 0.075 | 0.073 | 0.088 | 0.094 |
| p | 5.3E-4 | 0.015 | 0.0064 | 0.0050 | 0.020 | 0.040 | 0.0047 | 0.11 | 0.018 |
| nCohort 1 | 148 | 288 | 152 | 148 | 288 | 152 | 148 | 288 | 152 |
| nCohort 2 | 28 | 15 | 18 | 27 | 15 | 17 | 17 | 12 | 10 |
| Cutoff 1 | 1.87 | 1.97 | 1.87 | 1.62 | 1.74 | 1.74 | 1.97 | 1.97 | 1.97 |
| Sens 1 | 71% | 73% | 72% | 70% | 73% | 71% | 71% | 75% | 70% |
| Spec 1 | 64% | 58% | 65% | 57% | 52% | 62% | 66% | 58% | 66% |
| Cutoff 2 | 1.70 | 1.74 | 1.74 | 1.51 | 1.63 | 0.909 | 1.74 | 1.63 | 1.87 |
| Sens 2 | 82% | 80% | 83% | 81% | 80% | 82% | 82% | 83% | 80% |
| Spec 2 | 57% | 52% | 62% | 49% | 49% | 40% | 61% | 49% | 65% |
| Cutoff 3 | 0.761 | 1.51 | 0.761 | 0.0209 | 1.51 | 0.0209 | 1.03 | 0.0209 | 1.74 |
| Sens 3 | 93% | 93% | 94% | 93% | 93% | 94% | 94% | 92% | 90% |
| Spec 3 | 35% | 42% | 36% | 9% | 42% | 13% | 39% | 9% | 62% |
| Cutoff 4 | 2.41 | 2.48 | 2.35 | 2.41 | 2.48 | 2.35 | 2.41 | 2.48 | 2.35 |
| Sens 4 | 54% | 47% | 44% | 56% | 47% | 47% | 59% | 42% | 50% |
| Spec 4 | 71% | 72% | 70% | 71% | 72% | 70% | 71% | 72% | 70% |
| Cutoff 5 | 2.79 | 3.06 | 3.05 | 2.79 | 3.06 | 3.05 | 2.79 | 3.06 | 3.05 |
| Sens 5 | 39% | 47% | 28% | 41% | 47% | 29% | 35% | 42% | 30% |
| Spec 5 | 80% | 80% | 80% | 80% | 80% | 80% | 80% | 80% | 80% |
| Cutoff 6 | 3.77 | 3.96 | 4.06 | 3.77 | 3.96 | 4.06 | 3.77 | 3.96 | 4.06 |
| Sens 6 | 29% | 27% | 22% | 30% | 27% | 24% | 29% | 33% | 20% |
| Spec 6 | 91% | 90% | 90% | 91% | 90% | 90% | 91% | 90% | 90% |
| OR Quart | 1.0 | 2.0 | 0.98 | 0.98 | 3.0 | 1.0 | 1.0 | 0.49 | >1.0 |
| 2 | 1.0 | 0.57 | 0.99 | 0.98 | 0.34 | 1.0 | 1.0 | 0.57 | <1.0 |

(continued)

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| p Value | 0.13 | 0.18 | 0.059 | 0.19 | 0.31 | 0.13 | 0.060 | 0.044 | >0.060 |
| 95% CI OR Quart2 | 7.4 | 23 | 16 | 5.1 | 30 | 7.5 | 17 | 5.6 | na |
| OR Quart 3 | 8.8 | 5.2 | 11 | 3.9 | 4.1 | 3.3 | 9.7 | 2.1 | >5.7 |
|  | 0.0062 | 0.14 | 0.025 | 0.050 | 0.21 | 0.16 | 0.037 | 0.41 | <0.12 |
| p Value | 1.9 | 0.59 | 1.3 | 1.00 | 0.45 | 0.63 | 1.2 | 0.37 | >0.64 |
| 95% CI of 42 OR Quart3 | 42 | 46 | 93 | 15 | 38 | 18 | 82 | 12 | na |
| OR Quart 4 | 7.0 | 7.5 | 8.0 | 4.4 | 7.5 | 3.9 | 8.0 | 2.6 | >4.3 |
|  | 0.015 | 0.062 | 0.058 | 0.031 | 0.062 | 0.10 | 0.057 | 0.26 | <0.20 |
| p Value | 1.5 | 0.90 | 0.94 | 1.1 | 0.90 | 0.76 | 0.94 | 0.49 | >0.46 |
| 95% CI of 34 OR Quart4 | 34 | 63 | 68 | 17 | 63 | 20 | 68 | 14 | na |

**Endostatin**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 6560 | 29400 | 6560 | 19200 | 6560 | 10700 |
| Average | 26500 | 53100 | 26500 | 45900 | 26500 | 38500 |
| Stdev | 42900 | 61100 | 42900 | 57700 | 42900 | 62400 |
| p(t-test) |  | 0.0016 |  | 0.021 |  | 0.24 |
| Min | 362 | 1980 | 362 | 1290 | 362 | 1790 |
| Max | 238000 | 227000 | 238000 | 227000 | 238000 | 227000 |
| n (Samp) | 203 | 36 | 203 | 34 | 203 | 22 |
| n (Patient) | 203 | 36 | 203 | 34 | 203 | 22 |
| sCr only | 0hr prior to AKI stage | | 24hr prior to AKI stage | | 48hr prior to AKI stage | |
|  | Cohort 1 | Cohort 2 | Cohort 1 | Cohort 2 | Cohort 1 | Cohort 2 |
| Median | 8080 | 42800 | 8080 | 33800 | 8080 | 20200 |
| Average | 26200 | 62300 | 26200 | 56700 | 26200 | 45100 |
| Stdev | 39100 | 66000 | 39100 | 66100 | 39100 | 66500 |
| p(t-test) |  | 1.3E-4 |  | 0.0012 |  | 0.077 |
| Min | 0.0130 | 1980 | 0.0130 | 1980 | 0.0130 | 3260 |
| Max | 238000 | 227000 | 238000 | 227000 | 238000 | 227000 |

(continued)

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| n (Samp) | 395 | 20 | 395 | 20 | 395 | 15 |
| n (Patient) | 395 | 20 | 395 | 20 | 395 | 15 |

| UO only | 0hr prior to AKI stage | | 24hr prior to AKI stage | 48hr prior to AKI stage | | |
|---|---|---|---|---|---|---|
| | Cohort 1 | Cohort 2 | Cohort 1 | Cohort 2 | Cohort 1 | Cohort 2 |
| Median | 7540 | 22700 | 7540 | 16300 | 7540 | 7270 |
| Average | 28500 | 51300 | 28500 | 44500 | 28500 | 48600 |
| Stdev | 43600 | 62400 | 43600 | 57400 | 43600 | 70300 |
| p(t-test) | | 0.027 | | 0.13 | | 0.12 |
| Min | 362 | 2950 | 362 | 1290 | 362 | 1790 |
| Max | 238000 | 190000 | 238000 | 178000 | 238000 | 178000 |
| n (Samp) | 215 | 22 | 215 | 20 | 215 | 13 |
| n (Patient) | 215 | 22 | 215 | 20 | 215 | 13 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.69 | 0.70 | 0.66 | 0.66 | 0.67 | 0.62 | 0.58 | 0.63 | 0.55 |
| SE | 0.052 | 0.067 | 0.066 | 0.054 | 0.068 | 0.069 | 0.067 | 0.079 | 0.085 |
| p | 1.9E-4 | 0.0023 | 0.015 | 0.0041 | 0.0097 | 0.085 | 0.20 | 0.11 | 0.56 |
| nCohort 1 | 203 | 395 | 215 | 203 | 395 | 215 | 203 | 395 | 215 |
| nCohort 2 | 36 | 20 | 22 | 34 | 20 | 20 | 22 | 15 | 13 |
| Cutoff 1 | 10500 | 10700 | 7220 | 9350 | 10700 | 7220 | 5500 | 9440 | 4050 |
| Sens 1 | 72% | 70% | 73% | 71% | 70% | 70% | 73% | 73% | 77% |
| Spec 1 | 60% | 57% | 49% | 58% | 57% | 49% | 43% | 54% | 30% |
| Cutoff 2 | 5500 | 9440 | 4870 | 4480 | 5880 | 4480 | 4050 | 5880 | 3410 |
| Sens 2 | 81% | 80% | 82% | 82% | 80% | 80% | 82% | 80% | 85% |
| Spec 2 | 43% | 54% | 35% | 38% | 40% | 35% | 33% | 40% | 25% |
| Cutoff 3 | 4050 | 4410 | 4410 | 3120 | 4410 | 4050 | 3120 | 3410 | 2780 |
| Sens 3 | 92% | 90% | 91% | 91% | 90% | 90% | 91% | 93% | 92% |
| Spec 3 | 33% | 31% | 34% | 26% | 31% | 30% | 26% | 21% | 22% |
| Cutoff 4 | 22300 | 24500 | 26500 | 22300 | 24500 | 26500 | 22300 | 24500 | 26500 |
| Sens 4 | 56% | 60% | 45% | 47% | 55% | 40% | 32% | 40% | 31% |
| Spec 4 | 70% | 70% | 70% | 70% | 70% | 70% | 70% | 70% | 70% |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Cutoff 5 | 43300 | 42100 | 45300 | 43300 | 42100 | 45300 | 43300 | 42100 | 45300 |
| Sens 5 | 42% | 50% | 36% | 38% | 45% | 35% | 23% | 27% | 31% |
| Spec 5 | 80% | 80% | 80% | 80% | 80% | 80% | 80% | 80% | 80% |
| Cutoff 6 | 73500 | 73500 | 89000 | 73500 | 73500 | 89000 | 73500 | 73500 | 89000 |
| Sens 6 | 22% | 30% | 18% | 18% | 25% | 15% | 18% | 20% | 23% |
| Spec 6 | 90% | 90% | 90% | 90% | 90% | 90% | 90% | 90% | 90% |
| OR Quart | 2.1 | 0.99 | 6.6 | 1.6 | 1.5 | 2.6 | 1.7 | 0.99 | 1.4 |
| 2 | 32 | 0.99 | 0.086 | 0.51 | 0.66 | 0.27 | 0.47 | 0.99 | 0.70 |
| p Value | 0.49 | 0.14 | 0.77 | 0.42 | 0.25 | 0.48 | 0.39 | 0.14 | 0.29 |
| 95% CI of OR Quart2 | 8.7 | 7.2 | 56 | 5.8 | 9.2 | 14 | 7.6 | 7.2 | 6.4 |
| OR Quart | 4.7 | 2.6 | 7.8 | 2.8 | 2.6 | 3.2 | 2.9 | 3.7 | 0.65 |
| 3 | 0.022 | 0.27 | 0.058 | 0.098 | 0.27 | 0.17 | 0.13 | 0.11 | 0.65 |
| p Value | 1.2 | 0.48 | 0.93 | 0.83 | 0.48 | 0.61 | 0.74 | 0.75 | 0.11 |
| 95% CI of OR Quart3 | 18 | 13 | 66 | 9.5 | 13 | 16 | 12 | 18 | 4.1 |
| OR Quart | 6.2 | 6.0 | 8.9 | 4.2 | 5.4 | 3.8 | 2.1 | 2.0 | 1.4 |
| 4 | 0.0059 | 0.022 | 0.042 | 0.017 | 0.033 | 0.11 | 0.32 | 0.42 | 0.70 |
| p Value | 1.7 | 1.3 | 1.1 | 1.3 | 1.1 | 0.75 | 0.49 | 0.36 | 0.29 |
| 95% CI of OR Quart4 | 23 | 28 | 74 | 14 | 25 | 19 | 8.8 | 11 | 6.4 |

**Proepiregulin**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 3.70 | 10.5 | 3.70 | 9.92 | 3.70 | 5.43 |
| Average | 5.74 | 15.9 | 5.74 | 15.2 | 5.74 | 9.29 |
| Stdev | 6.51 | 18.4 | 6.51 | 18.7 | 6.51 | 11.5 |
| p(t-test) | | 4.4E-7 | | 3.7E-6 | | 0.054 |
| Min | 0.123 | 0.945 | 0.123 | 0.945 | 0.123 | 0.376 |

(continued)

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Max | 46.9 | 81.9 | 46.9 | 81.9 | 46.9 | 49.4 |
| n (Samp) | 147 | 28 | 147 | 27 | 147 | 17 |
| n (Patient) | 147 | 28 | 147 | 27 | 147 | 17 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 4.85 | 9.92 | 4.85 | 9.33 | 4.85 | 7.09 |
| Average | 9.22 | 15.2 | 9.22 | 14.6 | 9.22 | 11.9 |
| Stdev | 19.0 | 16.3 | 19.0 | 16.4 | 19.0 | 12.8 |
| p(t-test) | | 0.23 | | 0.29 | | 0.62 |
| Min | 0.123 | 1.42 | 0.123 | 1.42 | 0.123 | 3.19 |
| Max | 279 | 57.1 | 279 | 57.1 | 279 | 49.4 |
| n (Samp) | 285 | 15 | 285 | 15 | 285 | 12 |
| n (Patient) | 285 | 15 | 285 | 15 | 285 | 12 |
| UO only | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 4.04 | 9.86 | 4.04 | 9.33 | 4.04 | 3.50 |
| Average | 6.57 | 13.9 | 6.57 | 13.2 | 6.57 | 4.19 |
| Stdev | 7.96 | 18.5 | 7.96 | 18.8 | 7.96 | 2.96 |
| p(t-test) | | 0.0025 | | 0.0077 | | 0.35 |
| Min | 0.123 | 0.945 | 0.123 | 0.945 | 0.123 | 0.376 |
| Max | 49.4 | 81.9 | 49.4 | 81.9 | 49.4 | 10.5 |
| n (Samp) | 151 | 18 | 151 | 17 | 151 | 10 |
| n (Patient) | 151 | 18 | 151 | 17 | 151 | 10 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.75 | 0.69 | 0.68 | 0.74 | 0.67 | 0.66 | 0.64 | 0.66 | 0.44 |
| SE | 0.056 | 0.078 | 0.073 | 0.058 | 0.078 | 0.075 | 0.076 | 0.087 | 0.097 |
| p | 5.5E-6 | 0.014 | 0.014 | 3.8E-5 | 0.026 | 0.030 | 0.070 | 0.062 | 0.54 |
| nCohort 1 | 147 | 285 | 151 | 147 | 285 | 151 | 147 | 285 | 151 |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| nCohort 2 | 28 | 15 | 18 | 27 | 15 | 17 | 17 | 12 | 10 |
| Cutoff 1 | 5.34 | 6.73 | 3.80 | 5.34 | 6.73 | 3.80 | 4.51 | 5.34 | 2.31 |
| Sens 1 | 71% | 73% | 72% | 70% | 73% | 71% | 71% | 75% | 70% |
| Spec 1 | 65% | 61% | 49% | 65% | 61% | 49% | 59% | 54% | 27% |
| Cutoff 2 | 3.80 | 5.34 | 2.97 | 3.80 | 5.34 | 2.97 | 3.15 | 4.91 | 2.16 |
| Sens 2 | 82% | 80% | 83% | 81% | 80% | 82% | 82% | 83% | 80% |
| Spec 2 | 54% | 54% | 38% | 54% | 54% | 38% | 41% | 51% | 26% |
| Cutoff 3 | 2.16 | 3.15 | 2.16 | 2.16 | 3.15 | 2.16 | 1.99 | 4.60 | 1.99 |
| Sens 3 | 93% | 93% | 94% | 93% | 93% | 94% | 94% | 92% | 90% |
| Spec 3 | 30% | 36% | 26% | 30% | 36% | 26% | 27% | 49% | 25% |
| Cutoff 4 | 5.99 | 9.07 | 6.30 | 5.99 | 9.07 | 6.30 | 5.99 | 9.07 | 6.30 |
| Sens 4 | 68% | 60% | 61% | 67% | 53% | 59% | 47% | 42% | 20% |
| Spec 4 | 70% | 70% | 70% | 70% | 70% | 70% | 70% | 70% | 70% |
| Cutoff 5 | 8.29 | 12.5 | 8.53 | 8.29 | 12.5 | 8.53 | 8.29 | 12.5 | 8.53 |
| Sens 5 | 61% | 33% | 56% | 56% | 27% | 53% | 29% | 25% | 10% |
| Spec 5 | 80% | 80% | 80% | 80% | 80% | 80% | 80% | 80% | 80% |
| Cutoff 6 | 13.0 | 19.4 | 13.6 | 13.0 | 19.4 | 13.6 | 13.0 | 19.4 | 13.6 |
| Sens 6 | 36% | 20% | 28% | 30% | 20% | 24% | 18% | 17% | 0% |
| Spec 6 | 90% | 90% | 91% | 90% | 90% | 91% | 90% | 90% | 91% |
| OR Quart 2 | 2.0 | 2.0 | 2.1 | 2.0 | 2.0 | 2.1 | 3.2 | >3.1 | 1.0 |
| | 0.42 | 0.57 | 0.41 | 0.42 | 0.57 | 0.41 | 0.33 | <0.33 | 0.98 |
| p Value | 0.36 | 0.18 | 0.36 | 0.36 | 0.18 | 0.36 | 0.31 | >0.32 | 0.14 |
| 95% CI ot OR Quart2 | 12 | 23 | 12 | 12 | 23 | 12 | 32 | na | 7.7 |
| OR Quart 3 | 2.6 | 6.4 | 1.0 | 3.3 | 7.6 | 1.0 | 8.2 | >6.5 | 2.2 |
| | 0.26 | 0.089 | 1.0 | 0.16 | 0.061 | 1.0 | 0.054 | <0.086 | 0.39 |
| p Value | 0.48 | 0.76 | 0.13 | 0.63 | 0.91 | 0.13 | 0.96 | >0.77 | 0.37 |
| 95% CI of OR Quart3 | 14 | 55 | 7.5 | 17 | 64 | 7.5 | 70 | na | 13 |
| OR Quart 4 | 13 | 6.4 | 6.1 | 11 | 5.3 | 5.5 | 6.9 | >3.1 | 1.0 |
| | 0.0012 | 0.089 | 0.026 | 0.0028 | 0.13 | 0.038 | 0.081 | <0.33 | 0.98 |
| p Value | 2.8 | 0.76 | 1.2 | 2.3 | 0.60 | 1.1 | 0.79 | >0.31 | 0.14 |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| 95% CI of OR Quart4 | 60 | 55 | 30 | 50 | 46 | 27 | 60 | na | 7.7 |

**Fibroblast growth factor 19**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.000114 | 0.000197 | 0.000114 | 0.000197 | 0.000114 | 0.000732 |
| Average | 0.0104 | 0.108 | 0.0104 | 0.0505 | 0.0104 | 0.0493 |
| Stdev | 0.0383 | 0.260 | 0.0383 | 0.143 | 0.0383 | 0.138 |
| p(t-test) | | 1.6E-5 | | 0.0032 | | 0.0070 |
| Min | 5.62E-6 | 1.88E-5 | 5.62E-6 | 1.66E-5 | 5.62E-6 | 1.66E-5 |
| Max | 0.260 | 1.02 | 0.260 | 0.560 | 0.260 | 0.560 |
| n (Samp) | 151 | 32 | 151 | 30 | 151 | 18 |
| n (Patient) | 151 | 32 | 151 | 30 | 151 | 18 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.000114 | 0.00341 | 0.000114 | 0.000732 | 0.000114 | 0.00127 |
| Average | 0.0111 | 0.159 | 0.0111 | 0.0551 | 0.0111 | 0.0642 |
| Stdev | 0.0451 | 0.324 | 0.0451 | 0.151 | 0.0451 | 0.162 |
| p(t-test) | | 5.1E-12 | | 0.0012 | | 5.8E-4 |
| Min | 5.62E-6 | 2.96E-5 | 5.62E-6 | 1.66E-5 | 5.62E-6 | 2.96E-5 |
| Max | 0.470 | 1.02 | 0.470 | 0.560 | 0.470 | 0.560 |
| n (Samp) | 317 | 18 | 317 | 18 | 317 | 13 |
| n (Patient) | 317 | 18 | 317 | 18 | 317 | 13 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 6.51E-5 | 4.39E-5 | 6.51E-5 | 3.88E-5 | 6.51E-5 | 0.000118 |
| Average | 0.0127 | 0.0848 | 0.0127 | 0.0908 | 0.0127 | 0.00682 |
| Stdev | 0.0478 | 0.251 | 0.0478 | 0.265 | 0.0478 | 0.0142 |
| p(t-test) | | 0.0011 | | 7.8E-4 | | 0.70 |
| Min | 5.62E-6 | 1.88E-5 | 5.62E-6 | 1.88E-5 | 5.62E-6 | 1.66E-5 |

(continued)

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Max | 0.359 | 1.02 | 0.359 | 1.02 | 0.359 | 0.0446 |
| n (Samp) | 172 | 19 | 172 | 17 | 172 | 10 |
| n (Patient) | 172 | 19 | 172 | 17 | 172 | 10 |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| AUC | 0.61 | 0.68 | 0.55 | 0.55 | 0.62 | 0.51 | 0.60 | 0.63 | 0.54 |
| SE | 0.057 | 0.071 | 0.071 | 0.059 | 0.072 | 0.074 | 0.074 | 0.085 | 0.096 |
| p | 0.052 | 0.012 | 0.47 | 0.36 | 0.10 | 0.94 | 0.18 | 0.14 | 0.70 |
| nCohort 1 | 151 | 317 | 172 | 151 | 317 | 172 | 151 | 317 | 172 |
| nCohort 2 | 32 | 18 | 19 | 30 | 18 | 17 | 18 | 13 | 10 |
| Cutoff 1 | 3.44E-5 | 8.63E-5 | 2.83E-5 | 3.21E-5 | 3.21E-5 | 2.83E-5 | 3.21E-5 | 2.83E-5 | 2.83E-5 |
| Sens 1 | 72% | 72% | 79% | 70% | 72% | 76% | 72% | 100% | 90% |
| Spec 1 | 36% | 49% | 29% | 34% | 35% | 29% | 34% | 26% | 29% |
| Cutoff 2 | 2.83E-5 | 2.83E-5 | 2.68E-5 | 2.83E-5 | 2.83E-5 | 2.53E-5 | 2.83E-5 | 2.83E-5 | 2.83E-5 |
| Sens 2 | 88% | 100% | 84% | 83% | 94% | 82% | 94% | 100% | 90% |
| Spec 2 | 25% | 26% | 26% | 25% | 26% | 21% | 25% | 26% | 29% |
| Cutoff 3 | 2.68E-5 | 2.83E-5 | 2.46E-5 | 2.46E-5 | 2.83E-5 | 2.38E-5 | 2.83E-5 | 2.83E-5 | 2.83E-5 |
| Sens 3 | 91% | 100% | 95% | 90% | 94% | 94% | 94% | 100% | 90% |
| Spec 3 | 22% | 26% | 16% | 13% | 26% | 14% | 25% | 26% | 29% |
| Cutoff 4 | 0.00146 | 0.00127 | 0.00121 | 0.00146 | 0.00127 | 0.00121 | 0.00146 | 0.00127 | 0.00121 |
| Sens 4 | 41% | 50% | 32% | 37% | 44% | 24% | 44% | 46% | 30% |
| Spec 4 | 70% | 70% | 70% | 70% | 70% | 70% | 70% | 70% | 70% |
| Cutoff 5 | 0.00836 | 0.00672 | 0.00590 | 0.00836 | 0.00672 | 0.00590 | 0.00836 | 0.00672 | 0.00590 |
| Sens 5 | 31% | 44% | 26% | 27% | 39% | 24% | 28% | 46% | 30% |
| Spec 5 | 80% | 80% | 80% | 80% | 80% | 80% | 80% | 80% | 80% |
| Cutoff 6 | 0.0157 | 0.0171 | 0.0157 | 0.0157 | 0.0171 | 0.0157 | 0.0157 | 0.0171 | 0.0157 |
| Sens 6 | 25% | 28% | 21% | 17% | 17% | 18% | 17% | 15% | 10% |
| Spec 6 | 90% | 91% | 90% | 90% | 91% | 90% | 90% | 91% | 90% |
| OR Quart | 2.5 | >5.3 | 2.5 | 1.7 | 5.2 | 1.6 | 1.4 | >5.3 | 4.2 |
| 2 | 0.15 | <0.13 | 0.20 | 0.37 | 0.14 | 0.51 | 0.69 | <0.13 | 0.21 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| p Value | 0.71 | >0.60 | 0.61 | 0.52 | 0.59 | 0.41 | 0.29 | >0.60 | 0.45 |
| 95% Clot OR Quart2 | 8.8 | na | 10 | 5.8 | 45 | 6.0 | 6.5 | na | 39 |
| OR Quart | 1.8 | >4.2 | 1.3 | 1.2 | 4.1 | 0.73 | 1.0 | >2.0 | 2.0 |
| 3 0 | 0.36 | <0.21 | 0.72 | 0.75 | 0.21 | 0.70 | 1.0 | <0.56 | 0.56 |
| p Value | 0.50 | >0.45 | 0.28 | 0.35 | 0.45 | 0.15 | 0.19 | >0.18 | 0.18 |
| 95% CI of OR Quart3 | 6.8 | na | 6.3 | 4.4 | 37 | 3.5 | 5.3 | na | 23 |
| OR Quart | 3.6 | >10.0 | 1.7 | 2.5 | 8.6 | 0.98 | 3.0 | >6.4 | 3.1 |
| 4 | 0.039 | <0.031 | 0.48 | 0.12 | 0.044 | 0.98 | 0.13 | <0.089 | 0.34 |
| p Value | 1.1 | >1.2 | 0.38 | 0.80 | 1.1 | 0.23 | 0.73 | >0.75 | 0.31 |
| 95% CI of OR Quart4 | 12 | na | 7.6 | 7.9 | 71 | 4.2 | 12 | na | 31 |

**Fibroblast growth factor 21**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.0243 | 0.124 | 0.0243 | 0.0612 | 0.0243 | 0.0328 |
| Average | 0.188 | 0.546 | 0.188 | 0.331 | 0.188 | 0.176 |
| Stdev | 0.436 | 1.20 | 0.436 | 0.496 | 0.436 | 0.298 |
| p(t-test) | | 0.0044 | | 0.11 | | 0.91 |
| Min | 7.25E-6 | 0.00670 | 7.25E-6 | 0.00673 | 7.25E-6 | 0.00644 |
| Max | 3.07 | 6.31 | 3.07 | 1.59 | 3.07 | 1.07 |
| n (Samp) | 151 | 32 | 151 | 30 | 151 | 18 |
| n (Patient) | 151 | 32 | 151 | 30 | 151 | 18 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.0253 | 0.0532 | 0.0253 | 0.0532 | 0.0253 | 0.0317 |
| Average | 0.284 | 0.556 | 0.284 | 0.290 | 0.284 | 0.139 |
| Stdev | 0.789 | 1.49 | 0.789 | 0.496 | 0.789 | 0.228 |
| p(t-test) | | 0.18 | | 0.98 | | 0.51 |
| Min | 6.78E-7 | 0.00928 | 6.78E-7 | 0.00804 | 6.78E-7 | 0.00804 |
| Max | 8.92 | 6.31 | 8.92 | 1.56 | 8.92 | 0.725 |
| n (Samp) | 317 | 18 | 317 | 18 | 317 | 13 |
| n (Patient) | 317 | 18 | 317 | 18 | 317 | 13 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.0258 | 0.226 | 0.0258 | 0.0598 | 0.0258 | 0.0249 |
| Average | 0.235 | 0.481 | 0.235 | 0.377 | 0.235 | 0.299 |
| Stdev | 0.505 | 0.718 | 0.505 | 0.546 | 0.505 | 0.551 |
| p(t-test) | | 0.056 | | 0.28 | | 0.70 |
| Min | 7.25E-6 | 0.00670 | 7.25E-6 | 0.00673 | 7.25E-6 | 0.00644 |
| Max | 3.07 | 2.69 | 3.07 | 1.59 | 3.07 | 1.56 |
| n (Samp) | 172 | 19 | 172 | 17 | 172 | 10 |
| n (Patient) | 172 | 19 | 172 | 17 | 172 | 10 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.69 | 0.64 | 0.67 | 0.66 | 0.63 | 0.63 | 0.57 | 0.54 | 0.54 |
| SE | 0.055 | 0.072 | 0.071 | 0.058 | 0.072 | 0.075 | 0.074 | 0.083 | 0.096 |
| p | 6.1E-4 | 0.048 | 0.016 | 0.0055 | 0.076 | 0.079 | 0.36 | 0.65 | 0.70 |
| nCohort 1 | 151 | 317 | 172 | 151 | 317 | 172 | 151 | 317 | 172 |
| nCohort 2 | 32 | 18 | 19 | 30 | 18 | 17 | 18 | 13 | 10 |
| Cutoff 1 | 0.0317 | 0.0317 | 0.0313 | 0.0317 | 0.0317 | 0.0313 | 0.0165 | 0.0148 | 0.0165 |
| Sens 1 | 72% | 72% | 74% | 70% | 72% | 71% | 72% | 77% | 70% |
| Spec 1 | 58% | 56% | 55% | 58% | 56% | 55% | 42% | 36% | 40% |
| Cutoff 2 | 0.0191 | 0.0191 | 0.0165 | 0.0171 | 0.0181 | 0.0165 | 0.0127 | 0.0134 | 0.0130 |
| Sens 2 | 81% | 83% | 84% | 80% | 83% | 82% | 83% | 85% | 80% |
| Spec 2 | 44% | 42% | 40% | 42% | 41% | 40% | 33% | 32% | 31% |
| Cutoff 3 | 0.0147 | 0.0148 | 0.00670 | 0.00912 | 0.00926 | 0.00766 | 0.00776 | 0.00926 | 0.00956 |
| Sens 3 | 91% | 94% | 95% | 90% | 94% | 94% | 94% | 92% | 90% |
| Spec 3 | 37% | 36% | 19% | 26% | 23% | 22% | 24% | 23% | 24% |
| Cutoff 4 | 0.0934 | 0.0850 | 0.144 | 0.0934 | 0.0850 | 0.144 | 0.0934 | 0.0850 | 0.144 |
| Sens 4 | 50% | 39% | 58% | 40% | 39% | 41% | 28% | 31% | 30% |
| Spec 4 | 70% | 70% | 70% | 70% | 70% | 70% | 70% | 70% | 70% |
| Cutoff 5 | 0.221 | 0.255 | 0.295 | 0.221 | 0.255 | 0.295 | 0.221 | 0.255 | 0.295 |
| Sens 5 | 44% | 28% | 42% | 33% | 28% | 35% | 22% | 23% | 20% |
| Spec 5 | 80% | 80% | 80% | 80% | 80% | 80% | 80% | 80% | 80% |
| Cutoff 6 | 0.514 | 0.740 | 0.719 | 0.514 | 0.740 | 0.719 | 0.514 | 0.740 | 0.719 |
| Sens 6 | 22% | 11% | 21% | 23% | 11% | 24% | 11% | 0% | 20% |
| Spec 6 | 90% | 90% | 90% | 90% | 90% | 90% | 90% | 90% | 90% |
| OR Quart 2 | 1.7 | 4.1 | 0.64 | 1.0 | 1.5 | 0.65 | 3.3 | 2.0 | 1.5 |
| | 0.48 | 0.21 | 0.63 | 1.0 | 0.66 | 0.65 | 0.16 | 0.42 | 0.67 |
| p Value | 0.38 | 0.45 | 0.10 | 0.23 | 0.24 | 0.10 | 0.63 | 0.36 | 0.24 |
| 95% CI of OR Quart2 | 7.6 | 37 | 4.0 | 4.3 | 9.2 | 4.1 | 18 | 11 | 9.4 |
| OR Quart 3 | 3.4 | 8.6 | 1.7 | 3.3 | 4.3 | 1.7 | 2.7 | 2.1 | 1.0 |
| | 0.082 | 0.044 | 0.48 | 0.056 | 0.072 | 3.46 | 0.25 | 0.41 | 1.0 |
| p Value | 0.86 | 1.1 | 0.38 | 0.97 | 0.88 | 0.39 | 0.49 | 0.37 | 0.13 |
| 95% CI ot OR Quart3 | 14 | 71 | 7.6 | 11 | 21 | 7.8 | 15 | 12 | 7.4 |

(continued)

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| OR Quart | 6.8 | 5.2 | 3.4 | 3.2 | 2.6 | 2.5 | 2.6 | 1.5 | 1.5 |
| 4 | 0.0046 | 0.14 | 0.082 | 0.062 | 0.27 | 0.20 | 0.26 | 0.66 | 0.67 |
| p Value | 1.8 | 0.59 | 0.85 | 0.94 | 0.48 | 0.61 | 0.48 | 0.24 | 0.24 |
| 95% CI of OR Quart4 | 25 | 45 | 13 | 11 | 14 | 10 | 14 | 9.2 | 9.4 |

**Heparin-binding EGF-like growth factor**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 150 | 147 | 150 | 148 | 150 | 142 |
| Average | 156 | 163 | 156 | 161 | 156 | 148 |
| Stdev | 51.8 | 74.3 | 51.8 | 77.4 | 51.8 | 46.6 |
| p(t-test) |  | 0.57 |  | 0.69 |  | 0.55 |
| Min | 38.4 | 46.0 | 38.4 | 46.0 | 38.4 | 76.6 |
| Max | 311 | 444 | 311 | 444 | 311 | 241 |
| n (Samp) | 145 | 28 | 145 | 27 | 145 | 17 |
| n (Patient) | 145 | 28 | 145 | 27 | 145 | 17 |

| sCr only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 151 | 170 | 151 | 170 | 151 | 156 |
| Average | 160 | 166 | 160 | 162 | 160 | 152 |
| Stdev | 62.6 | 52.1 | 62.6 | 56.8 | 62.6 | 53.9 |
| p(t-test) |  | 0.69 |  | 0.89 |  | 0.67 |
| Min | 38.4 | 102 | 38.4 | 76.6 | 38.4 | 76.6 |
| Max | 444 | 265 | 444 | 265 | 444 | 241 |
| n (Samp) | 284 | 15 | 284 | 15 | 284 | 12 |
| n (Patient) | 284 | 15 | 284 | 15 | 284 | 12 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 153 | 144 | 153 | 142 | 153 | 127 |
| Average | 156 | 153 | 156 | 154 | 156 | 139 |
| Stdev | 52.4 | 87.0 | 52.4 | 89.7 | 52.4 | 46.9 |
| p(t-test) | | 0.87 | | 0.90 | | 0.33 |
| Min | 38.4 | 46.0 | 38.4 | 46.0 | 38.4 | 82.1 |
| Max | 311 | 444 | 311 | 444 | 311 | 241 |
| n (Samp) | 149 | 18 | 149 | 17 | 149 | 10 |
| n (Patient) | 149 | 18 | 149 | 17 | 149 | 10 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.51 | 0.55 | 0.44 | 0.50 | 0.52 | 0.44 | 0.45 | 0.48 | 0.40 |
| SE | 0.060 | 0.078 | 0.074 | 0.061 | 0.078 | 0.076 | 0.076 | 0.086 | 0.098 |
| p | 0.86 | 0.52 | 0.43 | 0.97 | 0.75 | 0.44 | 0.54 | 0.80 | 0.30 |
| nCohort 1 | 145 | 284 | 149 | 145 | 284 | 149 | 145 | 284 | 149 |
| nCohort 2 | 28 | 15 | 18 | 27 | 15 | 17 | 17 | 12 | 10 |
| Cutoff 1 | 131 | 128 | 110 | 128 | 118 | 110 | 119 | 105 | 119 |
| Sens 1 | 71% | 73% | 72% | 70% | 73% | 71% | 71% | 75% | 70% |
| Spec 1 | 36% | 35% | 22% | 32% | 29% | 22% | 25% | 22% | 28% |
| Cutoff 2 | 110 | 118 | 102 | 105 | 105 | 102 | 105 | 104 | 105 |
| Sens 2 | 82% | 80% | 83% | 81% | 80% | 82% | 82% | 83% | 80% |
| Spec 2 | 20% | 29% | 15% | 18% | 22% | 15% | 18% | 21% | 21% |
| Cutoff 3 | 102 | 104 | 46.0 | 74.0 | 102 | 46.0 | 94.6 | 94.8 | 94.6 |
| Sens 3 | 93% | 93% | 94% | 93% | 93% | 94% | 94% | 92% | 90% |
| Spec 3 | 13% | 21% | 1% | 3% | 19% | 1% | 10% | 14% | 12% |
| Cutoff 4 | 178 | 186 | 183 | 178 | 186 | 183 | 178 | 186 | 183 |
| Sens 4 | 32% | 27% | 17% | 33% | 27% | 18% | 24% | 17% | 10% |
| Spec 4 | 70% | 70% | 70% | 70% | 70% | 70% | 70% | 70% | 70% |
| Cutoff 5 | 194 | 212 | 198 | 194 | 212 | 198 | 194 | 212 | 198 |
| Sens 5 | 14% | 20% | 11% | 15% | 20% | 12% | 12% | 17% | 10% |
| Spec 5 | 80% | 80% | 81% | 80% | 80% | 81% | 80% | 80% | 81% |
| Cutoff 6 | 220 | 247 | 220 | 220 | 247 | 220 | 220 | 247 | 220 |
| Sens 6 | 14% | 7% | 11% | 15% | 7% | 12% | 12% | 0% | 10% |
| Spec 6 | 90% | 90% | 91% | 90% | 90% | 91% | 90% | 90% | 91% |

(continued)

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| OR Quart | 1.2 | 1.3 | 2.7 | 1.7 | 0.73 | 2.8 | 2.8 | 2.1 | 2.1 |
| 2 | 0.78 | 0.71 | 0.25 | 0.37 | 0.69 | 0.24 | 0.24 | 0.41 0.56 | |
| p Value | 0.39 | 0.29 | 0.49 | 0.52 | 0.16 | 0.51 | 0.51 | 0.37 0.18 | |
| 95% CI of OR Quart2 | 3.6 | 6.2 | 15 | 5.8 | 3.4 | 15 | 15 | 12 | 24 |
| OR Quart | 1.0 | 1.7 | 2.7 | 1.5 | 1.2 | 2.1 | 3.3 | 1.0 | 4.3 |
| 3 | 1.0 | 0.48 | 0.25 | 0.54 | 0.75 | 0.41 | 0.16 | 1.0 | 0.20 |
| p Value | 0.32 | 0.39 | 0.49 | 0.43 | 0.32 | 0.36 | 0.63 | 0.14 | 0.46 |
| 95% CI of OR Quart3 | 3.1 | 7.3 | 15 | 5.1 | 4.9 | 12 | 18 | 7.3 | 41 |
| OR Quart | 0.81 | 0.99 | 3.4 | 1.5 | 0.73 | 3.4 | 2.2 | 2.1 | 3.2 |
| 4 | 0.73 | 0.99 | 0.15 | 0.54 | 0.69 | 0.15 | 0.39 | 0.41 | 3.32 |
| p Value | 0.25 | 0.19 | 0.65 | 0.43 | 0.16 | 0.65 | 0.37 | 0.37 | 0.32 |
| 95% CI of OR Quart4 | 2.6 | 5.1 | 18 | 5.1 | 3.4 | 18 | 13 | 12 | 33 |

**Thrombospondin-2**

| sCr or UO | 0hr prior to AKI stage | | 124hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 1570 | 3440 | 1570 | 2270 | 1570 | 1780 |
| Average | 2500 | 8940 | 2500 | 8470 | 2500 | 6780 |
| Stdev | 3600 | 17000 | 3600 | 17500 | 3600 | 16800 |
| p(t-test) |  | 2.1E-6 |  | 1.8E-5 |  | 0.0023 |
| Min | 33.0 | 90.7 | 33.0 | 90.7 | 33.0 | 201 |
| Max | 33000 | 80100 | 33000 | 80100 | 33000 | 80100 |
| n (Samp) | 203 | 36 | 203 | 34 | 203 | 22 |
| n (Patient) | 203 | 36 | 203 | 34 | 203 | 22 |
| sCr only | 0hr prior to AKI stage | | 24hr prior to AKI stage | | 48hr prior to AKI stage | |
|  | Cohort 1 | Cohort 2 | Cohort 1 | Cohort 2 | Cohort 1 | Cohort 2 |
| Median | 1810 | 2100 | 1810 | 1980 | 1810 | 1940 |
| Average | 3180 | 7650 | 3180 | 7290 | 3180 | 8250 |

(continued)

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Stdev | 5450 | 17500 | 5450 | 17400 | 5450 | 20200 |
| p(t-test) | | 0.0029 | | 0.0061 | | 0.0033 |
| Min | 0.0376 | 90.7 | 0.0376 | 90.7 | 0.0376 | 890 |
| Max | 68100 | 80100 | 68100 | 80100 | 68100 | 80100 |
| n (Samp) | 395 | 20 | 395 | 20 | 395 | 15 |
| n (Patient) | 395 | 20 | 395 | 20 | 395 | 15 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1720 | 2890 | 1720 | 1950 | 1720 | 1250 |
| Average | 2700 | 8160 | 2700 | 7650 | 2700 | 2540 |
| Stdev | 3680 | 14700 | 3680 | 15500 | 3680 | 3590 |
| p(t-test) | | 2.1E-5 | | 2.3E-4 | | 0.88 |
| Min | 33.0 | 201 | 33.0 | 201 | 33.0 | 201 |
| Max | 33000 | 68100 | 33000 | 68100 | 33000 | 12300 |
| n (Samp) | 215 | 22 | 215 | 20 | 215 | 13 |
| n (Patient) | 215 | 22 | 215 | 20 | 215 | 13 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.71 | 0.64 | 0.63 | 0.66 | 0.61 | 0.58 | 0.60 | 0.58 | 0.43 |
| SE | 0.051 | 0.068 | 0.066 | 0.054 | 0.068 | 0.069 | 0.067 | 0.078 | 0.085 |
| p | 5.2E-5 | 0.045 | 0.043 | 0.0023 | 0.10 | 0.24 | 0.15 | 0.33 | 0.42 |
| nCohort 1 | 203 | 395 | 215 | 203 | 395 | 215 | 203 | 395 | 215 |
| nCohort 2 | 36 | 20 | 22 | 34 | 20 | 20 | 22 | 15 | 13 |
| Cutoff 1 | 1760 | 1810 | 1220 | 1460 | 1570 | 1220 | 1170 | 1240 | 813 |
| Sens 1 | 72% | 70% | 73% | 71% | 70% | 70% | 73% | 73% | 77% |
| Spec 1 | 56% | 50% | 39% | 47% | 43% | 39% | 42% | 36% | 27% |
| Cutoff 2 | 1270 | 1660 | 952 | 1190 | 1450 | 952 | 952 | 1170 | 525 |
| Sens 2 | 81% | 80% | 82% | 82% | 80% | 80% | 82% | 80% | 85% |
| Spec 2 | 45% | 46% | 30% | 44% | 41% | 30% | 34% | 34% | 16% |
| Cutoff 3 | 813 | 1240 | 525 | 813 | 1020 | 525 | 813 | 952 | 277 |
| Sens 3 | 92% | 90% | 91% | 91% | 90% | 90% | 91% | 93% | 92% |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Spec 3 | 31% | 36% | 16% | 31% | 29% | 16% | 31% | 28% | 10% |
| Cutoff 4 | 2360 | 2910 | 2690 | 2360 | 2910 | 2690 | 2360 | 2910 | 2690 |
| Sens 4 | 56% | 45% | 55% | 47% | 45% | 40% | 36% | 33% | 23% |
| Spec 4 | 70% | 70% | 70% | 70% | 70% | 70% | 70% | 70% | 70% |
| Cutoff 5 | 3330 | 4190 | 3610 | 3330 | 4190 | 3610 | 3330 | 4190 | 3610 |
| Sens 5 | 50% | 40% | 45% | 44% | 40% | 35% | 32% | 27% | 15% |
| Spec 5 | 80% | 80% | 80% | 80% | 80% | 80% | 80% | 80% | 80% |
| Cutoff 6 | 4930 | 6560 | 5440 | 4930 | 6560 | 5440 | 4930 | 6560 | 5440 |
| Sens 6 | 44% | 15% | 41% | 35% | 15% | 25% | 27% | 13% | 15% |
| Spec 6 | 90% | 90% | 90% | 90% | 90% | 90% | 90% | 90% | 90% |
| OR Quart | 2.5 | 6.2 | 2.5 | 3.4 | 8.5 | 2.5 | 3.9 | >7.4 | 1.0 |
| 2 | 0.21 | 0.093 | 0.20 | 0.081 | 0.046 | 0.21 | 0.10 | <0.063 | 1.0 |
| p Value | 0.61 | 0.74 | 0.62 | 0.86 | 1.0 | 0.61 | 0.76 | >0.90 | 0.14 |
| 95% CI ot OR Quart2 | 10 | 53 | 10 | 13 | 69 | 10 | 19 | na | 7.4 |
| OR Quart | 2.9 | 4.1 | 0.65 | 2.5 | 2.0 | 0.98 | 3.2 | >3.1 | 3.2 |
| 3 | 0.13 | 0.21 | 0.65 | 0.20 | 0.57 | 0.98 | 0.16 | <0.33 | 0.16 |
| p Value | 0.72 | 0.45 | 0.11 | 0.62 | 0.18 | 0.19 | 0.62 | >0.32 | 0.62 |
| 95% CI of OR Quart3 | 11 | 37 | 4.1 | 10 | 22 | 5.1 | 17 | na | 17 |
| OR Quart | 8.0 | 9.7 | 3.7 | 6.2 | 9.7 | 2.5 | 3.8 | >5.2 | 1.5 |
| 4 | 0.0015 | 0.033 | 0.055 | 0.0059 | 0.033 | 0.21 | 0.11 | <0.14 | 0.65 |
| p Value | 2.2 | 1.2 | 0.97 | 1.7 | 1.2 | 0.61 | 0.75 | >0.60 | 0.25 |
| 95% CI of OR Quart4 | 29 | 78 | 14 | 23 | 78 | 10 | 19 | na | 9.5 |

**Tenascin**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 13.9 | 20.6 | 13.9 | 20.6 | 13.9 | 18.2 |
| Average | 21.5 | 298 | 21.5 | 304 | 21.5 | 20.3 |

(continued)

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Stdev | 43.9 | 1420 | 43.9 | 1450 | 43.9 | 19.3 |
| p(t-test) | | 0.018 | | 0.017 | | 0.92 |
| Min | 0.0190 | 0.00398 | 0.0190 | 0.00398 | 0.0190 | 0.0190 |
| Max | 470 | 7540 | 470 | 7540 | 470 | 69.1 |
| n (Samp) | 148 | 28 | 148 | 27 | 148 | 17 |
| n (Patient) | 148 | 28 | 148 | 27 | 148 | 17 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 16.2 | 20.6 | 16.2 | 20.6 | 16.2 | 18.2 |
| Average | 54.1 | 23.7 | 54.1 | 23.7 | 54.1 | 20.7 |
| Stdev | 447 | 19.8 | 447 | 19.8 | 447 | 19.8 |
| p(t-test) | | 0.79 | | 0.79 | | 0.80 |
| Min | 0.00398 | 0.338 | 0.00398 | 0.338 | 0.00398 | 0.338 |
| Max | 7540 | 69.1 | 7540 | 69.1 | 7540 | 69.1 |
| n (Samp) | 288 | 15 | 288 | 15 | 288 | 12 |
| n (Patient) | 288 | 15 | 288 | 15 | 288 | 12 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 15.2 | 20.6 | 15.2 | 20.6 | 15.2 | 13.0 |
| Average | 28.8 | 448 | 28.8 | 466 | 28.8 | 17.5 |
| Stdev | 86.4 | 1770 | 86.4 | 1820 | 86.4 | 17.8 |
| p(t-test) | | 0.0035 | | 0.0031 | | 0.68 |
| Min | 0.0190 | 0.00398 | 0.0190 | 0.00398 | 0.0190 | 0.0190 |
| Max | 945 | 7540 | 945 | 7540 | 945 | 46.6 |
| n (Samp) | 152 | 18 | 152 | 17 | 152 | 10 |
| n (Patient) | 152 | 18 | 152 | 17 | 152 | 10 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.64 | 0.55 | 0.59 | 0.62 | 0.55 | 0.57 | 0.55 | 0.50 | 0.47 |
| SE | 0.060 | 0.078 | 0.074 | 0.062 | 0.078 | 0.076 | 0.076 | 0.085 | 0.096 |
| p | 0.025 | 0.55 | 0.21 | 0.046 | 0.55 | 0.35 | 0.51 | 0.98 | 0.73 |

(continued)

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| nCohort 1 | 148 | 288 | 152 | 148 | 288 | 152 | 148 | 288 | 152 |
| nCohort 2 | 28 | 15 | 18 | 27 | 15 | 17 | 17 | 12 | 10 |
| Cutoff 1 | 14.8 | 8.27 | 10.0 | 14.8 | 8.27 | 10.0 | 6.41 | 7.00 | 6.24 |
| Sens 1 | 71% | 73% | 72% | 70% | 73% | 71% | 71% | 75% | 70% |
| Spec 1 | 55% | 27% | 36% | 55% | 27% | 36% | 33% | 26% | 30% |
| Cutoff 2 | 6.24 | 7.00 | 0.103 | 6.24 | 7.00 | 0.103 | 0.103 | 6.36 | 0.103 |
| Sens 2 | 82% | 80% | 89% | 81% | 80% | 88% | 88% | 83% | 80% |
| Spec 2 | 33% | 26% | 11% | 33% | 26% | 11% | 12% | 26% | 11% |
| Cutoff 3 | 0.103 | 0.103 | 0.0840 | 0.103 | 0.103 | 0.0840 | 0.0840 | 0.103 | 0.0840 |
| Sens 3 | 93% | 100% | 94% | 93% | 100% | 94% | 94% | 100% | 90% |
| Spec 3 | 12% | 10% | 10% | 12% | 10% | 10% | 10% | 10% | 10% |
| Cutoff 4 | 19.8 | 26.2 | 22.0 | 19.8 | 26.2 | 22.0 | 19.8 | 26.2 | 22.0 |
| Sens 4 | 61% | 33% | 44% | 59% | 33% | 41% | 47% | 25% | 30% |
| Spec 4 | 70% | 70% | 70% | 70% | 70% | 70% | 70% | 70% | 70% |
| Cutoff 5 | 28.1 | 36.6 | 28.1 | 28.1 | 36.6 | 28.1 | 28.1 | 36.6 | 28.1 |
| Sens 5 | 36% | 20% | 39% | 33% | 20% | 35% | 24% | 17% | 30% |
| Spec 5 | 83% | 80% | 80% | 83% | 80% | 80% | 83% | 80% | 80% |
| Cutoff 6 | 44.7 | 57.5 | 45.0 | 44.7 | 57.5 | 45.0 | 44.7 | 57.5 | 45.0 |
| Sens 6 | 21% | 7% | 22% | 19% | 7% | 18% | 12% | 8% | 10% |
| Spec 6 | 91% | 90% | 90% | 91% | 90% | 90% | 91% | 90% | 90% |
| OR Quart 2 | 0.57 | 2.0 | 0.71 | 0.56 | 2.0 | 0.73 | 0.73 | 2.1 | 0.67 |
|  | 0.46 | 0.42 | 0.67 | 0.44 | 0.42 | 0.69 | 0.69 | 0.41 | 0.67 |
| p Value | 0.13 | 0.36 | 0.15 | 0.12 | 0.36 | 0.15 | 0.15 | 0.37 | 0.11 |
| 95% CI of OR Quart2 | 2.6 | 11 | 3.4 | 2.5 | 11 | 3.5 | 3.5 | 12 | 4.2 |
| OR Quart 3 | 1.7 | 2.6 | 0.73 | 1.4 | 2.6 | 0.73 | 1.0 | 2.1 | 0.65 |
|  | 0.37 | 0.27 | 0.69 | 0.56 | 0.27 | 0.69 | 1.0 | 0.41 | 0.65 |
| p Value | 0.52 | 0.48 | 0.15 | 0.42 | 0.48 | 0.15 | 0.23 | 0.37 | 0.10 |
| 95% CI of OR Quart3 | 5.8 | 14 | 3.5 | 4.9 | 14 | 3.5 | 4.3 | 12 | 4.1 |
| OR Quart 4 | 2.9 | 2.0 | 2.2 | 2.8 | 2.0 | 1.8 | 1.5 | 1.0 | 1.0 |
|  | 0.066 | 0.42 | 0.24 | 0.073 | 0.42 | 0.36 | 0.53 | 1.0 | 0.97 |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| p Value | 0.93 | 0.36 | 0.60 | 0.91 | 0.36 | 0.50 | 0.40 | 0.14 | 0.19 |
| 95% CI of OR Quart4 | 9.2 | 11 | 7.8 | 9.0 | 11 | 6.8 | 5.9 | 7.3 | 5.4 |

Fig. 5: Comparison of marker levels in EDTA samples collected from Cohort 1 (patients that did not progress beyond RIFLE stage 0) and in EDTA samples collected from subjects at 0, 24 hours, and 48 hours prior to reaching stage R, I or F in Cohort 2.

**Angiogenin**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 167000 | 198000 | 167000 | 187000 | 167000 | 174000 |
| Average | 186000 | 212000 | 186000 | 183000 | 186000 | 166000 |
| Stdev | 183000 | 103000 | 183000 | 91400 | 183000 | 92300 |
| p(t-test) | | 0.27 | | 0.90 | | 0.67 |
| Min | 20700 | 61200 | 20700 | 37100 | 20700 | 34500 |
| Max | 2240000 | 548000 | 2240000 | 446000 | 2240000 | 378000 |
| n (Samp) | 158 | 73 | 158 | 56 | 158 | 15 |
| n (Patient) | 88 | 73 | 88 | 56 | 88 | 15 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 175000 | 202000 | 175000 | 192000 | 175000 | 245000 |
| Average | 193000 | 200000 | 193000 | 182000 | 193000 | 197000 |
| Stdev | 144000 | 73800 | 144000 | 65100 | 144000 | 103000 |
| p(t-test) | | 0.82 | | 0.80 | | 0.95 |
| Min | 10800 | 35700 | 10800 | 61200 | 10800 | 31500 |
| Max | 2240000 | 410000 | 2240000 | 292000 | 2240000 | 308000 |
| n (Samp) | 380 | 20 | 380 | 11 | 380 | 7 |
| n (Patient) | 176 | 20 | 176 | 11 | 176 | 7 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 174000 | 195000 | 174000 | 182000 | 174000 | 173000 |

(continued)

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Average | 193000 | 212000 | 193000 | 186000 | 193000 | 164000 |
| Stdev | 175000 | 110000 | 175000 | 106000 | 175000 | 99200 |
| p(t-test) | | 0.42 | | 0.78 | | 0.49 |
| Min | 20700 | 23900 | 20700 | 37100 | 20700 | 34500 |
| Max | 2240000 | 548000 | 2240000 | 552000 | 2240000 | 378000 |
| n (Samp) | 184 | 64 | 184 | 62 | 184 | 18 |
| n (Patient) | 89 | 64 | 89 | 62 | 89 | 18 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.61 | 0.58 | 0.57 | 0.52 | 0.52 | 0.49 | 0.46 | 0.56 | 0.43 |
| SE | 0.041 | 0.068 | 0.042 | 0.045 | 0.089 | 0.043 | 0.080 | 0.11 | 0.073 |
| p | 0.0094 | 0.23 | 0.093 | 0.61 | 0.84 | 0.85 | 0.64 | 0.58 | 0.31 |
| nCohort 1 | 158 | 380 | 184 | 158 | 380 | 184 | 158 | 380 | 184 |
| nCohort 2 | 73 | 20 | 64 | 56 | 11 | 62 | 15 | 7 | 18 |
| Cutoff 1 | 149000 | 179000 | 143000 | 109000 | 174000 | 109000 | 104000 | 174000 | 99900 |
| Sens 1 | 71% | 70% | 70% | 71% | 73% | 71% | 73% | 71% | 72% |
| Spec 1 | 42% | 52% | 38% | 20% | 49% | 20% | 20% | 49% | 16% |
| Cutoff 2 | 124000 | 166000 | 123000 | 92900 | 124000 | 91600 | 100000 | 88200 | 60300 |
| Sens 2 | 81% | 80% | 81% | 80% | 82% | 81% | 80% | 86% | 83% |
| Spec 2 | 30% | 47% | 27% | 12% | 29% | 12% | 16% | 12% | 6% |
| Cutoff 3 | 109000 | 123000 | 96600 | 81100 | 104000 | 81100 | 44000 | 24100 | 44000 |
| Sens 3 | 90% | 90% | 91% | 91% | 91% | 90% | 93% | 100% | 94% |
| Spec 3 | 20% | 29% | 14% | 10% | 19% | 10% | 4% | 1% | 4% |
| Cutoff 4 | 207000 | 225000 | 217000 | 207000 | 225000 | 217000 | 207000 | 225000 | 217000 |
| Sens 4 | 45% | 30% | 42% | 41% | 27% | 35% | 13% | 57% | 17% |
| Spec 4 | 70% | 70% | 70% | 70% | 70% | 70% | 70% | 70% | 70% |
| Cutoff 5 | 227000 | 253000 | 247000 | 227000 | 253000 | 247000 | 227000 | 253000 | 247000 |
| Sens 5 | 33% | 15% | 27% | 30% | 9% | 21% | 13% | 43% | 17% |
| Spec 5 | 80% | 80% | 80% | 80% | 80% | 80% | 80% | 80% | 80% |
| Cutoff 6 | 278000 | 326000 | 293000 | 278000 | 326000 | 293000 | 278000 | 326000 | 293000 |
| Sens 6 | 15% | 5% | 20% | 16% | 0% | 18% | 13% | 0% | 17% |
| Spec 6 | 91% | 90% | 90% | 91% | 90% | 90% | 91% | 90% | 90% |
| OR Quart 2 | 1.6 | 5.2 | 1.3 | 0.37 | 0.99 | 0.67 | 4.1 | 0.49 | 2.2 |
| | 0.31 | 0.14 | 0.51 | 0.039 | 0.99 | 0.34 | 0.091 | 0.56 | 0.29 |
| p Value | 0.66 | 0.60 | 0.56 | 0.14 | 0.14 | 0.30 | 0.80 | 0.044 | 0.51 |

(continued)

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| 95% CI of OR Quart2 | 3.6 | 45 | 3.1 | 0.95 | 7.2 | 1.5 | 21 | 5.5 | 9.3 |
| OR Quart 3 | 1.8 | 9.8 | 1.6 | 0.69 | 2.6 | 0.38 | 0.50 | 0 | 0.65 |
|  | 0.16 | 0.032 | 0.29 | 0.39 | 0.27 | 0.035 | 0.58 | na | 0.65 |
| p Value | 0.79 | 1.2 | 0.68 | 0.29 | 0.48 | 0.16 | 0.044 | na | 0.10 |
| 95% CI of OR Quart3 | 4.2 | 79 | 3.7 | 1.6 | 13 | 0.94 | 5.7 | na | 4.1 |
| OR Quart 4 | 2.8 | 5.2 | 2.0 | 1.1 | 0.99 | 1.1 | 2.8 | 2.0 | 2.6 |
|  | 0.013 | 0.14 | 0.10 | 0.89 | 0.99 | 0.80 | 0.24 | 0.42 | 0.18 |
| p Value | 1.2 | 0.60 | 0.87 | 0.47 | 0.14 | 0.52 | 0.51 | 0.36 | 0.63 |
| 95% CI of OR Quart4 | 6.5 | 45 | 4.5 | 2.4 | 7.2 | 2.4 | 15 | 11 | 11 |

**Angiopoietin-related protein 4**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 24.9 | 37.7 | 24.9 | 36.3 | 24.9 | 18.2 |
| Average | 53.1 | 48.0 | 53.1 | 54.1 | 53.1 | 40.0 |
| Stdev | 160 | 42.6 | 160 | 56.2 | 160 | 49.0 |
| p(t-test) |  | 0.79 |  | 0.96 |  | 0.75 |
| Min | 1.77 | 6.18 | 1.77 | 4.89 | 1.77 | 5.00 |
| Max | 1900 | 243 | 1900 | 339 | 1900 | 176 |
| n (Samp) | 157 | 73 | 157 | 56 | 157 | 15 |
| n (Patient) | 88 | 73 | 88 | 56 | 88 | 15 |

| sCr only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 31.1 | 52.3 | 31.1 | 51.8 | 31.1 | 43.8 |
| Average | 47.7 | 60.3 | 47.7 | 60.2 | 47.7 | 37.7 |
| Stdev | 108 | 44.6 | 108 | 34.1 | 108 | 30.4 |
| p(t-test) |  | 0.60 |  | 0.70 |  | 0.81 |
| Min | 1.77 | 8.18 | 1.77 | 19.4 | 1.77 | 8.28 |
| Max | 1900 | 192 | 1900 | 127 | 1900 | 94.2 |
| n (Samp) | 379 | 20 | 379 | 11 | 379 | 7 |

(continued)

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| n (Patient) | 176 | 20 | 176 | 11 | 176 | 7 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 31.8 | 36.3 | 31.8 | 36.3 | 31.8 | 26.3 |
| Average | 61.4 | 48.0 | 61.4 | 53.4 | 61.4 | 45.6 |
| Stdev | 152 | 43.1 | 152 | 55.5 | 152 | 45.6 |
| p(t-test) | | 0.49 | | 0.69 | | 0.66 |
| Min | 2.68 | 6.18 | 2.68 | 4.89 | 2.68 | 5.00 |
| Max | 1900 | 243 | 1900 | 339 | 1900 | 176 |
| n (Samp) | 183 | 64 | 183 | 62 | 183 | 18 |
| n (Patient) | 89 | 64 | 89 | 62 | 89 | 18 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.63 | 0.68 | 0.54 | 0.63 | 0.71 | 0.55 | 0.44 | 0.49 | 0.46 |
| SE | 0.041 | 0.067 | 0.042 | 0.045 | 0.089 | 0.043 | 0.080 | 0.11 | 0.073 |
| p | 0.0019 | 0.0060 | 0.32 | 0.0040 | 0.016 | 0.25 | 0.45 | 0.95 | 0.62 |
| nCohort 1 | 157 | 379 | 183 | 157 | 379 | 183 | 157 | 379 | 183 |
| nCohort 2 | 73 | 20 | 64 | 56 | 11 | 62 | 15 | 7 | 18 |
| Cutoff 1 | 26.3 | 40.8 | 25.8 | 23.2 | 37.1 | 22.8 | 10.7 | 15.3 | 17.6 |
| Sens 1 | 71% | 70% | 70% | 71% | 73% | 71% | 73% | 71% | 72% |
| Spec 1 | 52% | 68% | 40% | 48% | 62% | 36% | 18% | 21% | 25% |
| Cutoff 2 | 21.8 | 33.9 | 20.7 | 19.4 | 32.5 | 19.4 | 10.4 | 9.30 | 10.7 |
| Sens 2 | 81% | 80% | 81% | 80% | 82% | 81% | 80% | 86% | 83% |
| Spec 2 | 44% | 56% | 31% | 38% | 54% | 28% | 17% | 8% | 13% |
| Cutoff 3 | 13.4 | 22.2 | 14.3 | 13.5 | 28.7 | 14.6 | 8.28 | 8.28 | 9.30 |
| Sens 3 | 90% | 90% | 91% | 91% | 91% | 90% | 93% | 100% | 94% |
| Spec 3 | 24% | 35% | 19% | 24% | 45% | 19% | 10% | 7% | 8% |
| Cutoff 4 | 37.7 | 43.0 | 50.4 | 37.7 | 43.0 | 50.4 | 37.7 | 43.0 | 50.4 |
| Sens 4 | 51% | 60% | 31% | 48% | 64% | 34% | 33% | 57% | 39% |
| Spec 4 | 70% | 70% | 70% | 70% | 70% | 70% | 70% | 70% | 70% |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Cutoff 5 | 51.6 | 57.5 | 71.1 | 51.6 | 57.5 | 71.1 | 51.6 | 57.5 | 71.1 |
| Sens 5 | 27% | 50% | 19% | 34% | 36% | 16% | 33% | 14% | 22% |
| Spec 5 | 80% | 80% | 80% | 80% | 80% | 80% | 80% | 80% | 80% |
| Cutoff 6 | 88.5 | 87.8 | 105 | 88.5 | 87.8 | 105 | 88.5 | 87.8 | 105 |
| Sens 6 | 10% | 15% | 5% | 14% | 27% | 11% | 13% | 14% | 11% |
| Spec 6 | 90% | 90% | 90% | 90% | 90% | 90% | 90% | 90% | 90% |
| OR Quart | 1.6 | 0.99 | 2.1 | 1.3 | >2.0 | 1.4 | 0 | 3.1 | 0.80 |
| 2 | 0.28 | 0.99 | 0.098 | 0.62 | <0.57 | 0.50 | na | 0.33 | 0.75 |
| p Value | 0.67 | 0.14 | 0.87 | 0.48 | >0.18 | 0.56 | na | 0.32 | 0.20 |
| 95% CI OR Quart2 | 4.0 | 7.2 | 5.0 | 3.4 | na | 3.3 | na | 30 | 3.2 |
| OR Quart | 3.9 | 3.1 | 2.4 | 2.3 | >3.1 | 2.4 | 0.78 | 0 | 0.80 |
| 3 | 0.0017 | 0.17 | 0.044 | 0.075 | <0.33 | 0.042 | 0.73 | na | 0.75 |
| p Value | 1.7 | 0.61 | 1.0 | 0.92 | >0.32 | 1.0 | 0.19 | na | 0.20 |
| 95% CI of OR Quart3 | 9.3 | 16 | 5.8 | 5.8 | na | 5.5 | 3.1 | na | 3.2 |
| OR Quart | 2.9 | 5.4 | 1.8 | 2.7 | >6.3 | 1.6 | 1.2 | 3.1 | 1.0 |
| 4 | 0.017 | 0.033 | 0.20 | 0.035 | <0.091 | 0.30 | 0.75 | 0.33 | 0.97 |
| p Value | 1.2 | 1.1 | 0.73 | 1.1 | >0.75 | 0.67 | 0.35 | 0.32 | 0.28 |
| 95% CI of OR Quart4 | 6.8 | 25 | 4.3 | 6.6 | na | 3.8 | 4.4 | 30 | 3.8 |

**Angiopoietin-related protein 6**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 63.6 | 70.1 | 63.6 | 63.3 | 63.6 | 48.2 |
| Average | 73.5 | 76.7 | 73.5 | 74.2 | 73.5 | 53.6 |
| Stdev | 46.5 | 38.7 | 46.5 | 41.0 | 46.5 | 33.3 |
| p(t-test) | | 0.62 | | 0.93 | | 0.11 |
| Min | 7.54 | 28.3 | 7.54 | 3.36 | 7.54 | 4.29 |

(continued)

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Max | 240 | 250 | 240 | 212 | 240 | 133 |
| n (Samp) | 157 | 73 | 157 | 56 | 157 | 15 |
| n (Patient) | 88 | 73 | 88 | 56 | 88 | 15 |

| sCr only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 64.7 | 79.7 | 64.7 | 64.8 | 64.7 | 64.8 |
| Average | 71.8 | 89.9 | 71.8 | 83.3 | 71.8 | 72.3 |
| Stdev | 41.0 | 56.4 | 41.0 | 52.5 | 41.0 | 31.3 |
| p(t-test) |  | 0.060 |  | 0.36 |  | 0.97 |
| Min | 3.36 | 36.7 | 3.36 | 38.7 | 3.36 | 47.8 |
| Max | 288 | 250 | 288 | 212 | 288 | 138 |
| n (Samp) | 379 | 20 | 379 | 11 | 379 | 7 |
| n (Patient) | 176 | 20 | 176 | 11 | 176 | 7 |

| UO only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 72.3 | 68.0 | 72.3 | 61.4 | 72.3 | 49.1 |
| Average | 82.2 | 72.7 | 82.2 | 73.4 | 82.2 | 60.5 |
| Stdev | 53.1 | 29.6 | 53.1 | 43.1 | 53.1 | 36.4 |
| p(t-test) |  | 0.18 |  | 0.24 |  | 0.093 |
| Min | 7.54 | 28.3 | 7.54 | 3.36 | 7.54 | 4.29 |
| Max | 283 | 154 | 283 | 250 | 283 | 133 |
| n (Samp) | 183 | 64 | 183 | 62 | 183 | 18 |
| n (Patient) | 89 | 64 | 89 | 62 | 89 | 18 |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| AUC | 0.55 | 0.59 | 0.49 | 0.53 | 0.55 | 0.47 | 0.37 | 0.53 | 0.38 |
| SE | 0.041 | 0.068 | 0.042 | 0.045 | 0.091 | 0.043 | 0.080 | 0.11 | 0.073 |
| p | 0.22 | 0.19 | 0.78 | 0.56 | 0.58 | 0.45 | 0.12 | 0.80 | 0.10 |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| nCohort 1 | 157 | 379 | 183 | 157 | 379 | 183 | 157 | 379 | 183 |
| nCohort 2 | 73 | 20 | 64 | 56 | 11 | 62 | 15 | 7 | 18 |
| Cutoff 1 | 52.7 | 48.3 | 53.7 | 49.6 | 53.7 | 47.6 | 40.3 | 53.7 | 41.8 |
| Sens 1 | 71% | 70% | 70% | 71% | 73% | 71% | 73% | 71% | 72% |
| Spec 1 | 42% | 32% | 36% | 37% | 40% | 30% | 27% | 40% | 23% |
| Cutoff 2 | 43.4 | 46.7 | 43.4 | 43.4 | 52.1 | 42.9 | 36.5 | 48.2 | 36.5 |
| Sens 2 | 81% | 80% | 81% | 80% | 82% | 81% | 80% | 86% | 83% |
| Spec 2 | 31% | 29% | 26% | 31% | 37% | 25% | 22% | 32% | 17% |
| Cutoff 3 | 38.1 | 43.4 | 38.1 | 36.5 | 44.0 | 36.5 | 22.6 | 47.7 | 21.7 |
| Sens 3 | 90% | 90% | 91% | 91% | 91% | 90% | 93% | 100% | 94% |
| Spec 3 | 23% | 27% | 18% | 22% | 28% | 17% | 8% | 31% | 5% |
| Cutoff 4 | 89.6 | 87.7 | 92.9 | 89.6 | 87.7 | 92.9 | 89.6 | 87.7 | 92.9 |
| Sens 4 | 29% | 35% | 22% | 25% | 18% | 26% | 13% | 14% | 22% |
| Spec 4 | 70% | 70% | 70% | 70% | 70% | 70% | 70% | 70% | 70% |
| Cutoff 5 | 102 | 96.5 | 114 | 102 | 96.5 | 114 | 102 | 96.5 | 114 |
| Sens 5 | 16% | 25% | 9% | 12% | 18% | 10% | 13% | 14% | 17% |
| Spec 5 | 80% | 80% | 80% | 80% | 80% | 80% | 80% | 80% | 80% |
| Cutoff 6 | 135 | 121 | 151 | 135 | 121 | 151 | 135 | 121 | 151 |
| Sens 6 | 7% | 20% | 2% | 9% | 18% | 8% | 0% | 14% | 0% |
| Spec 6 | 90% | 90% | 90% | 90% | 90% | 90% | 90% | 90% | 90% |
| OR Quart 2 | 2.7 | 2.6 | 2.0 | 3.4 | 4.1 | 1.2 | 1.0 | >3.1 | 0.67 |
| | 0.019 | 0.27 | 0.097 | 0.010 | 0.21 | 0.63 | 1.0 | <0.34 | 0.66 |
| p Value | 1.2 | 0.48 | 0.88 | 1.3 | 0.45 | 0.52 | 0.13 | >0.31 | 0.11 |
| 95% CI of OR Quart2 | 6.4 | 13 | 4.8 | 8.7 | 37 | 3.0 | 7.4 | na | 4.2 |
| OR Quart 3 | 2.6 | 4.2 | 2.4 | 2.0 | 4.1 | 2.4 | 4.0 | >3.1 | 2.6 |
| | 0.025 | 0.073 | 0.043 | 0.16 | 0.21 | 0.041 | 0.097 | <0.33 | 0.18 |
| p Value | 1.1 | 0.87 | 1.0 | 0.77 | 0.45 | 1.0 | 0.78 | >0.32 | 0.63 |
| 95% CI of OR Quart3 | 6.1 | 20 | 5.5 | 5.3 | 38 | 5.3 | 20 | na | 11 |
| OR Quart 4 | 1.7 | 2.6 | 1.3 | 2.0 | 2.0 | 1.2 | 2.1 | >1.0 | 2.2 |
| | 0.21 | 0.27 | 0.62 | 0.17 | 0.57 | 0.63 | 0.41 | <1.0 | 0.29 |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| p Value | 0.73 | 0.48 | 0.51 | 0.75 | 0.18 | 0.52 | 0.36 | >0.062 | 0.51 |
| 95% CI of OR Quart4 | 4.1 | 13 | 3.1 | 5.2 | 22 | 3.0 | 12 | na | 9.3 |

**Amphiregulin**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 20.4 | 20.9 | 20.4 | 25.2 | 20.4 | 17.7 |
| Average | 25.5 | 24.6 | 25.5 | 33.1 | 25.5 | 33.0 |
| Stdev | 17.7 | 16.7 | 17.7 | 25.5 | 17.7 | 31.9 |
| p(t-test) | | 0.70 | | 0.019 | | 0.16 |
| Min | 0.00246 | 0.00246 | 0.00246 | 5.07 | 0.00246 | 6.76 |
| Max | 121 | 72.6 | 121 | 133 | 121 | 113 |
| n (Samp) | 156 | 71 | 156 | 53 | 156 | 15 |
| n (Patient) | 87 | 71 | 87 | 53 | 87 | 15 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 21.3 | 20.3 | 21.3 | 31.4 | 21.3 | 43.9 |
| Average | 27.2 | 26.7 | 27.2 | 33.1 | 27.2 | 41.8 |
| Stdev | 21.5 | 17.9 | 21.5 | 14.4 | 21.5 | 29.6 |
| p(t-test) | | 0.92 | | 0.37 | | 0.079 |
| Min | 0.00246 | 7.08 | 0.00246 | 12.3 | 0.00246 | 14.3 |
| Max | 178 | 72.6 | 178 | 61.8 | 178 | 98.8 |
| n (Samp) | 372 | 19 | 372 | 11 | 372 | 7 |
| n (Patient) | 173 | 19 | 173 | 11 | 173 | 7 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 21.1 | 20.9 | 21.1 | 24.4 | 21.1 | 19.5 |
| Average | 26.6 | 24.6 | 26.6 | 34.1 | 26.6 | 31.7 |
| Stdev | 21.4 | 16.8 | 21.4 | 29.9 | 21.4 | 30.2 |
| p(t-test) | | 0.51 | | 0.035 | | 0.36 |

(continued)

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Min | 0.00246 | 0.00246 | 0.00246 | 5.07 | 0.00246 | 4.60 |
| Max | 198 | 72.6 | 198 | 162 | 198 | 113 |
| n (Samp) | 181 | 63 | 181 | 59 | 181 | 18 |
| n (Patient) | 88 | 63 | 88 | 59 | 88 | 18 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or | sCr only | UO only | sCr or UO | sCr only | UO only |
| AUC | 0.48 | 0.50 | 0.47 | 0.58 | 0.66 | 0.56 | 0.50 | 0.67 | 0.49 |
| SE | 0.042 | 0.068 | 0.043 | 0.046 | 0.091 | 0.044 | 0.078 | 0.11 | 0.072 |
| p | 0.65 | 0.98 | 0.55 | 0.095 | 0.071 | 0.16 | 0.97 | 0.14 | 0.90 |
| nCohort 1 | 156 | 372 | 181 | 156 | 372 | 181 | 156 | 372 | 181 |
| nCohort 2 | 71 | 19 | 63 | 53 | 11 | 59 | 15 | 7 | 18 |
| Cutoff 1 | 13.6 | 14.3 | 13.6 | 16.8 | 25.6 | 16.8 | 14.7 | 21.1 | 14.7 |
| Sens 1 | 72% | 74% | 71% | 72% | 73% | 71% | 73% | 71% | 72% |
| Spec 1 | 24% | 27% | 23% | 39% | 61% | 37% | 29% | 50% | 28% |
| Cutoff 2 | 11.4 | 11.7 | 10.3 | 13.5 | 21.8 | 13.5 | 13.9 | 16.1 | 12.5 |
| Sens 2 | 80% | 84% | 81% | 81% | 82% | 81% | 80% | 86% | 83% |
| Spec 2 | 17% | 17% | 13% | 24% | 51% | 22% | 26% | 33% | 18% |
| Cutoff 3 | 8.58 | 8.58 | 8.57 | 11.0 | 18.1 | 11.0 | 6.93 | 14.3 | 6.01 |
| Sens 3 | 90% | 95% | 90% | 91% | 91% | 92% | 93% | 100% | 94% |
| Spec 3 | 12% | 10% | 10% | 15% | 41% | 14% | 6% | 26% | 4% |
| Cutoff 4 | 30.9 | 30.4 | 30.9 | 30.9 | 30.4 | 30.9 | 30.9 | 30.4 | 30.9 |
| Sens 4 | 25% | 32% | 25% | 38% | 55% | 34% | 27% | 57% | 28% |
| Spec 4 | 71% | 70% | 70% | 71% | 70% | 70% | 71% | 70% | 70% |
| Cutoff 5 | 35.6 | 37.3 | 37.0 | 35.6 | 37.3 | 37.0 | 35.6 | 37.3 | 37.0 |
| Sens 5 | 18% | 21% | 16% | 32% | 27% | 29% | 27% | 57% | 22% |
| Spec 5 | 80% | 80% | 81% | 80% | 80% | 81% | 80% | 80% | 81% |
| | sCr or UO | sCr only | UO only | sCr or UO | sCr only | UO only | sCr or UO | sCr only | UO only |
| Cutoff 6 | 47.0 | 52.4 | 45.5 | 47.0 | 52.4 | 45.5 | 47.0 | 52.4 | 45.5 |
| Sens 6 | 11% | 11% | 11% | 23% | 9% | 22% | 20% | 29% | 22% |
| Spec 6 | 90% | 90% | 90% | 90% | 90% | 90% | 90% | 90% | 90% |
| OR Quart | 1.4 | 0.99 | 1.3 | 0.79 | 0.99 | 0.74 | 1.7 | >3.1 | 1.3 |
| 2 | 0.42 | 0.99 | 0.53 | 0.63 | 0.99 | 0.50 | 0.48 | <0.34 | 0.73 |

(continued)

|  | sCr or UO | sCr only | UO only | sCr or UO | sCr only | UO only | sCr or UO | sCr only | UO only |
|---|---|---|---|---|---|---|---|---|---|
| p Value | 0.63 | 0.28 | 0.57 | 0.31 | 0.061 | 0.30 | 0.38 | >0.31 | 0.32 |
| 95% CI ot OR Quart2 | 3.1 | 3.5 | 2.9 | 2.0 | 16 | 1.8 | 7.7 | na | 5.1 |
| OR Quart | 0.92 | 0.78 | 1.0 | 1.2 | 5.2 | 1.2 | 0.98 | >0 | 1.3 |
| 3 | 0.83 | 0.72 | 1.0 | 0.65 | 0.14 | 0.67 | 0.98 | <na | 0.73 |
| p Value | 0.40 | 0.20 | 0.43 | 0.50 | 0.59 | 0.52 | 0.19 | >na | 0.32 |
| 95% CI of OR Quart3 | 2.1 | 3.0 | 2.3 | 3.0 | 45 | 2.7 | 5.1 | na | 5.1 |
| OR Quart | 1.4 | 0.99 | 1.4 | 1.6 | 4.1 | 1.4 | 1.3 | >4.1 | 1.0 |
| 4 | 0.38 | 0.99 | 0.41 | 0.30 | 0.21 | 0.41 | 0.72 | <0.21 | 0.98 |
| p Value | 0.64 | 0.28 | 0.62 | 0.66 | 0.45 | 0.62 | 0.28 | >0.45 | 0.24 |
| 95% CI of OR Quart4 | 3.2 | 3.5 | 3.2 | 3.7 | 37 | 3.2 | 6.4 | na | 4.3 |

**Betacellulin**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 4.02 | 3.84 | 4.02 | 4.08 | 4.02 | 3.93 |
| Average | 4.29 | 4.55 | 4.29 | 5.49 | 4.29 | 5.61 |
| Stdev | 2.94 | 6.28 | 2.94 | 5.39 | 2.94 | 3.80 |
| p(t-test) |  | 0.67 |  | 0.043 |  | 0.12 |
| Min | 0.00226 | 0.00274 | 0.00226 | 0.00282 | 0.00226 | 1.92 |
| Max | 15.0 | 50.2 | 15.0 | 32.2 | 15.0 | 13.9 |
| n (Samp) | 155 | 72 | 155 | 54 | 155 | 14 |
| n (Patient) | 87 | 72 | 87 | 54 | 87 | 14 |

| sCr only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 4.02 | 2.23 | 4.02 | 4.58 | 4.02 | 3.61 |
| Average | 4.43 | 5.58 | 4.43 | 5.03 | 4.43 | 5.58 |
| Stdev | 3.21 | 11.4 | 3.21 | 3.31 | 3.21 | 6.34 |
| p(t-test) |  | 0.22 |  | 0.54 |  | 0.36 |

(continued)

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Min | 0.00226 | 0.00282 | 0.00226 | 0.00282 | 0.00226 | 0.00274 |
| Max | 21.0 | 50.2 | 21.0 | 11.8 | 21.0 | 17.6 |
| n (Samp) | 372 | 19 | 372 | 11 | 372 | 7 |
| n (Patient) | 174 | 19 | 174 | 11 | 174 | 7 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 3.74 | 4.05 | 3.74 | 4.08 | 3.74 | 4.24 |
| Average | 4.87 | 3.93 | 4.87 | 5.98 | 4.87 | 5.34 |
| Stdev | 7.41 | 2.74 | 7.41 | 7.75 | 7.41 | 3.44 |
| p(t-test) | | 0.32 | | 0.32 | | 0.79 |
| Min | 0.00226 | 0.00274 | 0.00226 | 0.00289 | 0.00226 | 1.92 |
| Max | 60.7 | 12.2 | 60.7 | 50.2 | 60.7 | 13.9 |
| n (Samp) | 180 | 64 | 180 | 60 | 180 | 17 |
| n (Patient) | 88 | 64 | 88 | 60 | 88 | 17 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.47 | 0.38 | 0.50 | 0.54 | 0.55 | 0.56 | 0.57 | 0.50 | 0.59 |
| SE | 0.041 | 0.070 | 0.042 | 0.046 | 0.091 | 0.044 | 0.083 | 0.11 | 0.075 |
| p | 0.45 | 0.077 | 0.93 | 0.41 | 0.55 | 0.19 | 0.40 | 0.98 | 0.24 |
| nCohort 1 | 155 | 372 | 180 | 155 | 372 | 180 | 155 | 372 | 180 |
| nCohort 2 | 72 | 19 | 64 | 54 | 11 | 60 | 14 | 7 | 17 |
| Cutoff 1 | 1.92 | 0.0204 | 2.42 | 2.92 | 3.54 | 2.92 | 3.54 | 1.59 | 3.54 |
| Sens 1 | 71% | 74% | 70% | 70% | 73% | 70% | 71% | 71% | 71% |
| Spec 1 | 23% | 9% | 31% | 34% | 42% | 40% | 41% | 16% | 46% |
| Cutoff 2 | 0.743 | 0.00282 | 1.18 | 2.26 | 2.89 | 1.92 | 2.26 | 0.0204 | 2.65 |
| Sens 2 | 81% | 89% | 83% | 81% | 82% | 80% | 93% | 86% | 82% |
| Spec 2 | 10% | 3% | 16% | 25% | 32% | 27% | 25% | 9% | 37% |
| Cutoff 3 | 0.00282 | 0.00274 | 0.00282 | 0.743 | 2.42 | 0.743 | 2.26 | 0.00226 | 2.26 |
| Sens 3 | 93% | 100% | 95% | 91% | 91% | 90% | 93% | 100% | 94% |
| Spec 3 | 4% | 2% | 8% | 10% | 26% | 14% | 25% | 1% | 31% |
| Cutoff 4 | 5.14 | 5.42 | 5.12 | 5.14 | 5.42 | 5.12 | 5.14 | 5.42 | 5.12 |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Sens 4 | 31% | 21% | 28% | 37% | 18% | 40% | 29% | 43% | 24% |
| Spec 4 | 70% | 70% | 70% | 70% | 70% | 70% | 70% | 70% | 70% |
| Cutoff 5 | 6.20 | 6.52 | 6.20 | 6.20 | 6.52 | 6.20 | 6.20 | 6.52 | 6.20 |
| Sens 5 | 21% | 21% | 17% | 28% | 18% | 27% | 29% | 43% | 24% |
| Spec 5 | 81% | 80% | 81% | 81% | 80% | 81% | 81% | 80% | 81% |
| Cutoff 6 | 7.89 | 8.40 | 7.89 | 7.89 | 8.40 | 7.89 | 7.89 | 8.40 | 7.89 |
| Sens 6 | 12% | 11% | 9% | 19% | 18% | 17% | 29% | 29% | 24% |
| Spec 6 | 90% | 90% | 90% | 90% | 90% | 90% | 90% | 90% | 90% |
| OR Quart 2 | 0.92 | 0.49 | 1.3 | 1.2 | 3.0 | 1.5 | 1.4 | 0 | 8.0 |
| | 0.84 | 0.42 | 0.54 | 0.65 | 0.34 | 0.39 | 0.69 | na | 0.056 |
| p Value | 0.42 | 0.088 | 0.58 | 0.50 | 0.31 | 0.62 | 0.29 | na | 0.95 |
| 95% CI of OR Quart2 | 2.0 | 2.7 | 2.9 | 3.0 | 30 | 3.4 | 6.5 | na | 68 |
| OR Quart 3 | 0.85 | 0.74 | 0.91 | 1.0 | 5.2 | 1.1 | 1.0 | 0.33 | 5.5 |
| | 0.68 | 0.70 | 0.83 | 1.0 | 0.14 | 0.82 | 1.0 | 0.34 | 0.13 |
| p Value | 0.38 | 0.16 | 0.40 | 0.40 | 0.59 | 0.46 | 0.19 | 0.033 | 0.61 |
| 95% CI of OR Quart3 | 1.9 | 3.4 | 2.1 | 2.5 | 45 | 2.7 | 5.3 | 3.2 | 49 |
| OR Quart 4 | 1.3 | 2.7 | 1.2 | 1.4 | 2.0 | 1.9 | 1.3 | 1.0 | 4.2 |
| | 0.51 | 0.10 | 0.68 | 0.41 | 0.57 | 0.15 | 0.72 | 0.99 | 0.21 |
| p Value | 0.60 | 0.82 | 0.53 | 0.60 | 0.18 | 0.80 | 0.28 | 0.20 | 0.45 |
| 95% CI of OR Quart4 | 2.8 | 8.9 | 2.7 | 3.4 | 22 | 4.3 | 6.4 | 5.1 | 39 |

**Endostatin**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 35400 | 42600 | 35400 | 43100 | 35400 | 48800 |
| Average | 43000 | 57500 | 43000 | 54500 | 43000 | 49500 |
| Stdev | 32700 | 49300 | 32700 | 44800 | 32700 | 36500 |
| p(t-test) | | 0.0085 | | 0.042 | | 0.47 |

(continued)

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Min | 4620 | 8430 | 4620 | 7440 | 4620 | 7440 |
| Max | 328000 | 275000 | 328000 | 241000 | 328000 | 133000 |
| n (Samp) | 158 | 73 | 158 | 56 | 158 | 15 |
| n (Patient) | 88 | 73 | 88 | 56 | 88 | 15 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 38700 | 56400 | 38700 | 69400 | 38700 | 65700 |
| Average | 47300 | 62400 | 47300 | 90000 | 47300 | 74800 |
| Stdev | 34800 | 38900 | 34800 | 58000 | 34800 | 40700 |
| p(t-test) | | 0.062 | | 1.1E-4 | | 0.040 |
| Min | 3600 | 13600 | 3600 | 19800 | 3600 | 11600 |
| Max | 328000 | 165000 | 328000 | 196000 | 328000 | 133000 |
| n (Samp) | 380 | 20 | 380 | 11 | 380 | 7 |
| n (Patient) | 176 | 20 | 176 | 11 | 176 | 7 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 37700 | 40000 | 37700 | 36800 | 37700 | 42800 |
| Average | 48100 | 56100 | 48100 | 49300 | 48100 | 69100 |
| Stdev | 36900 | 51500 | 36900 | 40400 | 36900 | 107000 |
| p(t-test) | | 0.18 | | 0.83 | | 0.073 |
| Min | 4620 | 8430 | 4620 | 7440 | 4620 | 7440 |
| Max | 328000 | 275000 | 328000 | 241000 | 328000 | 483000 |
| n (Samp) | 184 | 64 | 184 | 62 | 184 | 18 |
| n (Patient) | 89 | 64 | 89 | 62 | 89 | 18 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.59 | 0.63 | 0.52 | 0.57 | 0.74 | 0.50 | 0.55 | 0.73 | 0.51 |
| SE | 0.041 | 0.068 | 0.042 | 0.045 | 0.087 | 0.042 | 0.080 | 0.11 | 0.072 |
| p | 0.034 | 0.054 | 0.59 | 0.13 | 0.0051 | 0.99 | 0.53 | 0.033 | 0.84 |
| nCohort 1 | 158 | 380 | 184 | 158 | 380 | 184 | 158 | 380 | 184 |
| nCohort 2 | 73 | 20 | 64 | 56 | 11 | 62 | 15 | 7 | 18 |

(continued)

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| Cutoff 1 | 30600 | 46100 | 29800 | 30600 | 52400 | 30900 | 23500 | 58300 | 25600 |
| Sens 1 | 71% | 70% | 70% | 71% | 73% | 71% | 73% | 71% | 72% |
| Spec 1 | 40% | 61% | 33% | 40% | 69% | 37% | 23% | 75% | 23% |
| Cutoff 2 | 23500 | 31900 | 22700 | 20400 | 52100 | 20100 | 15200 | 55100 | 15700 |
| Sens 2 | 81% | 80% | 81% | 80% | 82% | 81% | 80% | 86% | 83% |
| Spec 2 | 23% | 35% | 18% | 16% | 68% | 14% | 9% | 72% | 8% |
| Cutoff 3 | 18700 | 20100 | 18700 | 14500 | 29300 | 14300 | 12500 | 11100 | 12500 |
| Sens 3 | 90% | 90% | 91% | 91% | 91% | 90% | 93% | 100% | 94% |
| Spec 3 | 13% | 14% | 11% | 7% | 30% | 6% | 4% | 4% | 4% |
| Cutoff 4 | 46600 | 53000 | 52100 | 46600 | 53000 | 52100 | 46600 | 53000 | 52100 |
| Sens 4 | 45% | 60% | 34% | 48% | 64% | 32% | 53% | 86% | 39% |
| Spec 4 | 70% | 70% | 70% | 70% | 70% | 70% | 70% | 70% | 70% |
| Cutoff 5 | 56200 | 66300 | 65300 | 56200 | 66300 | 65300 | 56200 | 66300 | 65300 |
| Sens 5 | 37% | 30% | 28% | 34% | 55% | 27% | 40% | 43% | 22% |
| Spec 5 | 80% | 80% | 80% | 80% | 80% | 80% | 80% | 80% | 80% |
| Cutoff 6 | 78000 | 84400 | 91700 | 78000 | 84400 | 91700 | 78000 | 84400 | 91700 |
| Sens 6 | 19% | 25% | 12% | 16% | 36% | 8% | 13% | 29% | 11% |
| Spec 6 | 91% | 90% | 90% | 91% | 90% | 90% | 91% | 90% | 90% |
| OR Quart 2 | 1.1 | 0.49 | 0.92 | 0.88 | 0.99 | 0.60 | 0 | 0 | 0.46 |
|  | 0.88 | 0.42 | 0.84 | 0.78 | 0.99 | 0.23 | na | na | 0.29 |
| p Value | 0.47 | 0.088 | 0.41 | 0.36 | 0.061 | 0.26 | na | na | 0.11 |
| 95% CI of OR Quart2 | 2.4 | 2.7 | 2.1 | 2.2 | 16 | 1.4 | na | na | 1.9 |
| OR Quart 3 | 0.98 | 1.5 | 0.76 | 0.90 | 3.0 | 0.85 | 0.78 | 2.0 | 0.64 |
|  | 0.96 | 0.52 | 0.53 | 0.82 | 0.34 | 0.69 | 0.73 | 0.57 | 0.51 |
| p Value | 0.42 | 0.42 | 0.33 | 0.37 | 0.31 | 0.39 | 0.19 | 0.18 | 0.17 |
| 95% CI ot 2.2 OR Quart3 | 2.2 | 5.6 | 1.8 | 2.2 | 30 | 1.9 | 3.1 | 22 | 2.4 |
| OR Quart 4 | 2.4 | 2.1 | 1.4 | 1.7 | 6.3 | 0.87 | 1.2 | 4.1 | 0.80 |
|  | 0.026 | 0.24 | 0.43 | 0.23 | 0.092 | 0.73 | 0.78 | 0.21 | 0.72 |
| p Value | 1.1 | 0.61 | 0.63 | 0.72 | 0.74 | 0.39 | 0.34 | 0.45 | 0.23 |
| 95% CI of OR Quart4 | 5.3 | 7.2 | 3.0 | 3.9 | 53 | 1.9 | 4.3 | 37 | 2.8 |

**Proepiregulin**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.392 | 0.374 | 0.392 | 0.399 | 0.392 | 0.329 |
| Average | 0.509 | 1.12 | 0.509 | 1.16 | 0.509 | 0.604 |
| Stdev | 0.628 | 3.14 | 0.628 | 4.21 | 0.628 | 0.563 |
| p(t-test) | | 0.020 | | 0.061 | | 0.59 |
| Min | 0.000152 | 0.000152 | 0.000152 | 0.000152 | 0.000152 | 0.0793 |
| Max | 6.96 | 20.6 | 6.96 | 31.1 | 6.96 | 1.68 |
| n (Samp) | 156 | 72 | 156 | 54 | 156 | 14 |
| n (Patient) | 87 | 72 | 87 | 54 | 87 | 14 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.396 | 0.392 | 0.396 | 0.298 | 0.396 | 0.472 |
| Average | 0.667 | 0.825 | 0.667 | 0.693 | 0.667 | 0.673 |
| Stdev | 1.42 | 1.41 | 1.42 | 1.04 | 1.42 | 0.713 |
| p(t-test) | | 0.64 | | 0.95 | | 0.99 |
| Min | 0.000152 | 0.000152 | 0.000152 | 0.106 | 0.000152 | 0.192 |
| Max | 18.0 | 4.75 | 18.0 | 3.65 | 18.0 | 2.24 |
| n (Samp) | 371 | 19 | 371 | 11 | 371 | 7 |
| n (Patient) | 173 | 19 | 173 | 11 | 173 | 7 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.383 | 0.363 | 0.383 | 0.399 | 0.383 | 0.365 |
| Average | 0.756 | 1.14 | 0.756 | 1.06 | 0.756 | 0.550 |
| Stdev | 2.51 | 3.29 | 2.51 | 3.99 | 2.51 | 0.524 |
| p(t-test) | | 0.33 | | 0.49 | | 0.74 |
| Min | 0.000152 | 0.000152 | 0.000152 | 0.000152 | 0.000152 | 0.0793 |
| Max | 32.1 | 20.6 | 32.1 | 31.1 | 32.1 | 1.68 |
| n (Samp) | 181 | 64 | 181 | 60 | 181 | 17 |
| n (Patient) | 88 | 64 | 88 | 60 | 88 | 17 |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| AUC | 0.50 | 0.45 | 0.51 | 0.50 | 0.46 | 0.52 | 0.51 | 0.54 | 0.49 |
| SE | 0.041 | 0.070 | 0.042 | 0.046 | 0.090 | 0.043 | 0.081 | 0.11 | 0.074 |
| p | 1.00 | 0.43 | 0.82 | 1.00 | 0.62 | 0.70 | 0.92 | 0.69 | 0.93 |
| nCohort 1 | 156 | 371 | 181 | 156 | 371 | 181 | 156 | 371 | 181 |
| nCohort 2 | 72 | 19 | 64 | 54 | 11 | 60 | 14 | 7 | 17 |
| Cutoff 1 | 0.188 | 0.134 | 0.192 | 0.274 | 0.188 | 0.274 | 0.238 | 0.286 | 0.238 |
| Sens 1 | 71% | 74% | 73% | 70% | 82% | 70% | 71% | 71% | 71% |
| Spec 1 | 20% | 13% | 24% | 30% | 21% | 33% | 25% | 34% | 29% |
| Cutoff 2 | 0.117 | 0.00127 | 0.126 | 0.134 | 0.188 | 0.179 | 0.139 | 0.278 | 0.139 |
| Sens 2 | 81% | 84% | 83% | 83% | 82% | 80% | 86% | 86% | 82% |
| Spec 2 | 12% | 1% | 15% | 12% | 21% | 21% | 14% | 33% | 18% |
| Cutoff 3 | 0.0201 | 0 | 0.0796 | 0.106 | 0.134 | 0.126 | 0.134 | 0.188 | 0.134 |
| Sens 3 | 90% | 100% | 91% | 91% | 91% | 90% | 93% | 100% | 94% |
| Spec 3 | 6% | 0% | 12% | 10% | 13% | 15% | 12% | 21% | 16% |
| Cutoff 4 | 0.589 | 0.645 | 0.587 | 0.589 | 0.645 | 0.587 | 0.589 | 0.645 | 0.587 |
| Sens 4 | 35% | 32% | 30% | 28% | 18% | 30% | 36% | 29% | 29% |
| Spec 4 | 71% | 70% | 70% | 71% | 70% | 70% | 71% | 70% | 70% |
| Cutoff 5 | 0.695 | 0.799 | 0.717 | 0.695 | 0.799 | 0.717 | 0.695 | 0.799 | 0.717 |
| Sens 5 | 32% | 21% | 27% | 19% | 18% | 20% | 36% | 14% | 29% |
| Spec 5 | 81% | 80% | 80% | 81% | 80% | 80% | 81% | 80% | 80% |
| Cutoff 6 | 0.913 | 1.20 | 0.967 | 0.913 | 1.20 | 0.967 | 0.913 | 1.20 | 0.967 |
| Sens 6 | 24% | 11% | 23% | 17% | 18% | 17% | 21% | 14% | 18% |
| Spec 6 | 90% | 90% | 90% | 90% | 90% | 90% | 90% | 90% | 90% |
| OR Quart 2 | 0.39 | 0.49 | 1.0 | 0.93 | 1.0 | 1.0 | 0.97 | 2.0 | 0.38 |
|  | 0.028 | 0.32 | 1.0 | 0.87 | 0.99 | 1.0 | 0.97 | 0.57 | 0.27 |
| p Value | 0.17 | 0.12 | 0.45 | 0.39 | 0.14 | 0.43 | 0.23 | 0.18 | 0.071 |
| 95% CI of OR Quart2 | 0.90 | 2.0 | 2.2 | 2.2 | 7.3 | 2.3 | 4.2 | 22 | 2.1 |
| OR Quart 3 | 0.44 | 0.32 | 0.63 | 0.91 | 1.5 | 1.1 | 0.23 | 2.0 | 1.2 |
|  | 0.046 | 0.17 | 0.29 | 0.82 | 0.65 | 0.83 | 0.20 | 0.57 | 0.75 |
| p Value | 0.19 | 0.063 | 0.27 | 0.38 | 0.25 | 0.47 | 0.025 | 0.18 | 0.35 |
| 95% CI of OR Quart3 | 0.99 | 1.6 | 1.5 | 2.2 | 9.3 | 2.5 | 2.2 | 23 | 4.3 |

(continued)

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| OR Quart 4 | 1.1 | 1.4 | 1.1 | 1.0 | 2.1 | 1.3 | 1.2 | 2.0 | 0.80 |
|  | 0.85 | 0.57 | 0.89 | 0.95 | 0.41 | 0.57 | 0.75 | 0.57 | 0.75 |
| p Value | 0.51 | 0.46 | 0.48 | 0.43 | 0.37 | 0.56 | 0.31 | 0.18 | 0.20 |
| 95% CI ot OR Quart4 | 2.3 | 4.1 | 2.3 | 2.4 | 12 | 2.9 | 5.0 | 22 | 3.2 |

**Fibroblast growth factor 19**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 0.170 | 0.194 | 0.170 | 0.119 | 0.170 | 0.0550 |
| Average | 0.199 | 0.224 | 0.199 | 0.162 | 0.199 | 0.107 |
| Stdev | 0.167 | 0.236 | 0.167 | 0.209 | 0.167 | 0.129 |
| p(t-test) |  | 0.36 |  | 0.18 |  | 0.040 |
| Min | 8.23E-5 | 0.000127 | 8.23E-5 | 2.92E-5 | 8.23E-5 | 8.23E-5 |
| Max | 0.948 | 1.41 | 0.948 | 1.20 | 0.948 | 0.347 |
| n (Samp) | 157 | 73 | 157 | 56 | 157 | 15 |
| n (Patient) | 88 | 73 | 88 | 56 | 88 | 15 |

| sCr only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 0.145 | 0.203 | 0.145 | 0.136 | 0.145 | 0.103 |
| Average | 0.193 | 0.293 | 0.193 | 0.210 | 0.193 | 0.113 |
| Stdev | 0.190 | 0.307 | 0.190 | 0.196 | 0.190 | 0.0833 |
| p(t-test) |  | 0.027 |  | 0.76 |  | 0.27 |
| Min | 2.92E-5 | 0.000127 | 2.92E-5 | 8.23E-5 | 2.92E-5 | 8.23E-5 |
| Max | 1.41 | 1.32 | 1.41 | 0.619 | 1.41 | 0.254 |
| n (Samp) | 379 | 20 | 379 | 11 | 379 | 7 |
| n (Patient) | 176 | 20 | 176 | 11 | 176 | 7 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.176 | 0.168 | 0.176 | 0.129 | 0.176 | 0.0787 |
| Average | 0.219 | 0.209 | 0.219 | 0.158 | 0.219 | 0.148 |
| Stdev | 0.216 | 0.233 | 0.216 | 0.193 | 0.216 | 0.160 |
| p(t-test) | | 0.75 | | 0.048 | | 0.17 |
| Min | 8.23E-5 | 0.000127 | 8.23E-5 | 2.92E-5 | 8.23E-5 | 8.23E-5 |
| Max | 2.09 | 1.41 | 2.09 | 1.20 | 2.09 | 0.522 |
| n (Samp) | 183 | 64 | 183 | 62 | 183 | 18 |
| n (Patient) | 89 | 64 | 89 | 62 | 89 | 18 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.53 | 0.62 | 0.48 | 0.39 | 0.52 | 0.37 | 0.31 | 0.38 | 0.36 |
| SE | 0.041 | 0.068 | 0.042 | 0.045 | 0.089 | 0.042 | 0.078 | 0.11 | 0.073 |
| p | 0.50 | 0.080 | 0.57 | 0.014 | 0.82 | 0.0016 | 0.014 | 0.29 | 0.062 |
| nCohort 1 | 157 | 379 | 183 | 157 | 379 | 183 | 157 | 379 | 183 |
| nCohort 2 | 73 | 20 | 64 | 56 | 11 | 62 | 15 | 7 | 18 |
| Cutoff 1 | 0.107 | 0.150 | 0.106 | 0.0421 | 0.0860 | 0.0449 | 0.0123 | 0.0885 | 0.0430 |
| Sens 1 | 71% | 70% | 70% | 71% | 73% | 71% | 73% | 71% | 72% |
| Spec 1 | 34% | 51% | 31% | 17% | 33% | 14% | 11% | 33% | 14% |
| Cutoff 2 | 0.0726 | 0.125 | 0.0718 | 0.0375 | 0.0804 | 0.0375 | 8.23E-5 | 0.0423 | 8.23E-5 |
| Sens 2 | 82% | 80% | 81% | 80% | 82% | 81% | 80% | 86% | 83% |
| Spec 2 | 24% | 45% | 20% | 15% | 31% | 12% | 4% | 18% | 2% |
| Cutoff 3 | 0.0209 | 0.0804 | 0.0209 | 0.00457 | 0.0240 | 0.00457 | 0 | 2.92E-5 | 0 |
| Sens 3 | 90% | 90% | 91% | 91% | 91% | 90% | 100% | 100% | 100% |
| Spec 3 | 12% | 31% | 9% | 10% | 12% | 7% | 0% | 1% | 0% |
| Cutoff 4 | 0.255 | 0.238 | 0.267 | 0.255 | 0.238 | 0.267 | 0.255 | 0.238 | 0.267 |
| Sens 4 | 34% | 40% | 25% | 11% | 27% | 11% | 20% | 14% | 28% |
| Spec 4 | 70% | 70% | 71% | 70% | 70% | 71% | 70% | 70% | 71% |
| Cutoff 5 | 0.308 | 0.297 | 0.321 | 0.308 | 0.297 | 0.321 | 0.308 | 0.297 | 0.321 |
| Sens 5 | 16% | 30% | 11% | 11% | 27% | 10% | 13% | 0% | 22% |
| Spec 5 | 80% | 80% | 80% | 80% | 80% | 80% | 80% | 80% | 80% |
| Cutoff 6 | 0.442 | 0.401 | 0.450 | 0.442 | 0.401 | 0.450 | 0.442 | 0.401 | 0.450 |
| Sens 6 | 5% | 15% | 3% | 9% | 18% | 6% | 0% | 0% | 6% |
| Spec 6 | 90% | 90% | 90% | 90% | 90% | 90% | 90% | 90% | 90% |

(continued)

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| OR Quart | 1.5 | 5.2 | 1.0 | 2.9 | 2.0 | 2.6 | 0.32 | >2.1 | 0.49 |
| 2 | 0.34 | 0.14 | 1.0 | 0.048 | 0.42 | 0.060 | 0.33 | <0.56 | 0.42 |
| p Value | 0.66 | 0.59 | 0.44 | 1.0 | 0.36 | 0.96 | 0.032 | >0.18 | 0.086 |
| 95% CI ot OR Quart2 | 3.3 | 45 | 2.3 | 8.2 | 11 | 6.8 | 3.2 | na | 2.8 |
| OR Quart | 1.4 | 6.3 | 1.3 | 3.2 | 1.0 | 3.0 | 0.65 | >3.1 | 0.75 |
| 3 | 0.41 | 0.092 | 0.54 | 0.030 | 1.0 | 0.024 | 0.65 | <0.33 | 0.72 |
| p Value | 0.62 | 0.74 | 0.58 | 1.1 | 0.14 | 1.2 | 0.10 | >0.32 | 0.16 |
| 95% CI of OR Quart3 | 3.1 | 53 | 2.9 | 8.9 | 7.2 | 8.0 | 4.1 | na | 3.5 |
| OR Quart | 1.4 | 8.5 | 1.1 | 5.2 | 1.5 | 4.8 | 3.5 | >2.1 | 2.6 |
| 4 | 0.45 | 0.045 | 0.79 | 0.0013 | 0.66 | 0.0012 | 0.074 | <0.56 | 0.14 |
| p Value | 0.61 | 1.0 | 0.49 | 1.9 | 0.25 | 1.9 | 0.88 | >0.18 | 0.74 |
| 95% CI of OR Quart4 | 3.1 | 69 | 2.5 | 14 | 9.2 | 12 | 14 | na | 9.0 |

**Fibroblast growth factor 21**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 0.0454 | 0.0567 | 0.0454 | 0.0514 | 0.0454 | 0.00987 |
| Average | 0.191 | 0.198 | 0.191 | 0.179 | 0.191 | 0.0861 |
| Stdev | 0.446 | 0.496 | 0.446 | 0.296 | 0.446 | 0.134 |
| p(t-test) |  | 0.91 |  | 0.85 |  | 0.37 |
| Min | 9.65E-6 | 7.01E-5 | 9.65E-6 | 4.60E-6 | 9.65E-6 | 0.00288 |
| Max | 3.21 | 3.36 | 3.21 | 1.29 | 3.21 | 0.438 |
| n (Samp) | 157 | 73 | 157 | 56 | 157 | 15 |
| n (Patient) | 88 | 73 | 88 | 56 | 88 | 15 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.0532 | 0.0603 | 0.0532 | 0.105 | 0.0532 | 0.0913 |
| Average | 0.224 | 0.186 | 0.224 | 0.309 | 0.224 | 0.300 |
| Stdev | 0.578 | 0.355 | 0.578 | 0.413 | 0.578 | 0.421 |
| p(t-test) | | 0.77 | | 0.63 | | 0.73 |
| Min | 4.60E-6 | 0.00263 | 4.60E-6 | 0.0202 | 4.60E-6 | 0.00631 |
| Max | 5.72 | 1.39 | 5.72 | 1.25 | 5.72 | 1.15 |
| n (Samp) | 379 | 20 | 379 | 11 | 379 | 7 |
| n (Patient) | 176 | 20 | 176 | 11 | 176 | 7 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.0535 | 0.0831 | 0.0535 | 0.0496 | 0.0535 | 0.0281 |
| Average | 0.213 | 0.208 | 0.213 | 0.229 | 0.213 | 0.246 |
| Stdev | 0.445 | 0.504 | 0.445 | 0.423 | 0.445 | 0.519 |
| p(t-test) | | 0.94 | | 0.80 | | 0.76 |
| Min | 9.65E-6 | 7.01E-5 | 9.65E-6 | 4.60E-6 | 9.65E-6 | 0.00288 |
| Max | 3.21 | 3.36 | 3.21 | 2.11 | 3.21 | 2.05 |
| n (Samp) | 183 | 64 | 183 | 62 | 183 | 18 |
| n (Patient) | 89 | 64 | 89 | 62 | 89 | 18 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.53 | 0.53 | 0.51 | 0.51 | 0.66 | 0.48 | 0.38 | 0.60 | 0.41 |
| SE | 0.041 | 0.067 | 0.042 | 0.045 | 0.091 | 0.043 | 0.080 | 0.11 | 0.073 |
| p | 0.51 | 0.61 | 0.75 | 0.82 | 0.074 | 0.67 | 0.14 | 0.40 | 0.22 |
| nCohort 1 | 157 | 379 | 183 | 157 | 379 | 183 | 157 | 379 | 183 |
| nCohort 2 | 73 | 20 | 64 | 56 | 11 | 62 | 15 | 7 | 18 |
| Cutoff 1 | 0.0172 | 0.0301 | 0.0209 | 0.0138 | 0.0787 | 0.0139 | 0.00459 | 0.0541 | 0.00472 |
| Sens 1 | 71% | 70% | 70% | 71% | 73% | 71% | 73% | 71% | 72% |
| Spec 1 | 24% | 37% | 23% | 22% | 61% | 15% | 10% | 51% | 7% |
| Cutoff 2 | 0.0111 | 0.0164 | 0.0109 | 0.00901 | 0.0288 | 0.00901 | 0.00440 | 0.00953 | 0.00440 |
| Sens 2 | 81% | 80% | 81% | 80% | 82% | 81% | 80% | 86% | 83% |
| Spec 2 | 20% | 28% | 13% | 18% | 37% | 11% | 10% | 21% | 5% |
| Cutoff 3 | 0.00481 | 0.0157 | 0.00481 | 0.00323 | 0.0241 | 0.00323 | 0.00301 | 0.00624 | 0.00288 |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Sens 3 | 90% | 90% | 91% | 91% | 91% | 90% | 93% | 100% | 94% |
| Spec 3 | 12% | 27% | 7% | 8% | 34% | 3% | 8% | 15% | 3% |
| Cutoff 4 | 0.0859 | 3.121 | 0.120 | 0.0859 | 0.121 | 0.120 | 0.0859 | 0.121 | 0.120 |
| Sens 4 | 44% | 30% | 38% | 43% | 45% | 37% | 27% | 43% | 33% |
| Spec 4 | 70% | 70% | 70% | 70% | 70% | 70% | 70% | 70% | 70% |
| Cutoff 5 | 0.133 | 0.224 | 0.182 | 0.133 | 0.224 | 0.182 | 0.133 | 0.224 | 0.182 |
| Sens 5 | 29% | 10% | 31% | 34% | 27% | 31% | 27% | 29% | 28% |
| Spec 5 | 80% | 80% | 80% | 80% | 80% | 80% | 80% | 80% | 80% |
| Cutoff 6 | 0.546 | 0.500 | 0.584 | 0.546 | 0.500 | 0.584 | 0.546 | 0.500 | 0.584 |
| Sens 6 | 5% | 10% | 5% | 9% | 27% | 8% | 0% | 29% | 11% |
| Spec 6 | 90% | 90% | 90% | 90% | 90% | 90% | 90% | 90% | 90% |
| OR Quart | 0.53 | 4.2 | 0.46 | 0.45 | >3.1 | 0.34 | 0.23 | 0 | 0.15 |
| 2 | 0.14 | 0.073 | 0.080 | 0.081 | <0.34 | 0.016 | 0.20 | na | 0.088 |
| p Value | 0.23 | 0.87 | 0.19 | 0.19 | >0.31 | 0.14 | 0.025 | na | 0.018 |
| 95% CI of OR Quart2 | 1.2 | 20 | 1.1 | 1.1 | na | 0.82 | 2.2 | na | 1.3 |
| OR Quart | 0.79 | 2.6 | 0.76 | 0.35 | >4.2 | 0.43 | 0.48 | 1.0 | 0.48 |
| 3 | 0.55 | 0.27 | 0.51 | 0.027 | <0.21 | 0.048 | 0.41 | 1.0 | 0.32 |
| p Value | 0.36 | 0.48 | 0.34 | 0.13 | >0.46 | 0.19 | 0.082 | 0.14 | 0.11 |
| 95% CI of OR Quart3 | 1.7 | 13 | 1.7 | 0.89 | na | 0.99 | 2.7 | 7.2 | 2.0 |
| OR Quart | 1.2 | 2.6 | 1.2 | 1.1 | >4.1 | 1.0 | 2.2 | 1.5 | 1.4 |
| 4 | 0.61 | 0.27 | 0.60 | 0.74 | <0.21 | 0.95 | 0.22 | 0.66 | 0.54 |
| p Value | 0.57 | 0.48 | 0.57 | 0.52 | >0.45 | 0.49 | 0.62 | 0.25 | 0.46 |
| 95% CI of OR Quart4 | 2.6 | 13 | 2.6 | 2.5 | na | 2.2 | 8.0 | 9.2 | 4.5 |

**Heparin-binding EGF-like growth factor**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 14.8 | 15.5 | 14.8 | 18.1 | 14.8 | 19.0 |
| Average | 17.6 | 20.5 | 17.6 | 26.0 | 17.6 | 22.0 |
| Stdev | 11.1 | 16.7 | 11.1 | 23.4 | 11.1 | 16.8 |
| p(t-test) | | 0.12 | | 5.2E-4 | | 0.17 |
| Min | 5.52 | 7.53 | 5.52 | 5.96 | 5.52 | 5.20 |
| Max | 98.9 | 109 | 98.9 | 113 | 98.9 | 69.4 |
| n (Samp) | 156 | 70 | 156 | 54 | 156 | 14 |
| n (Patient) | 87 | 70 | 87 | 54 | 87 | 14 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 15.6 | 18.1 | 15.6 | 18.3 | 15.6 | 25.9 |
| Average | 18.6 | 24.9 | 18.6 | 23.3 | 18.6 | 33.9 |
| Stdev | 12.4 | 24.3 | 12.4 | 14.1 | 12.4 | 19.4 |
| p(t-test) | | 0.042 | | 0.24 | | 0.0016 |
| Min | 5.20 | 9.73 | 5.20 | 8.57 | 5.20 | 9.67 |
| Max | 113 | 109 | 113 | 50.8 | 113 | 61.5 |
| n (Samp) | 371 | 19 | 371 | 10 | 371 | 7 |
| n (Patient) | 173 | 19 | 173 | 10 | 173 | 7 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 15.5 | 15.5 | 15.5 | 18.1 | 15.5 | 21.1 |
| Average | 20.2 | 19.5 | 20.2 | 26.9 | 20.2 | 21.5 |
| Stdev | 19.3 | 13.6 | 19.3 | 24.7 | 19.3 | 14.6 |
| p(t-test) | | 0.81 | | 0.031 | | 0.79 |
| Min | 5.52 | 7.53 | 5.52 | 5.96 | 5.52 | 5.20 |
| Max | 186 | 73.8 | 186 | 113 | 186 | 69.4 |
| n (Samp) | 180 | 62 | 180 | 60 | 180 | 17 |
| n (Patient) | 88 | 62 | 88 | 60 | 88 | 17 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.56 | 0.60 | 0.52 | 0.62 | 0.60 | 0.61 | 0.57 | 0.78 | 0.58 |
| SE | 0.042 | 0.070 | 0.043 | 0.046 | 0.096 | 0.043 | 0.083 | 0.10 | 0.075 |
| p | 0.14 | 0.14 | 0.60 | 0.0082 | 0.29 | 0.014 | 0.41 | 0.0073 | 0.28 |
| nCohort 1 | 156 | 371 | 180 | 156 | 371 | 180 | 156 | 371 | 180 |
| nCohort 2 | 70 | 19 | 62 | 54 | 10 | 60 | 14 | 7 | 17 |
| Cutoff 1 | 13.6 | 14.7 | 13.2 | 15.8 | 17.4 | 15.9 | 11.5 | 22.5 | 17.1 |
| Sens 1 | 70% | 74% | 71% | 70% | 70% | 70% | 71% | 71% | 71% |
| Spec 1 | 45% | 45% | 39% | 54% | 61% | 51% | 26% | 80% | 57% |
| Cutoff 2 | 12.8 | 13.8 | 12.4 | 12.6 | 12.1 | 13.7 | 8.14 | 21.3 | 11.2 |
| Sens 2 | 80% | 84% | 81% | 81% | 80% | 80% | 86% | 86% | 82% |
| Spec 2 | 38% | 39% | 33% | 38% | 26% | 42% | 6% | 78% | 21% |
| Cutoff 3 | 10.4 | 11.5 | 10.3 | 10.2 | 8.69 | 10.4 | 5.52 | 9.66 | 5.52 |
| Sens 3 | 90% | 95% | 90% | 91% | 90% | 90% | 93% | 100% | 94% |
| Spec 3 | 18% | 22% | 16% | 17% | 7% | 17% | 1% | 10% | 1% |
| Cutoff 4 | 19.4 | 19.5 | 20.2 | 19.4 | 19.5 | 20.2 | 19.4 | 19.5 | 20.2 |
| Sens 4 | 27% | 37% | 26% | 37% | 40% | 33% | 50% | 86% | 59% |
| Spec 4 | 71% | 70% | 70% | 71% | 70% | 70% | 71% | 70% | 70% |
| Cutoff 5 | 21.3 | 22.5 | 22.8 | 21.3 | 22.5 | 22.8 | 21.3 | 22.5 | 22.8 |
| Sens 5 | 21% | 16% | 18% | 31% | 40% | 27% | 36% | 71% | 41% |
| Spec 5 | 80% | 80% | 80% | 80% | 80% | 80% | 80% | 80% | 80% |
| Cutoff 6 | 26.6 | 29.0 | 31.2 | 26.6 | 29.0 | 31.2 | 26.6 | 29.0 | 31.2 |
| Sens 6 | 13% | 11% | 6% | 24% | 30% | 23% | 21% | 43% | 12% |
| Spec 6 | 90% | 90% | 90% | 90% | 90% | 90% | 90% | 90% | 90% |
| OR Quart 2 | 3.1 | 2.6 | 2.2 | 1.4 | 0.49 | 2.0 | 0.23 | 0 | 0 |
| | 0.010 | 0.27 | 0.072 | 0.48 | 0.57 | 0.16 | 0.19 | na | na |
| p Value | 1.3 | 0.48 | 0.93 | 0.53 | 0.044 | 0.76 | 0.024 | na | na |
| 95% CI of OR Quart2 | 7.4 | 13 | 5.1 | 3.9 | 5.5 | 5.1 | 2.1 | na | na |
| OR Quart 3 | 2.4 | 3.1 | 1.8 | 2.9 | 1.5 | 3.2 | 0.73 | 0 | 1.0 |
| | 0.055 | 0.17 | 0.20 | 0.027 | 0.65 | 0.012 | 0.69 | na | 1.0 |
| p Value | 0.98 | 0.62 | 0.74 | 1.1 | 0.25 | 1.3 | 0.15 | na | 0.27 |
| 95% CI of OR Quart3 | 5.7 | 16 | 4.2 | 7.5 | 9.3 | 8.1 | 3.5 | na | 3.7 |

(continued)

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| OR Quart 4 | 2.1 | 3.1 | 1.3 | 2.6 | 2.0 | 2.8 | 1.5 | 6.3 | 1.4 |
|  | 0.095 | 0.17 | 0.53 | 0.049 | 0.42 | 0.030 | 0.53 | 0.092 | 0.56 |
| p Value | 0.88 | 0.61 | 0.55 | 1.0 | 0.36 | 1.1 | 0.40 | 0.74 | 0.42 |
| 95% CI of OR Quart4 | 5.1 | 16 | 3.2 | 6.7 | 11 | 7.0 | 5.9 | 53 | 4.9 |

**Tenascin**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 477 | 610 | 477 | 714 | 477 | 860 |
| Average | 830 | 1060 | 830 | 1140 | 830 | 938 |
| Stdev | 1330 | 1520 | 1330 | 1500 | 1330 | 817 |
| p(t-test) |  | 0.24 |  | 0.15 |  | 0.76 |
| Min | 94.2 | 86.2 | 94.2 | 160 | 94.2 | 143 |
| Max | 13100 | 9040 | 13100 | 9500 | 13100 | 3300 |
| n (Samp) | 158 | 73 | 158 | 55 | 158 | 15 |
| n (Patient) | 88 | 73 | 88 | 55 | 88 | 15 |

| sCr only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 564 | 756 | 564 | 1140 | 564 | 642 |
| Average | 1010 | 1070 | 1010 | 1470 | 1010 | 664 |
| Stdev | 1680 | 983 | 1680 | 1470 | 1680 | 226 |
| p(t-test) |  | 0.89 |  | 0.37 |  | 0.58 |
| Min | 43.9 | 205 | 43.9 | 180 | 43.9 | 385 |
| Max | 18400 | 4250 | 18400 | 5760 | 18400 | 1030 |
| n (Samp) | 378 | 20 | 378 | 11 | 378 | 7 |
| n (Patient) | 175 | 20 | 175 | 11 | 175 | 7 |

| UO only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 527 | 668 | 527 | 684 | 527 | 852 |

(continued)

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Average | 1330 | 1100 | 1330 | 1050 | 1330 | 942 |
| Stdev | 2900 | 1570 | 2900 | 1320 | 2900 | 738 |
| p(t-test) | | 0.55 | | 0.46 | | 0.57 |
| Min | 63.0 | 86.2 | 63.0 | 160 | 63.0 | 143 |
| Max | 26100 | 9040 | 26100 | 9500 | 26100 | 3300 |
| n (Samp) | 184 | 64 | 184 | 61 | 184 | 18 |
| n (Patient) | 89 | 64 | 89 | 61 | 89 | 18 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.60 | 0.59 | 0.56 | 0.60 | 0.73 | 0.55 | 0.61 | 0.55 | 0.59 |
| SE | 0.041 | 0.068 | 0.042 | 0.046 | 0.088 | 0.043 | 0.080 | 0.11 | 0.073 |
| p | 0.019 | 0.18 | 0.18 | 0.027 | 0.0098 | 0.22 | 0.15 | 0.67 | 0.21 |
| nCohort 1 | 158 | 378 | 184 | 158 | 378 | 184 | 158 | 378 | 184 |
| nCohort 2 | 73 | 20 | 64 | 55 | 11 | 61 | 15 | 7 | 18 |
| Cutoff 1 | 417 | 486 | 434 | 357 | 1030 | 394 | 411 | 624 | 565 |
| Sens 1 | 71% | 70% | 70% | 71% | 73% | 70% | 73% | 71% | 72% |
| Spec 1 | 41% | 44% | 40% | 37% | 76% | 37% | 41% | 56% | 55% |
| Cutoff 2 | 371 | 450 | 379 | 256 | 901 | 265 | 379 | 417 | 411 |
| Sens 2 | 81% | 80% | 81% | 80% | 82% | 80% | 80% | 86% | 83% |
| Spec 2 | 40% | 39% | 36% | 18% | 70% | 20% | 41% | 35% | 38% |
| Cutoff 3 | 221 | 257 | 221 | 228 | 720 | 240 | 162 | 382 | 162 |
| Sens 3 | 90% | 90% | 91% | 91% | 91% | 90% | 93% | 100% | 94% |
| Spec 3 | 15% | 16% | 15% | 16% | 62% | 16% | 7% | 32% | 7% |
| Cutoff 4 | 678 | 901 | 886 | 678 | 901 | 886 | 678 | 901 | 886 |
| Sens 4 | 47% | 40% | 34% | 55% | 82% | 43% | 60% | 14% | 39% |
| Spec 4 | 70% | 70% | 70% | 70% | 70% | 70% | 70% | 70% | 70% |
| Cutoff 5 | 895 | 1180 | 1200 | 895 | 1180 | 1200 | 895 | 1180 | 1200 |
| Sens 5 | 34% | 35% | 23% | 45% | 45% | 31% | 40% | 0% | 17% |
| Spec 5 | 80% | 80% | 80% | 80% | 80% | 80% | 80% | 80% | 80% |
| Cutoff 6 | 1840 | 2000 | 2700 | 1840 | 2000 | 2700 | 1840 | 2000 | 2700 |
| Sens 6 | 10% | 10% | 8% | 13% | 9% | 5% | 13% | 0% | 6% |
| Spec 6 | 91% | 90% | 90% | 91% | 90% | 90% | 91% | 90% | 90% |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| OR Quart | 1.4 | 1.3 | 1.5 | 0.50 | 0 | 0.82 | 0.65 | >2.0 | 0.64 |
| 2 | 0.43 | 0.71 | 0.38 | 0.16 | na | 0.66 | 0.65 | <0.56 | 0.63 |
| p Value | 0.61 | 0.29 | 0.62 | 0.19 | na | 0.34 | 0.10 | >0.18 | 0.10 |
| 95% CI of OR Quart2 | 3.2 | 6.1 | 3.5 | 1.3 | na | 2.0 | 4.1 | na | 4.0 |
| OR Quart | 1.5 | 2.1 | 2.2 | 0.65 | 2.0 | 1.0 | 1.0 | >4.2 | 3.0 |
| 3 | 0.32 | 0.32 | 0.065 | 0.36 | 0.57 | 1.0 | 1.0 | <0.21 | 0.12 |
| p Value | 0.66 | 0.50 | 0.95 | 0.26 | 0.18 | 0.43 | 0.19 | >0.46 | 0.74 |
| 95% CI of OR Quart3 | 3.5 | 8.5 | 5.1 | 1.6 | 23 | 2.3 | 5.3 | na | 12 |
| OR Quart | 2.6 | 2.4 | 1.9 | 2.1 | 8.5 | 1.7 | 2.5 | >1.0 | 1.7 |
| 4 | 0.022 | 0.21 | 0.14 | 0.081 | 0.045 | 0.18 | 0.20 | <1.0 | 0.48 |
| p Value | 1.1 | 0.60 | 0.81 | 0.92 | 1.0 | 0.78 | 0.61 | >0.062 | 0.38 |
| 95% CI of OR Quart4 | 5.8 | 9.6 | 4.4 | 4.7 | 70 | 3.8 | 10 | na | 7.5 |

Fig. 6: Comparison of marker levels in EDTA samples collected from Cohort 1 (patients that did not progress beyond RIFLE stage 0 or R) and in EDTA samples collected from subjects at 0,24 hours, and 48 hours prior to reaching stage I or F in Cohort 2.

**Angiopoietin-related protein 4**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 31.6 | 39.3 | 31.6 | 42.7 | 31.6 | 47.1 |
| Average | 48.9 | 60.6 | 48.9 | 69.8 | 48.9 | 58.5 |
| Stdev | 111 | 63.3 | 111 | 69.1 | 111 | 48.0 |
| p(t-test) | | 0.58 | | 0.28 | | 0.69 |
| Min | 1.77 | 9.35 | 1.77 | 12.8 | 1.77 | 8.79 |
| Max | 1900 | 243 | 1900 | 339 | 1900 | 179 |
| n (Samp) | 356 | 28 | 356 | 34 | 356 | 22 |
| n (Patient) | 175 | 28 | 175 | 34 | 175 | 22 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 32.7 | 39.3 | 32.7 | 44.3 | 32.7 | 49.6 |
| Average | 50.3 | 60.2 | 50.3 | 69.5 | 50.3 | 61.2 |
| Stdev | 112 | 63.1 | 112 | 68.2 | 112 | 49.3 |
| p(t-test) | | 0.64 | | 0.32 | | 0.67 |
| Min | 2.68 | 9.35 | 2.68 | 12.8 | 2.68 | 8.79 |
| Max | 1900 | 243 | 1900 | 339 | 1900 | 179 |
| n (Samp) | 347 | 28 | 347 | 35 | 347 | 20 |
| n (Patient) | 161 | 28 | 161 | 35 | 161 | 20 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.59 | nd | 0.58 | 0.65 | nd | 0.64 | 0.63 | nd | 0.63 |
| SE | 0.058 | nd | 0.058 | 0.053 | nd | 0.052 | 0.065 | nd | 0.069 |
| p | 0.11 | nd | 0.18 | 0.0043 | nd | 0.0068 | 0.050 | nd | 0.054 |
| nCohort 1 | 356 | nd | 347 | 356 | nd | 347 | 356 | nd | 347 |
| nCohort 2 | 28 | nd | 28 | 34 | nd | 35 | 22 | nd | 20 |
| Cutoff 1 | 29.1 | nd | 29.1 | 30.3 | nd | 30.3 | 25.8 | nd | 28.9 |
| Sens 1 | 71% | nd | 71% | 71% | nd | 71% | 73% | nd | 70% |
| Spec 1 | 47% | nd | 45% | 48% | nd | 46% | 42% | nd | 44% |
| Cutoff 2 | 19.0 | nd | 19.0 | 25.0 | nd | 27.0 | 22.1 | nd | 24.9 |
| Sens 2 | 82% | nd | 82% | 82% | nd | 80% | 82% | nd | 80% |
| Spec 2 | 30% | nd | 27% | 41% | nd | 41% | 37% | nd | 38% |
| Cutoff 3 | 13.6 | nd | 13.6 | 22.1 | nd | 22.1 | 15.3 | nd | 17.6 |
| Sens 3 | 93% | nd | 93% | 91% | nd | 91% | 91% | nd | 90% |
| Spec 3 | 19% | nd | 16% | 37% | nd | 34% | 22% | nd | 24% |
| Cutoff 4 | 44.8 | nd | 45.5 | 44.8 | nd | 45.5 | 44.8 | nd | 45.5 |
| Sens 4 | 36% | nd | 36% | 47% | nd | 46% | 64% | nd | 65% |
| Spec 4 | 70% | nd | 70% | 70% | nd | 70% | 70% | nd | 70% |
| Cutoff 5 | 61.9 | nd | 62.7 | 61.9 | nd | 62.7 | 61.9 | nd | 62.7 |
| Sens 5 | 25% | nd | 25% | 32% | nd | 31% | 23% | nd | 25% |
| Spec 5 | 80% | nd | 80% | 80% | nd | 80% | 80% | nd | 80% |
| Cutoff 6 | 87.8 | nd | 88.3 | 87.8 | nd | 88.3 | 87.8 | nd | 88.3 |
| Sens 6 | 21% | nd | 18% | 24% | nd | 23% | 18% | nd | 20% |
| Spec 6 | 90% | nd | 90% | 90% | nd | 90% | 90% | nd | 90% |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| OR Quart | 1.2 | nd | 1.4 | 5.4 | nd | 6.6 | 1.7 | nd | 1.3 |
| 2 | 0.76 | nd | 0.57 | 0.033 | nd | 0.015 | 0.48 | nd | 0.71 |
| p Value | 0.36 | nd | 0.43 | 1.2 | nd | 1.4 | 0.39 | nd | 0.29 |
| 95% CI of OR Quart2 | 4.1 | nd | 4.6 | 25 | nd | 31 | 7.3 | nd | 6.1 |
| OR Quart | 1.7 | nd | 1.6 | 5.5 | nd | 4.3 | 1.7 | nd | 1.7 |
| 3 | 0.39 | nd | 0.40 | 0.031 | nd | 0.071 | 0.47 | nd | 0.48 |
| p Value | 0.52 | nd | 0.52 | 1.2 | nd | 0.88 | 0.40 | nd | 0.39 |
| 95% CI of OR Quart3 | 5.3 | nd | 5.2 | 26 | nd | 21 | 7.3 | nd | 7.3 |
| OR Quart | 1.9 | nd | 1.6 | 6.6 | nd | 7.3 | 3.2 | nd | 2.8 |
| 4 | 0.27 | nd | 0.40 | 0.015 | nd | 0.010 | 0.091 | nd | 0.14 |
| p Value | 0.61 | nd | 0.52 | 1.4 | nd | 1.6 | 0.83 | nd | 0.72 |
| 95% CI OR Quart4 | 5.8 | nd | 5.2 | 30 | nd | 33 | 12 | nd | 11 |

**Amphiregulin**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 22.0 | 26.7 | 22.0 | 26.2 | 22.0 | 23.7 |
| Average | 27.4 | 29.5 | 27.4 | 33.1 | 27.4 | 41.5 |
| Stdev | 21.5 | 20.0 | 21.5 | 24.5 | 21.5 | 41.2 |
| p(t-test) | | 0.61 | | 0.16 | | 0.0056 |
| Min | 0.00246 | 0.00246 | 0.00246 | 4.44 | 0.00246 | 2.76 |
| Max | 198 | 93.0 | 198 | 133 | 198 | 162 |
| n (Samp) | 349 | 28 | 349 | 32 | 349 | 22 |
| n (Patient) | 172 | 28 | 172 | 32 | 172 | 22 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 21.7 | 26.7 | 21.7 | 25.1 | 21.7 | 23.7 |
| Average | 27.1 | 29.8 | 27.1 | 32.8 | 27.1 | 40.0 |
| Stdev | 21.5 | 20.0 | 21.5 | 24.2 | 21.5 | 40.9 |
| p(t-test) | | 0.53 | | 0.16 | | 0.016 |
| Min | 0.00246 | 0.00246 | 0.00246 | 4.44 | 0.00246 | 2.76 |
| Max | 198 | 93.0 | 198 | 133 | 198 | 162 |
| n (Samp) | 340 | 28 | 340 | 33 | 340 | 20 |
| n (Patient) | 158 | 28 | 158 | 33 | 158 | 20 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.55 | nd | 0.56 | 0.58 | nd | 0.58 | 0.55 | nd | 0.55 |
| SE | 0.058 | nd | 0.058 | 0.055 | nd | 0.054 | 0.065 | nd | 0.068 |
| p | 0.38 | nd | 0.31 | 0.13 | nd | 0.12 | 0.40 | nd | 0.49 |
| nCohort 1 | 349 | nd | 340 | 349 | nd | 340 | 349 | nd | 340 |
| nCohort 2 | 28 | nd | 28 | 32 | nd | 33 | 22 | nd | 20 |
| Cutoff 1 | 17.0 | nd | 17.0 | 18.8 | nd | 18.8 | 14.3 | nd | 14.3 |
| Sens 1 | 71% | nd | 71% | 72% | nd | 73% | 73% | nd | 70% |
| Spec 1 | 36% | nd | 37% | 41% | nd | 42% | 27% | nd | 27% |
| Cutoff 2 | 12.3 | nd | 12.3 | 17.0 | nd | 17.0 | 11.7 | nd | 11.7 |
| Sens 2 | 82% | nd | 82% | 81% | nd | 82% | 82% | nd | 80% |
| Spec 2 | 18% | nd | 18% | 36% | nd | 37% | 17% | nd | 17% |
| Cutoff 3 | 8.57 | nd | 8.57 | 12.3 | nd | 12.3 | 11.0 | nd | 11.0 |
| Sens 3 | 93% | nd | 93% | 91% | nd | 91% | 91% | nd | 90% |
| Spec 3 | 10% | nd | 10% | 18% | nd | 18% | 15% | nd | 15% |
| Cutoff 4 | 30.9 | nd | 30.4 | 30.9 | nd | 30.4 | 30.9 | nd | 30.4 |
| Sens 4 | 39% | nd | 43% | 47% | nd | 45% | 36% | nd | 35% |
| Spec 4 | 71% | nd | 70% | 71% | nd | 70% | 71% | nd | 70% |
| Cutoff 5 | 39.0 | nd | 37.0 | 39.0 | nd | 37.0 | 39.0 | nd | 37.0 |
| Sens 5 | 25% | nd | 29% | 25% | nd | 36% | 36% | nd | 35% |
| Spec 5 | 81% | nd | 80% | 81% | nd | 80% | 81% | nd | 80% |
| Cutoff 6 | 52.6 | nd | 50.7 | 52.6 | nd | 50.7 | 52.6 | nd | 50.7 |
| Sens 6 | 11% | nd | 11% | 16% | nd | 18% | 27% | nd | 25% |
| Spec 6 | 90% | nd | 90% | 90% | nd | 90% | 90% | nd | 90% |

(continued)

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| OR Quart | 0.55 | nd | 0.55 | 1.9 | nd | 2.1 | 0.99 | nd | 0.79 |
| 2 | 0.36 | nd | 0.36 | 0.27 | nd | 0.19 | 0.99 | nd | 0.73 |
| p Value | 0.16 | nd | 0.16 | 0.61 | nd | 0.70 | 0.28 | nd | 0.21 |
| 95% CI of OR Quart2 | 2.0 | nd | 2.0 | 5.8 | nd | 6.5 | 3.5 | nd | 3.0 |
| OR Quart | 1.2 | nd | 1.2 | 1.2 | nd | 1.0 | 0.78 | nd | 0.79 |
| 3 | 0.79 | nd | 0.79 | 0.76 | nd | 1.0 | 0.72 | nd | 0.73 |
| p Value | 0.40 | nd | 0.40 | 0.36 | nd | 0.28 | 0.20 | nd | 0.21 |
| 95% CI of OR Quart3 | 3.3 | nd | 3.3 | 4.1 | nd | 3.6 | 3.0 | nd | 3.0 |
| OR Quart | 1.3 | nd | 1.3 | 2.6 | nd | 2.8 | 1.6 | nd | 1.4 |
| 4 | 0.62 | nd | 0.60 | 0.088 | nd | 0.058 | 0.40 | nd | 0.55 |
| p Value | 0.46 | nd | 0.47 | 0.87 | nd | 0.96 | 0.52 | nd | 0.44 |
| 95% CI of OR Quart4 | 3.6 | nd | 3.7 | 7.6 | nd | 8.3 | 5.2 | nd | 4.7 |

**Betacellulin**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 3.86 | 3.89 | 3.86 | 4.40 | 3.86 | 4.54 |
| Average | 4.76 | 4.02 | 4.76 | 6.09 | 4.76 | 6.45 |
| Stdev | 5.85 | 2.57 | 5.85 | 7.48 | 5.85 | 6.05 |
| p(t-test) |  | 0.50 |  | 0.22 |  | 0.19 |
| Min | 0.00226 | 0.00282 | 0.00226 | 0.00289 | 0.00226 | 0.00274 |
| Max | 60.7 | 10.0 | 60.7 | 43.4 | 60.7 | 24.5 |
| n (Samp) | 349 | 28 | 349 | 33 | 349 | 22 |
| n (Patient) | 173 | 28 | 173 | 33 | 173 | 22 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 3.73 | 3.63 | 3.73 | 4.36 | 3.73 | 4.54 |
| Average | 4.62 | 3.87 | 4.62 | 6.00 | 4.62 | 6.21 |
| Stdev | 5.86 | 2.68 | 5.86 | 7.39 | 5.86 | 5.63 |
| p(t-test) | | 0.50 | | 0.20 | | 0.24 |
| Min | 0.00226 | 0.00282 | 0.00226 | 0.00289 | 0.00226 | 0.700 |
| Max | 60.7 | 10.0 | 60.7 | 43.4 | 60.7 | 24.5 |
| n (Samp) | 340 | 28 | 340 | 34 | 340 | 20 |
| n (Patient) | 159 | 28 | 159 | 34 | 159 | 20 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.50 | nd | 0.49 | 0.57 | nd | 0.57 | 0.58 | nd | 0.60 |
| SE | 0.057 | nd | 0.057 | 0.054 | nd | 0.053 | 0.065 | nd | 0.069 |
| | 0hr prior to AKI stage | | | 24hr prior to AKI stage | | | 48hr prior to AKI stage | | |
| | sCr or UO | sCr only | UO only | sCr or UO | sCr only | UO only | sCr or UO | sCr only | UO only |
| p | 0.97 | nd | 0.90 | 0.23 | nd | 0.18 | 0.24 | nd | 0.16 |
| nCohort 1 | 349 | nd | 340 | 349 | nd | 340 | 349 | nd | 340 |
| nCohort 2 | 28 | nd | 28 | 33 | nd | 34 | 22 | nd | 20 |
| Cutoff 1 | 3.11 | nd | 2.65 | 3.09 | nd | 3.09 | 2.89 | nd | 3.54 |
| Sens 1 | 71% | nd | 71% | 73% | nd | 71% | 73% | nd | 70% |
| Spec 1 | 38% | nd | 35% | 38% | nd | 39% | 35% | nd | 46% |
| Cutoff 2 | 1.34 | nd | 0.549 | 2.62 | nd | 2.62 | 2.26 | nd | 2.65 |
| Sens 2 | 82% | nd | 82% | 82% | nd | 82% | 82% | nd | 80% |
| Spec 2 | 16% | nd | 14% | 32% | nd | 34% | 26% | nd | 35% |
| Cutoff 3 | 0.00282 | nd | 0.00282 | 1.78 | nd | 1.78 | 0.743 | nd | 1.92 |
| Sens 3 | 96% | nd | 96% | 91% | nd | 91% | 91% | nd | 90% |
| Spec 3 | 5% | nd | 6% | 23% | nd | 25% | 13% | nd | 26% |
| Cutoff 4 | 5.42 | nd | 5.30 | 5.42 | nd | 5.30 | 5.42 | nd | 5.30 |
| Sens 4 | 25% | nd | 32% | 33% | nd | 32% | 36% | nd | 35% |
| Spec 4 | 70% | nd | 71% | 70% | nd | 71% | 70% | nd | 71% |
| Cutoff 5 | 6.65 | nd | 6.31 | 6.65 | nd | 6.31 | 6.65 | nd | 6.31 |
| Sens 5 | 14% | nd | 18% | 24% | nd | 26% | 32% | nd | 30% |
| Spec 5 | 80% | nd | 80% | 80% | nd | 80% | 80% | nd | 80% |
| Cutoff 6 | 8.60 | nd | 8.27 | 8.60 | nd | 8.27 | 8.60 | nd | 8.27 |

(continued)

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| Sens 6 | 4% | nd | 4% | 15% | nd | 18% | 23% | nd | 20% |
| Spec 6 | 90% | nd | 90% | 90% | nd | 90% | 90% | nd | 90% |
| OR Quart 2 | 1.4 | nd | 1.2 | 1.6 | nd | 1.6 | 1.2 | nd | 2.6 |
|  | 0.57 | nd | 0.77 | 0.40 | nd | 0.40 | 0.75 | nd | 0.26 |
| p Value | 0.46 | nd | 0.38 | 0.52 | nd | 0.52 | 0.32 | nd | 0.49 |
| 95% CI of OR Quart2 | 4.1 | nd | 3.7 | 5.2 | nd | 5.2 | 4.8 | nd | 14 |
| OR Quart 3 | 1.4 | nd | 1.4 | 2.1 | nd | 2.4 | 1.5 | nd | 3.7 |
|  | 0.57 | nd | 0.58 | 0.19 | nd | 0.13 | 0.53 | nd | 0.11 |
| p Value | 0.46 | nd | 0.45 | 0.70 | nd | 0.79 | 0.41 | nd | 0.75 |
| 95% CI of OR Quart3 | 4.1 | nd | 4.1 | 6.4 | nd | 7.1 | 5.6 | nd | 18 |
| OR Quart 4 | 1.0 | nd | 1.2 | 2.1 | nd | 2.1 | 1.8 | nd | 3.1 |
|  | 0.98 | nd | 0.77 | 0.19 | nd | 0.19 | 0.37 | nd | 0.17 |
| p Value | 0.31 | nd | 0.38 | 0.69 | nd | 0.69 | 0.51 | nd | 0.62 |
| 95% CI of OR Quart4 | 3.3 | nd | 3.7 | 6.4 | nd | 6.4 | 6.3 | nd | 16 |

**Endostatin**

| sCr or UO | 0hr prior to AKI stage | | 24hr prior to | AKI stage | 48hr prior to AKI stage | |
|---|---|---|---|---|---|---|
|  | Cohort 1 | Cohort 2 | Cohort 1 | Cohort 2 | Cohort 1 | Cohort 2 |
| Median | 39200 | 48000 | 39200 | 33300 | 39200 | 47800 |
| sCr or UO | 0hr prior to AKI stage | | 24hr prior to AKI stage | | 48hr prior to AKI stage | |
|  | Cohort 1 | Cohort 2 | Cohort 1 | Cohort 2 | Cohort 1 | Cohort 2 |
| Average | 49200 | 62300 | 49200 | 53400 | 49200 | 46300 |
| Stdev | 41200 | 42600 | 41200 | 51700 | 41200 | 25400 |
| p(t-test) |  | 0.11 |  | 0.59 |  | 0.74 |
| Min | 3600 | 13300 | 3600 | 10400 | 3600 | 12800 |
| Max | 483000 | 180000 | 483000 | 239000 | 483000 | 104000 |
| n (Samp) | 357 | 28 | 357 | 34 | 357 | 22 |
| n (Patient) | 175 | 28 | 175 | 34 | 175 | 22 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 40400 | 48000 | 40400 | 34000 | 40400 | 41700 |
| Average | 49800 | 59800 | 49800 | 55800 | 49800 | 44900 |
| Stdev | 41400 | 38100 | 41400 | 52800 | 41400 | 26200 |
| p(t-test) | | 0.22 | | 0.43 | | 0.60 |
| Min | 3600 | 13300 | 3600 | 10400 | 3600 | 12800 |
| Max | 483000 | 180000 | 483000 | 239000 | 483000 | 104000 |
| n (Samp) | 348 | 28 | 348 | 35 | 348 | 20 |
| n (Patient) | 161 | 28 | 161 | 35 | 161 | 20 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.60 | nd | 0.59 | 0.47 | nd | 0.48 | 0.52 | nd | 0.48 |
| SE | 0.058 | nd | 0.058 | 0.053 | nd | 0.052 | 0.064 | nd | 0.067 |
| p | 0.094 | nd | 0.13 | 0.62 | nd | 0.74 | 0.81 | nd | 0.81 |
| nCohort 1 | 357 | nd | 348 | 357 | nd | 348 | 357 | nd | 348 |
| nCohort 2 | 28 | nd | 28 | 34 | nd | 35 | 22 | nd | 20 |
| Cutoff 1 | 33300 | nd | 33300 | 25600 | nd | 25600 | 31800 | nd | 31800 |
| Sens 1 | 71% | nd | 71% | 71% | nd | 71% | 73% | nd | 70% |
| Spec 1 | 39% | nd | 38% | 22% | nd | 22% | 34% | nd | 33% |
| Cutoff 2 | 30600 | nd | 30600 | 18900 | nd | 21000 | 18300 | nd | 18300 |
| Sens 2 | 82% | nd | 82% | 85% | nd | 80% | 82% | nd | 80% |
| Spec 2 | 32% | nd | 31% | 12% | nd | 14% | 11% | nd | 10% |
| Cutoff 3 | 19600 | nd | 19600 | 13600 | nd | 13600 | 14200 | nd | 14200 |
| Sens 3 | 93% | nd | 93% | 91% | nd | 91% | 91% | nd | 90% |
| Spec 3 | 13% | nd | 12% | 6% | nd | 5% | 6% | nd | 5% |
| Cutoff 4 | 53100 | nd | 53500 | 53100 | nd | 53500 | 53100 | nd | 53500 |
| Sens 4 | 46% | nd | 46% | 32% | nd | 34% | 41% | nd | 35% |
| Spec 4 | 70% | nd | 70% | 70% | nd | 70% | 70% | nd | 70% |
| Cutoff 5 | 65300 | nd | 65900 | 65300 | nd | 65900 | 65300 | nd | 65900 |
| Sens 5 | 39% | nd | 39% | 29% | nd | 29% | 27% | nd | 25% |
| Spec 5 | 80% | nd | 80% | 80% | nd | 80% | 80% | nd | 80% |
| Cutoff 6 | 88800 | nd | 88800 | 88800 | nd | 88800 | 88800 | nd | 88800 |
| Sens 6 | 21% | nd | 21% | 12% | nd | 14% | 5% | nd | 5% |
| Spec 6 | 90% | nd | 90% | 90% | nd | 90% | 90% | nd | 90% |
| OR Quart | 1.4 | nd | 1.7 | 0.34 | nd | 0.30 | 0.64 | nd | 0.65 |
| 2 | 0.55 | nd | 0.39 | 0.071 | nd | 0.046 | 0.51 | nd | 0.52 |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| p Value | 0.44 | nd | 0.52 | 0.10 | nd | 0.094 | 0.18 | nd | 0.18 |
| 95% CI of OR Quart2 | 4.7 | nd | 5.3 | 1.1 | nd | 0.98 | 2.4 | nd | 2.4 |
| OR Quart 3 | 0.59 | nd | 0.39 | 0.61 | nd | 0.55 | 0.81 | nd | 0.65 |
| | 0.47 | nd | 0.26 | 0.33 | nd | 0.23 | 0.74 | nd | 0.52 |
| p Value | 0.14 | nd | 0.073 | 0.23 | nd | 0.21 | 0.24 | nd | 0.18 |
| 95% CI of OR Quart3 | 2.5 | nd | 2.0 | 1.6 | nd | 1.5 | 2.8 | nd | 2.4 |
| OR Quart 4 | 2.8 | nd | 2.9 | 1.1 | nd | 1.0 | 1.2 | nd | 1.0 |
| | 0.059 | nd | 0.056 | 0.80 | nd | 0.98 | 0.79 | nd | 1.0 |
| p Value | 0.96 | nd | 0.98 | 0.47 | nd | 0.43 | 0.38 | nd | 0.31 |
| 95% CI ot OR Quart4 | 8.2 | nd | 8.4 | 2.7 | nd | 2.4 | 3.6 | nd | 3.2 |

**Proepiregulin**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.383 | 0.437 | 0.383 | 0.406 | 0.383 | 0.360 |
| Average | 0.636 | 2.03 | 0.636 | 1.81 | 0.636 | 0.789 |
| Stdev | 1.83 | 4.87 | 1.83 | 5.61 | 1.83 | 0.797 |
| p(t-test) | | 0.0014 | | 0.0072 | | 0.70 |
| Min | 0.000152 | 0.0957 | 0.000152 | 0.0249 | 0.000152 | 0.0995 |
| Max | 32.1 | 20.6 | 32.1 | 31.1 | 32.1 | 2.56 |
| n (Samp) | 349 | 28 | 349 | 33 | 349 | 22 |
| n (Patient) | 172 | 28 | 172 | 33 | 172 | 22 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.383 | 0.437 | 0.383 | 0.399 | 0.383 | 0.360 |
| Average | 0.637 | 2.04 | 0.637 | 1.77 | 0.637 | 0.741 |
| Stdev | 1.86 | 4.87 | 1.86 | 5.53 | 1.86 | 0.759 |
| p(t-test) | | 0.0015 | | 0.0097 | | 0.80 |
| Min | 0.000152 | 0.0957 | 0.000152 | 0.0249 | 0.000152 | 0.0995 |
| Max | 32.1 | 20.6 | 32.1 | 31.1 | 32.1 | 2.56 |
| n (Samp) | 340 | 28 | 340 | 34 | 340 | 20 |

(continued)

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| n (Patient) | 158 | 28 | 158 | 34 | 158 | 20 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.58 | nd | 0.60 | 0.57 | nd | 0.56 | 0.56 | nd | 0.55 |
| SE | 0.058 | nd | 0.059 | 0.054 | nd | 0.053 | 0.065 | nd | 0.068 |
| p | 0.16 | nd | 0.098 | 0.22 | nd | 0.23 | 0.33 | nd | 0.43 |
| nCohort 1 | 349 | nd | 340 | 349 | nd | 340 | 349 | nd | 340 |
| nCohort 2 | 28 | nd | 28 | 33 | nd | 34 | 22 | nd | 20 |
| Cutoff 1 | 0.321 | nd | 0.356 | 0.293 | nd | 0.293 | 0.200 | nd | 0.200 |
| Sens 1 | 71% | nd | 71% | 73% | nd | 71% | 73% | nd | 70% |
| Spec 1 | 41% | nd | 46% | 38% | nd | 39% | 28% | nd | 28% |
| Cutoff 2 | 0.224 | nd | 0.232 | 0.259 | nd | 0.259 | 0.179 | nd | 0.179 |
| Sens 2 | 82% | nd | 82% | 82% | nd | 82% | 82% | nd | 80% |
| Spec 2 | 30% | nd | 31% | 32% | nd | 32% | 21% | nd | 21% |
| Cutoff 3 | 0.134 | nd | 0.188 | 0.188 | nd | 0.188 | 0.134 | nd | 0.134 |
| Sens 3 | 96% | nd | 93% | 91% | nd | 91% | 91% | nd | 90% |
| Spec 3 | 16% | nd | 23% | 23% | nd | 23% | 16% | nd | 17% |
| Cutoff 4 | 0.589 | nd | 0.587 | 0.589 | nd | 0.587 | 0.589 | nd | 0.587 |
| Sens 4 | 36% | nd | 36% | 36% | nd | 35% | 45% | nd | 45% |
| Spec 4 | 70% | nd | 70% | 70% | nd | 70% | 70% | nd | 70% |
| Cutoff 5 | 0.734 | nd | 0.734 | 0.734 | nd | 0.734 | 0.734 | nd | 0.734 |
| Sens 5 | 29% | nd | 29% | 27% | nd | 26% | 36% | nd | 35% |
| Spec 5 | 80% | nd | 80% | 80% | nd | 80% | 80% | nd | 80% |
| Cutoff 6 | 1.09 | nd | 1.07 | 1.09 | nd | 1.07 | 1.09 | nd | 1.07 |
| Sens 6 | 21% | nd | 21% | 18% | nd | 18% | 23% | nd | 20% |
| Spec 6 | 90% | nd | 90% | 90% | nd | 90% | 90% | nd | 90% |
| OR Quart 2 | 1.8 | nd | 2.8 | 1.5 | nd | 2.3 | 0.99 | nd | 1.2 |
| | 0.36 | nd | 0.13 | 0.43 | nd | 0.13 | 0.98 | nd | 0.76 |
| p Value | 0.51 | nd | 0.73 | 0.52 | nd | 0.78 | 0.31 | nd | 0.36 |
| 95% CI of OR Quart2 | 6.4 | nd | 11 | 4.5 | nd | 7.0 | 3.2 | nd | 4.1 |

(continued)

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| OR Quart | 2.4 | nd | 3.2 | 1.6 | nd | 1.9 | 0.16 | nd | 0.19 |
| 3 | 0.16 | nd | 0.088 | 0.42 | nd | 0.27 | 0.088 | nd | 0.13 |
| p Value | 0.71 | nd | 0.84 | 0.53 | nd | 0.61 | 0.018 | nd | 0.022 |
| 95% CI of OR Quart3 | 8.0 | nd | 12 | 4.5 | nd | 5.9 | 1.3 | nd | 1.7 |
| OR Quart | 2.1 | nd | 2.8 | 1.5 | nd | 1.9 | 1.5 | nd | 1.7 |
| 4 | 0.25 | nd | 0.13 | 0.43 | nd | 0.28 | 0.43 | nd | 0.39 |
| p Value | 0.60 | nd | 0.73 | 0.52 | nd | 0.60 | 0.52 | nd | 0.52 |
| 95% CI of OR Quart4 | 7.1 | nd | 11 | 4.5 | nd | 5.8 | 4.5 | nd | 5.3 |

**Fibroblast growth factor 19**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 0.163 | 0.226 | 0.163 | 0.172 | 0.163 | 0.106 |
| Average | 0.200 | 0.261 | 0.200 | 0.221 | 0.200 | 0.130 |
| Stdev | 0.207 | 0.213 | 0.207 | 0.211 | 0.207 | 0.105 |
| p(t-test) |  | 0.14 |  | 0.59 |  | 0.12 |
| Min | 2.92E-5 | 0.0472 | 2.92E-5 | 2.92E-5 | 2.92E-5 | 0.000127 |
| Max | 2.09 | 1.08 | 2.09 | 0.971 | 2.09 | 0.427 |
| n (Samp) | 356 | 28 | 356 | 34 | 356 | 22 |
| n (Patient) | 175 | 28 | 175 | 34 | 175 | 22 |

| UO only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 0.168 | 0.226 | 0.168 | 0.172 | 0.168 | 0.0967 |
| Average | 0.202 | 0.260 | 0.202 | 0.226 | 0.202 | 0.122 |
| Stdev | 0.207 | 0.213 | 0.207 | 0.210 | 0.207 | 0.105 |
| p(t-test) |  | 0.16 |  | 0.53 |  | 0.086 |
| Min | 2.92E-5 | 0.0472 | 2.92E-5 | 2.92E-5 | 2.92E-5 | 0.000127 |

(continued)

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Max | 2.09 | 1.08 | 2.09 | 0.971 | 2.09 | 0.427 |
| n (Samp) | 347 | 28 | 347 | 35 | 347 | 20 |
| n (Patient) | 161 | 28 | 161 | 35 | 161 | 20 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or | sCr only | UO only | sCr or UO | sCr only | UO only |
| AUC | 0.61 | nd | 0.61 | 0.53 | nd | 0.53 | 0.40 | nd | 0.37 |
| SE | 0.058 | nd | 0.058 | 0.053 | nd | 0.052 | 0.066 | nd | 0.068 |
| p | 0.050 | nd | 0.067 | 0.63 | nd | 0.55 | 0.11 | nd | 0.049 |
| nCohort 1 | 356 | nd | 347 | 356 | nd | 347 | 356 | nd | 347 |
| nCohort 2 | 28 | nd | 28 | 34 | nd | 35 | 22 | nd | 20 |
| Cutoff 1 | 0.128 | nd | 0.128 | 0.0839 | nd | 0.0820 | 0.0584 | nd | 0.0584 |
| Sens 1 | 71% | nd | 71% | 71% | nd | 71% | 73% | nd | 70% |
| Spec 1 | 43% | nd | 41% | 29% | nd | 28% | 22% | nd | 21% |
| Cutoff 2 | 0.0791 | nd | 0.0791 | 0.0536 | nd | 0.0564 | 0.0521 | nd | 0.0521 |
| Sens 2 | 82% | nd | 82% | 82% | nd | 80% | 82% | nd | 80% |
| Spec 2 | 27% | nd | 27% | 20% | nd | 20% | 20% | nd | 19% |
| Cutoff 3 | 0.0511 | nd | 0.0511 | 0.0120 | nd | 0.0120 | 0.0240 | nd | 0.0240 |
| Sens 3 | 93% | nd | 93% | 91% | nd | 91% | 91% | nd | 90% |
| Spec 3 | 20% | nd | 19% | 10% | nd | 9% | 12% | nd | 12% |
| Cutoff 4 | 0.243 | nd | 0.248 | 0.243 | nd | 0.248 | 0.243 | nd | 0.248 |
| Sens 4 | 39% | nd | 39% | 32% | nd | 34% | 18% | nd | 10% |
| Spec 4 | 70% | nd | 70% | 70% | nd | 70% | 70% | nd | 70% |
| Cutoff 5 | 0.300 | nd | 0.302 | 0.300 | nd | 0.302 | 0.300 | nd | 0.302 |
| Sens 5 | 39% | nd | 36% | 26% | nd | 29% | 5% | nd | 5% |
| Spec 5 | 80% | nd | 80% | 80% | nd | 80% | 80% | nd | 80% |
| Cutoff 6 | 0.390 | nd | 0.390 | 0.390 | nd | 0.390 | 0.390 | nd | 0.390 |
| Sens 6 | 18% | nd | 18% | 18% | nd | 20% | 5% | nd | 5% |
| Spec 6 | 90% | nd | 90% | 90% | nd | 90% | 90% | nd | 90% |
| | sCr or UO | sCr only | UO only | sCr or UO | sCr only | UO only | sCr or UO | sCr only | UO only |
| OR Quart | 1.5 | nd | 1.5 | 0.75 | nd | 0.75 | 2.6 | nd | 1.5 |
| 2 | 0.52 | nd | 0.53 | 0.59 | nd | 0.59 | 0.26 | nd | 0.65 |

(continued)

| | sCr or UO | sCr only | UO only | sCr or UO | sCr only | UO only | sCr or UO | sCr only | UO only |
|---|---|---|---|---|---|---|---|---|---|
| p Value | 0.42 | nd | 0.41 | 0.27 | nd | 0.27 | 0.49 | nd | 0.25 |
| 95% CI of OR Quart2 | 5.6 | nd | 5.6 | 2.1 | nd | 2.1 | 14 | nd | 9.3 |
| OR Quart | 1.8 | nd | 2.1 | 0.88 | nd | 0.88 | 3.7 | nd | 3.7 |
| 3 | 0.36 | nd | 0.25 | 0.80 | nd | 0.80 | 0.11 | nd | 0.11 |
| p Value | 0.51 | nd | 0.60 | 0.32 | nd | 0.32 | 0.75 | nd | 0.75 |
| 95% CI of OR Quart3 | 6.4 | nd | 7.1 | 2.4 | nd | 2.4 | 18 | nd | 18 |
| OR Quart | 3.0 | nd | 2.6 | 1.1 | nd | 1.2 | 4.3 | nd | 4.3 |
| 4 | 0.070 | nd | 0.11 | 0.83 | nd | 0.65 | 0.069 | nd | 0.068 |
| p Value | 0.91 | nd | 0.80 | 0.43 | nd | 0.49 | 0.89 | nd | 0.90 |
| 95% CI of OR Quart4 | 9.7 | nd | 8.8 | 2.9 | nd | 3.1 | 21 | nd | 21 |

**Fibroblast growth factor 21**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.0459 | 0.0866 | 0.0459 | 0.0750 | 0.0459 | 0.0792 |
| Average | 0.210 | 0.234 | 0.210 | 0.279 | 0.210 | 0.401 |
| Stdev | 0.518 | 0.444 | 0.518 | 0.969 | 0.518 | 0.598 |
| p(t-test) | | 0.82 | | 0.50 | | 0.097 |
| Min | 4.60E-6 | 0.00381 | 4.60E-6 | 9.65E-6 | 4.60E-6 | 0.00226 |
| Max | 4.46 | 2.26 | 4.46 | 5.72 | 4.46 | 2.11 |
| n (Samp) | 356 | 28 | 356 | 34 | 356 | 22 |
| n (Patient) | 175 | 28 | 175 | 34 | 175 | 22 |

| UO only | 0hr prior to AKI stage | | 24hr prior to AKI stage 48hr prior to | | | AKI stage |
|---|---|---|---|---|---|---|
| | Cohort 1 | Cohort 2 | Cohort 1 | Cohort 2 | Cohort 1 | Cohort 2 |
| Median | 0.0524 | 0.0866 | 0.0524 | 0.0712 | 0.0524 | 0.0664 |
| Average | 0.224 | 0.235 | 0.224 | 0.272 | 0.224 | 0.426 |
| Stdev | 0.529 | 0.444 | 0.529 | 0.956 | 0.529 | 0.623 |
| p(t-test) | | 0.91 | | 0.64 | | 0.10 |

(continued)

| UO only | 0hr prior to AKI stage | | 24hr prior to AKI stage 48hr prior to | | | AKI stage |
|---|---|---|---|---|---|---|
| | Cohort 1 | Cohort 2 | Cohort 1 | Cohort 2 | Cohort 1 | Cohort 2 |
| Min | 4.60E-6 | 0.00381 | 4.60E-6 | 9.65E-6 | 4.60E-6 | 0.00226 |
| Max | 4.46 | 2.26 | 4.46 | 5.72 | 4.46 | 2.11 |
| n (Samp) | 347 | 28 | 347 | 35 | 347 | 20 |
| n (Patient) | 161 | 28 | 161 | 35 | 161 | 20 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 3.58 | nd | 0.56 | 0.57 | nd | 0.54 | 0.61 | nd | 0.58 |
| SE | 0.058 | nd | 0.058 | 0.053 | nd | 0.052 | 0.066 | nd | 0.068 |
| p | 0.18 | nd | 0.30 | 0.22 | nd | 0.44 | 0.10 | nd | 0.25 |
| nCohort 1 | 356 | nd | 347 | 356 | nd | 347 | 356 | nd | 347 |
| nCohort 2 | 28 | nd | 28 | 34 | nd | 35 | 22 | nd | 20 |
| Cutoff 1 | 0.0322 | nd | 0.0322 | 0.0325 | nd | 0.0325 | 0.0301 | nd | 0.0301 |
| Sens 1 | 71% | nd | 71% | 71% | nd | 71% | 73% | nd | 70% |
| Spec 1 | 40% | nd | 37% | 41% | nd | 38% | 38% | nd | 36% |
| Cutoff 2 | 0.0126 | nd | 0.0126 | 0.0209 | nd | 0.0252 | 0.0202 | nd | 0.0202 |
| Sens 2 | 82% | nd | 82% | 82% | nd | 80% | 82% | nd | 80% |
| Spec 2 | 24% | nd | 21% | 32% | nd | 33% | 31% | nd | 29% |
| Cutoff 3 | 0.00659 | nd | 0.00654 | 0.0141 | nd | 0.0141 | 0.00901 | nd | 0.00901 |
| Sens 3 | 93% | nd | 93% | 91% | nd | 91% | 91% | nd | 90% |
| Spec 3 | 16% | nd | 13% | 25% | nd | 22% | 20% | nd | 17% |
| Cutoff 4 | 0.122 | nd | 0.133 | 0.122 | nd | 0.133 | 0.122 | nd | 0.133 |
| Sens 4 | 36% | nd | 36% | 32% | nd | 29% | 45% | nd | 45% |
| Spec 4 | 70% | nd | 70% | 70% | nd | 70% | 70% | nd | 70% |
| Cutoff 5 | 0.214 | nd | 0.224 | 0.214 | nd | 0.224 | 0.214 | nd | 0.224 |
| Sens 5 | 21% | nd | 25% | 18% | nd | 17% | 41% | nd | 35% |
| Spec 5 | 80% | nd | 80% | 80% | nd | 80% | 80% | nd | 80% |
| Cutoff 6 | 0.474 | nd | 0.495 | 0.474 | nd | 0.495 | 0.474 | nd | 0.495 |
| Sens 6 | 18% | nd | 18% | 3% | nd | 3% | 27% | nd | 30% |
| Spec 6 | 90% | nd | 90% | 90% | nd | 90% | 90% | nd | 90% |
| OR Quart | 0.41 | nd | 0.41 | 2.4 | nd | 1.5 | 0.99 | nd | 0.99 |
| 2 | 0.21 | nd | 0.20 | 0.17 | nd | 0.43 | 0.99 | nd | 0.99 |
| p Value | 0.10 | nd | 0.10 | 0.70 | nd | 0.52 | 0.24 | nd | 0.24 |

(continued)

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| 95% CI of OR Quart2 | 1.6 | nd | 1.6 | 7.9 | nd | 4.5 | 4.1 | nd | 4.1 |
| OR Quart | 1.3 | nd | 1.5 | 3.3 | nd | 1.9 | 1.3 | nd | 0.99 |
| 3 | 0.60 | nd | 0.46 | 0.046 | nd | 0.21 | 0.73 | nd | 0.99 |
| p Value | 0.47 | nd | 0.53 | 1.0 | nd | 0.69 | 0.33 | nd | 0.24 |
| 95% CI ot OR Quart3 | 3.7 | nd | 4.0 | 11 | nd | 5.5 | 4.9 | nd | 4.1 |
| OR Quart | 1.3 | nd | 1.1 | 2.4 | nd | 1.5 | 2.4 | nd | 2.1 |
| 4 | 0.60 | nd | 0.80 | 0.17 | nd | 0.43 | 0.17 | nd | 0.25 |
| p Value | 0.47 | nd | 0.40 | 0.70 | nd | 0.52 | 0.70 | nd | 0.60 |
| 95% CI ot OR Quart4 | 3.7 | nd | 3.3 | 7.9 | nd | 4.5 | 7.9 | nd | 7.1 |

**Tenascin**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 562 | 799 | 562 | 664 | 562 | 949 |
| Average | 1170 | 1140 | 1170 | 982 | 1170 | 1330 |
| Stdev | 2460 | 1190 | 2460 | 741 | 2460 | 1050 |
| p(t-test) |  | 0.94 |  | 0.67 |  | 0.76 |
| Min | 43.9 | 146 | 43.9 | 167 | 43.9 | 200 |
| Max | 26100 | 5700 | 26100 | 3000 | 26100 | 4290 |
| n (Samp) | 357 | 28 | 357 | 32 | 357 | 22 |
| n (Patient) | 175 | 28 | 175 | 32 | 175 | 22 |
| UO only | 0hr prior to AKI stage | | 24hr prior to AKI stage | | 48hr prior to AKI stage | |
|  | Cohort 1 | Cohort 2 | Cohort 1 | Cohort 2 | Cohort 1 | Cohort 2 |
| Median | 564 | 799 | 564 | 728 | 564 | 949 |
| Average | 1190 | 1130 | 1190 | 978 | 1190 | 1370 |
| Stdev | 2490 | 1190 | 2490 | 729 | 2490 | 1090 |
| p(t-test) |  | 0.91 |  | 0.63 |  | 0.74 |
| Min | 43.9 | 146 | 43.9 | 167 | 43.9 | 200 |
| Max | 26100 | 5700 | 26100 | 3000 | 26100 | 4290 |

(continued)

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| n (Samp) | 348 | 28 | 348 | 33 | 348 | 20 |
| n (Patient) | 161 | 28 | 161 | 33 | 161 | 20 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.60 | nd | 0.59 | 0.58 | nd | 0.58 | 0.71 | nd | 0.70 |
| SE | 0.058 | nd | 0.058 | 0.055 | nd | 0.054 | 0.063 | nd | 0.067 |
| p | 0.10 | nd | 0.13 | 0.14 | nd | 0.14 | 9.3E-4 | nd | 0.0023 |
| nCohort 1 | 357 | nd | 348 | 357 | nd | 348 | 357 | nd | 348 |
| nCohort 2 | 28 | nd | 28 | 32 | nd | 33 | 22 | nd | 20 |
| Cutoff 1 | 482 | nd | 482 | 426 | nd | 426 | 817 | nd | 817 |
| Sens 1 | 71% | nd | 71% | 72% | nd | 73% | 73% | nd | 70% |
| Spec 1 | 45% | nd | 44% | 38% | nd | 38% | 68% | nd | 67% |
| Cutoff 2 | 389 | nd | 389 | 358 | nd | 358 | 704 | nd | 704 |
| Sens 2 | 82% | nd | 82% | 81% | nd | 82% | 82% | nd | 80% |
| Spec 2 | 33% | nd | 33% | 29% | nd | 29% | 63% | nd | 62% |
| Cutoff 3 | 223 | nd | 223 | 310 | nd | 310 | 486 | nd | 486 |
| Sens 3 | 93% | nd | 93% | 91% | nd | 91% | 91% | nd | 90% |
| Spec 3 | 14% | nd | 13% | 24% | nd | 24% | 45% | nd | 44% |
| Cutoff 4 | 875 | nd | 895 | 875 | nd | 895 | 875 | nd | 895 |
| Sens 4 | 43% | nd | 43% | 44% | nd | 42% | 64% | nd | 65% |
| Spec 4 | 70% | nd | 70% | 70% | nd | 70% | 70% | nd | 70% |
| Cutoff 5 | 1160 | nd | 1170 | 1160 | nd | 1170 | 1160 | nd | 1170 |
| Sens 5 | 32% | nd | 29% | 34% | nd | 30% | 32% | nd | 35% |
| Spec 5 | 80% | nd | 80% | 80% | nd | 80% | 80% | nd | 80% |
| Cutoff 6 | 1950 | nd | 1950 | 1950 | nd | 1950 | 1950 | nd | 1950 |
| Sens 6 | 18% | nd | 18% | 12% | nd | 12% | 18% | nd | 20% |
| Spec 6 | 90% | nd | 90% | 90% | nd | 90% | 90% | nd | 90% |
| OR Quart 2 | 1.0 | nd | 1.0 | 2.7 | nd | 1.9 | 3.0 | nd | 3.1 |
| | 1.0 | nd | 1.0 | 0.11 | nd | 0.27 | 0.34 | nd | 0.34 |
| p Value | 0.28 | nd | 0.28 | 0.81 | nd | 0.61 | 0.31 | nd | 0.31 |
| 95% CI of OR Quart2 | 3.6 | nd | 3.6 | 8.8 | nd | 5.8 | 30 | nd | 30 |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| OR Quart | 1.7 | nd | 1.7 | 1.0 | nd | 1.0 | 8.6 | nd | 7.5 |
| 3 | 0.39 | nd | 0.39 | 1.0 | nd | 1.0 | 0.045 | nd | 0.062 |
| p Value | 0.52 | nd | 0.52 | 0.24 | nd | 0.28 | 1.0 | nd | 0.90 |
| 95% CI of OR Quart3 | 5.3 | nd | 5.3 | 4.1 | nd | 3.6 | 70 | nd | 62 |
| OR Quart | 2.1 | nd | 2.1 | 3.9 | nd | 3.1 | 11 | nd | 9.9 |
| 4 | 0.19 | nd | 0.19 | 0.021 | nd | 0.039 | 0.024 | nd | 0.032 |
| p Value | 0.69 | nd | 0.70 | 1.2 | nd | 1.1 | 1.4 | nd | 1.2 |
| 95% CI of OR Quart4 | 6.4 | nd | 6.5 | 12 | nd | 8.9 | 87 | nd | 80 |

Fig. 7: Comparison of marker levels in EDTA samples collected within 12 hours of reaching stage R from Cohort 1 (patients that reached, but did not progress beyond, RIFLE stage R) and from Cohort 2 (patients that reached RIFLE stage I or F).

**Angiopoietin-related protein 4**

| | sCr or UO | | sCr only | | UO only | |
|---|---|---|---|---|---|---|
| | Cohort 1 | Cohort 2 | Cohort 1 | Cohort 2 | Cohort 1 | Cohort 2 |
| Median | 37.7 | 31.0 | 52.4 | 47.4 | 35.1 | 30.5 |
| Average | 46.3 | 50.4 | 68.0 | 64.4 | 38.8 | 49.2 |
| Stdev | 43.5 | 44.4 | 65.2 | 59.7 | 22.9 | 41.0 |
| p(t-test) | | 0.67 | | 0.90 | | 0.16 |
| Min | 2.71 | 8.79 | 8.18 | 20.5 | 2.71 | 8.79 |
| Max | 317 | 192 | 317 | 179 | 101 | 192 |
| n (Samp) | 65 | 31 | 26 | 6 | 50 | 26 |
| n (Patient) | 65 | 31 | 26 | 6 | 50 | 26 |

| AUC | 0.50 | 0.45 | 0.53 |
|---|---|---|---|
| SE | 0.063 | 0.13 | 0.071 |
| p | 0.98 | 0.70 | 0.65 |
| nCohort 1 | 65 | 26 | 50 |
| nCohort 2 | 31 | 6 | 26 |
| Cutoff 1 | 24.5 | 22.3 | 25.1 |

(continued)

| | | | |
|---|---|---|---|
| Sens 1 | 71% | 83% | 73% |
| Spec 1 | 28% | 15% | 32% |
| Cutoff 2 | 22.1 | 22.3 | 22.1 |
| Sens 2 | 81% | 83% | 81% |
| Spec 2 | 23% | 15% | 26% |
| Cutoff 3 | 15.1 | 15.1 | 14.5 |
| Sens 3 | 90% | 100% | 92% |
| Spec 3 | 14% | 12% | 12% |
| Cutoff 4 | 45.5 | 72.2 | 43.0 |
| Sens 4 | 42% | 17% | 42% |
| Spec 4 | 71% | 73% | 70% |
| Cutoff 5 | 63.2 | 78.2 | 51.6 |
| Sens 5 | 19% | 17% | 38% |
| Spec 5 | 80% | 81% | 80% |
| Cutoff 6 | 82.3 | 154 | 75.6 |
| Sens 6 | 13% | 17% | 19% |
| Spec 6 | 91% | 92% | 90% |
| OR Quart 2 | 1.0 | 2.3 | 2.0 |
| p Value | 1.0 | 0.53 | 0.32 |
| 95% CI of | 0.29 | 0.17 | 0.52 |
| OR Quart2 | 3.5 | 33 | 7.3 |
| OR Quart 3 | 1.2 | 1.0 | 0.25 |
| p Value | 0.76 | 1.0 | 0.13 |
| 95% CI of | 0.36 | 0.052 | 0.044 |
| OR Quart3 | 4.1 | 19 | 1.5 |
| OR Quart 4 | 1.5 | 2.3 | 2.0 |
| p Value | 0.54 | 0.53 | 0.32 |
| 95% CI of | 0.44 | 0.17 | 0.52 |
| OR Quart4 | 4.9 | 33 | 7.3 |

**Amphiregulin**

| | sCr or UO | | sCr only | | UO only | |
|---|---|---|---|---|---|---|
| | Cohort 1 | Cohort 2 | Cohort 1 | Cohort 2 | Cohort 1 | Cohort 2 |
| Median | 22.0 | 22.0 | 24.1 | 22.4 | 20.9 | 21.1 |
| Average | 31.8 | 30.9 | 39.2 | 46.8 | 27.0 | 26.9 |
| Stdev | 34.4 | 30.7 | 44.4 | 57.8 | 24.3 | 18.6 |
| p(t-test) | | 0.90 | | 0.72 | | 0.98 |

(continued)

|  | sCr or UO | | sCr only | | UO only | |
|---|---|---|---|---|---|---|
|  | Cohort 1 | Cohort 2 | Cohort 1 | Cohort 2 | Cohort 1 | Cohort 2 |
| Min | 2.70 | 0.00246 | 4.60 | 13.6 | 2.70 | 0.00246 |
| Max | 198 | 162 | 198 | 162 | 145 | 72.6 |
| n (Samp) | 63 | 30 | 25 | 6 | 49 | 25 |
| n (Patient) | 63 | 30 | 25 | 6 | 49 | 25 |

|  | At Enrollment | | |
|---|---|---|---|
|  | sCr or UO | sCr only | UO only |
| AUC | 0.51 | 0.51 | 0.53 |
| SE | 0.064 | 0.13 | 0.072 |
| p | 0.91 | 0.92 | 0.66 |
| nCohort 1 | 63 | 25 | 49 |
| nCohort 2 | 30 | 6 | 25 |
| Cutoff 1 | 16.1 | 13.9 | 17.4 |
| Sens 1 | 70% | 83% | 72% |
| Spec 1 | 32% | 24% | 41% |
| Cutoff 2 | 13.5 | 13.9 | 13.9 |
| Sens 2 | 80% | 83% | 80% |
| Spec 2 | 27% | 24% | 35% |
| Cutoff 3 | 9.97 | 11.9 | 8.92 |
| Sens 3 | 90% | 100% | 92% |
| Spec 3 | 16% | 20% | 12% |
| Cutoff 4 | 30.4 | 40.6 | 27.6 |
| Sens 4 | 30% | 33% | 28% |
| Spec 4 | 71% | 72% | 71% |
| Cutoff 5 | 40.6 | 51.0 | 36.0 |
| Sens 5 | 20% | 17% | 20% |
| Spec 5 | 81% | 80% | 82% |
| Cutoff 6 | 60.5 | 72.6 | 60.5 |
| Sens 6 | 10% | 17% | 8% |
| Spec 6 | 90% | 92% | 92% |
| OR Quart 2 | 1.8 | 2.0 | 1.5 |
| p Value | 0.35 | 0.61 | 0.56 |
| 95% CI of | 0.52 | 0.14 | 0.38 |
| OR Quart2 | 6.4 | 28 | 6.1 |
| OR Quart 3 | 1.0 | 0.86 | 1.3 |
| p Value | 1.0 | 0.92 | 0.72 |

(continued)

| | At Enrollment | | |
|---|---|---|---|
| | sCr or UO | sCr only | UO only |
| 95% CI of | 0.27 | 0.044 | 0.31 |
| OR Quart3 | 3.7 | 17 | 5.4 |
| OR Quart 4 | 1.7 | 2.0 | 1.5 |
| p Value | 0.40 | 0.61 | 0.56 |
| 95% CI of | 0.49 | 0.14 | 0.38 |
| OR Quart4 | 5.9 | 28 | 6.1 |

**Endostatin**

| | sCr or UO sCr only | | | | UO only | |
|---|---|---|---|---|---|---|
| | Cohort 1 | Cohort 2 | Cohort 1 | Cohort 2 | Cohort 1 | Cohort 2 |
| Median | 44200 | 37400 | 58300 | 34700 | 41500 | 34100 |
| Average | 59000 | 57100 | 77700 | 41500 | 48000 | 62200 |
| Stdev | 62600 | 61300 | 88700 | 32600 | 30200 | 69700 |
| p(t-test) | | 0.89 | | 0.34 | | 0.22 |
| | sCr or UO | | sCr only | | UO only | |
| | Cohort 1 | Cohort 2 | Cohort 1 | Cohort 2 | Cohort 1 | Cohort 2 |
| Min | 6940 | 9280 | 13600 | 13800 | 6940 | 9280 |
| Max | 483000 | 275000 | 483000 | 102000 | 146000 | 275000 |
| n (Samp) | 65 | 31 | 26 | 6 | 50 | 26 |
| n (Patient) | 65 | 31 | 26 | 6 | 50 | 26 |

| | At Enrollment | | |
|---|---|---|---|
| | sCr or UO | sCr only | UO only |
| AUC | 0.46 | 0.28 | 0.48 |
| SE | 0.064 | 0.13 | 0.070 |
| p | 0.49 | 0.077 | 0.80 |
| nCohort 1 | 65 | 26 | 50 |
| nCohort 2 | 31 | 6 | 26 |
| Cutoff 1 | 29900 | 13800 | 26300 |
| Sens 1 | 71% | 83% | 73% |
| Spec 1 | 28% | 4% | 24% |
| Cutoff 2 | 26000 | 13800 | 23600 |
| Sens 2 | 81% | 83% | 81% |
| Spec 2 | 20% | 4% | 22% |

(continued)

| | At Enrollment | | |
| --- | --- | --- | --- |
| | sCr or UO | sCr only | UO only |
| Cutoff 3 | 17000 | 13600 | 17000 |
| Sens 3 | 90% | 100% | 92% |
| Spec 3 | 11% | 4% | 12% |
| Cutoff 4 | 61800 | 73100 | 53400 |
| Sens 4 | 29% | 17% | 31% |
| Spec 4 | 71% | 73% | 70% |
| Cutoff 5 | 69800 | 93800 | 66900 |
| Sens 5 | 23% | 17% | 27% |
| Spec 5 | 80% | 81% | 80% |
| Cutoff 6 | 115000 | 119000 | 80400 |
| Sens 6 | 6% | 0% | 19% |
| Spec 6 | 91% | 92% | 90% |
| OR Quart 2 | 0.53 | 0 | 0.61 |
| p Value | 0.33 | na | 0.49 |
| 95% CI of | 0.14 | na | 0.15 |
| OR Quart2 | 1.9 | na | 2.4 |
| OR Quart 3 | 1.4 | 4.2 | 1.0 |
| p Value | 0.55 | 0.27 | 1.0 |
| 95% CI of | 0.44 | 0.33 | 0.27 |
| OR Quart3 | 4.6 | 53 | 3.7 |
| OR Quart 4 | 1.0 | 2.3 | 1.0 |
| p Value | 1.0 | 0.53 | 1.0 |
| 95% CI of | 0.30 | 0.17 | 0.27 |
| OR Quart4 | 3.3 | 33 | 3.7 |

**Fibroblast growth factor 19**

| | sCr or UO | | sCr only | | UO only | |
| --- | --- | --- | --- | --- | --- | --- |
| | Cohort 1 | Cohort 2 | Cohort 1 | Cohort 2 | Cohort 1 | Cohort 2 |
| Median | 0.164 | 0.149 | 0.223 | 0.104 | 0.140 | 0.150 |
| Average | 0.209 | 0.236 | 0.295 | 0.149 | 0.182 | 0.240 |
| Stdev | 0.210 | 0.287 | 0.279 | 0.181 | 0.205 | 0.297 |
| p(t-test) | | 0.60 | | 0.23 | | 0.32 |
| Min | 0.000127 | 8.23E-5 | 0.000127 | 8.23E-5 | 0.000127 | 0.00974 |
| Max | 1.41 | 1.32 | 1.32 | 0.482 | 1.41 | 1.32 |
| n (Samp) | 65 | 31 | 26 | 6 | 50 | 26 |

(continued)

| | sCr or UO | | sCr only | | UO only | |
|---|---|---|---|---|---|---|
| | Cohort 1 | Cohort 2 | Cohort 1 | Cohort 2 | Cohort 1 | Cohort 2 |
| n (Patient) | 65 | 31 | 26 | 6 | 50 | 26 |

| | At Enrollment | | |
|---|---|---|---|
| | sCr or UO | sCr only | UO only |
| AUC | 0.48 | 0.28 | 0.54 |
| SE | 0.064 | 0.13 | 0.071 |
| p | 0.71 | 0.082 | 0.61 |
| nCohort 1 | 65 | 26 | 50 |
| nCohort 2 | 31 | 6 | 26 |
| Cutoff 1 | 0.0804 | 8.23E-5 | 0.0816 |
| Sens 1 | 71% | 83% | 73% |
| Spec 1 | 23% | 0% | 28% |
| Cutoff 2 | 0.0659 | 8.23E-5 | 0.0676 |
| Sens 2 | 81% | 83% | 81% |
| Spec 2 | 22% | 0% | 24% |
| Cutoff 3 | 0.0209 | 0 | 0.0616 |
| Sens 3 | 90% | 100% | 92% |
| Spec 3 | 11% | 0% | 22% |
| Cutoff 4 | 0.265 | 0.326 | 0.215 |
| Sens 4 | 32% | 17% | 31% |
| Spec 4 | 71% | 73% | 70% |
| Cutoff 5 | 0.303 | 0.357 | 0.265 |
| Sens 5 | 23% | 17% | 31% |
| Spec 5 | 80% | 81% | 80% |
| Cutoff 6 | 0.346 | 0.543 | 0.304 |
| Sens 6 | 16% | 0% | 19% |
| Spec 6 | 91% | 92% | 90% |
| OR Quart 2 | 0.67 | 0 | 0.61 |
| p Value | 0.53 | na | 0.49 |
| 95% CI of | 0.19 | na | 0.15 |
| OR Quart2 | 2.3 | na | 2.4 |
| OR Quart 3 | 1.0 | 2.3 | 0.79 |
| p Value | 1.0 | 0.53 | 0.73 |
| 95% CI of | 0.30 | 0.17 | 0.21 |
| OR Quart3 | 3.3 | 33 | 3.0 |

(continued)

| At | At Enrollment | | |
|---|---|---|---|
| | sCr or UO | sCr only | UO only |
| OR Quart 4 | 1.2 | 4.2 | 1.2 |
| p Value | 0.76 | 0.27 | 0.74 |
| 95% CI of | 0.37 | 0.33 | 0.34 |
| OR Quart4 | 3.9 | 53 | 4.6 |

**Fibroblast growth factor 21**

| | sCr or UO | | sCr only | | UO only | |
|---|---|---|---|---|---|---|
| | Cohort 1 | Cohort 2 | Cohort 1 | Cohort 2 | Cohort 1 | Cohort 2 |
| Median | 0.0451 | 0.0670 | 0.0773 | 0.288 | 0.0610 | 0.0618 |
| Average | 0.212 | 0.402 | 0.257 | 0.634 | 0.191 | 0.340 |
| Stdev | 0.512 | 1.08 | 0.489 | 0.803 | 0.485 | 1.11 |
| p(t-test) | | 0.24 | | 0.14 | | 0.42 |
| Min | 7.01E-5 | 0.00323 | 0.00263 | 0.0306 | 7.01E-5 | 0.00323 |
| Max | 3.36 | 5.72 | 2.05 | 2.11 | 3.36 | 5.72 |
| n (Samp) | 65 | 31 | 26 | 6 | 50 | 26 |
| n (Patient) | 65 | 31 | 26 | 6 | 50 | 26 |

| | At Enrollment | | |
|---|---|---|---|
| | sCr or UO | sCr only | UO only |
| AUC | 0.58 | 0.71 | 0.52 |
| SE | 0.064 | 0.13 | 0.071 |
| p | 0.21 | 0.099 | 0.73 |
| nCohort 1 | 65 | 26 | 50 |
| nCohort 2 | 31 | 6 | 26 |
| Cutoff 1 | 0.0322 | 0.0938 | 0.0231 |
| Sens 1 | 71% | 83% | 73% |
| Spec 1 | 43% | 58% | 38% |
| Cutoff 2 | 0.0231 | 0.0938 | 0.0189 |
| Sens 2 | 81% | 83% | 81% |
| Spec 2 | 40% | 58% | 32% |
| Cutoff 3 | 0.0126 | 0.0217 | 0.00762 |
| Sens 3 | 90% | 100% | 92% |
| Spec 3 | 22% | 27% | 20% |
| Cutoff 4 | 0.129 | 0.181 | 0.170 |

(continued)

|  | At Enrollment | | |
|---|---|---|---|
|  | sCr or UO | sCr only | UO only |
| Sens 4 | 35% | 50% | 31% |
| Spec 4 | 71% | 73% | 70% |
| Cutoff 5 | 0.253 | 0.206 | 0.252 |
| Sens 5 | 19% | 50% | 12% |
| Spec 5 | 80% | 81% | 80% |
| Cutoff 6 | 0.430 | 1.05 | 0.312 |
| Sens 6 | 13% | 17% | 12% |
| Spec 6 | 91% | 92% | 90% |
| OR Quart 2 | 3.0 | 0 | 3.4 |
| p Value | 0.11 | na | 0.094 |
| 95% CI of | 0.77 | na | 0.81 |
| OR Quart2 | 12 | na | 14 |
| OR Quart 3 | 3.6 | 2.3 | 2.2 |
| p Value | 0.064 | 0.53 | 0.29 |
| 95% CI of | 0.93 | 0.17 | 0.52 |
| OR Quart3 | 14 | 33 | 9.3 |
| OR Quart 4 | 2.5 | 4.2 | 1.7 |
| p Value | 0.19 | 0.27 | 0.46 |
| 95% CI of | 0.64 | 0.33 | 0.40 |
| OR Quart4 | 9.8 | 53 | 7.5 |

**Thrombospondin-2**

|  | sCr or UO | | sCr only | | UO only | |
|---|---|---|---|---|---|---|
|  | Cohort 1 | Cohort 2 | Cohort 1 | Cohort 2 | Cohort 1 | Cohort 2 |
| Median | 41900 | 21700 | 59900 | 18300 | 40400 | 24200 |
| Average | 66700 | 67900 | 89800 | 87900 | 65600 | 52300 |
| Stdev | 75700 | 119000 | 102000 | 169000 | 81200 | 83000 |
| p(t-test) |  | 0.95 |  | 0.97 |  | 0.50 |
| Min | 1240 | 1620 | 1400 | 4470 | 1240 | 1620 |
| Max | 434000 | 433000 | 418000 | 433000 | 434000 | 351000 |
| n (Samp) | 66 | 31 | 26 | 6 | 51 | 26 |
| n (Patient) | 66 | 31 | 26 | 6 | 51 | 26 |

|  | At Enrollment | | |
| --- | --- | --- | --- |
|  | sCr or UO | sCr only | UO only |
| AUC | 0.39 | 0.34 | 0.41 |
| SE | 0.063 | 0.13 | 0.070 |
| p | 0.076 | 0.23 | 0.18 |
| nCohort 1 | 66 | 26 | 51 |
| nCohort 2 | 31 | 6 | 26 |
| Cutoff 1 | 15500 | 9090 | 16500 |
| Sens 1 | 71% | 83% | 73% |
| Spec 1 | 26% | 15% | 27% |
| Cutoff 2 | 8620 | 9090 | 13100 |
| Sens 2 | 81% | 83% | 81% |
| Spec 2 | 14% | 15% | 20% |
| Cutoff 3 | 4470 | 2860 | 3360 |
| Sens 3 | 90% | 100% | 92% |
| Spec 3 | 6% | 8% | 6% |
| Cutoff 4 | 67700 | 106000 | 55600 |
| Sens 4 | 16% | 17% | 15% |
| Spec 4 | 71% | 73% | 71% |
| Cutoff 5 | 112000 | 123000 | 112000 |
| Sens 5 | 13% | 17% | 12% |
| Spec 5 | 80% | 81% | 80% |
| Cutoff 6 | 152000 | 237000 | 160000 |
| Sens 6 | 13% | 17% | 12% |
| Spec 6 | 91% | 92% | 90% |
| OR Quart 2 | 1.1 | 0 | 1.4 |
| p Value | 0.94 | na | 0.64 |
| 95% CI of | 0.26 | na | 0.32 |
| OR Quart2 | 4.2 | na | 6.4 |
| OR Quart 3 | 4,7 | 2.3 | 5.5 |
| p Value | 0.016 | 0.53 | 0.019 |
| 95% CI of | 1.3 | 0.17 | 1.3 |
| OR Quart3 | 17 | 33 | 23 |
| OR Quart 4 | 2.0 | 4.2 | 1.8 |
| p Value | 0.29 | 0.27 | 0.41 |
| 95% CI of | 0.55 | 0.33 | 0.43 |
| OR Quart4 | 7.3 | 53 | 8.0 |

Fig. 8: Comparison of the maximum marker levels in EDTA samples collected from Cohort 1 (patients that did not progress beyond RIFLE stage 0) and the maximum values in EDTA samples collected from subjects between enrollment and 0, 24 hours, and 48 hours prior to reaching stage F in Cohort 2.

Angiogenin

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 179000 | 204000 | 179000 | 193000 | 179000 | 250000 |
| Average | 209000 | 206000 | 209000 | 197000 | 209000 | 251000 |
| Stdev | 234000 | 104000 | 234000 | 109000 | 234000 | 96600 |
| p(t-test) | | 0.97 | | 0.87 | | 0.64 |
| Min | 33100 | 48000 | 33100 | 48000 | 33100 | 88800 |
| Max | 2240000 | 345000 | 2240000 | 345000 | 2240000 | 345000 |
| n (Samp) | 88 | 11 | 88 | 11 | 88 | 7 |
| n (Patient) | 88 | 11 | 88 | 11 | 88 | 7 |

| UO only | 0hr prior to AKI stage | | 24hr prior to AKI stage | |
|---|---|---|---|---|
| | Cohort 1 | Cohort 2 | Cohort 1 | Cohort 2 |
| Median | 198000 | 141000 | 198000 | 140000 |
| Average | 221000 | 170000 | 221000 | 156000 |
| Stdev | 235000 | 104000 | 235000 | 106000 |
| p(t-test) | | 0.57 | | 0.47 |
| Min | 33100 | 48000 | 33100 | 48000 |
| Max | 2240000 | 345000 | 2240000 | 1345000 |
| n (Samp) | 89 | 7 | 89 | 7 |
| n (Patient) | 89 | 7 | 89 | 7 |

| | 0hr prior to AKI stage | | 24hr prior to AKI stage | | 48hr prior to AKI stage |
|---|---|---|---|---|---|
| | sCr or UO | UO only | sCr or UO | UO only | sCr or UO |
| AUC | 0.55 | 0.40 | 0.52 | 0.35 | 0.70 |
| SE | 0.095 | 0.12 | 0.093 | 0.12 | 0.11 |
| p | 0.61 | 0.40 | 0.87 | 0.21 | 0.083 |
| nCohort 1 | 88 | 89 | 88 | 89 | 88 |
| nCohort 2 | 11 | 7 | 11 | 7 | 7 |
| Cutoff 1 | 140000 | 109000 | 140000 | 87400 | 202000 |
| Sens 1 | 73% | 71% | 73% | 71% | 71% |
| Spec 1 | 35% | 17% | 35% | 9% | 60% |
| Cutoff 2 | 109000 | 87400 | 87400 | 76300 | 191000 |
| Sens 2 | 82% | 86% | 82% | 86% | 86% |
| Spec 2 | 17% | 9% | 9% | 6% | 56% |
| Cutoff 3 | 87400 | 33100 | 76300 | 33100 | 87400 |

(continued)

|  | 0hr prior to AKI stage | | 24hr prior to AKI stage | | 48hr prior to AKI stage |
|---|---|---|---|---|---|
|  | sCr or UO | UO only | sCr or UO | UO only | sCr or UO |
| Sens 3 | 91% | 100% | 91% | 100% | 100% |
| Spec 3 | 9% | 1% | 7% | 1% | 9% |
| Cutoff 4 | 225000 | 245000 | 225000 | 245000 | 225000 |
| Sens 4 | 36% | 29% | 36% | 29% | 57% |
| Spec 4 | 70% | 71% | 70% | 71% | 70% |
| Cutoff 5 | 259000 | 271000 | 259000 | 271000 | 259000 |
| Sens 5 | 27% | 14% | 27% | 14% | 43% |
| Spec 5 | 81% | 81% | 81% | 81% | 81% |
| Cutoff 6 | 285000 | 330000 | 285000 | 330000 | 285000 |
| Sens 6 | 27% | 14% | 27% | 14% | 43% |
| Spec 6 | 91% | 91% | 91% | 91% | 91% |
| OR Quart | 0.29 | 2.1 | 0.61 | 1.0 | 0 |
| 2 | 0.30 | 0.56 | 0.61 | 1.0 | na |
| p Value | 0.028 | 0.18 | 0.092 | 0.059 | na |
| 95% CI of OR Quart2 | 3.0 | 25 | 4.0 | 17 | na |
| OR Quart | 0.95 | 1.0 | 0.61 | 2.1 | 2.0 |
| 3 | 0.96 | 1.0 | 0.61 | 0.56 | 0.58 |
| p Value | 0.17 | 0.059 | 0.092 | 0.18 | 0.17 |
| 95% CI of OR Quart3 | 5.3 | 17 | 4.0 | 25 | 24 |
| OR Quart | 1.3 | 3.3 | 1.3 | 3.3 | 4.4 |
| 4 | 0.73 | 0.32 | 0.73 | 0.32 | 0.20 |
| p Value | 0.27 | 0.32 | 0.27 | 0.32 | 0.45 |
| 95% CI of OR Quart4 | 6.7 | 34 | 6.7 | 34 | 43 |

**Angiopoietin-related protein 4**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 23.2 | 94.8 | 23.2 | 94.8 | 23.2 | 61.9 |
| Average | 65.5 | 136 | 65.5 | 122 | 65.5 | 78.2 |
| Stdev | 212 | 101 | 212 | 89.2 | 212 | 48.4 |
| p(t-test) |  | 0.28 |  | 0.39 |  | 0.88 |
| Min | 1.77 | 34.0 | 1.77 | 34.0 | 1.77 | 34.0 |
| Max | 1900 | 339 | 1900 | 339 | 1900 | 179 |
| n (Samp) | 88 | 11 | 88 | 11 | 88 | 7 |

(continued)

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| n (Patient) | 88 | 11 | 88 | 11 | 88 | 7 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 31.8 | 179 | 31.8 | 133 | nd | nd |
| Average | 78.1 | 185 | 78.1 | 163 | nd | nd |
| Stdev | 213 | 95.1 | 213 | 88.7 | nd | nd |
| p(t-test) | | 0.19 | | 0.30 | nd | nd |
| Min | 2.68 | 71.1 | 2.68 | 71.1 | nd | nd |
| Max | 1900 | 339 | 1900 | 339 | nd | nd |
| n (Samp) | 89 | 7 | 89 | 7 | nd | nd |
| n (Patient) | 89 | 7 | 89 | 7 | nd | nd |

| | 0hr prior to AKI stage | | 24hr prior to AKI stage | | 48hr prior to AKI stage |
|---|---|---|---|---|---|
| | sCr or UO | UO only | sCr or UO | sCr or UO | UO only |
| AUC | 0.88 | 0.90 | 3.87 | 0.90 | 0.83 |
| SE | 0.069 | 0.078 | 3.070 | 0.080 | 0.098 |
| p | 6.1E-8 | 2.8E-7 | 1.3E-7 | 8.7E-7 | 7.5E-4 |
| nCohort 1 | 88 | 89 | 88 | 89 | 88 |
| nCohort 2 | 11 | 7 | 11 | 7 | 7 |
| Cutoff 1 | 55.0 | 127 | 55.0 | 127 | 51.6 |
| Sens 1 | 73% | 71% | 73% | 71% | 71% |
| Spec 1 | 82% | 91% | 82% | 91% | 81% |
| Cutoff 2 | 51.6 | 93.9 | 51.6 | 93.9 | 47.5 |
| Sens 2 | 82% | 86% | 82% | 86% | 86% |
| Spec 2 | 81% | 85% | 81% | 85% | 80% |
| Cutoff 3 | 47.5 | 69.3 | 47.5 | 69.3 | 33.7 |
| Sens 3 | 91% | 100% | 91% | 100% | 100% |
| Spec 3 | 80% | 76% | 80% | 76% | 66% |
| Cutoff 4 | 36.9 | 51.6 | 36.9 | 51.6 | 36.9 |
| Sens 4 | 91% | 100% | 91% | 100% | 86% |
| Spec 4 | 70% | 71% | 70% | 71% | 70% |
| Cutoff 5 | 51.6 | 83.6 | 51.6 | 83.6 | 51.6 |
| Sens 5 | 82% | 86% | 82% | 86% | 71% |

(continued)

|  | 0hr prior to AKI stage | | 24hr prior to AKI stage | | 48hr prior to AKI stage |
| --- | --- | --- | --- | --- | --- |
|  | sCr or UO | UO only | sCr or UO | sCr or UO | UO only |
| Spec 5 | 81% | 81% | 81% | 81% | 81% |
| Cutoff 6 | 93.9 | 127 | 93.9 | 127 | 93.9 |
| Sens 6 | 55% | 71% | 55% | 71% | 29% |
| Spec 6 | 91% | 91% | 91% | 91% | 91% |
| OR Quart | >0 | >0 | >0 | >0 | >0 |
| 2 | <na | <na | <na | <na | <na |
| p Value | >na | >na | >na | >na | >na |
| 95% CI of OR Quart2 | na | na | na | na | na |
| OR Quart | >3.3 | >1.0 | >3.3 | >1.0 | >1.0 |
| 3 | <0.32 | <0.98 | <0.32 | <0.98 | <1.0 |
| p Value | >0.32 | >0.062 | >0.32 | >0.062 | >0.059 |
| 95% CI of OR Quart3 | na | na | na | na | na |
| OR Quart | >11 | >8.0 | >11 | >8.0 | >7.7 |
| 4 | <0.029 | <0.064 | <0.029 | <0.064 | <0.070 |
| p Value | >1.3 | >0.88 | >1.3 | >0.88 | >0.85 |
| 95% CI of OR Quart4 | na | na | na | na | na |

**Angiopoietin-related protein 6**

| sCr or UO | 0hr prior to AKI stage | | 24hr prior to AKI stage | | 48hr prior to AKI stage | |
| --- | --- | --- | --- | --- | --- | --- |
|  | Cohort 1 | Cohort 2 | Cohort 1 | Cohort 2 | Cohort 1 | Cohort 2 |
| Median | 65.2 | 95.0 | 65.2 | 85.1 | 65.2 | 96.4 |
| Average | 76.4 | 104 | 76.4 | 98.6 | 76.4 | 122 |
| Stdev | 50.5 | 69.2 | 50.5 | 70.8 | 50.5 | 80.1 |
| p(t-test) |  | 0.10 |  | 0.19 |  | 0.030 |
| Min | 7.54 | 21.5 | 7.54 | 21.5 | 7.54 | 21.5 |
| Max | 240 | 250 | 240 | 250 | 240 | 250 |
| n (Samp) | 88 | 11 | 88 | 11 | 88 | 7 |
| n (Patient) | 88 | 11 | 88 | 11 | 88 | 7 |

| UO only | 0hr prior to AKI stage | | 24hr prior to AKI stage | |
| --- | --- | --- | --- | --- |
|  | Cohort 1 | Cohort 2 | Cohort 1 | Cohort 2 |
| Median | 75.7 | 82.6 | 75.7 | 51.5 |
| Average | 87.4 | 88.8 | 87.4 | 79.7 |

(continued)

| UO only | 0hr prior to AKI stage | | 24hr prior to AKI stage | |
|---|---|---|---|---|
| | Cohort 1 | Cohort 2 | Cohort 1 | Cohort 2 |
| Stdev | 57.4 | 63.1 | 57.4 | 63.2 |
| p(t-test) | | 0.95 | | 0.74 |
| Min | 7.54 | 21.5 | 7.54 | 21.5 |
| Max | 283 | 211 | 283 | 211 |
| n (Samp) | 89 | 7 | 89 | 7 |
| n (Patient) | 89 | 7 | 89 | 7 |

| | 0hr prior to AKI stage | | 24hr prior to AKI stage | | 48hr prior to AKI stage |
|---|---|---|---|---|---|
| | sCr or UO | UO only | sCr or UO | UO only | sCr or UO |
| AUC | 0.64 | 3.51 | 0.60 | 0.44 | 0.71 |
| SE | 0.094 | 0.11 | 0.095 | 0.12 | 0.11 |
| p | 0.13 | 0.92 | 0.29 | 0.61 | 0.065 |
| nCohort 1 | 88 | 89 | 88 | 89 | 88 |
| nCohort 2 | 11 | 7 | 11 | 7 | 7 |
| Cutoff 1 | 69.6 | 48.9 | 50.4 | 48.9 | 92.9 |
| Sens 1 | 73% | 71% | 73% | 71% | 71% |
| Spec 1 | 55% | 29% | 35% | 29% | 77% |
| Cutoff 2 | 49.9 | 42.2 | 49.9 | 42.2 | 69.6 |
| Sens 2 | 82% | 86% | 82% | 86% | 86% |
| Spec 2 | 35% | 22% | 35% | 22% | 55% |
| Cutoff 3 | 42.2 | 18.9 | 42.2 | 18.9 | 18.9 |
| Sens 3 | 91% | 100% | 91% | 100% | 100% |
| Spec 3 | 27% | 3% | 27% | 3% | 6% |
| Cutoff 4 | 89.6 | 92.9 | 89.6 | 92.9 | 89.6 |
| Sens 4 | 55% | 43% | 45% | 29% | 71% |
| Spec 4 | 70% | 71% | 70% | 71% | 70% |
| Cutoff 5 | 96.5 | 117 | 96.5 | 117 | 96.5 |
| Sens 5 | 36% | 14% | 27% | 14% | 43% |
| Spec 5 | 81% | 81% | 81% | 81% | 81% |
| Cutoff 6 | 142 | 186 | 142 | 186 | 142 |
| Sens 6 | 18% | 14% | 18% | 14% | 29% |
| Spec 6 | 91% | 91% | 91% | 91% | 91% |
| OR Quart | 2.0 | 0.48 | 3.1 | 2.1 | 0 |
| 2 | 0.58 | 0.56 | 0.34 | 0.56 | na |
| p Value | 0.17 | 0.040 | 0.30 | 0.18 | na |

(continued)

|  | 0hr prior to AKI stage | | 24hr prior to AKI stage | | 48hr prior to AKI stage |
|  | sCr or UO | UO only | sCr or UO | UO only | sCr or UO |
| --- | --- | --- | --- | --- | --- |
| 95% CI of OR Quart2 | 24 | 5.7 | 32 | 25 | na |
| OR Quart | 3.1 | 1.0 | 2.0 | 2.1 | 2.0 |
| 3 | 0.34 | 1.0 | 0.58 | 0.56 | 0.58 |
| p Value | 0.30 | 0.13 | 0.17 | 0.18 | 0.17 |
| 95% CI of OR Quart3 | 32 | 7.7 | 24 | 25 | 24 |
| OR Quart | 5.8 | 1.0 | 5.8 | 2.1 | 4.4 |
| 4 | 0.12 | 1.0 | 0.12 | 0.56 | 0.20 |
| p Value | 0.62 | 0.13 | 0.62 | 0.18 | 0.45 |
| 95% CI of OR Quart4 | 53 | 7.7 | 53 | 25 | 43 |

**Amphiregulin**

| sCr or UO | 0hr prior to AKI stage | | 24hr prior to AKI stage | | 48hr prior to AKI stage | |
|  | Cohort 1 | Cohort 2 | Cohort 1 | Cohort 2 | Cohort 1 | Cohort 2 |
| --- | --- | --- | --- | --- | --- | --- |
| Median | 22.9 | 37.9 | 22.9 | 37.9 | 22.9 | 26.0 |
| Average | 28.0 | 51.7 | 28.0 | 49.9 | 28.0 | 46.3 |
| Stdev | 20.9 | 42.8 | 20.9 | 41.2 | 20.9 | 52.2 |
| p(t-test) |  | 0.0028 |  | 0.0051 |  | 0.058 |
| Min | 0.672 | 16.1 | 0.672 | 16.1 | 0.672 | 16.1 |
| Max | 121 | 162 | 121 | 162 | 121 | 162 |
| n (Samp) | 87 | 11 | 87 | 11 | 87 | 7 |
| n (Patient) | 87 | 11 | 87 | 11 | 87 | 7 |

| UO only | 0hr prior to AKI stage | | 24hr prior to AKI stage | |
|  | Cohort 1 | Cohort 2 | Cohort 1 | Cohort 2 |
| --- | --- | --- | --- | --- |
| Median | 24.2 | 47.0 | 24.2 | 47.0 |
| Average | 31.0 | 65.5 | 31.0 | 62.6 |
| Stdev | 27.1 | 48.3 | 27.1 | 47.0 |
| p(t-test) |  | 0.0030 |  | 0.0062 |
| Min | 0.672 | 25.1 | 0.672 | 25.1 |
| Max | 198 | 162 | 198 | 162 |
| n (Samp) | 88 | 7 | 88 | 7 |
| n (Patient) | 88 | 7 | 88 | 7 |

| | 0hr prior to AKI stage | | 24hr prior to AKI stage | | 48hr prior to AKI stage |
|---|---|---|---|---|---|
| | sCr or UO | UO only | sCr or UO | UO only | sCr or UO |
| AUC | 0.73 | 0.82 | 0.73 | 0.81 | 0.63 |
| SE | 0.090 | 0.100 | 0.090 | 0.10 | 0.12 |
| p | 0.0095 | 0.0015 | 0.010 | 0.0018 | 0.27 |
| nCohort 1 | 87 | 88 | 87 | 88 | 87 |
| nCohort 2 | 11 | 7 | 11 | 7 | 7 |
| Cutoff 1 | 25.5 | 37.0 | 25.5 | 37.0 | 24.8 |
| Sens 1 | 73% | 71% | 73% | 71% | 71% |
| Spec 1 | 59% | 75% | 59% | 75% | 57% |
| Cutoff 2 | 24.8 | 32.6 | 24.8 | 32.6 | 18.3 |
| Sens 2 | 82% | 86% | 82% | 86% | 86% |
| Spec 2 | 57% | 68% | 57% | 68% | 43% |
| Cutoff 3 | 18.3 | 24.8 | 18.3 | 24.8 | 16.0 |
| Sens 3 | 91% | 100% | 91% | 100% | 100% |
| Spec 3 | 43% | 53% | 43% | 53% | 33% |
| Cutoff 4 | 32.2 | 34.0 | 32.2 | 34.0 | 32.2 |
| Sens 4 | 64% | 71% | 64% | 71% | 29% |
| Spec 4 | 70% | 70% | 70% | 70% | 70% |
| Cutoff 5 | 41.4 | 42.2 | 41.4 | 42.2 | 41.4 |
| Sens 5 | 45% | 57% | 45% | 57% | 29% |
| Spec 5 | 80% | 82% | 80% | 82% | 80% |
| Cutoff 6 | 55.7 | 56.8 | 55.7 | 56.8 | 55.7 |
| Sens 6 | 27% | 43% | 27% | 43% | 14% |
| Spec 6 | 91% | 91% | 91% | 91% | 91% |
| OR Quart 2 | >2.1 <0.56 | >0 <na | >2.1 <0.56 | >0 <na | >2.1 <0.56 |
| p Value | >0.18 | >na | >0.18 | >na | >0.18 |
| 95% CI of OR Quart2 | na | na | na | na | na |
| OR Quart 3 | >3.4 <0.30 | >3.3 <0.32 | >3.4 <0.30 | >3.3 <0.32 | >3.4 <0.30 |
| p Value | >0.33 | >0.32 | >0.33 | >0.32 | >0.33 |
| 95% Clot, OR Quart3 | na | na | na | na | na |
| OR Quart 4 | >7.6 <0.071 | >4.6 <0.19 | >7.6 <0.071 | >4.6 <0.19 | >2.1 <0.56 |
| p Value | >0.84 | >0.47 | >0.84 | >0.47 | >0.18 |
| 95% CI of OR Quart4 | na | na | na | na | na |

**Betacellulin**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 4.68 | 4.10 | 4.68 | 4.10 | 4.68 | 5.12 |
| Average | 5.23 | 11.0 | 5.23 | 11.0 | 5.23 | 15.1 |
| Stdev | 3.00 | 15.7 | 3.00 | 15.7 | 3.00 | 18.7 |
| p(t-test) | | 0.0025 | | 0.0025 | 2.2E-5 | 2.2E-5 |
| Min | 0.0147 | 0.00289 | 0.0147 | 0.00289 | 0.0147 | 2.42 |
| Max | 15.0 | 50.2 | 15.0 | 50.2 | 15.0 | 50.2 |
| n (Samp) | 87 | 11 | 87 | 11 | 87 | 7 |
| n (Patient) | 87 | 11 | 87 | 11 | 87 | 7 |

| UO only | 0hr prior to AKI stage | | 24hr prior to AKI stage | |
|---|---|---|---|---|
| | Cohort 1 | Cohort 2 | Cohort 1 | Cohort 2 |
| Median | 4.80 | 3.40 | 4.80 | 3.40 |
| Average | 5.62 | 7.76 | 5.62 | 7.76 |
| Stdev | 6.59 | 11.1 | 6.59 | 11.1 |
| p(t-test) | | 0.44 | | 0.44 |
| Min | 0.00274 | 0.00289 | 0.00274 | 0.00289 |
| Max | 60.7 | 32.2 | 60.7 | 32.2 |
| n (Samp) | 88 | 7 | 88 | 7 |
| n (Patient) | 88 | 7 | 88 | 7 |

| | 0hr prior to AKI stage | | 24hr prior to AKI stage | | 48hr prior to AKI stage |
|---|---|---|---|---|---|
| | sCr or UO | UO only | sCr or UO | UO only | sCr or UO |
| AUC | 0.51 | 0.43 | 0.51 | 0.43 | 0.60 |
| SE | 0.093 | 0.12 | 3.093 | 0.12 | 0.12 |
| p | 0.94 | 0.52 | 0.94 | 0.52 | 0.39 |
| nCohort 1 | 87 | 88 | 87 | 88 | 87 |
| nCohort 2 | 11 | 7 | 11 | 7 | 7 |
| Cutoff 1 | 3.32 | 3.09 | 3.32 | 3.09 | 4.08 |
| Sens 1 | 73% | 71% | 73% | 71% | 71% |
| Spec 1 | 24% | 24% | 24% | 24% | 39% |
| Cutoff 2 | 3.09 | 1.92 | 3.09 | 1.92 | 3.40 |
| Sens 2 | 82% | 86% | 82% | 86% | 86% |
| Spec 2 | 22% | 15% | 22% | 15% | 25% |
| Cutoff 3 | 1.92 | 0.00282 | 1.92 | 0.00282 | 1.92 |

(continued)

|  | 0hr prior to AKI stage | | 24hr prior to AKI stage | | 48hr prior to AKI stage |
|---|---|---|---|---|---|
|  | sCr or UO | UO only | sCr or UO | UO only | sCr or UO |
| Sens 3 | 91% | 100% | 91% | 100% | 100% |
| Spec 3 | 11% | 2% | 11% | 2% | 11% |
| Cutoff 4 | 6.11 | 6.11 | 6.11 | 6.11 | 6.11 |
| Sens 4 | 36% | 29% | 36% | 29% | 43% |
| Spec 4 | 70% | 70% | 70% | 70% | 70% |
| Cut off 5 | 7.64 | 7.64 | 7.64 | 7.64 | 7.64 |
| Sens 5 | 36% | 29% | 36% | 29% | 43% |
| Spec 5 | 80% | 81% | 80% | 81% | 80% |
| Cutoff 6 | 8.88 | 8.84 | 8.88 | 8.84 | 8.88 |
| Sens 6 | 27% | 29% | 27% | 29% | 29% |
| Spec 6 | 91% | 91% | 91% | 91% | 91% |
| OR Quart | 0.95 | 0 | 0.95 | 0 | 2.0 |
| 2 | 0.96 | na | 0.96 | na | 0.58 |
| p Value | 0.17 | na | 0.17 | na | 0.17 |
| 95% CI of OR Quart2 | 5.3 | na | 5.3 | na | 24 |
| OR Quart | 0.30 | 1.6 | 0.30 | 1.6 | 1.0 |
| 3 | 0.32 | 0.64 | 0.32 | 0.64 | 1.0 |
| p Value | 0.029 | 0.24 | 0.029 | 0.24 | 0.059 |
| 95% CI OR Quart3 | 3.2 | 10 | 3.2 | 10 | 17 |
| OR Quart | 1.3 | 1.0 | 1.3 | 1.0 | 3.1 |
| 4 | 0.73 | 0.96 | 0.73 | 0.96 | 0.34 |
| p Value | 0.27 | 0.13 | 0.27 | 0.13 | 0.30 |
| 95% CI ot OR Quart4 | 6.7 | 8.1 | 6.7 | 8.1 | 33 |

**Endostatin**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 38800 | 65700 | 38800 | 65700 | 38800 | 58900 |
| Average | 49600 | 90000 | 49600 | 89000 | 49600 | 96500 |
| Stdev | 39800 | 75300 | 39800 | 75500 | 39800 | 91900 |
| p(t-test) |  | 0.0058 |  | 0.0071 |  | 0.0094 |
| Min | 10500 | 14300 | 10500 | 14300 | 10500 | 14300 |
| Max | 328000 | 275000 | 328000 | 275000 | 328000 | 275000 |
| n (Samp) | 88 | 11 | 88 | 11 | 88 | 7 |
| n (Patient) | 88 | 11 | 88 | 11 | 88 | 7 |

| UO only | 0hr prior to AKI stage | | 24hr prior to AKI stage | |
|---|---|---|---|---|
| | Cohort 1 | Cohort 2 | Cohort 1 | Cohort 2 |
| Median | 44700 | 83700 | 44700 | 73100 |
| Average | 56000 | 110000 | 56000 | 108000 |
| Stdev | 43600 | 89900 | 43600 | 90500 |
| p(t-test) | | 0.0053 | | 0.0067 |
| Min | 10500 | 14300 | 10500 | 14300 |
| Max | 328000 | 275000 | 328000 | 275000 |
| n (Samp) | 89 | 7 | 89 | 7 |
| n (Patient) | 89 | 7 | 89 | 7 |

| | 0hr prior to AKI stage | | 24hr prior to AKI stage | | 48hr prior to AKI stage |
|---|---|---|---|---|---|
| | sCr or UO | UO only | sCr or UO | UO only | sCr or UO |
| AUC | 0.70 | 0.69 | 0.69 | 0.68 | 0.68 |
| SE | 0.092 | 0.11 | 0.092 | 0.12 | 0.12 |
| p | 0.030 | 0.094 | 0.036 | 0.11 | 0.13 |
| nCohort 1 | 88 | 89 | 88 | 89 | 88 |
| nCohort 2 | 11 | 7 | 11 | 7 | 7 |
| Cutoff 1 | 51400 | 65300 | 51400 | 65300 | 51400 |
| Sens 1 | 73% | 71% | 73% | 71% | 71% |
| Spec 1 | 68% | 73% | 68% | 73% | 68% |
| Cutoff 2 | 37100 | 32400 | 37100 | 32400 | 37100 |
| Sens 2 | 82% | 86% | 82% | 86% | 86% |
| Spec 2 | 47% | 28% | 47% | 28% | 47% |
| Cutoff 3 | 32400 | 13300 | 32400 | 13300 | 13300 |
| Sens 3 | 91% | 100% | 91% | 100% | 100% |
| Spec 3 | 33% | 4% | 33% | 4% | 6% |
| Cutoff 4 | 54400 | 62700 | 54400 | 62700 | 54400 |
| Sens 4 | 64% | 71% | 64% | 71% | 57% |
| Spec 4 | 70% | 71% | 70% | 71% | 70% |
| Cutoff 5 | 67500 | 75500 | 67500 | 75500 | 67500 |
| Sens 5 | 45% | 57% | 45% | 43% | 43% |
| Spec 5 | 81% | 81% | 81% | 81% | 81% |
| Cutoff 6 | 84900 | 112000 | 84900 | 112000 | 84900 |
| Sens 6 | 27% | 43% | 27% | 43% | 29% |
| Spec 6 | 91% | 91% | 91% | 91% | 91% |
| OR Quart | 2.0 | 1.0 | 2.0 | 1.0 | 0.96 |
| 2 | 0.58 | 1.0 | 0.58 | 1.0 | 0.98 |

(continued)

| | 0hr prior to AKI stage | | 24hr prior to AKI stage | | 48hr prior to AKI stage |
| | sCr or UO | UO only | sCr or UO | UO only | sCr or UO |
|---|---|---|---|---|---|
| p Value | 0.17 | 0.059 | 0.17 | 0.059 | 0.056 |
| 95% CI of OR Quart2 | 24 | 17 | 24 | 17 | 16 |
| OR Quart | 3.1 | 1.0 | 3.1 | 1.0 | 2.0 |
| 3 | 0.34 | 1.0 | 0.34 | 1.0 | 0.58 |
| p Value | 0.30 | 0.059 | 0.30 | 0.059 | 0.17 |
| 95% CI of OR Quart3 | 32 | 17 | 32 | 17 | 24 |
| OR Quart | 5.8 | 4.6 | 5.8 | 4.6 | 3.1 |
| 4 | 0.12 | 0.19 | 0.12 | 0.19 | 0.34 |
| p Value | 0.62 | 0.47 | 0.62 | 0.47 | 0.30 |
| 95% CI of OR Quart4 | 53 | 45 | 53 | 45 | 33 |

**Proepiregulin**

| sCr or UO | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
|---|---|---|---|---|---|---|
| Median | 0.416 | 1.00 | 0.416 | 0.626 | 0.416 | 0.561 |
| Average | 0.599 | 3.17 | 0.599 | 1.67 | 0.599 | 0.714 |
| Stdev | 0.808 | 5.62 | 0.808 | 3.24 | 0.808 | 0.508 |
| p(t-test) | | 9.6E-5 | | 0.011 | | 0.71 |
| Min | 0.000896 | 0.0249 | 0.000896 | 0.0249 | 0.000896 | 0.188 |
| Max | 6.96 | 17.1 | 6.96 | 11.3 | 6.96 | 1.47 |
| n (Samp) | 87 | 11 | 87 | 11 | 87 | 7 |
| n (Patient) | 87 | 11 | 87 | 11 | 87 | 7 |

| UO only | 0hr prior to AKI stage | | 24hr prior to AKI stage | |
| | Cohort 1 | Cohort 2 | Cohort 1 | Cohort 2 |
|---|---|---|---|---|
| Median | 0.472 | 1.19 | 0.472 | 0.626 |
| Average | 1.04 | 4.50 | 1.04 | 2.15 |
| Stdev | 3.51 | 6.85 | 3.51 | 4.07 |
| p(t-test) | | 0.023 | | 0.43 |
| Min | 0.000896 | 0.0249 | 0.000896 | 0.0249 |
| Max | 32.1 | 17.1 | 32.1 | 11.3 |
| n (Samp) | 88 | 7 | 88 | 7 |
| n (Patient) | 88 | 7 | 88 | 7 |

| | 0hr prior to AKI stage | | 24hr prior to AKI stage | | 48hr prior to AKI stage |
|---|---|---|---|---|---|
| | sCr or UO | UO only | sCr or UO | UO only | sCr or UO |
| AUC | 0.67 | 0.64 | 0.64 | 0.59 | 0.61 |
| SE | 0.094 | 0.12 | 0.095 | 0.12 | 0.12 |
| p | 0.067 | 0.23 | 0.15 | 0.45 | 0.36 |
| nCohort 1 | 87 | 88 | 87 | 88 | 87 |
| nCohort 2 | 11 | 7 | 11 | 7 | 7 |
| Cutoff 1 | 0.356 | 0.356 | 0.356 | 0.356 | 0.286 |
| Sens 1 | 73% | 71% | 73% | 71% | 71% |
| Spec 1 | 43% | 38% | 43% | 38% | 31% |
| Cutoff 2 | 0.261 | 0.179 | 0.261 | 0.179 | 0.261 |
| Sens 2 | 82% | 86% | 82% | 86% | 86% |
| Spec 2 | 31% | 20% | 31% | 20% | 31% |
| Cutoff 3 | 0.179 | 0.0201 | 0.179 | 0.0201 | 0.179 |
| Sens 3 | 91% | 100% | 91% | 100% | 100% |
| Spec 3 | 23% | 5% | 23% | 5% | 23% |
| Cutoff 4 | 0.695 | 0.717 | 0.695 | 0.717 | 0.695 |
| Sens 4 | 55% | 57% | 45% | 43% | 43% |
| Spec 4 | 71% | 70% | 71% | 70% | 71% |
| Cutoff 5 | 0.848 | 0.893 | 0.848 | 0.893 | 0.848 |
| Sens 5 | 55% | 57% | 45% | 43% | 43% |
| Spec 5 | 82% | 81% | 82% | 81% | 82% |
| Cutoff 6 | 1.00 | 1.07 | 1.00 | 1.07 | 1.00 |
| Sens 6 | 55% | 57% | 45% | 43% | 43% |
| Spec 6 | 91% | 91% | 91% | 91% | 91% |
| OR Quart | 0.96 | 0.46 | 0.96 | 0.46 | 2.0 |
| 2 | 0.97 | 0.53 | 0.97 | 0.53 | 0.58 |
| p Value | 0.12 | 0.039 | 0.12 | 0.039 | 0.17 |
| 95% CI of OR Quart2 | 7.4 | 5.4 | 7.4 | 5.4 | 24 |
| OR Quart | 0.48 | 0 | 1.0 | 0.46 | 1.0 |
| 3 | 0.56 | na | 1.0 | 0.53 | 1.0 |
| p Value | 0.040 | na | 0.13 | 0.039 | 3.059 |
| 95% CI of OR Quart3 | 5.7 | na | 7.7 | 5.4 | 17 |
| OR Quart | 3.5 | 2.1 | 2.8 | 1.5 | 3.1 |
| 4 | 0.15 | 0.42 | 0.26 | 0.67 | 0.34 |
| p Value | 0.63 | 0.35 | 0.48 | 0.23 | 0.30 |
| 95% CI of OR Quart4 | 19 | 13 | 16 | 9.9 | 33 |

**Fibroblast growth factor 19**

| sCr or UO | Ohr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.197 | 0.274 | 0.197 | 0.274 | 0.197 | 0.274 |
| Average | 0.208 | 0.379 | 0.208 | 0.379 | 0.208 | 0.275 |
| Stdev | 0.188 | 0.380 | 0.188 | 0.381 | 0.188 | 0.167 |
| p(t-test) | | 0.015 | | 0.015 | | 0.36 |
| Min | 8.23E-5 | 0.000127 | 8.23E-5 | 0.000127 | 8.23E-5 | 0.000127 |
| Max | 0.948 | 1.32 | 0.948 | 1.32 | 0.948 | 0.482 |
| n (Samp) | 88 | 11 | 88 | 11 | 88 | 7 |
| n (Patient) | 88 | 11 | 88 | 11 | 88 | 7 |

| UO only | 0hr prior to AKI stage | | 24hr prior to AKI stage | |
|---|---|---|---|---|
| | Cohort 1 | Cohort 2 | Cohort 1 | Cohort 2 |
| Median | 0.213 | 0.385 | 0.213 | 0.385 |
| Average | 0.255 | 0.433 | 0.255 | 0.433 |
| Stdev | 0.272 | 0.470 | 0.272 | 0.471 |
| p(t-test) | | 0.12 | | 0.12 |
| Min | 8.23E-5 | 0.000127 | 8.23E-5 | 0.000127 |
| Max | 2.09 | 1.32 | 2.09 | 1.32 |
| n (Samp) | 89 | 7 | 89 | 7 |
| n (Patient) | 89 | 7 | 89 | 7 |

| | 0hr prior to AKI stage | | 24hr prior to AKI stage | | 48hr prior to AKI stage |
|---|---|---|---|---|---|
| | sCr or UO | UO only | sCr or UO | UO only | sCr or UO |
| AUC | 0.66 | 0.60 | 0.66 | 0.60 | 0.65 |
| SE | 0.094 | 0.12 | 0.094 | 0.12 | 0.12 |
| p | 0.099 | 0.41 | 0.099 | 0.41 | 0.19 |
| nCohort 1 | 88 | 89 | 88 | 89 | 88 |
| nCohot 2 | 11 | 7 | 11 | 7 | 7 |
| Cutoff 1 | 0.125 | 0.124 | 0.125 | 0.124 | 0.260 |
| Sens 1 | 73% | 71% | 73% | 71% | 71% |
| Spec 1 | 42% | 31% | 42% | 31% | 70% |
| Cutoff 2 | 0.124 | 0.0430 | 0.124 | 0.0430 | 0.124 |
| Sens 2 | 82% | 86% | 82% | 86% | 86% |
| Spec 2 | 42% | 19% | 42% | 19% | 42% |
| Cutoff 3 | 0.0421 | 8.23E-5 | 0.0421 | 8.23E-5 | 8.23E-5 |

(continued)

|  | 0hr prior to AKI stage | | 24hr prior to AKI stage | | 48hr prior to AKI stage |
|  | sCr or UO | UO only | sCr or UO | UO only | sCr or UO |
| --- | --- | --- | --- | --- | --- |
| Sens 3 | 91% | 100% | 91% | 100% | 100% |
| Spec 3 | 24% | 2% | 24% | 2% | 7% |
| Cutoff 4 | 0.260 | 0.285 | 0.260 | 0.285 | 0.260 |
| Sens 4 | 64% | 57% | 64% | 57% | 71% |
| Spec 4 | 70% | 71% | 70% | 71% | 70% |
| Cutoff 5 | 0.321 | 0.368 | 0.321 | 0.368 | 0.321 |
| Sens 5 | 45% | 57% | 45% | 57% | 43% |
| Spec 5 | 81% | 81% | 81% | 81% | 81% |
| Cutoff 6 | 0.485 | 0.508 | 0.485 | 0.508 | 0.485 |
| Sens 6 | 18% | 29% | 18% | 29% | 0% |
| Spec 6 | 91% | 91% | 91% | 91% | 91% |
| OR Quart | 0.96 | 0.48 | 0.96 | 0.48 | 0.96 |
| 2 | 0.97 | 0.56 | 0.97 | 0.56 | 0.98 |
| p Value | 0.12 | 0.040 | 0.12 | 0.040 | 0.056 |
| 95% CI of OR Quart2 | 7.4 | 5.7 | 7.4 | 5.7 | 16 |
| OR Quart | 0.96 | 0 | 0.96 | 0 | 2.0 |
| 3 | 0.97 | na | 0.97 | na | 0.58 |
| p Value | 0.12 | na | 0.12 | na | 0.17 |
| 95% CI of OR Quart3 | 7.4 | na | 7.4 | na | 24 |
| OR Quart | 2.8 | 2.2 | 2.8 | 2.2 | 3.1 |
| 4 | 0.26 | 0.39 | 0.26 | 0.39 | 0.34 |
| p Value | 0.48 | 0.36 | 0.48 | 0.36 | 0.30 |
| 95% CI of OR Quart4 | 16 | 13 | 16 | 13 | 33 |

**Fibroblast growth factor 21**

| sCr or UO | 0hr prior to AKI stage | | 24hr prior to AKI stage | | 48hr prior to AKI stage | |
|  | Cohort 1 | Cohort 2 | Cohort 1 | Cohort 2 | Cohort 1 | Cohort 2 |
| --- | --- | --- | --- | --- | --- | --- |
| Median | 0.0451 | 0.418 | 0.0451 | 0.311 | 0.0451 | 0.295 |
| Average | 0.230 | 0.798 | 0.230 | 0.647 | 0.230 | 0.670 |
| Stdev | 0.539 | 0.794 | 0.539 | 0.655 | 0.539 | 0.799 |
| p(t-test) |  | 0.0025 |  | 0.020 |  | 0.048 |
| Min | 9.65E-6 | 0.0658 | 9.65E-6 | 0.0658 | 9.65E-6 | 0.0658 |
| Max | 3.21 | 2.26 | 3.21 | 2.11 | 3.21 | 2.11 |
| n (Samp) | 88 | 11 | 88 | 11 | 88 | 7 |
| n (Patient) | 88 | 11 | 88 | 11 | 88 | 7 |

| UO only | 0hr prior to AKI stage | | 24hr prior to AKI stage | |
|---|---|---|---|---|
| | Cohort 1 | Cohort 2 | Cohort 1 | Cohort 2 |
| Median | 0.0674 | 0.605 | 0.0674 | 0.418 |
| Average | 0.284 | 0.955 | 0.284 | 0.718 |
| Stdev | 0.564 | 0.869 | 0.564 | 0.694 |
| p(t-test) | | 0.0046 | | 0.057 |
| Min | 9.65E-6 | 0.159 | 9.65E-6 | 0.159 |
| Max | 3.21 | 2.26 | 3.21 | 2.11 |
| n (Samp) | 89 | 7 | 89 | 7 |
| n (Patient) | 89 | 7 | 89 | 7 |

| | 0hr prior to AKI stage | | 24hr prior to AKI stage | | 48hr prior to AKI stage |
|---|---|---|---|---|---|
| | sCr or UO | UO only | sCr or UO | UO only | sCr or UO |
| AUC | 0.87 | 0.87 | 0.86 | 0.84 | 0.84 |
| SE | 0.071 | 0.087 | 0.073 | 0.095 | 0.095 |
| p | 1.9E-7 | 1.8E-5 | 1.2E-6 | 3.3E-4 | 3.0E-4 |
| nCohort 1 | 88 | 89 | 88 | 89 | 88 |
| nCohort 2 | 11 | 7 | 11 | 7 | 7 |
| Cutoff 1 | 0.277 | 0.387 | 0.198 | 0.281 | 0.194 |
| Sens 1 | 73% | 71% | 73% | 71% | 71% |
| Spec 1 | 86% | 83% | 83% | 82% | 83% |
| Cutoff 2 | 0.186 | 0.281 | 0.186 | 0.186 | 0.186 |
| Sens 2 | 82% | 86% | 82% | 86% | 86% |
| Spec 2 | 83% | 82% | 83% | 74% | 83% |
| Cutoff 3 | 0.143 | 0.143 | 0.143 | 0.143 | 0.0619 |
| Sens 3 | 91% | 100% | 91% | 100% | 100% |
| Spec 3 | 81% | 72% | 81% | 72% | 59% |
| Cutoff 4 | 0.0949 | 0.134 | 0.0949 | 0.134 | 0.0949 |
| Sens 4 | 91% | 100% | 91% | 100% | 86% |
| Spec 4 | 70% | 71% | 70% | 71% | 70% |
| Cutoff 5 | 0.143 | 0.267 | 0.143 | 0.267 | 0.143 |
| Sens 5 | 91% | 86% | 91% | 71% | 86% |
| Spec 5 | 81% | 81% | 81% | 81% | 81% |
| Cutoff 6 | 0.687 | 1.23 | 0.687 | 1.23 | 0.687 |
| Sens 6 | 36% | 29% | 36% | 14% | 29% |
| Spec 6 | 91% | 91% | 91% | 91% | 91% |
| OR Quart | >0 | >0 | >0 | >0 | >0 |
| 2 | <na | <na | <na | <na | <na |

(continued)

|  | 0hr prior to AKI stage | | 24hr prior to AKI stage | | 48hr prior to AKI stage |
|---|---|---|---|---|---|
|  | sCr or UO | UO only | sCr or UO | UO only | sCr or UO |
| p Value | >na | >na | >na | >na | >na |
| 95% CI of OR Quart2 | na | na | na | na | na |
| OR Quart 3 | >2.1 | >1.0 | >2.1 | >2.2 | >1.0 |
|  | <0.56 | <0.98 | <0.56 | <0.54 | <1.0 |
| p Value | >0.18 | >0.062 | >0.18 | >0.18 | >0.059 |
| 95% CI of OR Quart3 | na | na | na | na | na |
| OR Quart | >14 | >8.0 | >14 | >6.3 | >7.7 |
|  | <0.018 | <0.064 | <0.018 | <0.11 | <0.070 |
| p Value | >1.6 | >0.88 | >1.6 | >0.68 | >0.85 |
| 95% CI of OR Quart4 | na | na | na | na | na |

**Heparin-binding EGF-like growth factor**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 17.3 | 36.4 | 17.3 | 31.2 | 17.3 | 22.6 |
| Average | 20.5 | 62.5 | 20.5 | 57.2 | 20.5 | 62.8 |
| Stdev | 13.5 | 61.2 | 13.5 | 61.5 | 13.5 | 77.9 |
| p(t-test) | 2.2E-7 | 2.2E-7 |  | 4.7E-6 |  | 1.8E-5 |
| Min | 7.16 | 11.6 | 7.16 | 11.6 | 7.16 | 11.6 |
| Max | 98.9 | 222 | 98.9 | 222 | 98.9 | 222 |
| n (Samp) | 87 | 11 | 87 | 11 | 87 | 7 |
| n (Patient) | 87 | 11 | 87 | 11 | 87 | 7 |

| UO only | 0hr prior to AKI stage | | 24hr prior to AKI stage | |
|---|---|---|---|---|
|  | Cohort 1 | Cohort 2 | Cohort 1 | Cohort 2 |
| Median | 18.6 | 44.7 | 18.6 | 36.4 |
| Average | 23.6 | 58.7 | 23.6 | 50.3 |
| Stdev | 22.4 | 31.2 | 22.4 | 30.7 |
| p(t-test) |  | 2.0E-4 |  | 0.0039 |
| Min | 7.16 | 29.4 | 7.16 | 26.9 |
| Max | 186 | 110 | 186 | 110 |
| n (Samp) | 88 | 7 | 88 | 7 |
| n (Patient) | 88 | 7 | 88 | 7 |

|  | 0hr prior to AKI stage | | 24hr prior to AKI stage | | 48hr prior to AKI stage |
|---|---|---|---|---|---|
|  | sCr or UO | UO only | sCr or UO | UO only | sCr or UO |
| AUC | 0.84 | 0.91 | 0.83 | 0.88 | 0.75 |
| SE | 0.076 | 0.075 | 0.079 | 0.085 | 0.11 |
| p | 8.6E-6 | 4.9E-8 | 3.4E-5 | 6.8E-6 | 0.025 |
| nCohort 1 | 87 | 88 | 87 | 88 | 87 |
| nCohort 2 | 11 | 7 | 11 | 7 | 7 |
| Cutoff 1 | 29.0 | 33.3 | 26.6 | 31.2 | 21.2 |
| Sens 1 | 73% | 71% | 73% | 71% | 71% |
| Spec 1 | 87% | 86% | 84% | 84% | 71% |
| Cutoff 2 | 21.8 | 31.2 | 21.8 | 29.0 | 20.9 |
| Sens 2 | 82% | 86% | 82% | 86% | 86% |
| Spec 2 | 74% | 84% | 74% | 81% | 69% |
| Cutoff 3 | 20.9 | 29.0 | 20.9 | 25.3 | 11.5 |
| Sens 3 | 91% | 100% | 91% | 100% | 100% |
| Spec 3 | 69% | 81% | 69% | 77% | 18% |
| Cutoff 4 | 21.1 | 21.8 | 21.1 | 21.8 | 21.1 |
| Sens 4 | 82% | 100% | 82% | 100% | 71% |
| Spec 4 | 70% | 70% | 70% | 70% | 70% |
| Cutoff 5 | 24.6 | 29.0 | 24.6 | 29.0 | 24.6 |
| Sens 5 | 73% | 100% | 73% | 86% | 43% |
| Spec 5 | 80% | 81% | 80% | 81% | 80% |
| Cutoff 6 | 31.2 | 40.1 | 31.2 | 40.1 | 31.2 |
| Sens 6 | 64% | 57% | 55% | 43% | 43% |
| Spec 6 | 91% | 91% | 91% | 91% | 91% |
| OR Quart | 0 | >0 | 0 | >0 | 0 |
| 2 | na | <na | na | <na | na |
| p Value | na | >na | na | >na | na |
| 95% CI of OR Quart2 | na | na | na | na | na |
| OR Quart | 2.1 | >0 | 2.1 | >1.0 | 3.3 |
| 3 | 0.56 | <na | 0.56 | <1.0 | 0.32 |
| p Value | 0.18 | >na | 0.18 | >0.059 | 0.32 |
| 95% CI of OR Quart3 | 25 | na | 25 | na | 34 |
| OR Quart | 11 | >9.5 | 11 | >7.7 | 3.1 |
| 4 | 0.032 | <0.044 | 0.032 | <0.070 | 0.34 |
| p Value | 1.2 | >1.1 | 1.2 | >0.85 | 0.30 |
| 95% CI of OR Quart4 | 95 | na | 95 | na | 33 |

**Thrombospondin-2**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 40300 | 95600 | 40300 | 95600 | 40300 | 72500 |
| Average | 68700 | 147000 | 68700 | 134000 | 68700 | 111000 |
| Stdev | 81100 | 159000 | 81100 | 143000 | 81100 | 146000 |
| p(t-test) | | 0.0093 | | 0.024 | | 0.22 |
| Min | 1030 | 15000 | 1030 | 14100 | 1030 | 15000 |
| Max | 591000 | 455000 | 591000 | 433000 | 591000 | 433000 |
| n (Samp) | 88 | 11 | 88 | 11 | | 7 |
| n (Patient) | 88 | 11 | 88 | 11 | 88 | 7 |

| UO only | 0hr prior to AKI stage | | 24hr prior to AKI stage | |
|---|---|---|---|---|
| | Cohort 1 | Cohort 2 | Cohort 1 | Cohort 2 |
| Median | 50300 | 95800 | 50300 | 95800 |
| Average | 75600 | 199000 | 75600 | 179000 |
| Stdev | 82100 | 180000 | 82100 | 164000 |
| p(t-test) | | 9.2E-4 | | 0.0043 |
| Min | 2770 | 24000 | 2770 | 14100 |
| Max | 591000 | 455000 | 591000 | 433000 |
| n (Samp) | 89 | 7 | 89 | 7 |
| n (Patient) | 89 | 7 | 89 | 7 |

| | 0hr prior to AKI stage | | 24hr prior to AKI stage | | 48hr prior to AKI stage |
|---|---|---|---|---|---|
| | sCr or UO | UO only | sCr or UO | UO only | sCr or UO |
| AUC | 0.67 | 0.74 | 0.63 | 0.69 | 0.58 |
| SE | 0.094 | 0.11 | 0.095 | 0.11 | 0.12 |
| p | 0.067 | 0.034 | 0.16 | 0.10 | 0.49 |
| nCohort 1 | 88 | 89 | 88 | 89 | 88 |
| nCohort 2 | 11 | 7 | 11 | 7 | 7 |
| Cutoff 1 | 50600 | 91600 | 27700 | 91600 | 27700 |
| Sens 1 | 73% | 71% | 73% | 71% | 71% |
| Spec 1 | 60% | 69% | 35% | 69% | 35% |
| Cutoff 2 | 27700 | 50500 | 23400 | 23400 | 23400 |
| Sens 2 | 82% | 86% | 82% | 86% | 86% |
| Spec 2 | 35% | 52% | 32% | 31% | 32% |
| Cutoff 3 | 23400 | 23400 | 14100 | 14100 | 13900 |

(continued)

| | 0hr prior to AKI stage | | 24hr prior to AKI stage | | 48hr prior to AKI stage |
| --- | --- | --- | --- | --- | --- |
| | sCr or UO | UO only | sCr or UO | UO only | sCr or UO |
| Sens 3 | 91% | 100% | 91% | 100% | 100% |
| Spec 3 | 32% | 31% | 17% | 19% | 17% |
| Cutoff 4 | 77700 | 100000 | 77700 | 100000 | 77700 |
| Sens 4 | 55% | 43% | 55% | 43% | 43% |
| Spec 4 | 70% | 71% | 70% | 71% | 70% |
| Cutoff 5 | 110000 | 126000 | 110000 | 126000 | 110000 |
| Sens 5 | 36% | 43% | 36% | 43% | 29% |
| Spec 5 | 81% | 81% | 81% | 81% | 81% |
| Cutoff 6 | 159000 | 171000 | 159000 | 171000 | 159000 |
| Sens 6 | 27% | 43% | 27% | 43% | 14% |
| Spec 6 | 91% | 91% | 91% | 91% | 91% |
| OR Quart | 2.0 | >2.2 | 0.96 | 1.0 | 2.0 |
| 2 | 0.58 | <0.54 | 0.97 | 1.0 | 0.58 |
| p Value | 0.17 | >0.18 | 0.12 | 0.059 | 0.17 |
| 95% CI of OR Quart2 | 24 | na | 7.4 | 17 | 24 |
| OR Quart | 4.4 | >2.2 | 1.5 | 2.1 | 0.96 |
| 3 | 0.20 | <0.54 | 0.67 | 0.56 | 0.98 |
| p Value | 0.45 | >0.18 | 0.23 | 0.18 | 0.056 |
| 95% CI of OR Quart3 | 42 | na | 9.9 | 25 | 16 |
| OR Quart | 4.4 | >3.4 | 2.1 | 3.3 | 3.1 |
| 4 | 0.20 | <0.30 | 0.42 | 0.32 | 0.34 |
| p Value | 0.45 | >0.33 | 0.35 | 0.32 | 0.30 |
| 95% CI of OR Quart4 | 42 | na | 13 | 34 | 33 |

**Tenascin**

| sCr or UO | 0hr prior to AKI stage | | 24hr prior to AKI stage | | 48hr prior to AKI stage | |
| --- | --- | --- | --- | --- | --- | --- |
| | Cohort 1 | Cohort 2 | Cohort 1 | Cohort 2 | Cohort 1 | Cohort 2 |
| Median | 483 | 1240 | 483 | 1240 | 483 | 1180 |
| Average | 912 | 1670 | 912 | 1410 | 912 | 1150 |
| Stdev | 1550 | 1380 | 1550 | 584 | 1550 | 333 |
| p(t-test) | | 0.13 | | 0.30 | | 0.69 |
| Min | 94.2 | 815 | 94.2 | 815 | 94.2 | 815 |
| Max | 13100 | 5700 | 13100 | 2830 | 13100 | 1780 |
| n (Samp) | 88 | 11 | 88 | 11 | 88 | 7 |
| n (Patient) | 88 | 11 | 88 | 11 | 88 | 7 |

| UO only | 0hr prior to AKI stage | | 24hr prior to AKI stage | |
|---|---|---|---|---|
| | Cohort 1 | Cohort 2 | Cohort 1 | Cohort 2 |
| Median | 527 | 1260 | 527 | 1260 |
| Average | 1410 | 1960 | 1410 | 1550 |
| Stdev | 3250 | 1670 | 3250 | 632 |
| p(t-test) | | 0.66 | | 0.91 |
| Min | 63.0 | 959 | 63.0 | 959 |
| Max | 26100 | 5700 | 26100 | 2830 |
| n (Samp) | 89 | 7 | 89 | 7 |
| n (Patient) | 89 | 7 | 89 | 7 |

| | 0hr prior to AKI stage | | 24hr prior to AKI stage | | 48hr prior to AKI stage |
|---|---|---|---|---|---|
| | sCr or UO | UO only | sCr or UO | UO only | sCr or UO |
| AUC | 0.86 | 0.82 | 0.85 | 0.82 | 0.83 |
| SE | 0.074 | 0.099 | 0.074 | 0.100 | 0.098 |
| p | 1.4E-6 | 0.0010 | 2.3E-6 | 0.0015 | 8.7E-4 |
| nCohort 1 | 88 | 89 | 88 | 89 | 88 |
| nCohort 2 | 11 | 7 | 11 | 7 | 7 |
| Cutoff 1 | 1140 | 1220 | 1140 | 1220 | 866 |
| Sens 1 | 73% | 71% | 73% | 71% | 71% |
| Spec 1 | 85% | 80% | 85% | 80% | 80% |
| Cutoff 2 | 866 | 1200 | 866 | 1200 | 815 |
| Sens 2 | 82% | 86% | 82% | 86% | 86% |
| Spec 2 | 80% | 80% | 80% | 80% | 76% |
| Cutoff 3 | 815 | 905 | 815 | 905 | 792 |
| Sens 3 | 91% | 100% | 91% | 100% | 100% |
| Spec 3 | 76% | 72% | 76% | 72% | 76% |
| Cutoff 4 | 697 | 866 | 697 | 866 | 697 |
| Sens 4 | 100% | 100% | 100% | 100% | 100% |
| Spec 4 | 70% | 71% | 70% | 71% | 70% |
| Cutoff 5 | 969 | 1250 | 969 | 1250 | 969 |
| Sens 5 | 73% | 57% | 73% | 57% | 57% |
| Spec 5 | 81% | 81% | 81% | 81% | 81% |
| Cutoff 6 | 2080 | 2750 | 2080 | 2750 | 2080 |
| Sens 6 | 9% | 14% | 9% | 14% | 0% |
| Spec 6 | 91% | 91% | 91% | 91% | 91% |
| OR Quart | >0 | >0 | >0 | >0 | >0 |
| 2 | <na | <na | <na | <na | <na |

(continued)

| | 0hr prior to AKI stage | | 24hr prior to AKI stage | | 48hr prior to AKI stage |
|---|---|---|---|---|---|
| | sCr or UO | UO only | sCr or UO | UO only | sCr or UO |
| p Value | >na | >na | >na | >na | >na |
| 95% CI OR Quart2 | na | na | na | na | na |
| OR Quart 3 | >3.3 | >1.0 | >3.3 | >1.0 | >2.1 |
| | <0.32 | <0.98 | <0.32 | <0.98 | <0.56 |
| p Value | >0.32 | >0.062 | >0.32 | >0.062 | >0.18 |
| 95% CI of OR Quart3 | na | na | na | na | na |
| OR Quart 4 | >11 | >8.0 | >11 | >8.0 | >6.1 |
| | <0.029 | <0.064 | <0.029 | <0.064 | <0.11 |
| p Value | >1.3 | >0.88 | >1.3 | >0.88 | >0.65 |
| 95% CI of OR Quart4 | na | na | na | na | na |

Fig. 9: Comparison of marker levels in urine samples collected from Cohort 1 (patients that did not progress beyond RIFLE stage 0, R, or I) and in urine samples collected from Cohort 2 (subjects who progress to RIFLE stage F) at 0, 24 hours, and 48 hours prior to the subject reaching RIFLE stage I.

**Angiogenin**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 6050 | 14100 | 6050 | 10600 | 6050 | 5430 |
| Average | 9790 | 13000 | 9790 | 12400 | 9790 | 8880 |
| Stdev | 8750 | 9340 | 8750 | 9330 | 8750 | 9610 |
| p(t-test) | | 0.080 | | 0.15 | | 0.70 |
| Min | 0.00873 | 647 | 0.00873 | 849 | 0.00873 | 54.6 |
| Max | 30600 | 30600 | 30600 | 30600 | 30600 | 30600 |
| n (Samp) | 1483 | 23 | 1483 | 24 | 1483 | 14 |
| n (Patient) | 493 | 23 | 493 | 24 | 493 | 14 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 6110 | 14100 | 6110 | 20000 | 6110 | 11200 |
| Average | 9850 | 15100 | 9850 | 15400 | 9850 | 13100 |
| Stdev | 8800 | 10300 | 8800 | 9770 | 8800 | 9260 |
| p(t-test) | | 0.076 | | 0.036 | | 0.23 |
| Min | 0.00873 | 647 | 0.00873 | 1440 | 0.00873 | 854 |
| Max | 30600 | 30600 | 30600 | 30600 | 30600 | 30600 |
| n (Samp) | 1540 | 9 | 1540 | 11 | 1540 | 11 |

(continued)

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| n (Patient) | 509 | 9 | 509 | 11 | 509 | 11 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 6360 | 20200 | 6360 | 9890 | nd | nd |
| Average | 10000 | 14600 | 10000 | 11000 | nd | nd |
| Stdev | 8840 | 9830 | 8840 | 9090 | nd | nd |
| p(t-test) | | 0.060 | | 0.62 | nd | nd |
| Min | 0.00873 | 903 | 0.00873 | 849 | nd | nd |
| Max | 30600 | 28600 | 30600 | 30600 | nd | nd |
| n (Samp) | 1513 | 13 | 1513 | 18 | nd | nd |
| n (Patient) | 472 | 13 | 472 | 18 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.60 | 0.66 | 0.61 | 0.59 | 0.67 | 0.54 | 0.44 | 0.62 | nd |
| SE | 0.063 | 0.100 | 0.084 | 0.062 | 0.090 | 0.070 | 0.080 | 0.091 | nd |
| p | 0.11 | 0.11 | 0.18 | 0.14 | 0.052 | 0.55 | 0.44 | 0.19 | nd |
| nCohort 1 | 1483 | 1540 | 1513 | 1483 | 1540 | 1513 | 1483 | 1540 | nd |
| nCohort 2 | 23 | 9 | 13 | 24 | 11 | 18 | 14 | 11 | nd |
| Cutoff 1 | 5510 | 8720 | 2120 | 4960 | 6090 | 4140 | 1620 | 6520 | nd |
| Sens 1 | 74% | 78% | 77% | 71% | 73% | 72% | 71% | 73% | nd |
| Spec 1 | 47% | 58% | 20% | 44% | 50% | 37% | 15% | 51% | nd |
| Cutoff 2 | 2120 | 4780 | 1750 | 2580 | 4960 | 2480 | 844 | 5660 | nd |
| Sens 2 | 83% | 89% | 85% | 83% | 82% | 83% | 86% | 82% | nd |
| Spec 2 | 21% | 43% | 16% | 25% | 44% | 24% | 7% | 48% | nd |
| Cutoff 3 | 964 | 645 | 964 | 2260 | 3510 | 1050 | 409 | 3240 | nd |
| Sens 3 | 91% | 100% | 92% | 92% | 91% | 94% | 93% | 91% | nd |
| Spec 3 | 9% | 5% | 8% | 22% | 33% | 9% | 3% | 31% | nd |
| Cutoff 4 | 14400 | 14600 | 14900 | 14400 | 14600 | 14900 | 14400 | 14600 | nd |
| Sens 4 | 48% | 44% | 54% | 42% | 64% | 33% | 29% | 45% | nd |
| Spec 4 | 70% | 70% | 70% | 70% | 70% | 70% | 70% | 70% | nd |
| Cutoff 5 | 19800 | 20000 | 20300 | 19800 | 20000 | 20300 | 19800 | 20000 | nd |
| Sens 5 | 39% | 33% | 46% | 29% | 55% | 22% | 21% | 36% | nd |
| Spec 5 | 80% | 80% | 80% | 80% | 80% | 80% | 80% | 80% | nd |

(continued)

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| Cutoff 6 | 22700 | 22700 | 23400 | 22700 | 22700 | 23400 | 22700 | 22700 | nd |
| Sens 6 | 13% | 33% | 15% | 12% | 18% | 11% | 7% | 9% | nd |
| Spec 6 | 90% | 90% | 90% | 90% | 90% | 90% | 90% | 90% | nd |
| OR Quart | 0.60 | 1.0 | 0 | 1.00 | 3.0 | 0.40 | 1.0 | 2.0 | nd |
| 2 | 0.48 | 1.0 | na | 1.00 | 0.34 | 0.27 | 1.00 | 0.57 | nd |
| p Value | 0.14 | 0.062 | na | 0.25 | 0.31 | 0.076 | 0.20 | 0.18 | nd |
| 95% CI of OR Quart2 | 2.5 | 16 | na | 4.0 | 29 | 2.1 | 5.0 | 22 | nd |
| OR Quart | 1.2 | 4.0 | 0.50 | 2.0 | 1.00 | 1.2 | 0.67 | 4.0 | nd |
| 3 | 0.76 | 0.21 | 0.42 | 0.26 | 1.00 | 0.76 | 0.66 | 0.21 | nd |
| p Value | 0.36 | 0.45 | 0.091 | 0.60 | 0.062 | 0.36 | 0.11 | 0.45 | nd |
| 95% CI of OR Quart3 | 4.0 | 36 | 2.7 | 6.8 | 16 | 4.0 | 4.0 | 36 | nd |
| OR Quart | 1.8 | 3.0 | 1.8 | 2.0 | 6.1 | 1.00 | 2.0 | 4.0 | nd |
| 4 | 0.29 | 0.34 | 0.37 | 0.26 | 0.096 | 1.00 | 0.32 | 0.21 | nd |
| p Value | 0.60 | 0.31 | 0.51 | 0.60 | 0.73 | 0.29 | 0.50 | 0.45 | nd |
| 95% CI of OR Quart4 | 5.5 | 29 | 6.1 | 6.8 | 51 | 3.5 | 8.1 | 36 | nd |

**Angiopoietin-related protein 4**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 12.5 | 52.3 | 12.5 | 17.2 | 12.5 | 8.12 |
| Average | 46.9 | 120 | 46.9 | 98.0 | 46.9 | 135 |
| Stdev | 108 | 178 | 108 | 141 | 108 | 289 |
| p(t-test) |  | 0.0066 |  | 0.033 |  | 0.013 |
| Min | 0.000466 | 3.67 | 0.000466 | 2.98 | 0.000466 | 0.794 |
| Max | 1370 | 647 | 1370 | 400 | 1370 | 878 |
| n (Samp) | 1276 | 17 | 1276 | 21 | 1276 | 10 |
| n (Patient) | 400 | 17 | 400 | 21 | 400 | 10 |

| sCr only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 12.6 | 191 | 12.6 | 18.2 | 12.6 | 17.4 |
| Average | 47.7 | 205 | 47.7 | 103 | 47.7 | 169 |

(continued)

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Stdev | 108 | 221 | 108 | 147 | 108 | 317 |
| p(t-test) | | 1.3E-4 | | 0.092 | | 0.0019 |
| Min | 0.000466 | 18.6 | 0.000466 | 2.98 | 0.000466 | 2.66 |
| Max | 1370 | 647 | 1370 | 413 | 1370 | 878 |
| n (Samp) | 1324 | 7 | 1324 | 11 | 1324 | 8 |
| n (Patient) | 414 | 7 | 414 | 11 | 414 | 8 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 12.7 | 16.8 | 12.7 | 12.9 | nd | nd |
| Average | 48.9 | 33.5 | 48.9 | 91.1 | nd | nd |
| Stdev | 111 | 28.2 | 111 | 150 | nd | nd |
| p(t-test) | | 0.68 | | 0.15 | nd | nd |
| Min | 0.000466 | 3.16 | 0.000466 | 0.000466 | nd | nd |
| Max | 1370 | 83.3 | 1370 | 400 | nd | nd |
| n (Samp) | 1331 | 9 | 1331 | 15 | nd | nd |
| n (Patient) | 397 | 9 | 397 | 15 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.68 | 0.85 | 0.60 | 0.63 | 0.66 | 0.52 | 0.44 | 0.57 | nd |
| SE | 0.072 | 0.091 | 0.10 | 0.066 | 0.090 | 0.076 | 0.095 | 0.11 | nd |
| p | 0.013 | 1.3E-4 | 0.32 | 0.048 | 0.072 | 0.74 | 0.50 | 0.51 | nd |
| nCohort 1 | 1276 | 1324 | 1331 | 1276 | 1324 | 1331 | 1276 | 1324 | nd |
| nCohort 2 | 17 | 7 | 9 | 21 | 11 | 15 | 10 | 8 | nd |
| Cutoff 1 | 16.2 | 56.8 | 11.2 | 14.0 | 17.2 | 7.57 | 5.34 | 9.32 | nd |
| Sens 1 | 71% | 71% | 78% | 71% | 73% | 73% | 70% | 75% | nd |
| Spec 1 | 59% | 83% | 44% | 53% | 60% | 31% | 21% | 39% | nd |
| Cutoff 2 | 8.64 | 23.3 | 8.64 | 11.2 | 14.1 | 5.81 | 4.48 | 6.86 | nd |
| Sens 2 | 82% | 86% | 89% | 81% | 82% | 80% | 80% | 88% | nd |
| Spec 2 | 37% | 67% | 36% | 45% | 53% | 23% | 17% | 28% | nd |
| Cutoff 3 | 6.65 | 18.5 | 3.15 | 5.81 | 14.0 | 2.97 | 2.29 | 2.62 | nd |
| Sens 3 | 94% | 100% | 100% | 90% | 91% | 93% | 90% | 100% | nd |

(continued)

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| Spec 3 | 27% | 62% | 9% | 23% | 53% | 9% | 6% | 7% | nd |
| Cutoff 4 | 24.6 | 25.5 | 24.7 | 24.6 | 25.5 | 24.7 | 24.6 | 25.5 | nd |
| Sens 4 | 53% | 71% | 44% | 38% | 36% | 33% | 20% | 25% | nd |
| Spec 4 | 70% | 70% | 70% | 70% | 70% | 70% | 70% | 70% | nd |
| Cutoff 5 | 44.7 | 47.0 | 46.0 | 44.7 | 47.0 | 46.0 | 44.7 | 47.0 | nd |
| Sens 5 | 53% | 71% | 44% | 33% | 36% | 27% | 20% | 25% | nd |
| Spec 5 | 80% | 80% | 80% | 80% | 80% | 80% | 80% | 80% | nd |
| Cutoff 6 | 118 | 125 | 130 | 118 | 125 | 130 | 118 | 125 | nd |
| Sens 6 | 29% | 57% | 0% | 24% | 27% | 20% | 20% | 25% | nd |
| Spec 6 | 90% | 90% | 90% | 90% | 90% | 90% | 90% | 90% | nd |
| OR Quart | 4.0 | >0 | 2.0 | 0.66 | 0 | 0.75 | 1.0 | 2.0 | nd |
| 2 | 0.21 | <na | 0.57 | 0.66 | na | 0.70 | 1.00 | 0.57 | nd |
| p Value | 0.45 | >na | 0.18 | 0.11 | na | 0.17 | 0.14 | 0.18 | nd |
| 95% CI of OR Quart2 | 36 | na | 22 | 4.0 | na | 3.4 | 7.2 | 22 | nd |
| OR Quart | 3.0 | >2.0 | 2.0 | 2.7 | 6.1 | 0.75 | 1.0 | 3.0 | nd |
| 3 | 0.34 | <0.57 | 0.57 | 0.14 | 0.096 | 0.70 | 1.0 | 0.34 | nd |
| p Value | 0.31 | >0.18 | 0.18 | 0.71 | 0.73 | 0.17 | 0.14 | 0.31 | nd |
| 95% CI of OR Quart3 | 29 | na | 22 | 10 | 51 | 3.4 | 7.1 | 29 | nd |
| OR Quart | 9.2 | >5.1 | 4.0 | 2.7 | 4.0 | 1.2 | 2.0 | 2.0 | nd |
| 4 | 3.036 | <0.14 | 0.21 | 0.15 | 0.21 | 0.74 | 0.42 | 0.57 | nd |
| p Value | 1.2 | >0.59 | 0.45 | 0.71 | 0.45 | 0.33 | 0.37 | 0.18 | nd |
| 95% CI of OR Quart4 | 73 | na | 36 | 10 | 36 | 4.7 | 11 | 22 | nd |

**Amphiregulin**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 24.5 | 61.0 | 24.5 | 49.9 | 24.5 | 40.5 |
| Average | 55.8 | 106 | 55.8 | 473 | 55.8 | 45.4 |

(continued)

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Stdev | 142 | 112 | 142 | 969 | 142 | 38.3 |
| p(t-test) | | 0.16 | | 9.9E-19 | | 0.82 |
| Min | 0.00131 | 9.94 | 0.00131 | 17.6 | 0.00131 | 5.13 |
| Max | 1650 | 364 | 1650 | 3480 | 1650 | 124 |
| n (Samp) | 884 | 16 | 884 | 18 | 884 | 10 |
| n (Patient) | 367 | 16 | 367 | 18 | 367 | 10 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | 25.1 | 44.5 | 25.1 | 72.7 |
| Average | nd | nd | 63.2 | 286 | 63.2 | 104 |
| Stdev | nd | nd | 195 | 579 | 195 | 71.9 |
| p(t-test) | nd | nd | | 0.0018 | | 0.58 |
| Min | nd | nd | 0.00131 | 29.8 | 0.00131 | 25.9 |
| Max | nd | nd | 3480 | 1710 | 3480 | 246 |
| n (Samp) | nd | nd | 916 | 8 | 916 | 7 |
| n (Patient) | nd | nd | 380 | 8 | 380 | 7 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | (Cohort 2 | Cohort 1 | Cohort 2 |
| Median | 25.6 | 36.4 | 25.6 | 45.7 | nd | nd |
| Average | 56.5 | 70.0 | 56.5 | 468 | nd | nd |
| Stdev | 145 | 71.3 | 145 | 1040 | nd | nd |
| p(t-test) | | 0.77 | | 3.4E-15 | nd | nd |
| Min | 0.00131 | 10.0 | 0.00131 | 10.1 | nd | nd |
| Max | 1710 | 229 | 1710 | 3480 | nd | nd |
| n (Samp) | 879 | 10 | 879 | 14 | nd | nd |
| n (Patient) | 342 | 10 | 342 | 14 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.69 | nd | 0.63 | 0.74 | 0.76 | 0.68 | 0.56 | 0.83 | nd |
| SE | 0.074 | nd | 0.095 | 0.068 | 0.099 | 0.080 | 0.095 | 0.095 | nd |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| p | 0.010 | nd | 0.17 | 4.5E-4 | 0.0075 | 0.022 | 0.50 | 4.8E-4 | nd |
| nCohort 1 | 884 | nd | 879 | 884 | 916 | 879 | 884 | 916 | nd |
| nCohort 2 | 16 | nd | 10 | 18 | 8 | 14 | 10 | 7 | nd |
| Cutoff 1 | 20.2 | nd | 20.2 | 33.8 | 40.8 | 23.2 | 19.3 | 67.3 | nd |
| Sens 1 | 75% | nd | 70% | 72% | 75% | 71% | 70% | 71% | nd |
| Spec 1 | 44% | nd | 42% | 62% | 66% | 46% | 41% | 83% | nd |
| Cutoff 2 | 19.3 | nd | 19.3 | 23.2 | 33.8 | 20.9 | 10.6 | 65.4 | nd |
| Sens 2 | 81% | nd | 80% | 83% | 88% | 86% | 80% | 86% | nd |
| Spec 2 | 41% | nd | 40% | 48% | 61% | 43% | 21% | 82% | nd |
| Cutoff 3 | 12.6 | nd | 16.5 | 20.9 | 29.8 | 18.9 | 7.58 | 25.8 | nd |
| Sens 3 | 94% | nd | 90% | 94% | 100% | 93% | 90% | 100% | nd |
| Spec 3 | 26% | nd | 35% | 45% | 56% | 39% | 14% | 51% | nd |
| Cutoff 4 | 43.9 | nd | 45.3 | 43.9 | 45.6 | 45.3 | 43.9 | 45.6 | nd |
| Sens 4 | 56% | nd | 40% | 56% | 50% | 50% | 50% | 86% | nd |
| Spec 4 | 70% | nd | 70% | 70% | 70% | 70% | 70% | 70% | nd |
| Cutoff 5 | 57.2 | nd | 57.7 | 57.2 | 58.7 | 57.7 | 57.2 | 58.7 | nd |
| Sens 5 | 50% | nd | 40% | 44% | 38% | 43% | 40% | 86% | nd |
| Spec 5 | 80% | nd | 80% | 80% | 80% | 80% | 80% | 80% | nd |
| Cutoff 6 | 96.9 | nd | 96.9 | 96.9 | 104 | 96.9 | 96.9 | 104 | nd |
| Sens 6 | 31% | nd | 20% | 33% | 38% | 29% | 10% | 43% | nd |
| Spec 6 | 90% | nd | 90% | 90% | 90% | 90% | 90% | 90% | nd |
| OR Quart 2 | 4.1 | nd | 3.0 | >5.1 | >0 | 4.1 | 0.33 | >0 | nd |
| | 0.21 | nd | 0.34 | <0.14 | <na | 0.21 | 0.34 | <na | nd |
| p Value | 0.45 | nd | 0.31 | >0.59 | >na | 0.45 | 0.034 | >na | nd |
| 95% CI of OR Quart2 | 37 | nd | 29 | na | na | 37 | 3.2 | na | nd |
| OR Quart 3 | 3.0 | nd | 2.0 | >4.1 | >5.1 | 2.0 | 0.33 | >1.0 | nd |
| | 0.34 | nd | 0.57 | <0.21 | <0.14 | 0.57 | 0.34 | <1.0 | nd |
| p Value | 0.31 | nd | 0.18 | >0.45 | >0.59 | 0.18 | 0.034 | >0.062 | nd |
| 95% CI of OR Quart3 | 29 | nd | 22 | na | na | 22 | 3.2 | na | nd |
| OR Quart | 8.3 | nd | 4.0 | >9.3 | >3.0 | 7.2 | 1.7 | >6.1 | nd |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| 4 | 0.047 | nd | 0.21 | <0.035 | <0.34 | 0.067 | 0.48 | <0.094 | nd |
| p Value | 1.0 | nd | 0.45 | >1.2 | >0.31 | 0.87 | 0.40 | >0.73 | nd |
| 95% CI of OR Quart4 | 67 | nd | 36 | na | na | 59 | 7.1 | na | nd |

**Betacellulin**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.761 | 2.00 | 0.761 | 1.56 | 0.761 | 1.47 |
| Average | 1.38 | 2.22 | 1.38 | 1.91 | 1.38 | 1.12 |
| Stdev | 2.23 | 1.03 | 2.23 | 2.67 | 2.23 | 0.804 |
| p(t-test) | | 0.13 | | 0.32 | | 0.72 |
| Min | 0.00179 | 0.00230 | 0.00179 | 0.00240 | 0.00179 | 0.00240 |
| Max | 28.3 | 3.96 | 28.3 | 11.6 | 28.3 | 1.94 |
| n (Samp) | 884 | 16 | 884 | 18 | 884 | 10 |
| n (Patient) | 367 | 16 | 367 | 18 | 367 | 10 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | 0.761 | 1.57 | 0.761 | 1.43 |
| Average | nd | nd | 1.41 | 1.73 | 1.41 | 1.33 |
| Stdev | nd | nd | 2.25 | 1.06 | 2.25 | 1.59 |
| p(t-test) | nd | nd | | 0.70 | | 0.93 |
| Min | nd | nd | 0.00179 | 0.0742 | 0.00179 | 0.00246 |
| Max | nd | nd | 28.3 | 3.65 | 28.3 | 4.42 |
| n (Samp) | nd | nd | 916 | 8 | 916 | 7 |
| n (Patient) | nd | nd | 380 | 8 | 380 | 7 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.761 | 2.09 | 0.761 | 1.56 | nd | nd |
| Average | 1.37 | 2.33 | 1.37 | 2.05 | nd | nd |

(continued)

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Stdev | 2.12 | 0.816 | 2.12 | 3.05 | nd | nd |
| p(t-test) | | 0.15 | | 0.23 | nd | nd |
| Min | 0.00179 | 1.30 | 0.00179 | 0.00240 | nd | nd |
| Max | 28.3 | 3.96 | 28.3 | 11.6 | nd | nd |
| n (Samp) | 879 | 10 | 879 | 14 | nd | nd |
| n (Patient) | 342 | 10 | 342 | 14 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.72 | nd | 0.77 | 0.59 | 0.65 | 0.57 | 0.47 | 0.47 | nd |
| SE | 0.073 | nd | 0.088 | 0.071 | 0.11 | 0.080 | 0.093 | 0.11 | nd |
| p | 0.0021 | nd | 0.0024 | 0.20 | 0.16 | 0.35 | 0.79 | 0.79 | nd |
| nCohort 1 | 884 | nd | 879 | 884 | 916 | 879 | 884 | 916 | nd |
| nCohort 2 | 16 | nd | 10 | 18 | 8 | 14 | 10 | 7 | nd |
| Cutoff 1 | 1.74 | nd | 1.94 | 0.135 | 1.51 | 0.108 | 1.10 | 0.0209 | nd |
| Sens 1 | 75% | nd | 70% | 72% | 75% | 71% | 70% | 71% | nd |
| Spec 1 | 69% | nd | 71% | 41% | 60% | 40% | 55% | 25% | nd |
| Cutoff 2 | 1.59 | nd | 1.74 | 0.0522 | 0.900 | 0.0209 | 0.00240 | 0.00240 | nd |
| Sens 2 | 81% | nd | 80% | 89% | 88% | 86% | 90% | 100% | nd |
| Spec 2 | 66% | nd | 69% | 34% | 51% | 27% | 7% | 7% | nd |
| Cutoff 3 | 1.10 | nd | 1.59 | 0.0209 | 0.0522 | 0.00342 | 0.00240 | 0.00240 | nd |
| Sens 3 | 94% | nd | 90% | 94% | 100% | 93% | 90% | 100% | nd |
| Spec 3 | 55% | nd | 66% | 26% | 33% | 20% | 7% | 7% | nd |
| Cutoff 4 | 1.87 | nd | 1.87 | 1.87 | 1.87 | 1.87 | 1.87 | 1.87 | nd |
| Sens 4 | 62% | nd | 70% | 33% | 38% | 43% | 10% | 14% | nd |
| Spec 4 | 71% | nd | 71% | 71% | 70% | 71% | 71% | 70% | nd |
| Cutoff 5 | 2.41 | nd | 2.41 | 2.41 | 2.42 | 2.41 | 2.41 | 2.42 | nd |
| Sens 5 | 38% | nd | 40% | 22% | 25% | 29% | 0% | 14% | nd |
| Spec 5 | 80% | nd | 80% | 80% | 81% | 80% | 80% | 81% | nd |
| Cutoff 6 | 3.36 | nd | 3.36 | 3.36 | 3.36 | 3.36 | 3.36 | 3.36 | nd |
| Sens 6 | 12% | nd | 10% | 11% | 12% | 14% | 0% | 14% | nd |
| Spec 6 | 91% | nd | 91% | 91% | 91% | 91% | 91% | 91% | nd |
| OR Quart | 0 | nd | >0 | 5.1 | >1.0 | 2.0 | >7.3 | 3.0 | nd |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| 2 | na | nd | <na | 0.14 | <1.00 | 0.42 | <0.065 | 0.34 | nd |
| p Value | na | nd | >na | 0.59 | >0.062 | 0.37 | >0.89 | 0.31 | nd |
| 95% CI of OR Quart2 | na | nd | na | 44 | na | 11 | na | 29 | nd |
| OR Quart | 9.3 | nd | >5.1 | 8.3 | >5.1 | 2.0 | >0 | 1.0 | nd |
| 3 | 0.035 | nd | <0.14 | 0.047 | <0.14 | 0.42 | <na | 1.0 | nd |
| p Value | 1.2 | nd | >0.59 | 1.0 | >0.59 | 0.37 | >na | 0.062 | nd |
| 95% CI of OR Quart3 | 74 | nd | na | 67 | na | 11 | na | 16 | nd |
| OR Quart | 6.1 | nd | >5.1 | 4.0 | >2.0 | 2.0 | >3.1 | 2.0 | nd |
| 4 | 0.094 | nd | <0.14 | 0.21 | <0.57 | 0.42 | <0.34 | 0.57 | nd |
| p Value | 0.73 | nd | >0.59 | 0.45 | >0.18 | 0.36 | >0.32 | 0.18 | nd |
| 95% CI of OR Quart4 | 51 | nd | na | 36 | na | 11 | na | 22 | nd |

**Endostatin**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 5660 | 17000 | 5660 | 9540 | 5660 | 3750 |
| Average | 17000 | 44500 | 17000 | 25900 | 17000 | 20200 |
| Stdev | 29300 | 65600 | 29300 | 34200 | 29300 | 39600 |
| p(t-test) | | 1.5E-5 | | 0.14 | | 0.69 |
| Min | 0.0130 | 932 | 0.0130 | 1110 | 0.0130 | 261 |
| Max | 238000 | 227000 | 238000 | 148000 | 238000 | 149000 |
| n (Samp) | 1483 | 23 | 1483 | 24 | 1483 | 14 |
| n (Patient) | 493 | 23 | 493 | 24 | 493 | 14 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 5760 | 17000 | 5760 | 8130 | 5760 | 19300 |
| Average | 17200 | 56500 | 17200 | 27500 | 17200 | 36000 |

(continued)

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Stdev | 29600 | 81800 | 29600 | 35200 | 29600 | 47400 |
| p(t-test) | | 9.7E-5 | | 0.25 | | 0.037 |
| Min | 0.0130 | 1980 | 0.0130 | 2050 | 0.0130 | 1790 |
| Max | 238000 | 227000 | 238000 | 110000 | 238000 | 149000 |
| n (Samp) | 1540 | 9 | 1540 | 11 | 1540 | 11 |
| n (Patient) | 509 | 9 | 509 | 11 | 509 | 11 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 5760 | 16700 | 5760 | 9520 | nd | nd |
| Average | 17500 | 42400 | 17500 | 24900 | nd | nd |
| Stdev | 30400 | 63700 | 30400 | 37100 | nd | nd |
| p(t-test) | | 0.0038 | | 0.31 | nd | nd |
| Min | 0.0130 | 932 | 0.0130 | 1110 | nd | nd |
| Max | 267000 | 190000 | 267000 | 148000 | nd | nd |
| n (Samp) | 1513 | 13 | 1513 | 18 | nd | nd |
| n (Patient) | 472 | 13 | 472 | 18 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.62 | 0.68 | 0.61 | 0.61 | 0.61 | 0.57 | 0.46 | 0.66 | nd |
| SE | 0.063 | 0.099 | 0.084 | 0.062 | 0.091 | 0.071 | 0.079 | 0.090 | nd |
| p | 0.050 | 0.078 | 0.17 | 0.063 | 0.22 | 0.30 | 0.59 | 0.069 | nd |
| nCohort 1 | 1483 | 1540 | 1513 | 1483 | 1540 | 1513 | 1483 | 1540 | nd |
| nCohort 2 | 23 | 9 | 13 | 24 | 11 | 18 | 14 | 11 | nd |
| Cutoff 1 | 4710 | 4940 | 4710 | 4640 | 4410 | 4410 | 3180 | 9560 | nd |
| Sens 1 | 74% | 78% | 77% | 71% | 73% | 72% | 71% | 73% | nd |
| Spec 1 | 43% | 45% | 43% | 43% | 41% | 41% | 29% | 66% | nd |
| Cutoff 2 | 2720 | 3120 | 2150 | 3560 | 4190 | 2970 | 1420 | 3250 | nd |
| Sens 2 | 83% | 89% | 85% | 83% | 82% | 83% | 86% | 82% | nd |
| Spec 2 | 25% | 28% | 17% | 33% | 39% | 26% | 9% | 30% | nd |
| Cutoff 3 | 2010 | 1960 | 2010 | 2950 | 2970 | 1410 | 1290 | 3180 | nd |
| Sens 3 | 91% | 100% | 92% | 92% | 91% | 94% | 93% | 91% | nd |
| Spec 3 | 16% | 16% | 15% | 27% | 27% | 8% | 8% | 29% | nd |
| Cutoff 4 | 11600 | 12000 | 12000 | 11600 | 12000 | 12000 | 11600 | 12000 | nd |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Sens 4 | 52% | 67% | 54% | 42% | 36% | 39% | 29% | 55% | nd |
| Spec 4 | 70% | 70% | 70% | 70% | 70% | 70% | 70% | 70% | nd |
| Cutoff 5 | 22600 | 22900 | 23700 | 22600 | 22900 | 23700 | 22600 | 22900 | nd |
| Sens 5 | 43% | 44% | 38% | 33% | 36% | 28% | 21% | 36% | nd |
| Spec 5 | 80% | 80% | 80% | 80% | 80% | 80% | 80% | 80% | nd |
| Cutoff 6 | 47200 | 47900 | 48800 | 47200 | 47900 | 48800 | 47200 | 47900 | nd |
| Sens 6 | 26% | 33% | 23% | 25% | 27% | 22% | 14% | 27% | nd |
| Spec 6 | 90% | 90% | 90% | 90% | 90% | 90% | 90% | 90% | nd |
| OR Quart | 0.80 | 2.0 | 0.33 | 4.1 | 4.0 | 1.7 | 0.50 | 2.0 | nd |
| 2 | 0.73 | 0.57 | 0.34 | 0.078 | 0.21 | 0.48 | 0.42 | 0.57 | nd |
| p Value | 0.21 | 0.18 | 0.034 | 0.86 | 0.45 | 0.40 | 0.091 | 0.18 | nd |
| 95% CI of OR Quart2 | 3.0 | 22 | 3.2 | 19 | 36 | 7.0 | 2.7 | 22 | nd |
| OR Quart | 0.40 | 1.0 | 0.66 | 2.5 | 2.0 | 1.3 | 1.0 | 2.0 | nd |
| 3 | 0.27 | 1.0 | 0.66 | 0.27 | 0.57 | 0.71 | 1.00 | 0.57 | nd |
| p Value | 0.076 | 0.062 | 0.11 | 0.48 | 0.18 | 0.30 | 0.25 | 0.18 | nd |
| 95% CI of OR Quart3 | 2.1 | 16 | 4.0 | 13 | 22 | 6.0 | 4.0 | 22 | nd |
| OR Quart | 2.4 | 5.0 | 2.4 | 4.6 | 4.0 | 2.0 | 1.0 | 6.1 | nd |
| 4 | 0.097 | 0.14 | 0.22 | 0.053 | 0.21 | 0.33 | 1.00 | 0.096 | nd |
| p Value | 0.85 | 0.59 | 0.60 | 0.98 | 0.45 | 0.50 | 0.25 | 0.73 | nd |
| 95% CI of OR Quart4 | 7.0 | 43 | 9.2 | 21 | 36 | 8.1 | 4.0 | 51 | nd |

**Proepiregulin**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 3.39 | 4.55 | 3.39 | 6.87 | 3.39 | 3.30 |
| Average | 6.14 | 9.42 | 6.14 | 14.7 | 6.14 | 4.98 |
| Stdev | 12.6 | 12.5 | 12.6 | 21.8 | 12.6 | 5.03 |
| p(t-test) | | 0.30 | | 0.0069 | | 0.77 |
| Min | 0.000104 | 1.42 | 0.000104 | 0.758 | 0.000104 | 0.376 |
| Max | 279 | 49.4 | 279 | 81.9 | 279 | 15.2 |
| n (Samp) | 854 | 16 | 854 | 17 | 854 | 10 |
| n (Patient) | 364 | 16 | 364 | 17 | 364 | 10 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | 3.46 | 6.47 | 3.46 | 6.79 |
| Average | nd | nd | 6.30 | 13.5 | 6.30 | 7.21 |
| Stdev | nd | nd | 12.8 | 17.7 | 12.8 | 4.19 |
| p(t-test) | nd | nd | | 0.11 | | 0.85 |
| Min | nd | nd | 0.000104 | 5.17 | 0.000104 | 2.96 |
| Max | nd | nd | 279 | 57.1 | 279 | 15.2 |
| n (Samp) | nd | nd | 885 | 8 | 885 | 7 |
| n (Patient) | nd | nd | 377 | 8 | 377 | 7 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 3.52 | 3.45 | 3.52 | 7.39 | nd | nd |
| Average | 6.37 | 5.92 | 6.37 | 13.2 | nd | nd |
| Stdev | 12.8 | 7.42 | 12.8 | 21.6 | nd | nd |
| p(t-test) | | 0.91 | | 0.061 | nd | nd |
| Min | 0.000104 | 1.79 | 0.000104 | 0.758 | nd | nd |
| Max | 279 | 26.4 | 279 | 81.9 | nd | nd |
| n (Samp) | 849 | 10 | 849 | 13 | nd | nd |
| n (Patient) | 339 | 10 | 339 | 13 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.62 | nd | 0.52 | 0.67 | 0.77 | 0.61 | 0.47 | 0.70 | nd |
| SE | 0.076 | nd | 0.093 | 0.073 | 0.098 | 0.084 | 0.094 | 0.11 | nd |
| p | 0.099 | nd | 0.79 | 0.017 | 0.0050 | 0.20 | 0.73 | 0.078 | nd |
| nCohort 1 | 854 | nd | 849 | 854 | 885 | 849 | 854 | 885 | nd |
| nCohort 2 | 16 | nd | 10 | 17 | 8 | 13 | 10 | 7 | nd |
| Cutoff 1 | 3.18 | nd | 2.37 | 5.16 | 5.96 | 1.95 | 1.37 | 4.73 | nd |
| Sens 1 | 75% | nd | 70% | 71% | 75% | 77% | 70% | 71% | nd |
| Spec 1 | 47% | nd | 35% | 66% | 70% | 30% | 21% | 63% | nd |
| Cutoff 2 | 2.22 | nd | 2.22 | 1.95 | 5.42 | 1.45 | 0.806 | 3.74 | nd |
| Sens 2 | 81% | nd | 80% | 82% | 88% | 85% | 80% | 86% | nd |
| Spec 2 | 35% | nd | 33% | 32% | 67% | 21% | 12% | 55% | nd |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Cutoff 3 | 1.78 | nd | 2.05 | 0.944 | 5.16 | 0.944 | 0.493 | 2.95 | nd |
| Sens 3 | 94% | nd | 90% | 94% | 100% | 92% | 90% | 100% | nd |
| Spec 3 | 29% | nd | 31% | 14% | 66% | 12% | 6% | 44% | nd |
| Cutoff 4 | 5.74 | nd | 6.02 | 5.74 | 5.90 | 6.02 | 5.74 | 5.90 | nd |
| Sens 4 | 44% | nd | 30% | 59% | 75% | 54% | 40% | 57% | nd |
| Spec 4 | 70% | nd | 70% | 70% | 70% | 70% | 70% | 70% | nd |
| Cutoff 5 | 8.36 | nd | 8.53 | 8.36 | 8.47 | 8.53 | 8.36 | 8.47 | nd |
| Sens 5 | 25% | nd | 10% | 41% | 38% | 46% | 20% | 29% | nd |
| Spec 5 | 80% | nd | 80% | 80% | 80% | 80% | 80% | 80% | nd |
| Cutoff 6 | 13.0 | nd | 13.4 | 13.0 | 13.4 | 13.4 | 13.0 | 13.4 | nd |
| Sens 6 | 19% | nd | 10% | 24% | 12% | 23% | 10% | 14% | nd |
| Spec 6 | 90% | nd | 90% | 90% | 90% | 90% | 90% | 90% | nd |
| OR Quart | 4.0 | nd | >6.1 | 0.33 | >0 | 0.66 | 0.66 | >1.0 | nd |
| 2 | 0.21 | nd | <0.094 | 0.34 | <na | 0.65 | 0.66 | <1.00 | nd |
| p Value | 0.45 | nd | >0.73 | 0.034 | >na | 0.11 | 0.11 | >0.062 | nd |
| 95% CI of OR Quart2 | 36 | nd | na | 3.2 | na | 4.0 | 4.0 | na | nd |
| OR Quart | 6.1 | nd | >2.0 | 1.7 | >5.1 | 0.33 | 0.33 | >3.0 | nd |
| 3 | 0.094 | nd | <0.57 | 0.48 | <0.14 | 0.34 | 0.34 | <0.34 | nd |
| p Value | 0.73 | nd | >0.18 | 0.40 | >0.59 | 0.034 | 0.034 | >0.31 | nd |
| 95% CI of OR Quart3 | 51 | nd | na | 7.1 | na | 3.2 | 3.2 | na | nd |
| OR Quart | 5.1 | nd | >2.0 | 2.7 | >3.0 | 2.4 | 1.3 | >3.0 | nd |
| 4 | 0.14 | nd | <0.57 | 0.14 | <0.34 | 0.22 | 0.70 | <0.34 | nd |
| p Value | 0.59 | nd | >0.18 | 0.71 | >0.31 | 0.60 | 0.30 | >0.31 | nd |
| 95% CI of OR Quart4 | 44 | nd | na | 10 | na | 9.3 | 6.1 | na | nd |

**Heparin-binding growth factor 1**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 848 | 289 | 848 | 549 | 848 | 429 |
| Average | 1200 | 488 | 1200 | 967 | 1200 | 582 |
| Stdev | 1420 | 551 | 1420 | 1200 | 1420 | 595 |
| p(t-test) | | 0.016 | | 0.43 | | 0.10 |
| Min | 0.00328 | 55.5 | 0.00328 | 8.50 | 0.00328 | 35.1 |
| Max | 16700 | 2430 | 16700 | 5020 | 16700 | 1910 |
| n (Samp) | 1482 | 23 | 1482 | 23 | 1482 | 14 |
| n (Patient) | 493 | 23 | 493 | 23 | 493 | 14 |

| sCr only | 'Ohr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 834 | 189 | 834 | 494 | 834 | 194 |
| Average | 1180 | 335 | 1180 | 624 | 1180 | 433 |
| Stdev | 1400 | 447 | 1400 | 504 | 1400 | 506 |
| p(t-test) | | 0.070 | | 0.21 | | 0.076 |
| Min | 0.00328 | 55.5 | 0.00328 | 90.5 | 0.00328 | 32.9 |
| Max | 16700 | 1470 | 16700 | 1650 | 16700 | 1500 |
| n (Samp) | 1539 | 9 | 1539 | 10 | 1539 | 11 |
| n (Patient) | 509 | 9 | 509 | 10 | 509 | 11 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 842 | 320 | 842 | 546 | nd | nd |
| Average | 1200 | 444 | 1200 | 827 | nd | nd |
| Stdev | 1430 | 350 | 1430 | 889 | nd | nd |
| p(t-test) | | 0.056 | | 0.27 | nd | nd |
| Min | 0.00328 | 70.1 | 0.00328 | 8.50 | nd | nd |
| Max | 16700 | 1140 | 16700 | 3420 | nd | nd |
| n (Samp) | 1511 | 13 | 1511 | 18 | nd | nd |
| n (Patient) | 472 | 13 | 472 | 18 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.28 | 0.20 | 0.28 | 0.42 | 0.37 | 0.40 | 0.32 | 0.26 | nd |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| SE | 0.061 | 0.089 | 0.081 | 0.063 | 0.095 | 0.071 | 0.080 | 0.086 | nd |
| p | 2.1E-4 | 9.2E-4 | 0.0059 | 0.19 | 0.16 | 0.17 | 0.026 | 0.0045 | nd |
| nCohort 1 | 1482 | 1539 | 1511 | 1482 | 1539 | 1511 | 1482 | 1539 | nd |
| nCohort 2 | 23 | 9 | 13 | 23 | 10 | 18 | 14 | 11 | nd |
| Cutoff 1 | 155 | 110 | 155 | 364 | 449 | 364 | 125 | 77.7 | nd |
| Sens 1 | 74% | 78% | 77% | 74% | 70% | 72% | 71% | 73% | nd |
| Spec 1 | 11% | 8% | 11% | 24% | 30% | 25% | 9% | 6% | nd |
| Cutoff 2 | 145 | 69.8 | 145 | 119 | 247 | 119 | 61.5 | 61.5 | nd |
| Sens 2 | 83% | 89% | 85% | 83% | 80% | 83% | 86% | 82% | nd |
| Spec 2 | 11% | 5% | 11% | 9% | 17% | 9% | 5% | 5% | nd |
| Cutoff 3 | 110 | 55.2 | 119 | 89.7 | 91.2 | 75.4 | 51.2 | 34.6 | nd |
| Sens 3 | 91% | 100% | 92% | 91% | 90% | 94% | 93% | 91% | nd |
| Spec 3 | 8% | 5% | 9% | 7% | 7% | 6% | 4% | 3% | nd |
| Cutoff 4 | 1380 | 1340 | 1360 | 1380 | 1340 | 1360 | 1380 | 1340 | nd |
| Sens 4 | 9% | 11% | 0% | 13% | 10% | 11% | 14% | 9% | nd |
| Spec 4 | 70% | 70% | 70% | 70% | 70% | 70% | 70% | 70% | nd |
| Cutoff 5 | 1730 | 1710 | 1730 | 1730 | 1710 | 1730 | 1730 | 1710 | nd |
| Sens 5 | 4% | 0% | 0% | 13% | 0% | 11% | 7% | 0% | nd |
| Spec 5 | 80% | 80% | 80% | 80% | 80% | 80% | 80% | 80% | nd |
| Cutoff 6 | 2450 | 2430 | 2460 | 2450 | 2430 | 2460 | 2450 | 2430 | nd |
| Sens 6 | 0% | 0% | 0% | 13% | 0% | 11% | 0% | 0% | nd |
| Spec 6 | 90% | 90% | 90% | 90% | 90% | 90% | 90% | 90% | nd |
| OR Quart 2 | 3.0 | >1.0 | >3.0 | 2.0 | 2.0 | 2.5 | 3.0 | >2.0 | nd |
| | 0.34 | <1.00 | <0.34 | 0.32 | 0.57 | 0.27 | 0.34 | <0.57 | nd |
| p Value | 0.31 | >0.062 | >0.31 | 0.50 | 0.18 | 0.49 | 0.31 | >0.18 | nd |
| 95% CI of OR Quart2 | 29 | na | na | 8.1 | 22 | 13 | 29 | na | nd |
| OR Quart 3 | 5.1 | >1.0 | >2.0 | 2.4 | 4.0 | 2.5 | 3.0 | >2.0 | nd |
| | 0.14 | <1.00 | <0.57 | 0.21 | 0.21 | 0.27 | 0.34 | <0.57 | nd |
| p Value | 0.59 | >0.062 | >0.18 | 0.61 | 0.45 | 0.49 | 0.31 | >0.18 | nd |
| 95% CI of OR Quart3 | 44 | na | na | 9.2 | 36 | 13 | 29 | na | nd |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| OR Quart 4 | 15 | >7.1 | >8.2 | 2.4 | 3.0 | 3.0 | 7.1 | >7.1 | nd |
| | 0.0099 | <0.067 | <0.048 | 0.21 | 0.34 | 0.17 | 0.067 | <0.066 | nd |
| p Value | 1.9 | >0.87 | >1.0 | 0.61 | 0.31 | 0.61 | 0.87 | >0.88 | nd |
| 95% CI of OR Quart4 | 110 | na | na | 9.2 | 29 | 15 | 58 | na | nd |

**Fibroblast growth factor 19**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 2.83E-5 | 2.83E-5 | 2.83E-5 | 0.000168 | 2.83E-5 | 2.63E-5 |
| Average | 0.00494 | 0.0778 | 0.00494 | 0.0504 | 0.00494 | 0.00105 |
| Stdev | 0.0336 | 0.249 | 0.0336 | 0.155 | 0.0336 | 0.00323 |
| p(t-test) | | 9.9E-12 | | 9.4E-8 | | 0.71 |
| Min | 5.62E-6 | 5.62E-6 | 5.62E-6 | 1.66E-5 | 5.62E-6 | 2.17E-5 |
| Max | 0.634 | 1.02 | 0.634 | 0.560 | 0.634 | 0.0102 |
| n (Samp) | 1276 | 17 | 1276 | 21 | 1276 | 10 |
| n (Patient) | 400 | 17 | 400 | 21 | 400 | 10 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 2.83E-5 | 0.00600 | 2.83E-5 | 0.000197 | 2.83E-5 | 2.55E-5 |
| Average | 0.00524 | 0.188 | 0.00524 | 0.0539 | 0.00524 | 2.62E-5 |
| Stdev | 0.0354 | 0.377 | 0.0354 | 0.168 | 0.0354 | 3.91E-6 |
| p(t-test) | | 2.0E-27 | | 2.7E-5 | | 0.68 |
| Min | 5.62E-6 | 7.53E-6 | 5.62E-6 | 1.88E-5 | 5.62E-6 | 2.17E-5 |
| Max | 0.634 | 1.02 | 0.634 | 0.560 | 0.634 | 3.21E-5 |
| n (Samp) | 1324 | 7 | 1324 | 11 | 1324 | 8 |
| n (Patient) | 414 | 7 | 414 | 11 | 414 | 8 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 2.83E-5 | 2.83E-5 | 2.83E-5 | 4.39E-5 | nd | nd |

(continued)

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Average | 0.00839 | 0.113 | 0.00839 | 0.0349 | nd | nd |
| Stdev | 0.0779 | 0.340 | 0.0779 | 0.121 | nd | nd |
| p(t-test) | | 1.3E-4 | | 0.19 | nd | nd |
| Min | 5.62E-6 | 1.88E-5 | 5.62E-6 | 1.66E-5 | nd | nd |
| Max | 2.28 | 1.02 | 2.28 | 0.470 | nd | nd |
| n (Samp) | 1331 | 9 | 1331 | 15 | nd | nd |
| UO only | 0hr prior to AKI stage | | 24hr prior to AKI stage | | 48hr prior to AKI | stage |
| | Cohort 1 | Cohort 2 | Cohort 1 | Cohort 2 | Cohort 1 | Cohort 2 |
| n (Patient) | 397 | 9 | 397 | 15 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.55 | 0.61 | 0.54 | 0.66 | 0.66 | 0.62 | 0.47 | 0.41 | nd |
| SE | 0.072 | 0.11 | 0.099 | 0.066 | 0.090 | 0.078 | 0.093 | 0.11 | nd |
| p | 0.52 | 0.34 | 0.69 | 0.015 | 0.075 | 0.12 | 0.77 | 0.37 | nd |
| nCohort 1 | 1276 | 1324 | 1331 | 1276 | 1324 | 1331 | 1276 | 1324 | nd |
| nCohort 2 | 17 | 7 | 9 | 21 | 11 | 15 | 10 | 8 | nd |
| Cutoff 1 | 1.92E-5 | 1.82E-5 | 2.17E-5 | 2.53E-5 | 2.17E-5 | 2.46E-5 | 2.38E-5 | 2.17E-5 | nd |
| Sens 1 | 71% | 71% | 78% | 76% | 73% | 73% | 70% | 75% | nd |
| Spec 1 | 26% | 18% | 30% | 39% | 29% | 36% | 34% | 29% | nd |
| Cutoff 2 | 1.82E-5 | 1.34E-5 | 1.88E-5 | 1.92E-5 | 1.88E-5 | 1.92E-5 | 2.17E-5 | 1.92E-5 | nd |
| Sens 2 | 88% | 86% | 89% | 81% | 82% | 80% | 80% | 100% | nd |
| Spec 2 | 18% | 10% | 22% | 26% | 22% | 26% | 29% | 26% | nd |
| Cutoff 3 | 1.34E-5 | 5.62E-6 | 1.82E-5 | 1.82E-5 | 1.82E-5 | 1.82E-5 | 1.92E-5 | 1.92E-5 | nd |
| Sens 3 | 94% | 100% | 100% | 95% | 100% | 93% | 100% | 100% | nd |
| Spec 3 | 10% | 4% | 18% | 18% | 18% | 18% | 26% | 26% | nd |
| Cutoff 4 | 4.39E-5 | 4.39E-5 | 4.39E-5 | 4.39E-5 | 4.39E-5 | 4.39E-5 | 4.39E-5 | 4.39E-5 | nd |
| Sens 4 | 35% | 57% | 11% | 57% | 64% | 47% | 10% | 0% | nd |
| Spec 4 | 72% | 73% | 73% | 72% | 73% | 73% | 72% | 73% | nd |
| Cutoff 5 Sens 5 | 0.000114 | 0.000114 | 0.000114 | 0.000114 | 0.000114 | 0.00011 4 | 0.000114 | 0.000114 | nd |
| Spec 5 | 35% | 57% | 11% | 52% | 55% | 47% | 10% | 0% | nd |
| | 81% | 81% | 81% | 81% | 81% | 81% | 81% | 81% | nd |
| Cutoff 6 | 0.00364 | 0.00364 | 0.00364 | 0.00364 | 0.00364 | 0.00364 | 0.00364 | 0.00364 | nd |
| Sens 6 | 29% | 57% | 11% | 19% | 36% | 20% | 10% | 0% | nd |
| Spec 6 | 90% | 90% | 90% | 90% | 90% | 90% | 90% | 90% | nd |
| OR Quart | 0.60 | 0 | 1.0 | 0.75 | 0.33 | 1.00 | 3.0 | >3.0 | nd |
| 2 | 0.48 | na | 1.0 | 0.70 | 0.34 | 1.00 | 0.34 | <0.34 | nd |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| p Value | 0.14 | na | 0.14 | 0.17 | 0.034 | 0.20 | 0.31 | >0.31 | nd |
| 95% CI of OR Quart2 | 2.5 | na | 7.1 | 3.4 | 3.2 | 5.0 | 29 | na | nd |
| OR Quart | 0.60 | 0 | 2.0 | 0.50 | 0 | 0.66 | 6.1 | >5.1 | nd |
| 3 | 0.48 | na | 0.42 | 0.42 | na | 0.66 | 0.095 | <0.14 | nd |
| p Value | 0.14 | na | 0.37 | 0.090 | na | 0.11 | 0.73 | >0.59 | nd |
| 95% CI of OR Quart3 | 2.5 | na | 11 | 2.7 | na | 4.0 | 51 | na | nd |
| OR Quart | 1.2 | 1.3 | 0.50 | 3.1 | 2.4 | 2.4 | 0 | >0 | nd |
| 4 | 0.77 | 0.71 | 0.57 | 0.054 | 0.22 | 0.22 | na | <na | nd |
| p Value | 0.36 | 0.30 | 0.045 | 0.98 | 0.60 | 0.60 | na | >na | nd |
| 95% CI of OR Quart4 | 4.0 | 6.0 | 5.5 | 9.6 | 9.2 | 9.2 | na | na | nd |

**Fibroblast growth factor 21**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.0140 | 0.0567 | 0.0140 | 0.0466 | 0.0140 | 0.00852 |
| Average | 0.168 | 0.249 | 0.168 | 0.214 | 0.168 | 0.0619 |
| Stdev | 0.559 | 0.393 | 0.559 | 0.393 | 0.559 | 0.149 |
| p(t-test) | | 0.55 | | 0.71 | | 0.55 |
| Min | 1.14E-9 | 0.00290 | 1.14E-9 | 7.54E-8 | 1.14E-9 | 0.000662 |
| Max | 8.92 | 1.53 | 8.92 | 1.59 | 8.92 | 0.482 |
| n (Samp) | 1276 | 17 | 1276 | 21 | 1276 | 10 |
| n (Patient) | 400 | 17 | 400 | 21 | 400 | 10 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.0145 | 0.318 | 0.0145 | 0.0466 | 0.0145 | 0.0115 |
| Average | 0.174 | 0.434 | 0.174 | 0.0590 | 0.174 | 0.110 |
| Stdev | 0.558 | 0.553 | 0.558 | 0.0659 | 0.558 | 0.185 |
| p(t-test) | | 0.22 | | 0.50 | | 0.75 |
| Min | 1.14E-9 | 0.00620 | 1.14E-9 | 7.54E-8 | 1.14E-9 | 0.00255 |
| Max | 8.92 | 1.53 | 8.92 | 0.206 | 8.92 | 0.482 |
| n (Samp) | 1324 | 7 | 1324 | 11 | 1324 | 8 |
| n (Patient) | 414 | 7 | 414 | 11 | 414 | 8 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.0142 | 0.0809 | 0.0142 | 0.0598 | nd | nd |
| Average | 0.175 | 0.176 | 0.175 | 0.287 | nd | nd |
| Stdev | 0.590 | 0.183 | 0.590 | 0.449 | nd | nd |
| p(t-test) | | 1.00 | | 0.46 | nd | nd |
| Min | 1.14E-9 | 0.00163 | 1.14E-9 | 7.54E-8 | nd | nd |
| Max | 8.92 | 0.442 | 8.92 | 1.59 | nd | nd |
| n (Samp) | 1331 | 9 | 1331 | 15 | nd | nd |
| n (Patient) | 397 | 9 | 397 | 15 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.66 | 0.71 | 0.66 | 0.59 | 0.56 | 0.60 | 0.42 | 0.50 | nd |
| SE | 0.073 | 0.11 | 0.100 | 0.066 | 0.090 | 0.078 | 0.095 | 0.10 | nd |
| p | 0.029 | 0.052 | 0.10 | 0.20 | 0.50 | 0.18 | 0.37 | 1.00 | nd |
| nCohort 1 | 1276 | 1324 | 1331 | 1276 | 1324 | 1331 | 1276 | 1324 | nd |
| nCohort 2 | 17 | 7 | 9 | 21 | 11 | 15 | 10 | 8 | nd |
| Cutoff 1 | 0.0171 | 0.0191 | 0.0172 | 0.00670 | 0.0116 | 0.00670 | 0.00324 | 0.00583 | nd |
| Sens 1 | 71% | 71% | 78% | 71% | 73% | 73% | 70% | 75% | nd |
| Spec 1 | 56% | 57% | 56% | 32% | 43% | 32% | 18% | 28% | nd |
| Cutoff 2 | 0.00618 | 0.0115 | 0.00289 | 0.00514 | 0.00484 | 0.00544 | 0.00254 | 0.00324 | nd |
| Sens 2 | 82% | 86% | 89% | 81% | 82% | 80% | 80% | 88% | nd |
| Spec 2 | 30% | 43% | 17% | 26% | 25% | 27% | 15% | 18% | nd |
| Cutoff 3 | 0.00293 | 0.00618 | 0.00162 | 0.00228 | 0.00108 | 0.00228 | 0.00105 | 0.00254 | nd |
| Sens 3 | 94% | 100% | 100% | 90% | 91% | 93% | 90% | 100% | nd |
| Spec 3 | 17% | 30% | 10% | 14% | 8% | 14% | 8% | 15% | nd |
| Cutoff 4 | 0.0347 | 0.0374 | 0.0353 | 0.0347 | 0.0374 | 0.0353 | 0.0347 | 0.0374 | nd |
| Sens 4 | 59% | 57% | 67% | 52% | 55% | 53% | 30% | 38% | nd |
| Spec 4 | 70% | 70% | 70% | 70% | 70% | 70% | 70% | 70% | nd |
| Cutoff 5 | 0.0850 | 0.0996 | 0.0863 | 0.0850 | 0.0996 | 3.0863 | 0.0850 | 0.0996 | nd |
| Sens 5 | 41% | 57% | 44% | 33% | 18% | 40% | 10% | 25% | nd |
| Spec 5 | 80% | 80% | 80% | 80% | 80% | 80% | 80% | 80% | nd |
| Cutoff 6 | 0.374 | 0.400 | 0.374 | 0.374 | 0.400 | 0.374 | 0.374 | 0.400 | nd |
| Sens 6 | 24% | 43% | 22% | 19% | 0% | 27% | 10% | 12% | nd |
| Spec 6 | 90% | 90% | 90% | 90% | 90% | 90% | 90% | 90% | nd |
| OR Quart 2 | 0.33 | >2.0 | 0 | 1.7 | 0.33 | 2.0 | 1.0 | 1.0 | nd |
| | 0.34 | <0.57 | na | 0.48 | 0.34 | 0.42 | 1.00 | 1.0 | nd |
| p Value | 0.034 | >0.18 | na | 0.40 | 0.034 | | 0.14 | 0.14 | nd |
| 95% CI of OR Quart2 | 3.2 | na | na | 7.1 | 3.2 | 11 | 7.2 | 7.1 | nd |
| OR Quart 3 | 1.3 | >1.0 | 0.50 | 1.0 | 0.66 | 0.50 | 1.0 | 1.0 | nd |
| | 0.70 | <1.0 | 0.57 | 1.0 | 0.65 | 0.57 | 1.0 | 1.0 | nd |
| p Value | 0.30 | >0.062 | 0.045 | 0.20 | 0.11 | 0.045 | 0.14 | 0.14 | nd |
| 95% CI of OR Quart3 | 6.0 | na | 5.5 | 5.0 | 4.0 | 5.5 | 7.1 | 7.1 | nd |

(continued)

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| OR Quart | 3.0 | >4.0 | 3.0 | 3.4 | 1.7 | 4.1 | 2.0 | 1.0 | nd |
| 4 | 0.097 | <0.21 | 0.18 | 0.065 | 0.48 | 0.078 | 0.42 | 1.0 | nd |
| p Value | 0.82 | >0.45 | 0.61 | 0.93 | 0.40 | 0.86 | 0.37 | 0.14 | nd |
| 95% CI of OR Quart4 | 11 | na | 15 | 12 | 7.1 | 19 | 11 | 7.1 | nd |

**Heparin-binding EGF-like growth factor**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 131 | 135 | 131 | 157 | 131 | 136 |
| Average | 140 | 141 | 140 | 163 | 140 | 129 |
| Stdev | 58.5 | 43.3 | 58.5 | 90.7 | 58.5 | 38.1 |
| p(t-test) |  | 0.94 |  | 0.14 |  | 0.56 |
| Min | 31.0 | 83.4 | 31.0 | 53.4 | 31.0 | 69.9 |
| Max | 360 | 234 | 360 | 444 | 360 | 177 |
| n (Samp) | 816 | 16 | 816 | 16 | 816 | 10 |
| n (Patient) | 362 | 16 | 362 | 16 | 362 | 10 |

| sCr only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | nd | nd | 130 | 151 | 130 | 170 |
| Average | nd | nd | 140 | 152 | 140 | 160 |
| Stdev | nd | nd | 58.9 | 61.3 | 58.9 | 42.7 |
| p(t-test) | nd | nd |  | 0.57 |  | 0.37 |
| Min | nd | nd | 31.0 | 74.6 | 31.0 | 76.6 |
| Max | nd | nd | 444 | 265 | 444 | 217 |
| n (Samp) | nd | nd | 847 | 8 | 847 | 7 |
| n (Patient) | nd | nd | 375 | 8 | 375 | 7 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 130 | 116 | 130 | 124 | nd | nd |
| Average | 140 | 129 | 140 | 149 | nd | nd |
| Stdev | 58.8 | 48.9 | 58.8 | 96.8 | nd | nd |
| p(t-test) | | 0.58 | | 0.56 | nd | nd |
| Min | 31.0 | 77.1 | 31.0 | 53.4 | nd | nd |
| Max | 360 | 234 | 360 | 444 | nd | nd |
| n (Samp) | 811 | 10 | 811 | | nd | nd |
| n (Patient) | 338 | 10 | 338 | 13 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.53 | nd | 0.46 | 0.56 | 0.56 | 0.49 | 0.47 | 0.64 | nd |
| SE | 0.074 | nd | 0.094 | 0.075 | 0.11 | 0.081 | 0.093 | 0.11 | nd |
| p | 0.72 | nd | 0.63 | 0.39 | 0.56 | 0.91 | 0.76 | 0.22 | nd |
| nCohort 1 | 816 | nd | 811 | 816 | 847 | 811 | 816 | 847 | nd |
| nCohort 2 | 16 | nd | 10 | 16 | 8 | 13 | 10 | 7 | nd |
| Cutoff 1 | 118 | nd | 100 | 110 | 102 | 100 | 120 | 164 | nd |
| Sens 1 | 75% | nd | 70% | 75% | 75% | 77% | 70% | 71% | nd |
| Spec 1 | 42% | nd | 31% | 36% | 31% | 31% | 42% | 70% | nd |
| Cutoff 2 | 107 | nd | 92.4 | 102 | 98.4 | 99.1 | 96.4 | 143 | nd |
| Sens 2 | 81% | nd | 80% | 81% | 88% | 85% | 80% | 86% | nd |
| Spec 2 | 34% | nd | 25% | 31% | 29% | 30% | 27% | 58% | nd |
| Cutoff 3 | 84.8 | nd | 84.8 | 74.4 | 74.4 | 81.8 | 76.5 | 76.5 | nd |
| Sens 3 | 94% | nd | 90% | 94% | 100% | 92% | 90% | 100% | nd |
| Spec 3 | 17% | nd | 18% | 10% | 11% | 16% | 12% | 12% | nd |
| Cutoff 4 | 166 | nd | 166 | 166 | 165 | 166 | 166 | 165 | nd |
| Sens 4 | 25% | nd | 20% | 31% | 38% | 23% | 20% | 57% | nd |
| Spec 4 | 70% | nd | 70% | 70% | 70% | 70% | 70% | 70% | nd |
| Cutoff 5 | 192 | nd | 192 | 192 | 190 | 192 | 192 | 190 | nd |
| Sens 5 | 12% | nd | 10% | 12% | 12% | 8% | 0% | 14% | nd |
| Spec 5 | 80% | nd | 80% | 80% | 80% | 80% | 80% | 80% | nd |
| Cutoff 6 | 222 | nd | 220 | 222 | 220 | 220 | 222 | 220 | nd |
| Sens 6 | 6% | nd | 10% | 12% | 12% | 8% | 0% | 0% | nd |
| Spec 6 | 90% | nd | 90% | 90% | 90% | 90% | 90% | 90% | nd |

(continued)

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| OR Quart 2 | 3.1 | nd | 1.0 | 2.0 | 2.0 | 2.0 | >6.2 | 0 | nd |
|  | 0.17 | nd | 1.00 | 0.42 | 0.57 | 0.42 | <0.092 | na | nd |
| p Value | 0.61 | nd | 0.14 | 0.37 | 0.18 | 0.37 | >0.74 | na | nd |
| 95% CI of OR Quart2 | 15 | nd | 7.2 | 11 | 22 | 11 | na | na | nd |
| OR Quart 3 | 2.0 | nd | 2.0 | 3.1 | 2.0 | 2.5 | >2.0 | 4.1 | nd |
|  | 0.42 | nd | 0.42 | 0.17 | 0.57 | 0.27 | <0.57 | 0.21 | nd |
| p Value | 0.37 | nd | 0.37 | 0.61 | 0.18 | 0.49 | >0.18 | 0.45 | nd |
| 95% CI of OR Quart3 | 11 | nd | 11 | 15 | 22 | 13 | na | 37 | nd |
| OR Quart 4 | 2.0 | nd | 1.0 | 2.0 | 3.0 | 1.0 | >2.0 | 2.0 | nd |
|  | 0.42 | nd | 1.00 | 0.42 | 0.34 | 1.0 | <0.56 | 0.57 | nd |
| p Value | 0.37 | nd | 0.14 | 0.37 | 0.31 | 0.14 | >0.18 | 0.18 | nd |
| 95% CI ot OR Quart4 | 11 | nd | 7.2 | 11 | 29 | 7.2 | na | 22 | nd |

**Thrombospondin-2**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 1300 | 2180 | 1300 | 1520 | 1300 | 1450 |
| Average | 2160 | 3460 | 2160 | 8940 | 2160 | 1480 |
| Stdev | 2890 | 3590 | 2890 | 20600 | 2890 | 1120 |
| p(t-test) |  | 0.032 |  | 2.1E-17 |  | 0.38 |
| Min | 0.0376 | 90.7 | 0.0376 | 113 | 0.0376 | 14.6 |
| Max | 45800 | 14600 | 45800 | 80100 | 45800 | 3740 |
| n (Samp) | 1483 | 23 | 1483 | 24 | 1483 | 14 |
| n (Patient) | 493 | 23 | 493 | 24 | 493 | 14 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1340 | 1920 | 1340 | 1840 | 1340 | 1590 |
| Average | 2320 | 3280 | 2320 | 9720 | 2320 | 1770 |
| Stdev | 3500 | 4440 | 3500 | 23400 | 3500 | 1070 |
| p(t-test) | | 0.41 | | 9.0E-10 | | 0.60 |
| Min | 0.0376 | 90.7 | 0.0376 | 679 | 0.0376 | 419 |
| Max | 68100 | 14600 | 68100 | 80100 | 68100 | 3740 |
| n (Samp) | 1540 | 9 | 1540 | 11 | 1540 | 11 |
| n (Patient) | 509 | 9 | 509 | 11 | 509 | 11 |

| UO only | 0hr prior to AKI stage | | 24hr prior to AKI stage | 48hr prior to AKI stage | |
|---|---|---|---|---|---|
| | Cohort 1 | Cohort 2 | Cohort 1 Cohort 2 | Cohort 1 | Cohort 2 |
| Median | 1370 | 1670 | 1370 1250 | nd | nd |
| Average | 2280 | 2790 | 2280 6600 | nd | nd |
| Stdev | 3540 | 2840 | 3540 16200 | nd | nd |
| p(t-test) | | 0.60 | 3.5E-6 | nd | nd |
| Min | 0.0376 | 150 | 0.0376 113 | nd | nd |
| Max | 80100 | 8180 | 80100 68100 | nd | nd |
| n (Samp) | 1513 | 13 | 1513 18 | nd | nd |
| n (Patient) | 472 | 13 | 472 18 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.60 | 0.58 | 0.53 | 0.57 | 0.66 | 0.48 | 0.46 | 0.54 | nd |
| SE | 0.063 | 0.100 | 0.082 | 0.061 | 0.090 | 0.069 | 0.079 | 0.089 | nd |
| p | 0.12 | 0.44 | 0.71 | 0.27 | 0.071 | 0.80 | 0.61 | 0.66 | nd |
| nCohort 1 | 1483 | 1540 | 1513 | 1483 | 1540 | 1513 | 1483 | 1540 | nd |
| nCohort 2 | 23 | 9 | 13 | 24 | 11 | 18 | 14 | 11 | nd |
| Cutoff 1 | 831 | 878 | 481 | 1020 | 1460 | 560 | 813 | 1250 | nd |
| Sens 1 | 74% | 78% | 77% | 71% | 73% | 72% | 71% | 73% | nd |
| Spec 1 | 33% | 34% | 17% | 40% | 54% | 21% | 32% | 47% | nd |
| Cutoff 2 | 404 | 831 | 333 | 560 | 1330 | 360 | 276 | 1020 | nd |
| Sens 2 | 83% | 89% | 85% | 83% | 82% | 83% | 86% | 82% | nd |
| Spec 2 | 16% | 33% | 11% | 22% | 50% | 12% | 9% | 40% | nd |
| Cutoff 3 | 163 | 90.6 | 163 | 291 | 1020 | 167 | 72.4 | 619 | nd |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Sens 3 | 91% | 100% | 92% | 92% | 91% | 94% | 93% | 91% | nd |
| Spec 3 | 4% | 1% | 4% | 10% | 40% | 4% | 1% | 24% | nd |
| Cutoff 4 | 2250 | 2330 | 2330 | 2250 | 2330 | 2330 | 2250 | 2330 | nd |
| Sens 4 | 48% | 33% | 46% | 38% | 36% | 28% | 21% | 18% | nd |
| Spec 4 | 70% | 70% | 70% | 70% | 70% | 70% | 70% | 70% | nd |
| Cutoff 5 | 3140 | 3250 | 3240 | 3140 | 3250 | 3240 | 3140 | 3250 | nd |
| Sens 5 | 39% | 22% | 31% | 29% | 36% | 22% | 7% | 18% | nd |
| Spec 5 | 80% | 80% | 80% | 80% | 80% | 80% | 80% | 80% | nd |
| Cutoff 6 | 4640 | 5100 | 4800 | 4640 | 5100 | 4800 | 4640 | 5100 | nd |
| Sens 6 | 35% | 11% | 31% | 21% | 27% | 17% | 0% | 0% | nd |
| Spec 6 | 90% | 90% | 90% | 90% | 90% | 90% | 90% | 90% | nd |
| OR Quart 2 | 0.80 | 2.0 | 0.50 | 1.00 | >3.0 | 0.60 | 1.7 | 1.5 | nd |
| | 0.73 | 0.57 | 0.42 | 1.00 | <0.34 | 0.48 | 0.48 | 0.66 | nd |
| p Value | 0.21 | 0.18 | 0.090 | 0.29 | >0.31 | 0.14 | 0.40 | 0.25 | nd |
| 95% CI ot OR Quart2 | 3.0 | 22 | 2.7 | 3.5 | na | 2.5 | 7.1 | 9.0 | nd |
| OR Quart 3 | 0.60 | 4.0 | 0.50 | 1.2 | >4.0 | 0.80 | 0.67 | 2.0 | nd |
| | 0.48 | 0.21 | 0.42 | 0.77 | <0.21 | 0.74 | 0.66 | 0.42 | nd |
| p Value | 0.14 | 0.45 | 0.091 | 0.36 | >0.45 | 0.21 | 0.11 | 0.37 | nd |
| 95% CI of OR Quart3 | 2.5 | 36 | 2.7 | 4.0 | na | 3.0 | 4.0 | 11 | nd |
| OR Quart 4 | 2.2 | 2.0 | 1.2 | 1.6 | >4.0 | 1.2 | 1.3 | 1.00 | nd |
| | 0.14 | 0.57 | 0.74 | 0.41 | <0.21 | 0.76 | 0.70 | 1.00 | nd |
| p Value | 0.77 | 0.18 | 0.33 | 0.52 | >0.45 | 0.37 | 0.30 | 0.14 | nd |
| 95% CI ot OR Quart4 | 6.5 | 22 | 4.7 | 5.0 | na | 4.0 | 6.0 | 7.1 | nd |

**Tenascin**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 10.6 | 9.54 | 10.6 | 15.6 | 10.6 | 0.221 |

(continued)

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Average | 19.1 | 19.1 | 19.1 | 437 | 19.1 | 10.9 |
| Stdev | 50.4 | 34.4 | 50.4 | 1770 | 50.4 | 20.2 |
| p(t-test) | | 1.00 | | 3.5E-12 | | 0.61 |
| Min | 0.00398 | 0.0190 | 0.00398 | 0.0190 | 0.00398 | 0.0184 |
| Max | 945 | 141 | 945 | 7540 | 945 | 50.9 |
| n (Samp) | 883 | 16 | 883 | 18 | 883 | 10 |
| n (Patient) | 367 | 16 | 367 | 18 | 367 | 10 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | 10.6 | 20.6 | 10.6 | 0.338 |
| Average | nd | nd | 27.3 | 23.6 | 27.3 | 9.59 |
| Stdev | nd | nd | 254 | 9.89 | 254 | 19.1 |
| p(t-test) | nd | nd | | 0.97 | 0.85 | 0.85 |
| Min | nd | nd | 0.00398 | 9.36 | 0.00398 | 0.0184 |
| Max | nd | nd | 7540 | 39.5 | 7540 | 50.9 |
| n (Samp) | nd | nd | 915 | 8 | 915 | 7 |
| n (Patient) | nd | nd | 380 | 8 | 380 | 7 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 Cohort 2 | | Cohort 1 | Cohort 2 |
| Median | 10.7 | 11.4 | 10.7 4.85 | | nd | nd |
| Average | 19.4 | 26.0 | 19.4 552 | | nd | nd |
| Stdev | 50.4 | 42.2 | 50.4 2010 | | nd | nd |
| p(t-test) | | 0.68 | 4.9E-15 | | nd | nd |
| Min | 0.00398 | 0.0208 | 0.00398 | | nd | nd |
| Max | 945 | 141 | 945 7540 | | nd | nd |
| n (Samp) | 878 | 10 | 878 14 | | nd | nd |
| n (Patient) | 342 | 10 | 342 14 | | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.51 | nd | 0.56 | 0.58 | 0.73 | 0.45 | 0.35 | 0.34 | nd |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| SE | 0.073 | nd | 0.095 | 0.071 | 0.10 | 0.080 | 0.095 | 0.11 | nd |
| p | 0.91 | nd | 0.54 | 0.28 | 0.026 | 0.52 | 0.12 | 0.17 | nd |
| nCohort 1 | 883 | nd | 878 | 883 | 915 | 878 | 883 | 915 | nd |
| nCohort 2 | 16 | nd | 10 | 18 | 8 | 14 | 10 | 7 | nd |
| Cutoff 1 | 0.338 | nd | 7.28 | 8.63 | 20.5 | 0.0748 | 0.0187 | 0.0187 | nd |
| Sens 1 | 75% | nd | 70% | 72% | 75% | 79% | 70% | 71% | nd |
| Spec 1 | 28% | nd | 43% | 46% | 72% | 18% | 11% | 11% | nd |
| Cutoff 2 | 0.315 | nd | 0.338 | 0.0840 | 15.6 | 0.0187 | 0.0184 | 0.0184 | nd |
| Sens 2 | 88% | nd | 80% | 83% | 88% | 93% | 80% | 86% | nd |
| Spec 2 | 26% | nd | 28% | 20% | 63% | 11% | 9% | 9% | nd |
| Cutoff 3 | 0.0190 | nd | 0.315 | 0.0748 | 8.63 | 0.0187 | 0.0179 | 0.0179 | nd |
| Sens 3 | 94% | nd | 90% | 94% | 100% | 93% | 100% | 100% | nd |
| Spec 3 | 13% | nd | 26% | 17% | 46% | 11% | 8% | 8% | nd |
| Cutoff 4 | 19.5 | nd | 19.8 | 19.5 | 19.5 | 19.8 | 19.5 | 19.5 | nd |
| Sens 4 | 31% | nd | 40% | 39% | 75% | 29% | 20% | 14% | nd |
| Spec 4 | 70% | nd | 70% | 70% | 70% | 70% | 70% | 70% | nd |
| Cutoff 5 | 27.8 | nd | 28.1 | 27.8 | 27.8 | 28.1 | 27.8 | 27.8 | nd |
| Sens 5 | 19% | nd | 30% | 22% | 38% | 14% | 20% | 14% | nd |
| Spec 5 | 80% | nd | 81% | 80% | 80% | 81% | 80% | 80% | nd |
| Cutoff 6 | 40.0 | nd | 41.6 | 40.0 | 40.0 | 41.6 | 40.0 | 40.0 | nd |
| Sens 6 | 6% | nd | 10% | 11% | 0% | 14% | 20% | 14% | nd |
| Spec 6 | 90% | nd | 90% | 90% | 90% | 90% | 90% | 90% | nd |
| OR Quart 2 | 4.1 | nd | 4.1 | 0.75 | >1.0 | 2.0 | 0.50 | 1.0 | nd |
| | 0.077 | nd | 0.21 | 0.70 | <1.0 | 0.42 | 0.57 | 1.00 | nd |
| p Value | 0.86 | nd | 0.45 | 0.17 | >0.062 | 0.37 | 0.045 | 0.062 | nd |
| 95% CI of OR Quart2 | 19 | nd | 37 | 3.4 | na | 11 | 5.6 | 16 | nd |
| OR Quart 3 | 1.00 | nd | 1.0 | 1.8 | >4.1 | 1.0 | 1.0 | 2.0 | nd |
| | 1.00 | nd | 1.0 | 0.37 | <0.21 | 1.0 | 1.00 | 0.57 | nd |
| p Value | 0.14 | nd | 0.062 | 0.51 | >0.45 | 0.14 | 0.14 | 0.18 | nd |
| 95% CI of OR Quart3 | 7.1 | nd | 16 | 6.1 | na | 7.2 | 7.2 | 22 | nd |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| OR Quart | 2.0 | nd | 4.1 | 1.00 | >3.0 | 3.1 | 2.5 | 3.0 | nd |
| 4 | 0.42 | nd | 0.21 | 0.99 | <0.34 | 0.17 | 0.27 | 0.34 | nd |
| p Value | 0.36 | nd | 0.45 | 0.25 | >0.31 | 0.61 | 0.49 | 0.31 | nd |
| 95% CI ot OR Quart4 | 11 | nd | 37 | 4.0 | na | 15 | 13 | 29 | nd |

Fig. 10: Comparison of marker levels in EDTA samples collected from Cohort 1 (patients that did not progress beyond RIFLE stage 0, R, or I) and in EDTA samples collected from Cohort 2 (subjects who progress to RIFLE stage F) at 0, 24 hours, and 48 hours prior to the subject reaching RIFLE stage I.

**Angiopoietin-related protein 4**

| sCr or UO | 24hr prior to AKI stage | |
|---|---|---|
| | Cohort 1 | Cohort 2 |
| Median | 32.9 | 96.2 |
| Average | 49.1 | 127 |
| Stdev | 97.1 | 115 |
| p(t-test) | | 0.051 |
| Min | 1.77 | 25.0 |
| Max | 1900 | 339 |
| n (Samp) | 487 | 6 |
| n (Patient) | 218 | 6 |

| | 24hr prior to AKI stage | | |
|---|---|---|---|
| | sCr or UO | sCr only | UO only |
| AUC | 0.80 | nd | nd |
| SE | 0.11 | nd | nd |
| p | 0.0049 | nd | nd |
| nCohort 1 | 487 | nd | nd |
| nCohort 2 | 6 | nd | nd |
| Cutoff 1 | 52.4 | nd | nd |
| Sens 1 | 83% | nd | nd |
| Spec 1 | 74% | nd | nd |
| Cutoff 2 | 52.4 | nd | nd |

(continued)

|  | 24hr prior to AKI stage | | |
|---|---|---|---|
|  | sCr or UO | sCr only | UO only |
| Sens 2 | 83% | nd | nd |
| Spec 2 | 74% | nd | nd |
| Cutoff 3 | 25.0 | nd | nd |
| Sens 3 | 100% | nd | nd |
| Spec 3 | 37% | nd | nd |
| Cutoff 4 | 47.5 | nd | nd |
| Sens 4 | 83% | nd | nd |
| Spec 4 | 70% | nd | nd |
| Cutoff 5 | 61.9 | nd | nd |
| Sens 5 | 50% | nd | nd |
| Spec 5 | 80% | nd | nd |
| Cutoff 6 | 92.2 | nd | nd |
| Sens 6 | 50% | nd | nd |
| Spec 6 | 90% | nd | nd |
| OR | >1.0 | nd | nd |
| Quart 2 | <1.00 | nd | nd |
| p Value | >0.062 | nd | nd |
| 95% CI of OR Quart2 | na | nd | nd |
| OR | >1.0 | nd | nd |
| Quart 3 | <1.00 | nd | nd |
| p Value | >0.062 | nd | nd |
| 95% CI of OR Quart3 | na | nd | nd |
| OR | >4.1 | nd | nd |
| Quart 4 | <0.21 | nd | nd |
| p Value | >0.45 | nd | nd |
| 95% CI of OR Quart4 | na | nd | nd |

**Amphiregulin**

| sCr or UO | 24hr prior to AKI stage | |
|---|---|---|
|  | Cohort 1 | Cohort 2 |
| Median | 22.4 | 41.7 |
| Average | 28.3 | 40.8 |
| Stdev | 22.9 | 16.4 |
| p(t-test) |  | 0.18 |
| Min | 0.00246 | 19.8 |

(continued)

| sCr or UO | 24hr prior to AKI stage | |
|---|---|---|
| | Cohort 1 | Cohort 2 |
| Max | 198 | 60.8 |
| n (Samp) | 478 | 6 |
| n (Patient) | 214 | 6 |

| | 24hr prior to AKI stage | | |
|---|---|---|---|
| | sCr or UO | sCr only | UO only |
| AUC | 0.75 | nd | nd |
| SE | 0.12 | nd | nd |
| p | 0.034 | nd | nd |
| nCohort 1 | 478 | nd | nd |
| nCohort 2 | 6 | nd | nd |
| Cutoff 1 | 24.8 | nd | nd |
| Sens 1 | 83% | nd | nd |
| Spec 1 | 56% | nd | nd |
| Cutoff 2 | 24.8 | nd | nd |
| Sens 2 | 83% | nd | nd |
| Spec 2 | 56% | nd | nd |
| Cutoff 3 | 19.6 | nd | nd |
| Sens 3 | 100% | nd | nd |
| Spec 3 | 43% | nd | nd |
| Cutoff 4 | 32.2 | nd | nd |
| Sens 4 | 67% | nd | nd |
| Spec 4 | 70% | nd | nd |
| Cutoff 5 | 39.9 | nd | nd |
| Sens 5 | 50% | nd | nd |
| Spec 5 | 80% | nd | nd |
| Cutoff 6 | 54.0 | nd | nd |
| Sens 6 | 33% | nd | nd |
| Spec 6 | 90% | nd | nd |
| OR | >1.0 | nd | nd |
| Quart 2 | <1.00 | nd | nd |
| p Value | >0.062 | nd | nd |
| 95% CI of OR Quart2 | na | nd | nd |
| OR | >1.0 | nd | nd |
| Quart 3 | <1.00 | nd | nd |
| p Value | >0.062 | nd | nd |

(continued)

|  | 24hr prior to AKI stage | | |
|---|---|---|---|
|  | sCr or UO | sCr only | UO only |
| 95% CI of OR Quart3 | na | nd | nd |
| OR | >4.1 | nd | nd |
| Quart 4 | <0.21 | nd | nd |
| p Value | >0.46 | nd | nd |
| 95% CI of OR Quart4 | na | nd | nd |

**Betacellulin**

| sCr or UO | 24hr prior to AKI stage | |
|---|---|---|
|  | Cohort 1 | Cohort 2 |
| Median | 4.02 | 6.55 |
| Average | 4.75 | 12.8 |
| Stdev | 5.32 | 16.1 |
| p(t-test) |  | 4.7E-4 |
| Min | 0.00226 | 0.00289 |
| Max | 60.7 | 43.4 |
| n (Samp) | 479 | 6 |
| n (Patient) | 216 | 6 |

|  | 24hr prior to AKI stage | | |
|---|---|---|---|
|  | sCr or UO | sCr only | UO only |
| AUC | 0.64 | nd | nd |
| SE | 0.12 | nd | nd |
| p | 0.25 | nd | nd |
| nCohort 1 | 479 | nd | nd |
| nCohort 2 | 6 | nd | nd |
| Cutoff 1 | 3.09 | nd | nd |
| Sens 1 | 83% | nd | nd |
| Spec 1 | 36% | nd | nd |
| Cutoff 2 | 3.09 | nd | nd |
| Sens 2 | 83% | nd | nd |
| Spec 2 | 36% | nd | nd |
| Cutoff 3 | 0.00282 | nd | nd |
| Sens 3 | 100% | nd | nd |
| Spec 3 | 5% | nd | nd |

(continued)

|  | 24hr prior to AKI stage | | |
|---|---|---|---|
|  | sCr or UO | sCr only | UO only |
| Cutoff 4 | 5.42 | nd | nd |
| Sens 4 | 50% | nd | nd |
| Spec 4 | 70% | nd | nd |
| Cutoff 5 | 6.66 | nd | nd |
| Sens 5 | 50% | nd | nd |
| Spec 5 | 80% | nd | nd |
| Cutoff 6 | 8.60 | nd | nd |
| Sens 6 | 50% | nd | nd |
| Spec 6 | 90% | nd | nd |
| OR | 1.0 | nd | nd |
| Quart 2 | 1.0 | nd | nd |
| p Value | 0.062 | nd | nd |
| 95% CI of OR Quart2 | 16 | nd | nd |
| OR | 1.0 | nd | nd |
| Quart 3 | 1.0 | nd | nd |
| p Value | 0.062 | nd | nd |
| 95% CI of OR Quart3 | 16 | nd | nd |
| OR | 3.0 | nd | nd |
| Quart 4 | 0.34 | nd | nd |
| p Value | 0.31 | nd | nd |
| 95% CI of OR Quart4 | 29 | nd | nd |

**Proepiregulin**

| sCr or UO | 24hr prior to AKI stage | |
|---|---|---|
|  | Cohort 1 | Cohort 2 |
| Median | 0.392 | 1.07 |
| Average | 0.771 | 2.58 |
| Stdev | 2.33 | 4.32 |
| p(t-test) |  | 0.063 |
| Min | 0.000152 | 0.0249 |
| Max | 32.1 | 11.3 |
| n (Samp) | 478 | 6 |
| n (Patient) | 215 | 6 |

| | 24hr prior to AKI stage | | |
|---|---|---|---|
| | sCr or UO | sCr only | UO only |
| AUC | 0.72 | nd | nd |
| SE | 0.12 | nd | nd |
| p | 0.067 | nd | nd |
| nCohort 1 | 478 | nd | nd |
| nCohort 2 | 6 | nd | nd |
| Cutoff 1 | 0.513 | nd | nd |
| Sens 1 | 83% | nd | nd |
| Spec 1 | 61% | nd | nd |
| Cutoff 2 | 0.513 | nd | nd |
| Sens 2 | 83% | nd | nd |
| Spec 2 | 61% | nd | nd |
| Cutoff 3 | 0.0201 | nd | nd |
| Sens 3 | 100% | nd | nd |
| Spec 3 | 5% | nd | nd |
| Cutoff 4 | 0.645 | nd | nd |
| Sens 4 | 67% | nd | nd |
| Spec 4 | 70% | nd | nd |
| Cutoff 5 | 0.806 | nd | nd |
| Sens 5 | 67% | nd | nd |
| Spec 5 | 81% | nd | nd |
| Cutoff 6 | 1.24 | nd | nd |
| Sens 6 | 50% | nd | nd |
| Spec 6 | 90% | nd | nd |
| OR | 0 | nd | nd |
| Quart 2 | na | nd | nd |
| p Value | na | nd | nd |
| 95% CI of OR Quart2 | na | nd | nd |
| OR | 1.0 | nd | nd |
| Quart 3 | 1.0 | nd | nd |
| p Value | 0.062 | nd | nd |
| 95% CI of OR Quart3 | 16 | nd | nd |
| OR | 4.1 | nd | nd |
| Quart 4 | 0.21 | nd | nd |
| p Value | 0.45 | nd | nd |
| 95% CI of OR Quart4 | 37 | nd | nd |

**Fibroblast growth factor 19**

| sCr or UO | 24hr prior to AKI stage | |
|---|---|---|
| | Cohort 1 | Cohort 2 |
| Median | 0.157 | 0.271 |
| Average | 0.198 | 0.414 |
| Stdev | 0.198 | 0.369 |
| p(t-test) | | 0.0090 |
| Min | 2.92E-5 | 0.0449 |
| Max | 2.09 | 0.971 |
| n (Samp) | 487 | 6 |
| n (Patient) | 218 | 6 |

| | 24hr prior to AKI stage | | |
|---|---|---|---|
| | sCr or UO | sCr only | UO only |
| AUC | 0.68 | nd | nd |
| SE | 0.12 | nd | nd |
| p | 0.14 | nd | nd |
| nCohort 1 | 487 | nd | nd |
| nCohort 2 | 6 | nd | nd |
| Cutoff 1 | 0.172 | nd | nd |
| Sens 1 | 83% | nd | nd |
| Spec 1 | 54% | nd | nd |
| Cutoff 2 | 0.172 | nd | nd |
| Sens 2 | 83% | nd | nd |
| Spec 2 | 54% | nd | nd |
| Cutoff 3 | 0.0430 | nd | nd |
| Sens 3 | 100% | nd | nd |
| Spec 3 | 16% | nd | nd |
| Cutoff 4 | 0.233 | nd | nd |
| Sens 4 | 50% | nd | nd |
| Spec 4 | 70% | nd | nd |
| Cutoff 5 | 0.300 | nd | nd |
| Sens 5 | 50% | nd | nd |
| Spec 5 | 80% | nd | nd |
| Cutoff 6 | 0.395 | nd | nd |
| Sens 6 | 33% | nd | nd |
| Spec 6 | 90% | nd | nd |

(continued)

|  | 24hr prior to AKI stage | | |
|---|---|---|---|
|  | sCr or UO | sCr only | UO only |
| OR | 0 | nd | nd |
| Quart 2 | na | nd | nd |
| p Value | na | nd | nd |
| 95% CI of OR Quart2 | na | nd | nd |
| OR | 2.0 | nd | nd |
| Quart 3 | 0.57 | nd | nd |
| p Value | 0.18 | nd | nd |
| 95% CI of OR Quart3 | 23 | nd | nd |
| OR | 3.0 | nd | nd |
| Quart 4 | 0.34 | nd | nd |
| p Value | 0.31 | nd | nd |
| 95% CI of OR Quart4 | 29 | nd | nd |

**Fibroblast growth factor 21**

| sCr or UO | 24hr prior to AKI stage | |
|---|---|---|
|  | Cohort 1 | Cohort 2 |
| Median | 0.0538 | 0.103 |
| Average | 0.214 | 0.141 |
| Stdev | 0.529 | 0.145 |
| p(t-test) |  | 0.74 |
| Min | 4.60E-6 | 0.0212 |
| Max | 5.72 | 0.418 |
| n (Samp) | 487 | 6 |
| n (Patient) | 218 | 6 |

|  | 24hr prior to AKI stage | | |
|---|---|---|---|
|  | sCr or UO | sCr only | UO only |
| AUC | 0.61 | nd | nd |
| SE | 0.12 | nd | nd |
| p | 0.39 | nd | nd |
| nCohort 1 | 487 | nd | nd |
| nCohort 2 | 6 | nd | nd |
| Cutoff 1 | 0.0388 | nd | nd |
| Sens 1 | 83% | nd | nd |

(continued)

|  | 24hr prior to AKI stage | | |
|---|---|---|---|
|  | sCr or UO | sCr only | UO only |
| Spec 1 | 42% | nd | nd |
| Cutoff 2 | 0.0388 | nd | nd |
| Sens 2 | 83% | nd | nd |
| Spec 2 | 42% | nd | nd |
| Cutoff 3 | 0.0209 | nd | nd |
| Sens 3 | 100% | nd | nd |
| Spec 3 | 29% | nd | nd |
| Cutoff 4 | 0.129 | nd | nd |
| Sens 4 | 33% | nd | nd |
| Spec 4 | 70% | nd | nd |
| Cutoff 5 | 0.224 | nd | nd |
| Sens 5 | 17% | nd | nd |
| Spec 5 | 80% | nd | nd |
| Cutoff 6 | 0.495 | nd | nd |
| Sens 6 | 0% | nd | nd |
| Spec 6 | 90% | nd | nd |
| OR | >2.0 | nd | nd |
| Quart 2 | <0.56 | nd | nd |
| p Value | >0.18 | nd | nd |
| 95% CI of OR Quart2 | na | nd | nd |
| OR | >3.1 | nd | nd |
| Quart 3 | <0.33 | nd | nd |
| p Value | >0.32 | nd | nd |
| 95% CI of OR Quart3 | na | nd | nd |
| OR | >1.0 | nd | nd |
| Quart 4 | <1.0 | nd | nd |
| p Value | >0.062 | nd | nd |
| 95% CI of OR Quart4 | na | nd | nd |

**Heparin-binding EGF-like growth factor**

| sCr or UO | 24hr prior to AKI stage | |
|---|---|---|
|  | Cohort 1 | Cohort 2 |
| Median | 16.3 | 46.9 |
| Average | 20.0 | 74.3 |

(continued)

| sCr or UO | 24hr prior to AKI stage | |
|---|---|---|
| | Cohort 1 | Cohort 2 |
| Stdev | 15.8 | 74.4 |
| p(t-test) | | 2.1E-13 |
| Min | 5.20 | 21.0 |
| Max | 186 | 222 |
| n (Samp) | 476 | 6 |
| n (Patient) | 215 | 6 |

| | 24hr prior to AKI stage | | |
|---|---|---|---|
| | sCr or UO | sCr only | UO only |
| AUC | 0.92 | nd | nd |
| SE | 0.076 | nd | nd |
| p | 2.4E-8 | nd | nd |
| nCohort 1 | 476 | nd | nd |
| nCohort 2 | 6 | nd | nd |
| Cutoff 1 | 35.8 | nd | nd |
| Sens 1 | 83% | nd | nd |
| Spec 1 | 92% | nd | nd |
| Cutoff 2 | 35.8 | nd | nd |
| Sens 2 | 83% | nd | nd |
| Spec 2 | 92% | nd | nd |
| Cutoff 3 | 20.9 | nd | nd |
| Sens 3 | 100% | nd | nd |
| Spec 3 | 73% | nd | nd |
| Cutoff 4 | 20.2 | nd | nd |
| Sens 4 | 100% | nd | nd |
| Spec 4 | 70% | nd | nd |
| Cutoff 5 | 23.6 | nd | nd |
| Sens 5 | 83% | nd | nd |
| Spec 5 | 80% | nd | nd |
| Cutoff 6 | 31.9 | nd | nd |
| Sens 6 | 83% | nd | nd |
| Spec 6 | 90% | nd | nd |
| OR | >0 | nd | nd |
| Quart 2 | <na | nd | nd |
| p Value | >na | nd | nd |

(continued)

|  | 24hr prior to AKI stage | | |
| --- | --- | --- | --- |
|  | sCr or UO | sCr only | UO only |
| 95% CI of OR Quart2 | na | nd | nd |
| OR | >1.0 | nd | nd |
| Quart 3 | <1.00 | nd | nd |
| p Value | >0.062 | nd | nd |
| 95% CI of OR Quart3 | na | nd | nd |
| OR | >5.2 | nd | nd |
| Quart 4 | <0.14 | nd | nd |
| p Value | >0.60 | nd | nd |
| 95% CI of OR Quart4 | na | nd | nd |

**Tenascin**

| sCr or UO | 24hr prior to AKI stage | |
| --- | --- | --- |
|  | Cohort 1 | Cohort 2 |
| Median | 593 | 1520 |
| Average | 1170 | 1380 |
| Stdev | 2220 | 718 |
| p(t-test) |  | 0.82 |
| Min | 43.9 | 323 |
| Max | 26100 | 2220 |
| n (Samp) | 486 | 6 |
| n (Patient) | 217 | 6 |

|  | 24hr prior to AKI stage | | |
| --- | --- | --- | --- |
|  | sCr or UO | sCr only | UO only |
| AUC | 0.72 | nd | nd |
| SE | 0.12 | nd | nd |
| p | 0.062 | nd | nd |
| nCohort 1 | 486 | nd | nd |
| nCohort 2 | 6 | nd | nd |
| Cutoff 1 | 814 | nd | nd |
| Sens 1 | 83% | nd | nd |
| Spec 1 | 64% | nd | nd |
| Cutoff 2 | 814 | nd | nd |
| Sens 2 | 83% | nd | nd |

(continued)

|  | 24hr prior to AKI stage | | |
|---|---|---|---|
|  | sCr or UO | sCr only | UO only |
| Spec 2 | 64% | nd | nd |
| Cutoff 3 | 322 | nd | nd |
| Sens 3 | 100% | nd | nd |
| Spec 3 | 23% | nd | nd |
| Cutoff 4 | 964 | nd | nd |
| Sens 4 | 67% | nd | nd |
| Spec 4 | 70% | nd | nd |
| Cutoff 5 | 1250 | nd | nd |
| Sens 5 | 67% | nd | nd |
| Spec 5 | 80% | nd | nd |
| Cutoff 6 | 2140 | nd | nd |
| Sens 6 | 17% | nd | nd |
| Spec 6 | 90% | nd | nd |
| OR | 0 | nd | nd |
| Quart 2 | na | nd | nd |
| p Value | na | nd | nd |
| 95% CI of OR Quart2 | na | nd | nd |
| OR | 1.0 | nd | nd |
| Quart 3 | 1.0 | nd | nd |
| p Value | 0.062 | nd | nd |
| 95% CI of OR Quart3 | 16 | nd | nd |
| OR | 4.1 | nd | nd |
| Quart 4 | 0.21 | nd | nd |
| p Value | 0.45 | nd | nd |
| 95% CI of OR Quart4 | 37 | nd | nd |

Fig. 11: Comparison of marker levels in enroll urine samples collected from Cohort 1 (patients that did not progress beyond RIFLE stage 0 or R within 48hrs) and in enroll urine samples collected from Cohort 2 (subjects reaching RIFLE stage I or F within 48hrs). Enroll samples from patients already at RIFLE stage I or F were included in Cohort 2.

**Angiogenin**

|  | sCr or UO | | sCr only | | UO only | |
|---|---|---|---|---|---|---|
|  | Cohort 1 | Cohort 2 | Cohort 1 | Cohort 2 | Cohort 1 | Cohort 2 |
| Median | 4420 | 8700 | 5060 | 8890 | 4570 | 8690 |
| Average | 7680 | 11200 | 8320 | 11600 | 7900 | 10900 |
| Stdev | 7650 | 8940 | 7980 | 10100 | 7910 | 8530 |

(continued)

|  | sCr or UO | | sCr only | | UO only | |
|---|---|---|---|---|---|---|
|  | Cohort 1 | Cohort 2 | Cohort 1 | Cohort 2 | Cohort 1 | Cohort 2 |
| p(t-test) |  | 1.9E-4 |  | 0.092 |  | 0.0030 |
| Min | 0.00873 | 54.6 | 0.00873 | 647 | 0.00873 | 54.6 |
| Max | 30600 | 30600 | 30600 | 30600 | 30600 | 30600 |
| n (Samp) | 352 | 93 | 421 | 18 | 343 | 82 |
| n (Patient) | 352 | 93 | 421 | 18 | 343 | 82 |

|  | At Enrollment | | |
|---|---|---|---|
|  | sCr or UO | sCr only | UO only |
| AUC | 0.62 | 0.58 | 0.61 |
| SE | 0.034 | 0.072 | 0.036 |
| p | 6.5E-4 | 0.28 | 0.0017 |
| nCohort 1 | 352 | 421 | 343 |
| nCohort 2 | 93 | 18 | 82 |
| Cutoff 1 | 4290 | 4330 | 4330 |
| Sens 1 | 71% | 72% | 71% |
| Spec 1 | 50% | 46% | 50% |
| Cutoff 2 | 2200 | 995 | 2580 |
| Sens 2 | 81% | 83% | 80% |
| Spec 2 | 28% | 12% | 33% |
| Cutoff 3 | 985 | 831 | 1050 |
| Sens 3 | 90% | 94% | 90% |
| Spec 3 | 12% | 9% | 13% |
| Cutoff 4 | 9070 | 10600 | 9500 |
| Sens 4 | 49% | 44% | 48% |
| Spec 4 | 70% | 70% | 70% |
| Cutoff 5 | 15600 | 16600 | 15800 |
| Sens 5 | 33% | 33% | 29% |
| Spec 5 | 80% | 80% | 80% |
| Cutoff 6 | 20400 | 20400 | 20400 |
| Sens 6 | 14% | 17% | 12% |
| Spec 6 | 93% | 92% | 92% |
| OR Quart 2 | 0.93 | 0.39 | 1.4 |
| p Value | 0.85 | 0.26 | 0.43 |
| 95% CI of | 0.44 | 0.073 | 0.63 |
| OR Quart2 | 2.0 | 2.0 | 3.0 |
| OR Quart 3 | 1.5 | 0.78 | 2.0 |

(continued)

|  | At Enrollment | | |
| --- | --- | --- | --- |
|  | sCr or UO | sCr only | UO only |
| p Value | 0.23 | 0.72 | 0.071 |
| 95% CI of | 0.77 | 0.21 | 0.94 |
| OR Quart3 | 3.0 | 3.0 | 4.2 |
| OR Quart 4 | 2.6 | 1.4 | 2.7 |
| p Value | 0.0038 | 0.57 | 0.0078 |
| 95% CI of | 1.4 | 0.43 | 1.3 |
| OR Quart4 | 5.0 | 4.6 | 5.5 |

**Angiopoietin-related protein 4**

|  | sCr or UO | | sCr only | | UO only | |
| --- | --- | --- | --- | --- | --- | --- |
|  | Cohort 1 | Cohort 2 | Cohort 1 | Cohort 2 | Cohort 1 | Cohort 2 |
| Median | 9.75 | 14.6 | 10.6 | 20.7 | 10.6 | 13.0 |
| Average | 39.3 | 51.6 | 40.1 | 74.6 | 39.8 | 49.3 |
| Stdev | 89.3 | 95.9 | 89.2 | 118 | 89.5 | 96.1 |
| p(t-test) |  | 0.29 |  | 0.13 |  | 0.43 |
| Min | 0.000466 | 0.000466 | 0.000466 | 0.000466 | 0.000466 | 0.000466 |
| Max | 708 | 413 | 708 | 413 | 708 | 413 |
| n (Samp) | 294 | 79 | 351 | 17 | 296 | 69 |
| n (Patient) | 294 | 79 | 351 | 17 | 296 | 69 |

|  | At Enrollment | | |
| --- | --- | --- | --- |
|  | sCr or UO | sCr only | UO only |
| AUC | 0.58 | 0.61 | 0.56 |
| SE | 0.037 | 0.074 | 0.039 |
| p | 0.036 | 0.12 | 0.13 |
| nCohort 1 | 294 | 351 | 296 |
| nCohort 2 | 79 | 17 | 69 |
| Cutoff 1 | 7.09 | 8.48 | 7.09 |
| Sens 1 | 71% | 71% | 71% |
| Spec 1 | 38% | 43% | 38% |
| Cutoff 2 | 5.29 | 5.29 | 5.10 |
| Sens 2 | 81% | 82% | 81% |
| Spec 2 | 30% | 28% | 28% |
| Cutoff 3 | 3.28 | 2.94 | 3.21 |

(continued)

|  | At Enrollment | | |
|---|---|---|---|
|  | sCr or UO | sCr only | UO only |
| Sens 3 | 91% | 94% | 91% |
| Spec 3 | 15% | 13% | 14% |
| Cutoff 4 | 18.1 | 19.6 | 19.4 |
| Sens 4 | 46% | 53% | 39% |
| Spec 4 | 70% | 70% | 70% |
| Cutoff 5 | 36.6 | 36.2 | 37.2 |
| Sens 5 | 25% | 47% | 22% |
| Spec 5 | 80% | 80% | 80% |
| Cutoff 6 | 96.2 | 96.5 | 96.2 |
| Sens 6 | 15% | 24% | 14% |
| Spec 6 | 90% | 90% | 90% |
| OR Quart 2 | 1.5 | 1.3 | 1.4 |
| p Value | 0.26 | 0.70 | 0.43 |
| 95% CI of | 0.73 | 0.29 | 0.63 |
| OR Quart2 | 3.3 | 6.2 | 3.0 |
| OR Quart 3 | 1.4 | 0.66 | 1.4 |
| p Value | 0.44 | 0.65 | 0.43 |
| 95% CI of | 0.63 | 0.11 | 0.63 |
| OR Quart3 | 2.9 | 4.0 | 3.0 |
| OR Quart 4 | 2.3 | 2.8 | 1.9 |
| p Value | 0.026 | 0.13 | 0.10 |
| 95% CI of | 1.1 | 0.73 | 0.88 |
| OR Quart4 | 4.7 | 11 | 4.0 |

**Amphiregulin**

|  | sCr or UO | | sCr only | | UO only | |
|---|---|---|---|---|---|---|
|  | Cohort 1 | Cohort 2 | Cohort 1 | Cohort 2 | Cohort 1 | Cohort 2 |
| Median | 23.5 | 39.4 | 25.0 | 42.8 | 25.2 | 35.9 |
| Average | 72.3 | 177 | 90.2 | 248 | 73.1 | 162 |
| Stdev | 202 | 527 | 298 | 478 | 211 | 522 |
| p(t-test) |  | 0.012 |  | 0.082 |  | 0.043 |
| Min | 0.00131 | 2.63 | 0.00131 | 10.1 | 0.00131 | 2.63 |
| Max | 1640 | 3480 | 3480 | 1710 | 1710 | 3480 |
| n (Samp) | 245 | 68 | 296 | 12 | 232 | 60 |
| n (Patient) | 245 | 68 | 296 | 12 | 232 | 60 |

|  | At Enrollment | | |
| --- | --- | --- | --- |
|  | sCr or UO | sCr only | UO only |
| AUC | 0.61 | 0.65 | 10.59 |
| SE | 0.040 | 0.088 | 0.042 |
| p | 0.0062 | 3.081 | 0.027 |
| nCohort 1 | 245 | 296 | 232 |
| nCohort 2 | 68 | 12 | 60 |
| Cutoff 1 | 18.9 | 18.9 | 18.9 |
| Sens 1 | 72% | 83% | 70% |
| Spec 1 | 41% | 39% | 39% |
| Cutoff 2 | 15.0 | 18.9 | 15.0 |
| Sens 2 | 81% | 83% | 80% |
| Spec 2 | 35% | 39% | 34% |
| Cutoff 3 | 10.0 | 12.6 | 10.0 |
| Sens 3 | 91% | 92% | 90% |
| Spec 3 | 21% | 26% | 20% |
| Cutoff 4 | 45.7 | 49.1 | 45.9 |
| Sens 4 | 44% | 42% | 45% |
| Spec 4 | 70% | 70% | 70% |
| Cutoff 5 | 59.5 | 68.6 | 59.5 |
| Sens 5 | 34% | 42% | 33% |
| Spec 5 | 80% | 80% | 80% |
| Cutoff 6 | 118 | 133 | 114 |
| Sens 6 | 22% | 42% | 20% |
| Spec 6 | 90% | 90% | 90% |
| OR Quart 2 | 2.0 | 3.1 | 1.9 |
| p Value | 0.099 | 0.33 | 0.14 |
| 95% CI of | 0.87 | 0.31 | 0.81 |
| OR Quart2 | 4.8 | 30 | 4.5 |
| OR Quart 3 | 1.5 | 3.1 | 1.0 |
| p Value | 0.38 | 0.33 | 1.0 |
| 95% CI of | 0.62 | 0.31 | 0.39 |
| OR Quart3 | 3.6 | 30 | 2.6 |
| OR Quart 4 | 3.3 | 5.3 | 2.9 |
| p Value | 0.0037 | 0.13 | 0.012 |
| 95% CI of | 1.5 | 0.60 | 1.3 |
| OR Quart4 | 7.5 | 46 | 6.6 |

**Betacellulin**

| | sCr or UO | | sCr only | | UO only | |
|---|---|---|---|---|---|---|
| | Cohort 1 | Cohort 2 | Cohort 1 | Cohort 2 | Cohort 1 | Cohort 2 |
| Median | 0.793 | 1.22 | 0.777 | 1.80 | 0.909 | 0.777 |
| Average | 1.41 | 1.56 | 1.42 | 2.12 | 1.35 | 1.47 |
| Stdev | 2.13 | 1.94 | 2.11 | 1.52 | 1.63 | 1.98 |
| p(t-test) | | 0.59 | | 0.25 | | 0.62 |
| Min | 0.00179 | 0.00240 | 0.00179 | 0.00352 | 0.00179 | 0.00240 |
| Max | 23.1 | 11.6 | 23.1 | 4.57 | 9.19 | 11.6 |
| n (Samp) | 245 | 68 | 296 | 12 | 232 | 60 |
| n (Patient) | 245 | 68 | 296 | 12 | 232 | 60 |

| | At Enrollment | | |
|---|---|---|---|
| | sCr or UO | sCr only | UO only |
| AUC | 0.53 | 0.67 | 0.50 |
| SE | 0.040 | 0.087 | 0.042 |
| p | 0.48 | 0.049 | 0.94 |
| nCohort 1 | 245 | 296 | 232 |
| nCohort 2 | 68 | 12 | 60 |
| Cutoff 1 | 0.0407 | 1.51 | 0.0407 |
| Sens 1 | 74% | 75% | 70% |
| Spec 1 | 28% | 59% | 28% |
| Cutoff 2 | 0.00342 | 0.793 | 0.00342 |
| Sens 2 | 82% | 83% | 80% |
| Spec 2 | 18% | 51% | 19% |
| Cutoff 3 | 0.00246 | 0.0522 | 0.00246 |
| Sens 3 | 91% | 92% | 90% |
| Spec 3 | 10% | 31% | 11% |
| Cutoff 4 | 1.76 | 1.87 | 1.78 |
| Sens 4 | 37% | 50% | 33% |
| Spec 4 | 70% | 70% | 70% |
| Cutoff 5 | 2.41 | 2.42 | 2.41 |
| Sens 5 | 25% | 33% | 23% |
| Spec 5 | 80% | 81% | 80% |
| Cutoff 6 | 3.36 | 3.36 | 3.36 |
| Sens 6 | 15% | 25% | 13% |
| Spec 6 | 91% | 90% | 91% |

(continued)

|  | At Enrollment | | |
|---|---|---|---|
|  | sCr or UO | sCr only | UO only |
| OR Quart 2 | 0.85 | 1.0 | 0.78 |
| p Value | 0.69 | 1.0 | 0.54 |
| 95% CI of | 0.39 | 0.061 | 0.35 |
| OR Quart2 | 1.9 | 16 | 1.7 |
| OR Quart 3 | 1.0 | 5.3 | 0.78 |
| p Value | 1.0 | 0.13 | 0.54 |
| 95% CI of | 0.47 | 0.60 | 0.35 |
| OR Quart3 | 2.1 | 46 | 1.7 |
| OR Quart 4 | 1.1 | 5.3 | 0.85 |
| p Value | 0.74 | 0.13 | 0.69 |
| 95% CI of | 0.54 | 0.60 | 0.39 |
| OR Quart4 | 2.4 | 46 | 1.9 |

**Endostatin**

|  | sCr or UO | | sCr only | | UO only | |
|---|---|---|---|---|---|---|
|  | Cohort 1 | Cohort 2 | Cohort 1 | Cohort 2 | Cohort 1 | Cohort 2 |
| Median | 4620 | 5530 | 4710 | 9920 | 4690 | 5400 |
| Average | 16600 | 17200 | 16300 | 24300 | 16400 | 16900 |
| Stdev | 32000 | 27800 | 31100 | 28800 | 31000 | 28600 |
| p(t-test) |  | 0.87 |  | 0.28 |  | 0.90 |
| Min | 0.0130 | 261 | 0.0130 | 748 | 0.0130 | 261 |
| Max | 238000 | 148000 | 238000 | 110000 | 238000 | 148000 |
| n (Samp) | 352 | 93 | 421 | 18 | 343 | 82 |
| n (Patient) | 352 | 93 | 421 | 18 | 343 | 82 |

|  | At Enrollment | | |
|---|---|---|---|
|  | sCr or UO | sCr only | UO only |
| AUC | 0.56 | 0.61 | 0.55 |
| SE | 0.034 | 0.072 | 0.036 |
| p | 0.096 | 0.12 | 0.20 |
| nCohort 1 | 352 | 421 | 343 |
| nCohort 2 | 93 | 18 | 82 |
| Cutoff 1 | 3530 | 2970 | 3530 |
| Sens 1 | 71% | 72% | 71% |

(continued)

|  | At Enrollment | | |
|---|---|---|---|
|  | sCr or UO | sCr only | UO only |
| Spec 1 | 42% | 35% | 41% |
| Cutoff 2 | 2540 | 2540 | 2570 |
| Sens 2 | 81% | 83% | 80% |
| Spec 2 | 32% | 30% | 32% |
| Cutoff 3 | 1510 | 1410 | 1510 |
| Sens 3 | 90% | 94% | 90% |
| Spec 3 | 15% | 13% | 15% |
| Cutoff 4 | 9580 | 9440 | 10000 |
| Sens 4 | 33% | 50% | 32% |
| Spec 4 | 70% | 70% | 70% |
| Cutoff 5 | 20800 | 20000 | 21900 |
| Sens 5 | 23% | 44% | 21% |
| Spec 5 | 80% | 80% | 80% |
| Cutoff 6 | 43700 | 43700 | 43700 |
| Sens 6 | 12% | 17% | 11% |
| Spec 6 | 90% | 90% | 90% |
| OR Quart 2 | 1.4 | 0.99 | 1.3 |
| p Value | 0.38 | 0.99 | 0.47 |
| 95% CI of | 0.68 | 0.20 | 0.64 |
| OR Quart2 | 2.7 | 5.0 | 2.7 |
| OR Quart 3 | 1.9 | 1.3 | 1.6 |
| p Value | 0.069 | 0.71 | 0.16 |
| 95% CI of | 0.95 | 0.29 | 0.82 |
| OR Quart3 | 3.6 | 6.1 | 3.3 |
| OR Quart 4 | 1.7 | 2.8 | 1.5 |
| p Value | 0.14 | 0.14 | 0.30 |
| 95% CI of | 0.85 | 0.72 | 0.72 |
| OR Quart4 | 3.3 | 11 | 3.0 |

**Proepiregulin**

|  | sCr or UO | | sCr only | | UO only | |
|---|---|---|---|---|---|---|
|  | Cohort 1 | Cohort 2 | Cohort 1 | Cohort 2 | Cohort 1 | Cohort 2 |
| Median | 3.42 | 5.13 | 3.55 | 3.48 | 3.52 | 5.13 |
| Average | 6.98 | 13.9 | 8.25 | 15.1 | 7.67 | 13.0 |
| Stdev | 19.3 | 22.1 | 20.3 | 20.4 | 20.3 | 21.9 |

(continued)

|  | sCr or UO | | sCr only | | UO only | |
|---|---|---|---|---|---|---|
|  | Cohort 1 | Cohort 2 | Cohort 1 | Cohort 2 | Cohort 1 | Cohort 2 |
| p(t-test) |  | 0.014 |  | 0.27 |  | 0.081 |
| Min | 0.0298 | 0.376 | 0.0298 | 1.42 | 0.0298 | 0.376 |
| Max | 279 | 134 | 279 | 57.1 | 279 | 134 |
| n (Samp) | 237 | 64 | 285 | 11 | 223 | 57 |
| n (Patient) | 237 | 64 | 285 | 11 | 223 | 57 |

|  | At Enrollment | | |
|---|---|---|---|
|  | sCr or UO | sCr only | UO only |
| AUC | 0.63 | 0.60 | 0.61 |
| SE | 0.041 | 0.092 | 0.043 |
| p | 0.0020 | 0.29 | 0.015 |
| nCohort 1 | 237 | 285 | 223 |
| nCohort 2 | 64 | 11 | 57 |
| Cutoff 1 | 2.85 | 2.57 | 2.85 |
| Sens 1 | 70% | 73% | 70% |
| Spec 1 | 43% | 38% | 42% |
| Cutoff 2 | 1.81 | 2.10 | 1.81 |
| Sens 2 | 81% | 82% | 81% |
| Spec 2 | 31% | 32% | 30% |
| Cutoff 3 | 0.971 | 1.46 | 0.750 |
| Sens 3 | 91% | 91% | 91% |
| Spec 3 | 16% | 22% | 9% |
| Cutoff 4 | 5.81 | 6.55 | 6.30 |
| Sens 4 | 48% | 45% | 46% |
| Spec 4 | 70% | 70% | 70% |
| Cutoff 5 | 8.47 | 10.2 | 9.47 |
| Sens 5 | 39% | 36% | 37% |
| Spec 5 | 80% | 80% | 80% |
| Cutoff 6 | 13.6 | 17.1 | 14.6 |
| Sens 6 | 27% | 27% | 23% |
| Spec 6 | 90% | 90% | 90% |
| OR Quart 2 | 1.9 | 2.1 | 1.5 |
| p Value | 0.14 | 0.41 | 0.37 |
| 95% CI of | 0.81 | 0.37 | 0.62 |
| OR Quart2 | 4.5 | 12 | 3.7 |
| OR Quart 3 | 1.1 | 0.49 | 1.1 |

(continued)

|  | At Enrollment | | |
|---|---|---|---|
|  | sCr or UO | sCr only | UO only |
| p Value | 0.81 | 0.57 | 0.81 |
| 95% CI of | 0.44 | 0.044 | 0.44 |
| OR Quart3 | 2.8 | 5.6 | 2.8 |
| OR Quart 4 | 3.4 | 2.1 | 2.8 |
| p Value | 0.0035 | 0.41 | 0.018 |
| 95% CI of | 1.5 | 0.37 | 1.2 |
| OR Quart4 | 7.7 | 12 | 6.4 |

**Heparin-binding growth factor 1**

|  | sCr or UO | | sCr only | | UO only | |
|---|---|---|---|---|---|---|
|  | Cohort 1 | Cohort 2 | Cohort 1 | Cohort 2 | Cohort 1 | Cohort 2 |
| Median | 867 | 712 | 869 | 396 | 861 | 820 |
| Average | 1250 | 1160 | 1250 | 768 | 1240 | 1200 |
| Stdev | 1400 | 1340 | 1390 | 1230 | 1420 | 1310 |
| p(t-test) |  | 0.58 |  | 0.15 |  | 0.82 |
| Min | 0.00328 | 2.62 | 0.00328 | 2.62 | 0.00328 | 2.62 |
| Max | 12300 | 6460 | 12300 | 5360 | 12300 | 6460 |
| n (Samp) | 352 | 93 | 421 | 18 | 343 | 82 |
| n (Patient) | 352 | 93 | 421 | 18 | 343 | 82 |

|  | At Enrollment | | |
|---|---|---|---|
|  | sCr or UO | sCr only | UO only |
| AUC | 0.46 | 0.32 | 0.48 |
| SE | 0.034 | 0.071 | 0.036 |
| p | 0.21 | 0.013 | 0.66 |
| nCohort 1 | 352 | 421 | 343 |
| nCohort 2 | 93 | 18 | 82 |
| Cutoff 1 | 338 | 221 | 417 |
| Sens 1 | 71% | 72% | 71% |
| Spec 1 | 22% | 14% | 29% |
| Cutoff 2 | 226 | 101 | 280 |
| Sens 2 | 81% | 83% | 80% |
| Spec 2 | 13% | 8% | 18% |
| Cutoff 3 | 101 | 49.5 | 119 |

(continued)

|  | At Enrollment | | |
|---|---|---|---|
|  | sCr or UO | sCr only | UO only |
| Sens 3 | 90% | 94% | 90% |
| Spec 3 | 8% | 4% | 9% |
| Cutoff 4 | 1430 | 1420 | 1420 |
| Sens 4 | 26% | 17% | 27% |
| Spec 4 | 70% | 70% | 70% |
| Cutoff 5 | 1850 | 1850 | 1820 |
| Sens 5 | 18% | 6% | 23% |
| Spec 5 | 80% | 80% | 80% |
| Cutoff 6 | 2630 | 2670 | 2670 |
| Sens 6 | 11% | 6% | 11% |
| Spec 6 | 90% | 90% | 90% |
| OR Quart 2 | 1.1 | 4.1 | 1.1 |
| p Value | 0.71 | 0.21 | 0.84 |
| 95% CI of | 0.59 | 0.45 | 0.54 |
| OR Quart2 | 2.2 | 37 | 2.1 |
| OR Quart 3 | 0.95 | 4.1 | 0.95 |
| p Value | 0.89 | 0.21 | 0.88 |
| 95% CI of | 0.48 | 0.45 | 0.47 |
| OR Quart3 | 1.9 | 37 | 1.9 |
| OR Quart 4 | 1.5 | 9.8 | 1.1 |
| p Value | 0.19 | 0.032 | 0.71 |
| 95% CI of | 0.81 | 1.2 | 0.58 |
| OR Quart4 | 2.9 | 79 | 2.2 |

**Thrombospondin-2**

|  | sCr or UO | | sCr only | | UO only | |
|---|---|---|---|---|---|---|
|  | Cohort 1 | Cohort 2 | Cohort 1 | Cohort 2 | Cohort 1 | Cohort 2 |
| Median | 988 | 1300 | 1050 | 2210 | 1050 | 1300 |
| Average | 1640 | 3290 | 1940 | 3080 | 1740 | 3270 |
| Stdev | 2280 | 7500 | 4070 | 3520 | 2430 | 7860 |
| p(t-test) | 4.1E-4 | 4.1E-4 |  | 0.24 |  | 0.0024 |
| Min | 0.0376 | 14.6 | 0.0376 | 90.7 | 0.0376 | 14.6 |
| Max | 26900 | 68100 | 68100 | 14600 | 26900 | 68100 |
| n (Samp) | 352 | 93 | 421 | 18 | 343 | 82 |
| n (Patient) | 352 | 93 | 421 | 18 | 343 | 82 |

|  | At Enrollment | | |
|---|---|---|---|
|  | sCr or UO | sCr only | UO only |
| AUC | 0.60 | 0.61 | 0.58 |
| SE | 0.034 | 0.072 | 0.036 |
| p | 0.0038 | 0.14 | 0.019 |
| nCohort 1 | 352 | 421 | 343 |
| nCohort 2 | 93 | 18 | 82 |
| Cutoff 1 | 760 | 679 | 760 |
| Sens 1 | 71% | 72% | 71% |
| Spec 1 | 39% | 34% | 37% |
| Cutoff 2 | 498 | 360 | 502 |
| Sens 2 | 81% | 83% | 80% |
| Spec 2 | 25% | 17% | 25% |
|  | At Enrollment | | |
|  | sCr or UO | sCr only | UO only |
| Cutoff 3 | 332 | 122 | 360 |
| Sens 3 | 90% | 94% | 90% |
| Spec 3 | 16% | 5% | 18% |
| Cutoff 4 | 1770 | 1830 | 1840 |
| Sens 4 | 41% | 56% | 39% |
| Spec 4 | 70% | 70% | 70% |
| Cutoff 5 | 2270 | 2430 | 2350 |
| Sens 5 | 40% | 44% | 37% |
| Spec 5 | 80% | 80% | 80% |
| Cutoff 6 | 3270 | 4010 | 3620 |
| Sens 6 | 28% | 28% | 22% |
| Spec 6 | 90% | 90% | 90% |
| OR Quart 2 | 0.71 | 0.19 | 0.86 |
| p Value | 0.36 | 0.13 | 0.70 |
| 95% CI of | 0.34 | 0.022 | 0.41 |
| OR Quart2 | 1.5 | 1.7 | 1.8 |
| OR Quart 3 | 1.1 | 0.58 | 1.1 |
| p Value | 0.86 | 0.47 | 0.85 |
| 95% CI of | 0.54 | 0.14 | 0.52 |
| OR Quart3 | 2.1 | 2.5 | 2.2 |
| OR Quart 4 | 2.2 | 1.9 | 2.2 |
| p Value | 0.011 | 0.28 | 0.018 |
| 95% CI of | 1.2 | 0.60 | 1.2 |
| OR Quart4 | 4.2 | 5.7 | 4.3 |

Fig. 12: Comparison of marker levels in enroll EDTA samples collected from Cohort 1 (patients that did not progress beyond RIFLE stage 0 or R within 48hrs) and in enroll EDTA samples collected from Cohort 2 (subjects reaching RIFLE stage I or F within 48hrs). Enroll samples from patients already at stage I or F were included in Cohort 2.

**Angiogenin**

|  | sCr or UO | | UO only | |
|---|---|---|---|---|
|  | Cohort 1 | Cohort 2 | Cohort 1 | Cohort 2 |
| Median | 179000 | 117000 | 179000 | 110000 |
| Average | 197000 | 159000 | 197000 | 157000 |
| Stdev | 197000 | 107000 | 201000 | 108000 |
| p(t-test) |  | 0.32 |  | 0.30 |
| Min | 10800 | 24100 | 10800 | 24100 |
| Max | 2240000 | 430000 | 2240000 | 430000 |
| n (Samp) | 141 | 30 | 135 | 29 |
| n (Patient) | 141 | 30 | 135 | 29 |

|  | At Enrollment | |
|---|---|---|
|  | sCr or UO | UO only |
| AUC | 0.40 | 0.39 |
| SE | 0.059 | 0.060 |
| p | 0.079 | 0.056 |
| nCohort 1 | 141 | 135 |
| nCohort 2 | 30 | 29 |
| Cutoff 1 | 90900 | 88800 |
| Sens 1 | 70% | 72% |
| Spec 1 | 15% | 14% |
| Cutoff 2 | 82700 | 80900 |
| Sens 2 | 80% | 83% |
| Spec 2 | 13% | 13% |
| Cutoff 3 | 76300 | 44000 |
| Sens 3 | 90% | 93% |
| Spec 3 | 12% | 4% |
| Cutoff 4 | 218000 | 215000 |
| Sens 4 | 23% | 24% |
| Spec 4 | 70% | 70% |
| Cutoff 5 | 241000 | 239000 |
| Sens 5 | 20% | 21% |
| Spec 5 | 80% | 80% |
| Cutoff 6 | 314000 | 332000 |

(continued)

|  | At Enrollment | |
| --- | --- | --- |
|  | sCr or UO | UO only |
| Sens 6 | 10% | 10% |
| Spec 6 | 90% | 90% |
| OR Quart 2 | 0.81 | 0.63 |
| p Value | 0.75 | 0.50 |
| 95% CI of | 0.23 | 0.16 |
| OR Quart2 | 2.9 | 2.4 |
| OR Quart 3 | 1.2 | 1.2 |
| p Value | 0.76 | 0.76 |
| 95% CI of | 0.37 | 0.37 |
| OR Quart3 | 3.9 | 3.9 |
| OR Quart 4 | 2.5 | 2.4 |
| p Value | 0.11 | 0.12 |
| 95% CI of | 0.83 | 0.81 |
| OR Quart4 | 7.4 | 7.2 |

**Angiopoietin-related protein 4**

|  | sCr or UO | | UO only | |
| --- | --- | --- | --- | --- |
|  | Cohort 1 | Cohort 2 | Cohort 1 | Cohort 2 |
| Median | 34.2 | 47.0 | 35.0 | 47.4 |
| Average | 63.6 | 73.3 | 65.6 | 75.0 |
| Stdev | 166 | 66.6 | 169 | 67.2 |
| p(t-test) |  | 0.75 |  | 0.77 |
| Min | 1.77 | 16.4 | 2.71 | 16.4 |
| Max | 1900 | 339 | 1900 | 339 |
| n (Samp) | 141 | 30 | 135 | 29 |
| n (Patient) | 141 | 30 | 135 | 29 |

|  | At Enrollment | |
| --- | --- | --- |
|  | sCr or UO | UO only |
| AUC | 0.65 | 0.65 |
| SE | 0.058 | 0.060 |
| p | 0.0094 | 0.011 |
| nCohort 1 | 141 | 135 |
| nCohort 2 | 30 | 29 |

(continued)

| | At Enrollment | |
| | sCr or UO | UO only |
|---|---|---|
| Cutoff 1 | 35.6 | 35.6 |
| Sens 1 | 70% | 72% |
| Spec 1 | 53% | 52% |
| Cutoff 2 | 29.1 | 29.1 |
| Sens 2 | 80% | 83% |
| Spec 2 | 41% | 39% |
| Cutoff 3 | 24.9 | 22.7 |
| Sens 3 | 90% | 93% |
| Spec 3 | 35% | 27% |
| Cutoff 4 | 51.8 | 53.8 |
| Sens 4 | 43% | 45% |
| Spec 4 | 70% | 70% |
| Cutoff 5 | 71.1 | 71.8 |
| Sens 5 | 30% | 31% |
| Spec 5 | 80% | 80% |
| Cutoff 6 | 98.7 | 104 |
| Sens 6 | 27% | 28% |
| Spec 6 | 90% | 90% |
| OR Quart 2 | 4.6 | 2.2 |
| p Value | 0.065 | 0.30 |
| 95% CI of | 0.91 | 0.50 |
| OR Quart2 | 23 | 9.4 |
| OR Quart 3 | 6.1 | 4.1 |
| p Value | 0.026 | 0.045 |
| 95% CI of | 1.2 | 1.0 |
| OR Quart3 | 30 | 16 |
| OR Quart 4 | 6.1 | 4.1 |
| p Value | 0.026 | 0.045 |
| 95% CI of | 1.2 | 1.0 |
| OR Quart4 | 30 | 16 |

**Amphiregulin**

| | sCr or UO | | UO only | |
| | Cohort 1 | Cohort 2 | Cohort 1 | Cohort 2 |
|---|---|---|---|---|
| Median | 24.9 | 27.5 | 24.5 | 31.5 |

(continued)

|  | sCr or UO | | UO only | |
|---|---|---|---|---|
|  | Cohort 1 | Cohort 2 | Cohort 1 | Cohort 2 |
| Average | 32.9 | 37.1 | 32.6 | 38.1 |
| Stdev | 29.0 | 32.2 | 29.3 | 32.3 |
| p(t-test) |  | 0.50 |  | 0.38 |
| Min | 0.00246 | 2.76 | 0.00246 | 2.76 |
| Max | 198 | 162 | 198 | 162 |
| n (Samp) | 136 | 29 | 130 | 28 |
| n (Patient) | 136 | 29 | 130 | 28 |

|  | At Enrollment | |
|---|---|---|
|  | sCr or UO | UO only |
| AUC | 0.53 | 0.56 |
| SE | 0.060 | 0.061 |
| p | 0.58 | 0.37 |
| nCohort 1 | 136 | 130 |
| nCohort 2 | 29 | 28 |
| Cutoff 1 | 16.8 | 17.0 |
| Sens 1 | 72% | 71% |
| Spec 1 | 29% | 30% |
| Cutoff 2 | 11.1 | 11.1 |
| Sens 2 | 83% | 86% |
| Spec 2 | 12% | 14% |
| Cutoff 3 | 8.21 | 8.93 |
| Sens 3 | 97% | 93% |
| Spec 3 | 9% | 12% |
| Cutoff 4 | 34.0 | 34.0 |
| Sens 4 | 48% | 50% |
| Spec 4 | 71% | 72% |
| Cutoff 5 | 44.7 | 44.1 |
| Sens 5 | 34% | 36% |
| Spec 5 | 80% | 80% |
| Cutoff 6 | 67.0 | 60.3 |
| Sens 6 | 14% | 14% |
| Spec 6 | 90% | 90% |
| OR Quart 2 | 0.57 | 0.65 |
| p Value | 0.37 | 0.50 |
| 95% CI of | 0.17 | 0.19 |

(continued)

|  | At Enrollment | |
| --- | --- | --- |
|  | sCr or UO | UO only |
| OR Quart2 | 1.9 | 2.3 |
| OR Quart 3 | 0.71 | 0.83 |
| p Value | 0.56 | 0.76 |
| 95% CI of OR Quart3 | 0.22 2.3 | 0.25 2.7 |
| OR Quart 4 | 1.3 | 1.5 |
| p Value | 0.64 | 0.45 |
| 95% CI of OR Quart4 | 0.45 3.7 | 0.51 4.5 |

**Betacellulin**

|  | sCr or UO | | UO only | |
| --- | --- | --- | --- | --- |
|  | Cohort 1 | Cohort 2 | Cohort 1 | Cohort 2 |
| Median | 3.93 | 3.11 | 3.73 | 3.11 |
| Average | 5.07 | 5.36 | 4.89 | 5.50 |
| Stdev | 5.50 | 7.04 | 5.49 | 7.12 |
| p(t-test) |  | 0.80 |  | 0.61 |
| Min | 0.00226 | 0.00274 | 0.00226 | 0.00274 |
| Max | 52.9 | 32.2 | 52.9 | 32.2 |
| n (Samp) | 137 | 30 | 131 | 29 |
| n (Patient) | 137 | 30 | 131 | 29 |

|  | At Enrollment | |
| --- | --- | --- |
|  | sCr or UO | UO only |
| AUC | 0.45 | 0.47 |
| SE | 0.059 | 0.060 |
| p | 0.36 | 0.60 |
| nCohort 1 | 137 | 131 |
| nCohort 2 | 30 | 29 |
| Cutoff 1 | 2.26 | 2.26 |
| Sens 1 | 70% | 72% |
| Spec 1 | 25% | 27% |
| Cutoff 2 | 1.78 | 1.78 |
| Sens 2 | 80% | 83% |

(continued)

| | At Enrollment | |
|---|---|---|
| | sCr or UO | UO only |
| Spec 2 | 21% | 23% |
| Cutoff 3 | 0.00282 | 0.00282 |
| Sens 3 | 97% | 97% |
| Spec 3 | 4% | 5% |
| Cutoff 4 | 5.88 | 5.83 |
| Sens 4 | 23% | 24% |
| Spec 4 | 70% | 70% |
| Cutoff 5 | 7.71 | 7.22 |
| Sens 5 | 17% | 17% |
| Spec 5 | 80% | 80% |
| Cutoff 6 | 10.1 | 9.07 |
| Sens 6 | 13% | 14% |
| Spec 6 | 91% | 90% |
| OR Quart 2 | 1.0 | 1.0 |
| p Value | 1.0 | 1.0 |
| 95% CI of | 0.29 | 0.29 |
| OR Quart2 | 3.4 | 3.4 |
| OR Quart 3 | 1.6 | 1.9 |
| p Value | 0.40 | 0.27 |
| 95% CI of | 0.53 | 0.61 |
| OR Quart3 | 5.1 | 5.8 |
| OR Quart 4 | 1.7 | 1.2 |
| p Value | 0.37 | 0.76 |
| 95% CI of | 0.54 | 0.37 |
| OR Quart4 | 5.3 | 4.0 |

**Proepiregulin**

| | sCr or UO | | UO only | |
|---|---|---|---|---|
| | Cohort 1 | Cohort 2 | Cohort 1 | Cohort 2 |
| Median | 0.411 | 0.383 | 0.406 | 0.394 |
| Average | 0.804 | 0.981 | 0.805 | 1.01 |
| Stdev | 2.80 | 2.10 | 2.86 | 2.13 |
| p(t-test) | | 0.75 | | 0.72 |
| Min | 0.000152 | 0.0249 | 0.000152 | 0.0249 |
| Max | 32.1 | 11.3 | 32.1 | 11.3 |

(continued)

|  | sCr or UO | | UO only | |
|---|---|---|---|---|
|  | Cohort 1 | Cohort 2 | Cohort 1 | Cohort 2 |
| n (Samp) | 136 | 29 | 130 | 28 |
| n (Patient) | 136 | 29 | 130 | 28 |

|  | At Enrollment | |
|---|---|---|
|  | sCr or UO | UO only |
| AUC | 0.51 | 0.54 |
| SE | 0.060 | 0.061 |
| p | 0.84 | 0.56 |
| nCohort 1 | 136 | 130 |
| nCohort 2 | 29 | 28 |
| Cutoff 1 | 0.242 | 0.242 |
| Sens 1 | 72% | 75% |
| Spec 1 | 31% | 32% |
| Cutoff 2 | 0.200 | 0.200 |
| Sens 2 | 83% | 82% |
| Spec 2 | 25% | 27% |
| Cutoff 3 | 0.0796 | 0.117 |
| Sens 3 | 93% | 93% |
| Spec 3 | 12% | 18% |
| Cutoff 4 | 0.647 | 0.589 |
| Sens 4 | 24% | 36% |
| Spec 4 | 72% | 71% |
| Cutoff 5 | 0.806 | 0.750 |
| Sens 5 | 21% | 25% |
| Spec 5 | 80% | 80% |
| Cutoff 6 | 1.24 | 1.20 |
| Sens 6 | 17% | 18% |
| Spec 6 | 90% | 90% |
| OR Quart 2 | 1.9 | 2.0 |
| p Value | 0.27 | 0.27 |
| 95% CI of | 0.61 | 0.60 |
| OR Quart2 | 5.8 | 6.5 |
| OR Quart 3 | 1.0 | 1.5 |
| p Value | 1.0 | 0.53 |
| 95% CI of | 0.29 | 0.43 |
| OR Quart3 | 3.4 | 5.2 |

(continued)

|  | At Enrollment | |
|---|---|---|
|  | sCr or UO | UO only |
| OR Quart 4 | 1.2 | 1.4 |
| p Value | 0.80 | 0.56 |
| 95% CI of | 0.36 | 0.42 |
| OR Quart4 | 3.8 | 5.0 |

While the invention has been described and exemplified in sufficient detail for those skilled in this art to make and use it, various alternatives, modifications, and improvements should be apparent without departing from the spirit and scope of the invention. The examples provided herein are representative of preferred embodiments, are exemplary, and are not intended as limitations on the scope of the invention. Modifications therein and other uses will occur to those skilled in the art. These modifications are encompassed within the spirit of the invention and are defined by the scope of the claims.

It will be readily apparent to a person skilled in the art that varying substitutions and modifications may be made to the invention disclosed herein without departing from the scope and spirit of the invention.

All patents and publications mentioned in the specification are indicative of the levels of those of ordinary skill in the art to which the invention pertains. All patents and publications are herein incorporated by reference to the same extent as if each individual publication was specifically and individually indicated to be incorporated by reference.

The invention illustratively described herein suitably may be practiced in the absence of any element or elements, limitation or limitations which is not specifically disclosed herein. Thus, for example, in each instance herein any of the terms "comprising", "consisting essentially of" and "consisting of" may be replaced with either of the other two terms. The terms and expressions which have been employed are used as terms of description and not of limitation, and there is no intention that in the use of such terms and expressions of excluding any equivalents of the features shown and described or portions thereof, but it is recognized that various modifications are possible within the scope of the invention claimed. Thus, it should be understood that although the present invention has been specifically disclosed by preferred embodiments and optional features, modification and variation of the concepts herein disclosed may be resorted to by those skilled in the art, and that such modifications and variations are considered to be within the scope of this invention as defined by the appended claims.

Other embodiments are set forth within the following claims.

The present application is also directed to the following aspects:

1. A method for evaluating renal status in a subject, comprising:

performing one or more assays configured to detect one or more biomarkers selected from the group consisting of Proheparin-binding EGF-like growth factor, Tenascin C, Angiopoietin-related protein 4, Fibroblast growth factor 19, Fibroblast growth factor 21, Heparin-binding growth factor 1, Angiopoietin-related protein 6, Proepiregulin, Probetacellulin, Amphiregulin, Angiogenin, Thrombospondin-2, and Collagen alpha-1(XVIII) chain on a body fluid sample obtained from the subject to provide an assay result; and

correlating the assay result(s) to the renal status of the subject.

2. A method according to aspect 1, wherein said correlation step comprises correlating the assay result(s) to one or more of risk stratification, diagnosis, staging, prognosis, classifying and monitoring of the renal status of the subject.

3. A method according to aspect 1, wherein said correlating step comprises assigning a likelihood of one or more future changes in renal status to the subject based on the assay result(s).

4. A method according to aspect 3, wherein said one or more future changes in renal status comprise one or more of a future injury to renal function, future reduced renal function, future improvement in renal function, and future acute renal failure (ARF).

5. A method according to one of aspects 1-4, wherein said assay results comprise at least 2, 3, or 4 of:

a measured concentration of Proheparin-binding EGF-like growth factor,

a measured concentration of Tenascin C,

a measured concentration of Angiopoietin-related protein 4,

a measured concentration of Fibroblast growth factor 19,

a measured concentration of Fibroblast growth factor 21,

a measured concentration of Heparin-binding growth factor 1,

a measured concentration of Angiopoietin-related protein 6,

a measured concentration of Proepiregulin,

a measured concentration of Probetacellulin,

a measured concentration of Amphiregulin,

a measured concentration of Angiogenin,

a measured concentration of Thrombospondin-2, and

a measured concentration of Collagen alpha-1(XVIII) chain.

6. A method according to one of aspects 1-5, wherein a plurality of assay results are combined using a function that converts the plurality of assay results into a single composite result.

7. A method according to aspect 3, wherein said one or more future changes in renal status comprise a clinical outcome related to a renal injury suffered by the subject.

8. A method according to aspect 3, wherein the likelihood of one or more future changes in renal status is that an event of interest is more or less likely to occur within 30 days of the time at which the body fluid sample is obtained from the subject.

9. A method according to aspect 8, wherein the likelihood of one or more future changes in renal status is that an event of interest is more or less likely to occur within a period selected from the group consisting of 21 days, 14 days, 7 days, 5 days, 96 hours, 72 hours, 48 hours, 36 hours, 24 hours, and 12 hours.

10. A method according to one of aspects 1-5, wherein the subject is selected for evaluation of renal status based on the pre-existence in the subject of one or more known risk factors for prerenal, intrinsic renal, or postrenal ARF.

11. A method according to one of aspects 1-5, wherein the subject is selected for evaluation of renal status based on an existing diagnosis of one or more of congestive heart failure, preeclampsia, eclampsia, diabetes mellitus, hypertension, coronary artery disease, proteinuria, renal insufficiency, glomerular filtration below the normal range, cirrhosis, serum creatinine above the normal range, sepsis, injury to renal function, reduced renal function, or ARF, or based on undergoing or having undergone major vascular surgery, coronary artery bypass, or other cardiac surgery, or based on exposure to NSAIDs, cyclosporines, tacrolimus, aminoglycosides, foscarnet, ethylene glycol, hemoglobin, myoglobin, ifosfamide, heavy metals, methotrexate, radiopaque contrast agents, or streptozotocin.

12. A method according to one of aspects 1-5, wherein said correlating step comprises assigning a diagnosis of the occurrence or nonoccurrence of one or more of an injury to renal function, reduced renal function, or ARF to the subject based on the assay result(s).

13. A method according to one of aspects 1-5, wherein said correlating step comprises assessing whether or not renal function is improving or worsening in a subject who has suffered from an injury to renal function, reduced renal function, or ARF based on the assay result(s).

14. A method according to one of aspects 1-5, wherein said method is a method of diagnosing the occurrence or nonoccurrence of an injury to renal function in said subject.

15. A method according to one of aspects 1-5, wherein said method is a method of diagnosing the occurrence or nonoccurrence of reduced renal function in said subject.

16. A method according to one of aspects 1-5, wherein said method is a method of diagnosing the occurrence or nonoccurrence of acute renal failure in said subject.

17. A method according to one of aspects 1-5, wherein said method is a method of diagnosing the occurrence or nonoccurrence of a need for renal replacement therapy in said subject.

18. A method according to one of aspects 1-5, wherein said method is a method of diagnosing the occurrence or nonoccurrence of a need for renal transplantation in said subject.

19. A method according to one of aspects 1-5, wherein said method is a method of assigning a risk of the future occurrence or nonoccurrence of an injury to renal function in said subject.

20. A method according to one of aspects 1-5, wherein said method is a method of assigning a risk of the future occurrence or nonoccurrence of reduced renal function in said subject.

21. A method according to one of aspects 1-5, wherein said method is a method of assigning a risk of the future occurrence or nonoccurrence of acute renal failure in said subject.

22. A method according to one of aspects 1-5, wherein said method is a method of assigning a risk of the future occurrence or nonoccurrence of a need for renal replacement therapy in said subject.

23. A method according to one of aspects 1-5, wherein said method is a method of assigning a risk of the future occurrence or nonoccurrence of a need for renal transplantation in said subject.

24. A method according to one of aspects 1-5, wherein said one or more future changes in renal status comprise one or more of a future injury to renal function, future reduced renal function, future improvement in renal function, and future acute renal failure (ARF) within 72 hours of the time at which the body fluid sample is obtained.

25. A method according to one of aspects 1-5, wherein said one or more future changes in renal status comprise one or more of a future injury to renal function, future reduced renal function, future improvement in renal function, and future acute renal failure (ARF) within 48 hours of the time at which the body fluid sample is obtained.

26. A method according to one of aspects 1-5, wherein said one or more future changes in renal status comprise one or more of a future injury to renal function, future reduced renal function, future improvement in renal function, and future acute renal failure (ARF) within 24 hours of the time at which the body fluid sample is obtained.

27. A method according to one of aspects 1-5, wherein the subject is in RIFLE stage 0 or R.

28. A method according to aspect 27, wherein the subject is in RIFLE stage 0, and said correlating step comprises assigning a likelihood that the subject will reach RIFLE stage R, I or F within 72 hours.

29. A method according to aspect 28, wherein the subject is in RIFLE stage 0, and said correlating step comprises assigning a likelihood that the subject will reach RIFLE stage I or F within 72 hours.

30. A method according to aspect 28, wherein the subject is in RIFLE stage 0, and said correlating step comprises assigning a likelihood that the subject will reach RIFLE stage F within 72 hours.

31. A method according to aspect 27, wherein the subject is in RIFLE stage 0 or R, and said correlating step comprises assigning a likelihood that the subject will reach RIFLE stage I or F within 72 hours.

32. A method according to aspect 31, wherein the subject is in RIFLE stage 0 or R, and said correlating step comprises assigning a likelihood that the subject will reach RIFLE stage F within 72 hours.

33. A method according to aspect 27, wherein the subject is in RIFLE stage R, and said correlating step comprises assigning a likelihood that the subject will reach RIFLE stage I or F within 72 hours.

34. A method according to aspect 33, wherein the subject is in RIFLE stage R, and said correlating step comprises assigning a likelihood that the subject will reach RIFLE stage F within 72 hours.

35. A method according to one of aspects 1-5, wherein the subject is in RIFLE stage 0, R, or I, and said correlating step comprises assigning a likelihood that the subject will reach RIFLE stage F within 72 hours.

36. A method according to aspect 35, wherein the subject is in RIFLE stage I, and said correlating step comprises assigning a likelihood that the subject will reach RIFLE stage F within 72 hours.

37. A method according to aspect 28, wherein said correlating step comprises assigning a likelihood that the subject will reach RIFLE stage R, I or F within 48 hours.

38. A method according to aspect 29, wherein said correlating step comprises assigning a likelihood that the subject will reach RIFLE stage I or F within 48 hours.

39. A method according to aspect 30, wherein said correlating step comprises assigning a likelihood that the subject will reach RIFLE stage F within 48 hours.

40. A method according to aspect 31, wherein said correlating step comprises assigning a likelihood that the subject will reach RIFLE stage I or F within 48 hours.

41. A method according to aspect 32, wherein said correlating step comprises assigning a likelihood that the subject will reach RIFLE stage F within 48 hours.

42. A method according to aspect 33, wherein said correlating step comprises assigning a likelihood that the subject will reach RIFLE stage I or F within 48 hours.

43. A method according to aspect 34, wherein said correlating step comprises assigning a likelihood that the subject will reach RIFLE stage F within 48 hours.

44. A method according to aspect 35, wherein said correlating step comprises assigning a likelihood that the subject will reach RIFLE stage F within 48 hours.

45. A method according to aspect 36, wherein said correlating step comprises assigning a likelihood that the subject will reach RIFLE stage F within 48 hours.

46. A method according to aspect 28, wherein said correlating step comprises assigning a likelihood that the subject will reach RIFLE stage R, I or F within 24 hours.

47. A method according to aspect 29, wherein said correlating step comprises assigning a likelihood that the subject will reach RIFLE stage I or F within 24 hours.

48. A method according to aspect 30, wherein said correlating step comprises assigning a likelihood that the subject will reach RIFLE stage F within 24 hours.

49. A method according to aspect 31, wherein said correlating step comprises assigning a likelihood that the subject will reach RIFLE stage I or F within 24 hours.

50. A method according to aspect 32, wherein said correlating step comprises assigning a likelihood that the subject will reach RIFLE stage F within 24 hours.

51. A method according to aspect 33, wherein said correlating step comprises assigning a likelihood that the subject will reach RIFLE stage I or F within 24 hours.

52. A method according to aspect 34, wherein said correlating step comprises assigning a likelihood that the subject will reach RIFLE stage F within 24 hours.

53. A method according to aspect 35, wherein said correlating step comprises assigning a likelihood that the subject will reach RIFLE stage F within 24 hours.

54. A method according to aspect 36, wherein said correlating step comprises assigning a likelihood that the subject will reach RIFLE stage F within 24 hours.

55. A method according to one of aspects 1-5, wherein the subject is not in acute renal failure.

56. A method according to one of aspects 1-5, wherein the subject has not experienced a 1.5-fold or greater increase in serum creatinine over a baseline value determined prior to the time at which the body fluid sample is obtained.

57. A method according to one of aspects 1-5, wherein the subject has a urine output of at least 0.5 ml/kg/hr over the 6 hours preceding the time at which the body fluid sample is obtained.

58. A method according to one of aspects 1-5, wherein the subject has not experienced an increase of 0.3 mg/dL or greater in serum creatinine over a baseline value determined prior to the time at which the body fluid sample is obtained.

59. A method according to one of aspects 1-5, wherein the subject (i) has not experienced a 1.5-fold or greater increase in serum creatinine over a baseline value determined prior to the time at which the body fluid sample is obtained, (ii) has a urine output of at least 0.5 ml/kg/hr over the 6 hours preceding the time at which the body fluid sample is obtained, and (iii) has not experienced an increase of 0.3 mg/dL or greater in serum creatinine over a baseline value determined prior to the time at which the body fluid sample is obtained.

60. A method according to one of aspects 1-5, wherein the subject has not experienced a 1.5-fold or greater increase in serum creatinine over a baseline value determined prior to the time at which the body fluid sample is obtained.

61. A method according to one of aspects 1-5, wherein the subject has a urine output of at least 0.5 ml/kg/hr over the 6 hours preceding the time at which the body fluid sample is obtained.

62. A method according to one of aspects 1-5, wherein the subject (i) has not experienced a 1.5-fold or greater increase in serum creatinine over a baseline value determined prior to the time at which the body fluid sample is obtained, (ii) has a urine output of at least 0.5 ml/kg/hr over the 12 hours preceding the time at which the body fluid sample is obtained, and (iii) has not experienced an increase of 0.3 mg/dL or greater in serum creatinine over a baseline value determined prior to the time at which the body fluid sample is obtained.

63. A method according to one of aspects 1-5, wherein said correlating step comprises assigning one or more of: a likelihood that within 72 hours the subject will (i) experience a 1.5-fold or greater increase in serum creatinine (ii) have a urine output of less than 0.5 ml/kg/hr over a 6 hour period, or (iii) experience an increase of 0.3 mg/dL or greater in serum creatinine.

64. A method according to aspect 63, wherein said correlating step comprises assigning one or more of: a likelihood that within 48 hours the subject will (i) experience a 1.5-fold or greater increase in serum creatinine (ii) have a urine output of less than 0.5 ml/kg/hr over a 6 hour period, or (iii) experience an increase of 0.3 mg/dL or greater in serum creatinine.

65. A method according to aspect 63, wherein said correlating step comprises assigning one or more of: a likelihood that within 24 hours the subject will (i) experience a 1.5-fold or greater increase in serum creatinine (ii) have a urine output of less than 0.5 ml/kg/hr over a 6 hour period, or (iii) experience an increase of 0.3 mg/dL or greater in serum creatinine.

66. A method according to aspect 63, wherein said correlating step comprises assigning a likelihood that within 72 hours the subject will experience a 1.5-fold or greater increase in serum creatinine.

67. A method according to aspect 63, wherein said correlating step comprises assigning a likelihood that within 72 hours the subject will have a urine output of less than 0.5 ml/kg/hr over a 6 hour period.

68. A method according to aspect 63, wherein said correlating step comprises assigning a likelihood that within 72 hours the subject will experience an increase of 0.3 mg/dL or greater in serum creatinine.

69. A method according to aspect 63, wherein said correlating step comprises assigning a likelihood that within 48 hours the subject will experience a 1.5-fold or greater increase in serum creatinine.

70. A method according to aspect 63, wherein said correlating step comprises assigning a likelihood that within 48 hours the subject will have a urine output of less than 0.5 ml/kg/hr over a 6 hour period.

71. A method according to aspect 63, wherein said correlating step comprises assigning a likelihood that within 48 hours the subject will experience an increase of 0.3 mg/dL or greater in serum creatinine.

72. A method according to aspect 63, wherein said correlating step comprises assigning a likelihood that within 24 hours the subject will experience a 1.5-fold or greater increase in serum creatinine.

73. A method according to aspect 63, wherein said correlating step comprises assigning a likelihood that within 24 hours the subject will have a urine output of less than 0.5 ml/kg/hr over a 6 hour period.

74. A method according to aspect 63, wherein said correlating step comprises assigning a likelihood that within 24 hours the subject will experience an increase of 0.3 mg/dL or greater in serum creatinine.

75. A method according to one of aspects 1-5, wherein the subject has not experienced a 2-fold or greater increase in serum creatinine over a baseline value determined prior to the time at which the body fluid sample is obtained.

76. A method according to one of aspects 1-5, wherein the subject has a urine output of at least 0.5 ml/kg/hr over the 12 hours preceding the time at which the body fluid sample is obtained.

77. A method according to one of aspects 1-5, wherein the subject (i) has not experienced a 2-fold or greater increase in serum creatinine over a baseline value determined prior to the time at which the body fluid sample is obtained, (ii) has a urine output of at least 0.5 ml/kg/hr over the 2 hours preceding the time at which the body fluid sample is obtained, and (iii) has not experienced an increase of 0.3 mg/dL or greater in serum creatinine over a baseline value determined prior to the time at which the body fluid sample is obtained.

78. A method according to one of aspects 1-5, wherein the subject has not experienced a 3-fold or greater increase in serum creatinine over a baseline value determined prior to the time at which the body fluid sample is obtained.

79. A method according to one of aspects 1-5, wherein the subject has a urine output of at least 0.3 ml/kg/hr over the 24 hours preceding the time at which the body fluid sample is obtained, or anuria over the 12 hours preceding the time at which the body fluid sample is obtained.

80. A method according to one of aspects 1-5, wherein the subject (i) has not experienced a 3-fold or greater increase in serum creatinine over a baseline value determined prior to the time at which the body fluid sample is obtained, (ii) has a urine output of at least 0.3 ml/kg/hr over the 24 hours preceding the time at which the body fluid sample is obtained, or anuria over the 12 hours preceding the time at which the body fluid sample is obtained, and (iii) has not experienced an increase of 0.3 mg/dL or greater in serum creatinine over a baseline value determined prior to the time at which the body fluid sample is obtained.

81. A method according to one of aspects 1-5, wherein said correlating step comprises assigning one or more of: a likelihood that within 72 hours the subject will (i) experience a 2-fold or greater increase in serum creatinine (ii) have a urine output of less than 0.5 ml/kg/hr over a 12 hour period, or (iii) experience an increase of 0.3 mg/dL or greater in serum creatinine.

82. A method according to aspect 81, wherein said correlating step comprises assigning one or more of: a likelihood that within 48 hours the subject will (i) experience a 2-fold or greater increase in serum creatinine (ii) have a urine output of less than 0.5 ml/kg/hr over a 6 hour period, or (iii) experience an increase of 0.3 mg/dL or greater in serum creatinine.

83. A method according to aspect 81, wherein said correlating step comprises assigning one or more of: a likelihood that within 24 hours the subject will (i) experience a 2-fold or greater increase in serum creatinine, or (ii) have a urine output of less than 0.5 ml/kg/hr over a 6 hour period.

84. A method according to aspect 81, wherein said correlating step comprises assigning a likelihood that within 72 hours the subject will experience a 2-fold or greater increase in serum creatinine.

85. A method according to aspect 81, wherein said correlating step comprises assigning a likelihood that within 72 hours the subject will have a urine output of less than 0.5 ml/kg/hr over a 6 hour period.

86. A method according to aspect 81, wherein said correlating step comprises assigning a likelihood that within 48 hours the subject will experience a 2-fold or greater increase in serum creatinine.

87. A method according to aspect 81, wherein said correlating step comprises assigning a likelihood that within 48 hours the subject will have a urine output of less than 0.5 ml/kg/hr over a 6 hour period.

88. A method according to aspect 81, wherein said correlating step comprises assigning a likelihood that within 24 hours the subject will experience a 2-fold or greater increase in serum creatinine.

89. A method according to aspect 81, wherein said correlating step comprises assigning a likelihood that within 24 hours the subject will have a urine output of less than 0.5 ml/kg/hr over a 6 hour period.

90. A method according to one of aspects 1-5, wherein said correlating step comprises assigning one or more of: a likelihood that within 72 hours the subject will (i) experience a 3-fold or greater increase in serum creatinine, or (ii) have a urine output of less than 0.3 ml/kg/hr over a 24 hour period or anuria over a 12 hour period.

91. A method according to aspect 90, wherein said correlating step comprises assigning one or more of: a likelihood that within 48 hours the subject will (i) experience a 3-fold or greater increase in serum creatinine, or (ii) have a urine output of less than 0.3 ml/kg/hr over a 24 hour period or anuria over a 12 hour period.

92. A method according to aspect 90, wherein said correlating step comprises assigning one or more of: a likelihood that within 24 hours the subject will (i) experience a 3-fold or greater increase in serum creatinine, or (ii) have a urine output of less than 0.3 ml/kg/hr over a 24 hour period or anuria over a 12 hour period.

93. A method according to aspect 90, wherein said correlating step comprises assigning a likelihood that within 72 hours the subject will experience a 3-fold or greater increase in serum creatinine.

94. A method according to aspect 90, wherein said correlating step comprises assigning a likelihood that within 72 hours the subject will have a urine output of less than 0.3 ml/kg/hr over a 24 hour period or anuria over a 12 hour period.

95. A method according to aspect 90, wherein said correlating step comprises assigning a likelihood that within 48 hours the subject will experience a 3-fold or greater increase in serum creatinine.

96. A method according to aspect 90, wherein said correlating step comprises assigning a likelihood that within 48 hours the subject will have a urine output of less than 0.3 ml/kg/hr over a 24 hour period or anuria over a 12 hour period.

97. A method according to aspect 90, wherein said correlating step comprises assigning a likelihood that within 24 hours the subject will experience a 3-fold or greater increase in serum creatinine.

98. A method according to aspect 90, wherein said correlating step comprises assigning a likelihood that within 24 hours the subject will have a urine output of less than 0.3 ml/kg/hr over a 24 hour period or anuria over a 12 hour period.

99. A method according to one of aspects 1-98, wherein the body fluid sample is a urine sample.

100. A method according to one of aspects 1-99, wherein said method comprises performing assays that detect one, two or three, or more of Proheparin-binding EGF-like growth factor, Tenascin C, Angiopoietin-related protein 4, Fibroblast growth factor 19, Fibroblast growth factor 21, Heparin-binding growth factor 1, Angiopoietin-related protein 6, Proepiregulin, Probetacellulin, Amphiregulin, Angiogenin, Thrombospondin-2, and Collagen alpha-1(XVIII) chain.

101. Measurement of one or more biomarkers selected from the group consisting of Proheparin-binding EGF-like growth factor, Tenascin C, Angiopoietin-related protein 4, Fibroblast growth factor 19, Fibroblast growth factor 21, Heparin-binding growth factor 1, Angiopoietin-related protein 6, Proepiregulin, Probetacellulin, Amphiregulin, Angiogenin, Thrombospondin-2, and Collagen alpha-1(XVIII) chain for the evaluation of renal injury.

102. Measurement of one or more biomarkers selected from the group consisting of Proheparin-binding EGF-like growth factor, Tenascin C, Angiopoietin-related protein 4, Fibroblast growth factor 19, Fibroblast growth factor 21, Heparin-binding growth factor 1, Angiopoietin-related protein 6, Proepiregulin, Probetacellulin, Amphiregulin, Angiogenin, Thrombospondin-2, and Collagen alpha-1(XVIII) chain for the evaluation of acute renal injury.

103. A kit, comprising:
reagents for performing one or more assays configured to detect one or more kidney injury markers selected from the group consisting of Proheparin-binding EGF-like growth factor, Tenascin C, Angiopoietin-related protein 4, Fibroblast growth factor 19, Fibroblast growth factor 21, Heparin-binding growth factor 1, Angiopoietin-related protein 6, Proepiregulin, Probetacellulin, Amphiregulin, Angiogenin, Thrombospondin-2, and Collagen alpha-1(XVIII) chain.

104. A kit according to aspect 103, wherein said reagents comprise one or more binding reagents, each of which specifically binds one of said of kidney injury markers.

105. A kit according to aspect 104, wherein a plurality of binding reagents are contained in a single assay device.

106. A kit according to aspect 103, wherein at least one of said assays is configured as a sandwich binding assay.

107. A kit according to aspect 103, wherein at least one of said assays is configured as a competitive binding assay.

108. A kit according to one of aspects 103-107, wherein said one or more assays comprise assays that detect one, two or three, or more of Proheparin-binding EGF-like growth factor, Tenascin C, Angiopoietin-related protein 4, Fibroblast growth factor 19, Fibroblast growth factor 21, Heparin-binding growth factor 1, Angiopoietin-related protein 6, Proepiregulin, Probetacellulin, Amphiregulin, Angiogenin, Thrombospondin-2, and Collagen alpha-1(XVIII) chain.

**Claims**

1. A method for evaluating renal status in a subject, comprising:

performing one or more assays configured to detect Tenascin C in a body fluid sample obtained from the subject to provide an assay result; and
correlating the assay result(s) to the renal status of the subject, wherein said correlating step comprises assigning a likelihood of one or more future changes in renal status to the subject based on the assay result(s).

2. A method according to claim 1, wherein said correlation step comprises correlating the assay result(s) to one or more of risk stratification, diagnosis, staging, prognosis, classifying and monitoring of the renal status of the subject.

3. A method according to claim 1, wherein said one or more future changes in renal status comprise one or more of a future injury to renal function, future reduced renal function, future improvement in renal function, future acute kidney injury (AKI), a clinical outcome related to a renal injury suffered by the subject, and death.

4. A method according to claim 1, comprising predicting a decreased or increased risk that the subject will recover from an injury to renal function or from AKI.

5. A method according to one of claims 1-4, wherein a plurality of assay results are combined using a function that converts the plurality of assay results into a single composite result.

6. A method according to one of claims 1-5, wherein the likelihood of one or more future changes in renal status is that an event of interest is more or less likely to occur within a period of time selected from the group consisting of 90 days, 30 days, 21 days, 14 days, 7 days, 5 days, 96 hours, 72 hours, 48 hours, 36 hours, 24 hours, and 12 hours of the time at which the body fluid sample is obtained from the subject.

7. The method according to one of claims 1-6, wherein the subject is suffering from an injury to renal function, reduced renal function and/or acute kidney injury.

8. A method according to one of claims 1-4, wherein the subject is selected for evaluation of renal status based on:

   (i) the pre-existence in the subject of one or more known risk factors for prerenal, intrinsic renal, or postrenal AKI;
   (ii) an existing diagnosis of one or more of congestive heart failure, preeclampsia, eclampsia, diabetes mellitus, hypertension, coronary artery disease, proteinuria, renal insufficiency, glomerular filtration below the normal range, cirrhosis, serum creatinine above the normal range, sepsis, injury to renal function, reduced renal function, or AKI;
   (iii) undergoing or having undergone major vascular surgery, coronary artery bypass, or other cardiac surgery; or
   (iv) exposure to NSAIDs, cyclosporines, tacrolimus, aminoglycosides, foscarnet, ethylene glycol, hemoglobin, myoglobin, ifosfamide, heavy metals, methotrexate, radiopaque contrast agents, or streptozotocin.

9. A method according to one of claims 1-4, wherein said correlating step comprises assessing whether or not renal function is improving or worsening in a subject who has suffered from an injury to renal function, reduced renal function, or AKI based on the assay result(s).

10. A method according to one of claims 1-4, wherein said method is a method of assigning a risk of the future occurrence or nonoccurrence of:

    (i) reduced renal function in said subject;
    (ii) acute renal failure in said subject;
    (iii) a need for renal replacement therapy in said subject; or
    (iv) a need for renal transplantation in said subject.

11. Use of Tenascin C for the evaluation of renal injury in a body fluid sample obtained from a subject, the use comprising performing one or more assays configured to detect Tenascin C and correlating the assay result(s) to the renal status of the subject, wherein said correlating step comprises assigning a likelihood of one or more future changes in renal status to the subject based on the assay result(s).

12. The use according to claim 11, wherein said one or more future changes in renal status comprise one or more of a future injury to renal function, future reduced renal function, future improvement in renal function, future acute kidney injury (AKI), a clinical outcome related to a renal injury suffered by the subject, and death.

13. The use according to claim 11 or 12, wherein the likelihood of one or more future changes in renal status is that an event of interest is more or less likely to occur within a period of time selected from the group consisting of 90 days, 30 days, 21 days, 14 days, 7 days, 5 days, 96 hours, 72 hours, 48 hours, 36 hours, 24 hours, and 12 hours of the time at which the body fluid sample is obtained from the subject.

14. The use according to one of claims 11-13, wherein the subject is suffering from an injury to renal function, reduced renal function and/or acute kidney injury.

15. The use according to claim 11, wherein said correlating step comprises assessing whether or not renal function is improving or worsening in a subject who has suffered from an injury to renal function, reduced renal function, or AKI based on the assay result(s).

Europäisches
Patentamt

European
Patent Office

Office européen
des brevets

# EUROPEAN SEARCH REPORT

Application Number

EP 20 20 1404

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | J. H. HORSTRUP: "Elevation of serum and urine levels of TIMP-1 and tenascin in patients with renal disease", NEPHROLOGY DIALYSIS TRANSPLANTATION, vol. 17, no. 6, 1 June 2002 (2002-06-01), pages 1005-1013, XP055529915, DOI: 10.1093/ndt/17.6.1005 * abstract; figure 4 * * page 1008, column 2, paragraph 2 * ----- | 1-15 | INV. G01N33/53 G01N33/68 C12Q1/6883 |
| A | TURBAT-HERRERA E A ET AL: "INTEGRATED EXPRESSION OF GLOMERULAR EXTRACELLULAR MATRIX PROTEINS AND BETA1 INTEGRINS IN MONOCLONAL LIGHT CHAIN-RELATED RENAL DISEASES", MODERN PATHOLOGY, NATURE PUBLISHING GROUP, GB, vol. 10, no. 5, 1 May 1997 (1997-05-01), pages 485-495, XP009007002, ISSN: 0893-3952 * the whole document * ----- | 1-15 | |
| A | SOPHIE LIABEUF ET AL: "High circulating levels of large splice variants of tenascin-C is associated with mortality and cardiovascular disease in chronic kidney disease patients", ATHEROSCLEROSIS, ELSEVIER, AMSTERDAM, NL, vol. 215, no. 1, 25 November 2010 (2010-11-25), pages 116-124, XP028168926, ISSN: 0021-9150, DOI: 10.1016/J.ATHEROSCLEROSIS.2010.11.038 [retrieved on 2010-12-05] ----- -/-- | 1-15 | TECHNICAL FIELDS SEARCHED (IPC) G01N C12Q |

The present search report has been drawn up for all claims

| Place of search | Date of completion of the search | Examiner |
|---|---|---|
| Munich | 26 April 2021 | Tilkorn, A |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

| | Europäisches Patentamt<br>European Patent Office<br>Office européen des brevets | **EUROPEAN SEARCH REPORT** | **Application Number**<br>EP 20 20 1404 |

## DOCUMENTS CONSIDERED TO BE RELEVANT

| Category | Citation of document with indication, where appropriate,<br>of relevant passages | Relevant<br>to claim | CLASSIFICATION OF THE<br>APPLICATION (IPC) |
|---|---|---|---|
| A | WO 2010/091236 A1 (ASTUTE MEDICAL INC [US]; ANDERBERG JOSEPH [US]; GRAY JEFF [US]; MCPHER) 12 August 2010 (2010-08-12)<br>* the whole document *<br>----- | 1-15 | |
| A | LIANG X L ET AL: "Beyond Early Diagnosis: Prognostic Biomarkers for Monitoring Acute Kidney Injury",<br>HONG KONG JOURNAL OF NEPHROLOGY, XX, HG,<br>vol. 12, no. 2,<br>1 October 2010 (2010-10-01), pages 45-49,<br>XP027547649,<br>ISSN: 1561-5413<br>[retrieved on 2010-10-01]<br>* the whole document *<br>----- | 1-15 | |
| | | | **TECHNICAL FIELDS<br>SEARCHED       (IPC)** |

The present search report has been drawn up for all claims

| Place of search | Date of completion of the search | Examiner |
|---|---|---|
| Munich | 26 April 2021 | Tilkorn, A |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

page 2 of 2

**EP 3 828 544 A1**

## ANNEX TO THE EUROPEAN SEARCH REPORT
## ON EUROPEAN PATENT APPLICATION NO.

EP 20 20 1404

This annex lists the patent family members relating to the patent documents cited in the above-mentioned European search report.
The members are as contained in the European Patent Office EDP file on
The European Patent Office is in no way liable for these particulars which are merely given for the purpose of information.

26-04-2021

| Patent document cited in search report | Publication date | Patent family member(s) | Publication date |
|---|---|---|---|
| WO 2010091236 A1 | 12-08-2010 | AU 2010210540 A1 | 15-09-2011 |
| | | CA 2751435 A1 | 12-08-2010 |
| | | CN 102439441 A | 02-05-2012 |
| | | EP 2394166 A1 | 14-12-2011 |
| | | HK 1167455 A1 | 30-11-2012 |
| | | JP 2012517594 A | 02-08-2012 |
| | | NZ 594771 A | 28-06-2013 |
| | | NZ 601575 A | 29-08-2014 |
| | | US 2012053072 A1 | 01-03-2012 |
| | | WO 2010091236 A1 | 12-08-2010 |

EPO FORM P0459

For more details about this annex : see Official Journal of the European Patent Office, No. 12/82

## REFERENCES CITED IN THE DESCRIPTION

**Patent documents cited in the description**

- US 61430977
- US 61430978 B
- US 61430979 B
- US 61430980 B
- US 61430981 B
- US 61430982 B
- US 61430983 B
- US 61430984 B
- US 61430985 B
- US 61430986 B
- US 61430987 B
- US 61430988 B
- US 61430989 B
- US 6143576 A
- US 6113855 A
- US 6019944 A
- US 5985579 A
- US 5947124 A
- US 5939272 A
- US 5922615 A
- US 5885527 A
- US 5851776 A
- US 5824799 A
- US 5679526 A
- US 5525524 A
- US 5480792 A
- US 5631171 A
- US 5955377 A
- US 5571698 A, Ladner
- US 6057098 A

**Non-patent literature cited in the description**

- Harrison's Principles of Internal Medicine. McGraw Hill, 1741-1830
- Current Medical Diagnosis & Treatment. McGraw Hill, 2008, 785-815
- Merck Manual
- **PRAUGHT ; SHLIPAK.** *Curr Opin Nephrol Hypertens,* 2005, vol. 14, 265-270
- **CHERTOW et al.** *J Am Soc Nephrol,* 2005, vol. 16, 3365-3370
- **LASSNIGG.** *J Am Soc Nephrol,* 2004, vol. 15, 1597-1605
- **BELLOMO et al.** *Crit Care,* 2004, vol. 8 (4), R204-12
- **KELLUM.** *Crit. Care Med.,* 2008, vol. 36, 141-45
- **RICCI et al.** *Kidney Int.,* 2008, vol. 73, 538-546
- **MEHTA et al.** *Crit. Care,* 2007, vol. 11, R31
- **MCCOLLOUGH et al.** *Rev Cardiovasc Med.,* 2006, vol. 7 (4), 177-197
- **THAKAR et al.** *J. Am. Soc. Nephrol.,* 2005, vol. 16, 162-68
- **MEHRAN et al.** *J. Am. Coll. Cardiol.,* 2004, vol. 44, 1393-99
- **WIJEYSUNDERA et al.** *JAMA,* 2007, vol. 297, 1801-9
- **GOLDSTEIN ; CHAWLA.** *Clin. J. Am. Soc. Nephrol.,* 2010, vol. 5, 943-49
- **CHAWLA et al.** *Kidney Intl.,* 2005, vol. 68, 2274-80
- The Immunoassay Handbook. Stockton Press, 1994
- Fundamental Immunology. Raven Press, 1993
- **WILSON.** *J. Immunol. Methods,* 1994, vol. 175, 267-273
- **YARMUSH.** *J. Biochem. Biophys. Methods,* 1992, vol. 25, 85-97
- **WARD et al.** *Nature,* 1989, vol. 341, 544-546
- **VAN ERP et al.** *J. Immunoassay,* 1991, vol. 12, 425-43
- **NELSON ; GRISWOLD.** *Comput. Methods Programs Biomed.,* 1988, vol. 27, 65-8
- **CWIRLA et al.** *Proc. Natl. Acad. Sci. USA,* 1990, vol. 87, 6378-82
- **DEVLIN et al.** *Science,* 1990, vol. 249, 404-6
- **SCOTT ; SMITH.** *Science,* 1990, vol. 249, 386-88
- **FISCHER et al.** *Intensive Care Med.,* 2003, vol. 29, 1043-51
- **BAGSHAW et al.** *Nephrol. Dial. Transplant.,* 2008, vol. 23, 1203-1210
- Merck Manual of Diagnosis and Therapy. Merck Research Laboratories, 1999
- **HANLEY, J. A. ; MCNEIL, B.J.** The meaning and use of the area under a receiver operating characteristic (ROC) curve. *Radiology,* 1982, vol. 143, 29-36